# EUROPEAN PATENT APPLICATION

(11) **EP 4 806 446 A2**
(43) Date of publication of application: **16.09.2026**
(21) Application number: 26195737.7
(22) Date of filing: 24.05.2024
(51) Int. Cl.: A61K 35/00

(54) **RAS INHIBITORS**

(30) Priority: 26.05.2023 US 202363469283 P
(62) Document number(s) of the earlier application(s) in accordance with Art. 76 EPC: 24816211.7 / 4 720 072
(71) Applicant: Revolution Medicines, Inc., Redwood City, CA 94063 (US)
(72) Inventor: KNOX, John E., Redwood City, CA 94063 (US); KOLTUN, Elena S., Redwood City, CA 94063 (US); PARSONS, Dylan E., Redwood City, CA 94063 (US); TOMLINSON, Aidan, Redwood City, CA 94063 (US); BURNETT, G. Leslie, Redwood City, CA 94063 (US); CREGG, James, Redwood City, CA 94063 (US); GILL, Adrian L., Redwood City, CA 94063 (US); BUCKL, Andreas, Redwood City, CA 94063 (US)
(74) Representative: Mewburn Ellis LLP

(57) **Abstract**

The disclosure features macrocyclic compounds, and pharmaceutical compositions and protein complexes thereof, capable of inhibiting Ras proteins, and their uses in the treatment of cancers.

## Description

### Background

The vast majority of small molecule drugs act by binding a functionally important pocket on a target protein, thereby modulating the activity of that protein. For example, cholesterol-lowering drugs known as statins bind the enzyme active site of HMG-CoA reductase, thus preventing the enzyme from engaging with its substrates. The fact that many such drug/target interacting pairs are known may have misled some into believing that a small molecule modulator could be discovered for most, if not all, proteins provided a reasonable amount of time, effort, and resources. This is far from the case. Current estimates are that only about 10% of all human proteins are targetable by small molecules. Bojadzic and Buchwald, Curr Top Med Chem 18: 674-699 (2019). The other 90% are currently considered refractory or intractable toward above-mentioned small molecule drug discovery. Such targets are commonly referred to as "undruggable." These undruggable targets include a vast and largely untapped reservoir of medically important human proteins. Thus, there exists a great deal of interest in discovering new molecular modalities capable of modulating the function of such undruggable targets.

It has been well established in literature that Ras proteins (K-Ras, H-Ras, and N-Ras) play an essential role in various human cancers and are therefore appropriate targets for anticancer therapy. Indeed, mutations in Ras proteins account for approximately 30% of all human cancers in the United States, many of which are fatal. Dysregulation of Ras proteins by activating mutations, overexpression or upstream activation is common in human tumors, and activating mutations in Ras are frequently found in human cancer. For example, activating mutations at codon 12 in Ras proteins function by inhibiting both GTPase-activating protein (GAP)-dependent and intrinsic hydrolysis rates of GTP, significantly skewing the population of Ras mutant proteins to the "on" (GTP-bound) state (Ras(ON)), leading to oncogenic MAPK signaling. Notably, Ras exhibits a picomolar affinity for GTP, enabling Ras to be activated even in the presence of low concentrations of this nucleotide. Mutations at codons 13 (e.g., G13C) and 61 (e.g., Q61K) of Ras are also responsible for oncogenic activity in some cancers.

Despite extensive drug discovery efforts against Ras during the last several decades, only two agents targeting the K-Ras G12C mutant have been approved in the U.S. (sotorasib and adagrasib). Additional efforts are needed to uncover additional medicines for cancers driven by the various Ras mutations.

### Summary

Provided herein are Ras inhibitors and compounds useful for studying Ras inhibition. The approach described herein entails formation of a high affinity three-component complex, or conjugate, between a synthetic ligand and two intracellular proteins which do not interact under normal physiological conditions: the target protein of interest (e.g., Ras), and a widely expressed cytosolic chaperone (presenter protein) in the cell (e.g., cyclophilin A). More specifically, in some embodiments, the inhibitors of Ras described herein induce a new binding pocket in Ras by driving formation of a high affinity tri-complex, or conjugate, between the Ras protein and the widely expressed cytosolic chaperone, cyclophilin A (CYPA). Without being bound by theory, the inventors believe that one way the inhibitory effect on Ras is effected by compounds of the invention and the complexes, or conjugates, they form is by steric occlusion of the interaction site between Ras and downstream effector molecules, such as RAF and PI3K, which are required for propagating the oncogenic signal.

As such, in some embodiments, the disclosure features a compound, or pharmaceutically acceptable salt thereof or a stereoisomer, of structural Formula I: 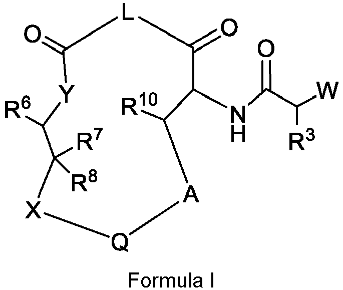 wherein:
Q is an optionally substituted 7- to 12-membered bicyclic arylene, an optionally substituted 7-to 12-membered bicyclic heteroarylene, or an optionally substituted 7- to 12-membered bicyclic heterocyclylene, wherein a first ring in Q is bonded to X, and a second ring in Q is bonded to A;
X is a bond; a straight chain C₁-C₃ alkylene optionally substituted with 1 to 3 substituents independently selected from fluoro, -CN, -C₁-C₃ alkyl, and -O-C₁-C₃ alkyl; -O-; -S(O)₀₋₂-; *-CH₂-O-; *-CH₂-S(O)₀₋₂-; *-O-CH₂-; or *-CH₂-S(O)₀₋₂-, wherein "*" represents a portion of X bound to -C(R⁷)(R⁸)-;
Y is -O-, -NH- or -N(C₁-C₃ alkyl)-;
R³ is optionally substituted C₁-C₆ alkyl, optionally substituted C₃-C₆ cycloalkyl, optionally substituted C₆-C₁₀ aryl, or optionally substituted 3- to 11-membered heterocyclyl;
W is: 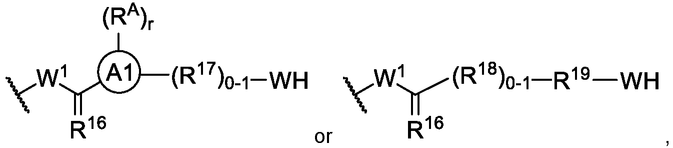
wherein:
   ring A1 is a 4- to 8-membered cycloalkyl or a 4- to 8-membered heterocyclyl;
   W¹ is -N(R²⁰)-, -O-, or -C(R^{20a})(R^{20b})-;
   each R^{A} is each independently halo, cyano, hydroxyl, optionally substituted amino, optionally substituted C₁-C₆ heteroalkyl, or optionally substituted C₁-C₃ alkyl;
   r is 0, 1, 2, 3, or 4;
   R¹⁷, if present, is optionally substituted C₁-C₆ heteroalkylene or optionally substituted C₁-C₆ alkylene;
   R¹⁸, if present, is optionally substituted C₁-C₄ alkylene;
   R¹⁹ is optionally substituted C₁-C₆ heteroalkylene, optionally substituted C₁-C₆ alkylene, -NH-, or
      -N(optionally substituted C₁-C₆ alkyl) or a saturated, nitrogen-containing 3- to 8-membered heterocyclyl;
   R²⁰ is hydrogen or -C₁-C₃ alkyl;
   R²⁰ is taken together with one R^{A}, the atoms to which they are respectively attached and any intervening atoms, to form an optionally substituted, 5- to 8-membered heterocyclyl that is fused or spiro-fused to ring A, or
   R²⁰ is taken together with any methylene unit in R¹⁸, or any methylene unit in R¹⁹, the atoms to which they are respectively attached and any intervening atoms, to form an optionally substituted, 5- to 8-membered heterocyclyl;
   each of R^{20a} and R^{20b} is, independently, hydrogen, or -C₁-C₃ alkyl, or R^{20a} and R^{20b} are taken together with the carbon atom to which they are bound to form a 3- to 6-membered cycloalkyl ring;
   R¹⁶ is O, S, N-CN, or N-O-C₁-C₃ alkyl;
   WH is 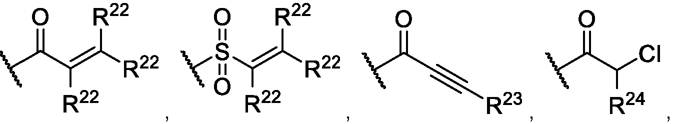 or 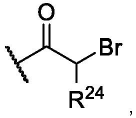
   each R²² is, independently, hydrogen, cyano, optionally substituted C₁-C₃ alkyl, or an optionally substituted 4- to 7-membered saturated heterocyclyl; or R²² is taken together with either of R¹⁷ or R¹⁹, the atoms to which they are attached and any intervening atoms to form an optionally substituted 5- to 8-membered ring system;
   R²³ is hydrogen, optionally substituted C₁-C₃ alkyl, or an optionally substituted 4- to 7-membered saturated heterocyclyl;
   R²⁴ is hydrogen, optionally substituted C₁-C₃ alkyl, or an optionally substituted 4- to 7-membered saturated heterocyclyl; or R²⁴ is taken together with either of R¹⁷ or R¹⁹, the atoms to which they are attached and any intervening atoms, to form an optionally substituted 5- to 8-membered ring system;
   A is optionally substituted C₂-C₄ alkylene, optionally substituted C₁-C₄ heteroalkylene, or optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   L has the structure of Formula VIIa or Vllb: 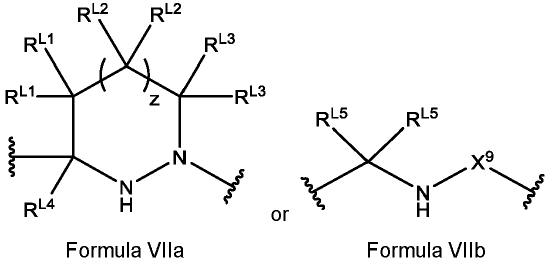
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 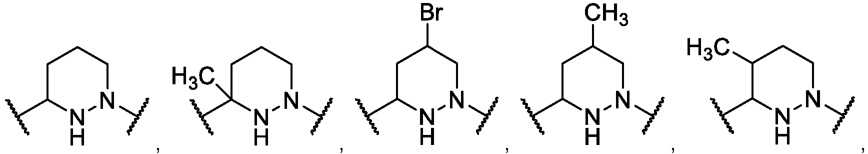 or 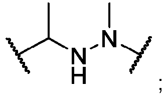
   R³ is optionally substituted C₁-C₆ alkyl, optionally substituted C₃-C₆ cycloalkyl, optionally substituted C₆ aryl, or optionally substituted 3- to 7-membered heterocyclyl;
   R¹⁰ is hydrogen, halogen, optionally substituted C₁-C₃ alkyl, or C₁-C₃ optionally substituted heteroalkyl;
   R⁷ is hydrogen, halogen, or optionally substituted C₁-C₃ alkyl;
   R⁸ is hydrogen, halogen, -OH, -CN, optionally substituted C₁-C₃ heteroalkyl, optionally substituted C₁-C₃ alkyl, optionally substituted C₂-C₆ alkenyl, optionally substituted C₂-C₆ alkynyl, optionally substituted C₆-C₁₀ aryl, optionally substituted 4- to 8-membered heteroaryl, optionally substituted C₃-C₆ cycloalkyl, or optionally substituted 3- to 7-membered heterocyclyl; or
   R⁷ and R⁸ are taken together to form =CH₂, an optionally substituted C₃-C₆ cycloalkyl, or a 3- to 7-membered saturated heterocyclyl; or
   R⁸ is taken together with a ring atom in Q, the carbon atom to which R⁷ is bound and X to form a 4- to 9-membered saturated or unsaturated heterocyclyl that is fused to Q;
   R⁶ is hydrogen or -CH₃;
   each R⁵ is, independently, halogen, optionally substituted C₁-C₃ alkyl, or optionally substituted C₁-C₃ haloalkyl; and
   p is 0, 1, 2, or 3.

In some embodiments, the disclosure features a compound, or a pharmaceutically acceptable salt, or a stereoisomer thereof of structural Formula IIa: 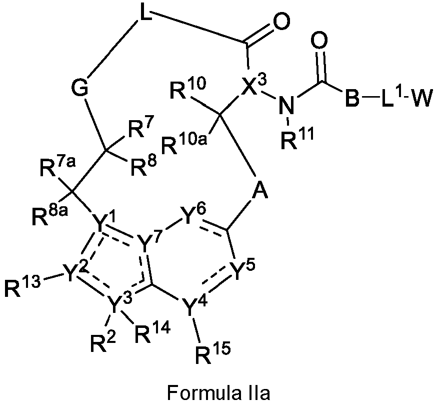
wherein the dotted lines represent zero, one, two, three, or four non-adjacent double bonds;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
L¹ is a linker;
W is hydrogen, cyano, S(O)₂R', optionally substituted amino, optionally substituted amido, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3- to 11-membered heterocycloalkyl, optionally substituted 3- to 8-membered cycloalkyl, or optionally substituted 3- to 8-membered heteroaryl;
L has the structure of Formula VIIa or VIIb: 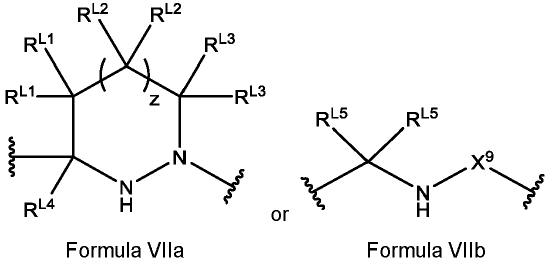
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 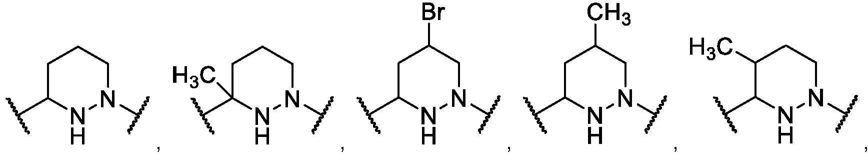 or 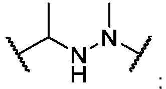
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R' is, independently, hydrogen or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl;
R¹⁴ is absent or R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ heteroalkyl, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halogen, optionally substituted C₁-C₃ alkyl, or combine with the carbon to which they are attached to form a carbonyl;
R^{7'} is hydrogen, halogen, or optionally substituted C₁-C₃ alkyl;
R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halogen, hydroxy, optionally substituted C₁-C₃ heteroalkyl, or optionally substituted C₁-C₃ alkyl;
R^{10a} is hydrogen or halogen;
R¹¹ is hydrogen or optionally substituted C₁-C₃ alkyl; and
R²¹ is hydrogen or optionally substituted C₁-C₃ alkyl.

In some embodiments, the disclosure features a compound, or a pharmaceutically acceptable salt, or a stereoisomer thereof, of structural Formula Ilb: 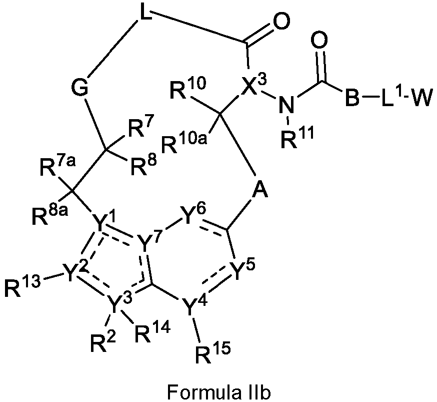
wherein the dotted lines represent zero, one, two, three, or four non-adjacent double bonds;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
L¹ is a linker;
W is a cross-linking group comprising a carbodiimide, an oxazoline, a thiazoline, a chloroethyl urea, a chloroethyl thiourea, a chloroethyl carbamate, a chloroethyl thiocarbamate, an aziridine, a trifluoromethyl ketone, a boronic acid, a boronic ester, an *N*-ethoxycarbonyl-2-ethoxy-1,2-dihydroquinoline (EEDQ), an iso--EEDQ or other EEDQ derivative, an epoxide, an oxazolium, or a glycal;
L has the structure of Formula VIIa or VIIb: 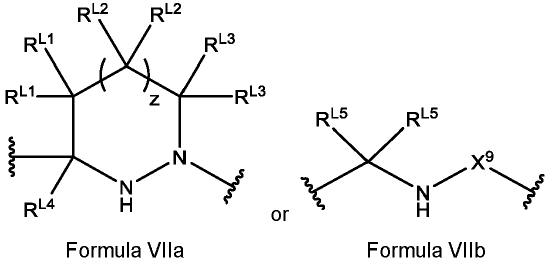
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 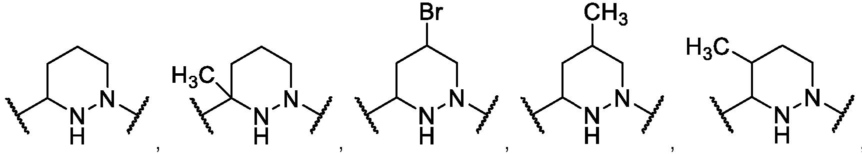 or 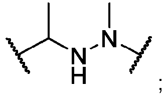
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, hydrogen or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ heteroalkyl, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halogen, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halogen, hydroxy, optionally substituted C₁-C₃ heteroalkyl, or optionally substituted C₁-C₃ alkyl;
R^{10a} is hydrogen or halogen;
R¹¹ is hydrogen or optionally substituted C₁-C₃ alkyl; and
R²¹ is hydrogen or optionally substituted C₁-C₃ alkyl.

In some embodiments, the disclosure features a compound, or a pharmaceutically acceptable salt, or a stereoisomer thereof, of structural Formula IIc: 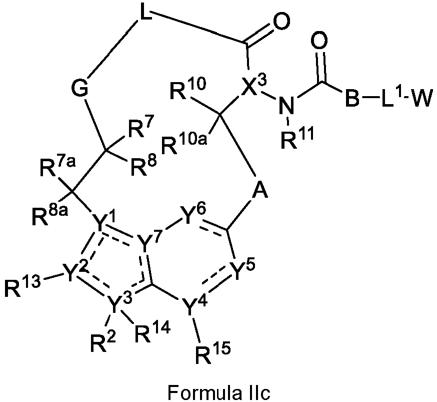
wherein the dotted lines represent zero, one, two, three, or four non-adjacent double bonds;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
G is optionally substituted C₁-C₄ alkylene, optionally substituted C₁-C₄ alkenylene, optionally substituted C₁-C₄ heteroalkylene, -C(O)O-**C**H(R⁶)- where C is bound to -C(R⁷R⁸)-, -C(O)NH-**C**H(R⁶)-where C is bound to -C(R⁷R⁸)-, optionally substituted C₁-C₄ heteroalkylene, or 3- to 8-membered heteroarylene;
L¹ is a linker;
W is a cross-linking group comprising a vinyl ketone, a vinyl sulfone, an ynone, a haloacetyl, or an alkynyl sulfone;
L has the structure of Formula VIIa or Vllb: 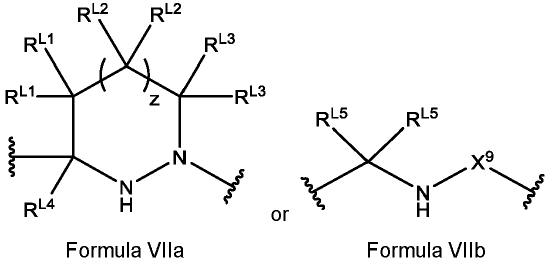
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 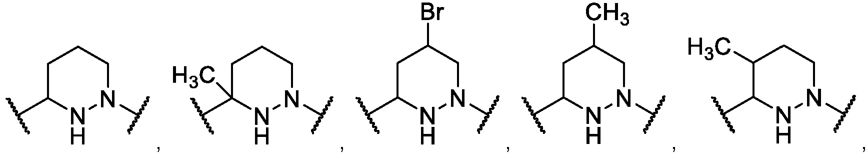 or 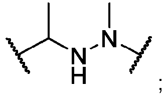
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, hydrogen or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ heteroalkyl, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halogen, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halogen, hydroxy, optionally substituted C₁-C₃ heteroalkyl, or optionally substituted C₁-C₃ alkyl;
R^{10a} is hydrogen or halogen;
R¹¹ is hydrogen or optionally substituted C₁-C₃ alkyl; and
R²¹ is hydrogen or optionally substituted C₁-C₃ alkyl.

In some embodiments, the disclosure features a compound, or a pharmaceutically acceptable salt, or a stereoisomer thereof, of structural Formula III: 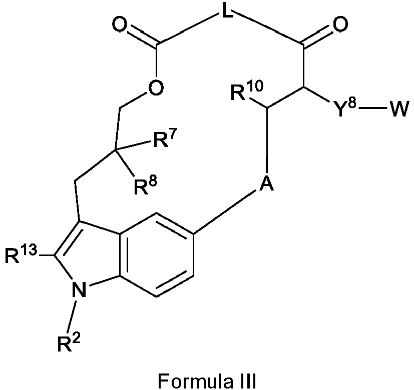
wherein A is optionally substituted 3- to 6-membered cycloalkylene, optionally substituted 3-to 6-membered heterocycloalkylene, optionally substituted 6-membered arylene, optionally substituted 5- to 6-membered heteroarylene, optionally substituted C₂-C₄ alkylene, optionally substituted C₁-C₄ heteroalkylene or optionally substituted C₂-C₄ alkenylene;
Y⁸ is 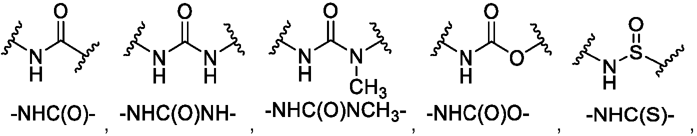 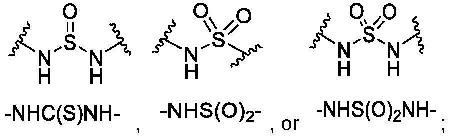
W is hydrogen, C₁-C₄ alkyl, optionally substituted C₁-C₃ heteroalkyl, optionally substituted 3-to 10-membered heterocycloalkyl, optionally substituted 3- to 10-membered cycloalkyl, optionally substituted 6- to 10-membered aryl, or optionally substituted 5- to 10-membered heteroaryl;
L has the structure of Formula VIIa or VIIb: 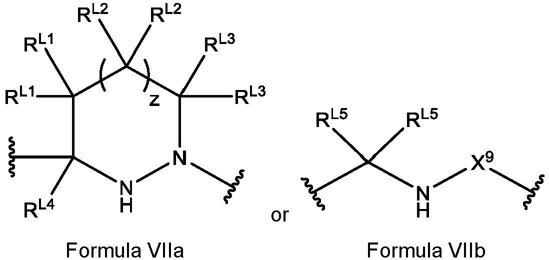
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 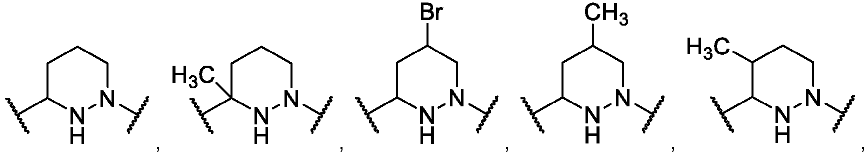 or 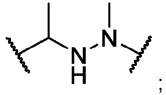
X⁴ and X⁵ are each, independently, CH₂, CH(CH₃) or NH;
R¹³ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 15-membered heterocycloalkyl, optionally substituted 6- to 10-membered aryl, or optionally substituted 5- to 10-membered heteroaryl;
R² is hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl;
R¹⁰ is hydrogen, hydroxy, optionally substituted C₁-C₆ alkoxy, optionally substituted C₁-C₃ alkyl, optionally substituted C₁-C₆ heteroalkyl, or optionally substituted 3- to 7-membered heterocycloalkyl; and
R⁷ and R⁸ are each, independently, selected from fluoro or CH₃, or R⁷ and R⁸ combine with the atoms to which they are attached to make a 3-membered cycloalkyl.

In some embodiments, the disclosure features a compound, or a pharmaceutically acceptable salt, or a stereoisomer thereof, of structural Formula IV: 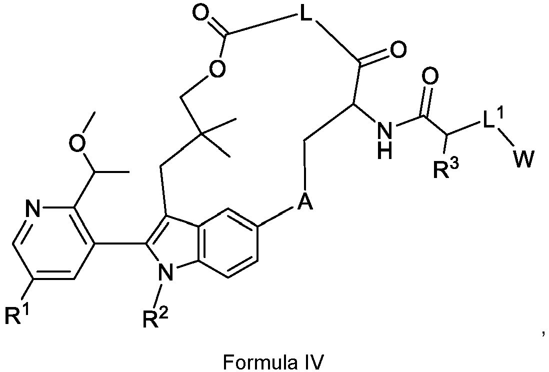
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
L¹ is a linker;
L has the structure of Formula VIIa or Vllb: 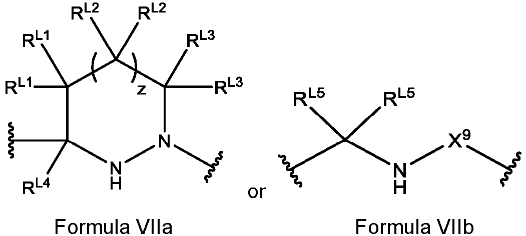
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 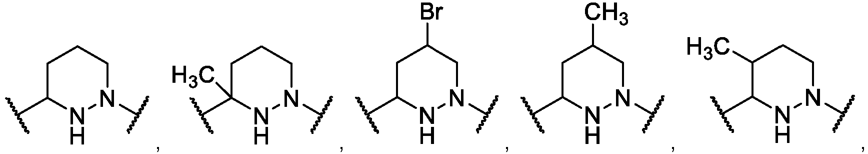 or 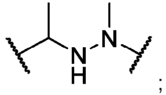
W is a cross-linking group comprising a vinyl ketone, vinyl sulfone, ynone, or an alkynyl sulfone;
R¹ is hydrogen, optionally substituted 3- to 10-membered heterocycloalkyl, or optionally substituted C₁-C₆ heteroalkyl;
R² is optionally substituted C₁-C₆ alkyl; and
R³ is optionally substituted C₁-C₆ alkyl or optionally substituted C₁-C₃ heteroalkyl.

In some embodiments, the disclosure features a compound, or a pharmaceutically acceptable salt, or a stereoisomer thereof, of structural Formula V: 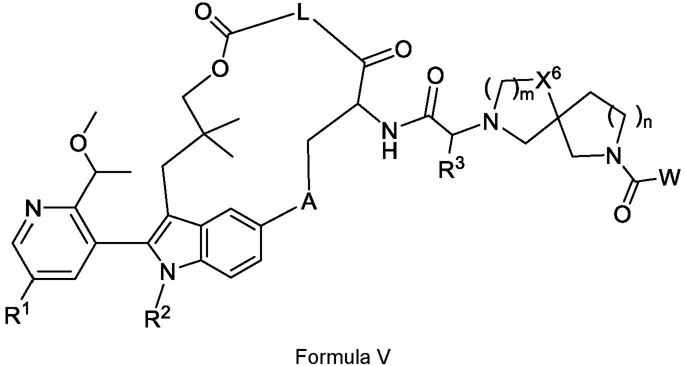
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
L has the structure of Formula VIIa or Vllb: 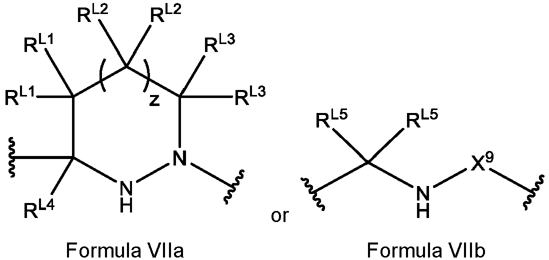
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 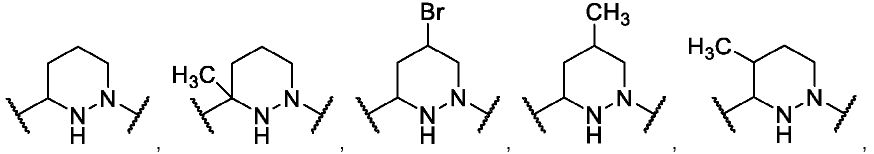 or 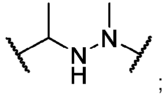
W is a cross-linking group comprising an aziridine, an epoxide, a carbodiimide, an oxazoline, a thiazoline, a chloroethyl urea, a chloroethyl thiourea, a chloroethyl carbamate, a chloroethyl thiocarbamate, a trifluoromethyl ketone, a boronic acid, a boronic ester, an N-ethoxycarbonyl-2-ethoxy-1,2-dihydroquinoline (EEDQ), an iso-EEDQ or other EEDQ derivative, an oxazolium, or a glycal;
X⁶ is CH₂ or O;
m is 1 or 2;
n is 0 or 1;
R¹ is hydrogen or optionally substituted 3- to 10-membered heterocycloalkyl; and
R² is optionally substituted C₁-C₆ alkyl.

In some embodiments, the disclosure features a compound, or a pharmaceutically acceptable salt, or a stereoisomer thereof, of structural Formula VI: 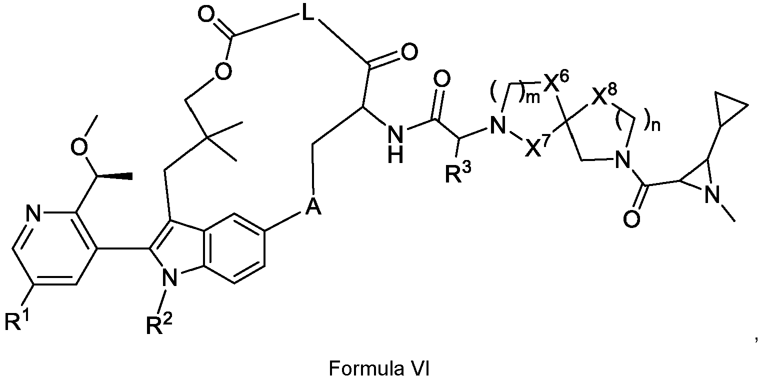
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
L has the structure of Formula VIIa or Vllb: 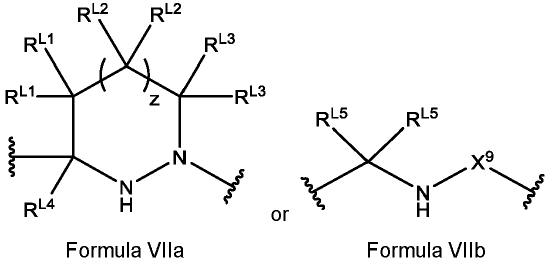
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
X⁶, X⁷, and X⁸ are each independently selected from CH₂, CHF, CF₂, C=O, or O;
m is 1 or 2;
n is 0 or 1;
R' is hydrogen, optionally substituted C₁-C₆ heteroalkyl, or optionally substituted 3- to 10-membered heterocycloalkyl;
R² is optionally substituted C₁-C₆ alkyl; and
R³ is optionally substituted C₁-C₆ alkyl, optionally substituted C₁-C₆ heteroalkyl, optionally substituted 3- to 6-membered cycloalkyl, or optionally substituted heterocycloalkyl,
and wherein each hydrogen is independently, optionally, isotopically enriched for deuterium.

In some embodiments, the disclosure provides a compound having Formula Villa: 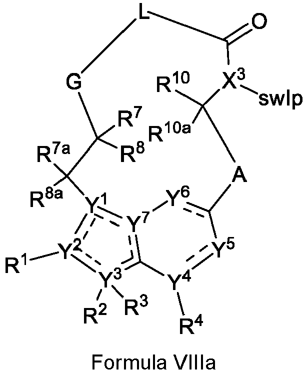 or a pharmaceutically acceptable salt or stereoisomer thereof, wherein:
the dotted lines represent zero, one, two, three, or four non-adjacent double bonds;
L has the structure of Formula VIIa or Vllb: 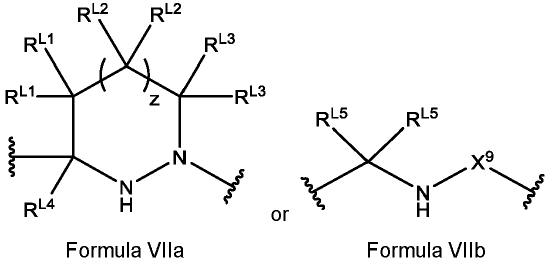
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 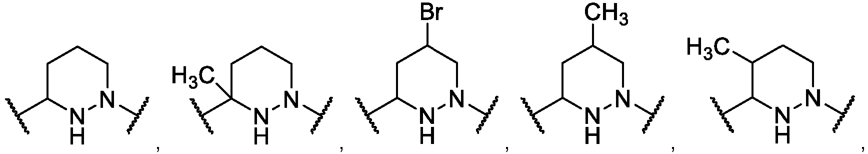 or 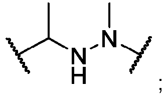
A is optionally substituted C₂-C₄ alkylene, optionally substituted C₁-C₄ heteroalkylene, or optionally substituted C₂-C₄ alkenylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3 to 8-membered heteroarylene;
swlp (Switch I/P-loop) is an organic moiety that non-covalently binds to both the Switch I binding pocket and residues 12 or 13 of the P-loop of a Ras protein;
X³ is N or CH;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl, or
R¹ and R² combine with the atoms to which they are attached to form an optionally substituted 3 to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3 to 6-membered cycloalkyl, optionally substituted 3 to 7-membered heterocycloalkyl, optionally substituted 6-membered aryl, optionally substituted 5 or 6-membered heteroaryl;
R³ is absent, or
R² and R³ combine with the atom to which they are attached to form an optionally substituted 3 to 8-membered cycloalkyl or optionally substituted 3 to 14-membered heterocycloalkyl;
R⁴ is absent, hydrogen, halogen, cyano, or methyl optionally substituted with 1 to 3 halogens;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3 to 6-membered cycloalkyl or optionally substituted 3 to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3 to 6-membered cycloalkyl, or optionally substituted 3 to 7-membered heterocycloalkyl;
R^{7a} and R^{8a} are, independently, hydrogen, halo, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
R^{7'} and R^{8'} combine with the carbon atom to which they are attached to form optionally substituted 3 to 6-membered cycloalkyl or optionally substituted 3 to 7-membered heterocycloalkyl;
R¹⁰ is hydrogen, halo, hydroxy, C₁-C₃ alkoxy, or C₁-C₃ alkyl; and
R^{10a} is hydrogen or halo.

Also provided are pharmaceutical compositions comprising a compound of Formula I, Formula IIa, Formula IIb, Formula IIc, Formula III, Formula IV, Formula V, Formula VI, Formula VIIIa, Formula VIIIb, Formula VIIIc, Formula VIIId, Formula VIIIe, Formula VIIIf, Formula VIIIg, or a pharmaceutically acceptable salt thereof, and a pharmaceutically acceptable excipient. Also provided are pharmaceutical compositions comprising a compound of Table 1, or a pharmaceutically acceptable salt thereof, and a pharmaceutically acceptable excipient.

Also provided is a method of treating cancer in a subject in need thereof, the method comprising administering to the subject a therapeutically effective amount of a compound of the present invention, or a pharmaceutically acceptable salt thereof.

In some embodiments, a method is provided of treating a Ras protein-related disorder in a subject in need thereof, the method comprising administering to the subject a therapeutically effective amount of a compound of the present invention, or a pharmaceutically acceptable salt thereof.

Further provided is a method of inhibiting a Ras protein in a cell, the method comprising contacting the cell with an effective amount of a compound of the present invention, or a pharmaceutically acceptable salt thereof.

It is specifically contemplated that any limitation discussed with respect to one embodiment of the invention may apply to any other embodiment of the invention. Furthermore, any compound or composition of the invention may be used in any method of the invention, and any method of the invention may be used to produce or to utilize any compound or composition of the invention.

### Definitions and Chemical Terms

In this application, unless otherwise clear from context, (i) the term "a" means "one or more"; (ii) the term "or" is used to mean "and/or" unless explicitly indicated to refer to alternatives only or the alternative are mutually exclusive, although the disclosure supports a definition that refers to only alternatives and "and/or"; (iii) the terms "comprising" and "including" are understood to encompass itemized components or steps whether presented by themselves or together with one or more additional components or steps; and (iv) where ranges are provided, endpoints are included.

As used herein, the term "about" is used to indicate that a value includes the standard deviation of error for the device or method being employed to determine the value. In certain embodiments, the term "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of a stated value, unless otherwise stated or otherwise evident from the context (e.g., where such number would exceed 100% of a possible value).

As used herein, the term "adjacent" in the context of describing adjacent atoms refers to bivalent atoms that are directly connected by a covalent bond.

A "compound of the present invention" and similar terms as used herein, whether explicitly noted or not, refers to Ras inhibitors described herein, including compounds of Formula I, Formula Ila, Formula IIb, Formula IIc, Formula III, Formula IV, Formula V, and Formula VI, and subformula thereof, for example, a compound of Table 1, as well as salts (e.g., pharmaceutically acceptable salts), solvates, hydrates, stereoisomers (including atropisomers), and tautomers thereof.

The term "wild-type" refers to an entity having a structure or activity as found in nature in a "normal" (as contrasted with mutant, diseased, altered, etc.) state or context. Those of ordinary skill in the art will appreciate that wild-type genes and polypeptides often exist in multiple different forms (e.g., alleles).

Those skilled in the art will appreciate that certain compounds described herein can exist in one or more different isomeric (e.g., stereoisomers, geometric isomers, atropisomers, tautomers) or isotopic (e.g., in which one or more atoms has been substituted with a different isotope of the atom, such as hydrogen substituted for deuterium) forms. Unless otherwise indicated or clear from context, a depicted structure can be understood to represent any such isomeric or isotopic form, individually or in combination.

Compounds described herein can be asymmetric (e.g., having one or more stereocenters). All stereoisomers, such as enantiomers and diastereomers, are intended unless otherwise indicated. Compounds of the present disclosure that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present disclosure. Cis and trans geometric isomers of the compounds of the present disclosure are described and may be isolated as a mixture of isomers or as separated isomeric forms.

In some embodiments, one or more compounds depicted herein may exist in different tautomeric forms. As will be clear from context, unless explicitly excluded, references to such compounds encompass all such tautomeric forms. In some embodiments, tautomeric forms result from the swapping of a single bond with an adjacent double bond and the concomitant migration of a proton. In certain embodiments, a tautomeric form may be a prototropic tautomer, which is an isomeric protonation states having the same empirical formula and total charge as a reference form. Examples of moieties with prototropic tautomeric forms are ketone - enol pairs, amide - imidic acid pairs, lactam - lactim pairs, amide - imidic acid pairs, enamine - imine pairs, and annular forms where a proton can occupy two or more positions of a heterocyclic system, such as, 1H- and 3H-imidazole, 1H-, 2H- and 4H-1,2,4-triazole, 1H- and 2H- isoindole, and 1H- and 2H-pyrazole. In some embodiments, tautomeric forms can be in equilibrium or sterically locked into one form by appropriate substitution. In certain embodiments, tautomeric forms result from acetal interconversion.

Unless otherwise stated, structures depicted herein are also meant to include compounds that differ only in the presence of one or more isotopically enriched atoms. Exemplary isotopes that can be incorporated into compounds of the present invention include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Isotopically labeled compounds (e.g., those labeled with ³H and ¹⁴C) can be useful in compound or substrate tissue distribution assays. Tritiated (i.e., ³H) and carbon-14 (i.e., ¹⁴C) isotopes can be useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements). In some embodiments, one or more hydrogen atoms are replaced by ²H or ³H, or one or more carbon atoms are replaced by ¹³C- or ¹⁴C-enriched carbon. Positron emitting isotopes such as ¹⁵O, ¹³N, ¹¹C, and ¹⁸F are useful for positron emission tomography (PET) studies to examine substrate receptor occupancy. Preparations of isotopically labelled compounds are known to those of skill in the art. For example, isotopically labeled compounds can generally be prepared by following procedures analogous to those disclosed for compounds of the present invention described herein, by substituting an isotopically labeled reagent for a non-isotopically labeled reagent.

Non-limiting examples of moieties that may contain one or more deuterium substitutions in compounds of the present invention, where any position "R" may be deuterium (D), include

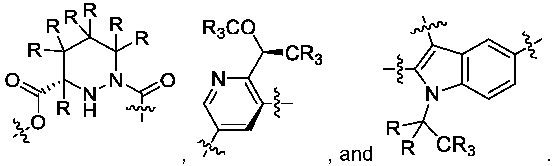

Additional examples include moieties such as 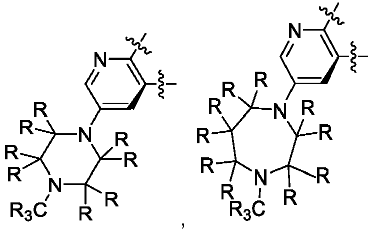 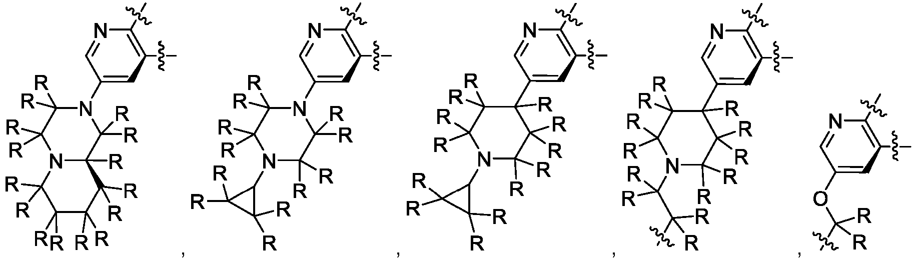 and deuteration of similar moieties, e.g., in compounds of Formula I, IIa, IIb, IIc, III, IV, V, and VI, and subformulae thereof). Moreover, deuteration of available positions in any A moiety of compounds of the Formulas described herein is also contemplated, such as 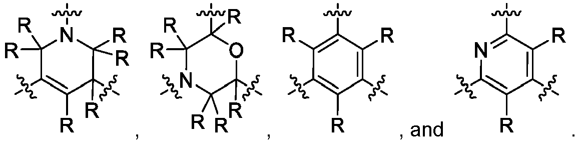 Further, deuterium substitution may 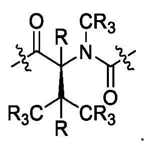 also take place in compounds of the present invention at the linker position, such as
Further, deuterium substitution may also take place in compounds of the present invention at the linker position, such as

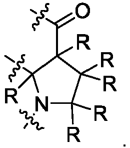

Further, deuterium substitution may also take place in compounds of the present invention at the linker position, such as

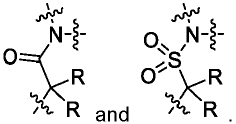

Additional deuteration substitution may also take place in compounds of the present invention as follows: 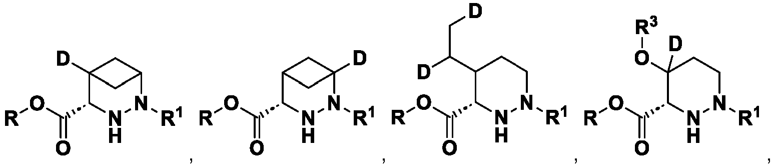 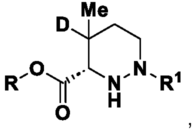 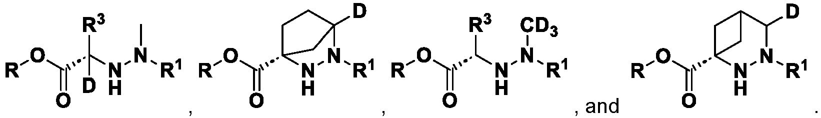 **Further,** R^{L1} - R^{L4} of Formula VIIa of any compound of the present invention may comprise one or more deuteriums:

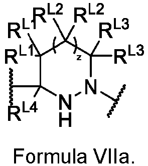

In a further embodiment, silylation substitution is also contemplated, such as in the linker as follows:

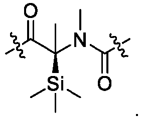

As is known in the art, many chemical entities can adopt a variety of different solid forms such as, for example, amorphous forms or crystalline forms (e.g., polymorphs, hydrates, solvate). In some embodiments, compounds of the present invention may be utilized in any such form, including in any solid form. In some embodiments, compounds described or depicted herein may be provided or utilized in hydrate or solvate form.

At various places in the present specification, substituents of compounds of the present disclosure are disclosed in groups or in ranges. It is specifically intended that the present disclosure include each and every individual subcombination of the members of such a groups and ranges. For example, the term "C₁-C₆ alkyl" is specifically intended to individually disclose methyl, ethyl, C₃ alkyl, C₄ alkyl, C₅ alkyl, and C₆ alkyl. Furthermore, where a compound includes a plurality of positions at which substituents are disclosed in groups or in ranges, unless otherwise indicated, the present disclosure is intended to cover individual compounds and groups of compounds (e.g., genera and subgenera) containing each and every individual subcombination of members at each position.

The term "optionally substituted X" (e.g., "optionally substituted alkyl") is intended to be equivalent to "X, wherein X is optionally substituted" (e.g., "alkyl, wherein said alkyl is optionally substituted"). It is not intended to mean that the feature "X" (e.g., alkyl) *per se* is optional. As described herein, certain compounds of interest may contain one or more "optionally substituted" moieties. In general, the term "substituted", whether preceded by the term "optionally" or not, means that one or more hydrogens of the designated moiety are replaced with a suitable substituent, e.g., any of the substituents or groups described herein. Unless otherwise indicated, an "optionally substituted" group may have a suitable substituent at each substitutable position of the group, and when more than one position in any given structure may be substituted with more than one substituent selected from a specified group, the substituent may be either the same or different at every position. For example, in the term "optionally substituted C₁-C₆ alkyl-C₂-C₉ heteroaryl," the alkyl portion, the heteroaryl portion, or both, may be optionally substituted. Combinations of substituents envisioned by the present disclosure are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group may be, independently, deuterium; halogen; -(CH₂)₀-₄R°; -(CH₂)₀-₄OR°; -O(CH₂)₀-₄R°; -O-(CH₂)₀-₄C(O)OR°; -(CH₂)₀-₄CH(OR°)₂; -(CH₂)₀-₄SR°; -(CH₂)₀-₄Ph, which may be substituted with R°; -(CH₂)₀-₄O(CH₂)₀-₁Ph which may be substituted with R°; -CH=CHPh, which may be substituted with R°; -(CH₂)₀-₄O(CH₂)₀-₁-pyridyl which may be substituted with R°; 4- to 8-membered saturated or unsaturated heterocycloalkyl (e.g., pyridyl); 3- to 8- membered saturated or unsaturated cycloalkyl (e.g., cyclopropyl, cyclobutyl, or cyclopentyl); -NO₂; -CN; -N₃; -(CH₂)₀-₄N(R°)₂; -(CH₂)₀-₄N(R°)C(O)R°; -N(R°)C(S)R°; -(CH₂)₀-₄N(R°)C(O)NR°₂; -N(R°)C(S)NR°₂; -(CH₂)₀-₄N(R°)C(O)OR°; -N(R°)N(R°)C(O)R°; -N(R°)N(R°)C(O)NR°₂; -N(R°)N(R°)C(O)OR°; -(CH₂)₀-₄C(O)R°; -C(S)R°; -(CH₂)₀-₄C(O)OR°; -(CH₂)₀-₄-C(O)-N(R°)₂; -(CH₂)₀-₄-C(O)-N(R°)-S(O)₂-R°; -C(NCN)NR°₂; -(CH₂)₀-₄C(O)SR°; -(CH₂)₀-₄C(O)OSiR°₃; -(CH₂)₀-₄OC(O)R°; -OC(O)(CH₂)₀-₄SR°; -SC(S)SR°; -(CH₂)₀-₄SC(O)R°; -(CH₂)₀-₄C(O)NR°₂; -C(S)NR°₂; -C(S)SR°; -(CH₂)₀-₄OC(O)NR°₂; -C(O)N(OR°)R°; -C(O)C(O)R°; -C(O)CH₂C(O)R°; -C(NOR°)R°; -(CH₂)₀-₄SSR°; -(CH₂)₀-₄S(O)₂R°; -(CH₂)₀-₄S(O)₂OR°; -(CH₂)₀-₄OS(O) ₂R°; -S(O)₂NR°₂; -(CH₂)₀-₄S(O)R°; -N(R°)S(O)₂NR°₂; -N(R°)S(O)₂R°; -N(OR°)R°; -C(NOR°)NR°₂; -C(N H)NR°₂; -P(O)₂R°; -P(O)R°₂; -P(O)(OR°)₂; -OP(O)R°₂; -OP(O)(OR°)₂; -OP(O)(OR°)R°; -SiR°₃; -(C₁-₄ straight or branched alkylene)O-N(R°)₂; or -(C₁-₄ straight or branched alkylene)C(O)O-N(R°)₂, wherein each R° may be substituted as defined below and is independently hydrogen, -C₁-₆ aliphatic, -CH₂Ph, -O(CH₂)₀-₁Ph, -CH₂-(5- to 6-membered heteroaryl ring), -CH₂-(5- to 10-membered heteroaryl ring), 3- to 15-membered saturated, partially unsaturated, or aryl ring having 0-4 heteroatoms independently selected from nitrogen, oxygen, sulfur, SO or SO₂, or a 3- to 6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3- to 12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), may be, independently, halogen, -(CH₂)₀-₂R^{•}, -(haloR^{•}), -(CH₂)₀-₂OH, -(CH₂)₀-₂OR^{•}, -(CH₂)₀-₂CH(OR^{•})₂; -O(haloR^{•}), -CN, -N₃, -(CH₂)₀-₂C(O)R^{•},-(CH₂)₀-₂C(O)OH, -(CH₂)₀-₂C(O)OR^{•}, -(CH₂)₀-₂SR^{•}, -(CH₂)₀-₂SH, -(CH₂)₀-₂NH₂, -(CH₂ )₀-₂NHR^{•}, -(CH₂)₀-₂NR^{•}₂, -NO₂, -SiR^{•}₃, -OSiR^{•}₃, -C(O)SR^{•}, -(C₁-₄ straight or branched alkylene)C(O)OR^{•}, or -SSR^{•} wherein each R^{•} is unsubstituted or where preceded by "halo" is substituted only with one or more halogens, and is independently selected from C₁-₄ aliphatic, -CH₂Ph, -O(CH₂)₀-₁Ph, a 3- to 10-membered saturated, partially unsaturated, or aryl ring having 0-4 heteroatoms independently selected from nitrogen, oxygen, or sulfur, or a 5- to 6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, =NNR^{*}₂, =NNHC(O)R^{*}, =NNHC(O)OR^{*}, =NNHS(O)₂R^{*}, =NR^{*}, =NOR^{*}, -O(C(R^{*}₂))₂-₃O-, or -S(C(R^{*}₂))₂-₃S-, wherein each independent occurrence of R^{*} is selected from hydrogen, C₁-₆ aliphatic which may be substituted as defined below, a 3- to 10-membered saturated, partially unsaturated, or aryl ring having 0-4 heteroatoms independently selected from nitrogen, oxygen, or sulfur, or an unsubstituted 5- to 6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR^{*}₂)₂-₃O-, wherein each independent occurrence of R^{*} is selected from hydrogen, C₁-₆ aliphatic which may be substituted as defined below, or an unsubstituted 5- to 6-membered saturated, partially unsaturated, or aryl ring having 0-4 heteroatoms independently selected from nitrogen, oxygen, or sulfur.

Suitable substituents on the aliphatic group of R^{*} include halogen, -R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁-₄ aliphatic, -CH₂Ph, -O(CH₂)₀-₁Ph, a 3- to 10-membered saturated, partially unsaturated, or aryl ring having 0-4 heteroatoms independently selected from nitrogen, oxygen, or sulfur, or a 5- to 6-membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, -C(O)CH₂C(O)R^{†}, -S(O)₂R^{†}, -S(O)₂NR^{†}₂, -C(S)N R^{†}₂, -C(NH)NR^{†}₂, or -N(R^{†})S(O)₂R^{†}; wherein each R^{†} is independently hydrogen, C₁-₆ aliphatic which may be substituted as defined below, unsubstituted -OPh, a 3- to 10-membered saturated, partially unsaturated, or aryl ring having 0-4 heteroatoms independently selected from nitrogen, oxygen, or sulfur, or an unsubstituted 3- to 6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3- to 12-membered saturated, partially unsaturated, or aryl mono- or bicyclic ring having 0-4 heteroatoms independently selected from nitrogen, oxygen, or sulfur.

Suitable substituents on an aliphatic group of R^{†} are independently halogen, -R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁-₄ aliphatic, -CH₂Ph, -O(CH₂)₀-₁Ph, a 3- to 10-membered saturated, partially unsaturated, or aryl ring having 0-4 heteroatoms independently selected from nitrogen, oxygen, or sulfur, or a 5- to 6-membered saturated, partially unsaturated, or aryl ring having 0-4 heteroatoms independently selected from nitrogen, oxygen, or sulfur. Suitable divalent substituents on a saturated carbon atom of R^{†} include =O and =S.

The term "acetyl," as used herein, refers to the group -C(O)CH₃.

The term "alkoxy," as used herein, refers to a -O-C₁-C₂₀ alkyl group, wherein the alkoxy group is attached to the remainder of the compound through an oxygen atom.

The term "alkyl," as used herein, refers to a saturated, straight or branched monovalent hydrocarbon group containing from 1 to 20 (e.g., from 1 to 10 or from 1 to 6) carbons. In some embodiments, an alkyl group is unbranched (i.e., is linear); in some embodiments, an alkyl group is branched. Alkyl groups are exemplified by, but not limited to, methyl, ethyl, n- and iso-propyl, *n-, sec-, iso-* and tert-butyl, and neopentyl.

The term "alkylene," as used herein, represents a saturated divalent hydrocarbon group derived from a straight or branched chain saturated hydrocarbon by the removal of two hydrogen atoms, and is exemplified by methylene, ethylene, isopropylene, and the like. The term "Cₓ-C_{y} alkylene" represents alkylene groups having between x and y carbons. Exemplary values for x are 1, 2, 3, 4, 5, and 6, and exemplary values for y are 2, 3, 4, 5, 6, 7, 8, 9, 10, 12, 14, 16, 18, or 20 (e.g., C₁-C₆, C₁-C₁₀, C₂-C₂₀, C₂-C₆, C₂-C₁₀, or C₂-C₂₀ alkylene). In some embodiments, the alkylene can be further substituted with 1, 2, 3, or 4 substituent groups as defined herein.

The term "alkenyl," as used herein, represents monovalent straight or branched chain groups of, unless otherwise specified, from 2 to 20 carbons (e.g., from 2 to 6 or from 2 to 10 carbons) containing one or more carbon-carbon double bonds and is exemplified by ethenyl, 1-propenyl, 2-propenyl, 2-methyl-1-propenyl, 1-butenyl, and 2-butenyl. Alkenyls include both cis and trans isomers. The term "alkenylene," as used herein, represents a divalent straight or branched chain groups of, unless otherwise specified, from 2 to 20 carbons (e.g., from 2 to 6 or from 2 to 10 carbons) containing one or more carbon-carbon double bonds.

The term "alkynyl," as used herein, represents monovalent straight or branched chain groups from 2 to 20 carbon atoms (e.g., from 2 to 4, from 2 to 6, or from 2 to 10 carbons) containing a carbon-carbon triple bond and is exemplified by ethynyl, and 1-propynyl.

The term "alkynyl sulfone," as used herein, represents a group comprising the structure 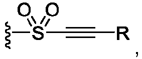 wherein R is any chemically feasible substituent described herein.

The term "amino," as used herein, represents -N(R^{†})₂, e.g., -NH₂ and -N(CH₃)₂.

The term "aminoalkyl," as used herein, represents an alkyl moiety substituted on one or more carbon atoms with one or more amino moieties.

The term "amino acid," as described herein, refers to a molecule having a side chain, an amino group, and an acid group (e.g., -CO₂H or -SO₃H), wherein the amino acid is attached to the parent molecular group by the side chain, amino group, or acid group (e.g., the side chain). As used herein, the term "amino acid" in its broadest sense, refers to any compound or substance that can be incorporated into a polypeptide chain, e.g., through formation of one or more peptide bonds. In some embodiments, an amino acid has the general structure H₂N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. Exemplary amino acids include alanine, arginine, asparagine, aspartic acid, cysteine, glutamic acid, glutamine, glycine, histidine, optionally substituted hydroxylnorvaline, isoleucine, leucine, lysine, methionine, norvaline, ornithine, phenylalanine, proline, pyrrolysine, selenocysteine, serine, taurine, threonine, tryptophan, tyrosine, and valine.

The term "aryl," as used herein, represents a monovalent monocyclic, bicyclic, or multicyclic ring system formed by carbon atoms, wherein the ring attached to the pendant group is aromatic. Examples of aryl groups are phenyl, naphthyl, phenanthrenyl, and anthracenyl. An aryl ring can be attached to its pendant group at any heteroatom or carbon ring atom that results in a stable structure and any of the ring atoms can be optionally substituted unless otherwise specified.

The term "C₀," as used herein, represents a bond. For example, part of the term -N(C(O)-(C₀-C₅ alkylene-H)- includes -N(C(O)-(C₀ alkylene-H)-, which is also represented by -N(C(O)-H)-.

The terms "carbocyclic" and "carbocyclyl," as used herein, refer to a monovalent, optionally substituted C₃-C₁₂ monocyclic, bicyclic, or tricyclic ring structure, which may be bridged, fused or spirocyclic, in which all the rings are formed by carbon atoms and at least one ring is non-aromatic. Carbocyclic structures include cycloalkyl, cycloalkenyl, and cycloalkynyl groups. Examples of carbocyclyl groups are cyclohexyl, cyclohexenyl, cyclooctynyl, 1,2-dihydronaphthyl, 1,2,3,4-tetrahydronaphthyl, fluorenyl, indenyl, indanyl, decalinyl, and the like. A carbocyclic ring can be attached to its pendant group at any ring atom that results in a stable structure and any of the ring atoms can be optionally substituted unless otherwise specified.

The term "carbonyl," as used herein, represents a C(O) group, which can also be represented as C=O.

The term "carboxyl," as used herein, means -CO₂H, (C=O)(OH), COOH, or C(O)OH or the unprotonated counterparts.

The term "cyano," as used herein, represents a -CN group.

The term "cycloalkyl," as used herein, represents a monovalent saturated cyclic hydrocarbon group, which may be bridged, fused or spirocyclic having from three to eight ring carbons, unless otherwise specified, and is exemplified by cyclopropyl, cyclobutyl, cyclopentyl, cyclohexyl, cycloheptyl, and cycloheptyl.

The term "cycloalkenyl," as used herein, represents a monovalent, non-aromatic, saturated cyclic hydrocarbon group, which may be bridged, fused or spirocyclic having from three to eight ring carbons, unless otherwise specified, and containing one or more carbon-carbon double bonds.

The term "diastereomer," as used herein, means stereoisomers that are not mirror images of one another and are non-superimposable on one another.

The term "enantiomer," as used herein, means each individual optically active form of a compound of the invention, having an optical purity or enantiomeric excess (as determined by methods standard in the art) of at least 80% (i.e., at least 90% of one enantiomer and at most 10% of the other enantiomer), preferably at least 90% and more preferably at least 98%.

The term "guanidinyl," refers to a group having the structure: 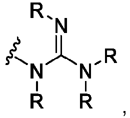 wherein each R is, **independently,** any chemically feasible substituent described herein.

The term "guanidinoalkyl alkyl," as used herein, represents an alkyl moiety substituted on one or more carbon atoms with one or more guanidinyl moieties.

The term "haloacetyl," as used herein, refers to an acetyl group wherein at least one of the hydrogens has been replaced by a halogen.

The term "haloalkyl," as used herein, represents an alkyl moiety substituted on one or more carbon atoms with one or more of the same of different halogen moieties.

The term "halogen," as used herein, represents a halogen selected from bromine, chlorine, iodine, or fluorine.

The term "heteroalkyl," as used herein, refers to an "alkyl" group, as defined herein, in which at least one carbon atom has been replaced with a heteroatom (e.g., an O, N, or S atom). The heteroatom may appear in the middle or at the end of the radical.

The term "heteroaryl," as used herein, represents a monovalent, monocyclic, or polycyclic ring structure that contains at least one fully aromatic ring: i.e., they contain 4*n*+2 pi electrons within the monocyclic or polycyclic ring system and contains at least one ring heteroatom selected from N, O, or S in that aromatic ring. Exemplary unsubstituted heteroaryl groups are of 1 to 12 (e.g., 1 to 11, 1 to 10, 1 to 9, 2 to 12, 2 to 11, 2 to 10, or 2 to 9) carbons. The term "heteroaryl" includes bicyclic, tricyclic, and tetracyclic groups in which any of the above heteroaromatic rings is fused to one or more, aryl or carbocyclic rings, e.g., a phenyl ring, or a cyclohexane ring. Examples of heteroaryl groups include, but are not limited to, pyridyl, pyrazolyl, benzooxazolyl, benzoimidazolyl, benzothiazolyl, imidazolyl, thiazolyl, quinolinyl, tetrahydroquinolinyl, and 4-azaindolyl. A heteroaryl ring can be attached to its pendant group at any ring atom that results in a stable structure and any of the ring atoms can be optionally substituted unless otherwise specified. In some embodiments, the heteroaryl is substituted with 1, 2, 3, or 4 substituents groups.

The term "heterocycloalkyl," as used herein, represents a monovalent monocyclic, bicyclic, or polycyclic ring system, which may be bridged, fused or spirocyclic, wherein at least one ring is non-aromatic and wherein the non-aromatic ring contains one, two, three, or four heteroatoms independently selected from the group consisting of nitrogen, oxygen, and sulfur. The 5-membered ring has zero to two double bonds, and the 6- and 7-membered rings have zero to three double bonds. Exemplary unsubstituted heterocycloalkyl groups are of 1 to 12 (e.g., 1 to 11, 1 to 10, 1 to 9, 2 to 12, 2 to 11, 2 to 10, or 2 to 9) carbons. The term "heterocycloalkyl" also represents a heterocyclic compound having a bridged multicyclic structure in which one or more carbons or heteroatoms bridges two non-adjacent members of a monocyclic ring, e.g., a quinuclidinyl group. The term "heterocycloalkyl" includes bicyclic, tricyclic, and tetracyclic groups in which any of the above heterocyclic rings is fused to one or more aromatic, carbocyclic, heteroaromatic, or heterocyclic rings, e.g., an aryl ring, a cyclohexane ring, a cyclohexene ring, a cyclopentane ring, a cyclopentene ring, a pyridine ring, or a pyrrolidine ring. Examples of heterocycloalkyl groups are pyrrolidinyl, piperidinyl, 1,2,3,4-tetrahydroquinolinyl, decahydroquinolinyl, dihydropyrrolopyridine, and decahydronapthyridinyl. A heterocycloalkyl ring can be attached to its pendant group at any ring atom that results in a stable structure and any of the ring atoms can be optionally substituted unless otherwise specified.

The term "hydroxy," as used herein, represents a -OH group.

The term "hydroxyalkyl," as used herein, represents an alkyl moiety substituted on one or more carbon atoms with one or more -OH moieties.

The term "isomer," as used herein, means any tautomer, stereoisomer, atropisomer, enantiomer, or diastereomer of any compound of the invention. It is recognized that the compounds of the invention can have one or more chiral centers or double bonds and, therefore, exist as stereoisomers, such as double-bond isomers (i.e., geometric E/Z isomers) or diastereomers (e.g., enantiomers (i.e., (+) or (-)) or cis/trans isomers). According to the invention, the chemical structures depicted herein, and therefore the compounds of the invention, encompass all the corresponding stereoisomers, that is, both the stereomerically pure form (e.g., geometrically pure, enantiomerically pure, or diastereomerically pure) and enantiomeric and stereoisomeric mixtures, e.g., racemates. Enantiomeric and stereoisomeric mixtures of compounds of the invention can typically be resolved into their component enantiomers or stereoisomers by well-known methods, such as chiral-phase gas chromatography, chiral-phase high performance liquid chromatography, crystallizing the compound as a chiral salt complex, or crystallizing the compound in a chiral solvent. Enantiomers and stereoisomers can also be obtained from stereomerically or enantiomerically pure intermediates, reagents, and catalysts by well-known asymmetric synthetic methods.

As used herein, the term "linker" refers to a divalent organic moiety connecting a first moiety (e.g., one portion of a macrocycle) to a second moiety (e.g., a second portion of the same macrocycle). In some embodiments, the linker results in a compound capable of achieving an IC50 of 3 uM or less in the Ras-RAF disruption assay protocol provided here:
The purpose of this biochemical assay is to measure the ability of test compounds to facilitate ternary complex formation between a nucleotide-loaded Ras isoform and cyclophilin A; the resulting ternary complex disrupts binding to a BRAF^{RBD} construct, inhibiting Ras signaling through a RAF effector.
In assay buffer containing 25 mM HEPES pH 7.3, 0.002% Tween20, 0.1% BSA, 100 mM NaCl and 5 mM MgCl₂, tagless cyclophilin A, His6-K-Ras-GMPPNP (or other Ras variant), and GST-BRAF^{RBD} are combined in a 384-well assay plate at final concentrations of 25 µM, 12.5 nM and 50 nM, respectively. Compound is present in plate wells as a 10-point 3-fold dilution series starting at a final concentration of 30 µM. After incubation at 25°C for 3 hours, a mixture of Anti-His Eu-W1024 and anti-GST allophycocyanin is then added to assay sample wells at final concentrations of 10 nM and 50 nM, respectively, and the reaction incubated for an additional 1.5 hours. TR-FRET signal is read on a microplate reader (Ex 320 nm, Em 665/615 nm). Compounds that facilitate disruption of a Ras:RAF complex are identified as those eliciting a decrease in the TR-FRET ratio relative to DMSO control wells.
This assay may be used to assess selectivity as well. In some embodiments, a compound of the present invention is selective for one or more particular Ras mutants over other Ras mutants or wild-type compared to what is known in the art.

In some embodiments, the linker comprises 20 or fewer linear atoms. In some embodiments, the linker comprises 15 or fewer linear atoms. In some embodiments, the linker comprises 10 or fewer linear atoms. In some embodiments, the linker has a molecular weight of under 500 g/mol. In some embodiments, the linker has a molecular weight of under 400 g/mol. In some embodiments, the linker has a molecular weight of under 300 g/mol. In some embodiments, the linker has a molecular weight of under 200 g/mol. In some embodiments, the linker has a molecular weight of under 100 g/mol. In some embodiments, the linker has a molecular weight of under 50 g/mol.

As used herein, a "monovalent organic moiety" is less than 500 kDa. In some embodiments, a "monovalent organic moiety" is less than 400 kDa. In some embodiments, a "monovalent organic moiety" is less than 300 kDa. In some embodiments, a "monovalent organic moiety" is less than 200 kDa. In some embodiments, a "monovalent organic moiety" is less than 100 kDa. In some embodiments, a "monovalent organic moiety" is less than 50 kDa. In some embodiments, a "monovalent organic moiety" is less than 25 kDa. In some embodiments, a "monovalent organic moiety" is less than 20 kDa. In some embodiments, a "monovalent organic moiety" is less than 15 kDa. In some embodiments, a "monovalent organic moiety" is less than 10 kDa. In some embodiments, a "monovalent organic moiety" is less than 1 kDa. In some embodiments, a "monovalent organic moiety" is less than 500 g/mol. In some embodiments, a "monovalent organic moiety" ranges between 500 g/mol and 500 kDa.

The term "stereoisomer," as used herein, refers to all possible different isomeric as well as conformational forms which a compound may possess (e.g., a compound of any formula described herein), in particular all possible stereochemically and conformationally isomeric forms, all diastereomers, enantiomers or conformers of the basic molecular structure, including atropisomers. Some compounds of the present invention may exist in different tautomeric forms, all of the latter being included within the scope of the present invention.

The term "sulfonyl," as used herein, represents an -S(O)₂- group.

The term "thiocarbonyl," as used herein, refers to a -C(S)- group.

The term "vinyl ketone," as used herein, refers to a group comprising a carbonyl group directly connected to a carbon-carbon double bond.

The term "vinyl sulfone," as used herein, refers to a group comprising a sulfonyl group directed connected to a carbon-carbon double bond.

The term "ynone," as used herein, refers to a group comprising the structure 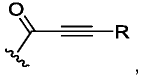 wherein R is any chemically feasible substituent described herein.

Those of ordinary skill in the art, reading the present disclosure, will appreciate that certain compounds described herein may be provided or utilized in any of a variety of forms such as, for example, salt forms, protected forms, pro-drug forms, ester forms, isomeric forms (e.g., optical or structural isomers), isotopic forms, etc. In some embodiments, reference to a particular compound may relate to a specific form of that compound. In some embodiments, reference to a particular compound may relate to that compound in any form. In some embodiments, for example, a preparation of a single stereoisomer of a compound may be considered to be a different form of the compound than a racemic mixture of the compound; a particular salt of a compound may be considered to be a different form from another salt form of the compound; a preparation containing one conformational isomer ((Z) or (E)) of a double bond may be considered to be a different form from one containing the other conformational isomer ((E) or (Z)) of the double bond; a preparation in which one or more atoms is a different isotope than is present in a reference preparation may be considered to be a different form.

### Detailed Description

### Compounds

Provided herein are Ras inhibitors. The approach described herein entails formation of a high affinity three-component complex, or conjugate, between a synthetic ligand and two intracellular proteins which do not interact under normal physiological conditions: the target protein of interest (e.g., Ras), and a widely expressed cytosolic chaperone (presenter protein) in the cell (e.g., cyclophilin A). More specifically, in some embodiments, the inhibitors of Ras described herein induce a new binding pocket in Ras by driving formation of a high affinity tri-complex, or conjugate, between the Ras protein and the widely expressed cytosolic chaperone, cyclophilin A (CYPA). Without being bound by theory, the inventors believe that one way the inhibitory effect on Ras is effected by compounds of the invention and the complexes, or conjugates, they form is by steric occlusion of the interaction site between Ras and downstream effector molecules, such as RAF, which are required for propagating the oncogenic signal.

Without being bound by theory, the inventors postulate that covalent, non-covalent or combinations of covalent and non-covalent interactions of a compound of the present invention with Ras and the chaperone protein (e.g., cyclophilin A) may contribute to the inhibition of Ras activity. In some embodiments, a compound of the present invention forms a covalent adduct with a Ras protein (e.g., the cysteine at position 12 or position 13 of a mutant Ras protein, the aspartic acid at position 12 or position 13 of a mutant Ras protein, or the histidine at position 61 of a mutant Ras protein). Covalent adducts may also be formed with other side chains of Ras. In addition, or alternatively, non-covalent interactions may be at play: for example, van der Waals, hydrophobic, hydrophilic and hydrogen bond interactions, and combinations thereof, may contribute to the ability of the compounds of the present invention to form complexes and act as Ras inhibitors.

Accordingly, a variety of Ras proteins may be inhibited by a compound of the present invention (e.g., K-Ras, N-Ras, H-Ras, and mutants thereof at positions 12, 13 and 61, such as G12C, G12D, G12V, G12S, G12R, G13C, G13D, Q61H, Q61K, Q61R and Q61L, and others described herein, or a combination thereof).

Methods of determining covalent adduct formation are known in the art. One method of determining covalent adduct formation is to perform a "cross-linking" assay, such as under these conditions.

*Note - the following protocol describes a procedure for monitoring cross-linking of K-Ras G12C (GMP-PNP) to a compound of the invention. This protocol may also be executed substituting other Ras proteins or nucleotides.*

The purpose of this biochemical assay is to measure the ability of test compounds to covalently label nucleotide-loaded K-Ras isoforms. In assay buffer containing 12.5 mM HEPES pH 7.4, 75 mM NaCl, 1 mM MgCl₂, 1 mM BME (if studying a cysteine Ras mutant, such as K-Ras G12C or G13C), 5 µM cyclophilin A and 2 µM test compound, a 5 µM stock of GMP-PNP-loaded K-Ras (1-169) G12C is diluted 10-fold to yield a final concentration of 0.5 µM; with final sample volume being 100 µL.

The sample is incubated at 25°C for a time period of up to 24 hours prior to quenching by the addition of 10 µL of 5% Formic Acid. Quenched samples are centrifuged at 15000 rpm for 15 minutes in a benchtop centrifuge before injecting a 10 µL aliquot onto a reverse phase C4 column and eluting into the mass spectrometer with an increasing acetonitrile gradient in the mobile phase. Analysis of raw data may be carried out using Waters MassLynx MS software, with % bound calculated from the deconvoluted protein peaks for labeled and unlabeled K-Ras.

Accordingly, provided herein is a compound, or pharmaceutically acceptable salt thereof, having the structure of Formula I: 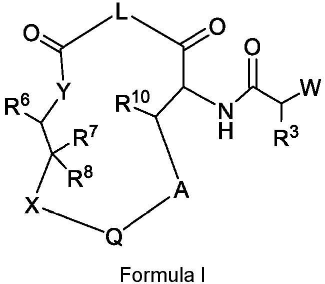
Q is an optionally substituted 7- to 12-membered bicyclic arylene, an optionally substituted 7-to 12-membered bicyclic heteroarylene, or an optionally substituted 7- to 12-membered bicyclic heterocyclylene, wherein a first ring in Q is bonded to X, and a second ring in Q is bonded to A;
X is a bond; a straight chain C₁-C₃ alkylene optionally substituted with 1 to 3 substituents independently selected from fluoro, -CN, -C₁-C₃ alkyl, and -O-C₁-C₃ alkyl; -O-; -S(O)₀₋₂-; *-CH₂-O-; *-CH₂-S(O)₀₋₂-; *-O-CH₂-; or *-CH₂-S(O)₀₋₂-, wherein "*" represents a portion of X bound to -C(R⁷)(R⁸)-;
Y is -O-, -NH- or -N(C₁-C₃ alkyl)-;
R³ is optionally substituted C₁-C₆ alkyl, optionally substituted C₃-C₆ cycloalkyl, optionally substituted C₆-C₁₀ aryl, or optionally substituted 3- to 11-membered heterocyclyl;
W is: 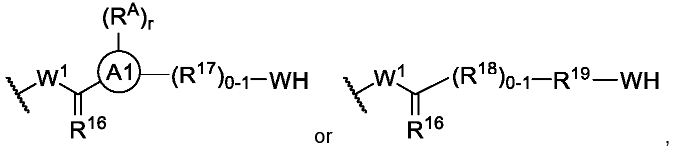
wherein:
   ring A1 is a 4- to 8-membered cycloalkyl or a 4- to 8-membered heterocyclyl;
   W¹ is -N(R²⁰)-, -O-, or -C(R^{20a})(R^{20b})-;
      each R^{A} is each independently halo, cyano, hydroxyl, optionally substituted amino, optionally substituted C₁-C₆ heteroalkyl, or optionally substituted C₁-C₃ alkyl;
   r is 0, 1, 2, 3, or 4;
      R¹⁷, if present, is optionally substituted C₁-C₆ heteroalkylene or optionally substituted C₁-C₆ alkylene;
   R¹⁸, if present, is optionally substituted C₁-C₄ alkylene;
   R¹⁹ is optionally substituted C₁-C₆ heteroalkylene, optionally substituted C₁-C₆ alkylene, -NH-, or
      -N(optionally substituted C₁-C₆ alkyl) or a saturated, nitrogen-containing 3- to 8-membered heterocyclyl;
   R²⁰ is hydrogen or -C₁-C₃ alkyl;
   R²⁰ is taken together with one R^{A}, the atoms to which they are respectively attached and any intervening atoms, to form an optionally substituted, 5- to 8-membered heterocyclyl that is fused or spiro-fused to ring A, or
   R²⁰ is taken together with any methylene unit in R¹⁸, or any methylene unit in R¹⁹, the atoms to which they are respectively attached and any intervening atoms, to form an optionally substituted, 5- to 8-membered heterocyclyl;
   each of R^{20a} and R^{20b} is, independently, hydrogen, or -C₁-C₃ alkyl, or R^{20a} and R^{20b} are taken together with the carbon atom to which they are bound to form a 3- to 6-membered cycloalkyl ring;
   R¹⁶ is O, S, N-CN, or N-O-C₁-C₃ alkyl;
   WH is 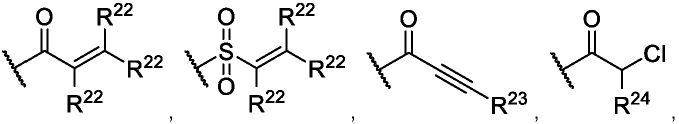 or 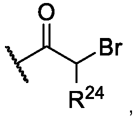
   each R²² is, independently, hydrogen, cyano, optionally substituted C₁-C₃ alkyl, or an optionally substituted 4- to 7-membered saturated heterocyclyl; or R²² is taken together with either of R¹⁷ or R¹⁹, the atoms to which they are attached and any intervening atoms to form an optionally substituted 5- to 8-membered ring system;
   R²³ is hydrogen, optionally substituted C₁-C₃ alkyl, or an optionally substituted 4- to 7-membered saturated heterocyclyl;
   R²⁴ is hydrogen, optionally substituted C₁-C₃ alkyl, or an optionally substituted 4- to 7-membered saturated heterocyclyl; or R²⁴ is taken together with either of R¹⁷ or R¹⁹, the atoms to which they are attached and any intervening atoms, to form an optionally substituted 5- to 8-membered ring system;
   A is optionally substituted C₂-C₄ alkylene, optionally substituted C₁-C₄ heteroalkylene, or optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   L has the structure of Formula VIIa or Vllb: 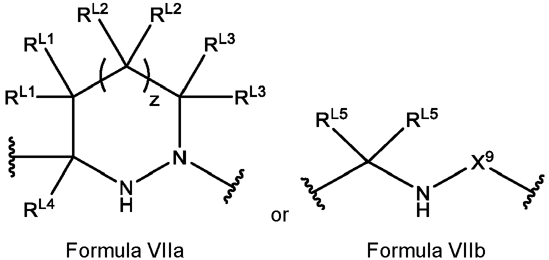
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 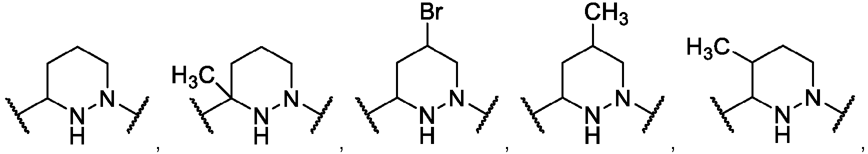 or 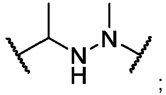
   R³ is optionally substituted C₁-C₆ alkyl, optionally substituted C₃-C₆ cycloalkyl, optionally substituted C₆ aryl, or optionally substituted 3- to 7-membered heterocyclyl;
   R¹⁰ is hydrogen, halogen, optionally substituted C₁-C₃ alkyl, or C₁-C₃ optionally substituted heteroalkyl;
   R⁷ is hydrogen, halogen, or optionally substituted C₁-C₃ alkyl;
   R⁸ is hydrogen, halogen, -OH, -CN, optionally substituted C₁-C₃ heteroalkyl, optionally substituted C₁-C₃ alkyl, optionally substituted C₂-C₆ alkenyl, optionally substituted C₂-C₆ alkynyl, optionally substituted C₆-C₁₀ aryl, optionally substituted 4- to 8-membered heteroaryl, optionally substituted C₃-C₆ cycloalkyl, or optionally substituted 3- to 7-membered heterocyclyl; or
   R⁷ and R⁸ are taken together to form =CH₂, an optionally substituted C₃-C₆ cycloalkyl, or a 3- to 7-membered saturated heterocyclyl; or
   R⁸ is taken together with a ring atom in Q, the carbon atom to which R⁷ is bound and X to form a 4- to 9-membered saturated or unsaturated heterocyclyl that is fused to Q;
   R⁶ is hydrogen or -CH₃;
   each R⁵ is, independently, halogen, optionally substituted C₁-C₃ alkyl, or optionally substituted C₁-C₃ haloalkyl; and
   p is 0, 1, 2, or 3.

In some embodiments of Formula I, the compound has the structure of formula (Ia): 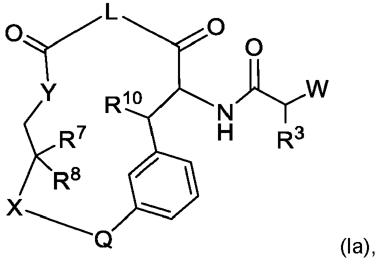 or a pharmaceutically acceptable salt, an enantiomer, a stereoisomer, or a tautomer thereof, wherein:
X is a bond, -O-, -CH₂-, -CH(CH₃)-, *-CH₂-O-, or -CH₂-CH₂-, where "*" represents a portion of X bound to C(R⁴)(R⁵);
Y is -O- or -NH-;
L has the structure of Formula VIIa or Vllb: 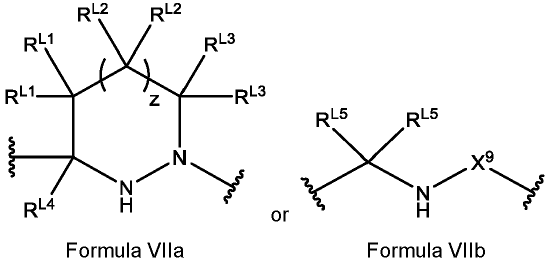
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of: 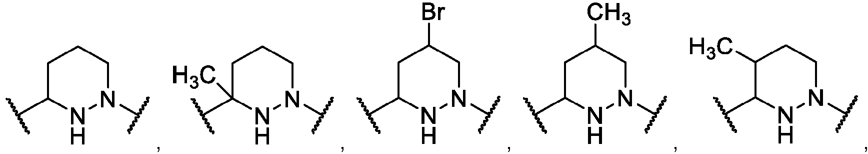 or 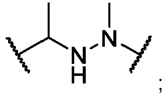
R³ is -C₁-C₄ alkyl, -(CH₂)₀-₁-(C₃-C₆ cycloalkyl), or -C₄-C₆ cycloalkyl;
R⁷ is hydrogen, halo, or C₁-C₃ alkyl;
R⁸ is hydrogen, halo, -OH, C₁- C₃ alkyl, C₁-C₃ hydroxyalkyl, C₁-C₃ alkylene-O-C₁-C₃ alkyl, C₁-C₃ haloalkyl, -(CH₂)₀-₁-C₃-C₆ cycloalkyl, C₁-C₃ cyanoalkyl, or -(CH₂)₀-₁-aryl (benzyl), or
R⁷ and R⁸ are taken together to form =CH₂, or a C₃-C₆ cycloalkyl, or
R⁸ is taken together with a ring atom of Q, the carbon atom to which it is bound and X to form a 5- to 7-membered saturated heterocyclyl;
W is: 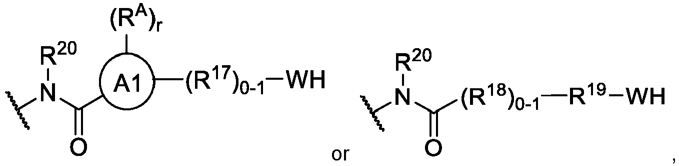
wherein:
   ring A1 is a 4- to 8-membered cycloalkyl or a 4- to 8-membered heterocyclyl;
   W¹ is -N(R²⁰)-, -O-, or -C(R^{20a})(R^{20b})-;
      each R^{A} is each independently halo, cyano, hydroxyl, optionally substituted amino, optionally substituted C₁-C₆ heteroalkyl, or optionally substituted C₁-C₃ alkyl;
   r is 0, 1, 2, 3, or 4;
      R¹⁷, if present, is optionally substituted C₁-C₆ heteroalkylene or optionally substituted C₁-C₆ alkylene;
   R¹⁸, if present, is optionally substituted C₁-C₄ alkylene;
   R¹⁹ is optionally substituted C₁-C₆ heteroalkylene, optionally substituted C₁-C₆ alkylene, -NH-, or
      -N(optionally substituted C₁-C₆ alkyl) or a saturated, nitrogen-containing 3- to 8-membered heterocyclyl;
   R²⁰ is hydrogen or -C₁-C₃ alkyl;
   R²⁰ is taken together with one R^{A}, the atoms to which they are respectively attached and any intervening atoms, to form an optionally substituted, 5- to 8-membered heterocyclyl that is fused or spiro-fused to ring A, or
   R²⁰ is taken together with any methylene unit in R¹⁸, or any methylene unit in R¹⁹, the atoms to which they are respectively attached and any intervening atoms, to form an optionally substituted, 5- to 8-membered heterocyclyl;
   each of R^{20a} and R^{20b} is, independently, hydrogen, or -C₁-C₃ alkyl, or R^{20a} and R^{20b} are taken together with the carbon atom to which they are bound to form a 3- to 6-membered cycloalkyl ring;
   R¹⁶ is O, S, N-CN, or N-O-C₁-C₃ alkyl; 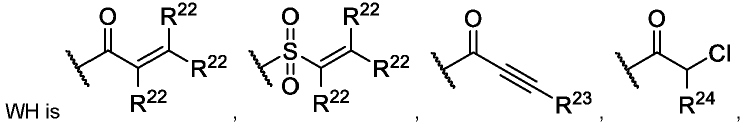 or 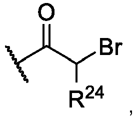
   each R²² is, independently, hydrogen, cyano, optionally substituted C₁-C₃ alkyl, or an optionally substituted 4- to 7-membered saturated heterocyclyl; or R²² is taken together with either of R¹⁷ or R¹⁹, the atoms to which they are attached and any intervening atoms to form an optionally substituted 5- to 8-membered ring system;
   R²³ is hydrogen, optionally substituted C₁-C₃ alkyl, or an optionally substituted 4- to 7-membered saturated heterocyclyl;
   R²⁴ is hydrogen, optionally substituted C₁-C₃ alkyl, or an optionally substituted 4- to 7-membered saturated heterocyclyl; or R²⁴ is taken together with either of R¹⁷ or R¹⁹, the atoms to which they are attached and any intervening atoms, to form an optionally substituted 5- to 8-membered ring system;
   Q is a bicyclic arylene, a bicyclic heteroarylene, or a bicyclic heterocyclylene, wherein:
      a first ring in Q is bonded to X, and a second ring in Q is bonded Z; and
      Q is optionally substituted with one or more independently selected substituents selected from =O; -CN; -C₁-C₅ alkyl optionally substituted with one or more independently selected halo, CN, OH, -O-(C₁-C₃ alkyl), -C(O)-(C₁-C₃ alkyl), -O-(C₂-C₃ alkynyl), -(C₃-C₆ cycloalkyl), or a 4- to 7-membered saturated heterocyclyl; -O-(C₁-C₃ alkyl) optionally substituted with one or more independently selected halo; C₂-C₅ alkenyl optionally substituted with one or more independently selected -CN, or -OH; C₂-C₃ alkynyl; -S(O)₂-C₁-C₃ alkyl; -(CH₂)₀-₁-C₃-C₆ cycloalkyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heteroaryl optionally substituted with one or more independently selected halo, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heterocyclyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-aryl optionally substituted with one or more independently selected halo, -CN, -C₁-C₃ alkyl optionally substituted with -CN or -O-C₁-C₃ alkyl, -C(O)-saturated heterocyclyl, -O-saturated heterocyclyl, O-cycloalkyl, or -O-aryl; -C(O)-NH-(C₁-C₃ alkyl); -C(O)-N(C₁-C₃ alkyl)₂; C₂-C₃ alkenylene=N-O-(C₁-C₃ alkyl) optionally substituted with C₃-C₆ cycloalkyl; or
      two substituents on the same or adjacent ring atoms of Q are taken together to form a 5- to 7-membered monocyclic ring or a 6- to 12-membered bicyclic ring optionally substituted with one or more independently selected halo, =O, -CN, C₁-C₃ alkyl, or -O-C₁-C₃ alkyl; and fused to Q.

In some embodiments, the compound, or pharmaceutically acceptable salt thereof, has the structure of formula (Ib): 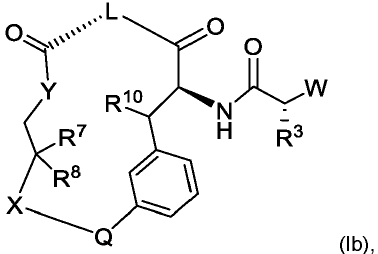 or a pharmaceutically acceptable salt, an enantiomer, a stereoisomer, or a tautomer thereof.

In some embodiments, the compound, or pharmaceutically acceptable salt thereof, has the structure of formula (Ic): 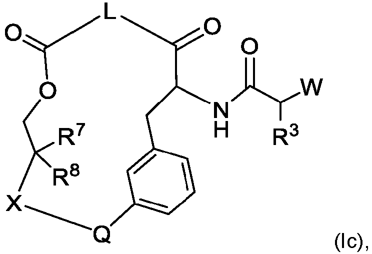 or a pharmaceutically acceptable salt, an enantiomer, a stereoisomer, or a tautomer thereof.

In some embodiments, Q is a 5,6 bicyclic heteroarylene, a 5,6 bicyclic heterocyclylene, a 6,6 bicyclic heteroarylene, or a 6,6 bicyclic heterocyclylene; and where Q is optionally substituted. In some embodiments, Q is a 5,6 bicyclic heteroarylene, wherein Q is optionally substituted. In some embodiments, Q is a 5,6 bicyclic heterocyclylene, wherein Q is optionally substituted. In some embodiments, Q is a 6,6 bicyclic heteroarylene, wherein Q is optionally substituted. In some embodiments, Q is a 6,6 bicyclic heterocyclylene, wherein Q is optionally substituted.

In some embodiments, Q is selected from the group consisting of: 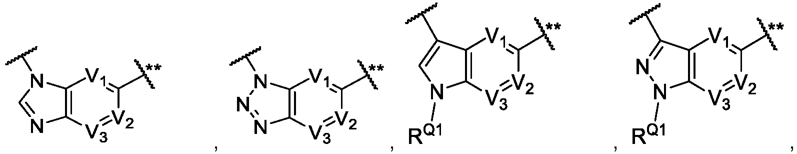 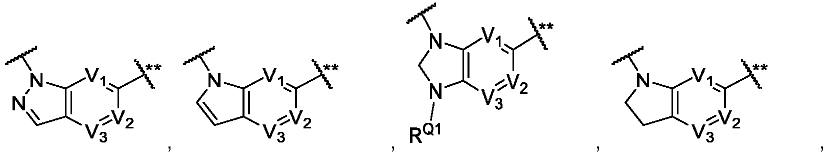 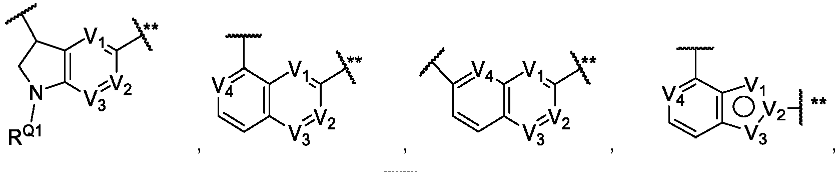 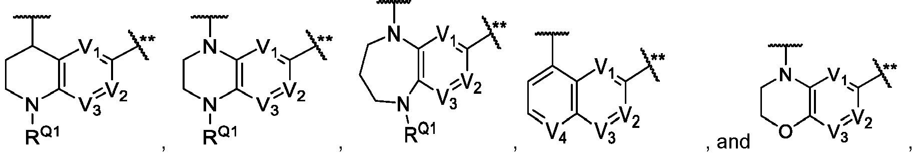 wherein:
each of V₁, V₂, V₃ and V₄ is independently C, CH, or N;
R^{Q1} is -S(O)₂-R^{Q11}, - C(O)-R^{Q11}, -S(O)₂-N(R^{Q11})R^{Q12}, -C(O)-N(R^{Q11})R^{Q12}, C₁-C₁₀ alkyl, C₃-C₁₀ cycloalkyl, a 4- to 14-membered heterocyclyl, aryl, or heteroaryl, where the alkyl, cycloalkyl, heterocyclyl, aryl, or heteroaryl are optionally substituted; or
R^{Q1} is taken together with the nitrogen atom to which it is attached and an adjacent ring atom to form an optionally substituted 4- to 8-membered ring, which is optionally further fused to a 5- to 6-membered ring;
each of R^{Q11} and R^{Q12} is independently C₁-C₁₀ alkyl, C₃-C₁₀ cycloalkyl, a 4- to 14-membered heterocyclyl, aryl, or heteroaryl, where each of R^{Q11} and R^{Q12} is optionally substituted; or
R^{Q11} and R^{Q12} are taken together with the nitrogen atom to which they are both attached to form an optionally substituted 4- to 8-membered ring, where the ring formed by taking R^{Q11} and R^{Q12} together is optionally fused to another 5- to 6-membered ring.
In some embodiments, Q is optionally additionally substituted with 1 to 4 substituents independently selected from =O; halo; -OH; -CN; -C₁-C₅ alkyl optionally substituted with one or more independently selected halo, CN, OH, -O-(C₁-C₃ alkyl), -C(O)-(C₁-C₃ alkyl), -O-C(O)-N(C₁-C₃ alkyl)₂, -O-(C₂-C₃ alkynyl), -(C₃-C₆ cycloalkyl), a 5- to 6-membered heteroaryl optionally substituted with one or more C₁-C₃ alkyl, or a 4- to 7-membered saturated heterocyclyl; -O-(C₁-C₃ alkyl) optionally substituted with one or more independently selected halo; -C₂-C₅ alkenyl optionally substituted with one or more independently selected -CN, or -OH; C₂-C₃ alkynyl optionally substituted with a heteroaryl; -S(O)₂-C₁-C₃ alkyl; -(CH₂)₀-₁-C₃-C₆ cycloalkyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heteroaryl optionally substituted with one or more independently selected halo, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heterocyclyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-aryl optionally substituted with one or more independently selected halo, -CN, -C₁-C₃ alkyl optionally substituted with -CN, -C(O)-O-C₁-C₃ alkyl, -C₁-C₃ alkylene-O-C₁-C₃ alkyl, -O-C₁-C₃ alkyl, NO₂, -C(O)-saturated heterocyclyl, -CH₂-saturated heterocyclyl, -O-saturated heterocyclyl, O-cycloalkyl, or -O-aryl; -CH₂-O-heteroaryl, -C(O)-NH-(C₁-C₃ alkyl); -C(O)-N(C₁-C₃ alkyl)₂; C₂-C₃ alkenylene=N-O-(C₁-C₃ alkyl) optionally substituted with C₃-C₆ cycloalkyl; or
two substituents on Q are taken together to form a 5- to 7-membered monocyclic ring or a 6- to 12-membered bicyclic ring optionally substituted with one or more independently selected halo, =O, -CN, C₁-C₃ alkyl, or -O-C₁-C₃ alkyl, and fused to Q; and
"**" represents a portion of Q that is bound to ring Z.

In some embodiments, Q is 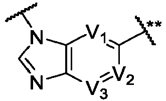 . In some embodiments, Q is 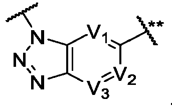 In some embodiments, Q is 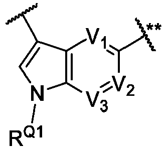 In some embodiments, Q is 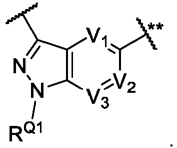 In some embodiments, Q is 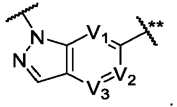 In some embodiments, Q is 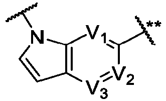 In some embodiments, Q is 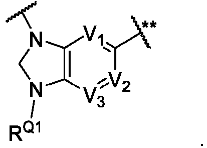 In some embodiments, Q is 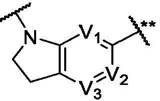 In some embodiments, Q is 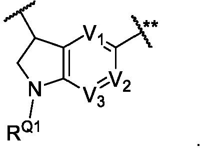 In some embodiments, Q is 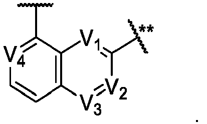 In some embodiments, Q is 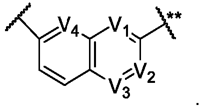 In some embodiments, Q is 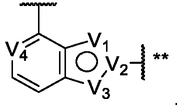 In some embodiments, Q is 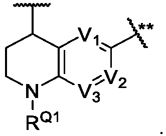 In some embodiments, Q is 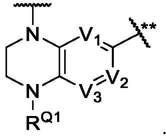 In some embodiments, Q is 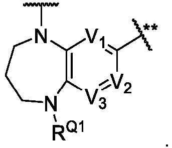 In some embodiments, Q is 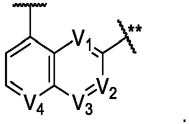 In some embodiments, Q is

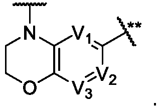

In some embodiments, Q is optionally additionally substituted with 1 to 4 substituents independently selected from chloro, fluoro, -CN, -CH₃, -CF₃, -CHF₂, -CH₂CH₃, -CH₂-CN, -(CH₂)₂-CN, -OCH₃, -CH₂-O-CH₃, -(CH₂)₂-O-CH₃, -CH₂-O-CH₂-CN, -CH(CN)-CH₃, -C(O)-N(CH₃)₂, -C(O)-NH-CH₃, -C(O)-CH₃, -S(O)₂CH₃, -C(CH₃)=N-O-CH(CH₃)₂, -C(CH₃)=N-O-CH₃, -C≡C-CH₃, -C=CH, -CH=CH-CN, -CH₂-O-CH₂-C≡CH, -C(CH₃)(CN)CH₂CN, -CH₂-O-C(O)-N(CH₃)₂, 1-(cyclopentyl)-1-cyanoethan-1-yl, 1-(tetrahydrofuran-3-yl)-1-cyanoethan-1-yl, 1-(tetrahydropyran-4-yl)-1-cyanoethan-1-yl, 1,3-dimethoxy-2-cyanopropan-2-yl, 1,4-dimethylpyrazol-5-yl, 1-cyanocyclobutyl, 1-cyanocyclopropyl, 1-cyanocylopentyl, 1-methyl-1,2,3,6-tetrahydropyridin-4-yl, 1-methylpiperidin-4-yl, 1-methylpyrazol-3-yl, 1-methylpyrazol-5-yl, (1-methylpyrazol-4-yl)cyanomethyl, 1-oxoindolin-5-yl, 1-oxoisoindolin-4-yl, 1-oxoisoindolin-6-yl, 2-(2-methoxyethan-1-yl)phenyl, 3-(1,1-dioxothiomorpholin-1-ylmethyl)phenyl, 2-(tetrahydropyran-4-yloxy)phenyl, 2,2-difluoro-benzo[d][1,3]dioxol-4-yl, 2-chlorophenyl, 2-cyano-2-tetrahydrofuran-3-ylpropanyl, 2-cyano-3-chlorophenyl, 2-cyano-3-fluorophenyl, 2-cyano-3-methoxyphenyl, 2-cyano-4-fluorophenyl, 2-cyano-4-chlorophenyl, 2-cyano-4-methoxybutan-2-yl, 2-cyano-5-chlorophenyl, 2-cyano-5-fluorophenyl, 2-cyano-5-methoxyphenyl, 2-cyano-5-(methoxymethyl)phenyl, 2-cyano-6-chlorophenyl, 2-cyano-6-fluorophenyl, 2-cyano-6-bromophenyl, 2-cyano-6-(methoxymethyl)phenyl, 2-cyano-6-(tetrahydropyran-4-yloxy)phenyl, 2-cyanomethylphenyl, 2-cyanophenyl, 2-cyanopropan-2-yl, 2-cyclopentylphenyl, 2-difluoromethoxyphenyl, 2-fluorophenyl, 2-methoxy-6-cyanophenyl, 2-methoxyphenyl, 2-methoxycarbonylphenyl, 2-(methoxymethyl)phenyl, 2-nitrophenyl, 2-oxopyrrolidin-1-yl, 2-phenoxyphenyl, 3-(2-methoxyethan-1-yl)phenyl, 3-methoxycarbonylphenyl, 3,5-difluoro-4-(pyrrolidin-1-ylcarbonyl)phenyl, 3-cyano-2-methylpropan-2-yl, 3-cyanomethylphenyl, 3-cyanopentan-3-yl , 3-cyanophenyl, 3-hydroxy-2-methylbutan-2-yl, 3-hydroxy-3-methyl-but-1-yne-1-yl, 3-methoxy-2-methylbutan-2-yl, 3-methoxyphenyl, 3-methoxymethyl-5-methylisoxazol-4-yl, 3-oxo-2-methylbutan-2-yl, 3-(tetrahydropyran-4-yl)-2-cyanopropan-2-yl, 4-cyanophenyl, 4-cyanotetrahydropyran-4-yl, 4-methoxyphenyl, benzo[d][1,3]dioxol-4-yl, benzo[d]oxazol-7-yl, benzo[d]thiazol-2-yl, benzo[d]thiazol-4-yl, benzo[d]thiazol-5-yl, benzo[d]thiazol-6-yl, benzo[d]thiazol-7-yl, cyclobutyl, cyclopropyl, cyclopropylcyanomethyl, morpholin-4-ylmethyl, N-methoxycyclopropanecarbimidoyl, phenyl, pyrazol-1-ylmethyl, pyridin-2-yl, pyridin-2-ylmethyl, pyridin-2-yloxymethyl, pyridin-3-yl, pyridin-3-yl-ethynyl, pyridin-3-ylmethyl, pyridin-4-ylmethyl, pyridin-4-yl-ethynyl, tetrahydrofuran-3-ylmethyl, tetrahydrofuran-3-ylcyanomethyl, tetrahydropyridin-4-yl, tetrahydropyran-4-ylmethyl, 2-(tetrahydropyran-4-yl)ethan-1-yl, tetrahydropyran-4-ylcyanomethyl, or tetrahydropyran-4-yl, or
two substituents attached to the same carbon atom are taken together to form =O, 2,3-dihydrobenzofuran-3,3-diyl, 2,3-dihydrofuro[2,3-b]pyridin-3,3-diyl, tetrahydropyran-3,3-diyl, 6,7-dihydro-5H-cyclopenta[c]pyridin-6,6-diyl, or tetrahydropyran-4,4-diyl, or
two substituents attached to adjacent carbon atoms are taken together to form 4-cyanobenzene-1,2-diyl, 3-cyanobenzene-1,2-diyl, 5-methyl-5-cyanotetrahydropyran-3,4-diyl, 3-cyanocyclohexan-1,2-diyl, 3-methoxybenzene-1,2-diyl, benzene-1,2-diyl, 3-oxocyclohexyl-1,2-diyl, 3-cyanocyclopentan-1,2-diyl, or pyridin-3,4-diyl.

In some embodiments, Q is selected from the group consisting of: 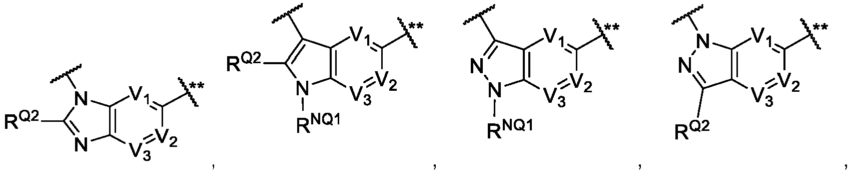 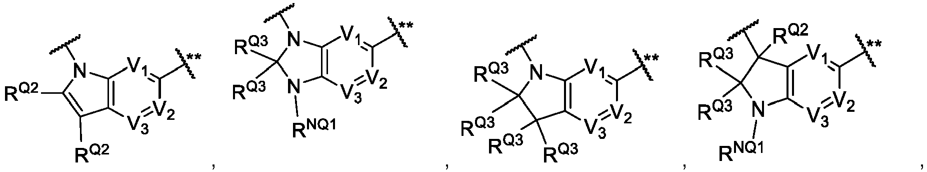 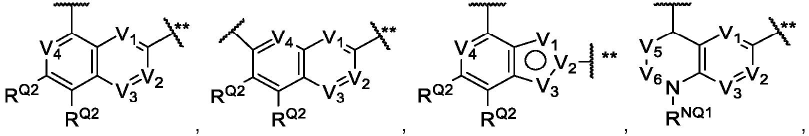 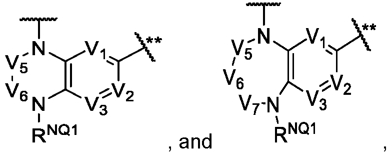 wherein:
each of V₁, V₂, V₃ and V₄ is independently CH, N, C(F), C(CH₃), C(OH), C(OCH₃), or C(CN);
each of V₅, V₆, and V₇ is independently, C(R^{17a})(R^{17b}), or C(=O), where each of R^{17a} and R^{17b} is independently selected from hydrogen, halo, -C₁-C₃ alkyl, -C₁-C₃ haloalkyl, -O-C₁-C₃ alkyl, -O-C₁-C₃ haloalkyl, and no more than two of V₅, V₆, and V₇ is C(=O);
R^{NQ1} is hydrogen, optionally
   substituted -S(O)₂-R^{Q11}, -C(O)-R^{Q11}, -S(O)₂-N(R^{Q11})R^{Q12}, -C(O)-N(R^{Q11})R^{Q12}, -C₁-C₁₀ alkyl, -C₃-C₁₀ cycloalkyl, a 4- to 14-membered heterocyclyl, aryl, or heteroaryl, where the alkyl, cycloalkyl, heterocyclyl, aryl, or heteroaryl are optionally substituted;
each R^{Q2} is independently hydrogen, CN, optionally
   substituted -S(O)₂-R^{Q11}, -C(O)-R^{Q11}, -S(O)₂-N(R^{Q11})R^{Q12}, -C(O)-N(R^{Q11})R^{Q12}, C₁-C₁₀ alkyl, C₃-C₁₀ cycloalkyl, a 4- to 14-membered heterocyclyl, aryl, or heteroaryl, where the alkyl, cycloalkyl, heterocyclyl, aryl, or heteroaryl are optionally substituted; or
R^{NQ1} and one R^{Q2} are taken together with the atoms to which they are bound to form an optionally substituted 4- to 8-membered ring, where the ring formed by taking R^{NQ1} and one R^{Q2} together is optionally further fused to a 5- to 6-membered ring;
each R^{Q3} is independently hydrogen, CN, optionally
   substituted -S(O)₂-R^{Q11}, - C(O)-R^{Q11}, -S(O)₂-N(R^{Q11})R^{Q12}, -C(O)-N(R^{Q11})R^{Q12}, C₁-C₁₀alkyl, C₃-C₁₀ cycloalkyl, a 4-14 membered heterocyclyl, aryl, or heteroaryl, where the alkyl, cycloalkyl, heterocyclyl, aryl, or heteroaryl are optionally substituted, or
two R^{Q3} bound to the same atom are taken together to form =CH, =O, =S, or =NR^{V4}; or
two R^{Q3} bound to the same atom are taken together with the atom to which they are bound to form an optionally substituted 4- to 8-membered ring, where the ring formed by taking each R^{Q3} together is optionally further fused to a 5- to 6-membered ring; or
R^{NQ1} and one R^{Q3} are taken together with the atoms to which they are bound to form an optionally substituted 4- to 8-membered ring, where the ring formed by taking R^{NQ1} and R^{Q3} together is optionally further fused to a 5- to 6-membered ring;
each of R^{Q11} and R^{Q12} is independently C₁-C₁₀ alkyl, C₃-C₁₀ cycloalkyl, a 4- to 14-membered heterocyclyl, aryl, or heteroaryl, where each of R^{Q11} and R^{Q12} is optionally substituted; or
R^{Q11} and R^{Q12} are taken together with the atoms to which they are attached to form an optionally substituted 4- to 8-membered ring, where the ring formed by taking R^{Q11} and R^{Q12} together is optionally fused to another 5- to 6-membered ring; and
"**" represents a portion of Q that is bound to ring Z.

In some embodiments, Q is 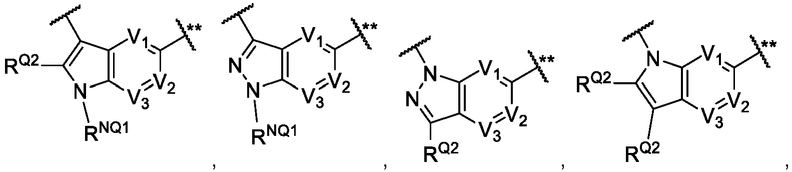 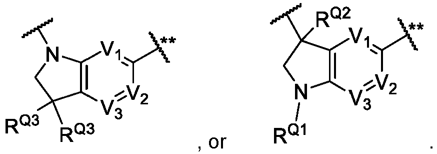 In some embodiments, Q is 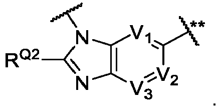 In some embodiments, Q is 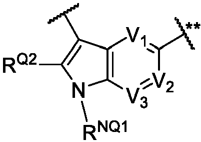 . In some embodiments, Q is 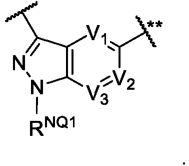 In some embodiments, Q is 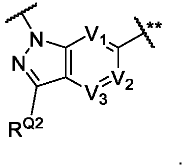 In some embodiments, Q is 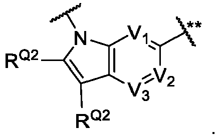 In some embodiments, Q is 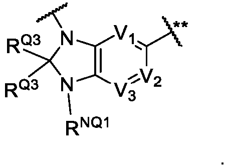 In some embodiments, Q is 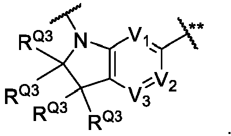 In some embodiments, Q is 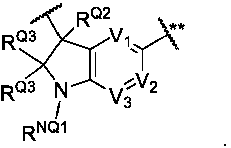 In some embodiments, Q is 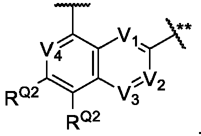 In some embodiments, Q is 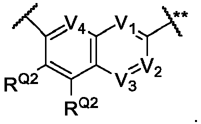 In some embodiments, Q is 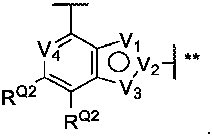 In some embodiments, Q is 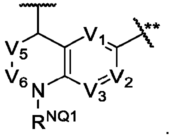 In some embodiments, Q is 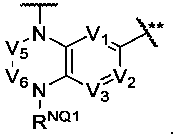 In some embodiments, Q is

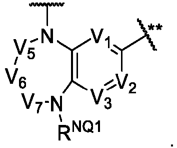

In some embodiments, Q is selected from the group consisting of: 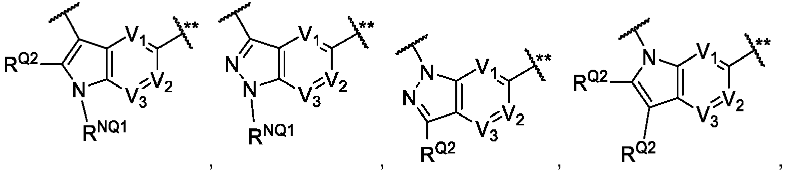 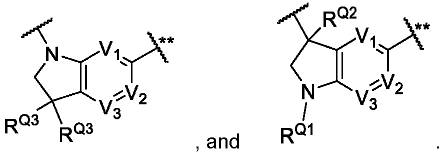 In some embodiments, Q is 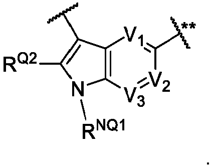 In some embodiments, Q is 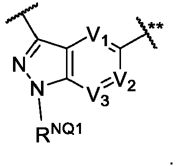 In some embodiments, Q is 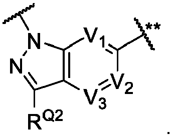 In some embodiments, Q is 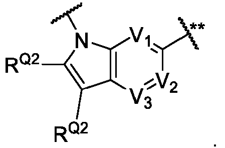 In some embodiments, Q is 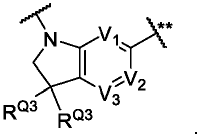 In some embodiments, Q is

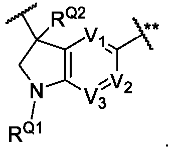

In some embodiments, the compound, or pharmaceutically acceptable salt thereof, has the structure of formula (Id): 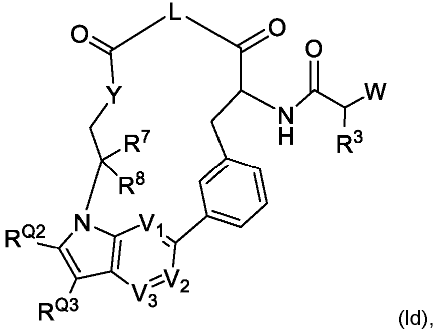 or a pharmaceutically acceptable salt, an enantiomer, a stereoisomer, or a tautomer thereof.

In some embodiments, the compound, or pharmaceutically acceptable salt thereof, has the structure of formula (le): 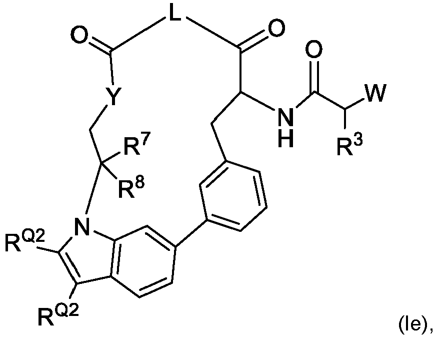 or a pharmaceutically acceptable salt, an enantiomer, a stereoisomer, or a tautomer thereof.

In some embodiments, the compound, or pharmaceutically acceptable salt thereof, has the structure of formula (Ig): 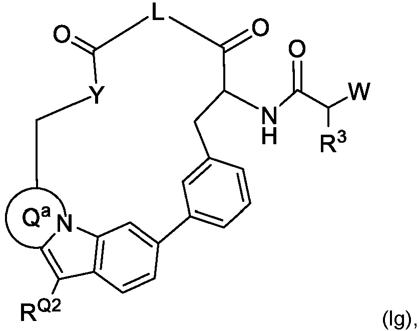 or a pharmaceutically acceptable salt, an enantiomer, a stereoisomer, or a tautomer thereof, where Q^{a} is a 4- to 9-membered saturated heterocyclyl.

In some embodiments, the compound, or pharmaceutically acceptable salt thereof, has the structure of formula (Ij): 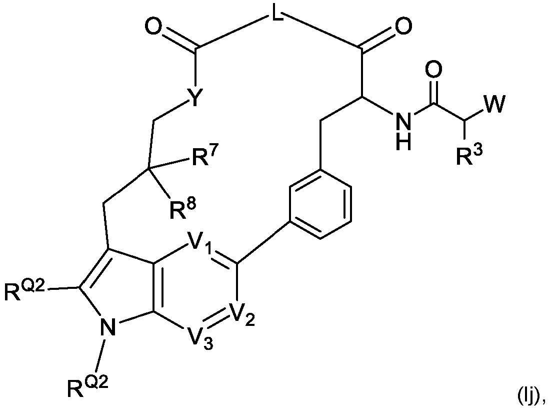 or a pharmaceutically acceptable salt, an enantiomer, a stereoisomer, or a tautomer thereof.

In some embodiments, the compound, or pharmaceutically acceptable salt thereof, has the structure of formula (Ik): 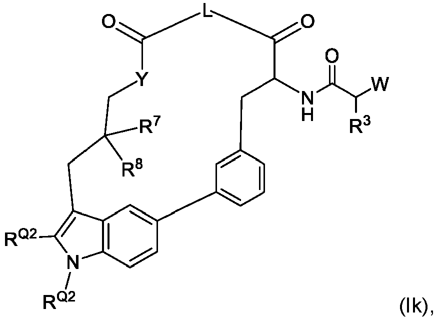 or a pharmaceutically acceptable salt, an enantiomer, a stereoisomer, or a tautomer thereof.

In some embodiments, the compound, or pharmaceutically acceptable salt thereof, has the structure of formula (Ik'): 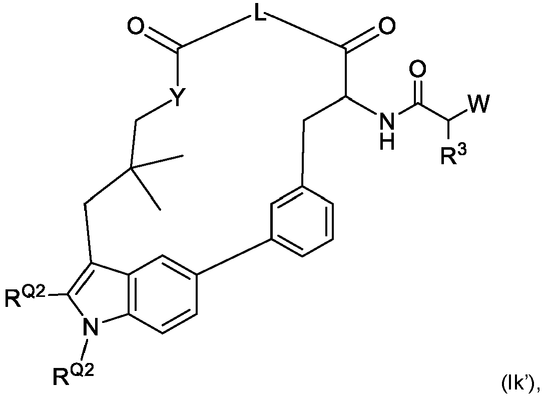 or a pharmaceutically acceptable salt, an enantiomer, a stereoisomer, or a tautomer thereof.

In some embodiments, Q is selected from the group consisting of: 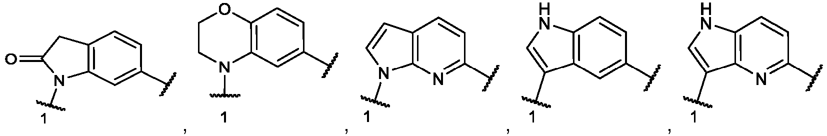 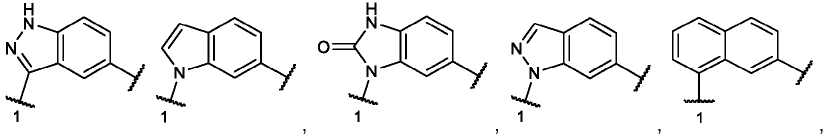 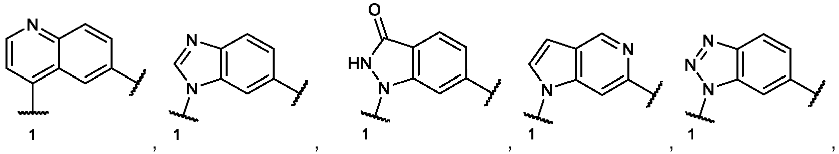 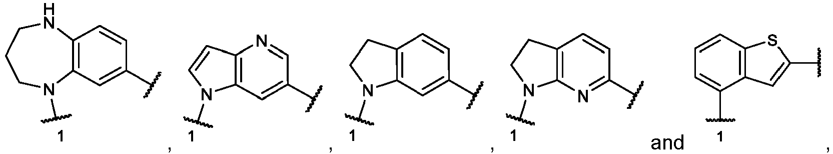 wherein:
"1" indicates a portion of Q bound to X; and Q is further optionally substituted. In some embodiments, Q is 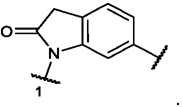 In some embodiments, Q is 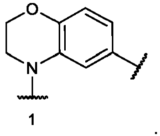 In some embodiments, Q is 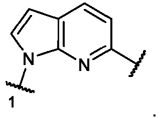 In some embodiments, Q is 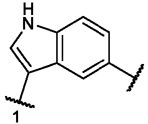 . In some embodiments, Q is 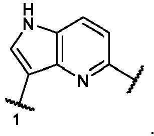 In some embodiments, Q is 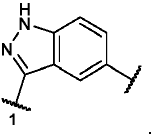 In some embodiments, Q is 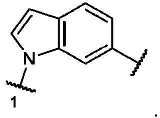 In some embodiments, Q is 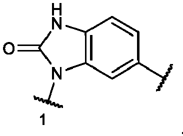 In some embodiments, Q is 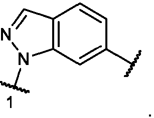 In some embodiments, Q is 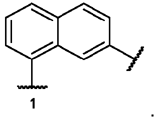 In some embodiments, Q is 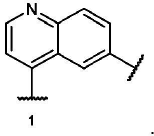 In some embodiments, Q is 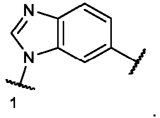 In some embodiments, Q is . In some embodiments, Q is 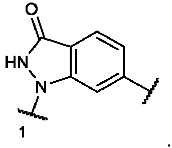 In some embodiments, Q is 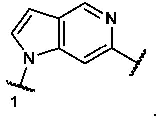 In some embodiments, Q is 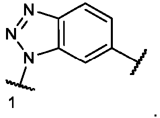 In some embodiments, Q is 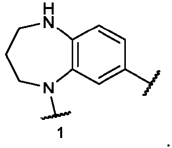 In some embodiments, Q is 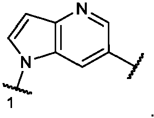 In some embodiments, Q is 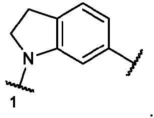 In some embodiments, Q is 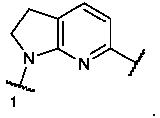 In some embodiments, Q is

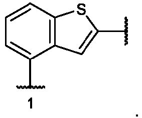

In some embodiments, Q is selected from the group consisting of: 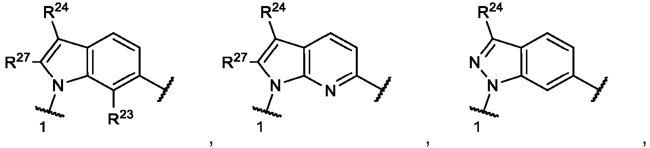 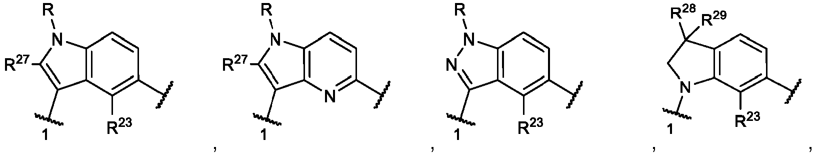 and 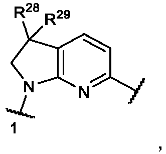 wherein:
R is -CH₂CH₃, -CH₂CH-OCH₃, -CH₂CHF₂, -CH₂-CN, CH₂(CH₃)₂-CN, -C(CH₃)₂-CH₂CN, - CH₂CH₂-CN, cyclohexyl, cyclobutyl, cyclopropyl, pyridin-4-yl, tetrahydropyran-4-yl, tetrahydropyran-4-ylmethyl, oxetan-3-ylmethyl, 2-cyano-5-methoxyphenyl, 2-cyano-5-methoxymethylphenyl, 2-cyano-6-(methoxymethyl)phenyl, 2-cyano-6-bromophenyl, 2-methoxyethan-1-yl, 2-cyanopropan-2-yl, 2-tetrahydropyran-4-ylethan-1-yl, 3-cyanopentan-3-yl, 2-cyano-4-methoxybutan-2-yl, or R is 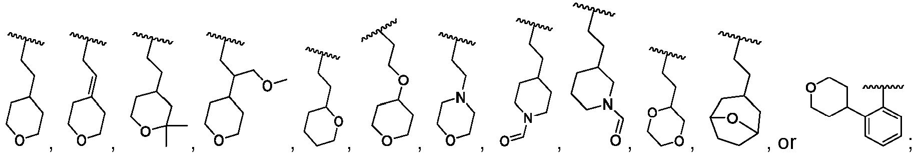 R²³ is hydrogen or fluoro;
R²⁴ is hydrogen,
   chloro, -CN, -CH₃, -CH₂CH₃, -CHF₂, -CF₃, -CH₂-CN, -CH(CN)-CH₃, -C(CH₃)₂-CN, -C(CH₂CH₃)₂-CN, -CH₂-CH₂-CN, -C(CH₃)=N-O-CH(CH₃)₂, -C(CH₃)=N-O-CH₃, -C(O)-N(CH₃)₂, -C(O)-NH-CH₃, -OCH₃, -CH₂-O-CH₃, -C=CH, -C≡C-CH₃, -S(O)₂CH₃, 1-(cyclopentyl)-1-cyanoethan-1-yl, 1-(tetrahydropyran-4-yl)-1-cyanoethan-1-yl, 1-(tetrahydrofuran-3-yl)-1-cyanoethan-1-yl, 1,3-dimethoxy-2-cyanopropan-2-yl, 1,4-dimethylpyrazol-5-yl, 1-cyanocyclobutyl, 1-cyanocyclopropyl, 1-cyanocylopentyl, 1-methyl-1,2,3,6-tetrahydropyridin-4-yl, 1-methylpyrazol-3-yl, 1-methylpyrazol-4-ylcyanomethyl, 1-methylpiperidin-4-yl, 1-methylpyrazol-5-yl, 1-oxoindolin-5-yl, 1-oxoisoindolin-4-yl, 1-oxoisoindolin-6-yl, 2-(2-methoxyethan-1-yl)phenyl, 2-(methoxymethyl)phenyl, 2-(tetrahydropyran-4-yloxy)phenyl, 2,2-difluoro-benzo[d][1,3]dioxol-4-yl, 2,3-dicyanopropan-2-yl, 2-chlorophenyl, 2-cyano-3-(tetrahydropyran-4-yl)propan-2-yl, 2-cyano-3-chlorophenyl, 2-cyano-3-fluorophenyl, 2-cyano-3-methoxyphenyl, 2-cyano-4-fluorophenyl, 2-cyano-4-chlorophenyl, 2-cyano-5-chlorophenyl, 2-cyano-5-fluorophenyl, 2-cyano-5-methoxyphenyl, 2-cyano-6-chlorophenyl, 2-cyano-6-fluorophenyl, 2-cyano-6-(tetrahydropyran-4-yloxy)phenyl, 2-cyanomethylphenyl, 2-cyanophenyl, 2-cyanopropan-2-yl, 2-cyclopentylphenyl, 2-difluoromethoxyphenyl, 2-fluorophenyl, 2-methoxy-6-cyanophenyl, 2-methoxyphenyl, 2-methoxycarbonylphenyl, 2-nitrophenyl, 2-oxopyrrolidin-1-yl, 2-phenoxyphenyl, 3-(1,1-dioxothiomorpholin-4-ylmethyl)phenyl, 3-(2-methoxyethan-1-yl)phenyl, 3,5-difluoro-4-(pyrrolidin-1-ylcarbonyl)phenyl, 3-cyano-2-methylpropan-2-yl, 3-cyanomethylphenyl, 3-cyanopentan-3-yl , 3-cyanophenyl, 3-hydroxy-2-methylbutan-2-yl, 3-hydroxy-3-methyl-but-1-yne-1-yl, 3-methoxy-2-methylbutan-2-yl, 3-methoxymethyl-5-methylisoxazol-4-yl, 3-methoxyphenyl, 3-methoxycarbonylphenyl, 3-oxo-2-methylbutan-2-yl, 4-cyanophenyl, 4-cyanotetrahydropyran-4-yl, 4-methoxyphenyl, benzo[d][1,3]dioxol-4-yl, benzo[d]oxazol-7-yl, benzo[d]thiazol-2-yl, benzo[d]thiazol-4-yl, benzo[d]thiazol-5-yl, benzo[d]thiazol-6-yl, benzo[d]thiazol-7-yl, cyclobutyl, cyclopropyl, cyclopropylcyanomethyl, N-methoxycyclopropanecarbimidoyl, phenyl, pyridin-2-ylmethyl, pyridin-3-yl, pyridin-3-ylmethyl, pyridin-4-ylmethyl, tetrahydrofuran-3-ylmethyl, tetrahydrofuran-3-ylcyanomethyl, tetrahydropyran-4-yl, or tetrahydropyran-4-ylcyanomethyl;
R²⁷ is hydrogen, -CH₃, -CHF₂, -CH₂CH₃, -CH₂-O-CH₃, -CH₂CN, -CN, -CH₂-O-CH₂-CN, -C(O)-N(CH₃)₂, -C(O)-NH-CH₃, -CH₂-O-CH₂-C≡CH, 2-methoxyphenyl, 3-methoxyphenyl, 2,2-difluorobenzo[d][1,3]dioxol-4-yl, 2-cyanophenyl, 3-cyanophenyl, phenyl, 2-benzyl methyl ether, 2-(2-methoxyethyl) benzene, 2-(2-difluoromethoxyethyl)benzene, 2-(2-dimethylmethoxyethyl)benzene, pyridin-3-yl, pyridin-2-yl, pyridin-3-ylmethyl, or tetrahydropyridin-4-yl, or
R²⁴ and R²⁷ are taken together to form 4-cyanobenzene-1,2-diyl, 3-cyanobenzene-1,2-diyl, 5-methyl-5-cyanotetrahydropyran-3,4-diyl, 3-cyanocyclohexan-1,2-diyl, 3-methoxybenzene-1,2-diyl, benzene-1,2-diyl, 3-oxocyclohexyl-1,2-diyl, 3-cyanocyclopentan-1,2-diyl, or pyridin-3,4-diyl;
R²⁸ is hydrogen, -CH₃, or -CH₂-O-CH₃; and
R²⁹ is hydrogen, acetyl, CN, -CH₂-CN, -CH₂-CH₂-CN, -CH₂-O-CH₃, -CH=CH-CN, -CH₂-O-C(O)-N(CH₃)₂, morpholin-4-ylmethyl, pyrazol-1-ylmethyl, pyridin-3-yl, pyridin-3-ylethynyl, pyridin-2-yloxymethyl, or 2-cyanopropan-2-yl, or
R²⁸ and R²⁹ are taken together to form 2,3-dihydrobenzofuran-3,3-diyl, 2,3-dihydrofuro[2,3-b]pyridin-3,3-diyl, tetrahydropyran-3,3-diyl, 6,7-dihydro-5H-cyclopenta[c]pyridin-6-yl, tetrahydropyran-4,4-diyl, or 4-methoxycyclohexane. In some embodiments, Q is 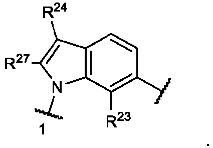 In some embodiments, Q is 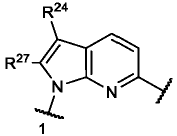 . In some embodiments, Q is 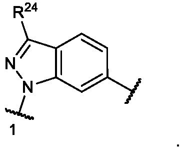 In some embodiments, Q is

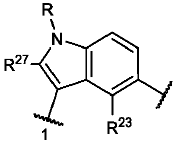

. In some embodiments, Q is 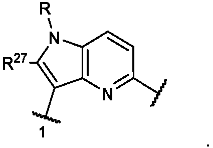 In some embodiments, Q is 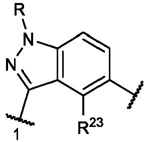 In some embodiments, Q is 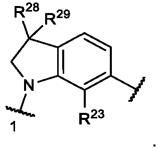 In some embodiments, Q is

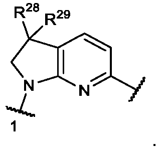

In some embodiments, R³ is -CH₃, -CH₂CH₃, -(CH₂)₂CH₃, -CH(CH₃)₂, -CH(CH₃)CH₂CH₃, cyclopropylmethyl, cyclobutylmethyl, cyclopentylmethyl, cyclohexylmethyl, cyclopropyl, cyclobutyl, cyclopentyl, cyclohexyl, phenyl, 4-methoxybenzyl, or tetrahydropyran-4-yl.

In some embodiments of compounds of Formula I or any subgeneric formula of Formula I, or a pharmaceutically acceptable salt or stereoisomer thereof, the compound is not:

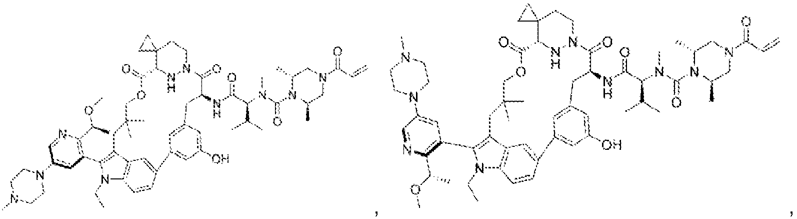

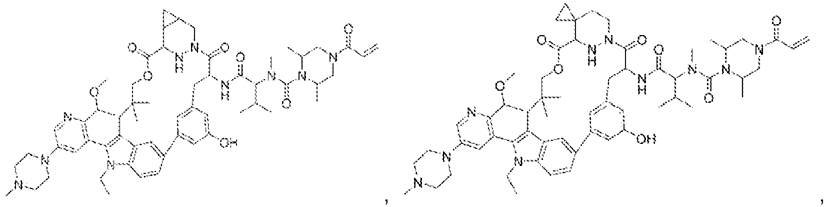

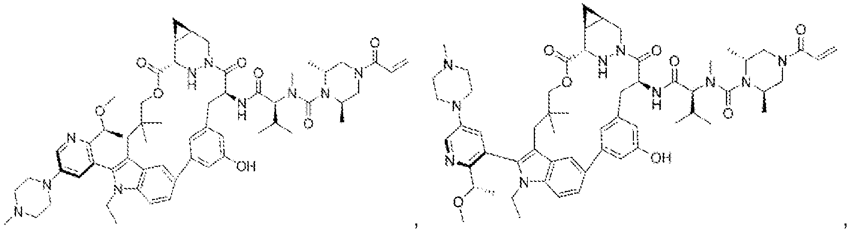

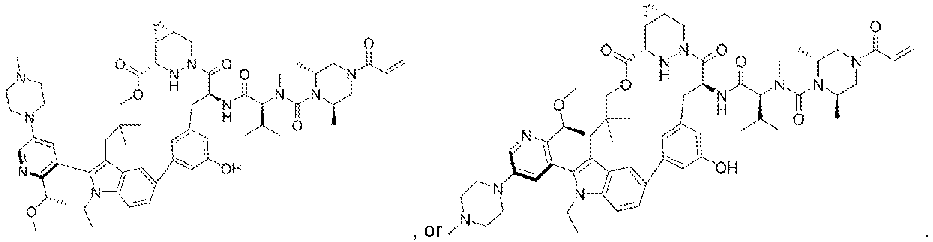

In some embodiments of compounds of Formula I or any subgeneric formula of Formula I, a pharmaceutically acceptable salt or stereoisomer thereof, when A is 1-hydroxy-benzene-3,5-diyl or optionally substituted phenylene, two R^{L1} substituents taken together form spiro cyclopropyl, and W is 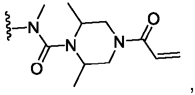 then, Q is not 1-ethyl-indole-2,5-diyl or indole-2,5-diyl substituted with C₁₋₄ alkyl.

In some embodiments of compounds of formula I, or any subgeneric formula of formula I, or a pharmaceutically acceptable salt or stereoisomer thereof, when A is 1-hydroxy-benzene-3,5-diyl or optionally substituted phenylene, two R^{L1} substituents taken together form spiro cyclopropyl, and W is 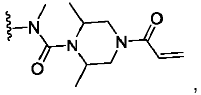 then, Q is not 1-ethyl-2,5-indol-diyl optionally substituted with 1, 2 or 3 substituents independently selected from halogen, C₁₋₄ alkyl and C₁₋₄ alkoxy, wherein the C₁₋₄ alkyl and C₁₋₄ alkoxy are each optionally substituted with 1, 2 or 3 substituents independently selected from halogen, OH, C₁₋₃ alkyl and C₁₋₃ alkoxy, wherein C₁₋₃ alkyl and C₁₋₃ alkoxy are each substituted with 1, 2 or 3 substituents independently selected from deuterium, halogen, OH, methyl, methoxy and OCF₃.

In some embodiments of compounds of formula I, or any subgeneric formula of formula I, or a pharmaceutically acceptable salt or stereoisomer thereof, when A is 1-hydroxy-benzene-3,5-diyl or optionally substituted phenylene, R^{L1} and R^{L2} taken together form fused cyclopropyl, and W is 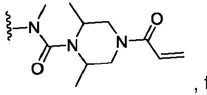 then Q is not 1-ethyl-2,5-indol-diyl or 2,5-indol-diyl substituted with C₁₋₄ alkyl.

In some embodiments of compounds of formula I or any subgeneric formula of formula I,or a pharmaceutically acceptable salt or stereoisomer thereof, when A is 1-hydroxy-benzene-3,5-diyl or optionally substituted phenylene, R^{L1} and R^{L2} taken together form fused cyclopropyl, and W is 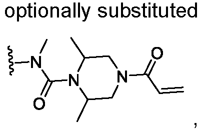 then Q is 1-ethyl-2,5-indol-diyl or 2,5-indol-diyl substituted with C₁₋₄ alkyl.

In some embodiments of compounds of formula I, or any subgeneric formula of formula I, or a pharmaceutically acceptable salt or stereoisomer thereof, when A is 1-hydroxy-benzene-3,5-diyl or optionally substituted phenylene, R^{L1} and R^{L2} taken together form fused cyclopropyl, and W is 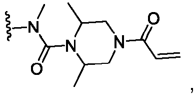 then Q is not 1-ethyl-2,5-indol-diyl optionally substituted with 1, 2 or 3 substituents independently selected from halogen, C₁₋₄ alkyl and C₁₋₄ alkoxy, wherein the C₁₋₄ alkyl and C₁₋₄ alkoxy are each optionally substituted with 1, 2 or 3 substituents independently selected from halogen, OH, C₁₋₃ alkyl and C₁₋₃ alkoxy, wherein C₁₋₃ alkyl and C₁₋₃ alkoxy are each substituted with 1, 2 or 3 substituents independently selected from deuterium, halogen, OH, methyl, methoxy and OCF₃.

In some embodiments of compounds of formula I, or any subgeneric formula of formula I, or a pharmaceutically acceptable salt or stereoisomer thereof, when A is -hydroxy-benzene-3,5-diyl or optionally substituted phenylene and two R^{L1} substituents taken together form spiro cyclopropyl, then W is not

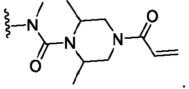

In some embodiments of compounds of formula I, or any subgeneric formula of formula I, or a pharmaceutically acceptable salt or stereoisomer thereof, when A is 1-hydroxy-benzene-3,5-diyl or optionally substituted phenylene, R^{L1} and R^{L2} taken together form fused cyclopropyl, then W is not

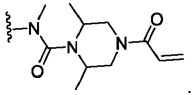

In some embodiments of compounds of formula I, or any subgeneric formula of formula I, or a pharmaceutically acceptable salt or stereoisomer thereof, when A is 1-hydroxy-benzene-3,5-diyl or optionally substituted phenylene, WH is 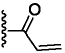 and two R^{L1} substituents taken together form spiro cyclopropyl or spiro cycloalkyl, then A1 is not 1,4-piperazin-diyl optionally substituted with methyl or C₁₋₄ alkyl.

In some embodiments of compounds of formula I, or any subgeneric formula of formula I, or a pharmaceutically acceptable salt or stereoisomer thereof, when A is 1-hydroxy-benzene-3,5-diyl or optionally substituted phenylene, WH is 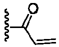 and R^{L1} and R^{L2} taken together form fused cyclopropyl or fused cycloalkyl, then A1 is not is not 1,4-piperazin-diyl optionally substituted with methyl or C₁₋₄ alkyl.

In some embodiments, the disclosure features a compound, or pharmaceutically acceptable salt thereof, of structural Formula IIa: 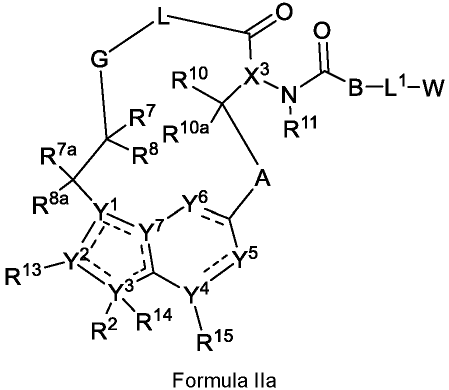
wherein the dotted lines represent zero, one, two, three, or four non-adjacent double bonds;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
G is optionally substituted C₁-C₄ alkylene, optionally substituted C₁-C₄ alkenylene, optionally substituted C₁-C₄ heteroalkylene, -C(O)O-**C**H(R⁶)- where **C** is bound to -C(R⁷R⁸)-, -C(O)NH-CH(R⁶)-where **C** is bound to -C(R⁷R⁸)-, optionally substituted C₁-C₄ heteroalkylene, or 3- to 8-membered heteroarylene;
L¹ is a linker;
W is hydrogen, cyano, S(O)₂R', optionally substituted amino, optionally substituted amido, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3- to 11-membered heterocycloalkyl, optionally substituted 3- to 8-membered cycloalkyl, or optionally substituted 3- to 8-membered heteroaryl;
L has the structure of Formula VIIa or Vllb: 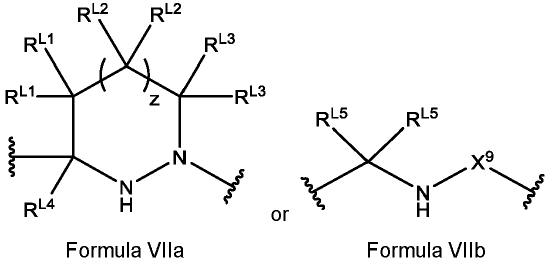
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 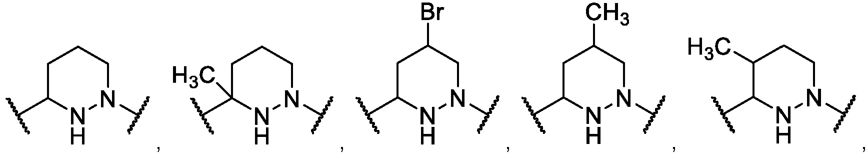 or 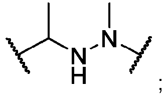
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R' is, independently, hydrogen or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 3- to 14-membered heterocycloalkyl, optionally substituted 6-membered aryl, optionally substituted 5- or 6-membered heteroaryl or optionally substituted 5- to 10-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ heteroalkyl, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halogen, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is hydrogen, F, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halogen, hydroxy, optionally substituted C₁-C₃ heteroalkyl, or optionally substituted C₁-C₃ alkyl;
R^{10a} is hydrogen or halogen;
R¹¹ is hydrogen or optionally substituted C₁-C₃ alkyl; and
R²¹ is hydrogen or optionally substituted C₁-C₃ alkyl.

In some embodiments of Formula IIa, W is hydrogen, optionally substituted amino, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 8-membered heterocycloalkyl, optionally substituted 3 to 8-membered cycloalkyl, or 3 to 8-membered heteroaryl.

In some embodiments of compounds of the present invention, W is hydrogen. In some embodiments, W is optionally substituted amino. In some embodiments, W is -NHCH₃ or -N(CH₃)₂. In some embodiments, W is optionally substituted C₁-C₄ alkoxy. In some embodiments, W is methoxy or iso-propoxy. In some embodiments, W is optionally substituted C₁-C₄ alkyl. In some embodiments, W is methyl, ethyl, iso-propyl, tert-butyl, or benzyl. In some embodiments, W is optionally substituted amido. In some embodiments, W is 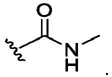 In some embodiments, W is optionally substituted amido. In some embodiments, W is 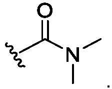 In some embodiments, W is optionally substituted C₁-C₄ hydroxyalkyl. In some embodiments, W is 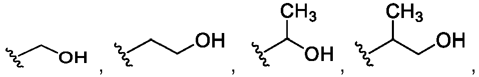 or 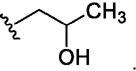 In some embodiments, W is optionally substituted C₁-C₄ aminoalkyl. In some embodiments, W is 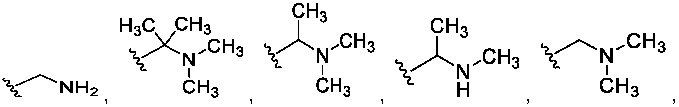 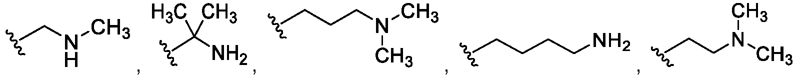 , or 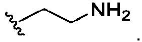 In some embodiments, W is optionally substituted C₁-C₄ haloalkyl. In some embodiments, W is 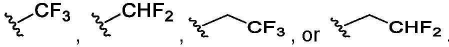 In some embodiments, W is optionally substituted C₁-C₄ guanidinoalkyl. In some embodiments, W is 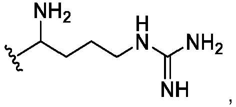 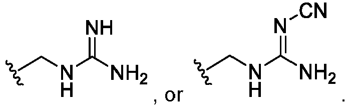 In some embodiments, W is C₀-C₄ alkyl optionally substituted 3 to 11-membered heterocycloalkyl. In some embodiments, W is 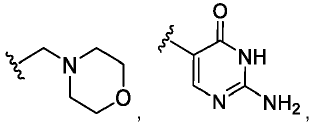 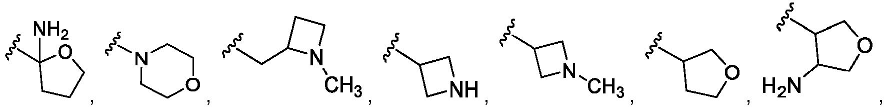 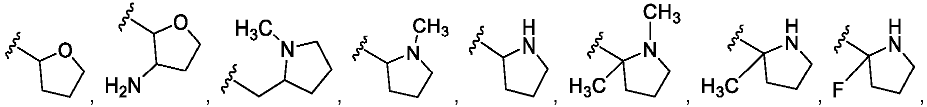 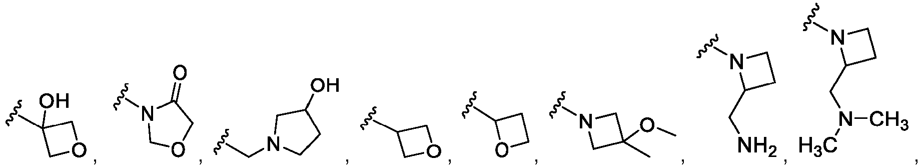 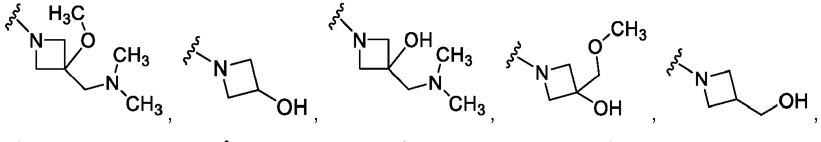 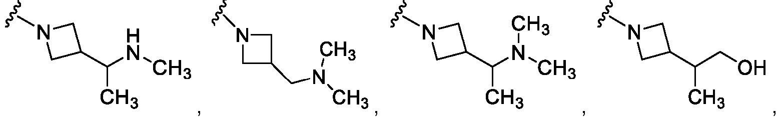 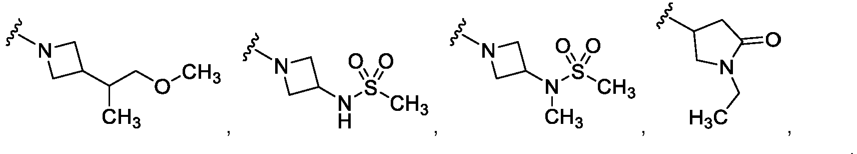 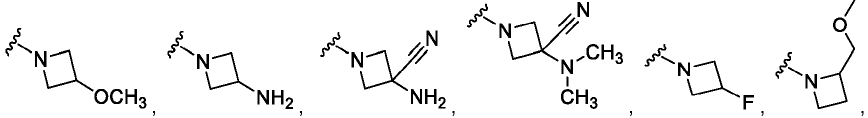 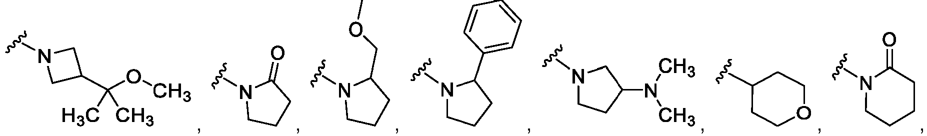 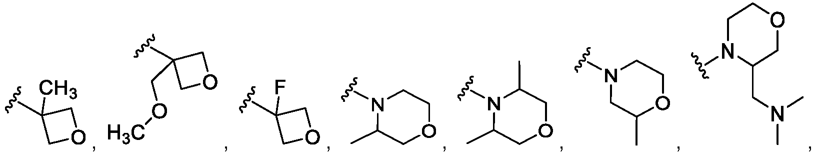 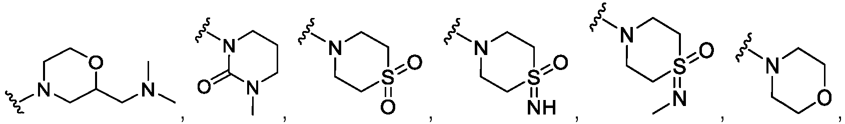 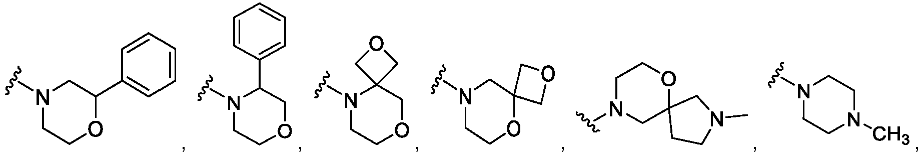 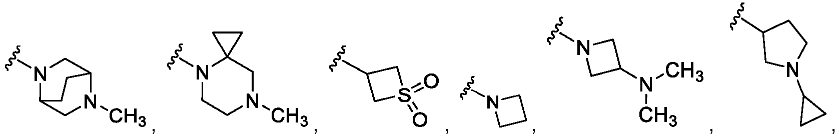 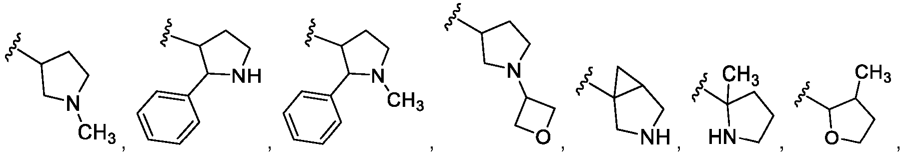 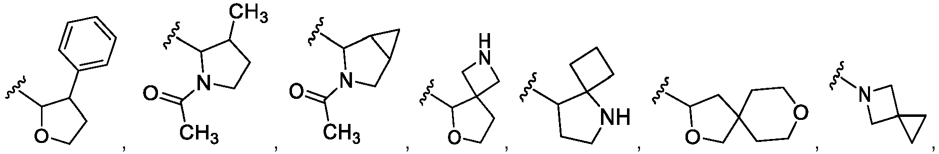 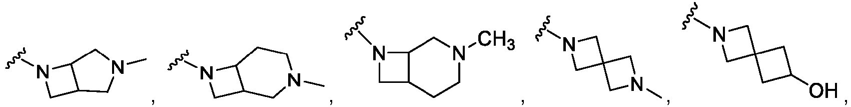 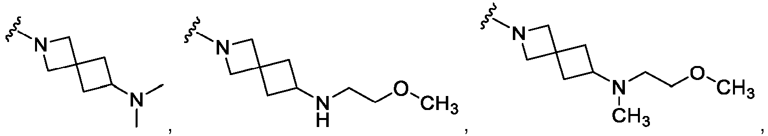 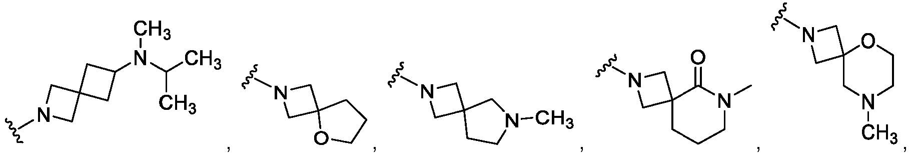 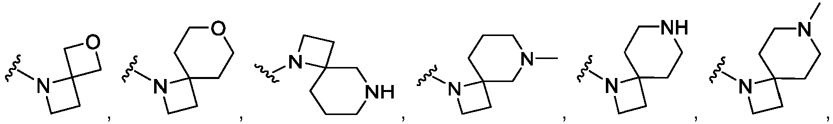 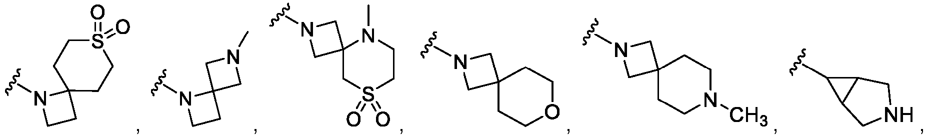 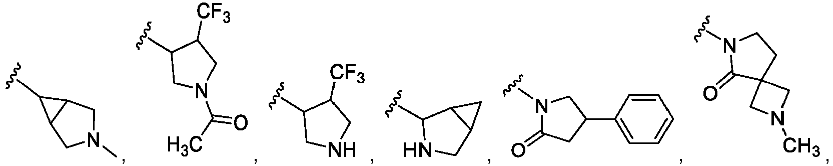 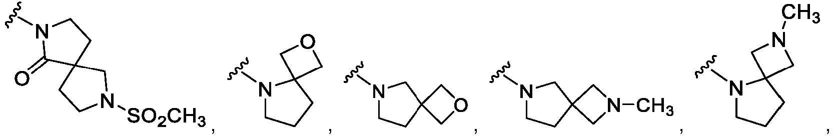 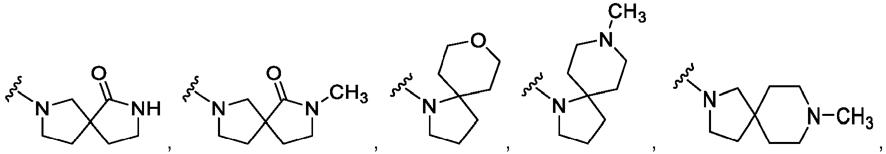 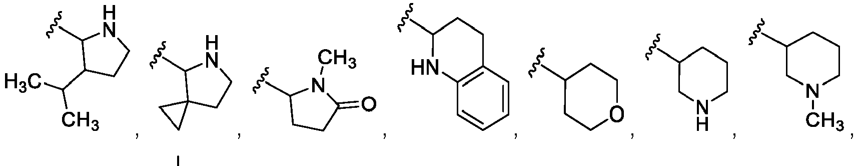 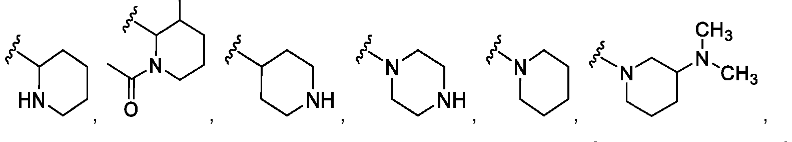 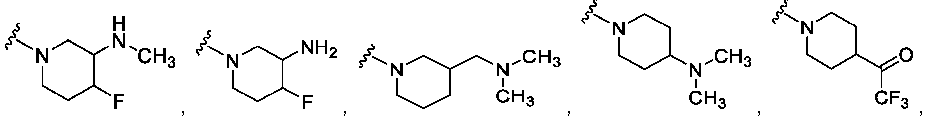 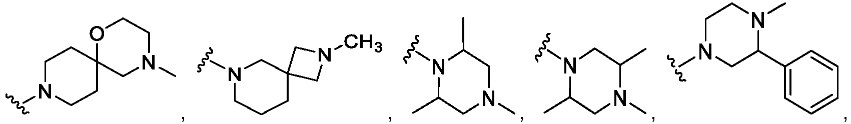 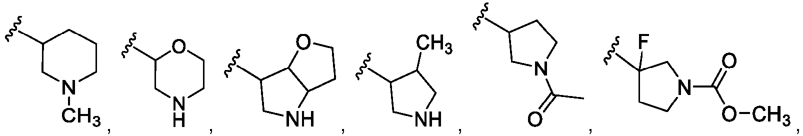 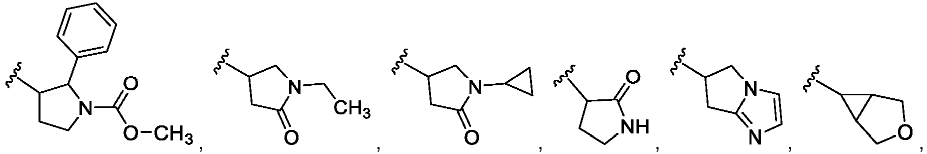 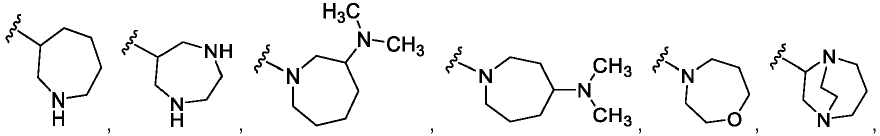 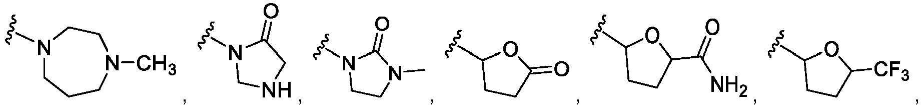 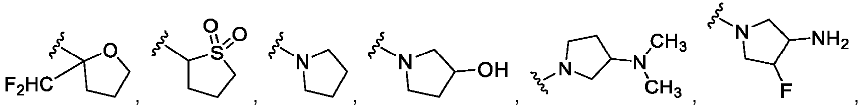 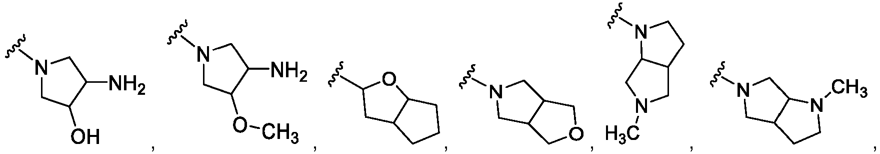 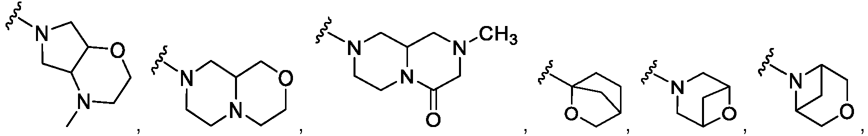 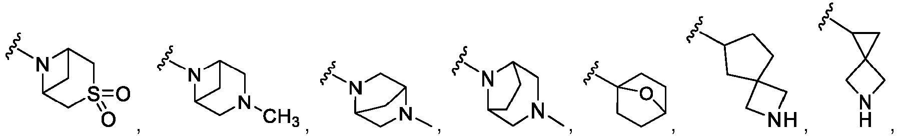 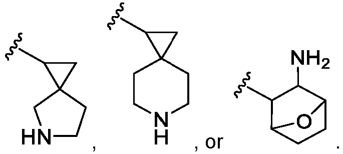 In some embodiments, W is optionally substituted 3 to 8-membered cycloalkyl. In some embodiments, W is 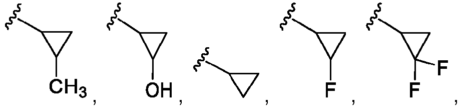 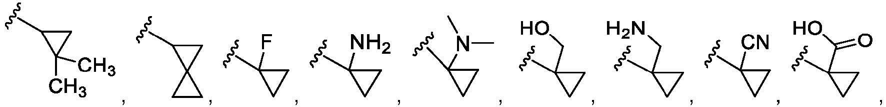 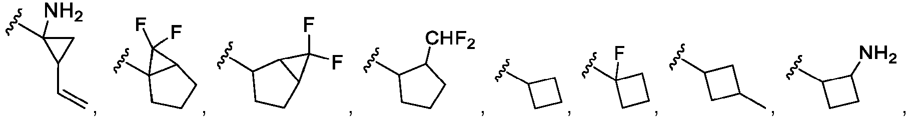 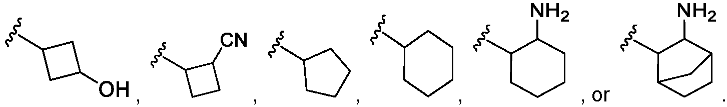 In some embodiments, W is optionally substituted 3 to 8-membered heteroaryl. In some embodiments, W is 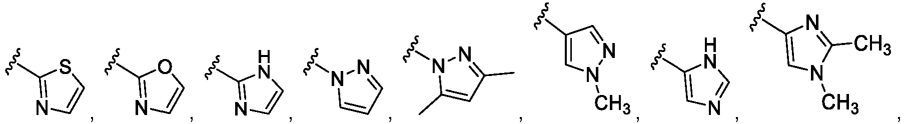 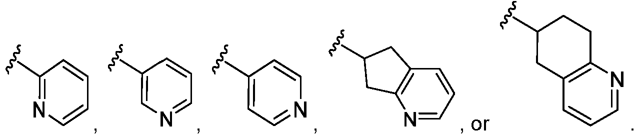 In some embodiments, W is optionally substituted 6- to 10-membered aryl (e.g., phenyl, 4-hydroxy-phenyl, or 2,4-methoxy-phenyl).

In some embodiments of compounds of formula IIb or any subgeneric formula of formula IIb, or a pharmaceutically acceptable salt or stereoisomer thereof, the compound is not

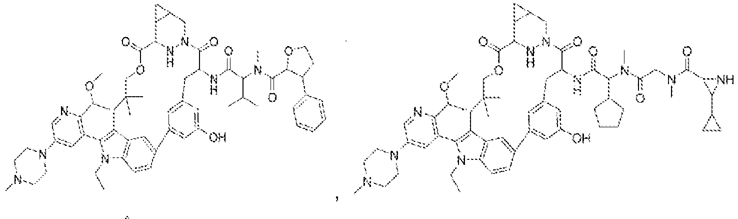

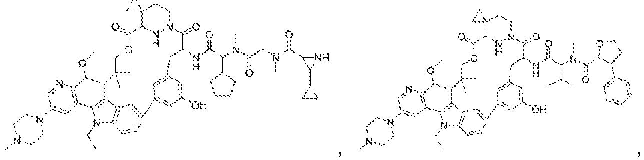

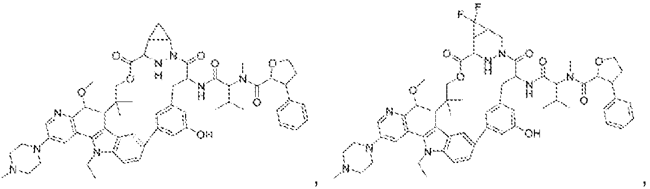

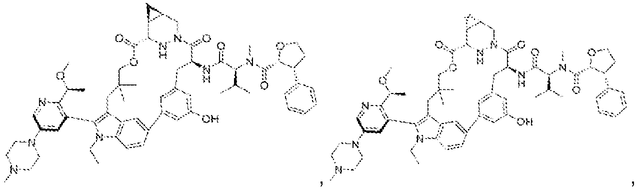

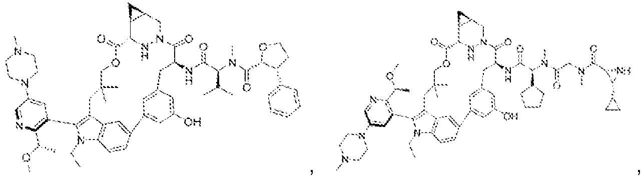

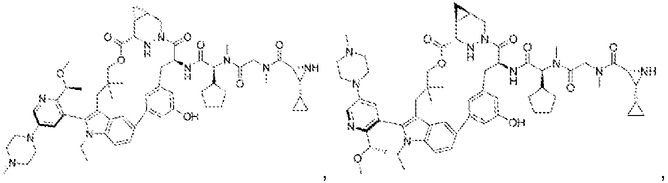

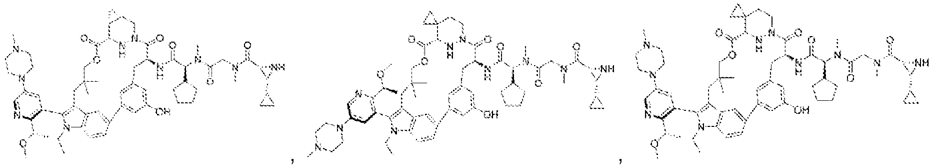

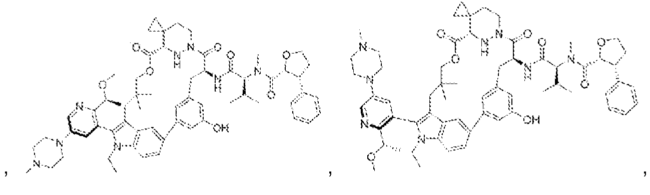

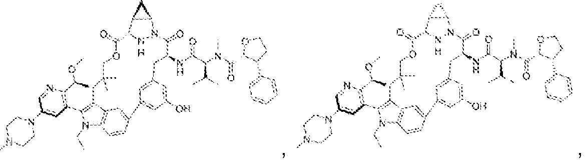

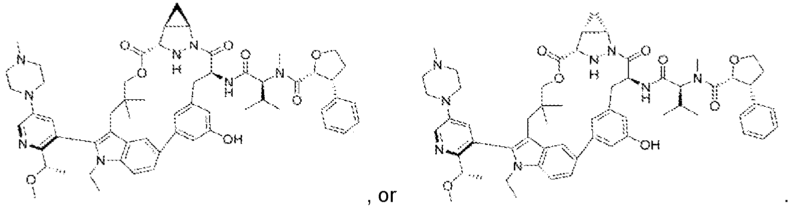

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, two R^{L1} substituents taken together form spiro cyclopropyl, L¹ is - N(CH₃)C(O)- or -N(CH₃)C(O)CH₂N(CH₃)- and B is -CH(R⁹)-, wherein R⁹ is isopropyl or cyclopentyl, then W is not 3-cyclopropylaziridin-2-yl, 3-phenyltetrahydrofuran-2-yl, optionally substituted aziridin-2-yl or optionally substituted tetrahydrofuran-2-yl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, two R^{L1} substituents taken together form spiro cyclopropyl, L¹ is - N(CH₃)C(O)- or -N(CH₃)C(O)CH₂N(CH₃)- and B is -CH(R⁹)-, wherein R⁹ is C₁₋₆ alkyl or 3- to 6-membered cycloalkyl, then W is not 3-cyclopropylaziridin-2-yl, 3-phenyltetrahydrofuran-2-yl, optionally substituted aziridin-2-yl or optionally substituted tetrahydrofuran-2-yl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)- and B is -CH(R⁹)-, wherein R⁹ is isopropyl or cyclopentyl, then W is not 3-cyclopropylaziridin-2-yl, 3-phenyltetrahydrofuran-2-yl, optionally substituted aziridin-2-yl or optionally substituted tetrahydrofuran-2-yl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)- and B is -CH(R⁹)-, wherein R⁹ is C₁₋₆ alkyl or 3- to 6-membered cycloalkyl, then W is not 3-cyclopropylaziridin-2-yl, 3-phenyltetrahydrofuran-2-yl, optionally substituted aziridin-2-yl or optionally substituted tetrahydrofuran-2-yl.

In some embodiments of compounds of Formula IIa or any subgeneric formula of Formula Ila, or a pharmaceutically acceptable salt or stereoisomer thereof, when A is 1-hydroxy-benzene-3,5-diyl or optionally substituted phenylene, two R^{L1} substituents taken together form optionally substituted spiro cyclopropyl, L¹ is -N(CH₃)C(O)- or -N(CH₃)C(O)CH₂N(CH₃)-, R¹⁴ is absent and B is -CH(R⁹)-, wherein R⁹ is isopropyl or cyclopentyl, then R² is not ethyl or optionally substituted C₁₋₆ alkyl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)-, R¹⁴ is absent and B is -CH(R⁹)-, wherein R⁹ is isopropyl or cyclopentyl, then R² is not ethyl or optionally substituted C₁₋₆ alkyl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene and two R^{L1} substituents taken together form optionally substituted spiro cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, R^{L1} and R^{L2} taken together form optionally substituted fused cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene and two R^{L1} substituents taken together form optionally substituted spiro cyclopropyl, then R² and R¹⁴ combine with the atom to which they are attached to form an optionally substituted 3- to 8-membered cycloalkyl or optionally substituted 3- to 14-membered heterocycloalkyl.

In some embodiments of compounds of Formula IIa or any subgeneric formula of Formula IIa, or a pharmaceutically acceptable salt or stereoisomer thereof, when A is 1-hydroxy-benzene-3,5-diyl or optionally substituted phenylene, R^{L1} and R^{L2} taken together form optionally substituted fused cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl.

In some embodiments, the compound, or pharmaceutically acceptable salt thereof, has the structure of Formula Ilb: 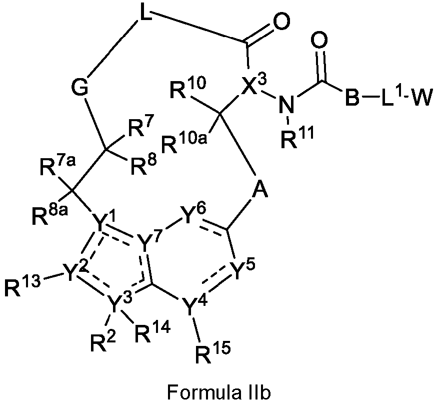
wherein the dotted lines represent zero, one, two, three, or four non-adjacent double bonds;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-**C**H(R⁶)-where C is bound to -C(R⁷R⁸)-, optionally substituted C₁-C₄ heteroalkylene, or 3- to 8-membered heteroarylene;
L¹ is a linker;
W is a cross-linking group comprising a carbodiimide, an oxazoline, a thiazoline, a chloroethyl urea, a chloroethyl thiourea, a chloroethyl carbamate, a chloroethyl thiocarbamate, an aziridine, a trifluoromethyl ketone, a boronic acid, a boronic ester, an N-ethoxycarbonyl-2-ethoxy-1,2-dihydroquinoline (EEDQ), an iso-EEDQ or other EEDQ derivative, an epoxide, an oxazolium, or a glycal;
L has the structure of Formula VIIa or Vllb: 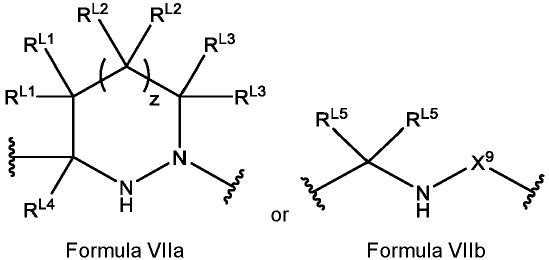
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 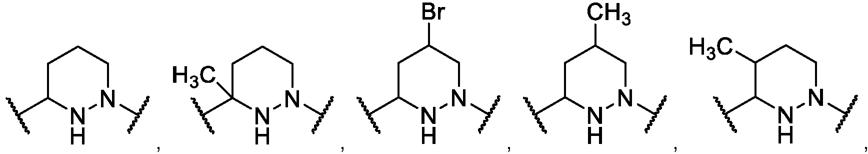 or 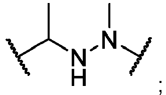
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R' is, independently, hydrogen or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ heteroalkyl, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halogen, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halogen, hydroxy, optionally substituted C₁-C₃ heteroalkyl, or optionally substituted C₁-C₃ alkyl;
R^{10a} is hydrogen or halogen;
R¹¹ is hydrogen or optionally substituted C₁-C₃ alkyl; and
R²¹ is hydrogen or optionally substituted C₁-C₃ alkyl.

In some embodiments of Formula IIb, W comprises a carbodiimide. In some embodiments, W has the structure of Formula Ilb-Illa: 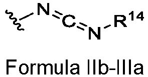 wherein R¹⁴ is optionally substituted C₁-C₆ alkyl, optionally substituted C₁-C₆ heteroalkyl, optionally substituted 3 to 8-membered cycloalkyl, optionally substituted 6 to 10-membered aryl, optionally substituted 3 to 14-membered heterocycloalkyl, or optionally substituted 5 to 10-membered heteroaryl. In some embodiments if Formula IIb, W has the structure:

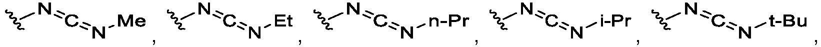

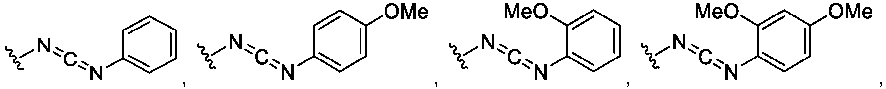

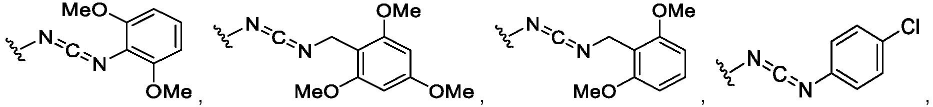

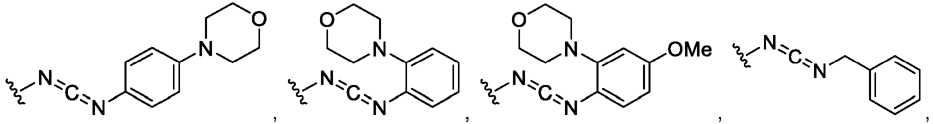

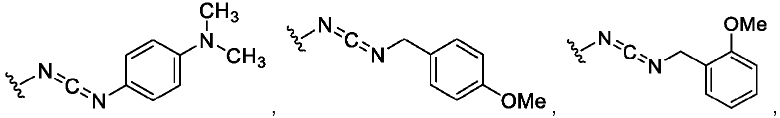

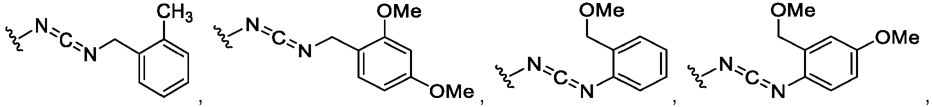

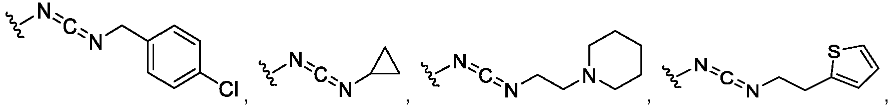

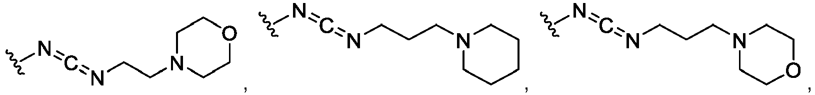

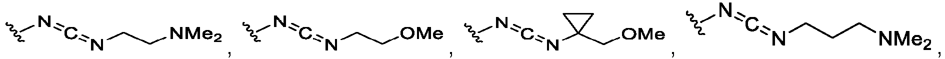

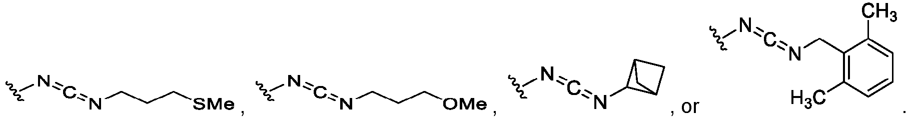

In some embodiments of Formula IIb, W comprises an oxazoline or thiazoline. In some embodiments, W has the structure of Formula Ilb-Illb: 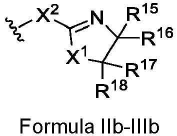
wherein X¹ is O or S;
X² is absent or NR¹⁹;
R¹⁵, R¹⁶, R¹⁷, and R¹⁸ are, independently, hydrogen or optionally substituted C₁-C₆ alkyl; and
R¹⁹ is hydrogen, C(O)(optionally substituted C₁-C₆ alkyl), optionally substituted C₁-C₆ alkyl, optionally substituted 6 to 10-membered aryl, optionally substituted 3 to 14-membered heterocycloalkyl, or optionally substituted 5 to 10-membered heteroaryl. In some embodiments, W is

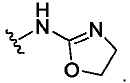

In some embodiments of Formula IIb, W comprises a chloroethyl urea, a chloroethyl thiourea, a chloroethyl carbamate, or a chloroethyl thiocarbamate. In some embodiments, W has the structure of Formula IIb-IIIc: 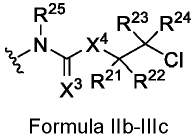
wherein X³ is O or S;
X⁴ is O, S, NR²⁶;
R²¹, R²², R²³, R²⁴, and R²⁶ are, independently, hydrogen or optionally substituted C₁-C₆ alkyl; and
R²⁵ is hydrogen, optionally substituted C₁-C₆ alkyl, optionally substituted 6 to 10-membered aryl, optionally substituted 3 to 14-membered heterocycloalkyl, or optionally substituted 5 to 10-membered heteroaryl. In some embodiments, W is

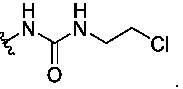

In some embodiments, W comprises an aziridine. In some embodiments, W has the structure of Formula Illd1, Formula Illd2, Formula Illd3, or Formula Illd4: 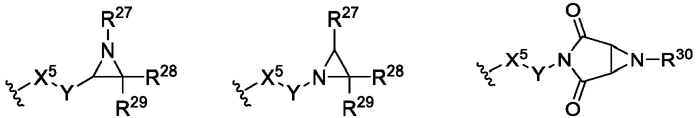 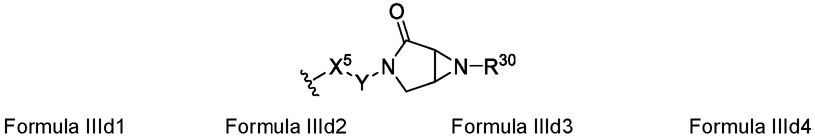
wherein X⁵ is absent or NR³⁰;
Y is absent or C(O), C(S), S(O), SO₂, or optionally substituted C₁-C₃ alkylene;
R²⁷ is hydrogen, -C(O)R³², -C(O)OR³², -SOR³³, -SO₂R³³, optionally substituted C₁-C₆ alkyl, optionally substituted 6 to 10-membered aryl, optionally substituted 3 to 14-membered heterocycloalkyl, or optionally substituted 5 to 10-membered heteroaryl;
R²⁸ and R²⁹ are, independently, hydrogen, CN, C(O)R³¹, CO₂R³¹, C(O)R³¹R³¹ optionally substituted C₁-C₆ alkyl, optionally substituted 3 to 10-membered cycloalkyl, optionally substituted 6 to 10-membered aryl, optionally substituted 3 to 14-membered heterocycloalkyl, or optionally substituted 5 to 10-membered heteroaryl;
each R³¹ is, independently, hydrogen, optionally substituted C₁-C₆ alkyl, optionally substituted 6 to 10-membered aryl, optionally substituted 3 to 14-membered heterocycloalkyl, or optionally substituted 5 to 10-membered heteroaryl;
R³⁰ is hydrogen or optionally substituted C₁-C₆ alkyl; and
R³² and R³³ are, independently, hydrogen, optionally substituted C₁-C₆ alkyl, optionally substituted 6 to 10-membered aryl, optionally substituted 3 to 14-membered heterocycloalkyl, or optionally substituted 5 to 10-membered heteroaryl. In some embodiments, W is:

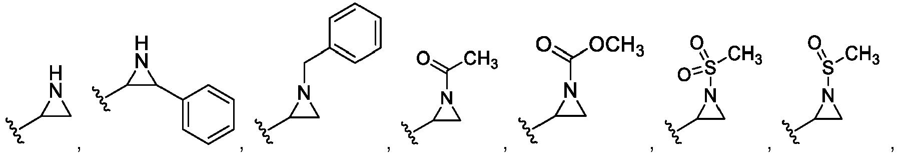

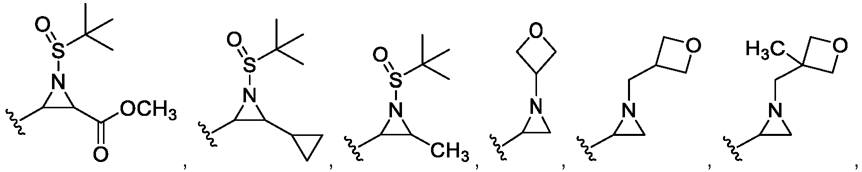

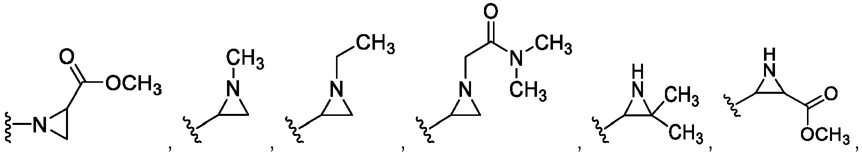

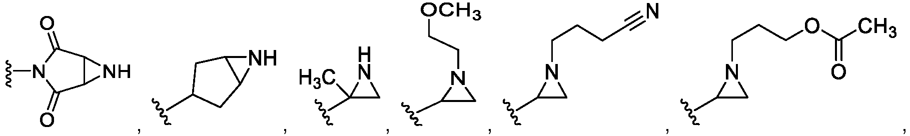

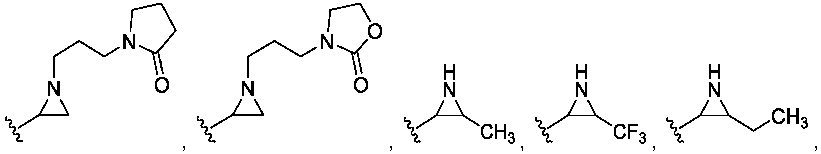

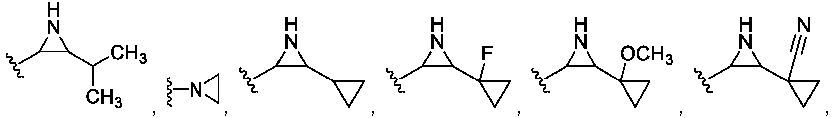

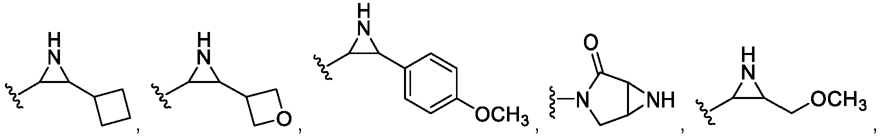

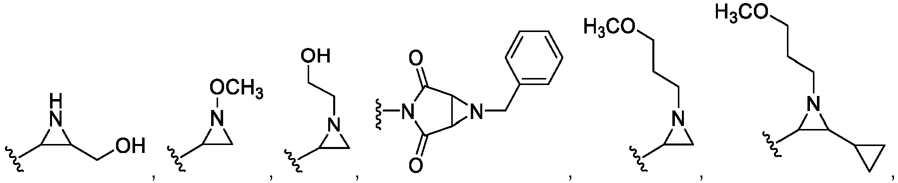

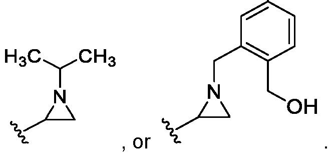

In some embodiments, W comprises an epoxide. In some embodiments, W is

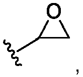

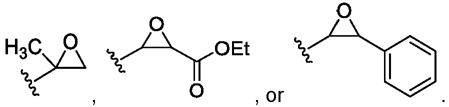

In some embodiments, W is a cross-linking group bound to an organic moiety that is a Ras binding moiety, i.e., RBM-W, wherein upon contact of an RBM-W compound with a Ras protein, the RBM-W binds to the Ras protein to form a conjugate. For example, the W moiety of an RBM-W compound may bind, e.g., cross-link, with an amino acid of the Ras protein to form the conjugate. In some embodiments, the Ras binding moiety is a K-Ras binding moiety. In some embodiments, the K-Ras binding moiety binds to a residue of a K-Ras Switch-II binding pocket of the K-Ras protein. In some embodiments, the Ras binding moiety is an H-Ras binding moiety that binds to a residue of an H-Ras Switch-II binding pocket of an H-Ras protein. In some embodiments, the Ras binding moiety is an N-Ras binding moiety that binds to a residue of an N-Ras Switch-II binding pocket of an N-Ras protein. The W of an RBM-W compound may comprise any W described herein. The Ras binding moiety typically has a molecular weight of under 1200 Da. See, e.g., see, e.g., Johnson et al., 292:12981-12993 (2017) for a description of Ras protein domains, incorporated herein by reference.

In some embodiments of compounds of formula IIb or any subgeneric formula of formula IIb, or a pharmaceutically acceptable salt or stereoisomer thereof, the compound is not: 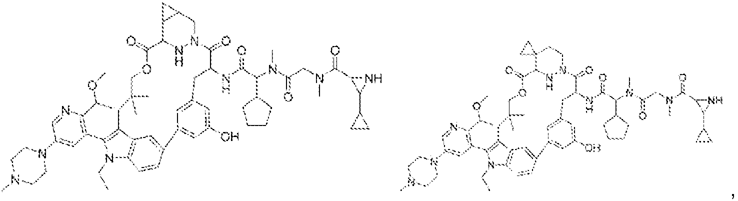 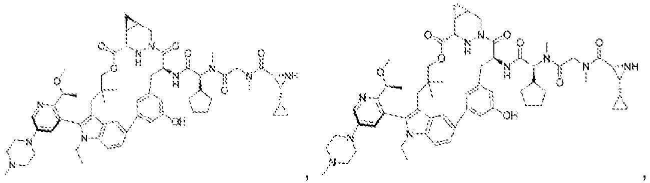 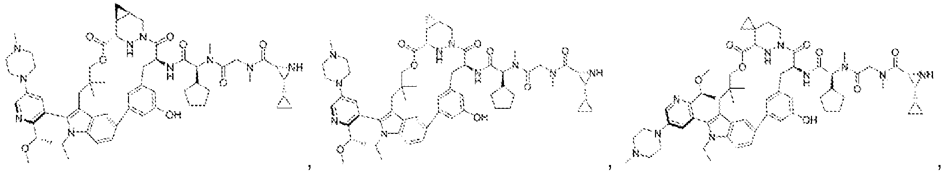 or

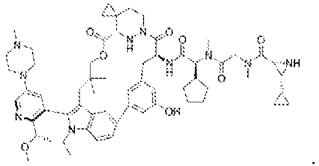

In some embodiments of compounds of formula IIb or any subgeneric formula of formula IIb, or a pharmaceutically acceptable salt or stereoisomer thereof, when A is 1-hydroxy-benzene-3,5-diyl or optionally substituted phenylene, W is 3-cyclopropylaziridin-2-yl or optionally substituted aziridine and two R^{L1} substituents taken together form spiro cyclopropyl, then R² is not ethyl or C₁₋₆ alkyl.

In some embodiments of compounds of formula IIb or any subgeneric formula of formula IIb, or a pharmaceutically acceptable salt or stereoisomer thereof, when A is 1-hydroxy-benzene-3,5-diyl or optionally substituted phenylene, W is 3-cyclopropylaziridin-2-yl or optionally substituted aziridine and R^{L1} and R^{L2} taken together form fused cyclopropyl, then R² is not ethyl or C₁₋₆ alkyl.

In some embodiments of compounds of formula IIb or any subgeneric formula of formula IIb, or a pharmaceutically acceptable salt or stereoisomer thereof, when A is 1-hydroxy-benzene-3,5-diyl or optionally substituted phenylene and two R^{L1} substituents taken together form spiro cyclopropyl, then W is not 3-cyclopropylaziridin-2-yl or optionally substituted aziridine.

In some embodiments of compounds of formula IIb or any subgeneric formula of formula IIb, or a pharmaceutically acceptable salt or stereoisomer thereof, when A is 1-hydroxy-benzene-3,5-diyl or optionally substituted phenylene and R^{L1} and R^{L2} taken together form fused cyclopropyl, then W is not 3-cyclopropylaziridin-2-yl or optionally substituted aziridine.

In some embodiments of compounds of formula IIb or any subgeneric formula of formula IIb, or a pharmaceutically acceptable salt or stereoisomer thereof, when A is 1-hydroxy-benzene-3,5-diyl or optionally substituted phenylene, W is 3-cyclopropylaziridin-2-yl or optionally substituted aziridine and two R^{L1} substituents taken together form spiro cyclopropyl, then L¹ is not - N(CH₃)C(O)CH₂N(CH₃)C(O)-.

In some embodiments of compounds of formula IIb or any subgeneric formula of formula IIb, or a pharmaceutically acceptable salt or stereoisomer thereof, when A is 1-hydroxy-benzene-3,5-diyl or optionally substituted phenylene, W is 3-cyclopropylaziridin-2-yl or optionally substituted aziridine and R^{L1} and R^{L2} taken together form fused cyclopropyl, then L¹ is not -N(CH₃)C(O)CH₂N(CH₃)C(O)-.

In some embodiments, the compound, or pharmaceutically acceptable salt thereof, has the structure of Formula IIc: 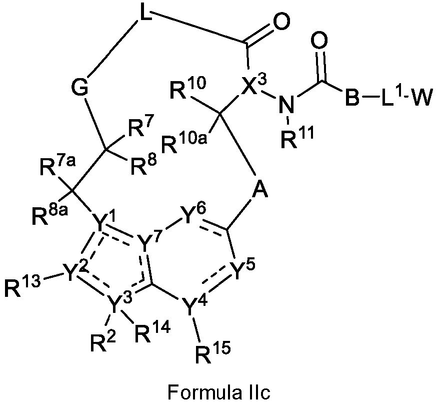
wherein the dotted lines represent zero, one, two, three, or four non-adjacent double bonds;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
L¹ is a linker;
W is a cross-linking group comprising a vinyl ketone, a vinyl sulfone, an ynone, a haloacetyl, or an alkynyl sulfone;
L has the structure of Formula VIIa or VIIb: 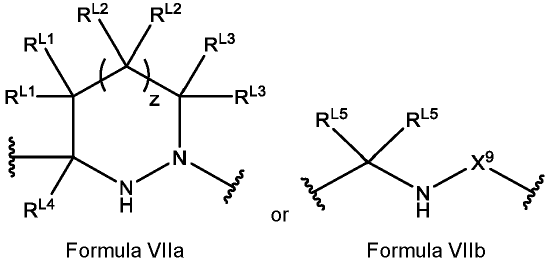
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 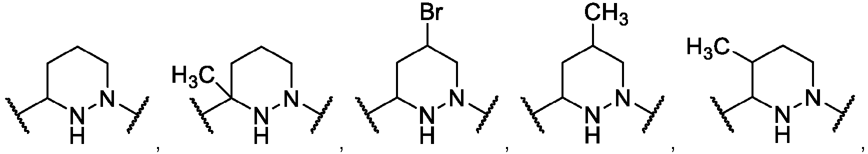 or 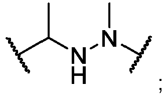
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, hydrogen or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ heteroalkyl, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halogen, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halogen, hydroxy, optionally substituted C₁-C₃ heteroalkyl, or optionally substituted C₁-C₃ alkyl;
R^{10a} is hydrogen or halogen;
R¹¹ is hydrogen or optionally substituted C₁-C₃ alkyl; and
R²¹ is hydrogen or optionally substituted C₁-C₃ alkyl.

In some embodiments of Formula IIc, W has the structure of Formula IIc-IIIa: 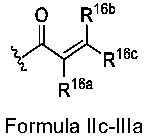
wherein R^{16a}, R^{16b}, and R^{16c} are, independently, hydrogen, -CN, halogen, or -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl. In some embodiments, W is: 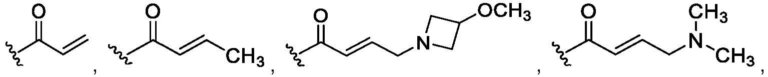 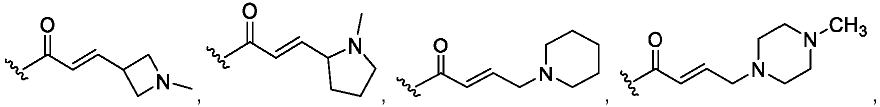 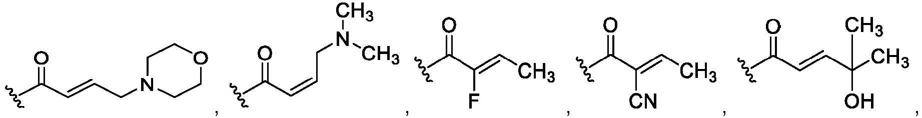 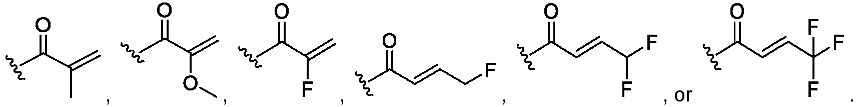 In some embodiments, W is a cross-linking group comprising an ynone. In some embodiments, W has the structure of Formula IIc-IIIb: 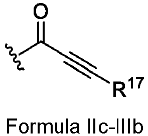
wherein R¹⁷ is hydrogen, -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl, or a 4 to 7-membered saturated heterocycloalkyl. In some embodiments, W is: 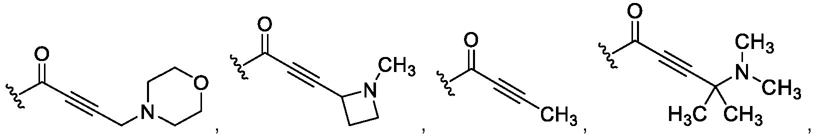 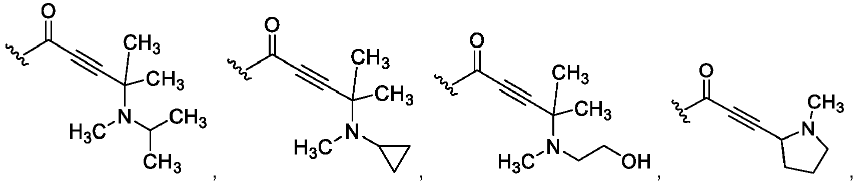 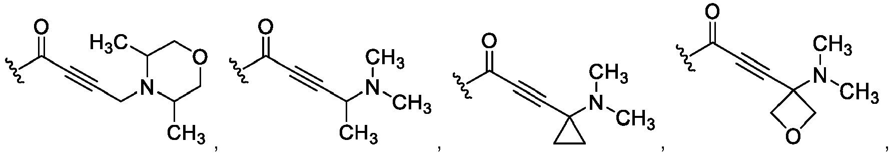 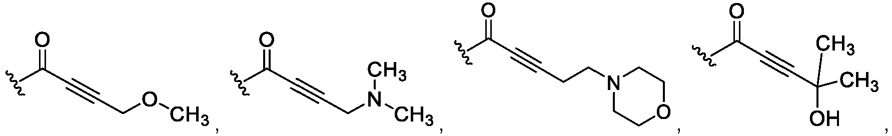 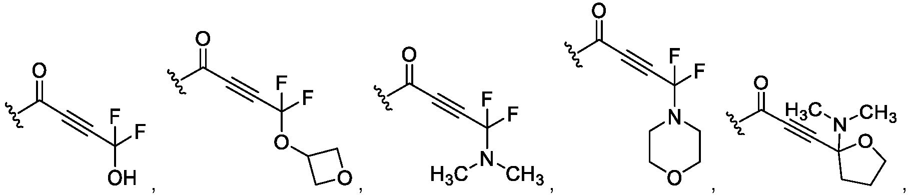 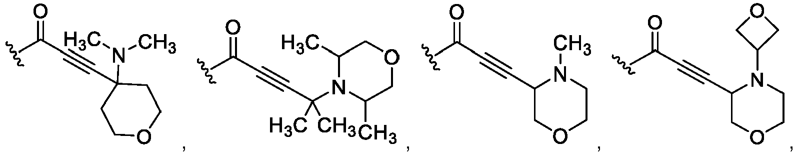 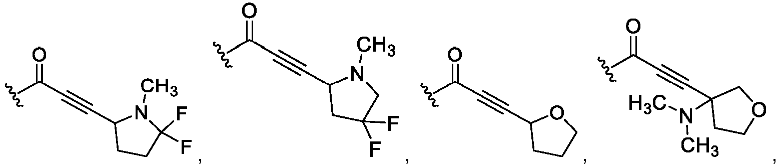 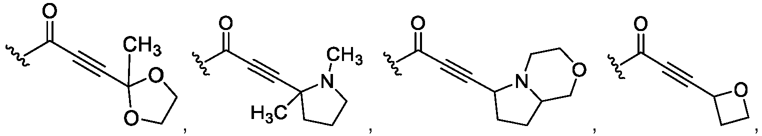 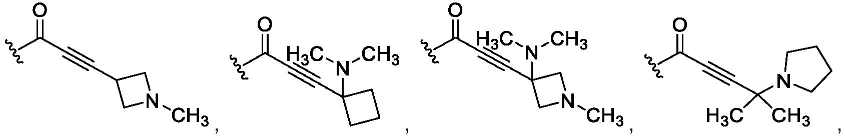 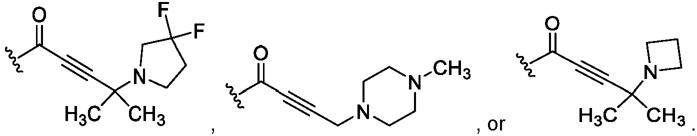 In some embodiments, W is

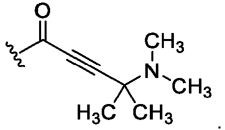

In some embodiments, W is a cross-linking group comprising a vinyl sulfone. In some embodiments, W has the structure of Formula IIc-IIIc: 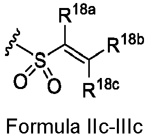 wherein R^{18a}, R^{18b}, and R^{18c} are, independently, hydrogen, -CN, or -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl. In some embodiments, W is:

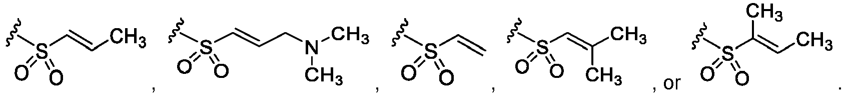

In some embodiments, W is a cross-linking group comprising an alkynyl sulfone. In some embodiments, W has the structure of Formula IIc-IIId: 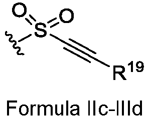
wherein R¹⁹ is hydrogen, -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl, or a 4 to 7-membered saturated heterocycloalkyl. In some embodiments, W is: 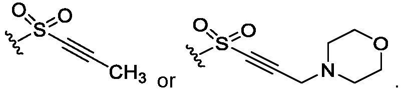 In some embodiments, W has the structure of Formula IIc-IIIe: 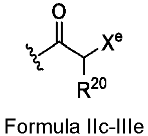
wherein X^{e} is a halogen; and
R²⁰ is hydrogen, -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl. In some embodiments, W is haloacetyl. In some embodiments, W is not haloacetyl.

In some embodiments, the compound of Formula IIa, IIb, or IIc, or pharmaceutically acceptable salt thereof, has the structure of Formula IId: 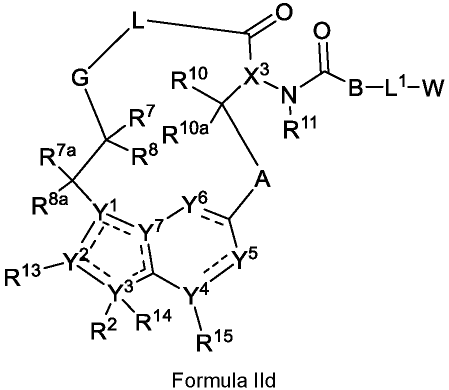
wherein the dotted lines represent zero, one, two, three, or four non-adjacent double bonds;
A is -N(H or CH₃)C(O)-(CH₂)- where the amino nitrogen is bound to the carbon atom of - CH(R¹⁰)-, optionally substituted 3- to 6-membered cycloalkylene, optionally substituted 3- to 6-membered heterocycloalkylene, optionally substituted 6-membered arylene, or optionally substituted 5- to 10-membered heteroarylene;
B is -CH(R⁹)- or >C=CR⁹R^{9'} where the carbon is bound to the carbonyl carbon of -N(R¹¹)C(O)- , optionally substituted 3- to 6-membered cycloalkylene, optionally substituted 3- to 6-membered heterocycloalkylene, optionally substituted 6-membered arylene, or 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
L¹ is absent or a linker;
W is as defined in Formula IIa, IIb, or IIc;
L is as defined in Formula IIa, IIb, or IIc;
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, H or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
R¹³ and R² combine with the atoms to which they are attached to form an optionally substituted 3- to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halo, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl, or
R⁹ and L combine with the atoms to which they are attached to form an optionally substituted 3- to 14-membered heterocycloalkyl;
R^{9'} is hydrogen or optionally substituted C₁-C₆ alkyl;
R¹⁰ is hydrogen, halo, hydroxy, C₁-C₃ alkoxy, or C₁-C₃ alkyl;
R^{10a} is hydrogen or halo; and
R¹¹ is hydrogen or C₁-C₃ alkyl.

In some embodiments, the Compound of Formula IIa, Ilb, or IIc, or pharmaceutically acceptable salt thereof, has the structure of Formula Ile: 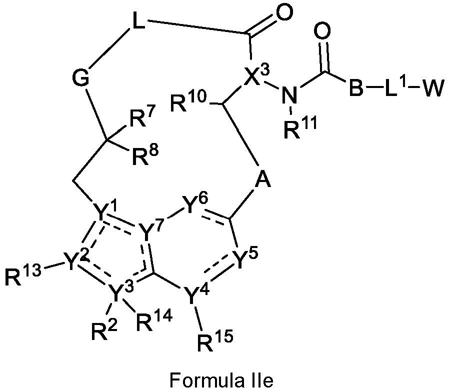
wherein the dotted lines represent zero, one, two, three, or four non-adjacent double bonds;
A is -N(H or CH₃)C(O)-(CH₂)- where the amino nitrogen is bound to the carbon atom of - CH(R¹⁰)-, optionally substituted 3 to 6-membered cycloalkylene, optionally substituted 3- to 6-membered heterocycloalkylene, optionally substituted 6-membered arylene, or optionally substituted 5- to 6-membered heteroarylene;
B is -CH(R⁹)- where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
L¹ is absent or a linker;
W is as defined in Formula IIa, Ilb, or llc;
L is as defined in Formula IIa, IIb, or IIc;
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, H or optionally substituted C₁-C₄ alkyl;
Y¹ is C, CH, or N;
Y², Y³, Y⁴, and Y⁷ are, independently, C or N;
Y⁵ and Y⁶ are, independently, CH or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3-- to 6-membered heterocycloalkyl, optionally substituted 6 to 10-membered aryl, or optionally substituted 5- to 10-membered heteroaryl;
R² is hydrogen, optionally substituted C₁-C₆ alkyl, optionally substituted C₂-C₆ alkenyl, optionally substituted 3 to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl;
R¹⁰ is hydrogen, hydroxy, C₁-C₃ alkoxy, or C₁-C₃ alkyl; and
R¹¹ is hydrogen or C₁-C₃ alkyl.

In some embodiments of compounds of the present invention, G is optionally substituted C₁-C₄ heteroalkylene.

In some embodiments, the Compound of Formula IIa, Ilb, or IIc, or pharmaceutically acceptable salt thereof, has the structure of Formula Ilf: 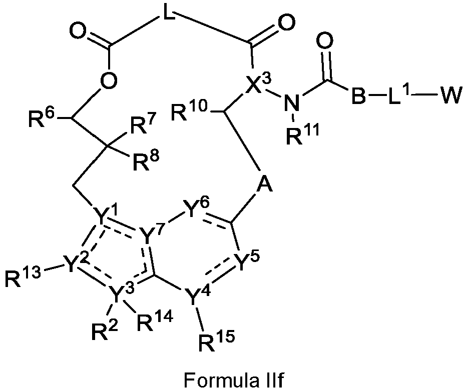
wherein the dotted lines represent zero, one, two, three, or four non-adjacent double bonds;
A is -N(H or CH₃)C(O)-(CH₂)- where the amino nitrogen is bound to the carbon atom of - CH(R¹⁰)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
B is -CH(R⁹)- where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
X² is O or NH;
X³ is N or CH;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, H or optionally substituted C₁-C₄ alkyl;
Y¹ is C, CH, or N;
Y², Y³, Y⁴, and Y⁷ are, independently, C or N;
Y⁵ and Y⁶ are, independently, CH or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl;
R² is hydrogen, optionally substituted C₁-C₆ alkyl, optionally substituted C₂-C₆ alkenyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl;
R¹⁰ is hydrogen, hydroxy, C₁-C₃ alkoxy, or C₁-C₃ alkyl; and
R¹¹ is hydrogen or C₁-C₃ alkyl.

In some embodiments of compounds of the present invention, X² is NH. In some embodiments, X³ is CH. In some embodiments, R¹¹ is hydrogen. In some embodiments, R¹¹ is C₁-C₃ alkyl. In some embodiments, R¹¹ is methyl.

In some embodiments, the compound of Formula IIa, IIb, or IIc, or pharmaceutically acceptable salt thereof, has the structure of Formula IIg: 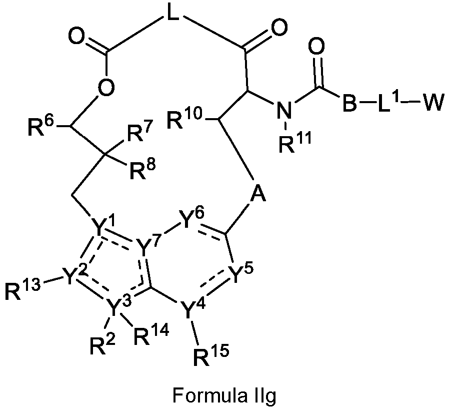
wherein the dotted lines represent zero, one, two, three, or four non-adjacent double bonds;
A is -N(H or CH₃)C(O)-(CH₂)- where the amino nitrogen is bound to the carbon atom of - CH(R¹⁰)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, H or optionally substituted C₁-C₄ alkyl;
Y¹ is C, CH, or N;
Y², Y³, Y⁴, and Y⁷ are, independently, C or N;
Y⁵ and Y⁶ are, independently, CH or N;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl;
R² is hydrogen, optionally substituted C₁-C₆ alkyl, optionally substituted C₂-C₆ alkenyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; and
R¹⁰ is hydrogen, hydroxy, C₁-C₃ alkoxy, or C₁-C₃ alkyl.
In some embodiments of a compound of the present invention, X¹ is optionally substituted C₁-C₂ alkylene. In some embodiments, X¹ is methylene. In some embodiments, X¹ is methylene substituted with a C₁-C₆ alkyl group or a halogen. In some embodiments, X¹ is -CH(Br)-. In some embodiments, X¹ is -CH(CH₃)-. In some embodiments, R⁵ is hydrogen. In some embodiments, R⁵ is C₁-C₄ alkyl optionally substituted with halogen. In some embodiments, R⁵ is methyl. In some embodiments, Y⁴ is C. In some embodiments, R⁴ is hydrogen. In some embodiments, Y⁵ is CH. In some embodiments, Y⁶ is CH. In some embodiments, Y¹ is C. In some embodiments, Y² is C. In some embodiments, Y³ is N. In some embodiments, R³ is absent. In some embodiments, Y⁷ is C.

In some embodiments, the compound of Formula IIa, IIb, or IIc, or pharmaceutically acceptable salt thereof, has the structure of Formula IIh: 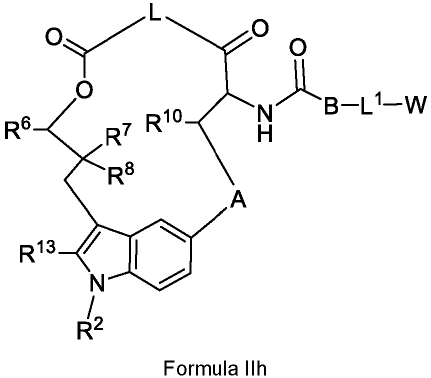
wherein A is -N(H or CH₃)C(O)-(CH₂)- where the amino nitrogen is bound to the carbon atom of -CH(R¹⁰)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula Ila, IIb, or IIc;
L is as defined in Formula IIa, IIb, or IIc;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl;
R² is hydrogen, optionally substituted C₁-C₆ alkyl, optionally substituted C₂-C₆ alkenyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; and
R¹⁰ is hydrogen, hydroxy, C₁-C₃ alkoxy, or C₁-C₃ alkyl.

In some embodiments of a compound of the present invention, R⁶ is hydrogen. In some embodiments, R² is hydrogen, cyano, optionally substituted C₁-C₆ alkyl, optionally substituted 3- to 6-membered cycloalkyl, or optionally substituted 3- to 6-membered heterocycloalkyl. In some embodiments, R² is optionally substituted C₁-C₆ alkyl. In some embodiments, R₂ is fluoroalkyl. In some embodiments, R² is ethyl. In some embodiments, R₂ is -CH₂CF₃. In some embodiments, R₂ is C₂-C₆ alkynyl. In some embodiments, R₂ is -CHC=CH. In some embodiments, R₂ is -CH₂C≡CCH₃. In some embodiments, R⁷ is optionally substituted C₁-C₃ alkyl. In some embodiments, R⁷ is C₁-C₃ alkyl. In some embodiments, R⁸ is optionally substituted C₁-C₃ alkyl. In some embodiments, R⁸ is C₁-C₃ alkyl.

In some embodiments, the compound of Formula IIa, IIb, or IIc has the structure of Formula Ili, or a pharmaceutically acceptable salt thereof: 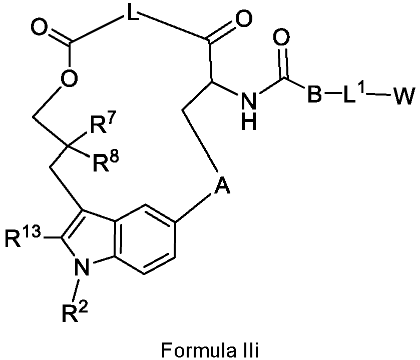
wherein A is -N(H or CH₃)C(O)-(CH₂)- where the amino nitrogen is bound to the carbon atom of -CH(R¹⁰)-, optionally substituted 3- to 6-membered cycloalkylene, optionally substituted 3- to 6-membered heterocycloalkylene, optionally substituted 6-membered arylene, or optionally substituted 5 to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl;
R² is C₁-C₆ alkyl or 3 to 6-membered cycloalkyl;
R⁷ is C₁-C₃ alkyl;
R⁸ is C₁-C₃ alkyl; and
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl.

In some embodiments of a compound of the present invention, R¹³ is optionally substituted 6-to 10-membered aryl, optionally substituted 3- to 6-membered cycloalkenyl, or optionally substituted 5- to 10-membered heteroaryl. In some embodiments, R¹³ is optionally substituted 6-membered aryl, optionally substituted 6-membered cycloalkenyl, or optionally substituted 6-membered heteroaryl.

In some embodiments of a compound of the present invention, R¹³ is 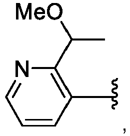 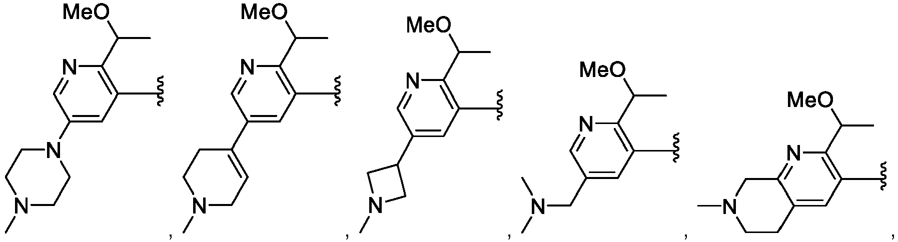 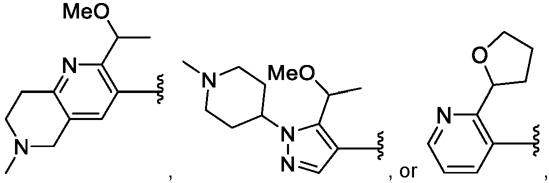 or a stereoisomer (e.g., atropisomer) thereof. In some embodiments of a compound of the present invention, R¹³ is 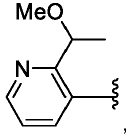 or a stereoisomer (e.g., atropisomer) thereof. In some embodiments of a compound of the present invention, R¹³ is 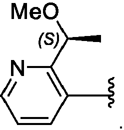 In some embodiments, R¹³ is 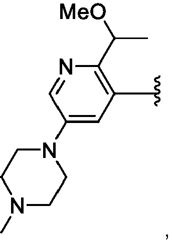 or a stereoisomer thereof. In some embodiments, R¹³ is

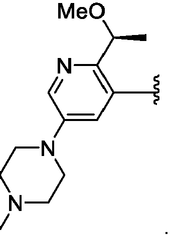

In some embodiments, the compound of Formula IIa, IIb, or IIc has the structure of Formula IIj, or a pharmaceutically acceptable salt thereof: 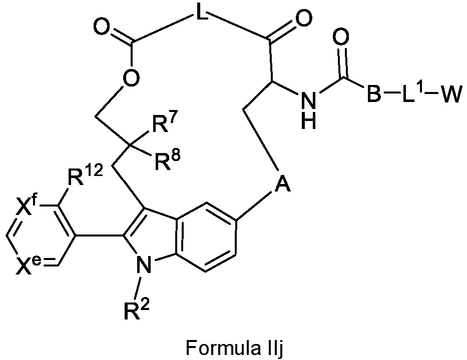
wherein A is -N(H or CH₃)C(O)-(CH₂)- where the amino nitrogen is bound to the carbon atom of -CH(R¹⁰)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
R² is C₁-C₆ alkyl, C₁-C₆ fluoroalkyl, or 3- to 6-membered cycloalkyl;
R⁷ is C₁-C₃ alkyl;
R⁸ is C₁-C₃ alkyl; and
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl X^{e} and X^{f} are, independently, N, CH or CR¹⁷; and

R¹² is optionally substituted C₁-C₆ alkyl, optionally substituted C₁-C₆ heteroalkyl, or optionally substituted 3- to 6-membered heterocycloalkylene.

R¹⁷ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl.

In some embodiments of a compound of the present invention, X^{e} is N and X^{f} is CH. In some embodiments, X^{e} is CH and X^{f} is N.

In some embodiments of compounds of the present invention, R¹² is optionally substituted C₁-C₆ heteroalkyl. In some embodiments, R¹² is 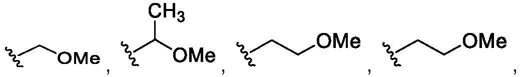 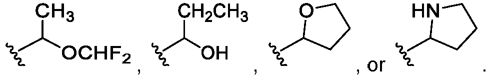 In some embodiments of a compound of the present invention, R¹² is optionally substituted C₁-C₆ heteroalkyl. In some embodiments, R¹² is

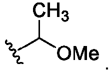

In some embodiments, the compound of Formula IIa, IIb, or IIc has the structure of Formula Ilk, or a pharmaceutically acceptable salt thereof: 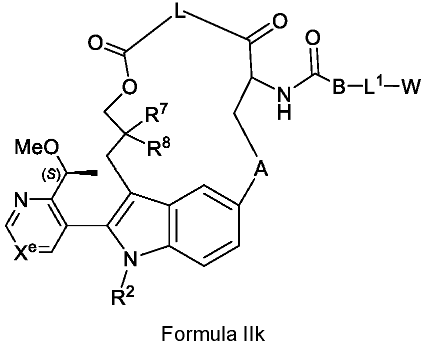
wherein A is optionally substituted 3- to 6-membered cycloalkylene, optionally substituted 3-to 6-membered heterocycloalkylene, optionally substituted 6-membered arylene, or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
R² is C₁-C₆ alkyl or 3- to 6-membered cycloalkyl;
R⁷ is C₁-C₃ alkyl;
R⁸ is C₁-C₃ alkyl;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl;
X^{e} is CH, or CR¹⁷; and
R¹⁷ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl.

In some embodiments, the compound of Formula IIa, IIb, or IIc has the structure of Formula Ilm, or a pharmaceutically acceptable salt thereof: 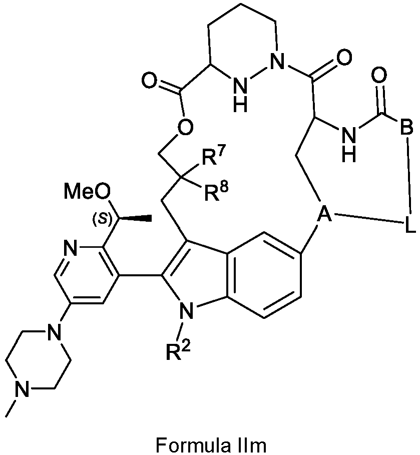
wherein A is optionally substituted 3- to 6-membered cycloalkylene, optionally substituted 3-to 6-membered heterocycloalkylene, optionally substituted 6-membered arylene, or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
R² is C₁-C₆ alkyl or 3- to 6-membered cycloalkyl;
R⁷ is C₁-C₃ alkyl;
R⁸ is C₁-C₃ alkyl; and
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl.

In some embodiments, the compound of Formula IIa, IIb, or IIc has the structure of Formula II-VI, or a pharmaceutically acceptable salt thereof: 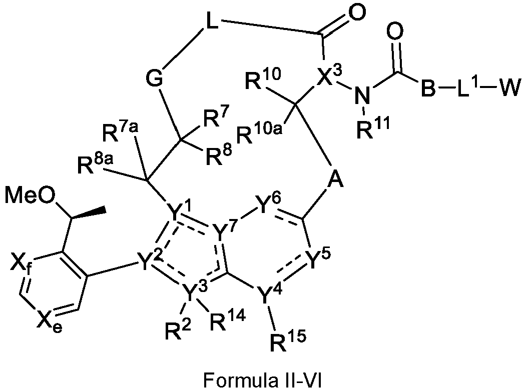 wherein the dotted lines represent zero, one, two, three, or four non-adjacent double bonds;
A is -N(H or CH₃)C(O)-(CH₂)- where the amino nitrogen is bound to the carbon atom of - CH(R¹⁰)-, optionally substituted 3- to 6-membered cycloalkylene, optionally substituted 3- to 6-membered heterocycloalkylene, optionally substituted 6-membered arylene (e.g., phenyl or phenol), or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
W is as defined in Formula IIa, IIb, or IIc;
L is as defined in Formula IIa, IIb, or IIc;
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂; each R^{'} is, independently, H or optionally substituted C₁-C₄ alkyl;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halo, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is H, F, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3- to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halo, hydroxy, C₁-C₃ alkoxy, or C₁-C₃ alkyl;
R^{10a} is hydrogen or halo;
R¹¹ is hydrogen or C₁-C₃ alkyl;
R²¹ is hydrogen or C₁-C₃ alkyl (e.g., methyl); and
X^{e} and X^{f} are, independently, N or CH.

In some embodiments, the compound of Formula IIa, Ilb, or IIc has the structure of Formula II-VIa, or a pharmaceutically acceptable salt thereof: 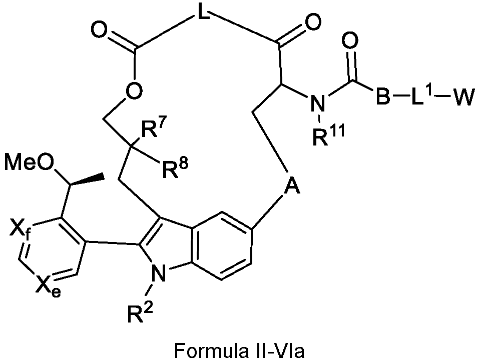
wherein A optionally substituted 3- to 6-membered cycloalkylene, optionally substituted 3- to 6-membered heterocycloalkylene, optionally substituted 6-membered arylene (e.g., phenyl or phenol), or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
L¹ is absent or a linker;
W is as defined in Formula IIa, IIb, or IIc;
L is as defined in Formula IIa, IIb, or IIc;
X¹ is optionally substituted C₁-C₂ alkylene, NR, O, or S(O)_{q};
X² is O or NH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
each R^{'} is, independently, H or optionally substituted C₁-C₄ alkyl;
R² is C₁-C₆ alkyl, C₁-C₆ fluoroalkyl, or 3- to 6-membered cycloalkyl;
R⁷ is C₁-C₃ alkyl;
R⁸ is C₁-C₃ alkyl; and
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl;
X^{e} and X^{f} are, independently, N or CH;
R¹¹ is hydrogen or C₁-C₃ alkyl; and
R²¹ is hydrogen or C₁-C₃ alkyl.

In some embodiments of a compound of the present invention, X^{e} is N and X^{f} is CH. In some embodiments, X^{e} is CH and X^{f} is N.

In some embodiments, the compound of Formula IIa, Ilb, or IIc has the structure of Formula II-Vlb, or a pharmaceutically acceptable salt thereof: 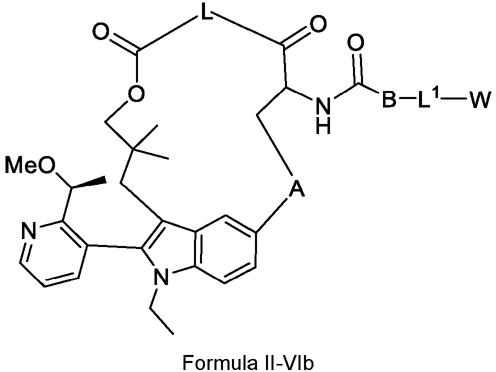
wherein A optionally substituted 3- to 6-membered cycloalkylene, optionally substituted 3- to 6-membered heterocycloalkylene, optionally substituted 6-membered arylene (e.g., phenyl or phenol), or optionally substituted 5- to 6-membered heteroarylene;
B is -CH(R⁹)- where the carbon is bound to the carbonyl carbon of -NHC(O)-, optionally substituted 3- to 6-membered cycloalkylene, optionally substituted 3- to 6-membered heterocycloalkylene, optionally substituted 6-membered arylene, or 5- to 6-membered heteroarylene;
R⁹ is optionally substituted C₁-C₆ alkyl, optionally substituted C₁-C₆ heteroalkyl, optionally substituted 3- to 6-membered cycloalkyl, or optionally substituted 3- to 7-membered heterocycloalkyl;
L¹ is absent or a linker; and
W is as defined in Formula IIa, IIb, or IIc;
L is as defined in Formula IIa, IIb, or IIc.

In some embodiments, the compound of Formula IIa, IIb, or IIc has the structure of Formula II-VIc, or a pharmaceutically acceptable salt thereof: 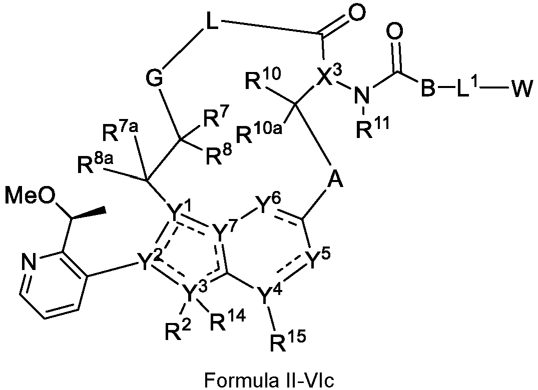 wherein the dotted lines represent zero, one, two, three, or four non-adjacent double bonds;
A is -N(H or CH₃)C(O)-(CH₂)- where the amino nitrogen is bound to the carbon atom of - CH(R¹⁰)-, optionally substituted 3- to 6-membered cycloalkylene, optionally substituted 3- to 6-membered heterocycloalkylene, optionally substituted 6-membered arylene (e.g., phenyl or phenol), or optionally substituted 5- to 10-membered heteroarylene;
B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or 5- to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
L¹ is absent or a linker;
W is as defined in Formula IIa, IIb, or IIc;
L is as defined in Formula IIa, IIb, or IIc;
X¹ is optionally substituted C₁-C₂ alkylene, NR, O, or S(O)_{q};
X² is O or NH;
X³ is N or CH;
q is 0, 1, or 2;
R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂; each R is, independently, H or optionally substituted C₁-C₄ alkyl;
   Y¹ is C, CH, or N;
Y², Y³, Y⁴, and Y⁷ are, independently, C or N;
Y⁵ is CH, CH₂, or N;
Y⁶ is C(O), CH, CH₂, or N;
R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent, or
R² and R¹⁴ combine with the atom to which they are attached to form an optionally substituted 3- to 8-membered cycloalkyl or optionally substituted 3- to 14-membered heterocycloalkyl;
R¹⁵ is absent, hydrogen, halogen, cyano, or methyl optionally substituted with 1 to 3 halogens;
R⁵ is hydrogen, C₁-C₄ alkyl optionally substituted with halogen, cyano, hydroxy, or C₁-C₄ alkoxy, cyclopropyl, or cyclobutyl;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3- to 6-membered cycloalkyl or optionally substituted 3- to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
R^{7a} and R^{8a} are, independently, hydrogen, halo, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
R^{7'} and R^{8'} combine with the carbon atom to which they are attached to form optionally substituted 3- to 6-membered cycloalkyl or optionally substituted 3- to 7-membered heterocycloalkyl;
R⁹ is H, F, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
R⁹ and L combine with the atoms to which they are attached to form an optionally substituted 3 to 14-membered heterocycloalkyl;
R^{9'} is hydrogen or optionally substituted C₁-C₆ alkyl; or
R⁹ and R^{9'}, combined with the atoms to which they are attached, form a 3- to 6-membered cycloalkyl or a 3- to 6-membered heterocycloalkyl;
R¹⁰ is hydrogen, halo, hydroxy, C₁-C₃ alkoxy, or C₁-C₃ alkyl;
R^{10a} is hydrogen or halo;
R¹¹ is hydrogen or C₁-C₃ alkyl; and
R²¹ is hydrogen or C₁-C₃ alkyl (e.g., methyl).

In some embodiments, A is optionally substituted C₂-C₄ alkylene. In some embodiments, A is optionally substituted C₃ alkylene. In some embodiments, A is:

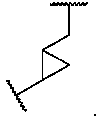

In some embodiments, A is optionally substituted C₂-C₄ alkenylene. In some embodiments, A is optionally substituted C₃ alkenylene. In some embodiments, A is optionally substituted C₁-C₄ heteroalkylene. In some embodiments, A is optionally substituted C₂ heteroalkylene. In some embodiments, A is:

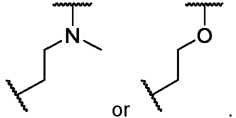

In some embodiments of compounds of the present invention, A is optionally substituted 6-membered arylene. In some embodiments, A has the structure: 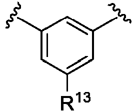 wherein R¹³ is hydrogen, hydroxy, amino, optionally substituted C₁-C₆ alkyl, or optionally substituted C₁-C₆ heteroalkyl. In some embodiments, R¹³ is hydrogen. In some embodiments, R¹³ is hydroxy.

In some embodiments of compounds of the present invention, B is -CHR⁹-. In some embodiments, R⁹ is optionally substituted C₁-C₆ alkyl or optionally substituted 3 to 6-membered cycloalkyl. In some embodiments, R⁹ is: 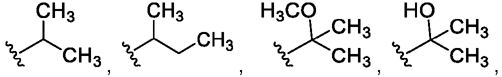 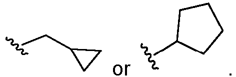 In some embodiments, R⁹ is: 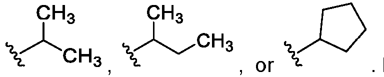 In some embodiments, R⁹ is: 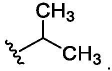 In some embodiments, R⁹ is optionally substituted C₁-C₆ alkyl, optionally substituted C₁-C₆ heteroalkyl, optionally substituted 3 to 6-membered cycloalkyl, or optionally substituted 3 to 7-membered heterocycloalkyl.

In some embodiments, B is optionally substituted 6-membered arylene. In some embodiments, B is 6-membered arylene. In some embodiments, B is:

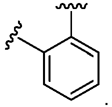

In some embodiments of compounds of the present invention, R⁷ is methyl.

In some embodiments of compounds of the present invention, R⁸ is methyl.

In some embodiments, R³⁴ is hydrogen.

In some embodiments, A has the structure: 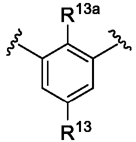 wherein R¹³ is hydrogen, halo, hydroxy, amino, optionally substituted C₁-C₆ alkyl, or optionally substituted C₁-C₆ heteroalkyl; and R^{13a} is hydrogen or halogen. In some embodiments, R¹³ is hydrogen. In some embodiments, R¹³ and R^{13a} are each hydrogen. In some embodiments, R¹³ is hydroxy, methyl, fluoro, or difluoromethyl.

In some embodiments, A is an optionally substituted 5- to 10-membered heteroarylene. In some embodiments, A is:

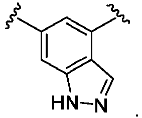

In some embodiments, A is optionally substituted 5- to 6-membered heteroarylene. In some embodiments, A is: 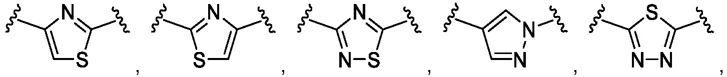 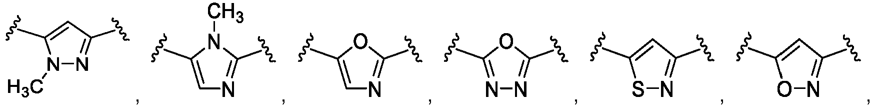 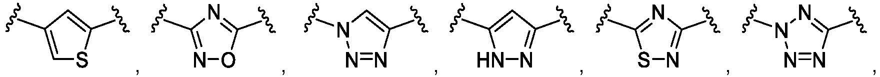 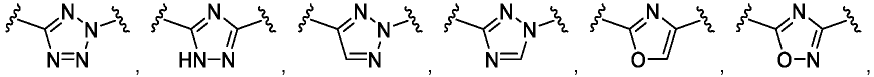 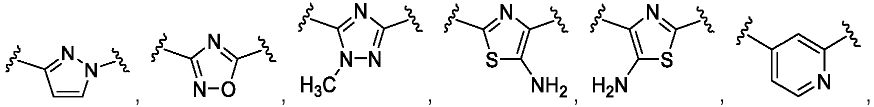 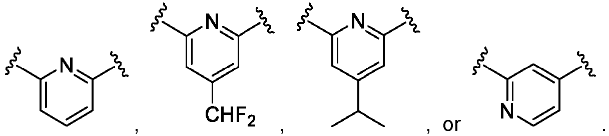 In some embodiments, A is: 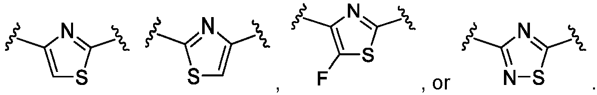 In some embodiments, A is

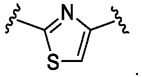

**In** some embodiments, A is optionally substituted C₁-C₄ heteroalkylene. In some embodiments, A is 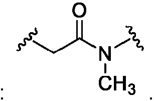 In some embodiments, A is optionally substituted 3- to 6-membered heterocycloalkylene. In some embodiments, A is 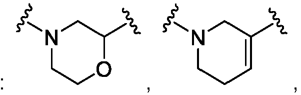 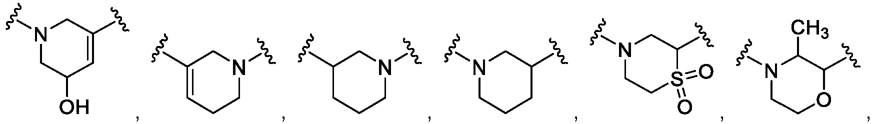 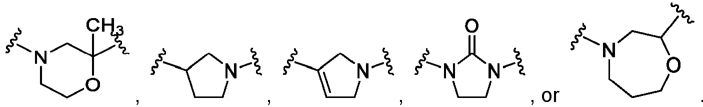 In some embodiments, A is

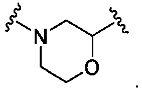

In some embodiments, R⁹ is H, F, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl. In some embodiments, R⁹ is: 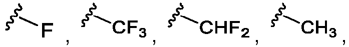 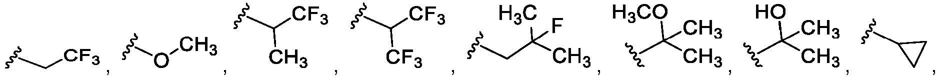 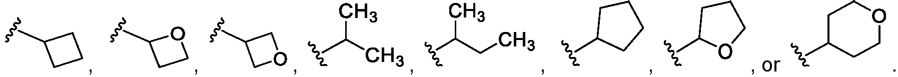 In some embodiments, R⁹ is: 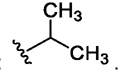 **In** some embodiments, R⁹ is H, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl.

In some embodiments of a compound of the present invention, B is optionally substituted 6-membered arylene. In some embodiments, B is 6-membered arylene. In some embodiments, B is:

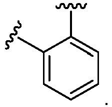

In some embodiments, R¹³ is

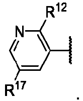

**In** some embodiments, R¹³ is

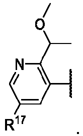

In some embodiments, R¹³ is 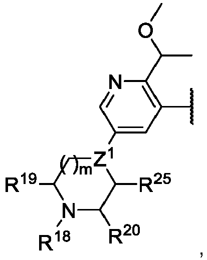
wherein Z¹ is N or CH;
m is 1 or 2;
R¹⁸, R¹⁹, R²⁰, and R²⁵ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5-to 10-membered heteroaryl; or
R¹⁸ and R²⁰ combine with the atoms to which they are attached to form an optionally substituted 3- to 8-membered cycloalkyl or an optionally substituted 3- to 8-membered heterocycloalkyl; or
R²⁰ and R²⁵ combine with the atoms to which they are attached to form an optionally substituted 3- to 8-membered heterocycloalkyl; or
R¹⁹ and R²⁰ combine with the atoms to which they are attached to form an optionally substituted 4- to 8-membered heterocycloalkyl.

In some embodiments, R¹³ is

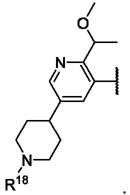

In some embodiments, R¹³ is

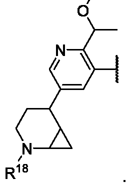

In some embodiments, R¹⁸ is methyl.

In some embodiments, R¹³ is

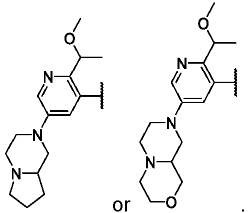

In some embodiments of a compound of the present invention, R⁷ is methyl.

In some embodiments of a compound of the present invention, R⁸ is methyl.

In some embodiments, R²¹ is hydrogen.

In some embodiments of a compound of the present invention, B is -CHR⁹-. In some embodiments, R⁹ is optionally substituted C₁-C₆ alkyl or optionally substituted 3- to 6-membered cycloalkyl. In some embodiments, B is optionally substituted 6-membered arylene. In some embodiments, B is absent.

In some embodiments of a compound of the present invention, the linker is the structure of Formula II-II:

A¹-(B¹)_{f}-(C¹)_{g}-(B²)ₕ-(D¹)-(B³)ᵢ-(C²)ⱼ-(B⁴)ₖ-A² Formula II-II

where A' is a bond between the linker and B; A² is a bond between A and the linker; B¹, B², B³, and B⁴ each, independently, is selected from optionally substituted C₁-C₂ alkylene, optionally substituted C₁-C₃ heteroalkylene, O, S, and NR^{N}; R^{N} is hydrogen, optionally substituted C₁₋₄ alkyl, optionally substituted C₂-C₄ alkenyl, optionally substituted C₂-C₄ alkynyl, optionally substituted 3- to 14-membered heterocycloalkyl, optionally substituted 6- to 10-membered aryl, or optionally substituted C₁-C₇ heteroalkyl; C¹ and C² are each, independently, selected from carbonyl, thiocarbonyl, sulphonyl, or phosphoryl; f, g, h, i, j, and k are each, independently, 0 or 1; and D¹ is optionally substituted C₁-C₁₀ alkylene, optionally substituted C₂-C₁₀ alkenylene, optionally substituted C₂-C₁₀ alkynylene, optionally substituted 3- to 14-membered heterocycloalkylene, optionally substituted 5- to 10-membered heteroarylene, optionally substituted 3- to 8-membered cycloalkylene, optionally substituted 6- to 10-membered arylene, optionally substituted C₂-C₁₀ polyethylene glycolene, or optionally substituted C₁-C₁₀ heteroalkylene, or a chemical bond linking A¹-(B¹)_{f}-(C¹)_{g}-(B²)ₕ- to -(B³)ᵢ-(C²)ⱼ-(B⁴)ₖ-A². In some embodiments, the linker is acyclic. In some embodiments, linker has the structure of Formula II-IIa: 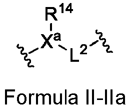
wherein X^{a} is absent or N;
R¹⁴ is absent, hydrogen or optionally substituted C₁-C₆ alkyl; and
L² is absent, -SO₂-, optionally substituted C₁-C₄ alkylene or optionally substituted C₁-C₄ heteroalkylene, wherein at least one of X^{a}, R¹⁴, or L² is present.

In some embodiments, the linker has the structure:

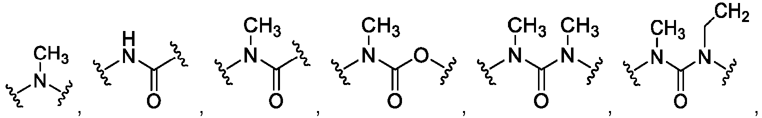

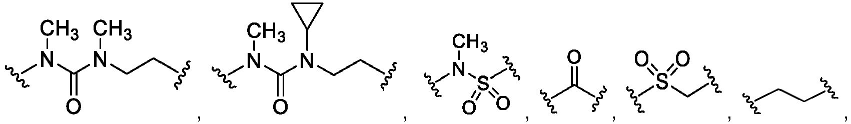

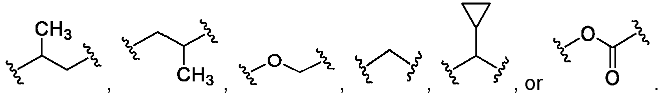

In some embodiments, the linker has the structure: 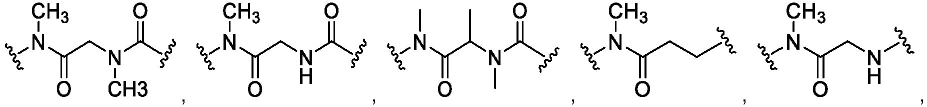 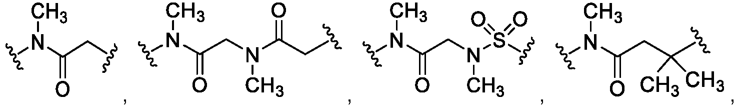 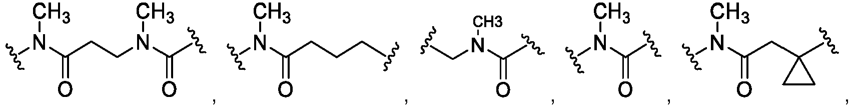 or

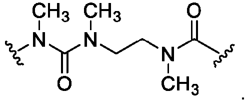

In some embodiments, the linker has the structure: 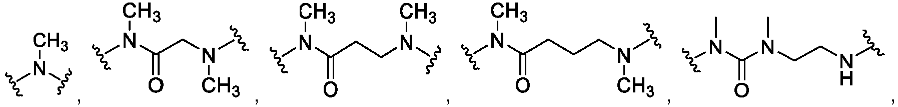 or 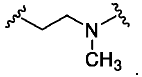 In some embodiments, the linker has the structure 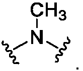 In some embodiments, the linker has the structure

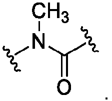

In some embodiments, the linker is or comprises a cyclic moiety. In some embodiments, the linker has the structure of Formula II-IIb: 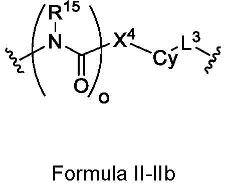
wherein o is 0 or 1;
R¹⁵ is hydrogen or optionally substituted C₁-C₆ alkyl, optionally substituted 3- to 8-membered cycloalkylene, or optionally substituted 3- to 8-membered heterocycloalkylene;
X⁴ is absent, optionally substituted C₁-C₄ alkylene, O, NCH₃, or optionally substituted C₁-C₄ heteroalkylene;
Cy is optionally substituted 3- to 8-membered cycloalkylene, optionally substituted 3- to 8-membered heterocycloalkylene, optionally substituted 6- to 10-membered arylene, or optionally substituted 5- to 10-membered heteroarylene; and
L³ is absent, -SO₂-, optionally substituted C₁-C₄ alkylene or optionally substituted C₁-C₄ heteroalkylene.

In some embodiments, the linker has the structure of Formula II-IIb-1: 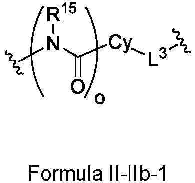
wherein o is 0 or 1;
R¹⁵ is hydrogen or optionally substituted C₁-C₆ alkyl, optionally substituted 3- to 8-membered cycloalkylene, or optionally substituted 3- to 8-membered heterocycloalkylene;
Cy is optionally substituted 3- to 8-membered cycloalkylene, optionally substituted 3- to 8-membered heterocycloalkylene, optionally substituted 6- to 10-membered arylene, or optionally substituted 5- to 10-membered heteroarylene; and
L³ is absent, -SO₂-, optionally substituted C₁-C₄ alkylene or optionally substituted C₁-C₄ heteroalkylene.

In some embodiments, the linker is or comprises a cyclic group. In some embodiments, linker has the structure of Formula II-IIb-2: 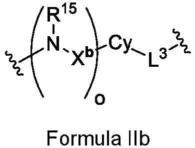
wherein o is 0 or 1;
X^{b} is C(O) or SO₂;
R¹⁵ is hydrogen or optionally substituted C₁-C₆ alkyl;
Cy is optionally substituted 3 to 8-membered cycloalkylene, optionally substituted 3 to 8-membered heterocycloalkylene, optionally substituted 6-10 membered arylene, or optionally substituted 5 to 10-membered heteroarylene; and
L³ is absent, -C(O)-, -SO₂-, optionally substituted C₁-C₄ alkylene or optionally substituted C₁-C₄ heteroalkylene.

In some embodiments, the linker has the structure: 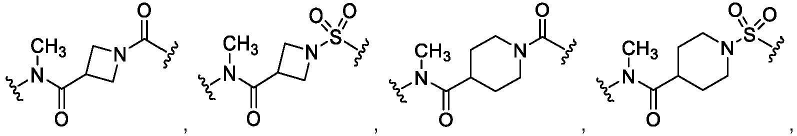 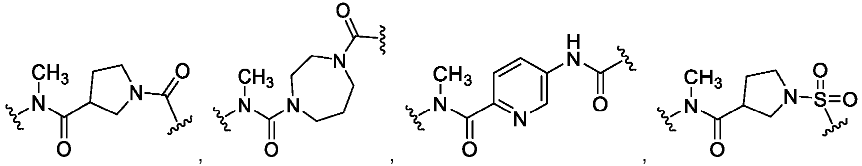 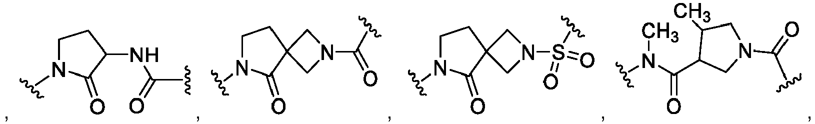 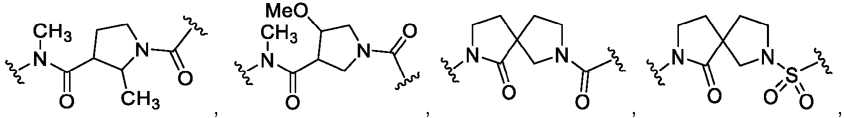 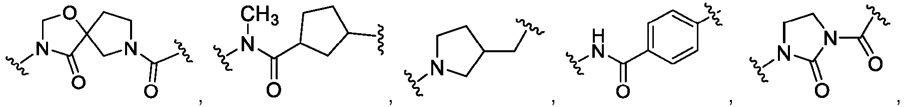 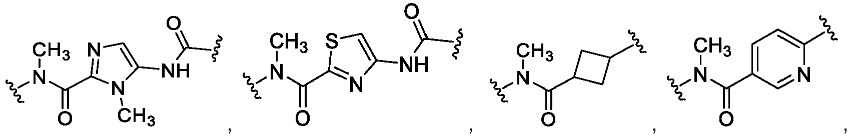 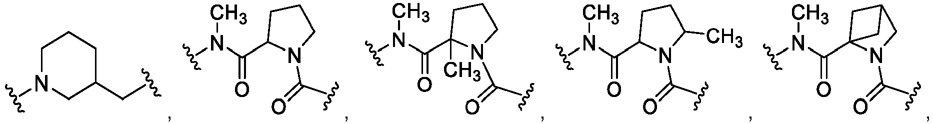 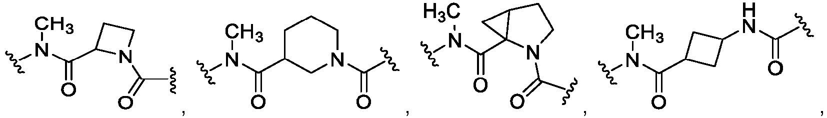 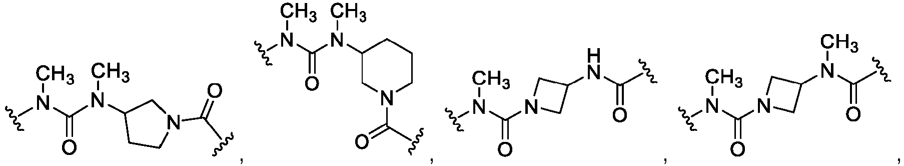 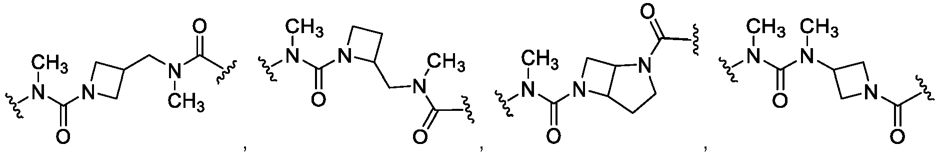 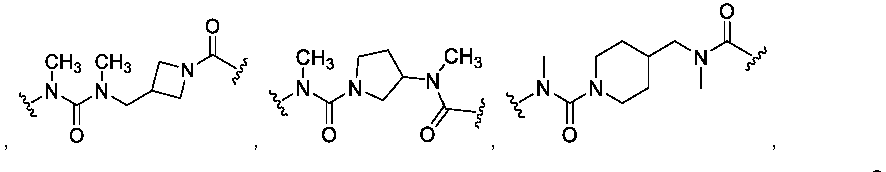 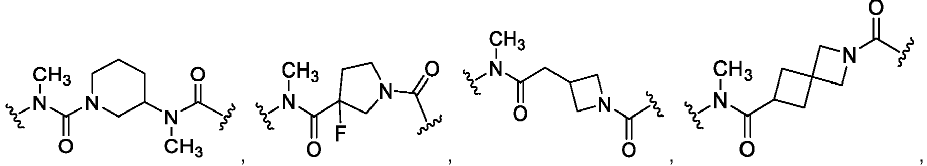 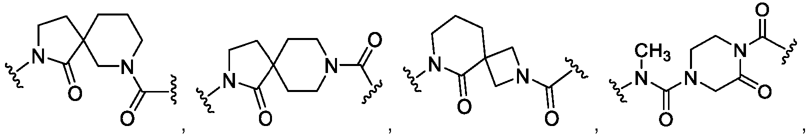 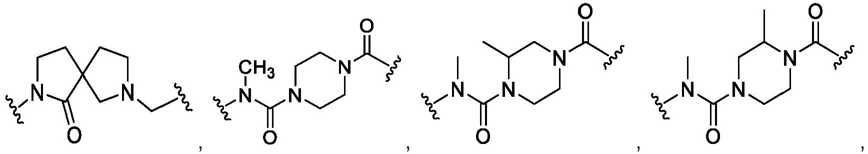 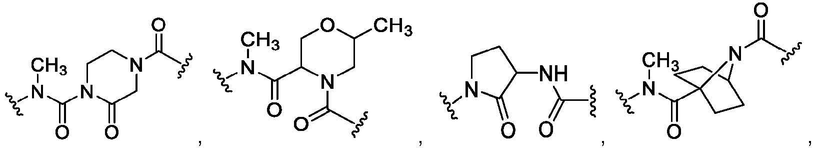 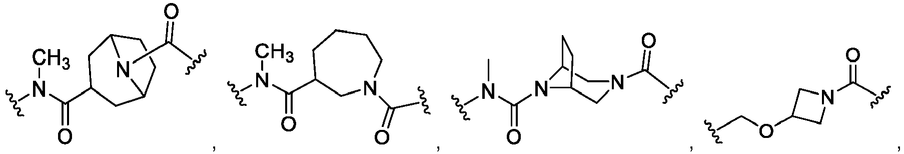 or

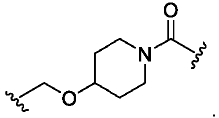

In some embodiments, linker has the structure:

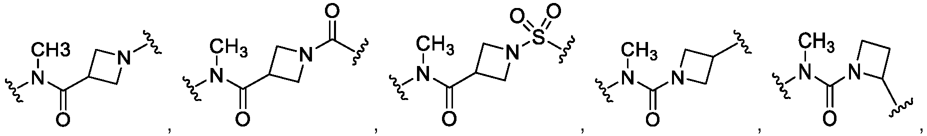

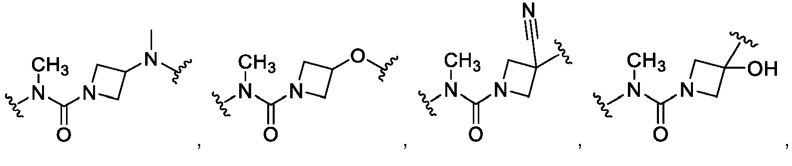

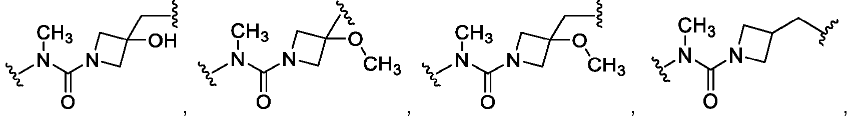

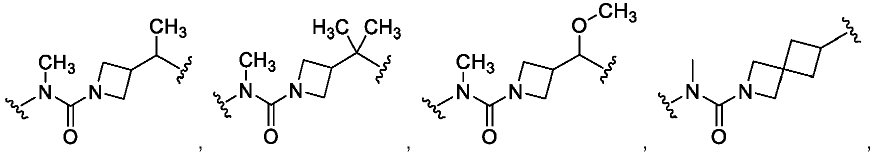

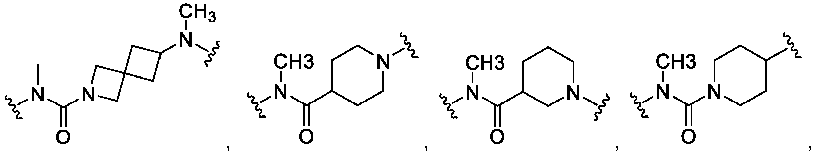

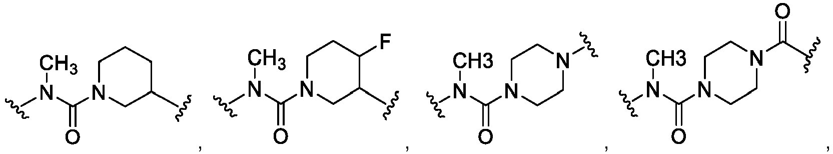

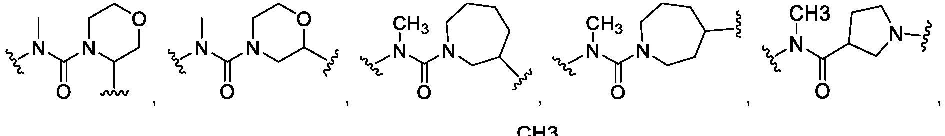

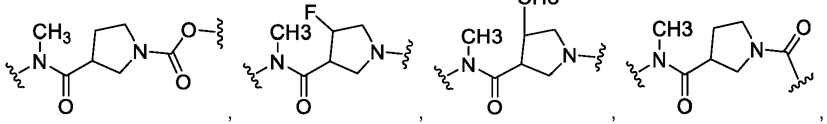

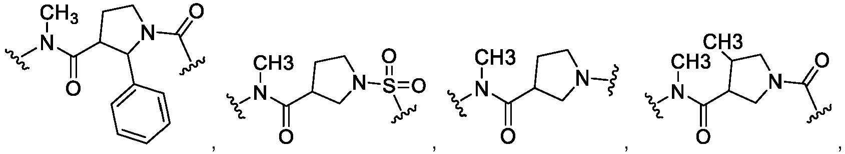

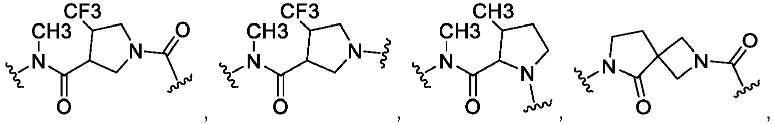

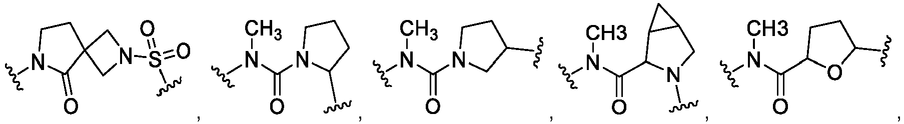

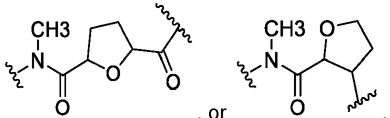

In some embodiments, the linker has the structure of Formula II-IIc: 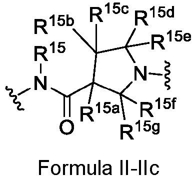
wherein R¹⁵ is hydrogen, optionally substituted C₁-C₆ alkyl, optionally substituted 3- to 8-membered cycloalkylene, or optionally substituted 3- to 8-membered heterocycloalkylene; and
R^{15a}, R^{15b}, R^{15c}, R^{15d}, R^{15e}, R^{15f}, and R^{15g} are, independently, hydrogen, halo, hydroxy, cyano, amino, optionally substituted C₁-C₆ alkyl, optionally substituted C₁-C₆ alkoxy, or , or R^{15b} and R^{15d} combine with the carbons to which they are attached to form an optionally substituted 3- to 8-membered cycloalkylene, or optionally substituted 3- to 8-membered heterocycloalkylene.

In some embodiments, the linker has the structure:

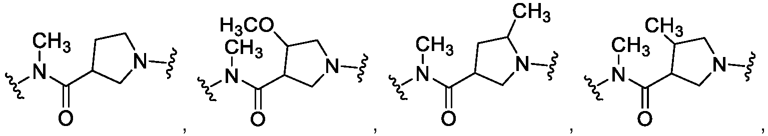

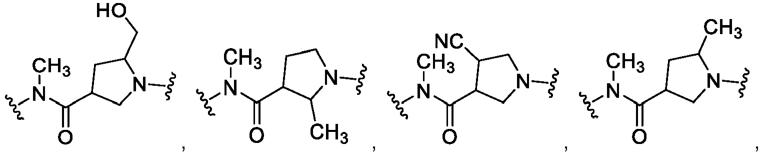

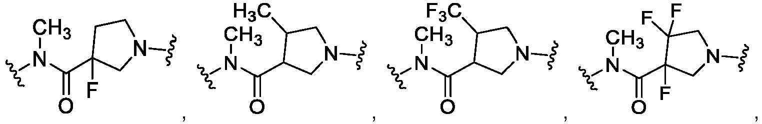

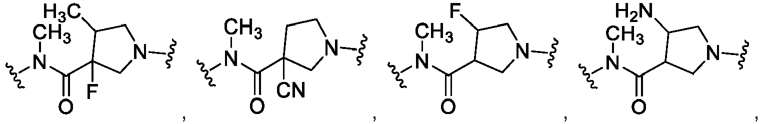

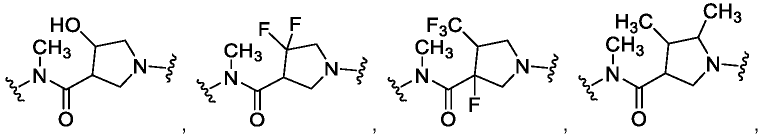

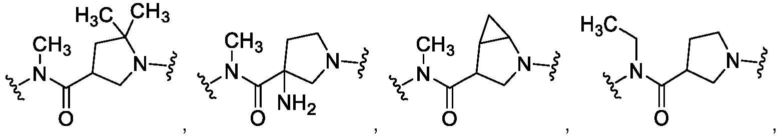

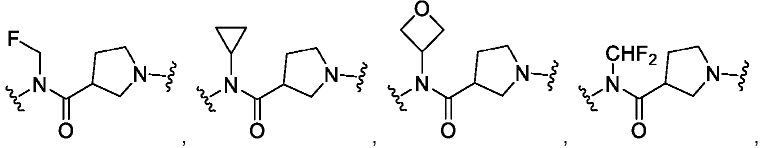

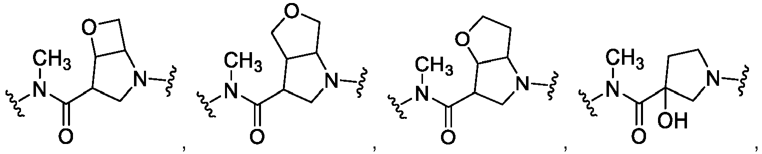

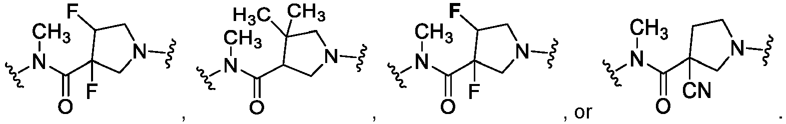

In some embodiments, the linker has the structure:

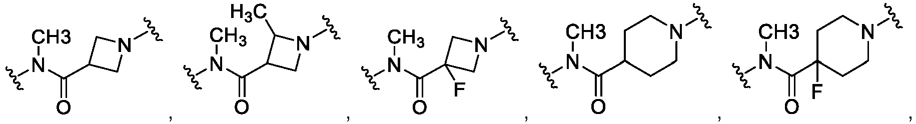

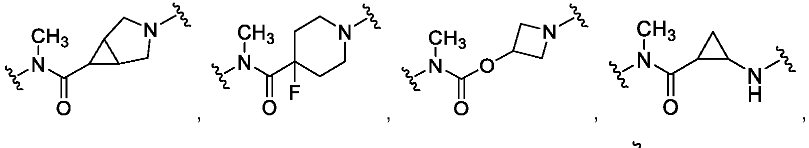

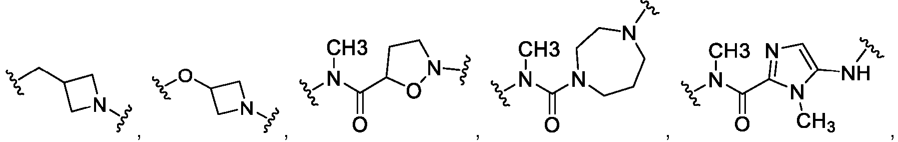

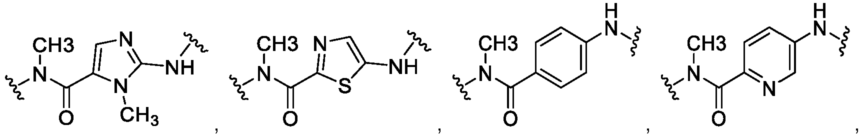

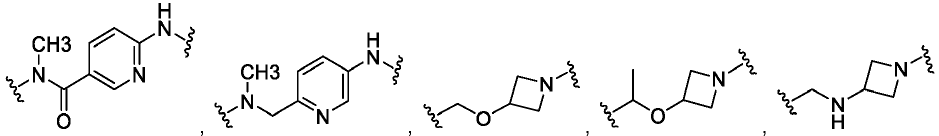

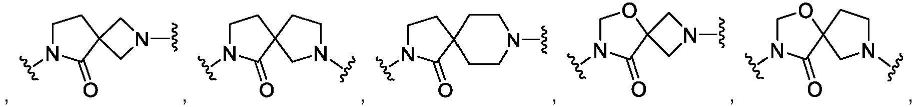

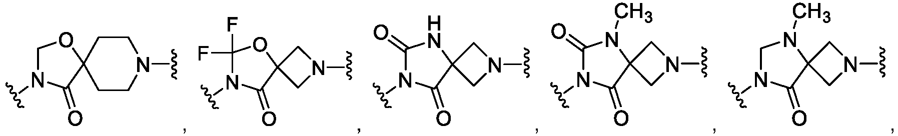

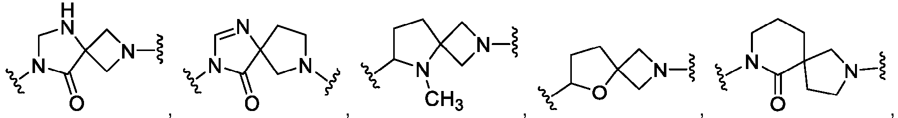

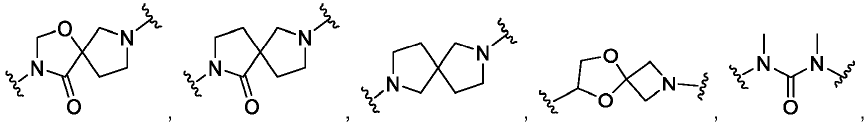

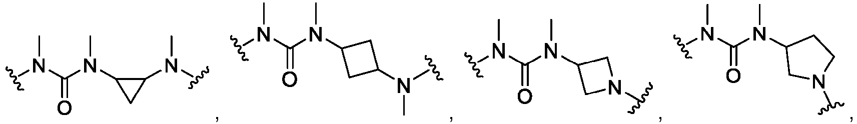

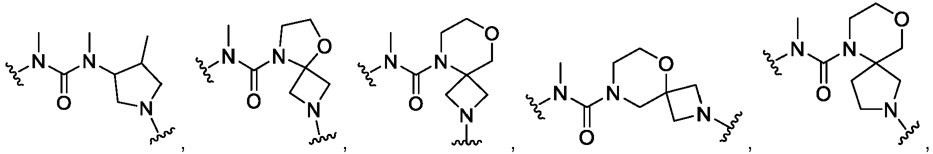

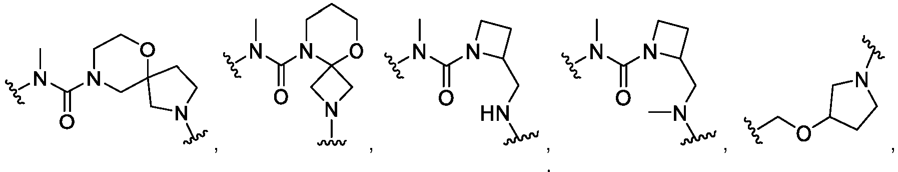

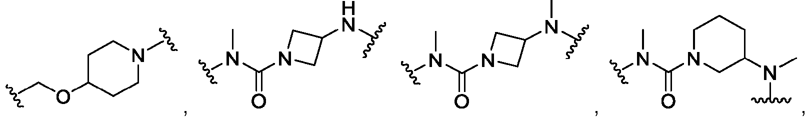

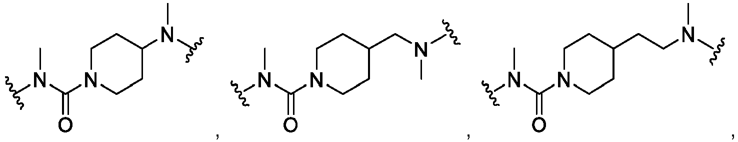

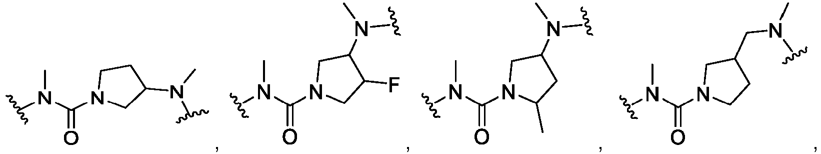

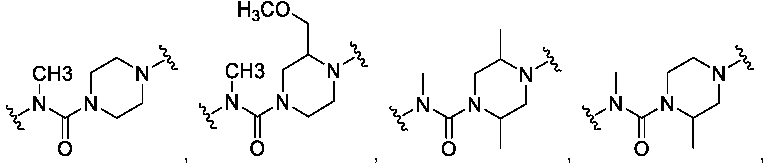

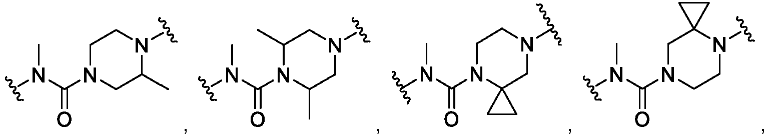

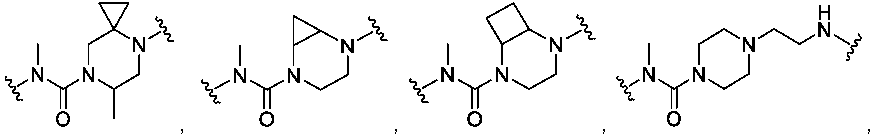

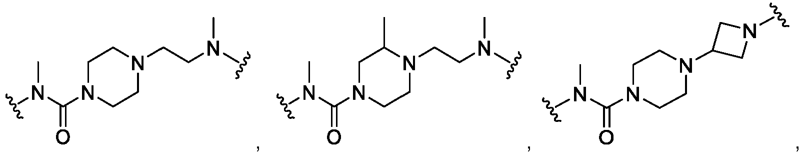

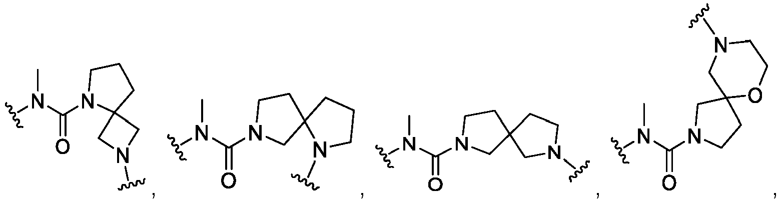

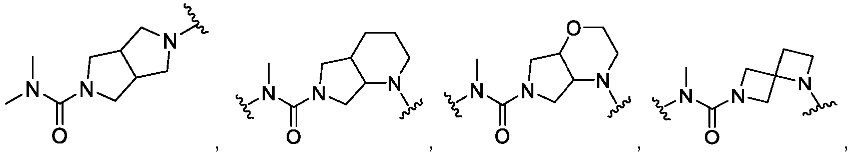

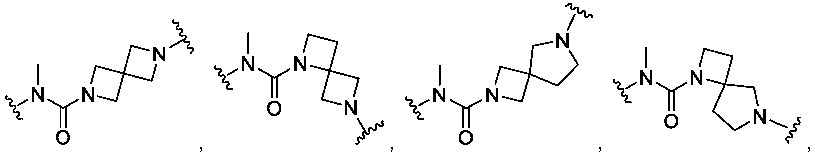

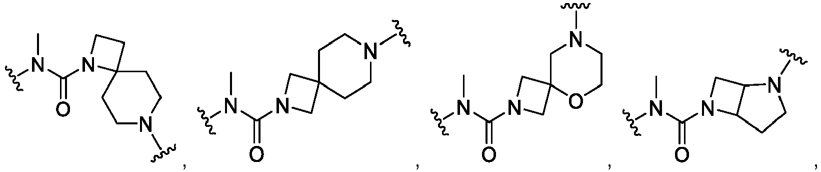

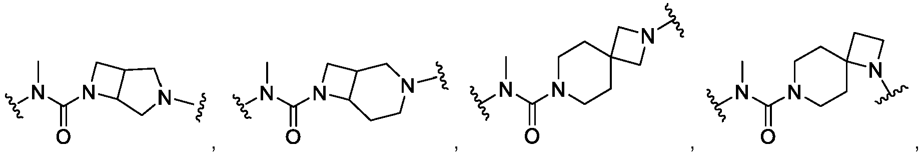

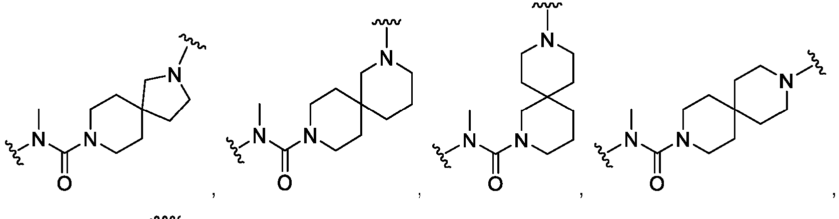

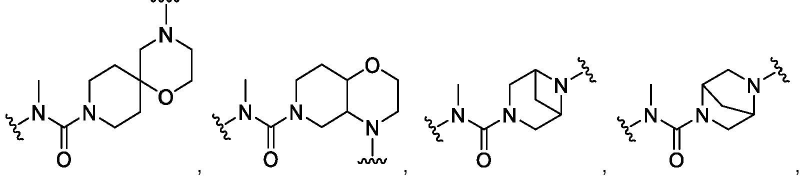

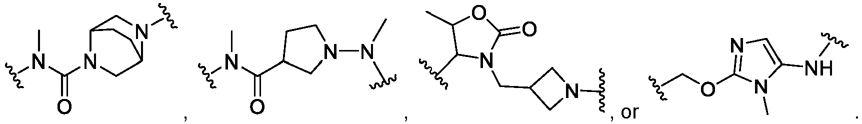

In some embodiments, the linker has the structure 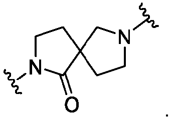 In some embodiments, the linker has the structure

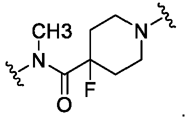

In some embodiments, a linker of Formula II is selected from the group consisting of

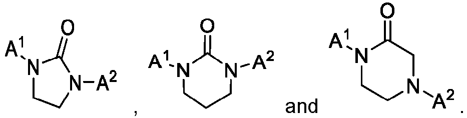

In some embodiments of compounds of formula IIc or any subformula of formula IIc, or a pharmaceutically acceptable salt or stereoisomer thereof, the compound is not:

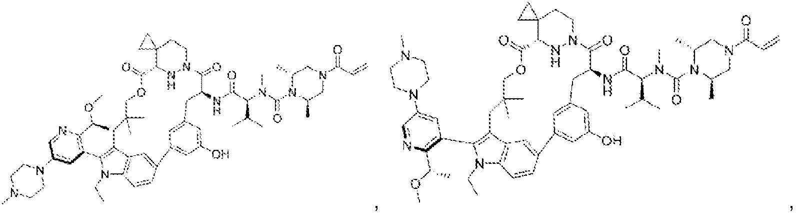

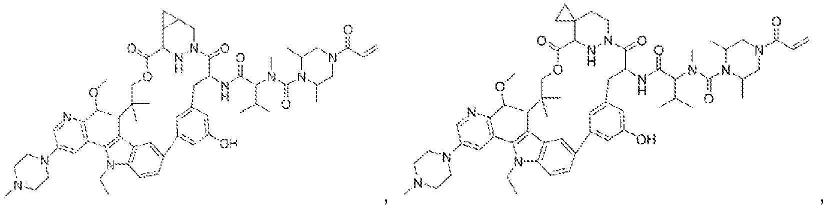

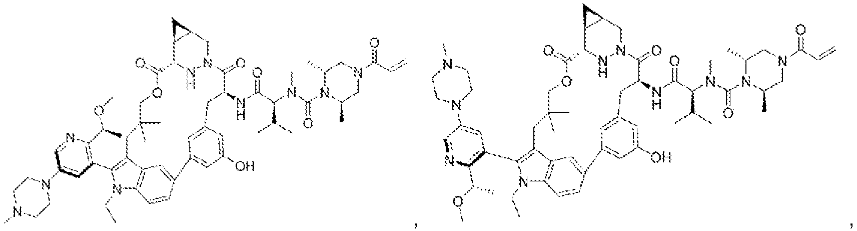

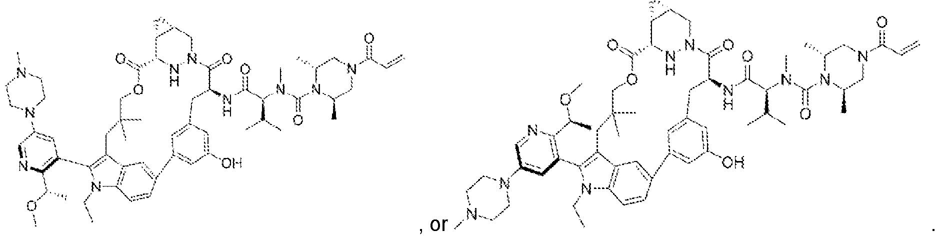

In some embodiments of compounds of Formula IIc or any subformula of Formula IIc, or a pharmaceutically acceptable salt or stereoisomer thereof, when A is 1-hydroxy-benzene-3,5-diyl or optionally substituted phenylene, two R^{L1} substituents taken together form spiro cyclopropyl, and W is vinyl ketone, then R² is not ethyl or C₁₋₆ alkyl.

In some embodiments of compounds of Formula IIc or any subformula of Formula IIc, or a pharmaceutically acceptable salt or stereoisomer thereof, when A is 1-hydroxy-benzene-3,5-diyl or optionally substituted phenylene, R^{L1} and R^{L2} taken together form fused cyclopropyl, and W is vinyl ketone, then R² is not ethyl or C₁. alkyl.

In some embodiments of compounds of Formula IIc or any subformula of Formula IIc, or a pharmaceutically acceptable salt or stereoisomer thereof, when A is 1-hydroxy-benzene-3,5-diyl or optionally substituted phenylene and two R^{L1} substituents taken together form spiro cyclopropyl, then W is not vinyl ketone.

In some embodiments of compounds of Formula IIc or any subformula of Formula IIc, or a pharmaceutically acceptable salt or stereoisomer thereof, when A is 1-hydroxy-benzene-3,5-diyl or optionally substituted phenylene, R^{L1} and R^{L2} taken together form fused cyclopropyl, then W is not vinyl ketone.

In some embodiments of compounds of Formula IIc or any subformula of Formula IIc, or a pharmaceutically acceptable salt or stereoisomer thereof, when A is 1-hydroxy-benzene-3,5-diyl or optionally substituted phenylene, W is vinyl ketone and two R^{L1} substituents taken together form spiro cyclopropyl or spiro cycloalkyl, then L¹ is other than

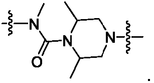

In some embodiments of compounds of formula IIc or any subformula of Formula IIc, or a pharmaceutically acceptable salt or stereoisomer thereof, when A is 1-hydroxy-benzene-3,5-diyl or optionally substituted phenylene, W is vinyl ketone and R^{L1} and R^{L2} taken together form fused cyclopropyl or fused cycloalkyl, then L¹ is other than

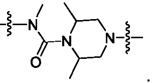

In some embodiments of compounds of formula IIc or any subformula of Formula IIc, or a pharmaceutically acceptable salt or stereoisomer thereof, when A is 1-hydroxy-benzene-3,5-diyl or optionally substituted phenylene, W is vinyl ketone and two R^{L1} substituents taken together form spiro cycloalkyl, then L¹ is not 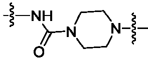 optionally substituted with 1, 2 or 3 independently selected C₁₋₆ alkyl.

In some embodiments of compounds of Formula IIc or any subformula of Formula IIc, or a pharmaceutically acceptable salt or stereoisomer thereof, when A is 1-hydroxy-benzene-3,5-diyl or optionally substituted phenylene, W is vinyl ketone and R^{L1} and R^{L2} taken together form fused cycloalkyl, then L¹ is not 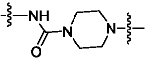 optionally substituted with 1, 2 or 3 independently selected C₁₋₆ alkyl.

In some embodiments, the disclosure features a compound, or pharmaceutically acceptable salt thereof, of structural Formula III: 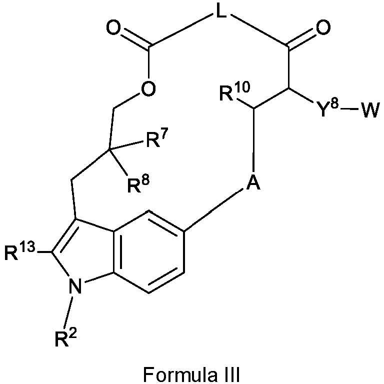
wherein A is optionally substituted 3- to 6-membered cycloalkylene, optionally substituted 3-to 6-membered heterocycloalkylene, optionally substituted 6-membered arylene, optionally substituted 5- to 6-membered heteroarylene, optionally substituted C₂-C₄ alkylene, optionally substituted C₁-C₄ heteroalkylene or optionally substituted C₂-C₄ alkenylene;
Y⁸ is 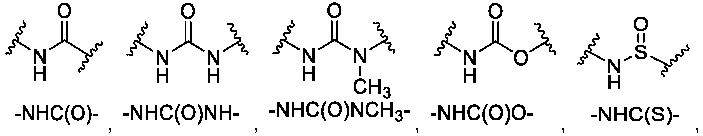 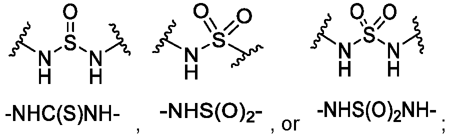
W is hydrogen, C₁-C₄ alkyl, optionally substituted C₁-C₃ heteroalkyl, optionally substituted 3-to 10-membered heterocycloalkyl, optionally substituted 3- to 10-membered cycloalkyl, optionally substituted 6- to 10-membered aryl, or optionally substituted 5- to 10-membered heteroaryl;
L has the structure of Formula VIIa or VIIb: 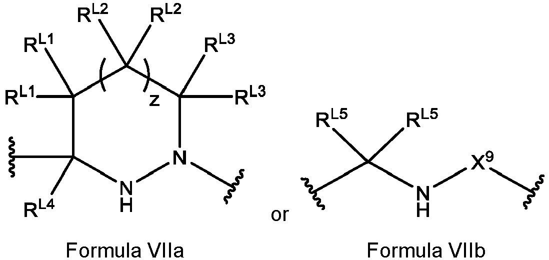
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl; or R^{L1} and R^{L2} taken together form a bond;
wherein L does not have the structure of 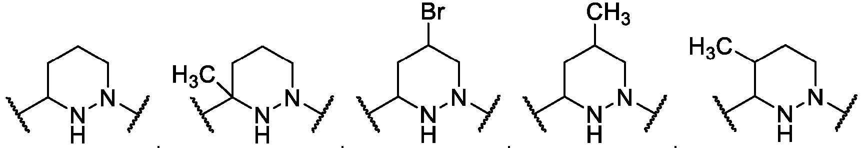 or 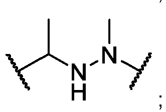
X⁴ and X⁵ are each, independently, CH₂, CH(CH₃) or NH;
R¹³ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 15-membered heterocycloalkyl, optionally substituted 6- to 10-membered aryl, or optionally substituted 5- to 10-membered heteroaryl;
R² is hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl;
R¹⁰ is hydrogen, hydroxy, optionally substituted C₁-C₆ alkoxy, optionally substituted C₁-C₃ alkyl, optionally substituted C₁-C₆ heteroalkyl, or optionally substituted 3- to 7-membered heterocycloalkyl; and
R⁷ and R⁸ are each, independently, selected from fluoro or CH₃, or R⁷ and R⁸ combine with the atoms to which they are attached to make a 3-membered cycloalkyl.

In some embodiments, the compound, or pharmaceutically acceptable salt thereof, has the structure of Formula III-a: 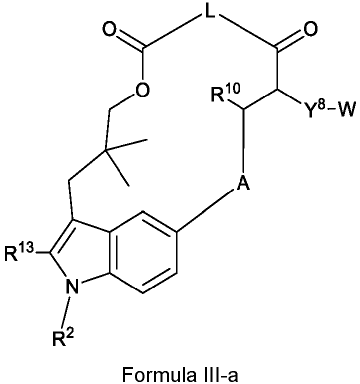
wherein A is optionally substituted 3- to 6-membered cycloalkylene, optionally substituted 3-to 6-membered heterocycloalkylene, optionally substituted 6-membered arylene, or optionally substituted 5- to 6-membered heteroarylene;
Y⁸ is 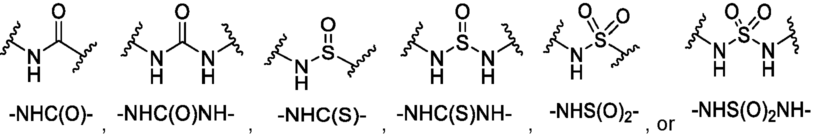
W is hydrogen, C₁-C₄ alkyl, optionally substituted C₁-C₃ heteroalkyl, optionally substituted 3-to 10-membered heterocycloalkyl, optionally substituted 3- to 10-membered cycloalkyl, optionally substituted 6- to 10-membered aryl, or optionally substituted 5- to 10-membered heteroaryl;
L has the structure of Formula VIIa or VIIb: 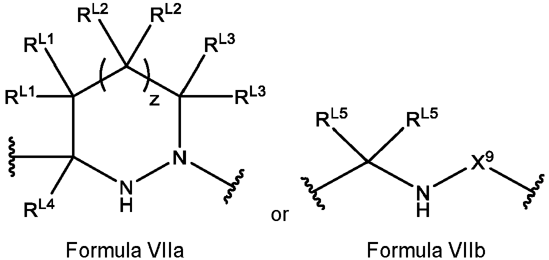
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 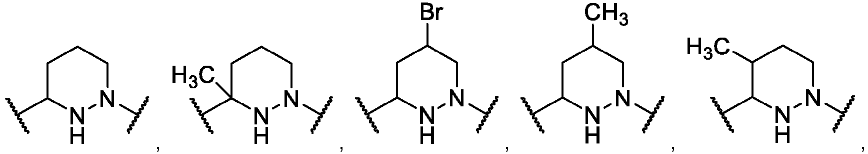 or 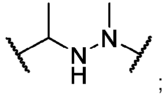
R¹³ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl;
R² is hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; and
R¹⁰ is hydrogen or optionally substituted C₁-C₆ heteroalkyl. In some embodiments, R¹⁰ is hydrogen.

In some embodiments, R¹³ is optionally substituted 6- to 10-membered aryl or optionally substituted 5- to 10-membered heteroaryl. In some embodiments, R¹³ is optionally substituted phenyl or optionally substituted pyridine.

In some embodiments, A is optionally substituted thiazole, optionally substituted triazole, optionally substituted morpholino, optionally substituted piperidinyl, optionally substituted pyridine, or optionally substituted phenyl. In some embodiments, A is optionally substituted thiazole, optionally substituted triazole, optionally substituted morpholino, or phenyl. In some embodiments, A is not an optionally substituted phenyl or benzimidazole. In some embodiments, A is not hydroxyphenyl.

In some embodiments, Y⁸ is -NHC(O)- or -NHC(O)NH-.

In some embodiments of Formula III, the compound, or a pharmaceutically acceptable salt thereof, has the structure of Formula Illa: 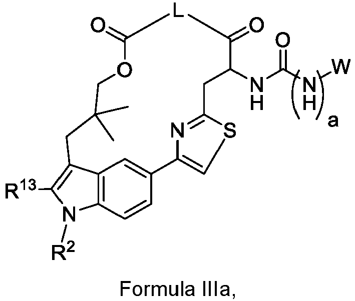 wherein a is 0 or 1.

In some embodiments of Formula III, the compound, or a pharmaceutically acceptable salt thereof, has the structure of Formula Illa-1: 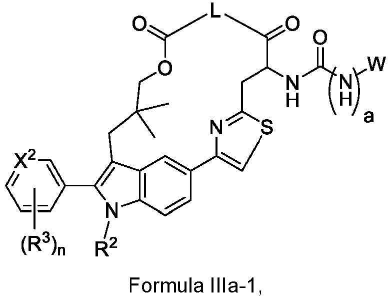
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula IIIa-2:

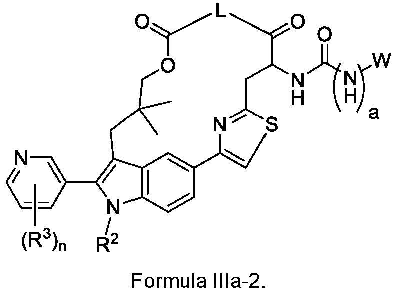

In some embodiments of Formula III, the compound, or a pharmaceutically acceptable salt thereof, has the structure of Formula IIIa-3: 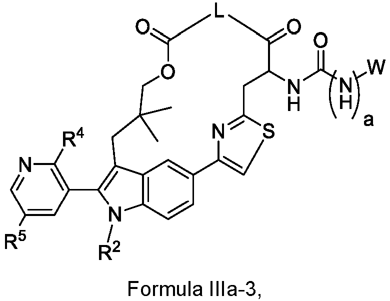 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula Illa-4:

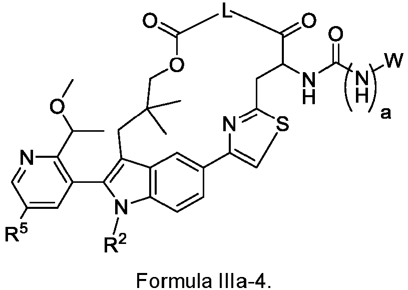

In some embodiments of Formula III, the compound, or a pharmaceutically acceptable salt thereof, has the structure of Formula Illa-5: 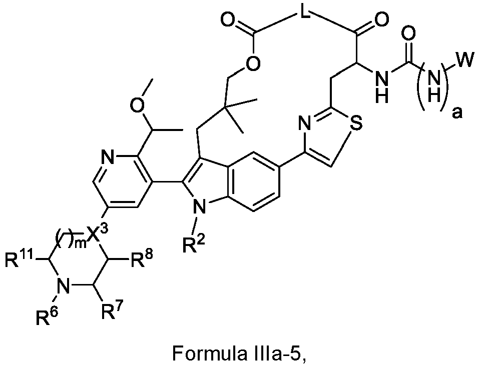
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is H. In some embodiments, X³ is N, m is 1, and R¹¹ is H.

In some embodiments of Formula III, the compound, or a pharmaceutically acceptable salt thereof, has the structure of Formula Illa-6:

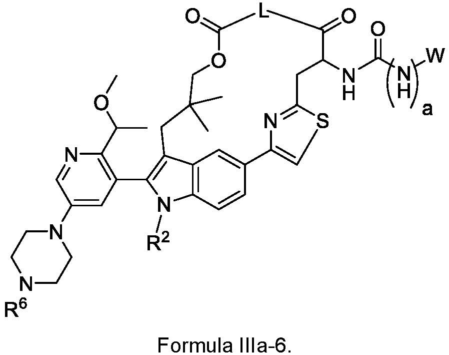

In some embodiments of Formula III, the compound, or a pharmaceutically acceptable salt thereof, has the structure of Formula IIIa-7: 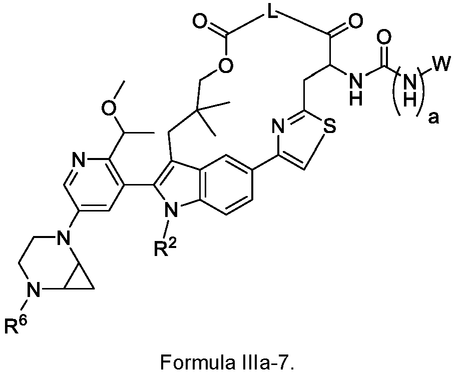 In some embodiments (e.g., of any one of Formulae Illa-6 or Illa-7), R⁶ is methyl.

In some embodiments of Formula III, the compound, or a pharmaceutically acceptable salt thereof, has the structure of Formula Illa-8 or Formula Illa-9:

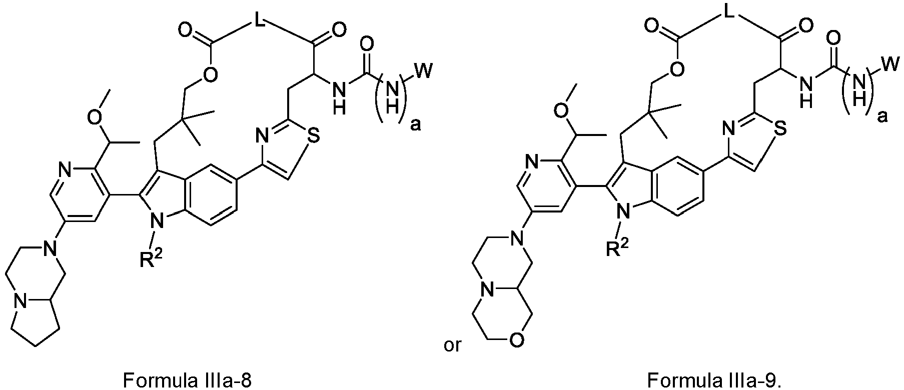

In some embodiments of Formula III, the compound, or a pharmaceutically acceptable salt thereof, has the structure of Formula IIIa-a: 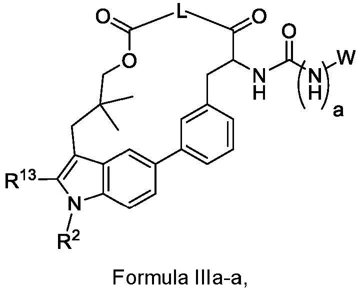 wherein a is 0 or 1.

In some embodiments of Formula III, the compound, or a pharmaceutically acceptable salt thereof, has the structure of Formula IIIa-a1: 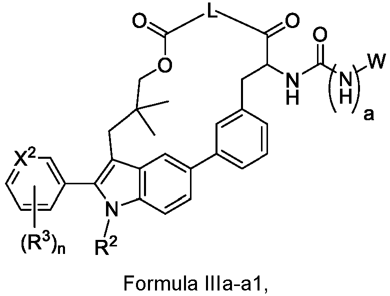
wherein X^{a} is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula Illa-a2:

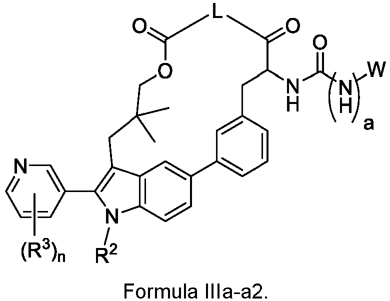

In some embodiments of Formula III, the compound, or a pharmaceutically acceptable salt thereof, has the structure of Formula Illa-a3: 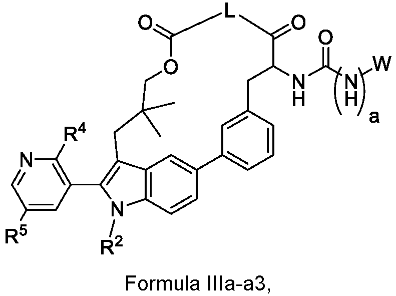 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula Illa-a4:

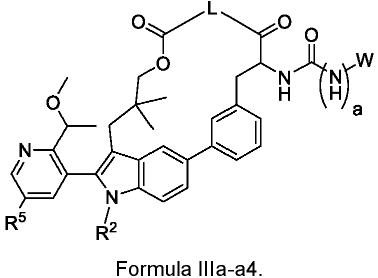

In some embodiments of Formula III, the compound, or a pharmaceutically acceptable salt thereof, has the structure of Formula Illa-a5: 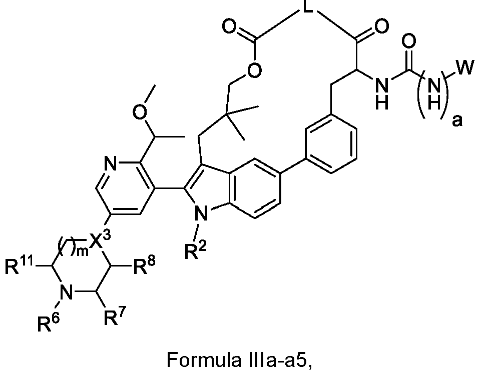
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of Formula III, the compound, or a pharmaceutically acceptable salt thereof, has the structure of Formula Illa-a6:

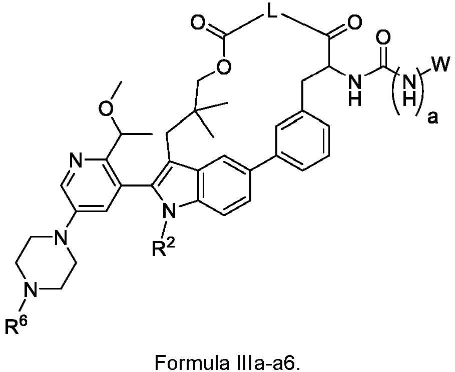

In some embodiments of Formula III, the compound, or a pharmaceutically acceptable salt thereof, has the structure of Formula Illa-a7:

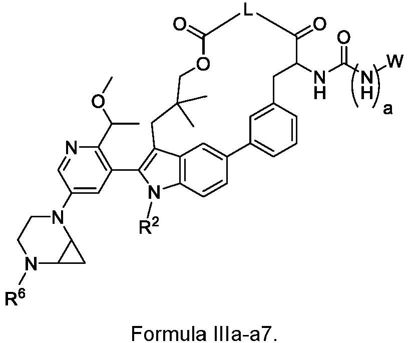

In some embodiments (e.g., of any one of Formulae Illa-a6 or Illa-a7), R⁶ is methyl.

In some embodiments of Formula III, the compound, or a pharmaceutically acceptable salt thereof, has the structure of Formula Illa-a8 or Formula Illa-a9:

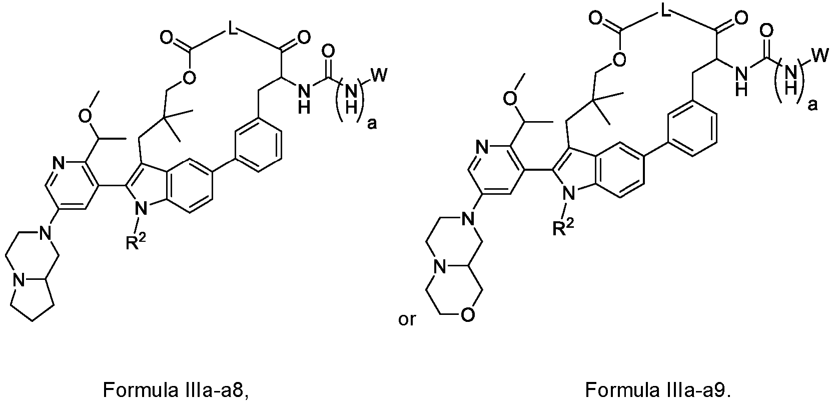

In some embodiments of Formula III, the compound, or a pharmaceutically acceptable salt thereof, has the structure of Formula III-IVa: 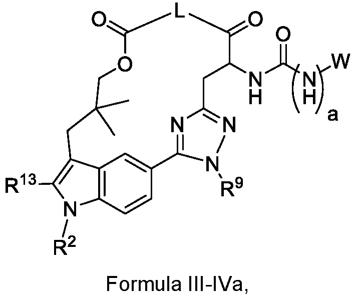
wherein R⁹ is H or C₁-C₆ alkyl; and
a is 0 or 1.

In some embodiments of Formula III, the compound, or a pharmaceutically acceptable salt thereof, has the structure of Formula III-IVa-1: 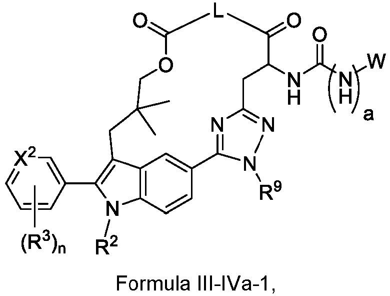
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3 to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-IVa-2:

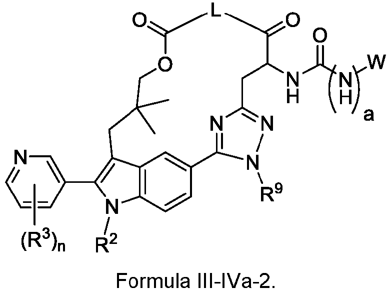

In some embodiments of Formula III, the compound, or a pharmaceutically acceptable salt thereof, has the structure of Formula III-Iva-3: 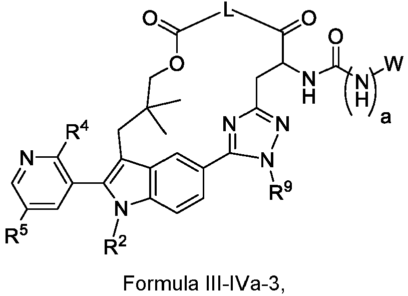 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-IVa-4:

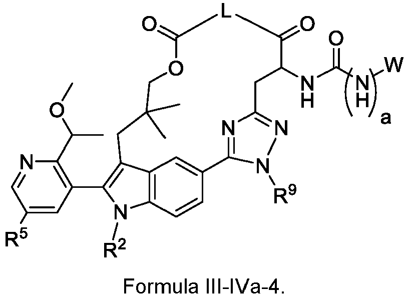

In some embodiments of Formula III, the compound, or a pharmaceutically acceptable salt thereof, has the structure of Formula III-IVa-5: 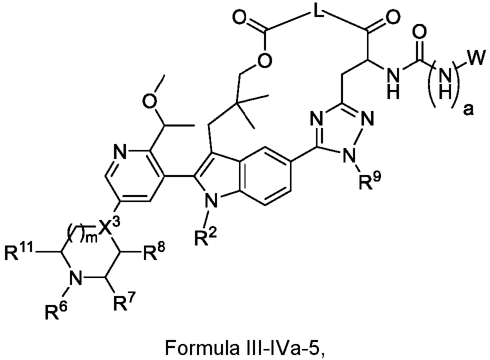
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl ; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of Formula III, the compound, or a pharmaceutically acceptable salt thereof, has the structure of Formula III-IVa-6:

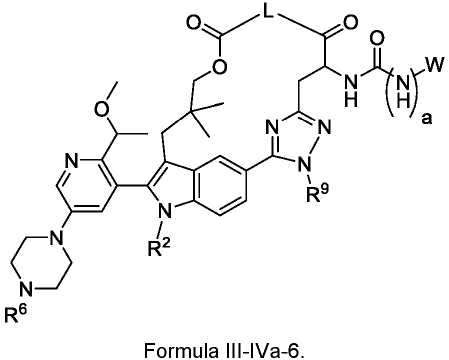

In some embodiments of Formula III, the compound, or a pharmaceutically acceptable salt thereof, has the structure of Formula III-IVa-7:

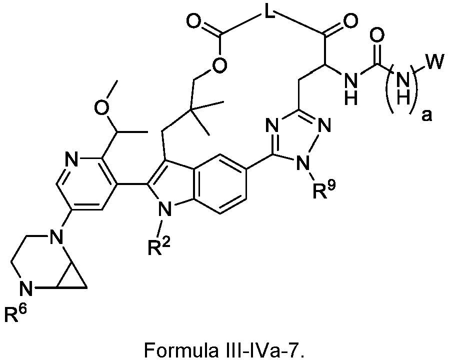

In some embodiments (e.g., of any one of Formulae III-IVa-6 or III-IVa-7), R⁶ is methyl.

In some embodiments of Formula III, the compound, or a pharmaceutically acceptable salt thereof, has the structure of Formula III-IVa-8 or Formula III-IVa-9:

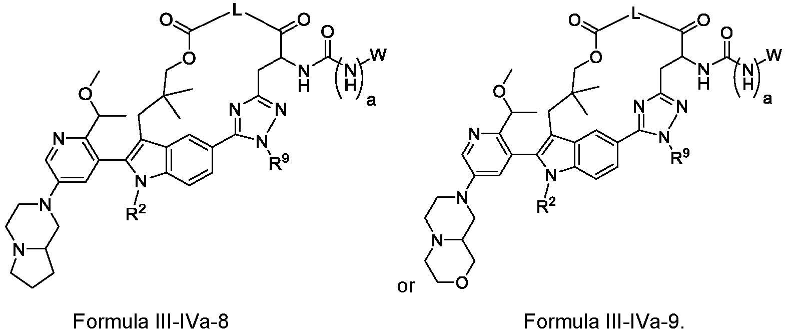

In some embodiments, Y⁸ is -NHS(O)₂- or -NHS(O)₂NH-.

In some embodiments of Formula III, the compound, or a pharmaceutically acceptable salt thereof, has the structure of Formula III-Va: 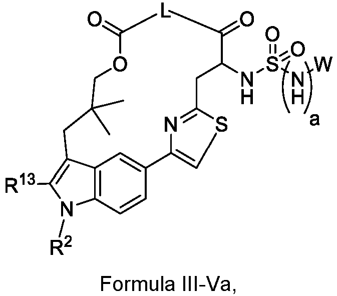 wherein a is 0 or 1.

In some embodiments of Formula III, the compound, or a pharmaceutically acceptable salt thereof, has the structure of Formula III-Va-1: 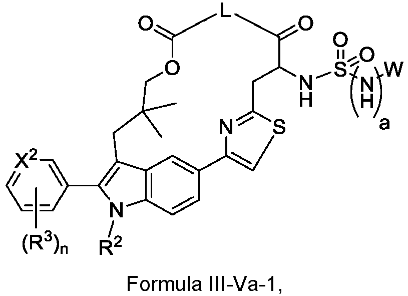
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-Va-2:

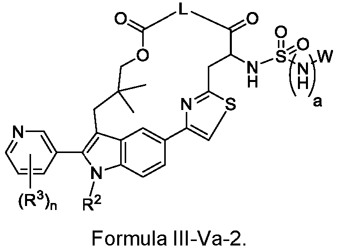

In some embodiments of Formula III, the compound, or a pharmaceutically acceptable salt thereof, has the structure of Formula III-Va-3: 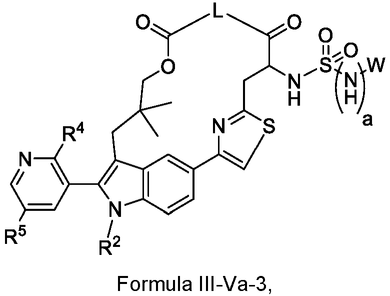 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-Va-4:

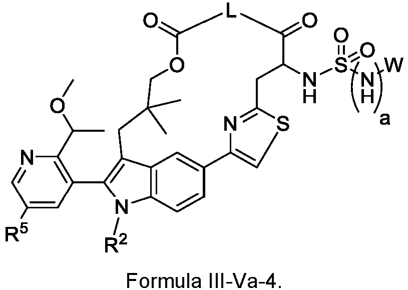

In some embodiments of Formula III, the compound, or a pharmaceutically acceptable salt thereof, has the structure of Formula III-Va-5: 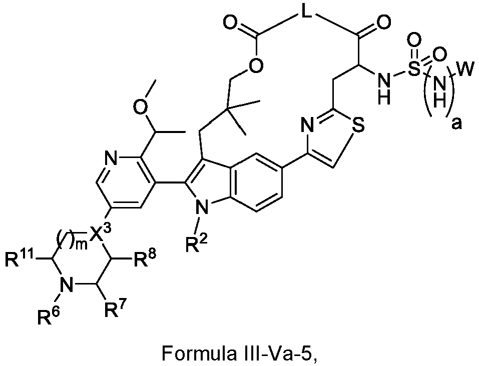
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of Formula III, the compound, or a pharmaceutically acceptable salt thereof, has the structure of Formula III-Vla: 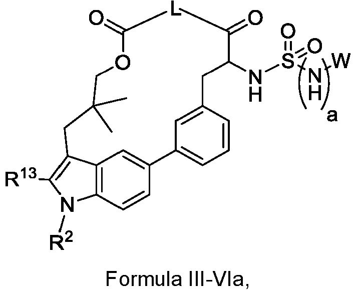 wherein a is 0 or 1.

In some embodiments of Formula III, the compound, or a pharmaceutically acceptable salt thereof, has the structure of Formula III-Vla-1: 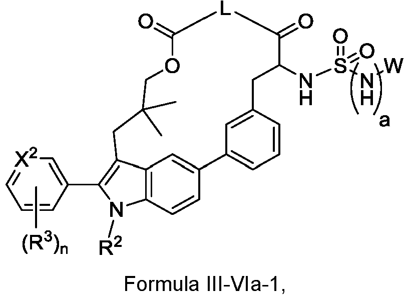
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-Vla-2:

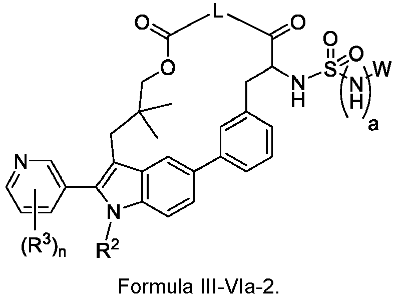

In some embodiments of Formula III, the compound, or a pharmaceutically acceptable salt thereof, has the structure of Formula III-Vla-3: 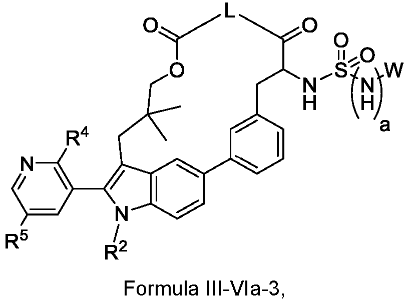 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-Vla-4:

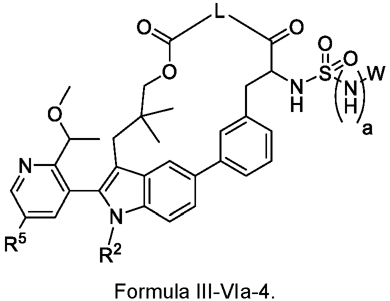

In some embodiments of Formula III, the compound, or a pharmaceutically acceptable salt thereof, has the structure of Formula III-Vla-5: 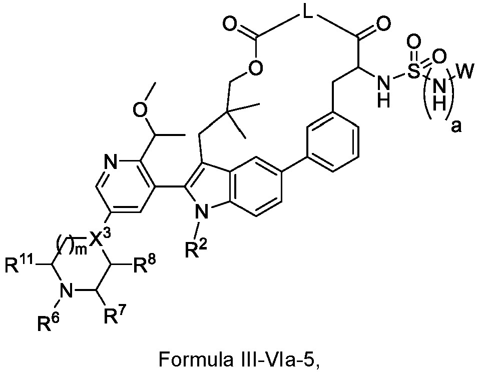
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of Formula III, the compound, or a pharmaceutically acceptable salt thereof, has the structure of Formula III-Vlla: 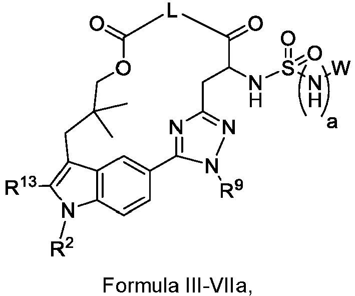
wherein R⁹ is H or C₁-C₆ alkyl; and
a is 0 or 1.

In some embodiments of Formula III, the compound, or a pharmaceutically acceptable salt thereof, has the structure of Formula III-Vlla-1: 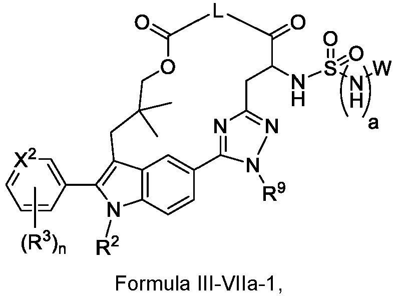
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-Vlla-2:

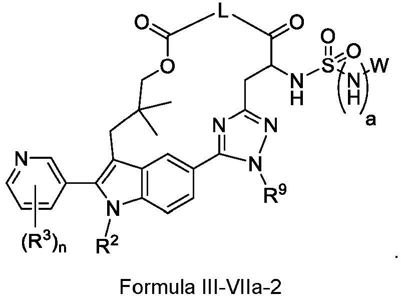

In some embodiments of Formula III, the compound, or a pharmaceutically acceptable salt thereof, has the structure of Formula III-Vlla-3: 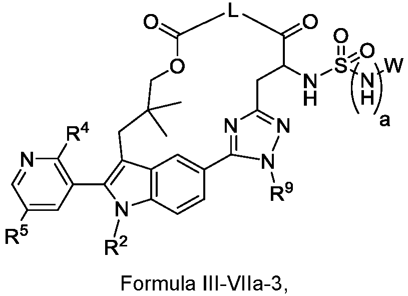 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-Vlla-4:

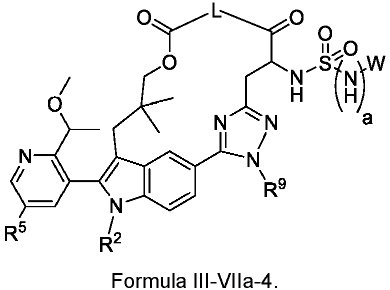

In some embodiments of Formula III, the compound, or a pharmaceutically acceptable salt thereof, has the structure of Formula III-Vlla-5: 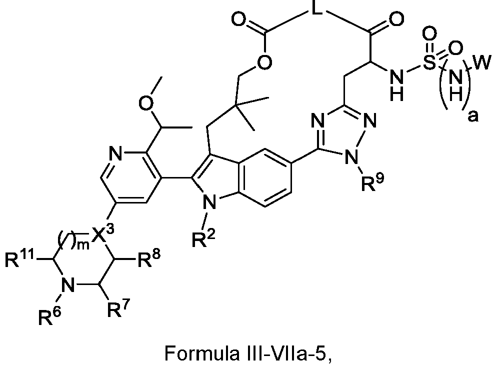
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments (e.g., of any one of Formulae VIIa, VIIa-1, VIIa-2, VIIa-3, VIIa-4, or Vlla-5), R⁹ is methyl.

In some embodiments, Y is -NHS(O)- or -NHS(O)NH-.

In some embodiments of Formula III, the compound, or a pharmaceutically acceptable salt thereof, has the structure of Formula III-Vllla: 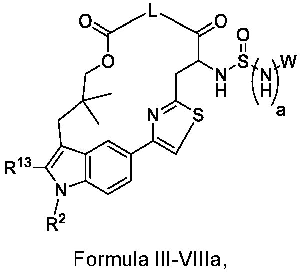 wherein a is 0 or 1.

In some embodiments of Formula III, the compound, or a pharmaceutically acceptable salt thereof, has the structure of Formula III-Vllla-1: 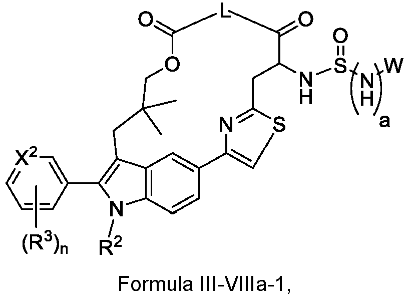
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-Vllla-2:

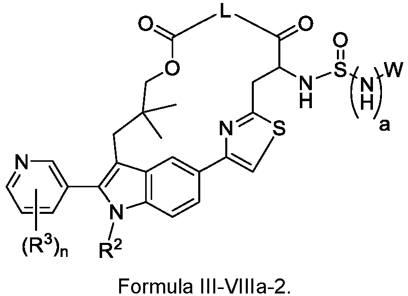

In some embodiments of Formula III, the compound, or a pharmaceutically acceptable salt thereof, has the structure of Formula III-Vllla-3: 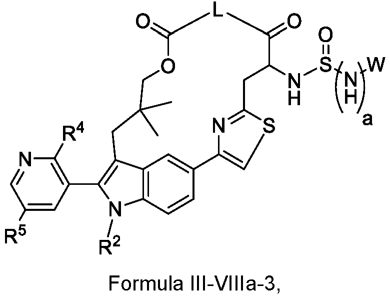 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-Vllla-4:

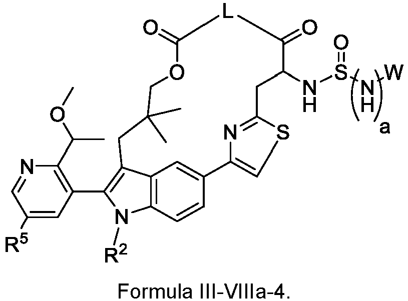

In some embodiments of Formula III, the compound, or a pharmaceutically acceptable salt thereof, has the structure of Formula III-Vllla-5: 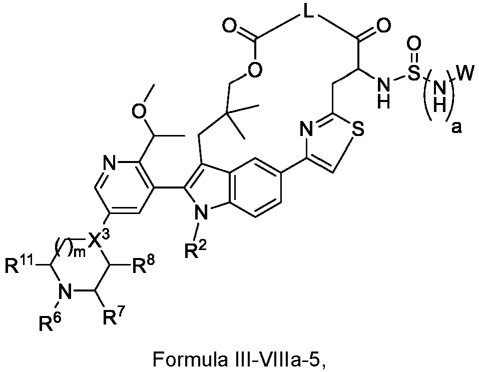
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of Formula III, the compound, or a pharmaceutically acceptable salt thereof, has the structure of Formula III-IXa: 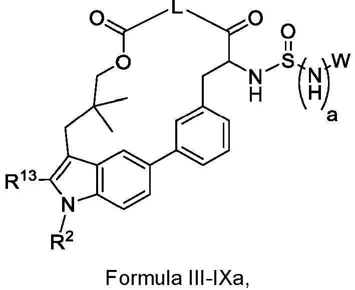 wherein a is 0 or 1.

In some embodiments of Formula III, the compound, or a pharmaceutically acceptable salt thereof, has the structure of Formula III-IXa-1: 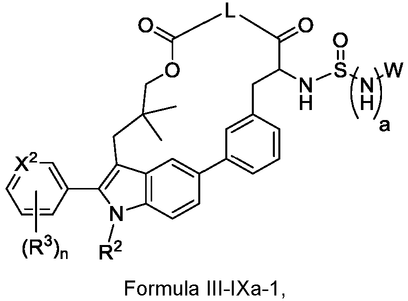
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-IXa-2:

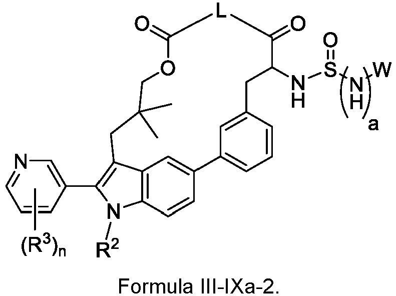

In some embodiments of Formula III, the compound, or a pharmaceutically acceptable salt thereof, has the structure of Formula III-IXa-3: 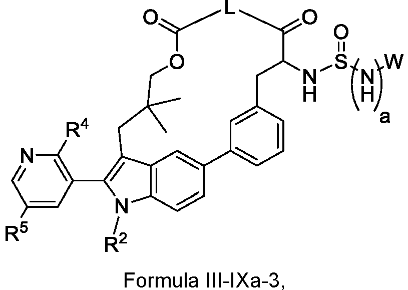 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-IXa-4:

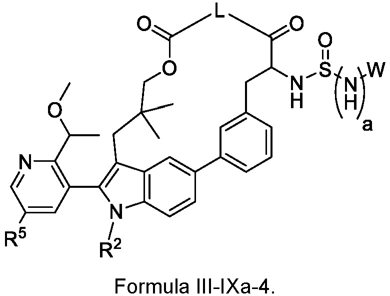

In some embodiments of Formula III, the compound, or a pharmaceutically acceptable salt thereof, has the structure of Formula III-IXa-5: 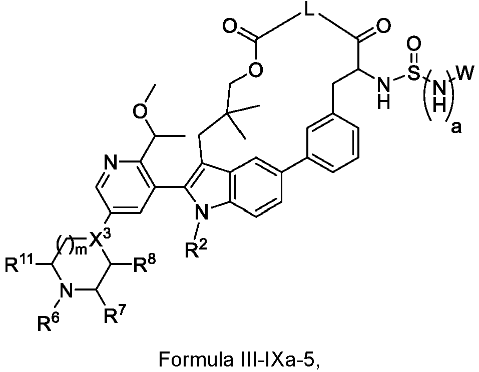
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of Formula III, the compound, or a pharmaceutically acceptable salt thereof, has the structure of Formula III-Xa: 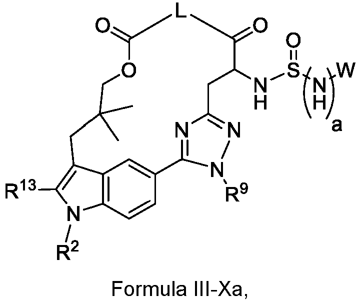 wherein a is 0 or 1.

In some embodiments of Formula III, the compound, or a pharmaceutically acceptable salt thereof, has the structure of Formula III-Xa-1: 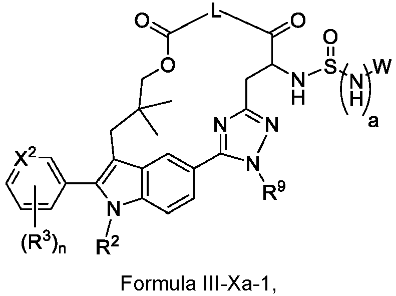
wherein X² is N or CH;
each R³ is independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl; and
n is an integer from 1 to 4.

In some embodiments of Formula III, the compound, or a pharmaceutically acceptable salt thereof, has the structure of Formula III-Xa-2:

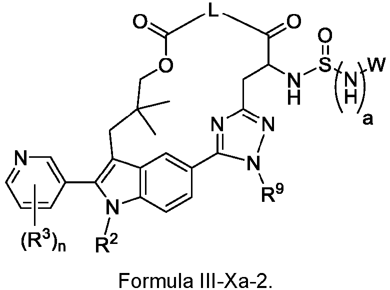

In some embodiments of Formula III, the compound, or a pharmaceutically acceptable salt thereof, has the structure of Formula III-Xa-3: 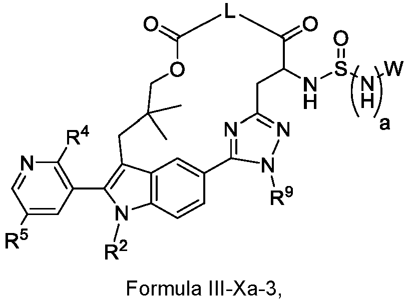 wherein R⁴ and R⁵ are each independently selected from halogen, cyano, hydroxy, optionally substituted amine, optionally substituted amido, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 11-membered heterocycloalkyl (e.g., optionally substituted 3- to 6-membered heterocycloalkyl), optionally substituted 6- to 10-membered aryl, or optionally substituted 5- to 10-membered heteroaryl.

In some embodiments of Formula III, the compound, or a pharmaceutically acceptable salt thereof, has the structure of Formula III-Xa-4:

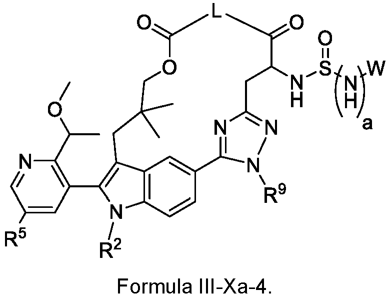

In some embodiments of Formula III, the compound, or a pharmaceutically acceptable salt thereof, has the structure of Formula III-Xa-5: 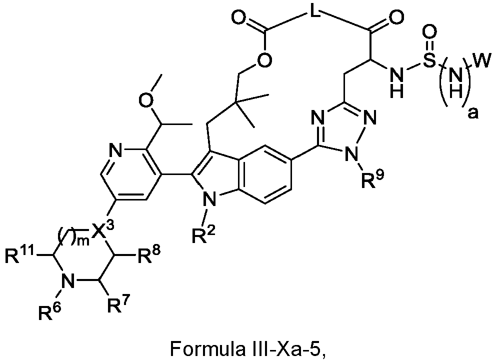
wherein X³ is N or CH;
m is 1 or 2;
R⁶, R⁷, R⁸, and R¹¹ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl; or
R⁶ and R⁷ combine with the atoms to which they are attached to form an optionally substituted 3- to 8-membered cycloalkyl or an optionally substituted 3- to 8-membered heterocycloalkyl; or
R⁷ and R⁸ combine with the atoms to which they are attached to form an optionally substituted 3- to 8-membered heterocycloalkyl; or
R⁷ and R¹¹ combine with the atoms to which they are attached to form an optionally substituted 4- to 8-membered heterocycloalkyl. In some embodiments, X³ is N. In some embodiments, m is 1. In some embodiments, R¹¹ is hydrogen. In some embodiments, X³ is N, m is 1, and R¹¹ is H.

In some embodiments of compounds of Formula III, a is 0. In some embodiments of any of the above, a is 0.

In some embodiments of compounds of Formula III, R² is optionally substituted C₁-C₆ alkyl. In some embodiments, R² is selected from -CH₂CH₃ or -CH₂CF₃. In some embodiments, R² is optionally substituted C₁-C₆ heteroalkyl optionally substituted with a 3 to 10-membered heterocycloalkyl. In some embodiments, R² is C₁-C₆ heteroalkyl 3 to 10-membered heterocycloalkyl.

In some embodiments of compounds of Formula III, W is C₁-C₄ alkyl. In some embodiments, W is:

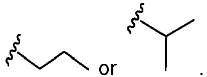

In some embodiments of compounds of Formula III, W is optionally substituted cyclopropyl, optionally substituted cyclobutyl, optionally substituted cyclopentyl, or optionally substituted cyclohexyl, optionally substituted piperidine, optionally substituted piperazine, optionally substituted pyridine, or optionally substituted phenyl.

In some embodiments of compounds of Formula III, W is optionally substituted 3 to 10-membered heterocycloalkyl, optionally substituted 3 to 10-membered cycloalkyl, optionally substituted 6 to 10-membered aryl, or optionally substituted 5 to 10-membered heteroaryl.

In some embodiments of compounds of Formula III, W is optionally substituted 3 to 10-membered heterocycloalkyl. In some embodiments, W is selected from the following, or a stereoisomer thereof: 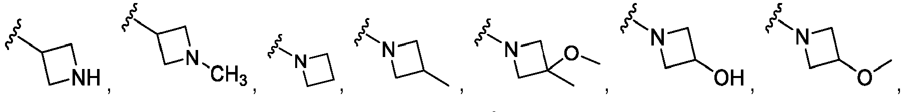 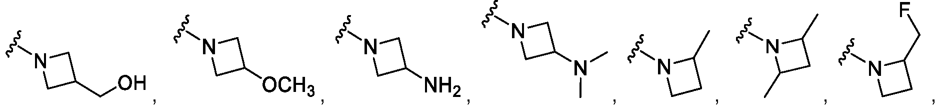 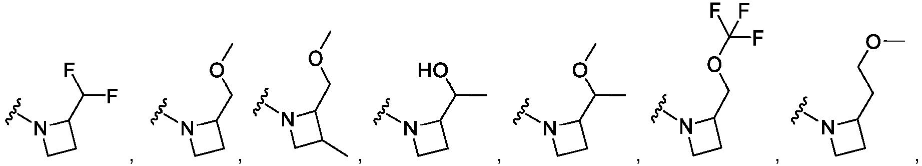 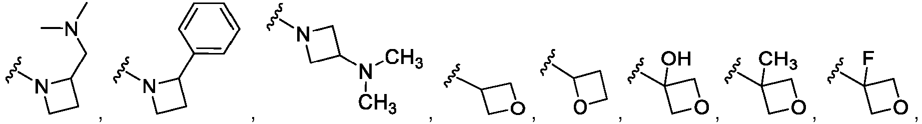 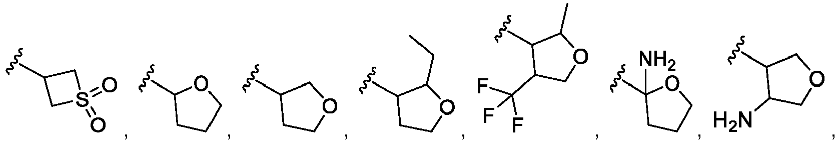 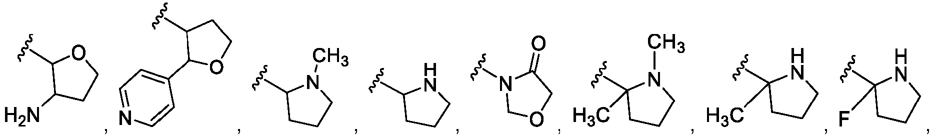 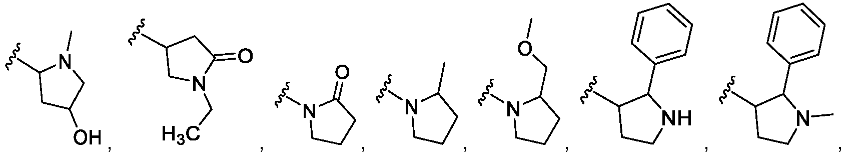 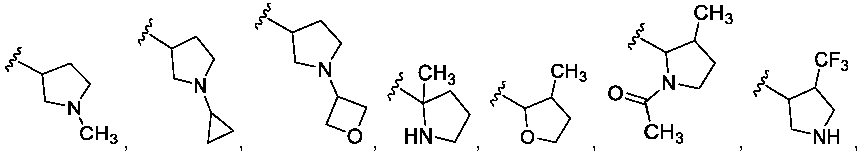 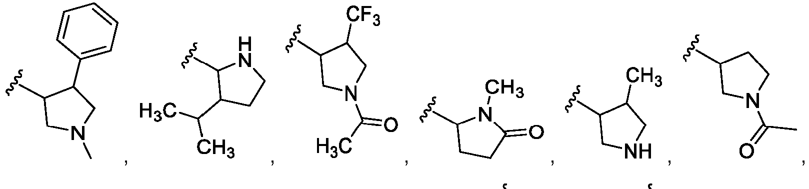 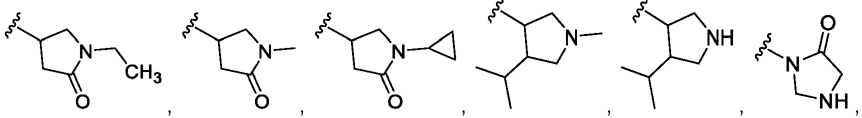 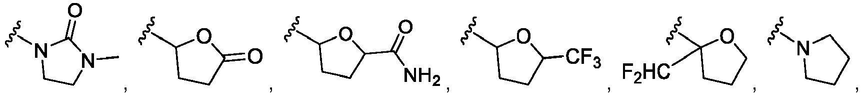 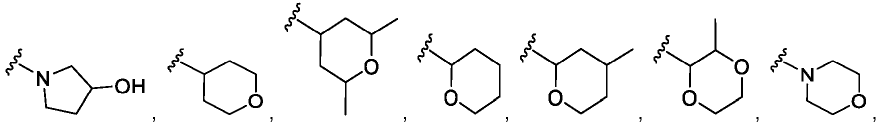 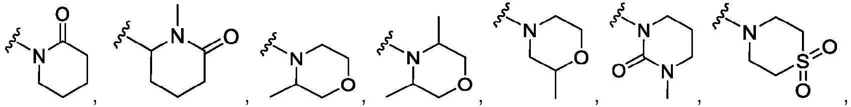 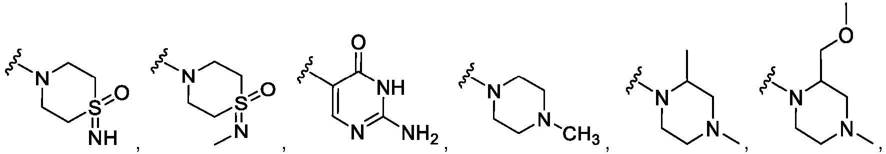 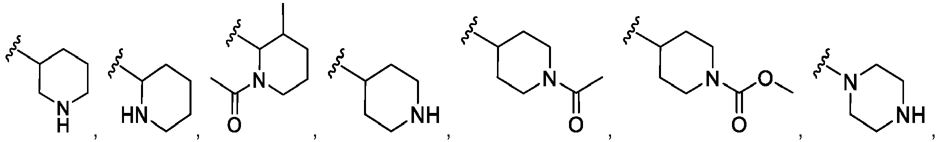 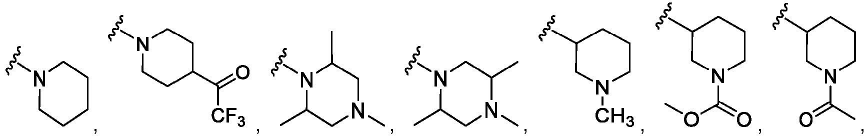 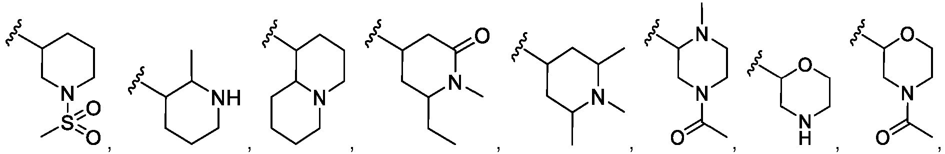 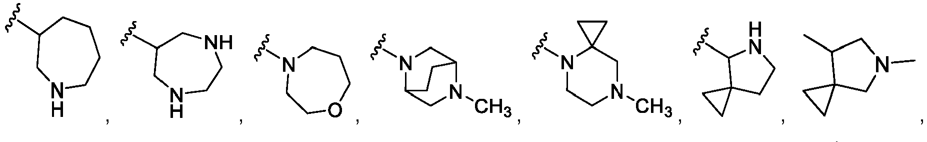 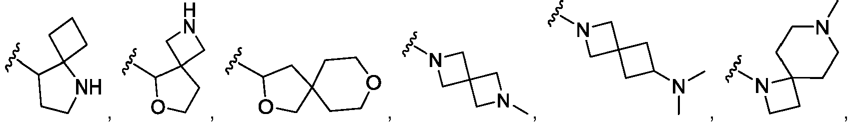 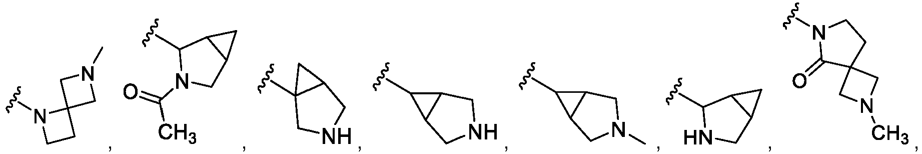 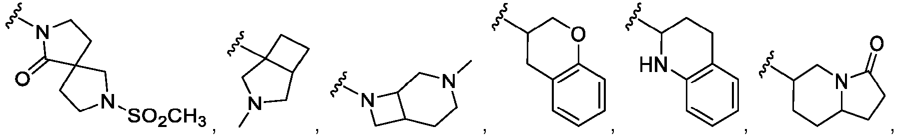 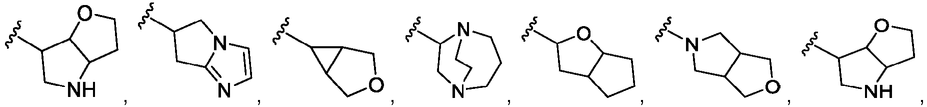 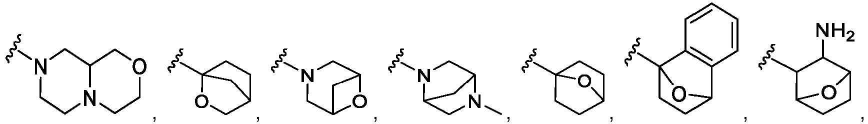 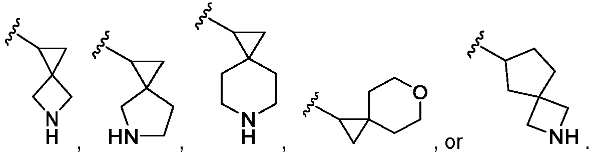 In some embodiments, W is selected from the following, or a stereoisomer thereof:

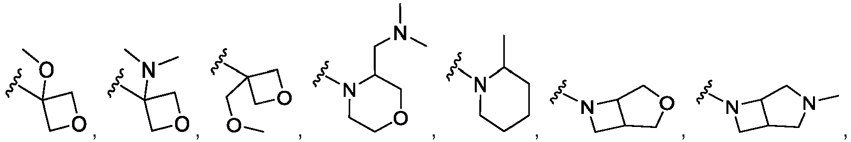

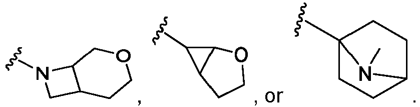

In some embodiments of any aspect described herein, W is optionally substituted 3 to 10-membered cycloalkyl. In some embodiments, W is selected from the following, or a stereoisomer thereof: 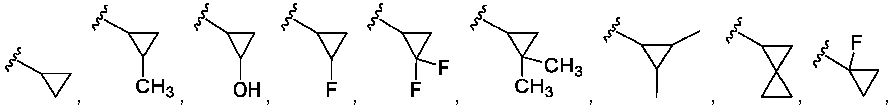 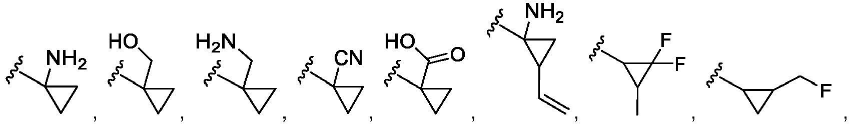 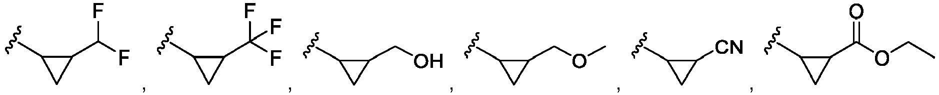 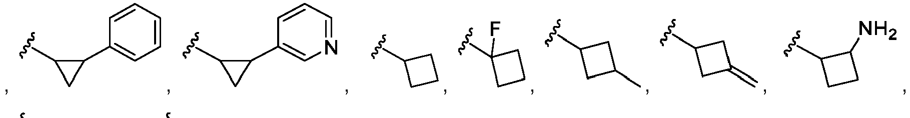 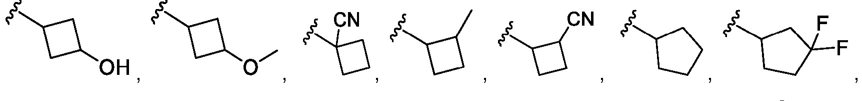 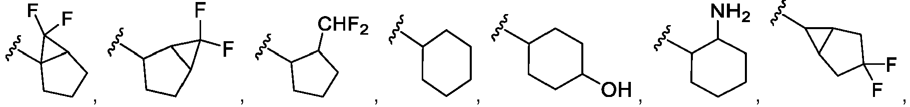 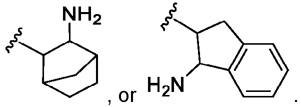 In some embodiments, W is selected from the following, or a stereoisomer thereof: 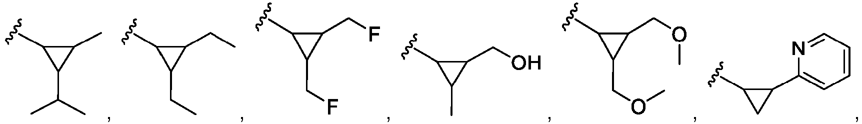 or

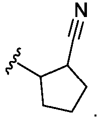

In some embodiments of compounds of Formula III, W is optionally substituted 5 to 10-membered heteroaryl. In some embodiments, W is selected from the following, or a stereoisomer thereof:

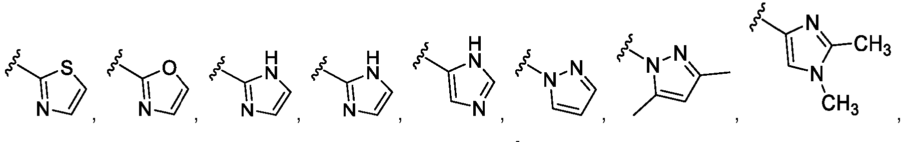

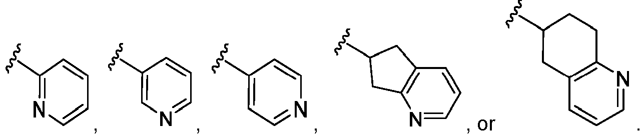

In some embodiments of compounds of Formula III, W is optionally substituted 6 to 10-membered aryl. In some embodiments, W is optionally substituted phenyl.

In some embodiments of compounds of Formula III, W is optionally substituted C₁-C₃ heteroalkyl. In some embodiments, W is selected from the following, or a stereoisomer thereof:

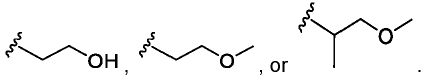

In some embodiments of compounds of Formula III or any subformula of Formula III, or a pharmaceutically acceptable salt or stereoisomer thereof, the compound is not E10 to E52 in Table 2 and those set forth in Table 5.

In some embodiments of compounds of Formula III or any subformula of Formula III, or a pharmaceutically acceptable salt or stereoisomer thereof, when A is thiazole-2,4-diyl and two R^{L1} substituents taken together form spiro cyclopropyl, then R² is not optionally substituted C₁₋₆ alkyl.

In some embodiments of compounds of formula III or any subformula of formula III, or a pharmaceutically acceptable salt or stereoisomer thereof, when A is thiazole-2,4-diyl and two R^{L2} substituents taken together form spiro cyclopropyl, then R² is not optionally substituted C₁₋₆ alkyl.

In some embodiments of compounds of formula III or any subformula of formula III, or a pharmaceutically acceptable salt or stereoisomer thereof, when A is thiazole-2,4-diyl and R^{L1} and R^{L2} taken together form optionally substituted fused cyclopropyl, then R² is not optionally substituted C₁₋₆ alkyl.

In some embodiments of compounds of formula III or any subformula of formula III, or a pharmaceutically acceptable salt or stereoisomer thereof, when A is thiazole-2,4-diyl and R^{L1} and R^{L2} taken together form a bond, then R² is not optionally substituted C₁₋₆ alkyl.

In some embodiments of compounds of formula III or any subformula of formula III, or a pharmaceutically acceptable salt or stereoisomer thereof, when A is thiazole-2,4-diyl and R^{L1} and R^{L2} taken together form fused cyclobutyl, then R² is not optionally substituted C₁₋₆ alkyl.

In some embodiments of compounds of formula III or any subformula of formula III, or a pharmaceutically acceptable salt or stereoisomer thereof, when A is thiazole-2,4-diyl and R^{L1} and R^{L3} together with the atoms to which they are attached form bridged cyclobutyl, then R² is not optionally substituted C₁₋₆ alkyl.

In some embodiments of compounds of formula III or any subformula of formula III, or a pharmaceutically acceptable salt or stereoisomer thereof, when A is thiazole-2,4-diyl and R^{L1} and R^{L3} together with the atoms to which they are attached form bridged cyclobutyl, then W is not 2,3-dimethylcyclopropyl or optionally substituted cyclopropyl.

In some embodiments, the disclosure features a compound, or pharmaceutically acceptable salt thereof, having the structure of Formula IV: 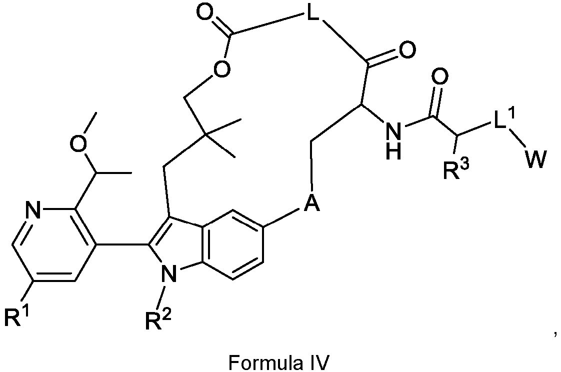
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
L¹ is a linker;
L has the structure of Formula VIIa or VIIb: 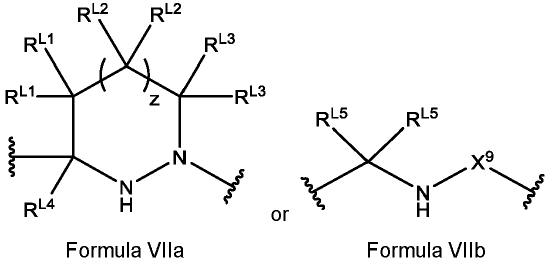
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 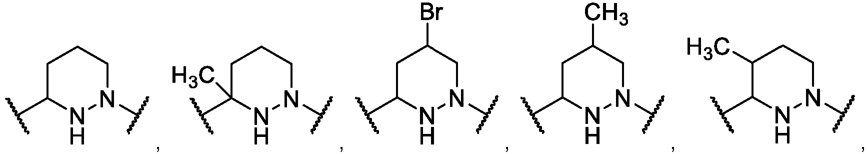 or 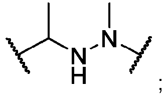
W is a cross-linking group comprising a vinyl ketone, vinyl sulfone, ynone, or an alkynyl sulfone;
R¹ is hydrogen, optionally substituted 3- to 10-membered heterocycloalkyl, or optionally substituted C₁-C₆ heteroalkyl;
R² is optionally substituted C₁-C₆ alkyl; and
R³ is optionally substituted C₁-C₆ alkyl or optionally substituted C₁-C₃ heteroalkyl.

In some embodiments, provided herein is a compound, or pharmaceutically acceptable salt thereof, having the structure of Formula IV-la:

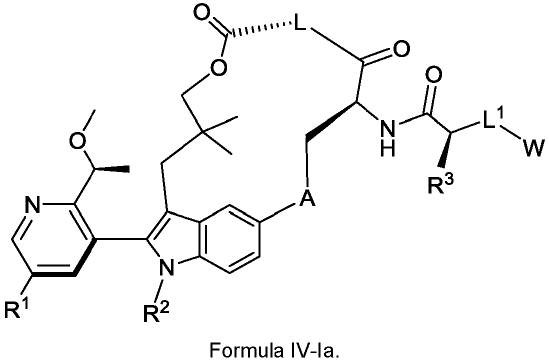

In some embodiments of compounds of the present invention, A is optionally substituted thiazole, optionally substituted oxazole, optionally substituted morpholino, optionally substituted pyrrolidinyl, optionally substituted pyridyl, optionally substituted azetidinyl, optionally substituted pyrazinyl, optionally substituted pyrimidine, optionally substituted piperidinyl, optionally substituted oxadiazole, optionally substituted thiadiazole, optionally substituted triazole, optionally substituted thiomorpholino, or optionally substituted phenyl.

In some embodiments, the disclosure features a compound, or pharmaceutically acceptable salt thereof, of structural Formula IV-II-1:

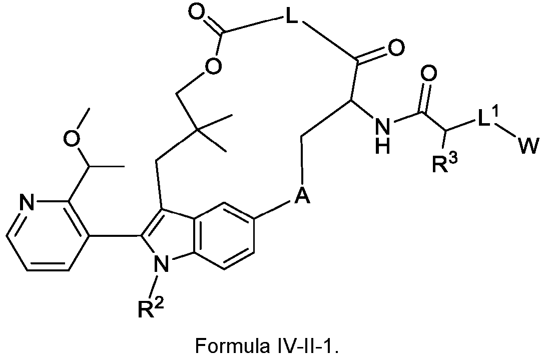

In some embodiments, a compound having the structure of Formula IV-II-2 is provided, or a pharmaceutically acceptable salt thereof: 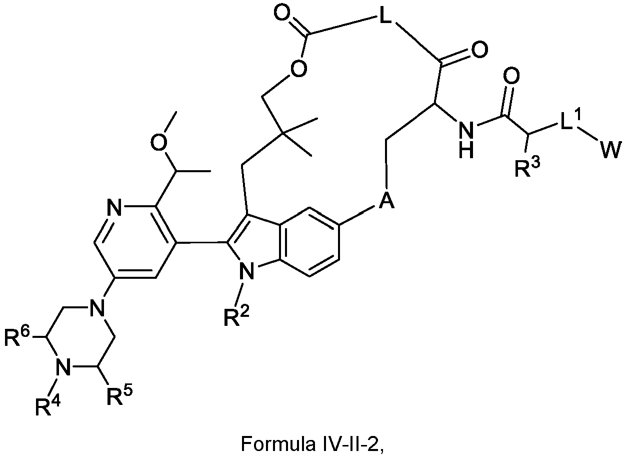
wherein R⁴, R⁵, and R⁶ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered heterocycloalkyl; or
R⁴ and R⁵ combine with the atoms to which they are attached to form an optionally substituted 3- to 8-membered cycloalkyl or an optionally substituted 3- to 8-membered heterocycloalkyl; or
R⁴ and R⁶ combine with the atoms to which they are attached to form an optionally substituted 3- to 8-membered cycloalkyl or an optionally substituted 3- to 8-membered heterocycloalkyl.

In some embodiments, a compound of the present invention has the structure of Formula IV-II-3, or a pharmaceutically acceptable salt thereof:

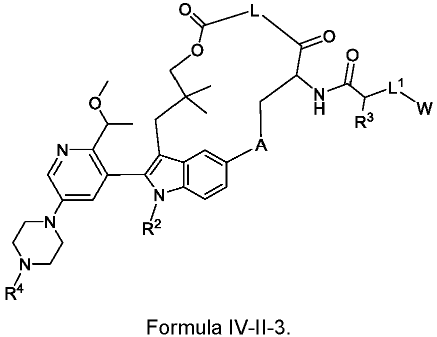

In some embodiments, a compound of the present invention has the structure of Formula IV-II-4, or a pharmaceutically acceptable salt thereof:

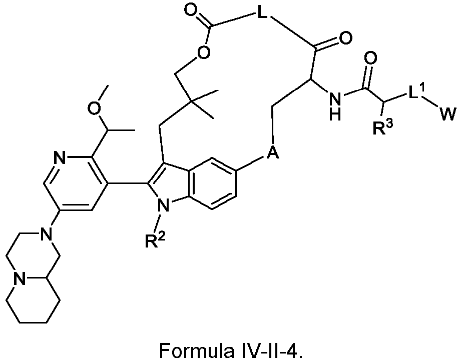

In some embodiments, a compound of the present invention has the structure of Formula IV-II-4b, or a pharmaceutically acceptable salt thereof:

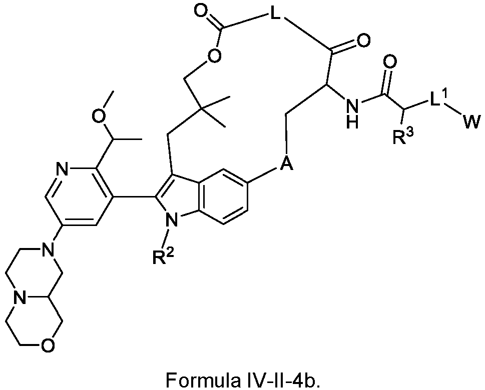

In some embodiments of a compound of the present invention, R² is:

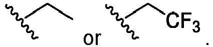

In some embodiments of a compound of the present invention, R³ is optionally substituted C₁-C₆ alkyl. In some embodiments, R³ is:

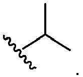

In some embodiments of a compound of the present invention, R³ is optionally substituted C₁-C₃ heteroalkyl. In some embodiments, R³ is:

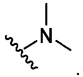

In some embodiments of a compound of the present invention, A is optionally substituted 5- to 10-membered heteroarylene. In some embodiments, A is:

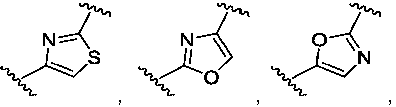

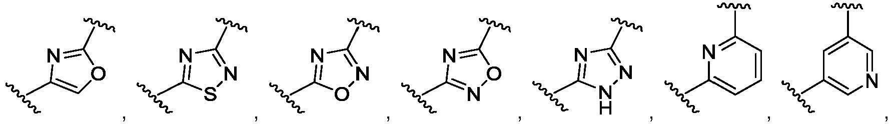

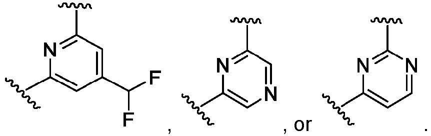

In some embodiments of a compound of the present invention, A is optionally substituted phenyl. In some embodiments, A is:

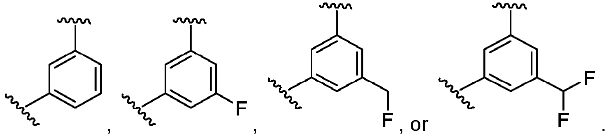

In some embodiments of a compound of the present invention, A is optionally substituted 3- to 6-membered heterocycloalkylene. In some embodiments, A is selected from the following, or a stereoisomer thereof:

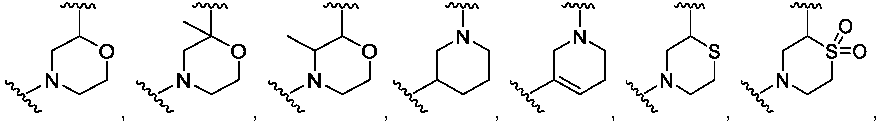

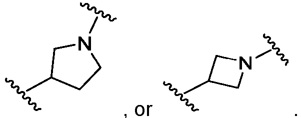

In some embodiments, A is selected from the following, or a stereoisomer thereof:

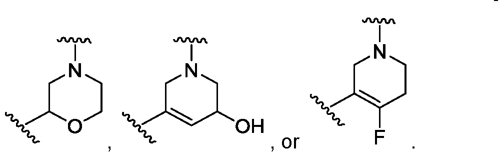

In some embodiments of a compound of the present invention, the linker is the structure of Formula IV-III:

A¹-(B¹)_{f}-(C¹)_{g}-(B²)ₕ-(D¹)-(B³)ᵢ-(C²)ⱼ-(B⁴)ₖ-A² Formula IV-III,

wherein A¹ is a bond between the linker and CH(R³); A² is a bond between A and the linker; B¹, B², B³, and B⁴ each, independently, is selected from optionally substituted C₁-C₂ alkylene, optionally substituted C₁-C₃ heteroalkylene, O, S, and NR^{N}; each R^{N} is, independently, hydrogen, optionally substituted C₁-C₄ alkyl, optionally substituted C₂-C₄ alkenyl, optionally substituted C₂-C₄ alkynyl, optionally substituted 3- to 14-membered heterocycloalkyl, optionally substituted 6- to 10-membered aryl, or optionally substituted C₁-C₇ heteroalkyl; C¹ and C² are each, independently, selected from carbonyl, thiocarbonyl, sulphonyl, or phosphoryl; f, g, h, i, j, and k are each, independently, 0 or 1; and D¹ is optionally substituted C₁-C₁₀ alkylene, optionally substituted C₂-C₁₀ alkenylene, optionally substituted C₂-C₁₀ alkynylene, optionally substituted 3- to 14-membered heterocycloalkylene, optionally substituted 5- to 10-membered heteroarylene, optionally substituted 3-to 8-membered cycloalkylene, optionally substituted 6- to 10-membered arylene, optionally substituted C₂-C₁₀ polyethylene glycolene, or optionally substituted C₁-C₁₀ heteroalkylene, or a chemical bond linking A¹-(B¹)_{f}-(C¹)_{g}-(B²)ₕ- to -(B³)ᵢ-(C²)ⱼ-(B⁴)ₖ-A².

In some embodiments of a compound of the present invention, the linker is or comprises a cyclic moiety. In some embodiments, the linker has the structure of Formula IV-Illa: 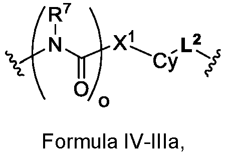
wherein o is 0 or 1;
R⁷ is hydrogen, optionally substituted C₁-C₆ alkyl, optionally substituted 3- to 8-membered cycloalkylene, or optionally substituted 3- to 8-membered heterocycloalkylene;
X¹ is absent, optionally substituted C₁-C₄ alkylene, O, NCH₃, or optionally substituted C₁-C₄ heteroalkylene;
Cy is optionally substituted 3- to 8-membered cycloalkylene, optionally substituted 3- to 12-membered heterocycloalkylene, optionally substituted 6- to 10-membered arylene, or optionally substituted 5- to 10-membered heteroarylene; and
L² is absent, -SO₂-, -NH-, optionally substituted C₁-C₄ alkylene, optionally substituted C₁-C₄ heteroalkylene, or optionally substituted 3- to 6-membered heterocycloalkylene.

In some embodiments, the linker is selected from, or a stereoisomer thereof: 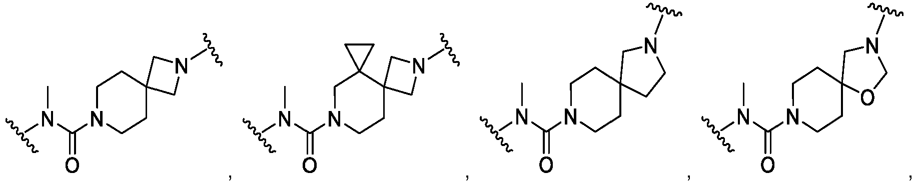 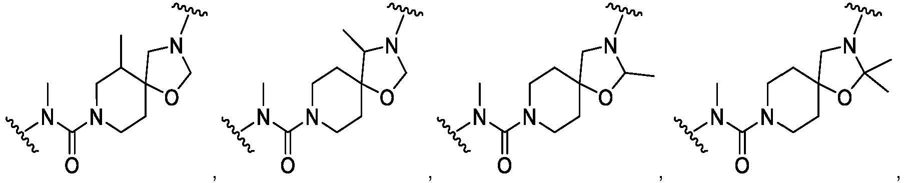 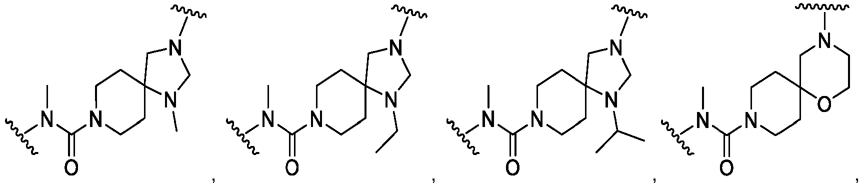 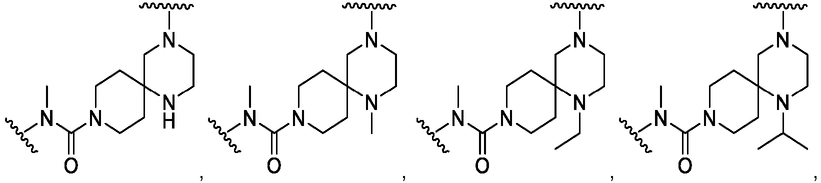 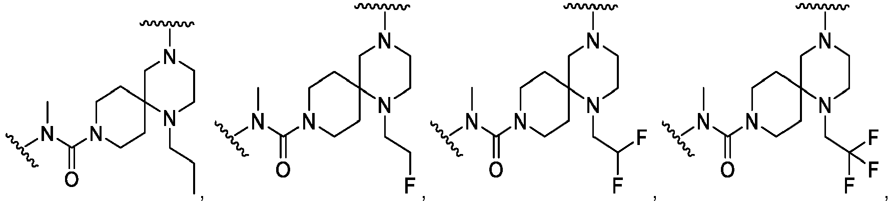 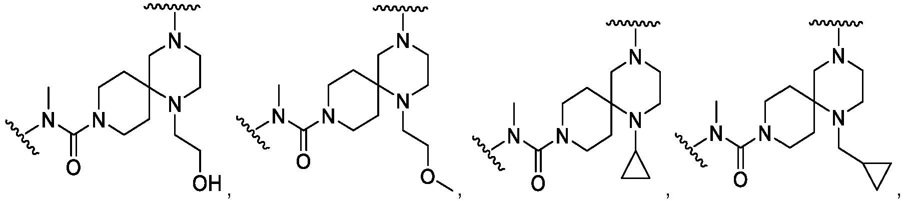 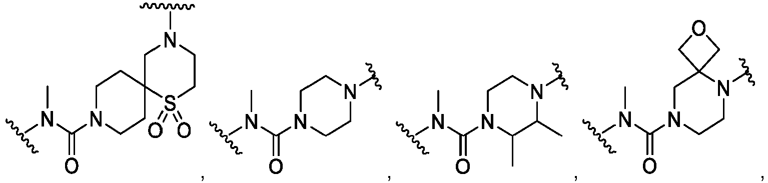 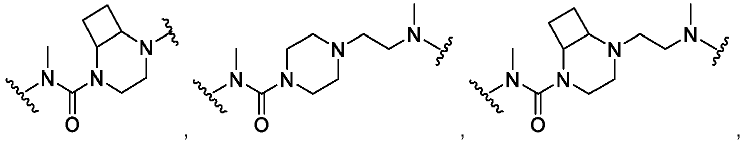 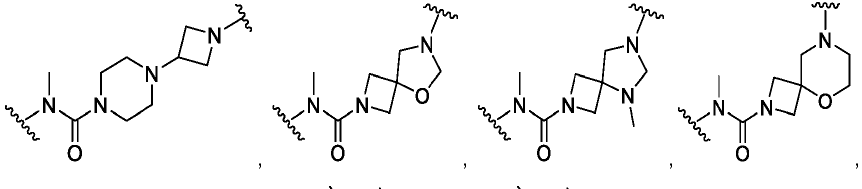 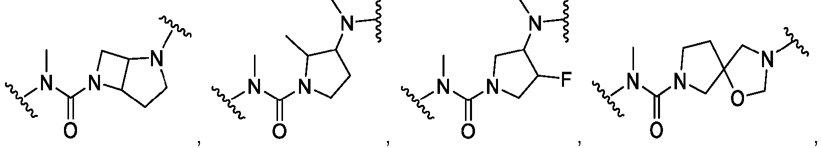 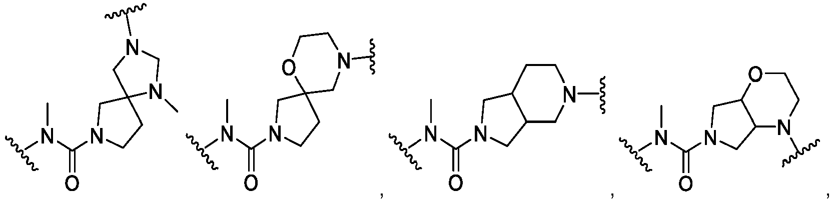 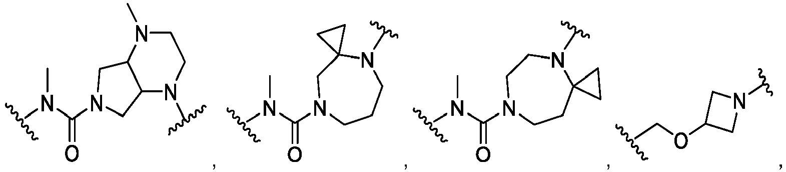 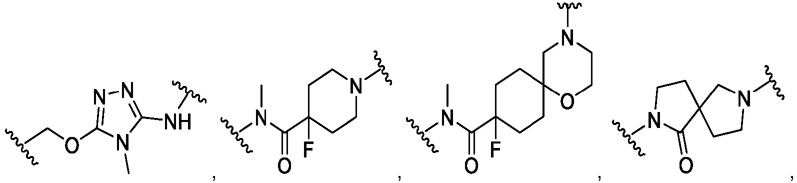 or

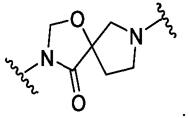

In some embodiments, the linker is selected from, or a stereoisomer thereof:

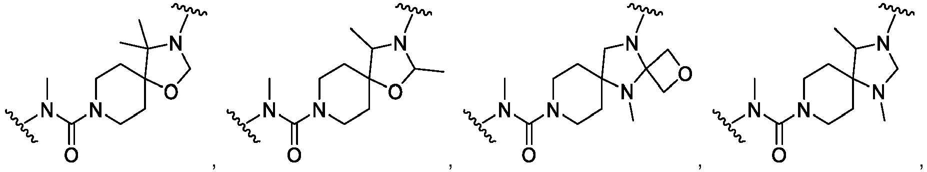

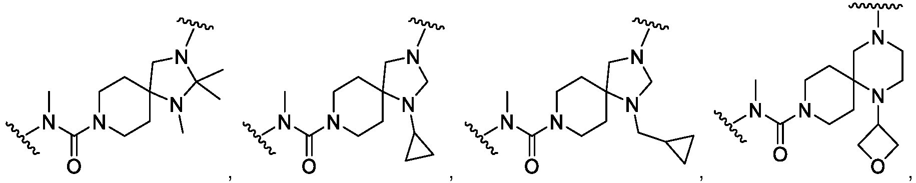

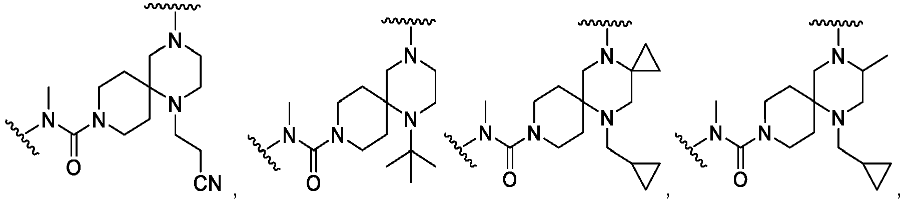

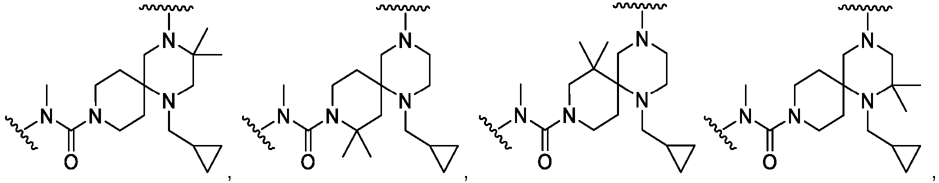

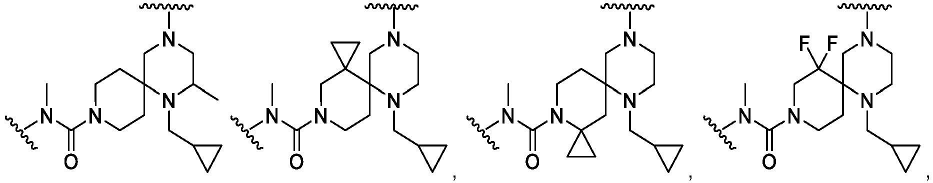

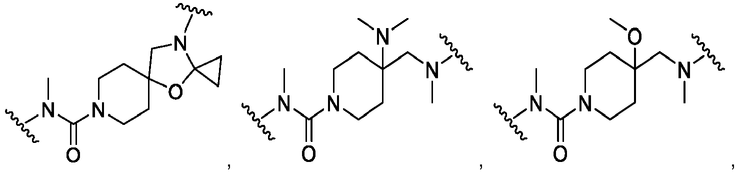

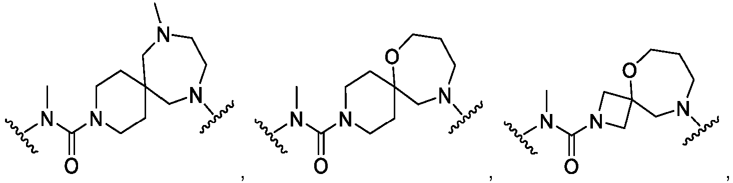

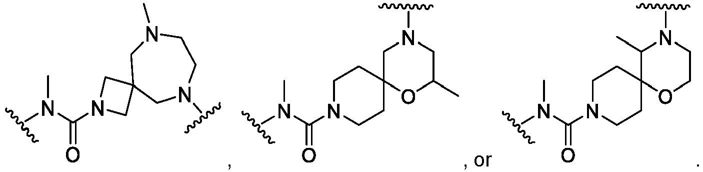

In some embodiments, a compound of the present invention has the structure of Formula IV-II-5, or a pharmaceutically acceptable salt thereof: 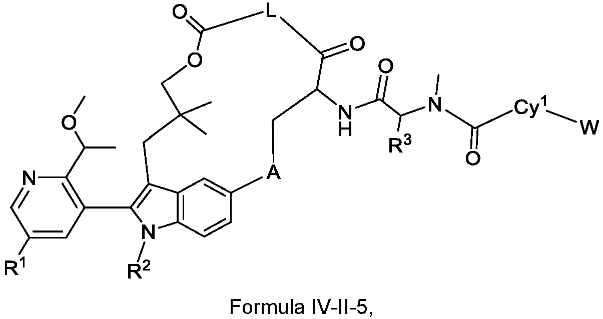
wherein Cy¹ is optionally substituted spirocyclic 8- to 11-membered heterocycloalkylene or optionally substituted bicyclic 7- to 9-membered heterocycloalkylene; and
wherein W comprises a vinyl ketone or a vinyl sulfone.

In some embodiments, Cy¹ is optionally substituted spirocyclic 10- to 11-membered heterocycloalkylene.

In some embodiments, a compound of the present invention has the structure of Formula IV-II-5a: 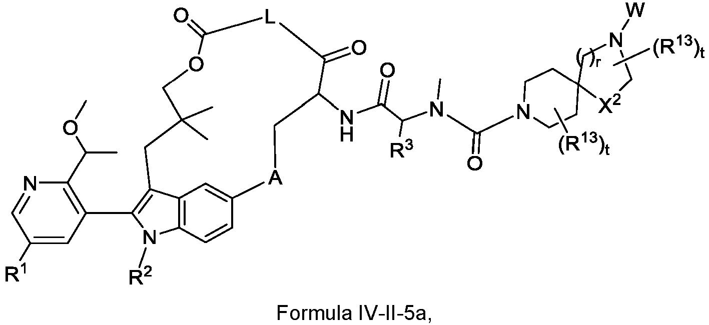
wherein X² is O, C(R¹¹)₂, NR¹², S, or SO₂;
r is 1 or 2;
each t is, independently, 0, 1, or 2;
R¹¹ and R¹² are each, independently, hydrogen, optionally substituted C₁-C₄ alkyl, optionally substituted C₂-C₄ heteroalkyl, or optionally substituted 3- to 5-membered cycloalkyl; and
each R¹³ is, independently, -CH₃.

In some embodiments, a compound of the present invention has the structure of Formula IV-II-5b: 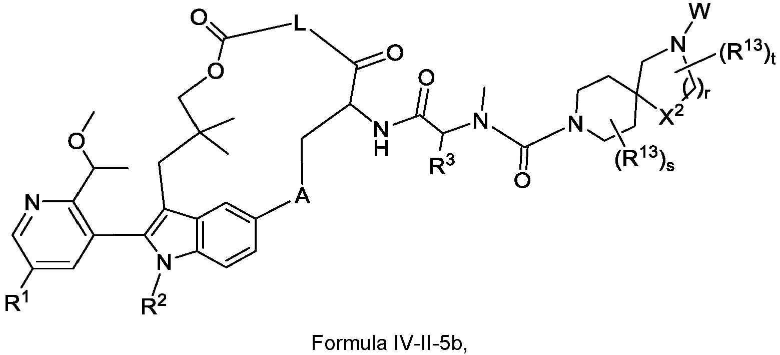
wherein X² is O, C(R¹¹)2, NR¹², S, or SO₂;
r is 1 or 2;
each t is, independently, 0, 1, or 2;
R¹¹ and R¹² are each, independently, hydrogen, optionally substituted C₁-C₄ alkyl, optionally substituted C₂-C₄ heteroalkyl, optionally substituted 3- to 6- membered heterocycloalkyl, or optionally substituted 3- to 5-membered cycloalkyl; and
each R¹³ is, independently, -CH₃, F, or two R¹³ attached to the same atom combine with the atom to which they are attached to form an optionally substituted C₃-C₆ cycloalkyl, or two R¹³ attached to the same atom combine with the atom to which they are attached to form an optionally substituted 3- to 6-membered heterocycloalkyl.

In some embodiments, a compound of the present invention has the structure of Formula IV-II-5c:

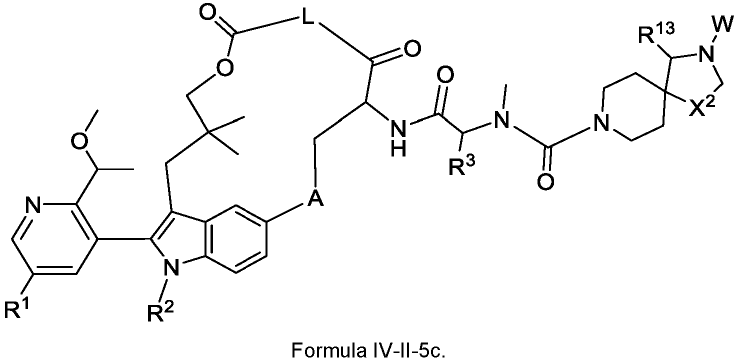

In some embodiments, a compound of the present invention has the structure of Formula IV-II-5d:

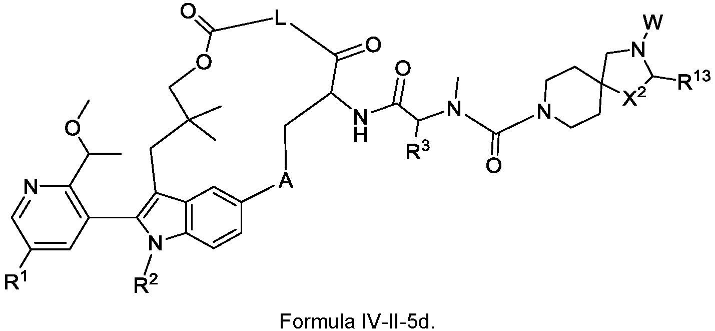

In some embodiments, a compound of the present invention has the structure of Formula IV-II-5e:

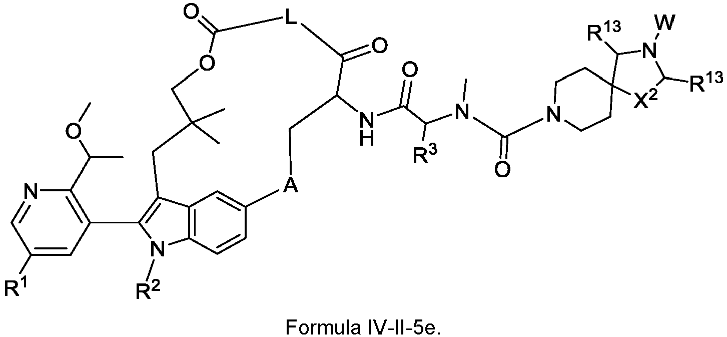

In some embodiments, r is 1. In some embodiments, r is 2. In some embodiments, X² is O. In some embodiments, X² is S. In some embodiments, X² is SO₂.

In some embodiments, X² is NR¹². In some embodiments, R¹² is selected from, or a stereoisomer thereof:
-CH₃, 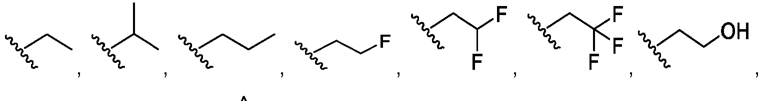 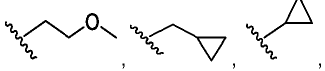 or -H. In some embodiments, R¹² is selected from, or a stereoisomer thereof:

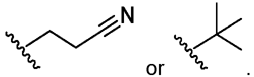

In some embodiments, X² is C(R¹¹)₂. In some embodiments, each R¹¹ is hydrogen.
In some embodiments of a compound of the present invention, W is a cross-linking group comprising a vinyl ketone. In some embodiments, W has the structure of Formula IV-IVa: 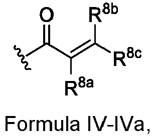 wherein R^{8a}, R^{8b}, and R^{8c} are, independently, hydrogen, -CN, halogen, or -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl. In some embodiments, W is selected from, or a stereoisomer thereof: 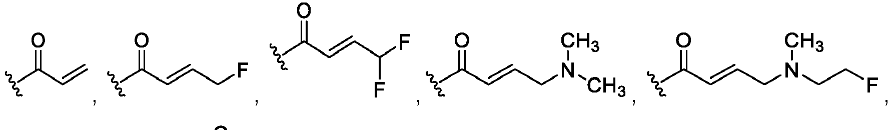 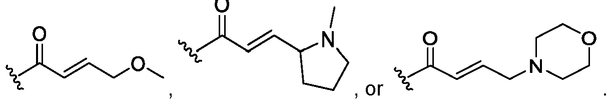 In some embodiments, W is selected from, or a stereoisomer thereof: 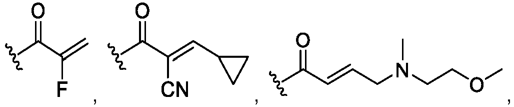 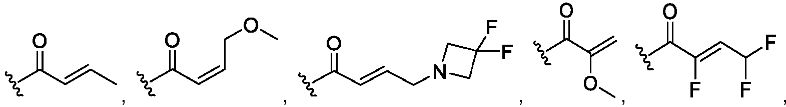 and

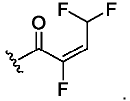

In some embodiments of a compound of the present invention, W is a cross-linking group comprising a vinyl sulfone. In some embodiments, W has the structure of Formula IV-IVc: 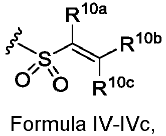 wherein R^{10a}, R^{10b}, and R^{10c} are, independently, hydrogen, -CN, or -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated heterocycloalkyl. In some embodiments, W is:

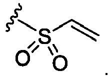

In some embodiments of a compound of the present invention, W is a cross-linking group comprising an ynone. In some embodiments, W has the structure of Formula IV-IVb: 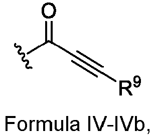 wherein R⁹ is hydrogen, -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or a 4 to 7-membered saturated cycloalkyl, or a 4 to 7-membered saturated heterocycloalkyl. In some embodiments, W is selected from:

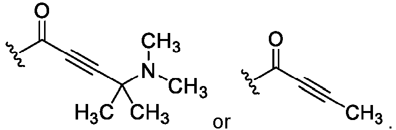

In some embodiments, a compound of the present invention has the structure of Formula IV-II-6: 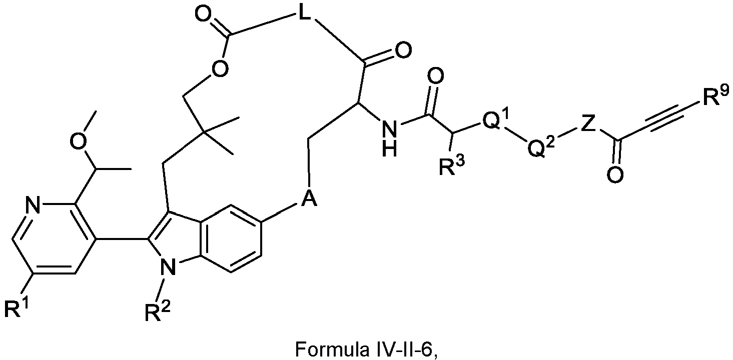
wherein Q¹ is CH₂, NR^{N}, or O;
Q² is CO, NR^{N}, or O; and
Z is optionally substituted 3- to 6-membered heterocycloalkylene or optionally substituted 5-to 10-membered heteroarylene; or
wherein Q¹-Q²-Z is an optionally substituted 9- to 10-membered spirocyclic heterocycloalkylene.
In some embodiments, a compound of the present invention has the structure of Formula IV-II-6a: 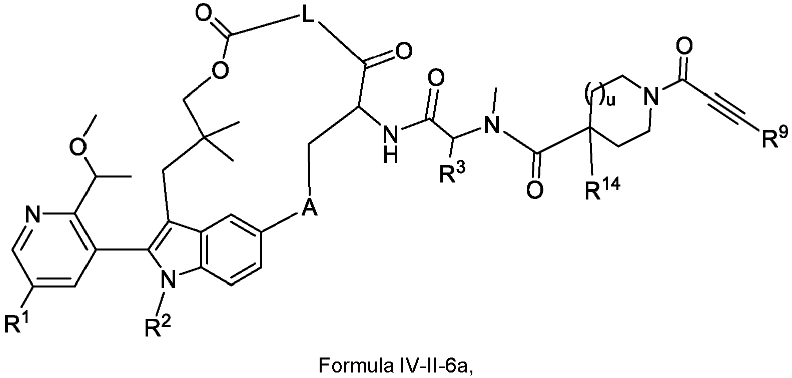
wherein R¹⁴ is fluoro, hydrogen, or C₁-C₃ alkyl; and
u is 0 or 1.

In some embodiments, R¹⁴ is fluoro and u is 1. In some embodiments, R¹⁴ is hydrogen and u is 0.

In some embodiments, a compound of the present invention has the structure of Formula IV-II-6b:

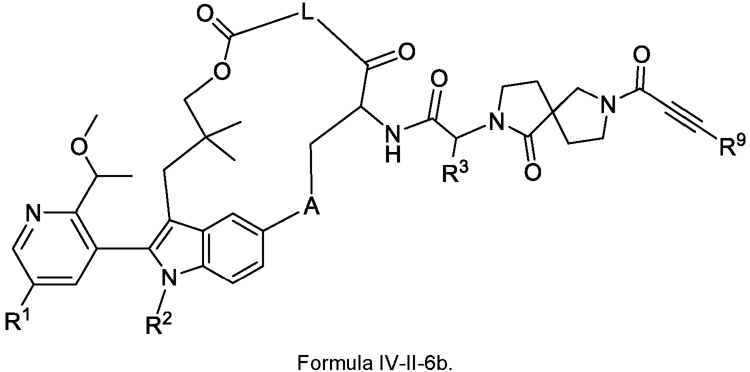

In some embodiments, a compound of the present invention has the structure of Formula IV-II-6c:

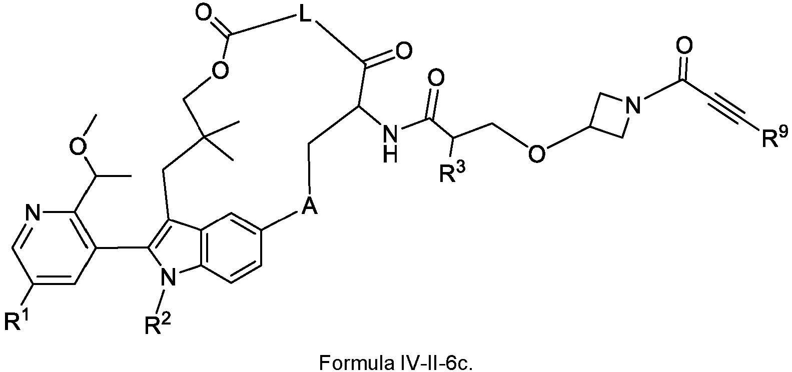

In some embodiments of compounds of Formula IV or a pharmaceutically acceptable salt or stereoisomer thereof, compound is not:

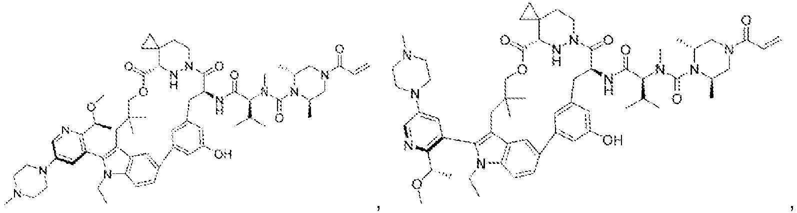

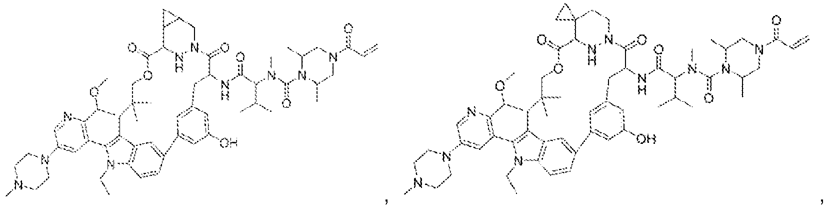

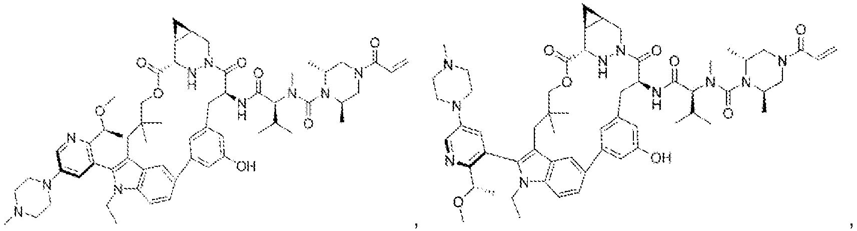

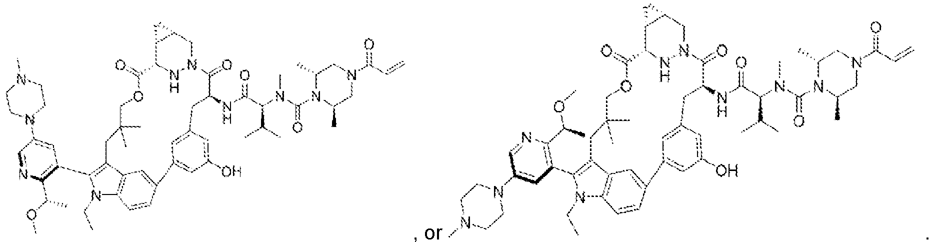

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, two R^{L1} substituents taken together form spiro cyclopropyl, and W is vinyl ketone, then R² is not ethyl.

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, R^{L1} and R^{L2} taken together form fused cyclopropyl, and W is vinyl ketone, then R² is not ethyl.

In some embodiments of compounds of Formula IV or any subformula of Formula IV, or a pharmaceutically acceptable salt or stereoisomer thereof, when A is 1-hydroxy-benzene-3,5-diyl or optionally substituted phenylene and two R^{L1} substituents taken together form spiro cyclopropyl, then W is not vinyl ketone.

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, R^{L1} and R^{L2} taken together form fused cyclopropyl, then W is not vinyl ketone.

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, W is vinyl ketone and two R^{L1} substituents taken together form spiro cyclopropyl or spiro cycloalkyl, then L¹ is other than

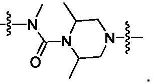

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, W is vinyl ketone and R^{L1} and R^{L2} taken together form fused cyclopropyl or fused cycloalkyl, then L¹ is not

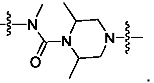

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, W is vinyl ketone and two R^{L1} substituents taken together form spiro cyclopropyl or spiro cycloalkyl, then L¹ is not 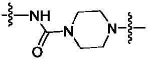 optionally substituted with 1, 2 or 3 independently selected C₁₋₆ alkyl.

In some embodiments of compounds of Formula IV or any subformula of Formula IV, or a pharmaceutically acceptable salt or stereoisomer thereof, when A is 1-hydroxy-benzene-3,5-diyl or optionally substituted phenylene, W is vinyl ketone and two R^{L1} and R^{L2} taken together form fused cyclopropyl or fused cycloalkyl, then L¹ is not 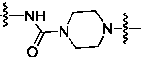 optionally substituted with 1, 2 or 3 independently selected C₁₋₆ alkyl.

In an aspect, the disclosure features a compound, or pharmaceutically acceptable salt thereof, having the structure of Formula V: 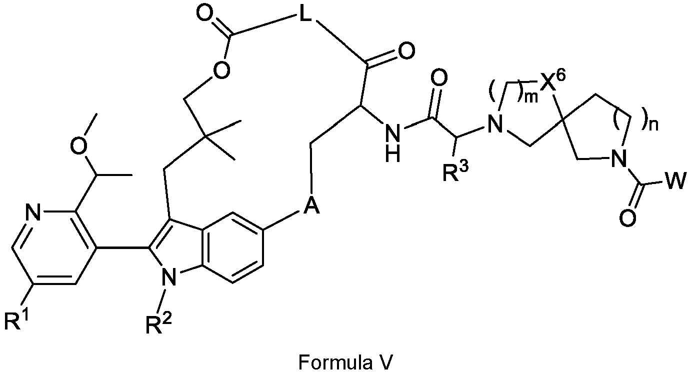 wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
L has the structure of Formula VIIa or Vllb: 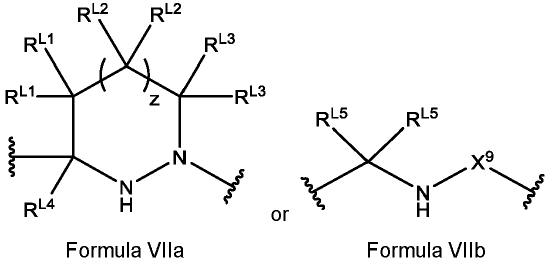
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 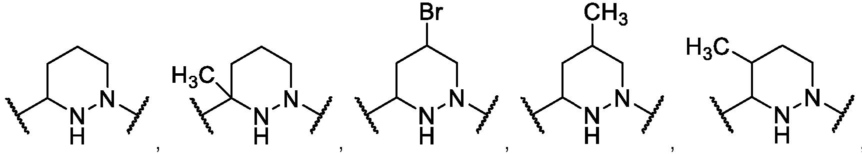 or 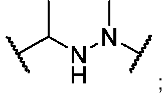
W is a cross-linking group comprising an aziridine, an epoxide, a carbodiimide, an oxazoline, a thiazoline, a chloroethyl urea, a chloroethyl thiourea, a chloroethyl carbamate, a chloroethyl thiocarbamate, a trifluoromethyl ketone, a boronic acid, a boronic ester, an N-ethoxycarbonyl-2-ethoxy-1,2-dihydroquinoline (EEDQ), an iso-EEDQ or other EEDQ derivative, an oxazolium, or a glycal;
X⁶ is CH₂ or O;
m is 1 or 2;
n is 0 or 1;
R¹ is hydrogen or optionally substituted 3- to 10-membered heterocycloalkyl; and
R² is optionally substituted C₁-C₆ alkyl.

In some embodiments, W is a cross-linking group comprising an aziridine or an epoxide.

In some embodiments, A is optionally substituted thiazole, optionally substituted oxazole, optionally substituted morpholino, optionally substituted pyrrolidinyl, optionally substituted piperidinyl, or optionally substituted phenyl.

In some embodiments, a compound of the present invention has the structure of Formula V-la, or a pharmaceutically acceptable salt thereof:

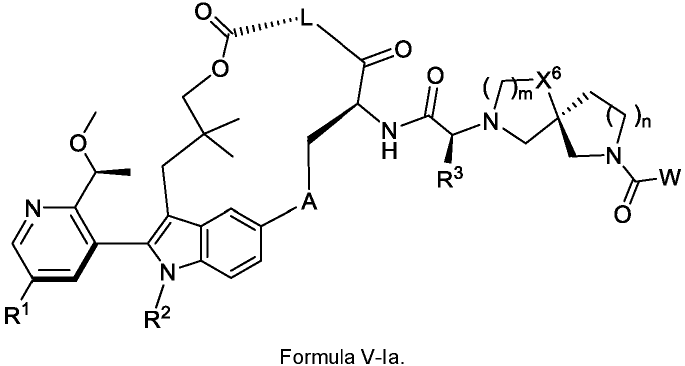

In some embodiments, a compound of the present invention has the structure of Formula V-II-1, or a pharmaceutically acceptable salt thereof:

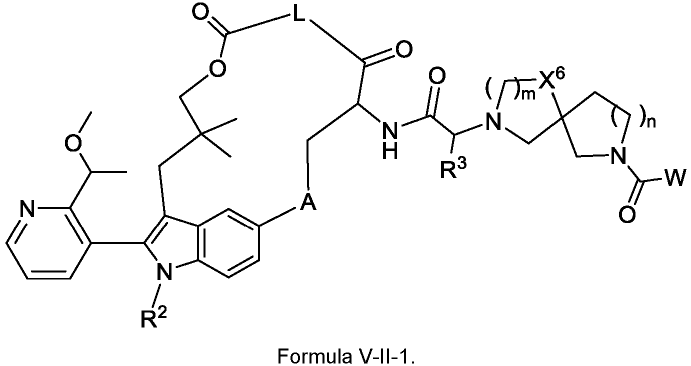

In some embodiments, a compound of the present invention has the structure of Formula V-II-2, or a pharmaceutically acceptable salt thereof: 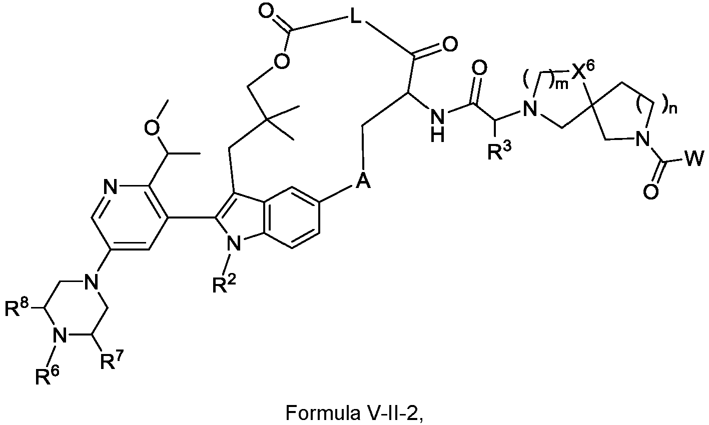
wherein R⁶, R⁷, and R⁸ are each independently selected from hydrogen, optionally substituted C₁-C₆ alkyl, optionally substituted C₁-C₆ heteroalkyl, optionally substituted 3- to 6-membered cycloalkyl, optionally substituted 3- to 6-membered heterocycloalkyl; or
R⁶ and R⁷ combine with the atoms to which they are attached to form an optionally substituted 3- to 8-membered cycloalkyl or an optionally substituted 3- to 8-membered heterocycloalkyl; or
R⁶ and R⁸ combine with the atoms to which they are attached to form an optionally substituted 3- to 8- membered cycloalkyl or an optionally substituted 3- to 8-membered heterocycloalkyl.

In some embodiments, a compound of the present invention has the structure of Formula V-II-3, or a pharmaceutically acceptable salt thereof:

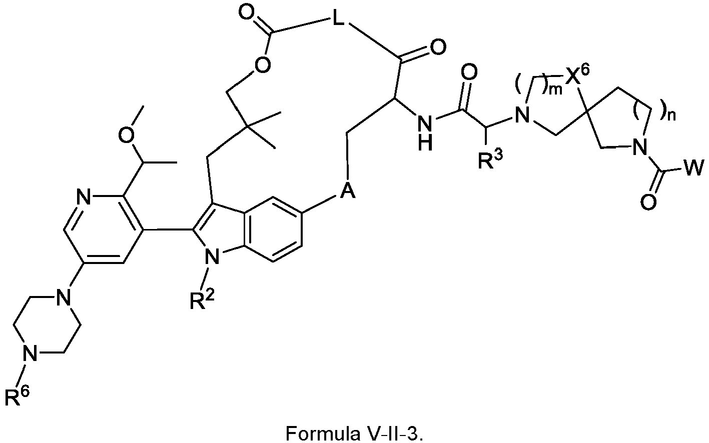

In some embodiments, a compound of the present invention has the structure of Formula V-II-4, or a pharmaceutically acceptable salt thereof: 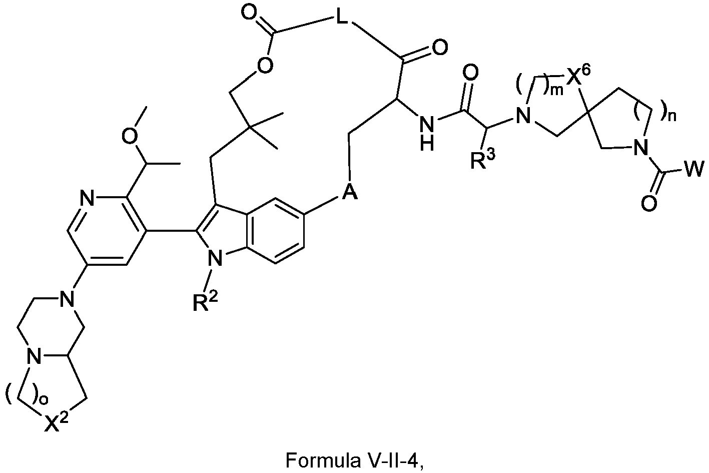
wherein X² is CH₂ or O; and
o is 1 or 2.

In some embodiments of a compound of the present invention, X² is CH₂. In some embodiments, o is 1. In some embodiments, o is 2.

In some embodiments of a compound of the present invention, X⁶ is O. In some embodiments, o is 1. In some embodiments, o is 2.

In some embodiments of a compound of the present invention, R² is:

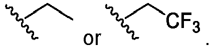

In some embodiments of a compound of the present invention, R³ is optionally substituted C₁-C₆ alkyl. In some embodiments, R³ is:

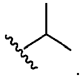

In some embodiments of a compound of the present invention, R³ is or optionally substituted 3- to 6-membered cycloalkyl. In some embodiments, R³ is:

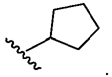

In some embodiments of a compound of the present invention, A is optionally substituted 5- to 10-membered heteroarylene. In some embodiments, A is:

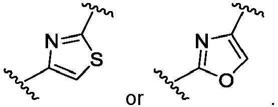

In some embodiments of a compound of the present invention, A is optionally substituted phenyl. In some embodiments, A is:

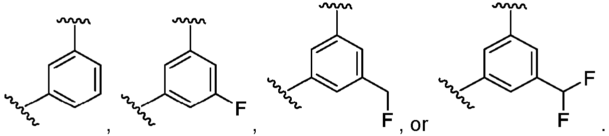

In some embodiments of a compound of the present invention, A is optionally substituted 3- to 6-membered heterocycloalkylene. In some embodiments, A is selected from the following, or a stereoisomer thereof:

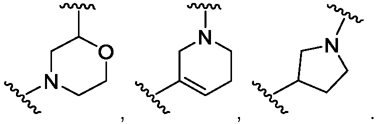

In some embodiments of a compound of the present invention, m is 1. In some embodiments, n is 1. In some embodiments, X¹ is CH₂. In some embodiments, X⁶ is O. In some embodiments, m is 1, n is 1, and X⁶ is CH₂. In some embodiments, m is 1, n is 1, and X⁶ is O.

In some embodiments of a compound of the present invention, m is 2. In some embodiments, X⁶ is CH₂. In some embodiments, n is 1. In some embodiments, n is 0. In some embodiments, m is 2, X⁶ is CH₂, and n is 1. In some embodiments, m is 2 and X⁶ is O. In some embodiments, m is 2, X⁶ is O, and n is 1. In some embodiments, m is 2, X⁶ is O, and n is 0.

In some embodiments of a compound of the present invention, W comprises an aziridine. In some embodiments, W comprises an optionally substituted cyclopropyl-aziridinyl moiety.
In some embodiments, W is selected from the following, or a stereoisomer thereof:

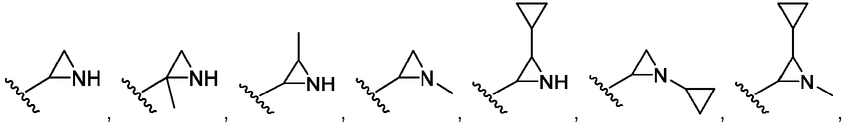

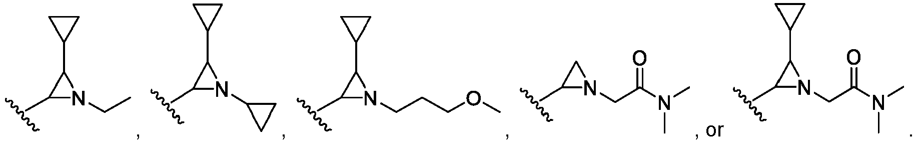

In some embodiments of a compound of the present invention, W comprises an epoxide. In some embodiments, W is selected from the following, or a stereoisomer thereof: 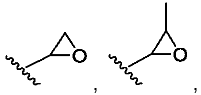 or 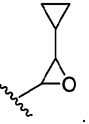 In some embodiments, the disclosure features a compound, or pharmaceutically acceptable salt thereof, having the structure of Formula VI: 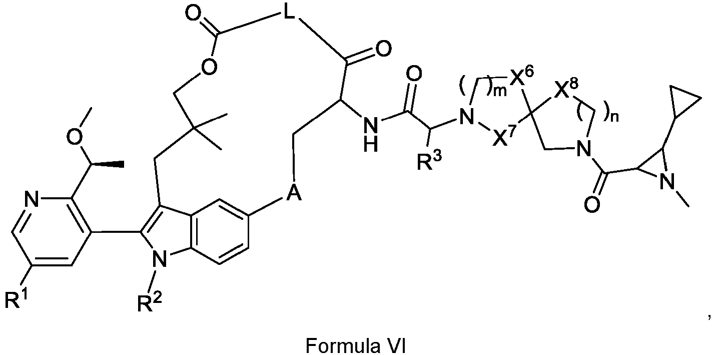
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
L has the structure of Formula VIIa or Vllb: 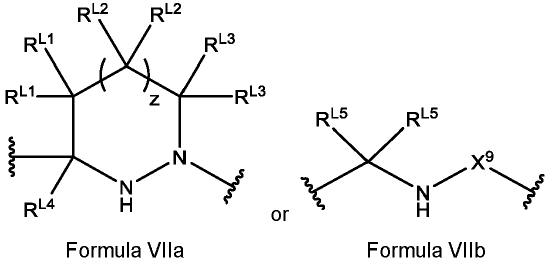
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
X⁶, X⁷, and X⁸ are each independently selected from CH₂, CHF, CF₂, C=O, or O;
m is 1 or 2;
n is 0 or 1;
R¹ is hydrogen, optionally substituted C₁-C₆ heteroalkyl, or optionally substituted 3- to 10-membered heterocycloalkyl;
R² is optionally substituted C₁-C₆ alkyl; and
R³ is optionally substituted C₁-C₆ alkyl, optionally substituted C₁-C₆ heteroalkyl, optionally substituted 3- to 6-membered cycloalkyl, or optionally substituted heterocycloalkyl,
and wherein each hydrogen is independently, optionally, isotopically enriched for deuterium.

In some embodiments, a compound of the present invention has the structure of Formula VI-Ia, Formula VI-Ib, Formula VI-Ic, or a pharmaceutically acceptable salt thereof: 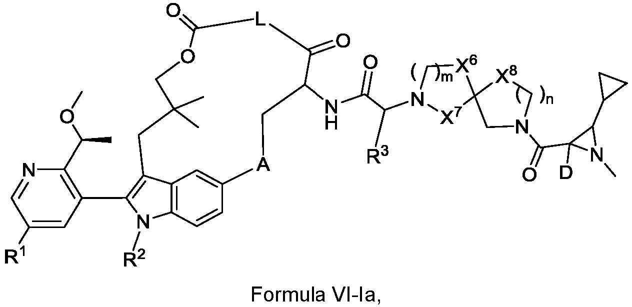 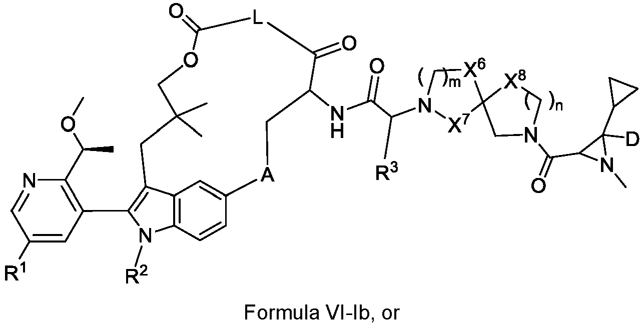 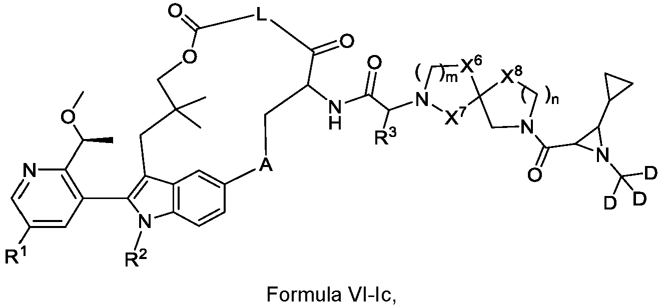 wherein each D indicates a hydrogen having an isotopic enrichment factor for deuterium of at least 5.

In some embodiments, a compound of the present invention has the structure of Formula VI-II, or a pharmaceutically acceptable salt thereof: 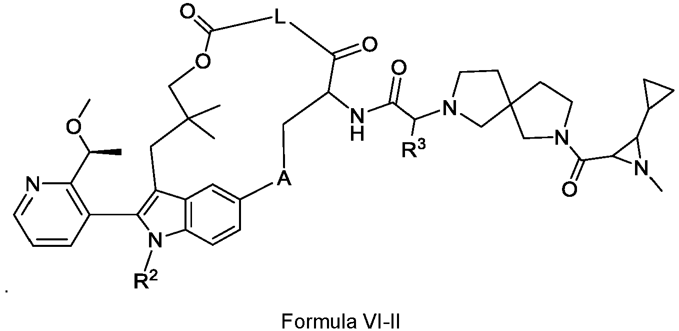
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
R² is optionally substituted C₁-C₆ alkyl; and
R³ is optionally substituted C₁-C₆ alkyl, optionally substituted 3- to 6-membered cycloalkyl, or optionally substituted heterocycloalkyl,
and wherein each hydrogen is independently, optionally, isotopically enriched for deuterium.

In some embodiments, a compound of the present invention has the structure of Formula VI-V, or a pharmaceutically acceptable salt thereof: 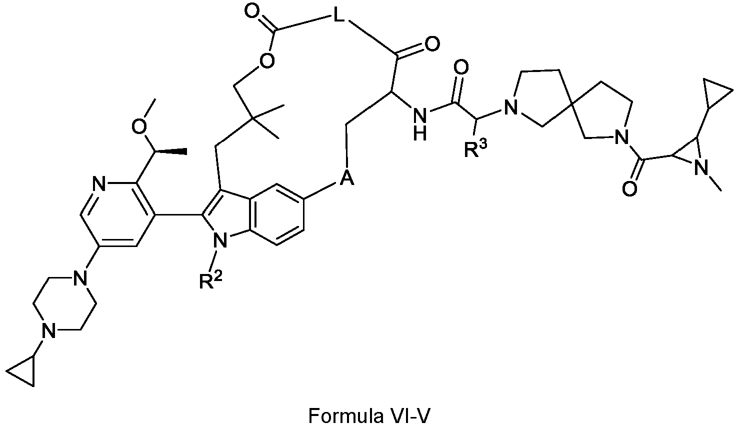
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
R² is optionally substituted C₁-C₆ alkyl; and
R³ is optionally substituted C₁-C₆ alkyl, optionally substituted 3- to 6-membered cycloalkyl, or optionally substituted heterocycloalkyl,
and wherein each hydrogen is independently, optionally, isotopically enriched for deuterium.

In some embodiments, a compound of the present invention has the structure of Formula VI-VI, or a pharmaceutically acceptable salt thereof: 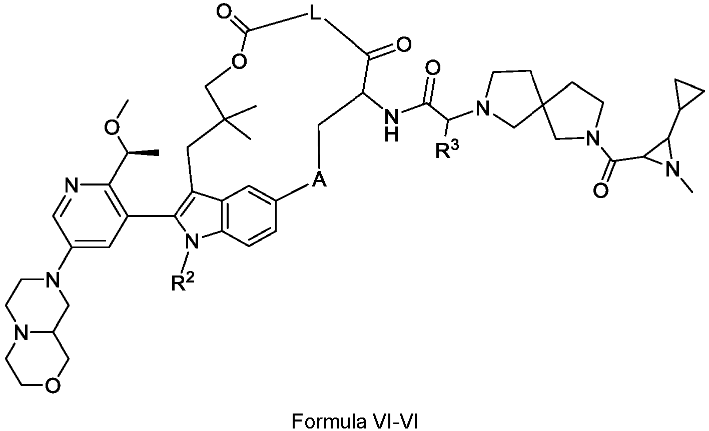
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
R² is optionally substituted C₁-C₆ alkyl; and
R³ is optionally substituted C₁-C₆ alkyl, optionally substituted 3- to 6-membered cycloalkyl, or optionally substituted heterocycloalkyl,
and wherein each hydrogen is independently, optionally, isotopically enriched for deuterium.

In some embodiments, a compound of the present invention has the structure of Formula VI-VII, or a pharmaceutically acceptable salt thereof: 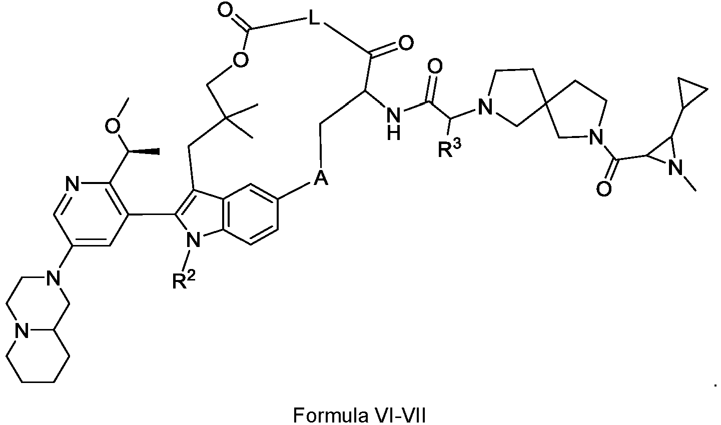
wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
R² is optionally substituted C₁-C₆ alkyl; and
R³ is optionally substituted C₁-C₆ alkyl, optionally substituted 3- to 6-membered cycloalkyl, or optionally substituted heterocycloalkyl,
and wherein each hydrogen is independently, optionally, isotopically enriched for deuterium.

In some embodiments, a compound of the present invention has the structure of Formula VIVa, Formula VI-Vb, Formula VI-Vc, or a pharmaceutically acceptable salt thereof: 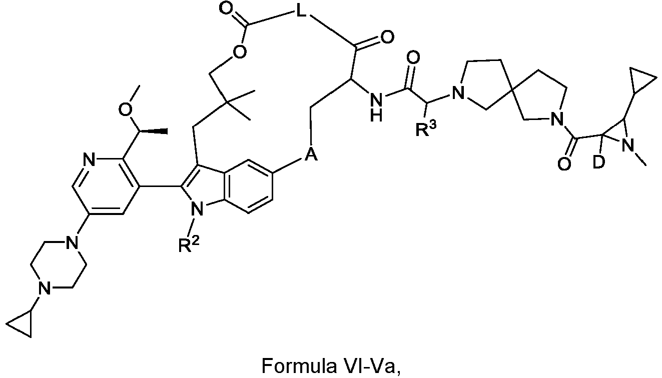 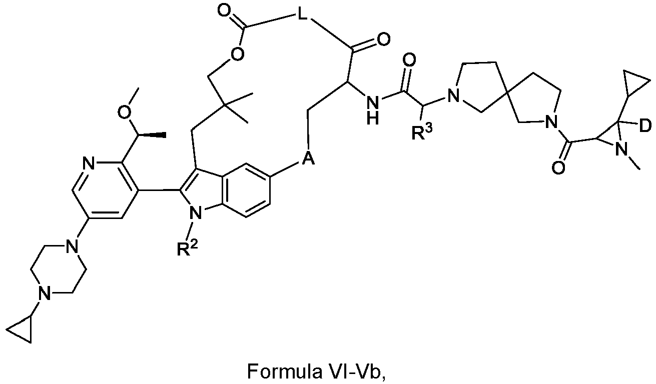 or 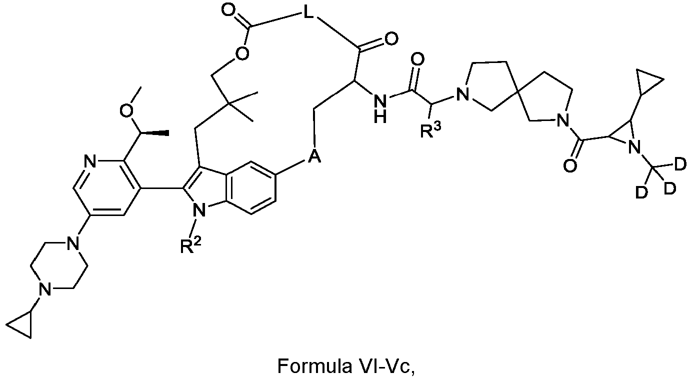 wherein each D indicates a hydrogen having an isotopic enrichment factor for deuterium of at least 5.

In some embodiments, a compound of the present invention has the structure of Formula VI-Vd, Formula VI-Ve, Formula VI-Vf, or a pharmaceutically acceptable salt thereof: 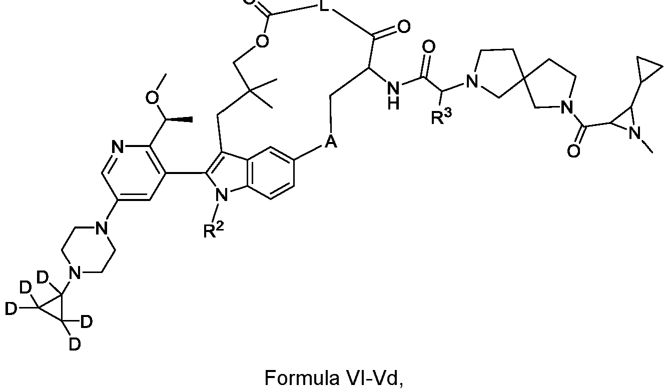 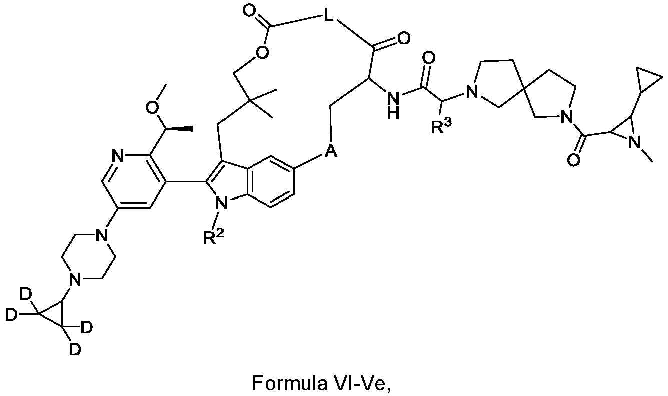 or 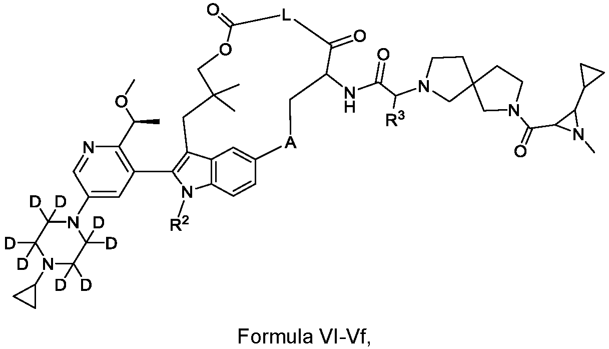 wherein each D indicates a hydrogen having an isotopic enrichment factor for deuterium of at least 5.

In some embodiments, the disclosure provides compounds having Formula VIIIa or a pharmaceutically acceptable salt or stereoisomer thereof: 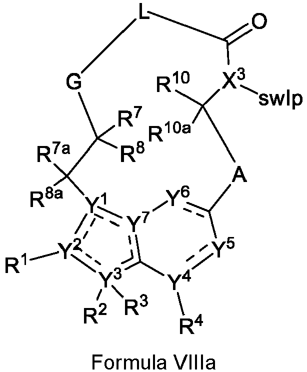 wherein the dotted lines represent zero, one, two, three, or four non-adjacent double bonds;
L has the structure of Formula VIIa or Vllb: 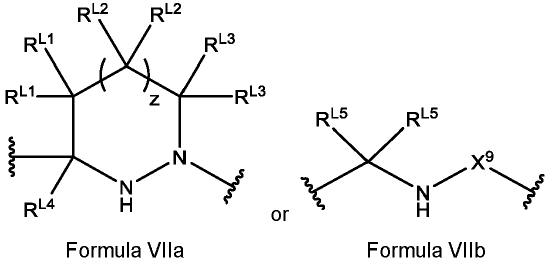
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 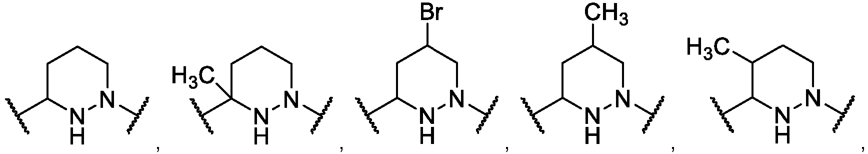 or 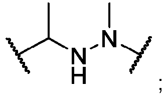
A is optionally substituted C₂-C₄ alkylene, optionally substituted C₁-C₄ heteroalkylene, or optionally substituted C₂-C₄ alkenylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3 to 8-membered heteroarylene;
swlp (Switch I/P-loop) is an organic moiety that non-covalently binds to both the Switch I binding pocket and residues 12 or 13 of the P-loop of a Ras protein;
X³ is N or CH;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl, or
R¹ and R² combine with the atoms to which they are attached to form an optionally substituted 3 to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3 to 6-membered cycloalkyl, optionally substituted 3 to 7-membered heterocycloalkyl, optionally substituted 6-membered aryl, optionally substituted 5 or 6-membered heteroaryl;
R³ is absent, or
R² and R³ combine with the atom to which they are attached to form an optionally substituted 3 to 8-membered cycloalkyl or optionally substituted 3 to 14-membered heterocycloalkyl;
R⁴ is absent, hydrogen, halogen, cyano, or methyl optionally substituted with 1 to 3 halogens;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3 to 6-membered cycloalkyl or optionally substituted 3 to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3 to 6-membered cycloalkyl, or optionally substituted 3 to 7-membered heterocycloalkyl;
R^{7a} and R^{8a} are, independently, hydrogen, halo, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
R^{7'} and R^{8'} combine with the carbon atom to which they are attached to form optionally substituted 3 to 6-membered cycloalkyl or optionally substituted 3 to 7-membered heterocycloalkyl;
R¹⁰ is hydrogen, halo, hydroxy, C₁-C₃ alkoxy, or C₁-C₃ alkyl; and
R^{10a} is hydrogen or halo.

In some embodiments, the disclosure provides compounds having Formula VIIIb or a pharmaceutically acceptable salt or stereoisomer thereof: 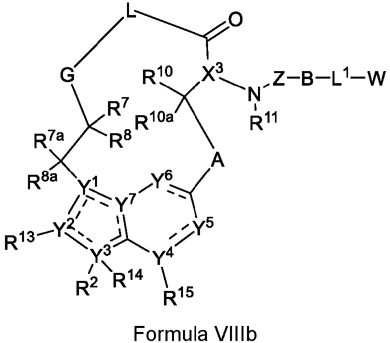 wherein the dotted lines represent zero, one, two, three, or four non-adjacent double bonds;
L has the structure of Formula VIIa or Vllb: 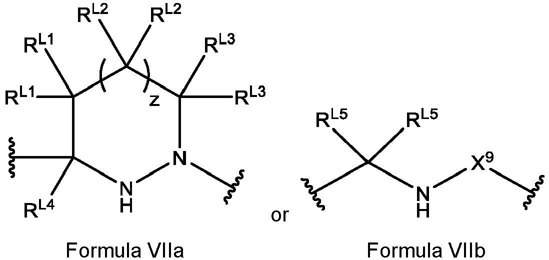
z is 0, 1, or 2;
X⁹ is -NR^{L6}-, -C(O)-, or -S(O)₂-;
each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
wherein L does not have the structure of 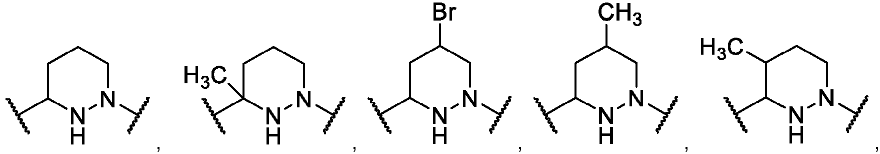 or 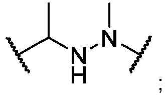
A is optionally substituted C₂-C₄ alkylene, optionally substituted C₁-C₄ heteroalkylene, or optionally substituted C₂-C₄ alkenylene;
B is absent, -NH-, -N(CH₃)-, -O-, -CH(R⁹)- or >C=CR⁹R^{9'} where the carbon is bound to the carbonyl carbon of -N(R¹¹)C(O)-, optionally substituted 3 to 6-membered cycloalkylene, optionally substituted 3 to 6-membered heterocycloalkylene, optionally substituted 6-membered arylene, or 5 to 6-membered heteroarylene;
G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3 to 8-membered heteroarylene;
L¹ is absent or a linker;
W is hydrogen, cyano, optionally substituted amino, optionally substituted amido, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 11-membered heterocycloalkyl, optionally substituted 3 to 10-membered cycloalkyl, optionally substituted 6 to 10-membered aryl, or optionally substituted 3 to 10-membered heteroaryl;
Z is -C(O)- or -S(O)₂-;
X³ is N or CH;
Y¹ is C, CH, or N;
Y², Y³, Y⁴, and Y⁷ are, independently, C or N;
Y⁵ is CH, CH₂, or N;
Y⁶ is C(O), CH, CH₂, or N;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl, or
R¹ and R² combine with the atoms to which they are attached to form an optionally substituted 3 to 14-membered heterocycloalkyl;
R² is absent, hydrogen, optionally substituted C₁-C₆ alkyl, optionally substituted C₂-C₆ alkenyl, optionally substituted C₂-C₆ alkynyl, optionally substituted 3 to 6-membered cycloalkyl, optionally substituted 3 to 7-membered heterocycloalkyl, optionally substituted 6-membered aryl, optionally substituted 5 or 6-membered heteroaryl; R³ is absent or R² and R³ combine with the atom to which they are attached to form an optionally substituted 3 to 8-membered cycloalkyl or optionally substituted 3 to 14-membered heterocycloalkyl;
R⁴ is absent, hydrogen, halogen, cyano, or methyl optionally substituted with 1 to 3 halogens;
R⁶ is hydrogen or methyl; R⁷ is hydrogen, halogen, or optionally substituted C₁-C₃ alkyl, or
R⁶ and R⁷ combine with the carbon atoms to which they are attached to form an optionally substituted 3 to 6-membered cycloalkyl or optionally substituted 3 to 7-membered heterocycloalkyl;
R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3 to 6-membered cycloalkyl, or optionally substituted 3 to 7-membered heterocycloalkyl;
R^{7a} and R^{8a} are, independently, hydrogen, halo, optionally substituted C₁-C₃ alkyl, or combine with the carbon to which they are attached to form a carbonyl;
R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
R^{7'} and R^{8'} combine with the carbon atom to which they are attached to form optionally substituted 3 to 6-membered cycloalkyl or optionally substituted 3 to 7-membered heterocycloalkyl;
R⁹ is hydrogen, F, optionally substituted C₁-C₆ alkyl, optionally substituted C₁-C₆ heteroalkyl, optionally substituted 3 to 6-membered cycloalkyl, or optionally substituted 3 to 7-membered heterocycloalkyl;
R⁹ and L combine with the atoms to which they are attached to form an optionally substituted 3 to 14-membered heterocycloalkyl;
R^{9'} is hydrogen or optionally substituted C₁-C₆ alkyl;
R¹⁰ is hydrogen, halo, hydroxy, C₁-C₃ alkoxy, or C₁-C₃ alkyl;
R^{10a} is hydrogen or halo; and
R¹¹ is hydrogen or C₁-C₃ alkyl.

In some embodiments, the disclosure provides compounds having Formula Vlllc or a pharmaceutically acceptable salt or stereoisomer thereof: 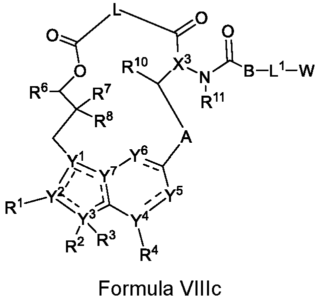 wherein the variables in Formula Vlllc are as defined in formula Vlllb.

In some embodiments of compounds of formula Vlllc, L is as defined in formula VIIIb; L¹ is absent or a linker; Y⁵ and Y⁶ are each independently CH or N;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl;
R² is hydrogen, optionally substituted C₁-C₆ alkyl, optionally substituted C₂-C₆ alkenyl, optionally substituted 3 to 6-membered cycloalkyl, optionally substituted 3 to 7-membered heterocycloalkyl, optionally substituted 6-membered aryl, optionally substituted 5 or 6-membered heteroaryl; R³ is absent or R² and R³ combine with the atom to which they are attached to form an optionally substituted 3 to 8-membered cycloalkyl or optionally substituted 3 to 14-membered heterocycloalkyl;
R⁹ is optionally substituted C₁-C₆ alkyl, optionally substituted C₁-C₆ heteroalkyl, optionally substituted 3 to 6-membered cycloalkyl, or optionally substituted 3 to 7-membered heterocycloalkyl; and
R¹⁰ is hydrogen, hydroxy, C₁-C₃ alkoxy, or C₁-C₃ alkyl.

In some embodiments, the disclosure provides compounds having Formula VIIId or a pharmaceutically acceptable salt or stereoisomer thereof: 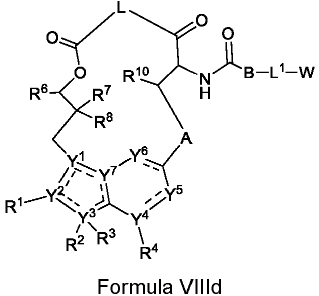 wherein the variables in Formula VIIId are as defined in formula VIIIb.

In some embodiments of compounds of formula VIIId, L is as defined in formula Vlllb; L¹ is absent or a linker; B is absent, -CH(R⁹)- where the carbon is bound to the carbonyl carbon of - NHC(O)-, optionally substituted 3 to 6-membered cycloalkylene, optionally substituted 3 to 6-membered heterocycloalkylene, optionally substituted 6-membered arylene, or 5 to 6-membered heteroarylene;
W is hydrogen, optionally substituted amino, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 11-membered heterocycloalkyl, optionally substituted 3 to 8-membered cycloalkyl, or optionally substituted 3 to 8-membered heteroaryl;
Y⁵ and Y⁶ are, independently, CH or N;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl;
R² is hydrogen, optionally substituted C₁-C₆ alkyl, optionally substituted C₂-C₆ alkenyl, optionally substituted 3 to 6-membered cycloalkyl, optionally substituted 3 to 7-membered heterocycloalkyl, optionally substituted 6-membered aryl, optionally substituted 5 or 6-membered heteroaryl; R³ is absent or R² and R³ combine with the atom to which they are attached to form an optionally substituted 3 to 8-membered cycloalkyl or optionally substituted 3 to 14-membered heterocycloalkyl;
R⁹ is optionally substituted C₁-C₆ alkyl, optionally substituted C₁-C₆ heteroalkyl, optionally substituted 3 to 6-membered cycloalkyl, or optionally substituted 3 to 7-membered heterocycloalkyl; and
R¹⁰ is hydrogen, hydroxy, C₁-C₃ alkoxy, or C₁-C₃ alkyl.

In some embodiments, the disclosure provides compounds having Formula Vllle or a pharmaceutically acceptable salt or stereoisomer thereof: 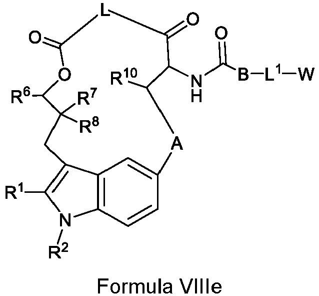 wherein the variables in Formula Vllle are as defined in formula VIIIb.

In some embodiments of compounds of formula Vllle, L is as defined in formula Vlllb; L¹ is absent or a linker; B is absent, -CH(R⁹)- where the carbon is bound to the carbonyl carbon of - NHC(O)-, optionally substituted 3 to 6-membered cycloalkylene, optionally substituted 3 to 6-membered heterocycloalkylene, optionally substituted 6-membered arylene, or 5 to 6-membered heteroarylene;
W is hydrogen, optionally substituted amino, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 11-membered heterocycloalkyl, optionally substituted 3 to 8-membered cycloalkyl, or optionally substituted 3 to 8-membered heteroaryl;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl;
R² is hydrogen, optionally substituted C₁-C₆ alkyl, optionally substituted C₂-C₆ alkenyl, optionally substituted 3 to 6-membered cycloalkyl, optionally substituted 3 to 7-membered heterocycloalkyl, optionally substituted 6-membered aryl, optionally substituted 5 or 6-membered heteroaryl; R³ is absent or R² and R³ combine with the atom to which they are attached to form an optionally substituted 3 to 8-membered cycloalkyl or optionally substituted 3 to 14-membered heterocycloalkyl;
R⁹ is optionally substituted C₁-C₆ alkyl, optionally substituted C₁-C₆ heteroalkyl, optionally substituted 3 to 6-membered cycloalkyl, or optionally substituted 3 to 7-membered heterocycloalkyl; and
R¹⁰ is hydrogen, hydroxy, C₁-C₃ alkoxy, or C₁-C₃ alkyl.

In some embodiments, the disclosure provides compounds having Formula VIIIf or a pharmaceutically acceptable salt or stereoisomer thereof: 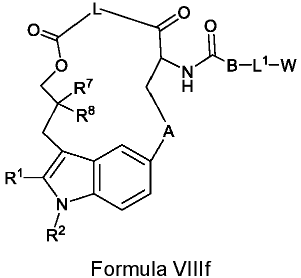 wherein the variables in Formula VIIIf are as defined in formula VIIIb.

In some embodiments of compounds of formula VIIIf, L is as defined in formula Vlllb; L¹ is absent or a linker; B is absent, -CH(R⁹)- where the carbon is bound to the carbonyl carbon of - NHC(O)-, optionally substituted 3 to 6-membered cycloalkylene, optionally substituted 3 to 6-membered heterocycloalkylene, optionally substituted 6-membered arylene, or 5 to 6-membered heteroarylene;
W is hydrogen, optionally substituted amino, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 11-membered heterocycloalkyl, optionally substituted 3 to 8-membered cycloalkyl, or optionally substituted 3 to 8-membered heteroaryl;
R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl;
R² is C₁-C₆ alkyl or 3 to 6-membered cycloalkyl;
R⁷ is C₁-C₃ alkyl;
R⁸ is C₁-C₃ alkyl; and
R⁹ is optionally substituted C₁-C₆ alkyl, optionally substituted C₁-C₆ heteroalkyl, optionally substituted 3 to 6-membered cycloalkyl, or optionally substituted 3 to 7-membered heterocycloalkyl.

In some embodiments, the disclosure provides compounds having Formula Vlllg or a pharmaceutically acceptable salt or stereoisomer thereof: 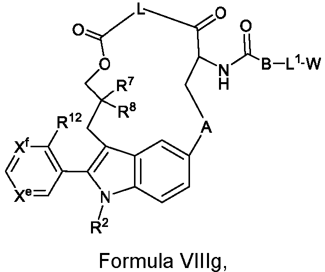 wherein
X^{e} is N, CH, or CR¹⁷;
X^{f} is N or CH;
R¹² is optionally substituted C₁-C₆ alkyl or optionally substituted C₁-C₆ heteroalkyl; and the other variables are as defined in formula Vlllb.

In some embodiments of compounds of formula Vlllg, L is as defined in formula Vlllb; L¹ is a linker or absent; B is absent, -CH(R⁹)- where the carbon is bound to the carbonyl carbon of -NHC(O)-, optionally substituted 3 to 6-membered cycloalkylene, optionally substituted 3 to 6-membered heterocycloalkylene, optionally substituted 6-membered arylene, or 5 to 6-membered heteroarylene;
W is hydrogen, optionally substituted amino, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 11-membered heterocycloalkyl, optionally substituted 3 to 8-membered cycloalkyl, or optionally substituted 3 to 8-membered heteroaryl;
R² is C₁-C₆ alkyl or 3 to 6-membered cycloalkyl;
R⁷ is C₁-C₃ alkyl;
R⁸ is C₁-C₃ alkyl;
R⁹ is optionally substituted C₁-C₆ alkyl, optionally substituted C₁-C₆ heteroalkyl, optionally substituted 3 to 6-membered cycloalkyl, or optionally substituted 3 to 7-membered heterocycloalkyl;
X^{e} is N, CH, or CR¹⁷;
X^{f} is N or CH;
R¹² is optionally substituted C₁-C₆ alkyl or optionally substituted C₁-C₆ heteroalkyl; and
R¹⁷ is optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl.

In some embodiments, A is optionally substituted thiazole-diyl, optionally substituted oxazole-diyl, optionally substituted morpholine-diyl, optionally substituted pyrrolidine-diyl, optionally substituted piperidine-diyl, or optionally substituted phenylene. In some embodiments, A is optionally substituted thiazole-diyl or optionally substituted morpholine-diyl. In some embodiments of a compound of the present invention, A is optionally substituted 5- to 10-membered heteroarylene. In some embodiments, A is: 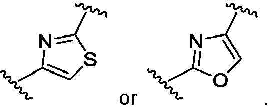 In some embodiments, A is

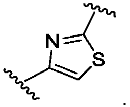

In some embodiments of a compound of the present invention, A is optionally substituted phenylene. In some embodiments, A is: 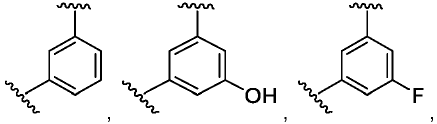 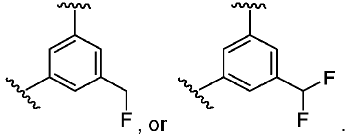 In some embodiments, A is

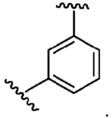

In some embodiments of a compound of the present invention, A is optionally substituted 3- to 6-membered heterocycloalkylene. In some embodiments, A is optionally substituted 6-membered heterocycloalkylene. In some embodiments, A is selected from the following, or a stereoisomer thereof: 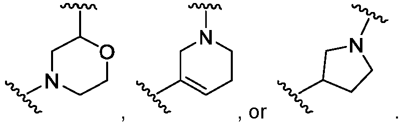 In some embodiments, A is selected from the following, or a stereoisomer thereof:

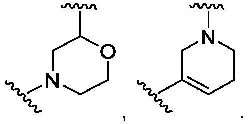

In some embodiments of a compound of the present invention, R¹ is hydrogen or optionally substituted 3- to 10-membered heterocycloalkyl. In some embodiments of a compound of the present invention, R¹ is optionally substituted 3- to 10-membered heterocycloalkyl. In some embodiments of a compound of the present invention, R¹ is:

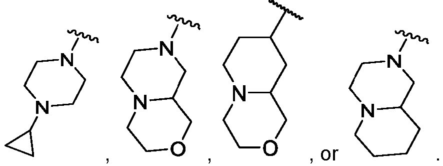

In some embodiments of a compound of the present invention, R¹ is: 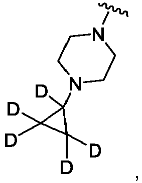 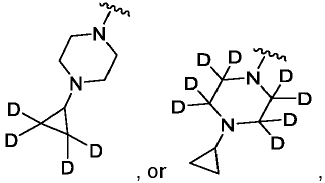 wherein each D indicates a hydrogen having an isotopic enrichment factor for deuterium of at least 5.

In some embodiments of a compound of the present invention, R² is:

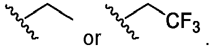

In some embodiments of a compound of the present invention, R² is: 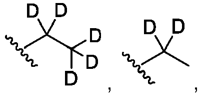 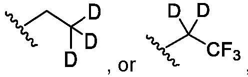 and wherein each D indicates a hydrogen having an isotopic enrichment factor for deuterium of at least 5.

In some embodiments of a compound of the present invention, R³ is optionally substituted C₁-C₆ alkyl or optionally substituted 3- to 6-membered cycloalkyl. In some embodiments of a compound of the present invention, R³ is optionally substituted C₁-C₆ alkyl. In some embodiments, R³ is: 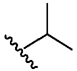 or 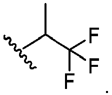 In some embodiments, R³ is: 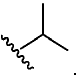 In some embodiments, R³ is 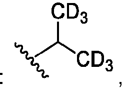 and wherein each D indicates a hydrogen having an isotopic enrichment factor for deuterium of at least 5.

In some embodiments of a compound of the present invention, R³ is or optionally substituted 3- to 6-membered cycloalkyl. In some embodiments, R³ is: 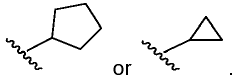 In some embodiments, R³ is:

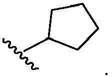

In some embodiments of a compound of the present invention, R² is 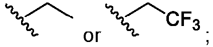 R³ is 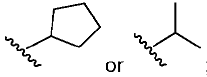 ; and A is

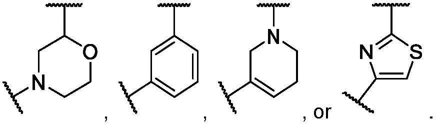

In some embodiments, R² is 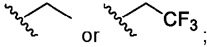 R³ is 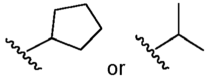 ; and A is

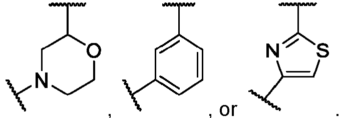

In some embodiments of a compound of the present invention, m is 1. In some embodiments, n is 1. In some embodiments, X⁶ is CH₂. In some embodiments, X⁷ is CH₂. In some embodiments, X⁸ is CH₂. In some embodiments, m is 1, n is 1, and each of X⁶, X⁷, and X⁸ is CH₂.

In some embodiments of any of the compounds described herein, L has the structure of Formula Vlla:

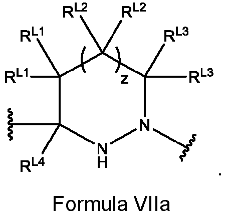

In some embodiments of Formula VIIa, z is 0.

In some embodiments of any of the compounds described herein, L has the structure of Formula Vlla-1:

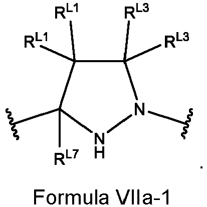

In some embodiments, L has the structure of

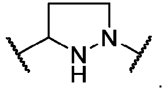

In some embodiments, of Formula VIIa, z is 1.

In some embodiments of any of the compounds described herein, L has the structure of Formula L has the structure of Formula Vlla-2:

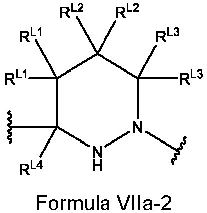

In some embodiments of any of the compounds described herein, L has the structure of Formula L has the structure of Formula Vlla-3:

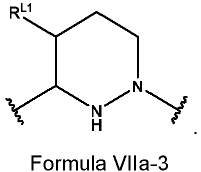

In some embodiments of any of the compounds described herein, L has the structure of Formula L has the structure of Formula Vlla-4:

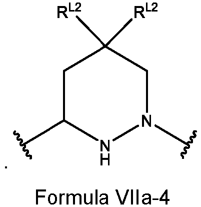

In some embodiments of any of the compounds described herein, L has the structure of Formula Vlla-5

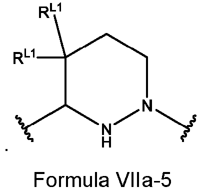

In some embodiments of Formula VIIa, z is 2.

In some embodiments of any of the compounds described herein, L has the structure of Formula Vlla-6:

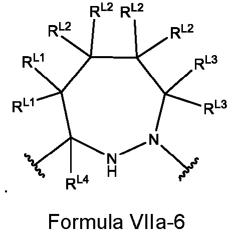

In some embodiments, wherein R^{L1} is hydrogen. In some embodiments, R^{L1} is optionally substituted C₁-C₆ alkyl. In some embodiments, R^{L1} is methyl, ethyl, or trifluoromethyl. In some embodiments, R^{L1} is optionally substituted C₁-C₆ heteroalkyl. In some embodiments, R^{L1} is methoxy or ethoxy. In some embodiments, R^{L1} is optionally substituted C₂-C₆ alkynyl. In some embodiments, R^{L1} is ethynyl.

In some embodiments, R^{L2} is hydrogen. In some embodiments, R^{L2} is halogen. In some embodiments, R^{L2} is fluoro.

In some embodiments, R^{L3} is hydrogen. In some embodiments, R^{L3} is optionally substituted C₁-C₆ alkyl. In some embodiments, R^{L3} is methyl.

In some embodiments, R^{L4} is hydrogen.

In some embodiments, R^{L1} and R^{L4} combine to form an optionally substituted C₄ cycloalkyl.

In some embodiments, R^{L1} and R^{L3} combine to form an optionally substituted C₄ cycloalkyl. In some embodiments, R^{L1} and R^{L3} combine to form an optionally substituted C₅ cycloalkyl.

In some embodiments, two R^{L1} combine to form an optionally substituted C₃-C₆ cycloalkyl.

In some embodiments, R^{L1} and R^{L2} combine to form an optionally substituted C₃-C₆ cycloalkyl.

In some embodiments, L is: 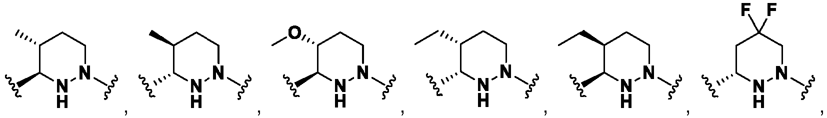 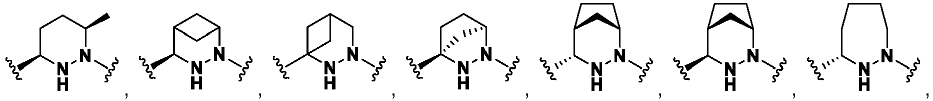 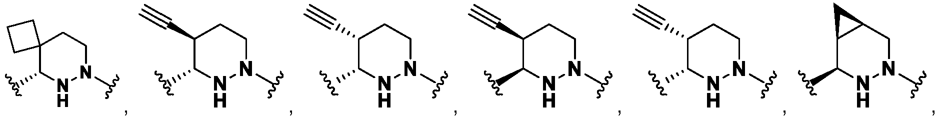 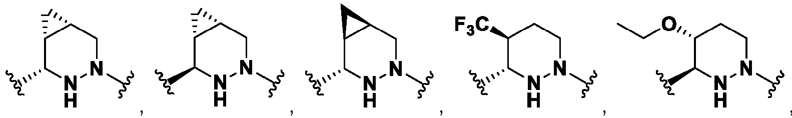 or

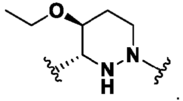

In some embodiments, L is:

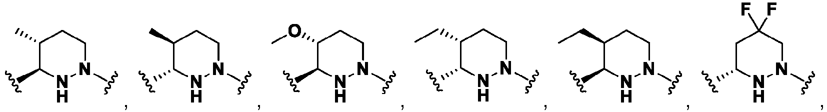

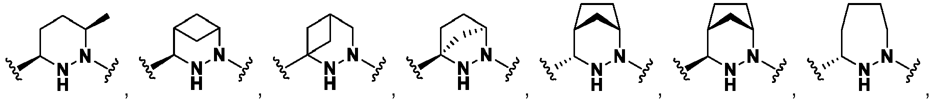

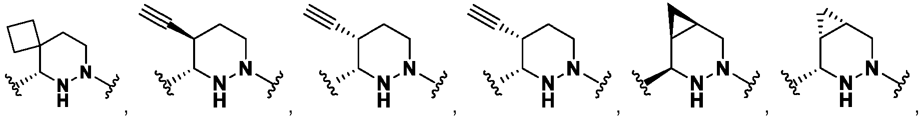

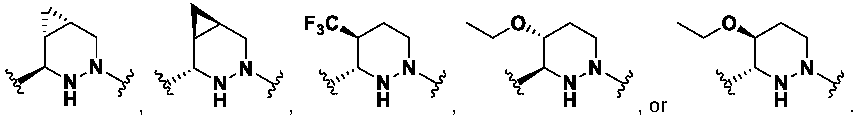

In some embodiments of any of the compounds described herein, L has the structure of Formula VIIb:

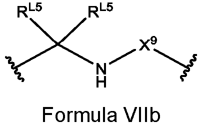

In some embodiments, X⁹ is -NR^{L6}-.

In some embodiments of any of the compounds described herein, L has the structure of Formula Vllb-1:

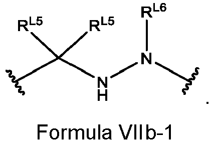

In some embodiments of any of the compounds described herein, L has the structure of Formula Vllb-2:

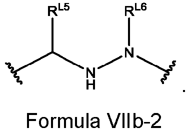

In some embodiments, R^{L6} is optionally substituted C₁-C₆ alkyl. In some embodiments, R^{L6} is methyl.

In some embodiments, X⁹ is -C(O)-. In some embodiments, X⁹ is -S(O)₂-.

In some embodiments, R^{L5} is hydrogen. In some embodiments, R^{L5} is optionally substituted C₁-C₆ alkyl. In some embodiments, R^{L5} is optionally substituted C₃-C₈ cycloalkyl. In some embodiments, two R^{L5} combine to form an optionally substituted C₃-C₈ cycloalkyl.

In some embodiments of any of the compounds described herein, L is:

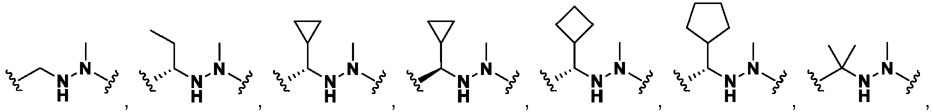

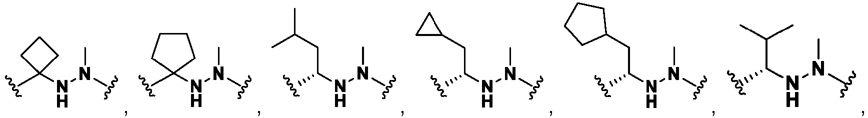

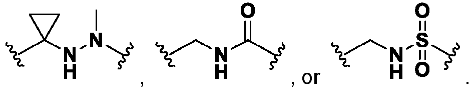

In some embodiments of any of the compounds described herein, L is: 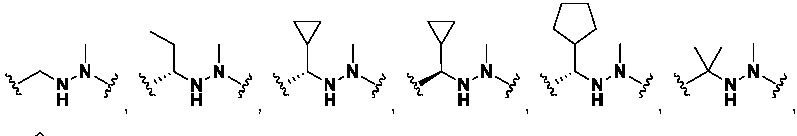 or

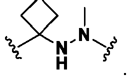

In some embodiments of any of the compounds described herein, L does not have the structure of:

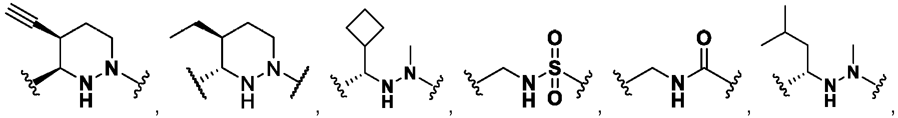

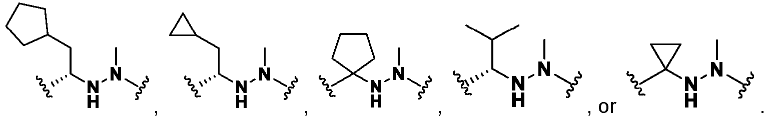

In addition, RAS inhibitors as disclosed in the following applications may be adapted to incorporate Formula VIIa or Formula Vllb using methodologies disclosed herein in combination with techniques known to those of skill in the art: WO 2024/067857, WO 2024/060966, WO 2024/017859, WO 2024/008834, WO 2024/008610, WO 2023/232776, WO 2023/208005, WO 2023/086341, WO 2023/025832, WO 2023/015559, CN 117720556, CN 117720555, CN 117720554, CN 1177534687, CN 11753685, and CN 11753684.

In some embodiments of any of the compounds described herein A is optionally substituted 3-to 6-membered heterocycloalkylene, optionally substituted 6-membered arylene, or optionally substituted 5- to 10-membered heteroarylene. In some embodiments, A is optionally substituted 6-membered arylene.

In some embodiments, A is:

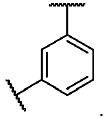

In some embodiments, A is optionally substituted 3- to 6-membered heterocycloalkylene. In some embodiments, A is:

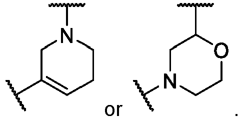

In some embodiments, A is optionally substituted 5- to 10-membered heteroarylene. In some embodiments, A is

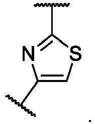

In some embodiments, R² is ethyl or haloethyl. In some embodiments, R³ is optionally substituted C₁-C₆ alkyl.

Other Ras inhibitors similar to those of Formula I, Formula lia, Formula lib, Formula lic, Formula III, Formula IV, Formula V, or Formula VI, such as those described in WO 2023/015559, WO 2023/025832 and WO2023/086341, may be modified to incorporate L groups as described herein using the methodologies described herein. In any embodiment herein, a Ras inhibitor described in WO 2023/015559, WO 2023/025832 or WO2023/086341 may be excluded. In any embodiments herein, any one or more of Compounds A11, A18, A19, A23, A24, A27, A29, A38, A42, A43, A56 and A58 may be excluded. In some embodiments, the compound is not A11, A18, A19, A23, A24, A27, A29, A38, A42, A43, A56 or A58.

In some embodiments, a compound of the present invention is selected from Table 1, or a pharmaceutically acceptable salt or stereoisomer thereof. In some embodiments, a compound of the present invention is selected from Table 1, or a pharmaceutically acceptable salt or atropisomer thereof.

**Table 1: Certain Compounds of the Present Invention**

| Ex# | Structure |
|---|---|
| A1 | 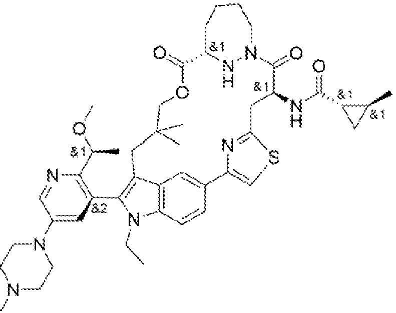 |
| A2 | 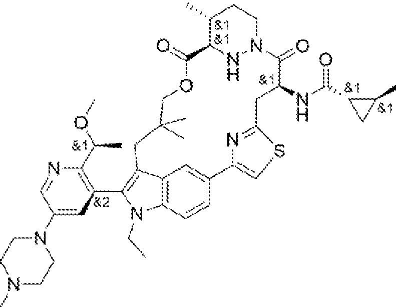 |
| A3 | 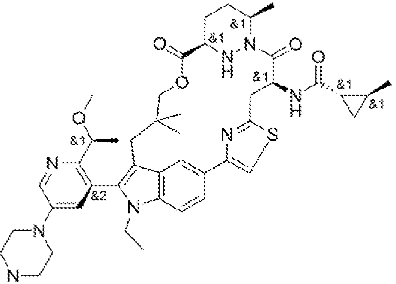 |
| A5 | 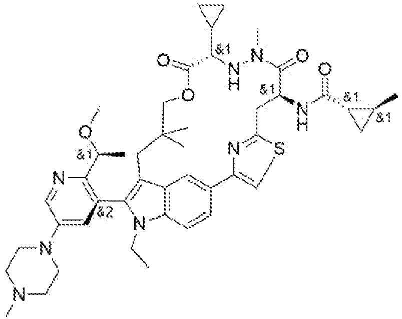 |
| A6 | 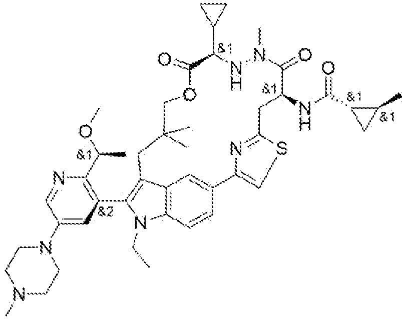 |
| A7 | 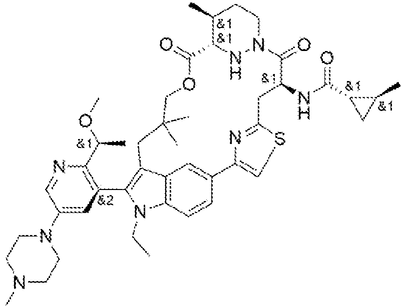 |
| A8 | 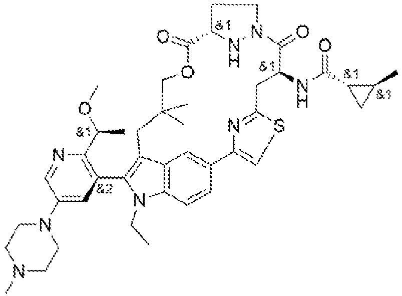 |
| A9 | 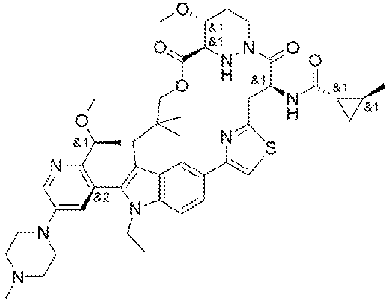 |
| A10 | 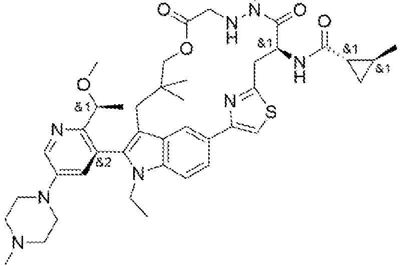 |
| A11 | 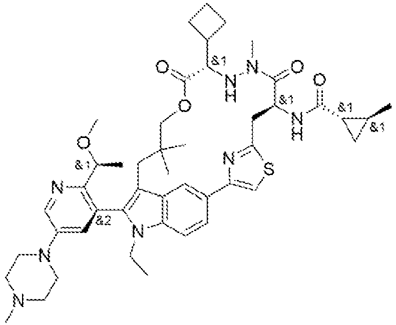 |
| A12 | 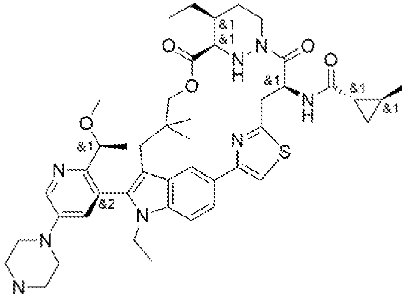 |
| A13 | 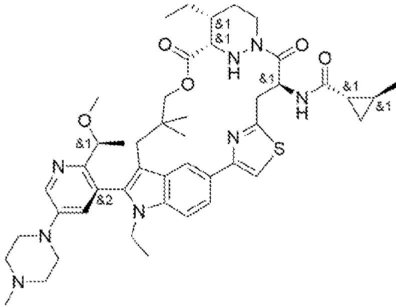 |
| A14 | 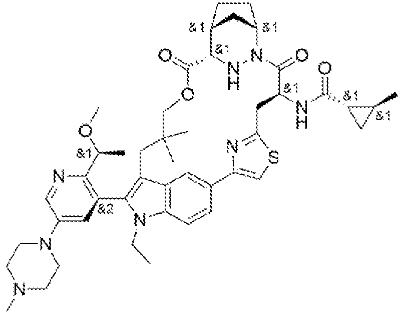 |
| A15 | 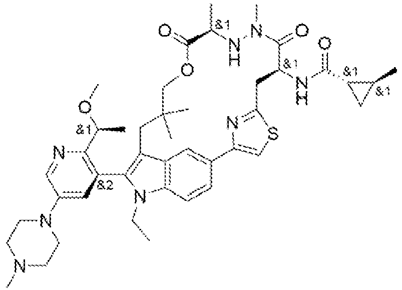 |
| A16 | 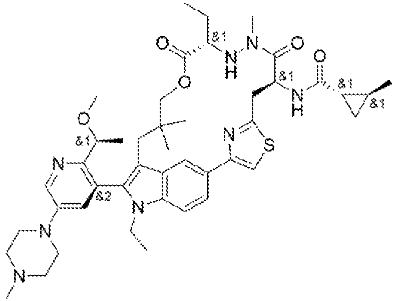 |
| A17 | 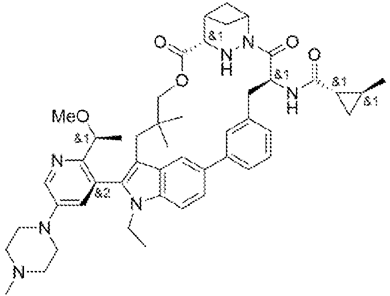 |
| A18 | 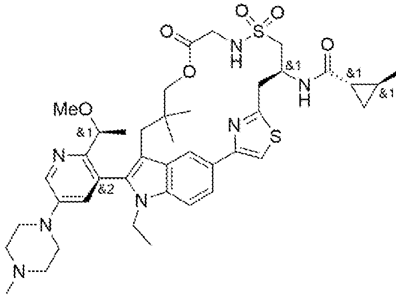 |
| A19 | 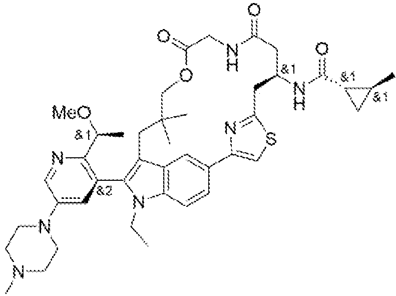 |
| A20 | 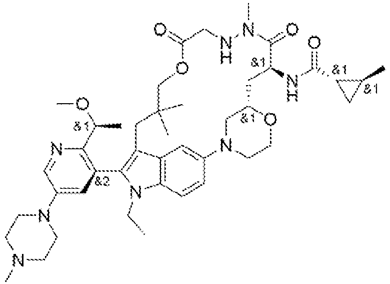 |
| A21 | 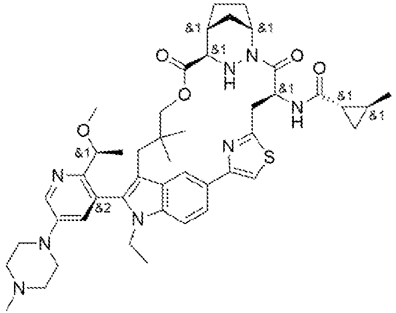 |
| A22 | 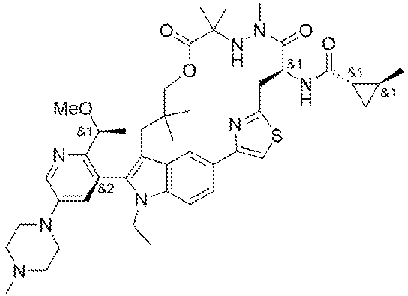 |
| A23 | 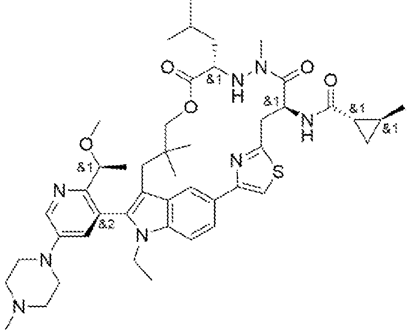 |
| A24 | 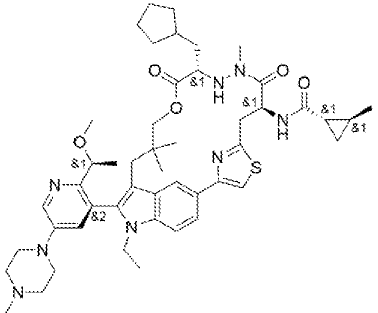 |
| A25 | 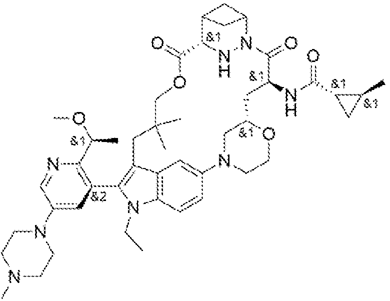 |
| A26 | 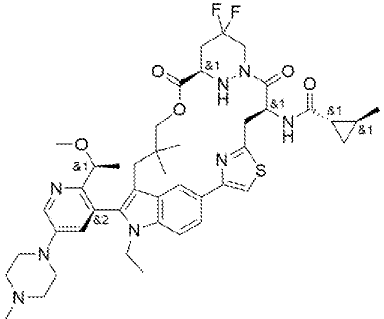 |
| A27 | 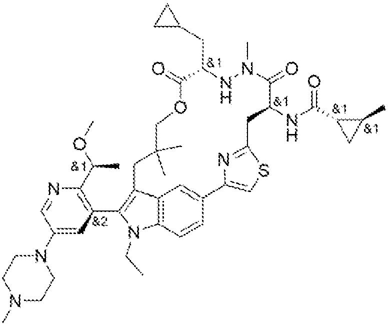 |
| A28 | 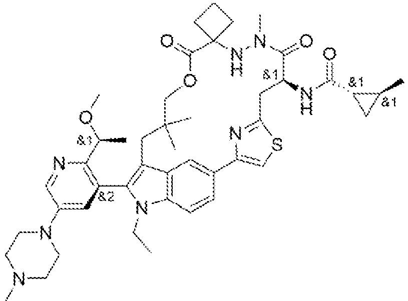 |
| A29 | 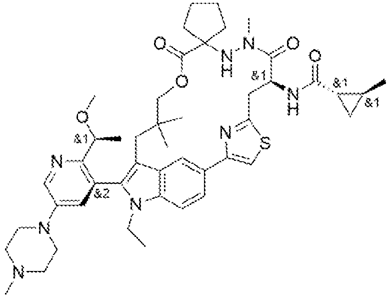 |
| A30 | 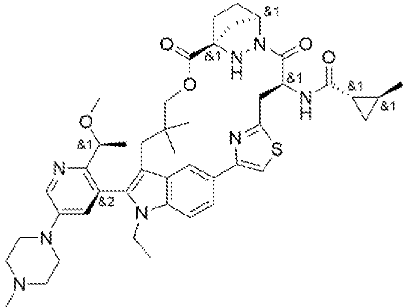 |
| A31 | 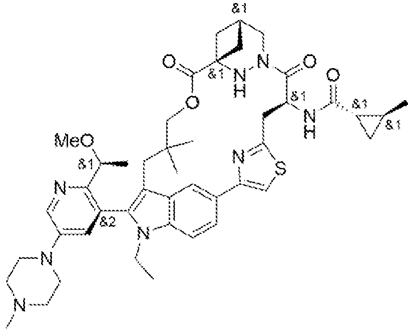 |
| A32 | 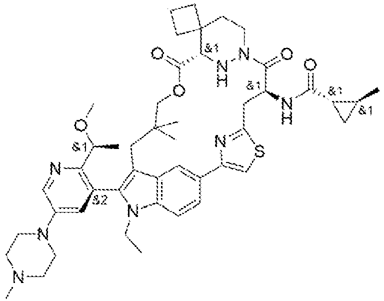 |
| A33 | 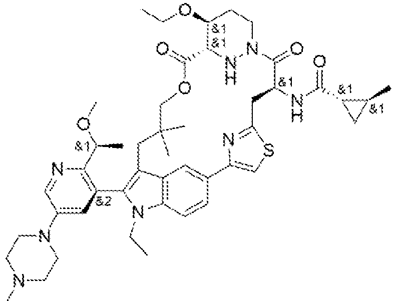 |
| A34 | 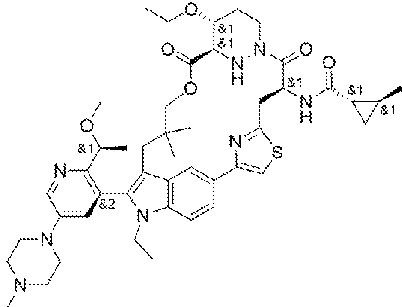 |
| A38 | 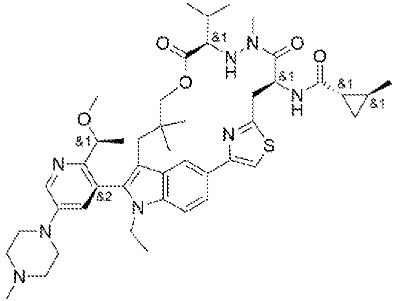 |
| A39 | 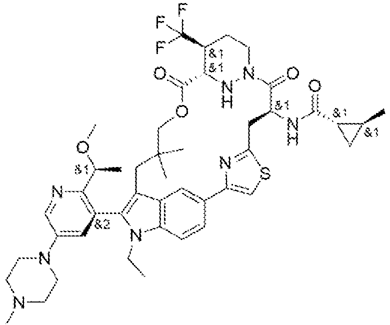 |
| A40 | 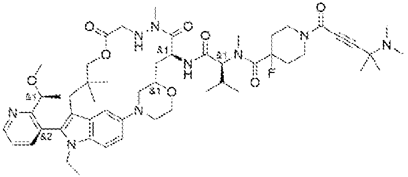 |
| A42 | 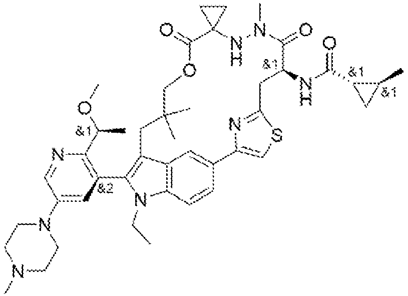 |
| A43 | 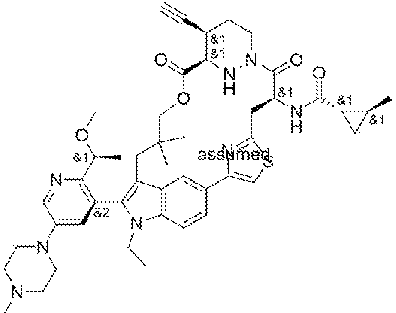 |
| A44 | 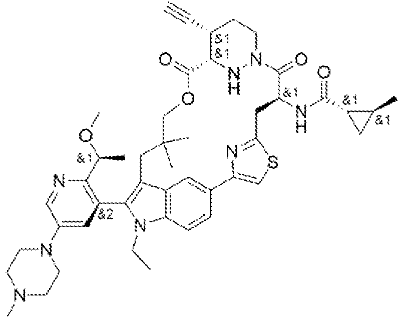 |
| A45 | 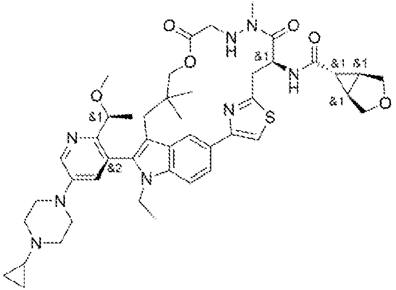 |
| A46 | 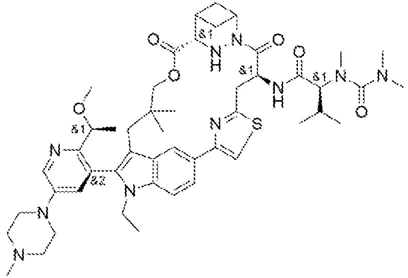 |
| A47 | 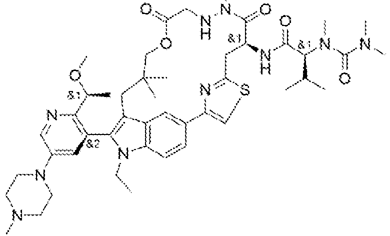 |
| A48 | 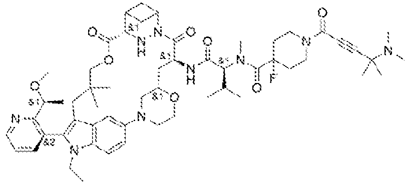 |
| A49 | 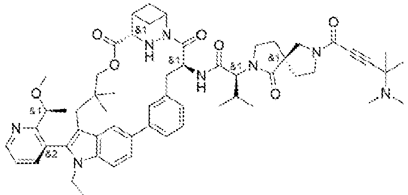 |
| A50 | 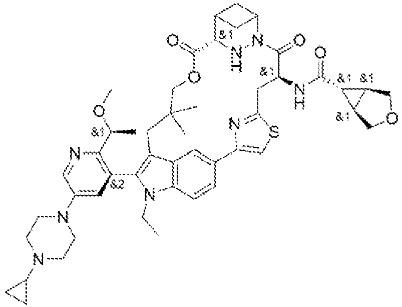 |
| A51 | 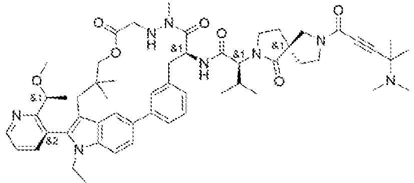 |
| A52 | 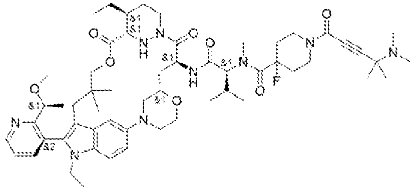 |
| A53 | 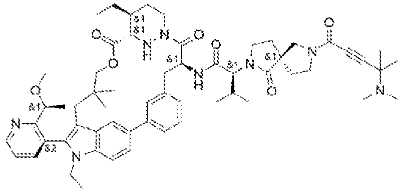 |
| A54 | 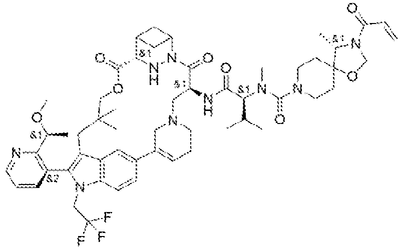 |
| A55 | 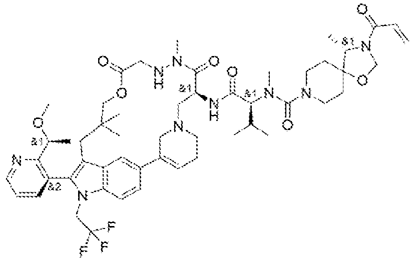 |
| A56 | 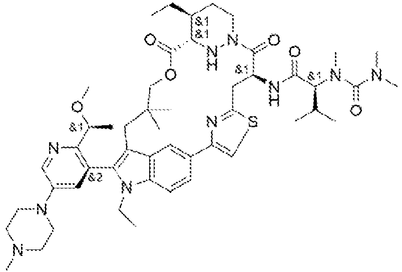 |
| A57 | 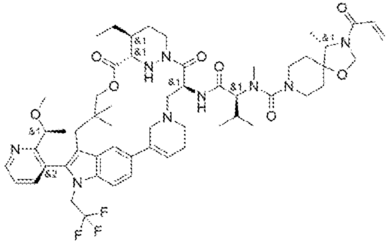 |
| A58 | 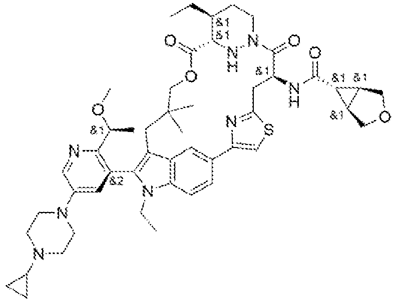 |
| A59 | 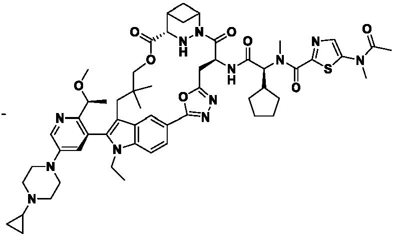 |
| A60 | 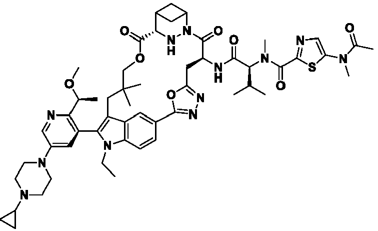 |

The compounds in Table 2 are excluded from the present invention.

**Table 2. Excluded compounds**

| No. | Structure | | No. | Structure |
|---|---|---|---|---|
| E1 | 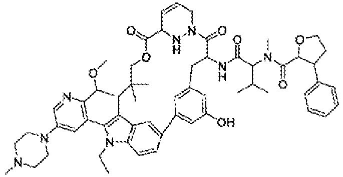 | | E2 | 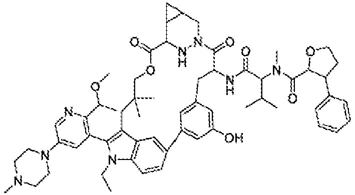 |
| E3 | 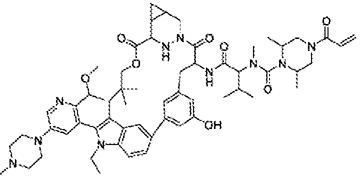 | | E4 | 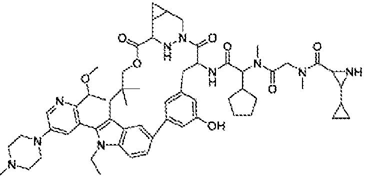 |
| E5 | 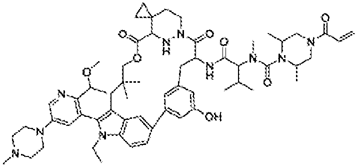 | | E6 | 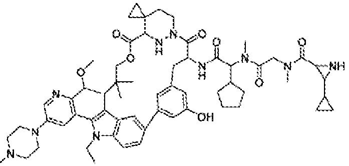 |
| E7 | 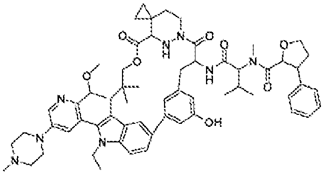 | | E8 | 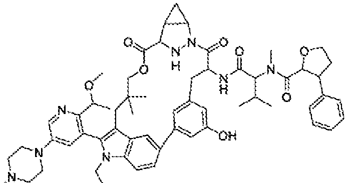 |
| E9 | 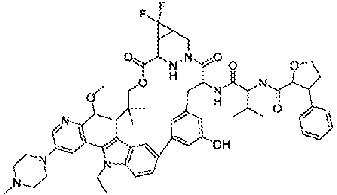 | | E10 | 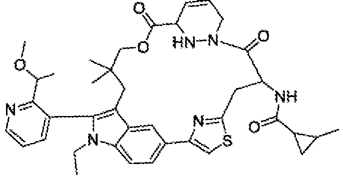 |
| E11 | 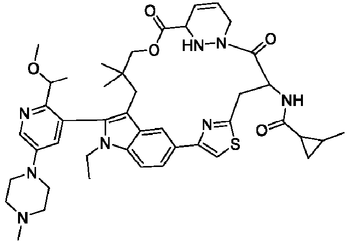 | | E12 | 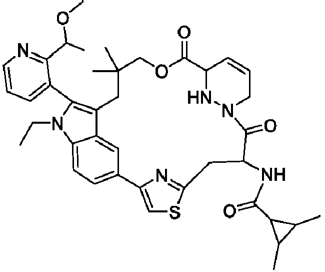 |
| E13 | 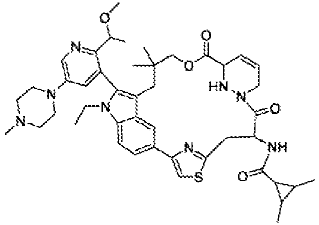 | | E14 | 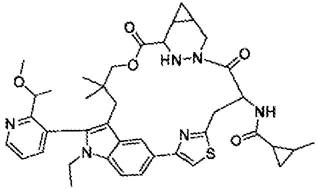 |
| E15 | 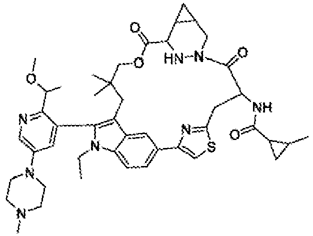 | | E16 | 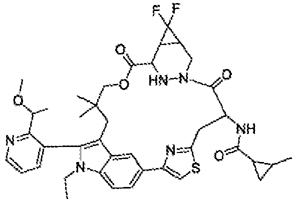 |
| E17 | 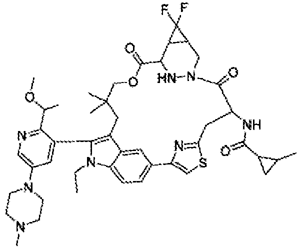 | | E18 | 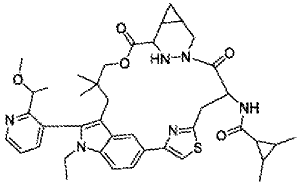 |
| E19 | 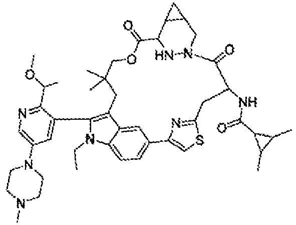 | | E20 | 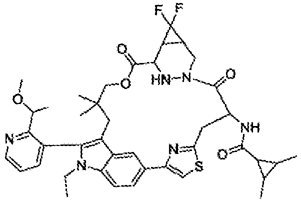 |
| E21 | 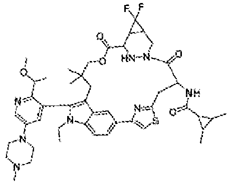 | | E22 | 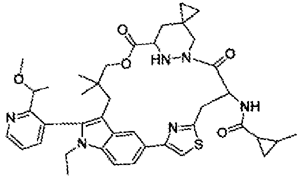 |
| E23 | 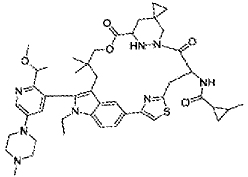 | | E24 | 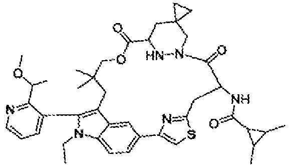 |
| E25 | 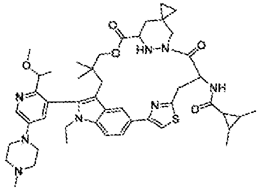 | | E26 | 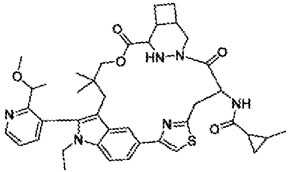 |
| E27 | 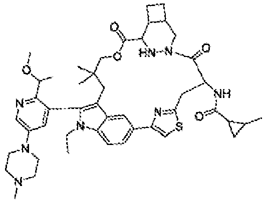 | | E28 | 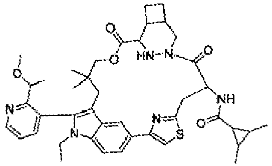 |
| E29 | 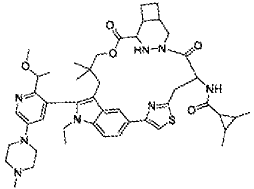 | | E30 | 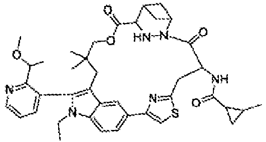 |
| E31 | 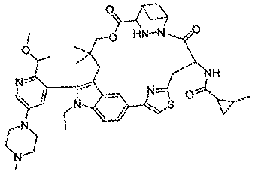 | | E32 | 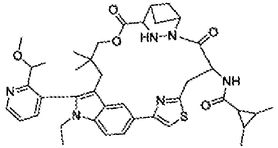 |
| E33 | 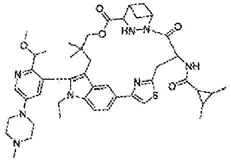 | | E34 | 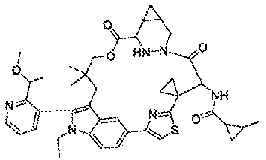 |
| E35 | 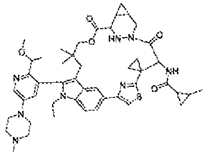 | | E36 | 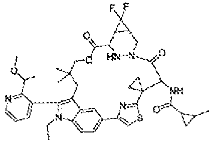 |
| E37 | 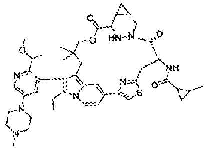 | | E38 | 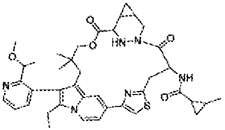 |
| E39 | 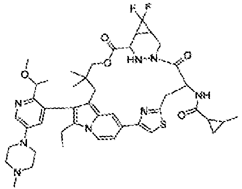 | | E40 | 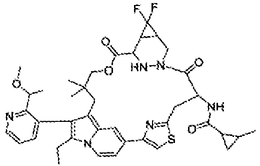 |
| E41 | 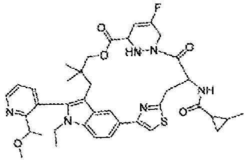 | | E42 | 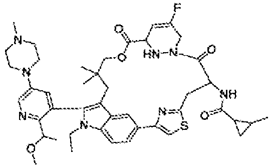 |
| E43 | 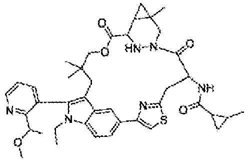 | | E44 | 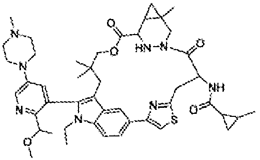 |
| E45 | 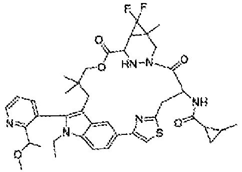 | | E46 | 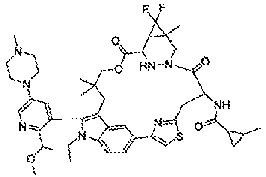 |
| E47 | 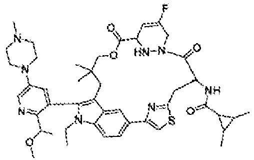 | | E48 | 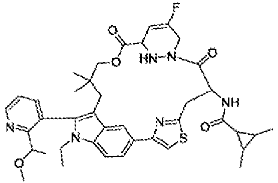 |
| E49 | 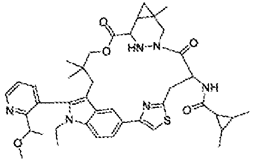 | | E50 | 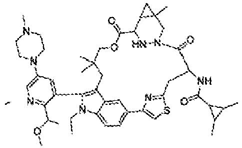 |
| E51 | 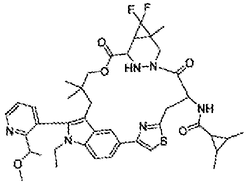 | | E52 | 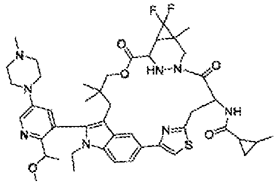 |

The compounds in Table 4 are excluded from the present invention.

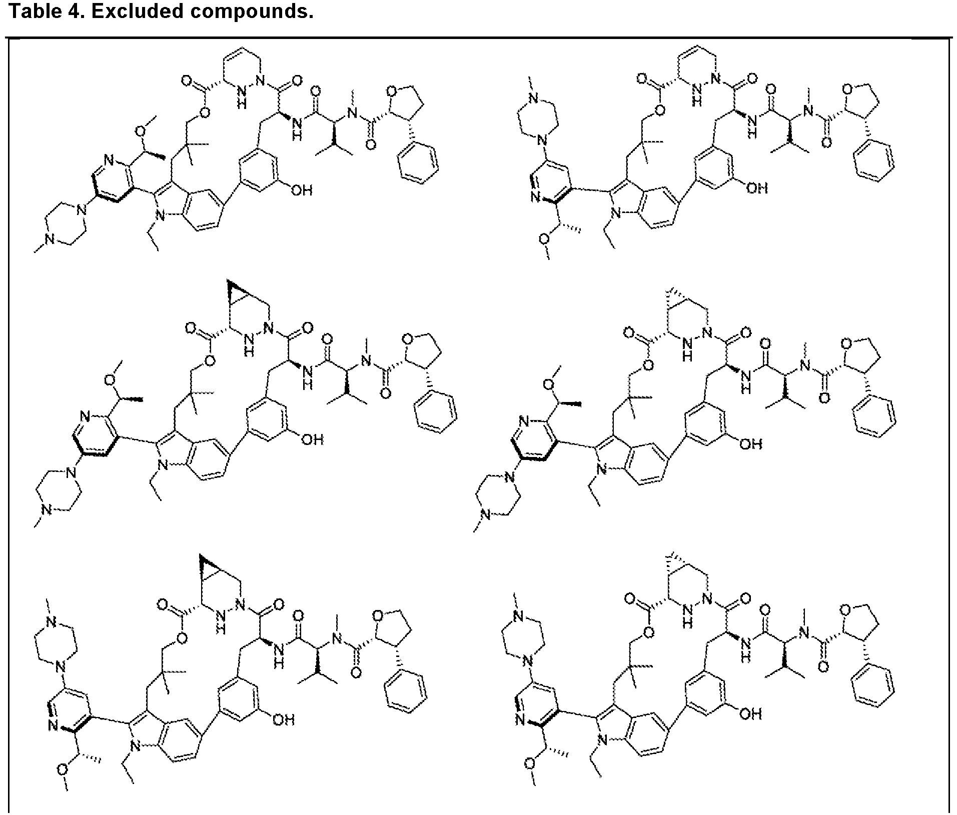

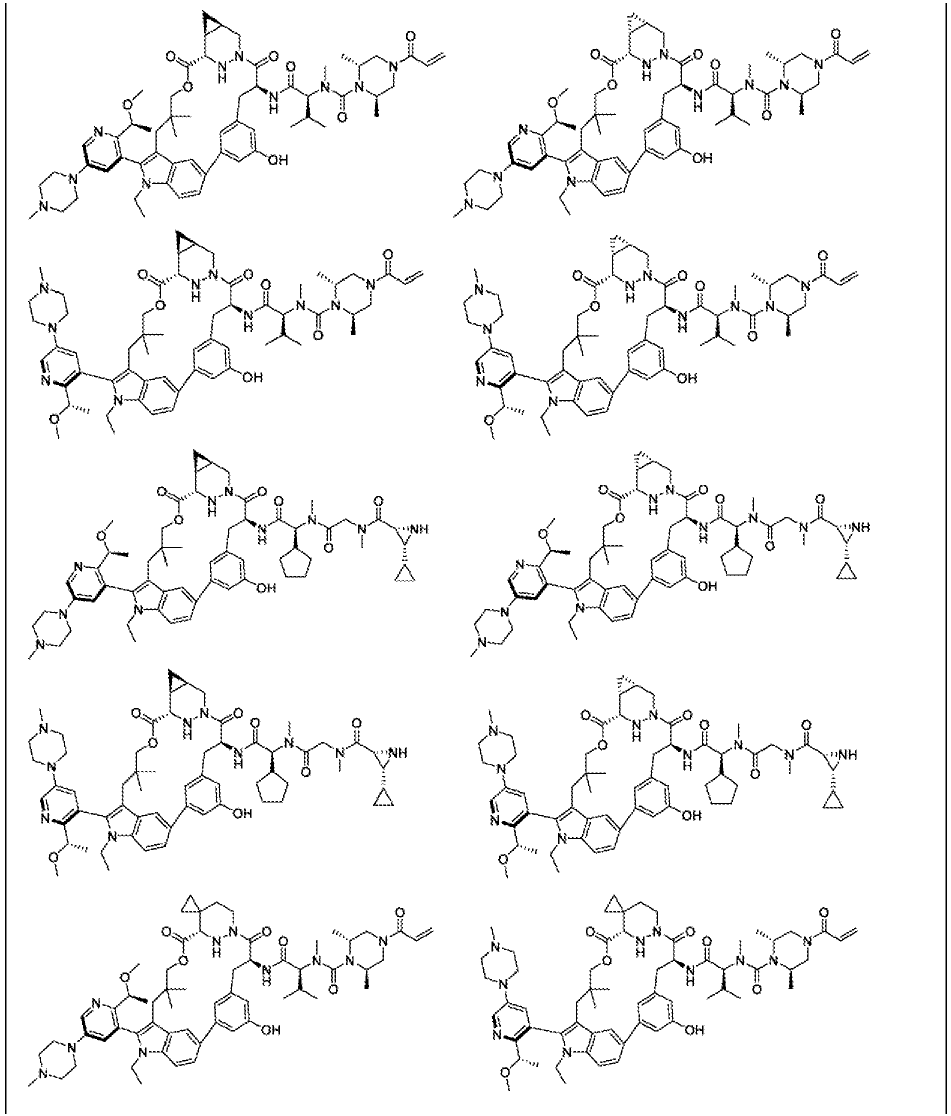

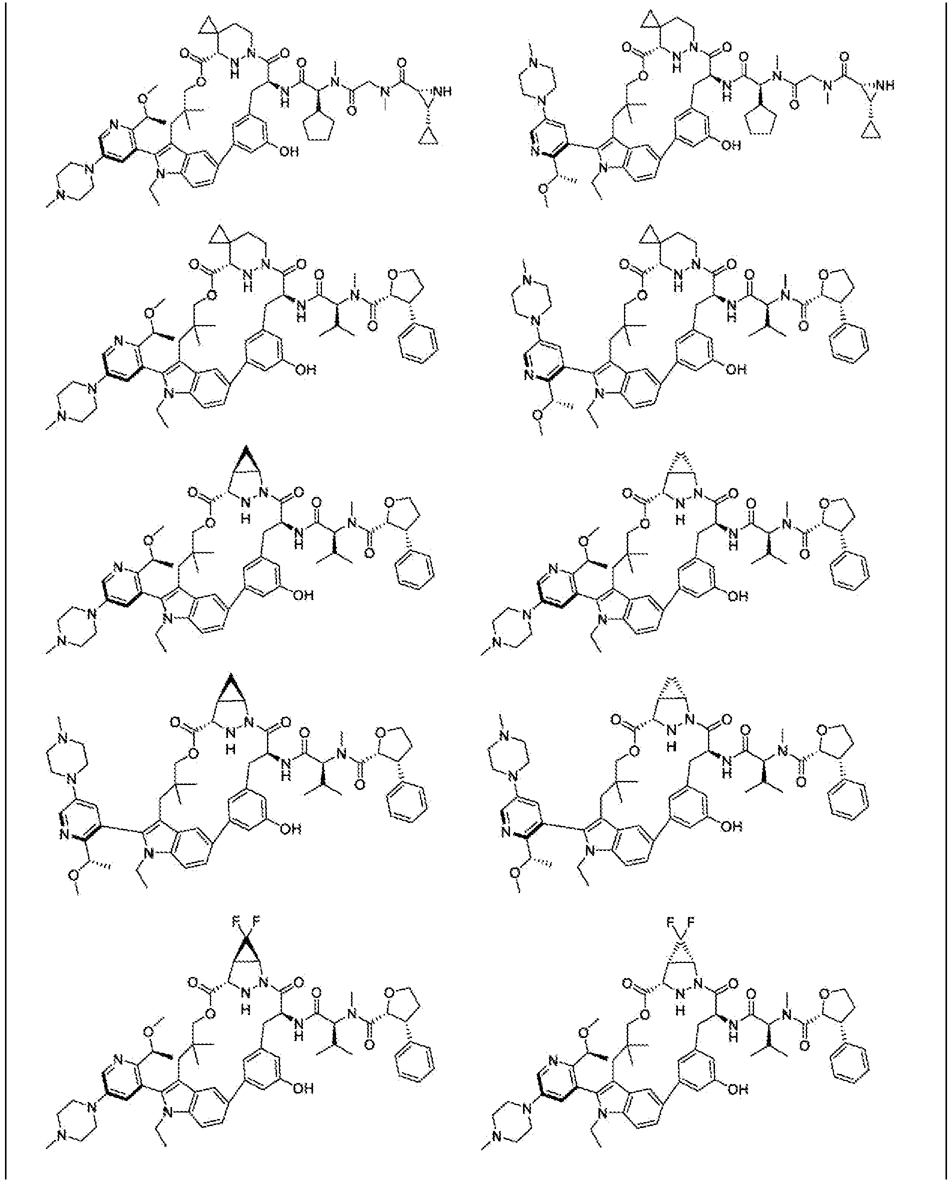

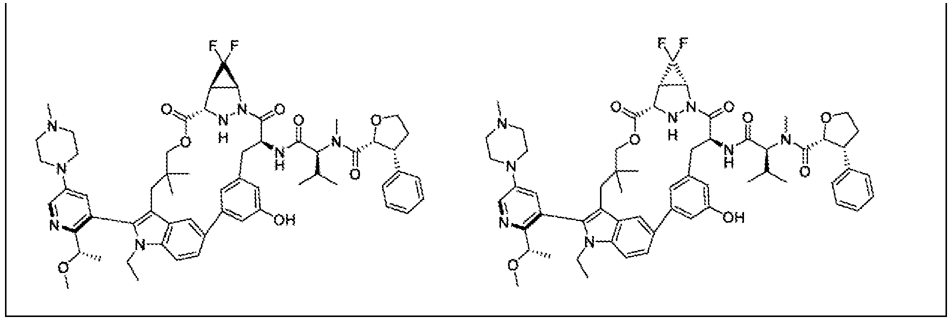

The compounds in Table 5 are excluded from the present invention.

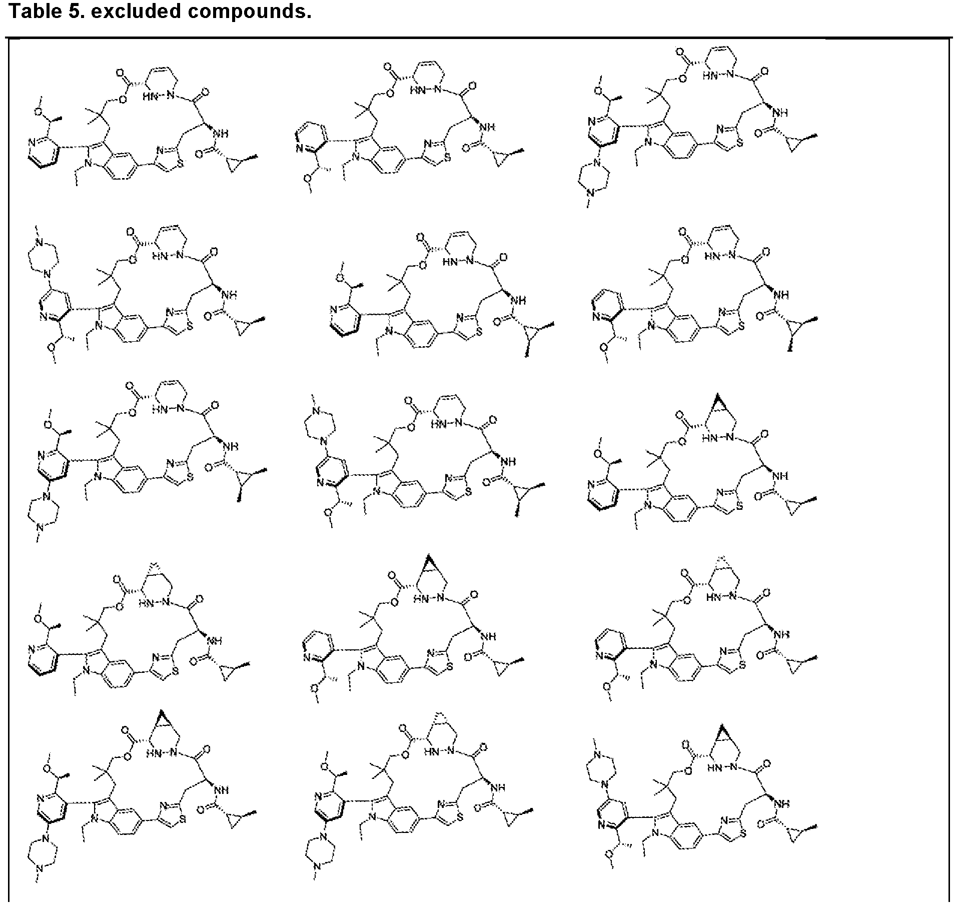

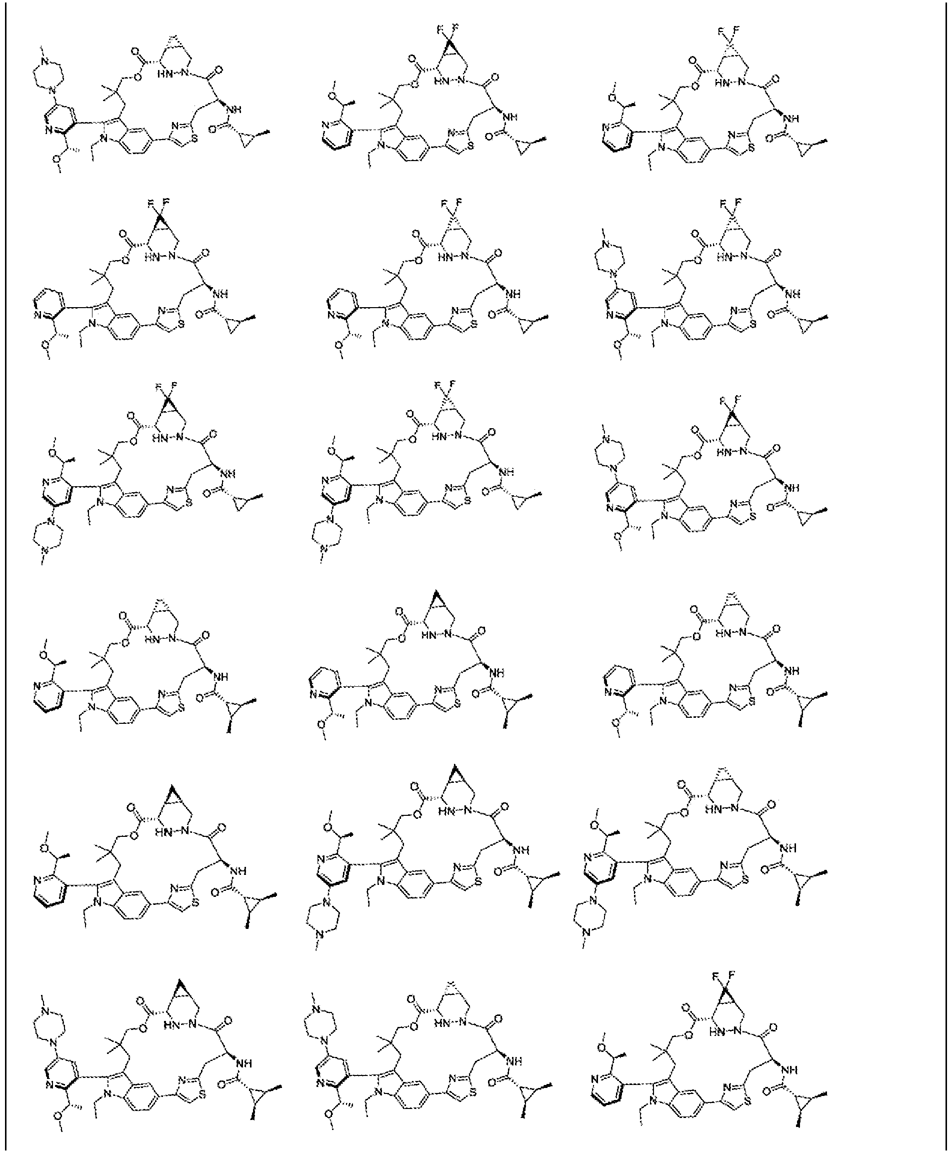

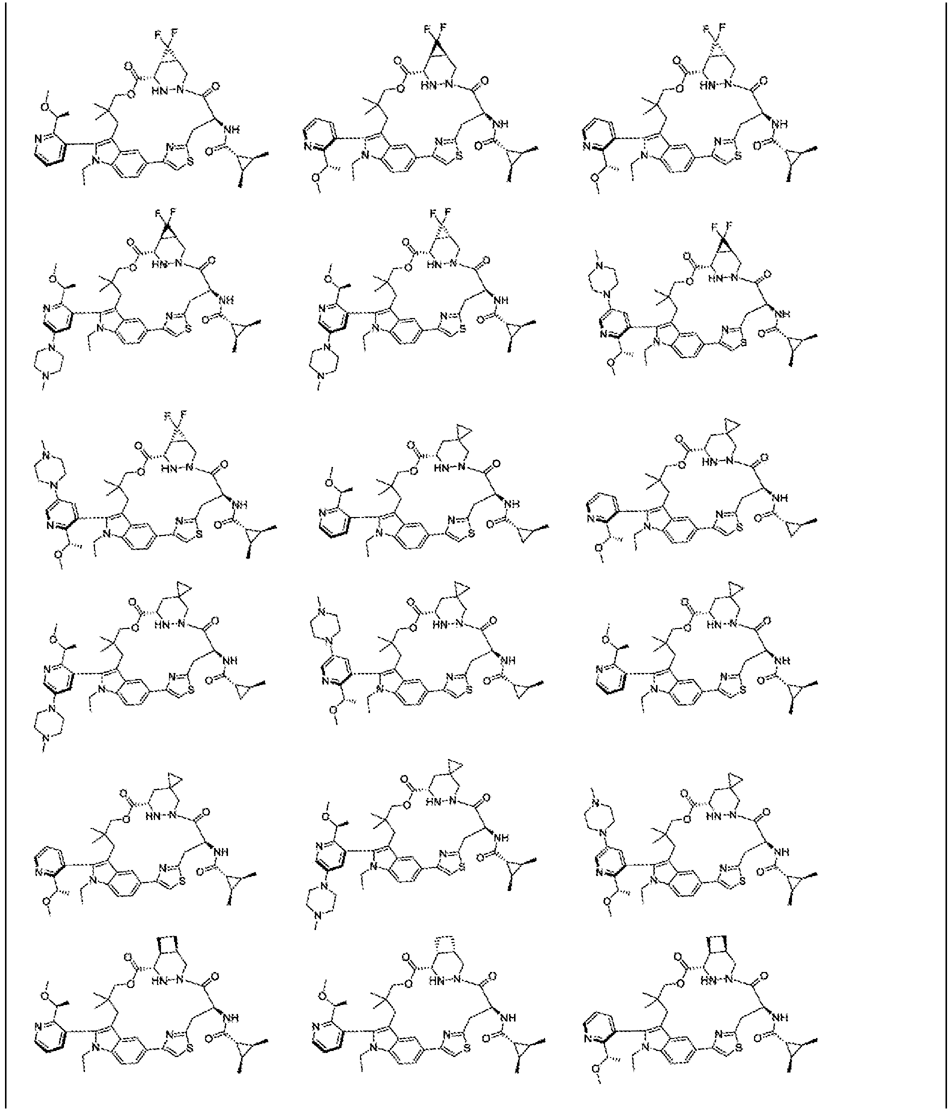

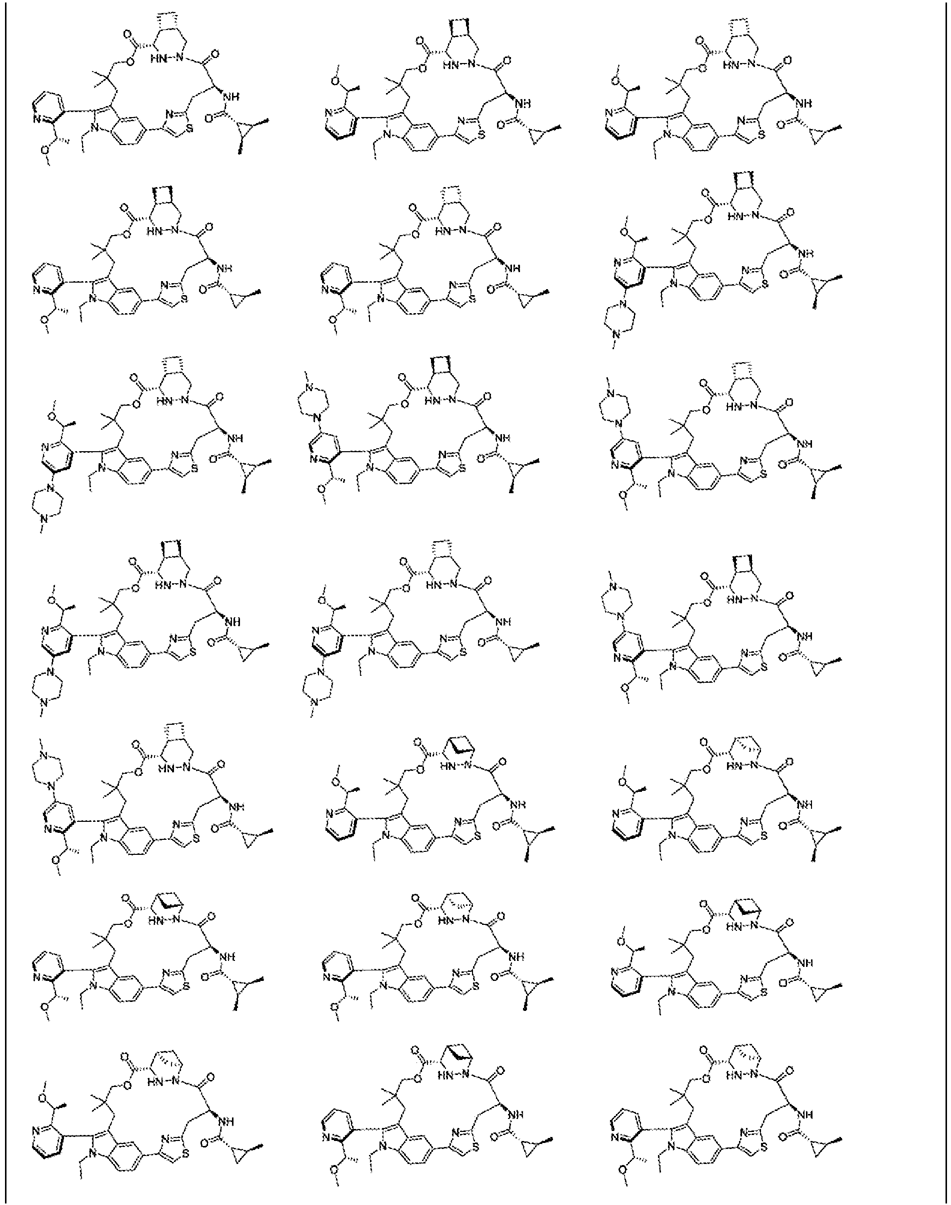

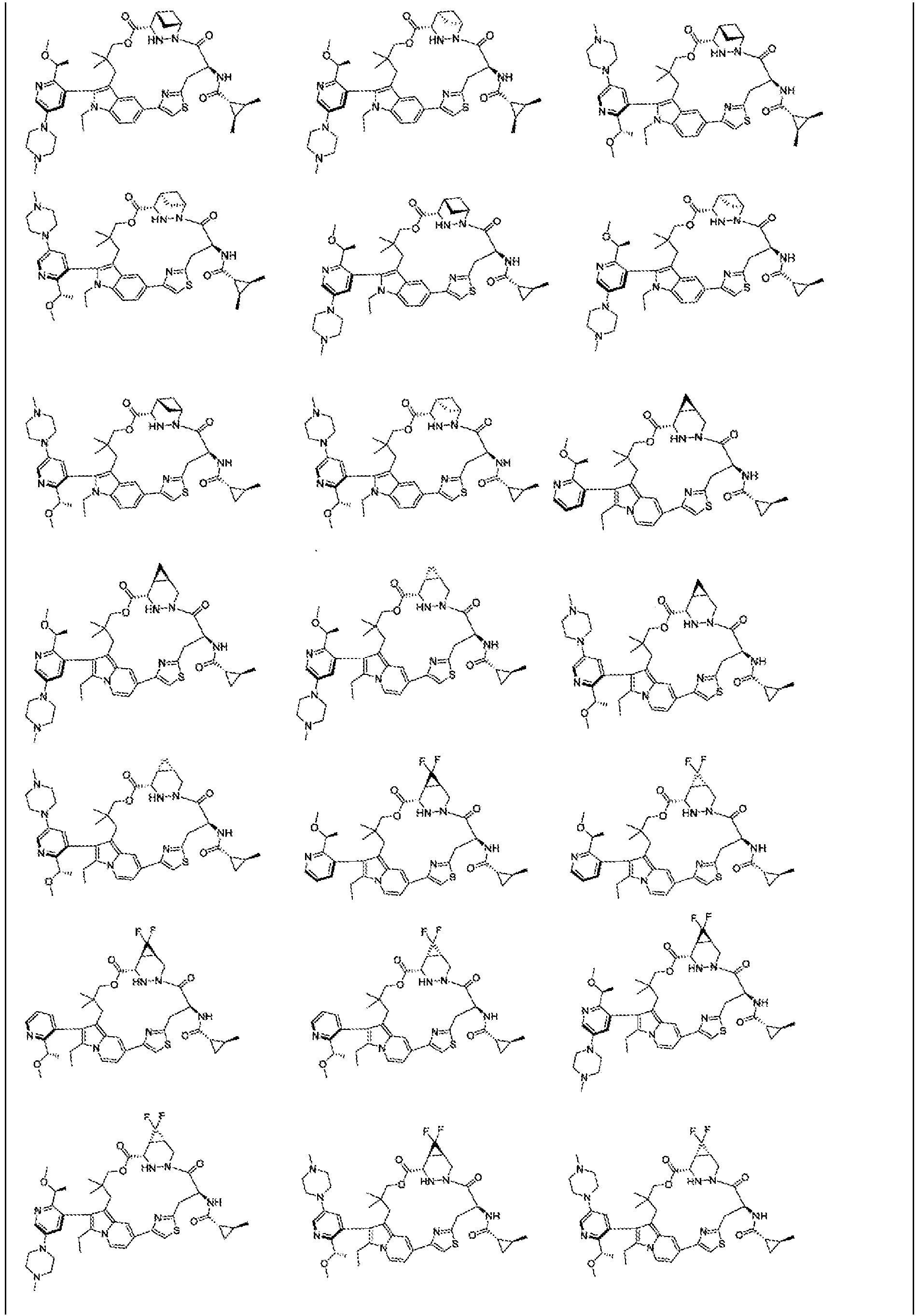

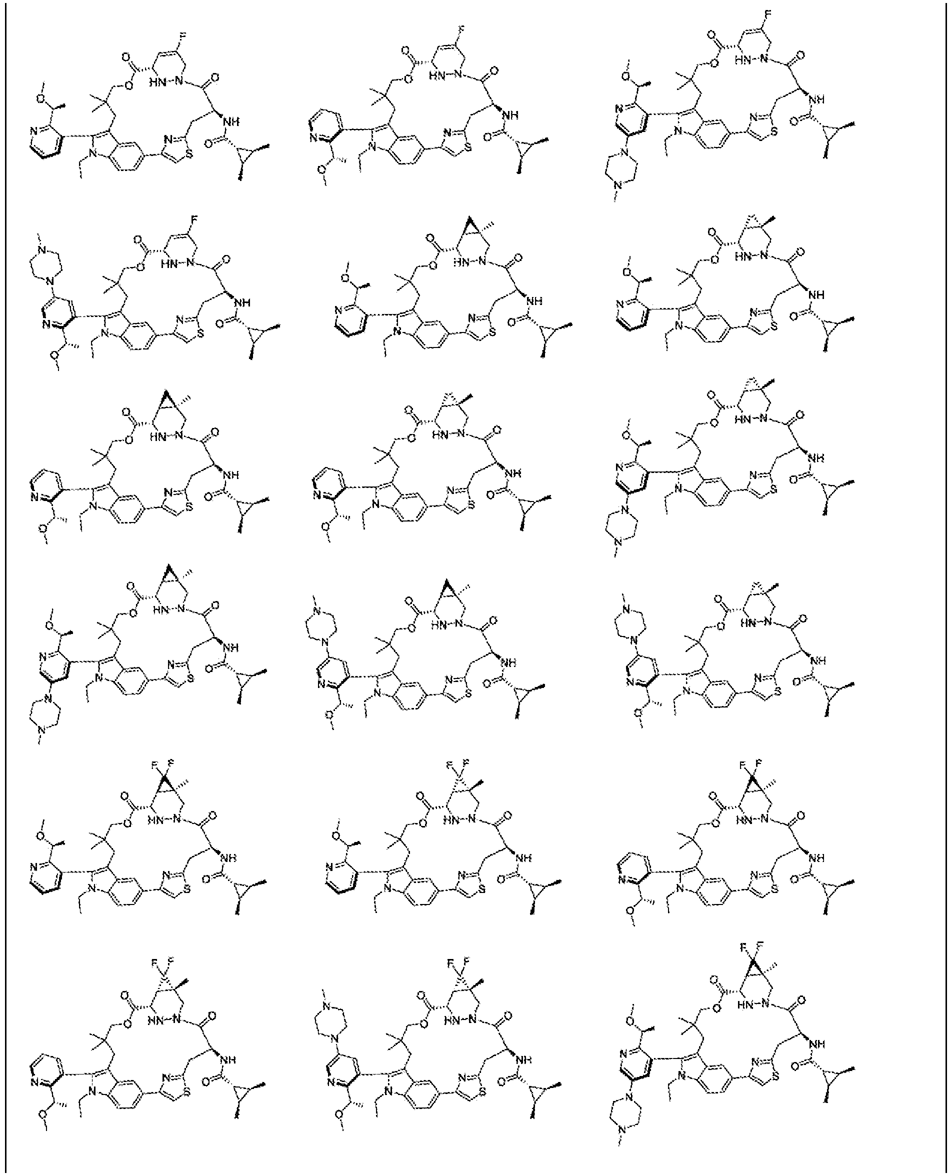

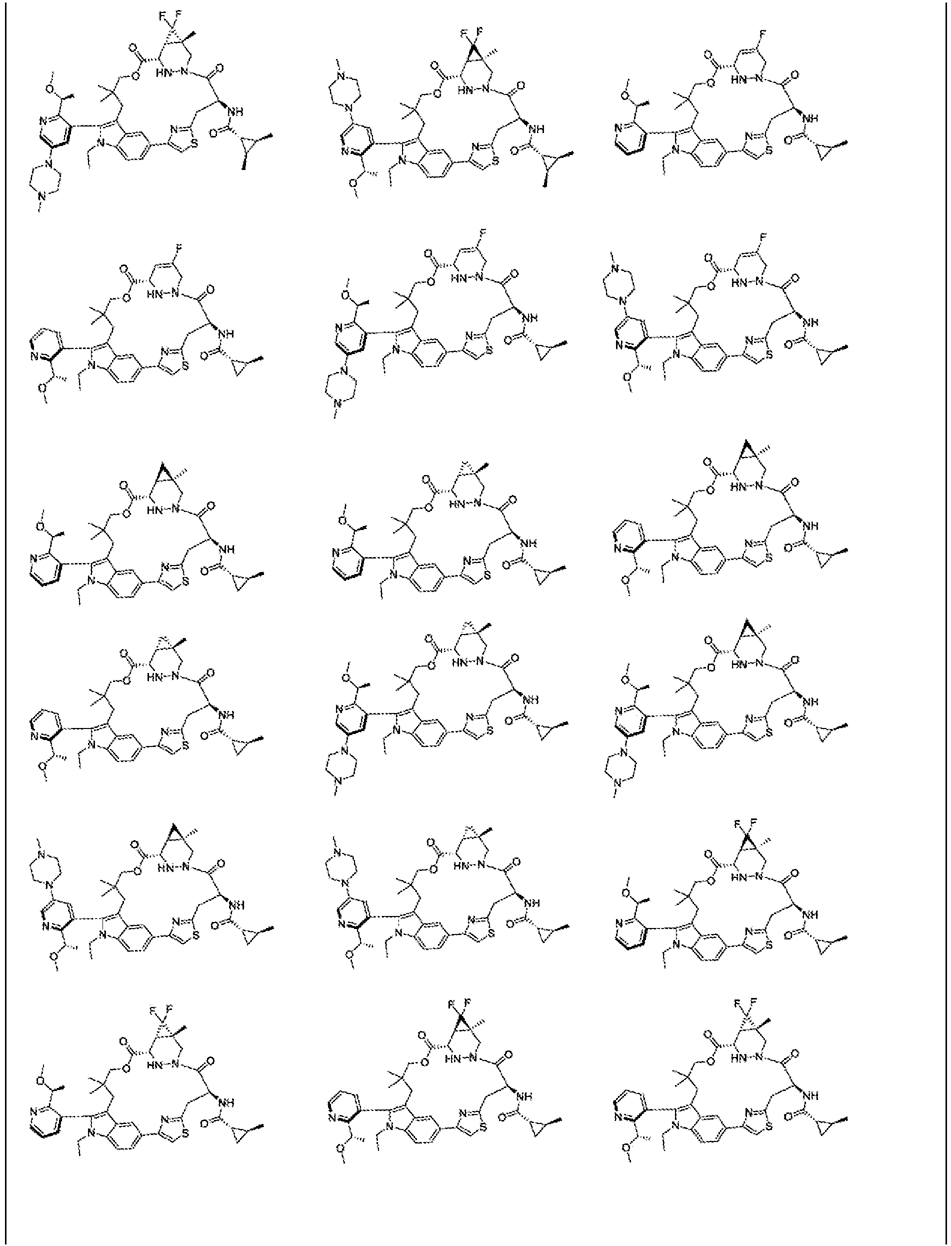

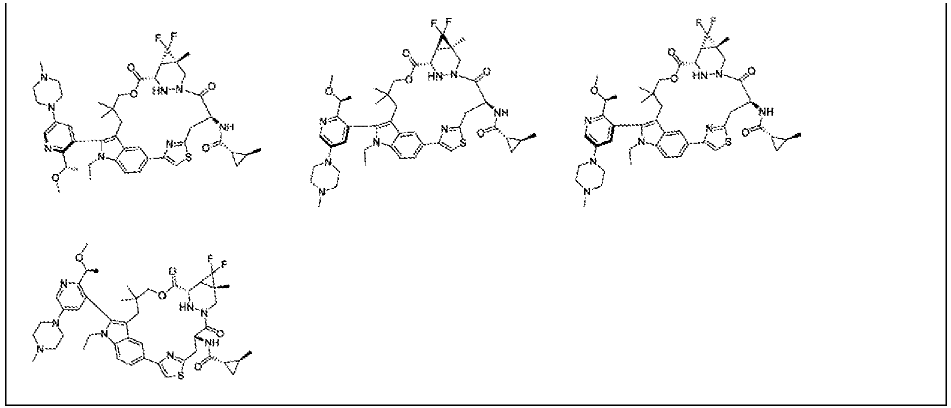

Also provided is a pharmaceutical composition comprising a compound of the present invention, or a pharmaceutically acceptable salt thereof, and a pharmaceutically acceptable excipient.

Further provided is a conjugate, or salt thereof, of a compound of the present invention, wherein the compound of the present invention has a covalent warhead, bound to a monovalent organic moiety.

In some embodiments of conjugates of the present invention, the monovalent organic moiety is a protein. In some embodiments, the protein is a Ras protein. In some embodiments, the Ras protein is K-Ras G12C, K-Ras G13C, H-Ras G12C, H-Ras G13C, N-Ras G12C, N-Ras G13C, K-Ras G12D, K-Ras G13D, K-Ras Q61H, H-Ras Q61H, N-Ras Q61H, N-Ras Q61K or N-Ras Q61R.

Compounds of the present invention are also adaptable for uses in antibody-drug conjugates as well as degrader applications.

Further provided is a method of treating cancer in a subject in need thereof, the method comprising administering to the subject a therapeutically effective amount of a compound of the present invention, or a pharmaceutically acceptable salt thereof. The cancer may, for example, be pancreatic cancer, pancreatic ductal adenocarcinoma, colorectal cancer, non-small cell lung cancer, acute myeloid leukemia, multiple myeloma, thyroid gland adenocarcinoma, a myelodysplastic syndrome, or squamous cell lung carcinoma. In some embodiments, the cancer is pancreatic cancer, pancreatic ductal adenocarcinoma, colorectal cancer, non-small cell lung cancer, acute myeloid leukemia, or multiple myeloma. In some embodiments, the cancer comprises a Ras mutation, such as K-Ras G12C, K-Ras G12D, K-Ras G12V, K-Ras G12S, K-Ras G13C, K-Ras G13D, K-Ras Q61H, K-Ras Q61R, K-Ras Q61K, or K-Ras Q61L, or a combination thereof. In some embodiments, the cancer comprises a Ras mutation, such as N-Ras G12D, N-Ras Q61R, N-Ras Q61K, N-Ras Q61L, N-Ras Q61H, or N-Ras Q61P, or a combination thereof. Other Ras mutations are described herein.

Further provided is a method of treating a Ras protein-related disorder in a subject in need thereof, the method comprising administering to the subject a therapeutically effective amount of a compound of the present invention, or a pharmaceutically acceptable salt thereof.

Further provided is a method of inhibiting a Ras protein in a cell, the method comprising contacting the cell with an effective amount of a compound of the present invention, or a

pharmaceutically acceptable salt thereof. For example, the Ras protein is K-Ras G12C, K-Ras G12D, K-Ras G12V, K-Ras G12S, K-Ras G13C, K-Ras G13D, K-Ras Q61H, K-Ras Q61R, K-Ras Q61K, or K-Ras Q61L. The Ras protein may be, for example, N-Ras G12D, N-Ras Q61R, N-Ras Q61K, N-Ras Q61L, N-Ras Q61H, or N-Ras Q61P. Other Ras proteins are described herein. The cell may be a cancer cell, such as a pancreatic cancer cell, a colorectal cancer cell, a lung cancer (e.g., non-small cell lung cancer cell), an acute myeloid leukemia cell, a multiple myeloma cell, a thyroid gland adenocarcinoma cell, a myelodysplastic syndrome cell, a melanoma cell, or a squamous cell lung carcinoma cell. Other cancer types are described herein. The cell may be in vivo or in vitro.

With respect to compounds of the present invention, one stereoisomer may exhibit better inhibition than another stereoisomer. For example, one atropisomer may exhibit inhibition, whereas the other atropisomer may exhibit little or no inhibition.

In some embodiments, a method or use described herein further comprises administering an additional anti-cancer therapy. In some embodiments, the additional anti-cancer therapy is a HER2 inhibitor, an EGFR inhibitor, a second Ras inhibitor, a SHP2 inhibitor, an SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, or a combination thereof. In some embodiments, the additional anticancer therapy is a SHP2 inhibitor. Other additional anti-cancer therapies are described herein.

### Methods of Synthesis

The compounds described herein may be made from commercially available starting materials or synthesized using known organic, inorganic, or enzymatic processes.

The compounds of the present invention can be prepared in a number of ways well known to those skilled in the art of organic synthesis. By way of example, compounds of the present invention can be synthesized using the methods described in the Scheme below, together with synthetic methods known in the art of synthetic organic chemistry, or variations thereon as appreciated by those skilled in the art. As a further example, synthetic methods described in WO 2020/132597, WO 2021/091982, WO 2021/091967, WO 2021/091956, WO 2022/060836, WO 2022/235864, WO 2022/235870, WO 2023/060253, and PCT/US2023/060288, the disclosure of each of which is incorporated herein by reference, may be useful in preparing compounds of the invention. These methods include but are not limited to those methods described in the Scheme below.

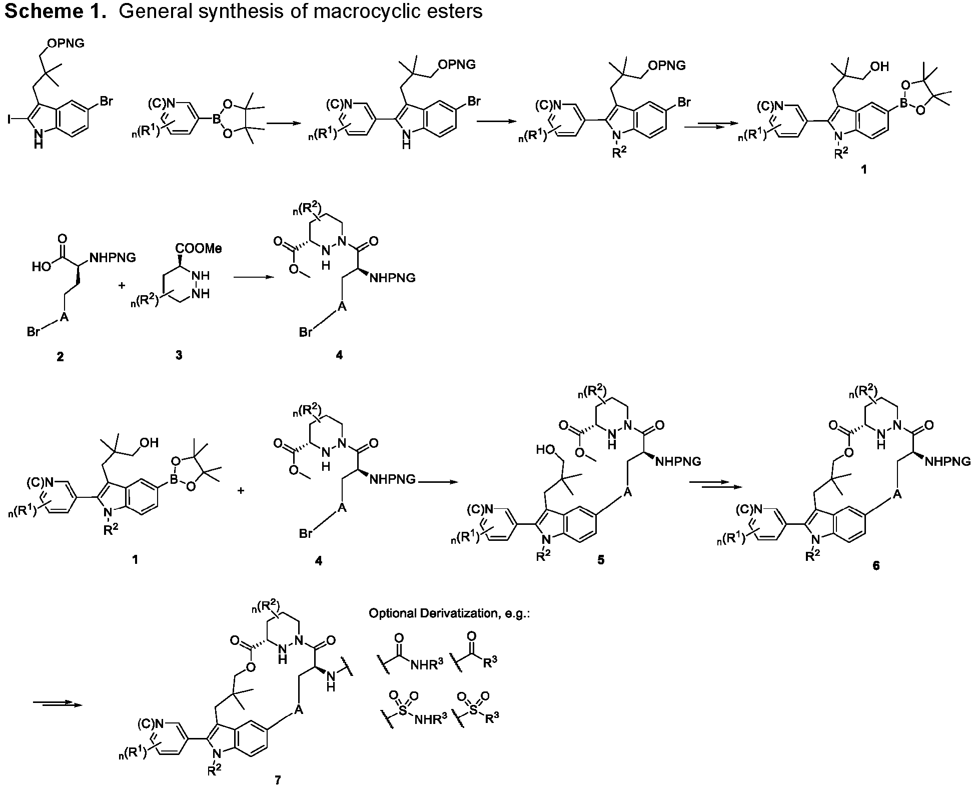

A general synthesis of macrocyclic esters is outlined in Scheme 1. An appropriately substituted indolyl boronic ester (1) can be prepared in four steps starting from protected 3-(5-bromo-2-iodo-1H-indol-3-yl)-2,2-dimethylpropan-1-ol and appropriately substituted boronic acid, including palladium mediated coupling, alkylation, de-protection, and palladium mediated borylation reactions.

Methyl (S)-hexahydropyridazine-3-carboxylate analogs (3) can be prepared by a variety of methods highlighted below.

Methyl-amino-3-(4-bromothiazol-2-yl)propanoyl)hexahydropyridazine-3-carboxylate (4) can be prepared via coupling of (S)-2-amino-3-(4-bromothiazol-2-yl)propanoic acid (2) with methyl (S)-hexahydropyridazine-3-carboxylate derivative (3).

The final macrocyclic esters can be made by coupling of methyl-amino-3-(4-bromothiazol-2-yl)propanoyl)hexahydropyridazine-3-carboxylate **(4)** and an appropriately substituted indolyl boronic ester **(1)** in the presence of Pd catalyst followed by hydrolysis and macrolactonization steps to result in an appropriately protected macrocyclic intermediate **(5).** Deprotection and coupling with an appropriately substituted carboxylic acid (or other coupling partner) results in a macrocyclic product. Additional deprotection or functionalization steps could be required to produce a final compound **(6).**

Further, with respect to Scheme 1, the thiazole may be replaced with an alternative optionally substituted 5 to 6-membered heteroarylene, an optionally substituted 3 to 6-membered cycloalkylene, optionally substituted 3 to 6-membered heterocycloalkylene (e.g., morpholino), or optionally substituted 6-membered arylene (e.g., phenyl).

### Pharmaceutical Compositions and Methods of Use

The compounds with which the invention is concerned are Ras inhibitors and are useful in the treatment of cancer. Accordingly, one embodiment of the present invention provides pharmaceutical compositions containing a compound of the invention or a pharmaceutically acceptable salt thereof, and a pharmaceutically acceptable excipient, as well as methods of using the compounds of the invention to prepare such compositions.

As used herein, the term "pharmaceutical composition" refers to a compound, such as a compound of the present invention, or a pharmaceutically acceptable salt thereof, formulated together with a pharmaceutically acceptable excipient.

In some embodiments, a compound is present in a pharmaceutical composition in unit dose amount appropriate for administration in a therapeutic regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

A "pharmaceutically acceptable excipient," as used herein, refers to any inactive ingredient (for example, a vehicle capable of suspending or dissolving the active compound) having the properties of being nontoxic and non-inflammatory in a subject. Typical excipients include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspensing or dispersing agents, sweeteners, or waters of hydration. Excipients include, but are not limited to: butylated optionally substituted hydroxyltoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, optionally substituted hydroxylpropyl cellulose, optionally substituted hydroxylpropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, stearic acid, sucrose, talc, titanium dioxide, vitamin A, vitamin E, vitamin C, and xylitol. Those of ordinary skill in the art are familiar with a variety of agents and materials useful as excipients. See, e.g., e.g., Ansel, et al., Ansel's Pharmaceutical Dosage Forms and Drug Delivery Systems. Philadelphia: Lippincott, Williams & Wilkins, 2004; Gennaro, et al., Remington: The Science and Practice of Pharmacy. Philadelphia: Lippincott, Williams & Wilkins, 2000; and Rowe, Handbook of Pharmaceutical Excipients. Chicago, Pharmaceutical Press, 2005. In some embodiments, a composition includes at least two different pharmaceutically acceptable excipients.

Compounds described herein, whether expressly stated or not, may be provided or utilized in salt form, e.g., a pharmaceutically acceptable salt form, unless expressly stated to the contrary. The term "pharmaceutically acceptable salt," as use herein, refers to those salts of the compounds described herein that are, within the scope of sound medical judgment, suitable for use in contact with the tissues of humans and other animals without undue toxicity, irritation, allergic response and the like, and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting the free base group with a suitable organic acid.

The compounds of the invention may have ionizable groups so as to be capable of preparation as pharmaceutically acceptable salts. These salts may be acid addition salts involving inorganic or organic acids or the salts may, in the case of acidic forms of the compounds of the invention, be prepared from inorganic or organic bases. In some embodiments, the compounds are prepared or used as pharmaceutically acceptable salts prepared as addition products of pharmaceutically acceptable acids or bases. Suitable pharmaceutically acceptable acids and bases are well-known in the art, such as hydrochloric, sulfuric, hydrobromic, acetic, lactic, citric, or tartaric acids for forming acid addition salts, and potassium hydroxide, sodium hydroxide, ammonium hydroxide, caffeine, various amines, and the like for forming basic salts. Methods for preparation of the appropriate salts are well-established in the art.

Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-optionally substituted hydroxyl-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts and the like. Representative alkali or alkaline earth metal salts include sodium, lithium, potassium, calcium, magnesium and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

As used herein, the term "subject" refers to any member of the animal kingdom. In some embodiments, "subject" refers to humans, at any stage of development. In some embodiments, "subject" refers to a human patient. In some embodiments, "subject" refers to non-human animals. In some embodiments, the non-human animal is a mammal (e.g., a rodent, a mouse, a rat, a rabbit, a monkey, a dog, a cat, a sheep, cattle, a primate, or a pig). In some embodiments, subjects include, but are not limited to, mammals, birds, reptiles, amphibians, fish, or worms. In some embodiments, a subject may be a transgenic animal, genetically-engineered animal, or a clone.

As used herein, the term "dosage form" refers to a physically discrete unit of a compound (e.g., a compound of the present invention) for administration to a subject. Each unit contains a predetermined quantity of compound. In some embodiments, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen). Those of ordinary skill in the art appreciate that the total amount of a therapeutic composition or compound administered to a particular subject is determined by one or more attending physicians and may involve administration of multiple dosage forms.

As used herein, the term "dosing regimen" refers to a set of unit doses (typically more than one) that are administered individually to a subject, typically separated by periods of time. In some embodiments, a given therapeutic compound (e.g., a compound of the present invention) has a recommended dosing regimen, which may involve one or more doses. In some embodiments, a dosing regimen comprises a plurality of doses each of which are separated from one another by a time period of the same length; in some embodiments, a dosing regimen comprises a plurality of doses and at least two different time periods separating individual doses. In some embodiments, all doses within a dosing regimen are of the same unit dose amount. In some embodiments, different doses within a dosing regimen are of different amounts. In some embodiments, a dosing regimen comprises a first dose in a first dose amount, followed by one or more additional doses in a second dose amount different from the first dose amount. In some embodiments, a dosing regimen comprises a first dose in a first dose amount, followed by one or more additional doses in a second dose amount same as the first dose amount. In some embodiments, a dosing regimen is correlated with a desired or beneficial outcome when administered across a relevant population (i.e., is a therapeutic dosing regimen).

A "therapeutic regimen" refers to a dosing regimen whose administration across a relevant population is correlated with a desired or beneficial therapeutic outcome.

The term "treatment" (also "treat" or "treating"), in its broadest sense, refers to any administration of a substance (e.g., a compound of the present invention) that partially or completely alleviates, ameliorates, relieves, inhibits, delays onset of, reduces severity of, or reduces incidence of one or more symptoms, features, or causes of a particular disease, disorder, or condition. In some embodiments, such treatment may be administered to a subject who does not exhibit signs of the relevant disease, disorder, or condition or of a subject who exhibits only early signs of the disease, disorder, or condition. Alternatively, or additionally, in some embodiments, treatment may be administered to a subject who exhibits one or more established signs of the relevant disease, disorder, or condition. In some embodiments, treatment may be of a subject who has been diagnosed as suffering from the relevant disease, disorder, or condition. In some embodiments, treatment may be of a subject known to have one or more susceptibility factors that are statistically correlated with increased risk of development of the relevant disease, disorder, or condition.

The term "therapeutically effective amount" means an amount that is sufficient, when administered to a population suffering from or susceptible to a disease, disorder, or condition in accordance with a therapeutic dosing regimen, to treat the disease, disorder, or condition. In some embodiments, a therapeutically effective amount is one that reduces the incidence or severity of, or delays onset of, one or more symptoms of the disease, disorder, or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered to patients in need of such treatment. It is specifically understood that particular subjects may, in fact, be "refractory" to a "therapeutically effective amount." In some embodiments, reference to a therapeutically effective amount may be a reference to an amount as measured in one or more specific tissues (e.g., a tissue affected by the disease, disorder, or condition) or fluids (e.g., blood, saliva, serum, sweat, tears, urine). Those of ordinary skill in the art will appreciate that, in some embodiments, a therapeutically effective amount may be formulated or administered in a single dose. In some embodiments, a therapeutically effective amount may be formulated or administered in a plurality of doses, for example, as part of a dosing regimen.

For use as treatment of subjects, the compounds of the invention, or a pharmaceutically acceptable salt thereof, can be formulated as pharmaceutical or veterinary compositions. Depending on the subject to be treated, the mode of administration, and the type of treatment desired, e.g., prevention, prophylaxis, or therapy, the compounds, or a pharmaceutically acceptable salt thereof, are formulated in ways consonant with these parameters. A summary of such techniques may be found in Remington: The Science and Practice of Pharmacy, 21st Edition, Lippincott Williams & Wilkins, (2005); and Encyclopedia of Pharmaceutical Technology, eds. J. Swarbrick and J. C. Boylan, 1988-1999, Marcel Dekker, New York, each of which is incorporated herein by reference.

Compositions can be prepared according to conventional mixing, granulating, or coating methods, respectively, and the present pharmaceutical compositions can contain from about 0.1% to about 99%, from about 5% to about 90%, or from about 1% to about 20% of a compound of the present invention, or pharmaceutically acceptable salt thereof, by weight or volume. In some embodiments, compounds, or a pharmaceutically acceptable salt thereof, described herein may be present in amounts totaling 1-95% by weight of the total weight of a composition, such as a pharmaceutical composition.

The composition may be provided in a dosage form that is suitable for intraarticular, oral, parenteral (e.g., intravenous, intramuscular), rectal, cutaneous, subcutaneous, topical, transdermal, sublingual, nasal, vaginal, intravesicular, intraurethral, intrathecal, epidural, aural, or ocular administration, or by injection, inhalation, or direct contact with the nasal, genitourinary, reproductive, or oral mucosa. Thus, the pharmaceutical composition may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, preparations suitable for iontophoretic delivery, or aerosols. The compositions may be formulated according to conventional pharmaceutical practice.

As used herein, the term "administration" refers to the administration of a composition (e.g., a compound, or a preparation that includes a compound as described herein) to a subject or system. Administration to an animal subject (e.g., to a human) may be by any appropriate route. For example, in some embodiments, administration may be bronchial (including by bronchial instillation), buccal, enteral, intradermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal, or vitreal.

Formulations may be prepared in a manner suitable for systemic administration or topical or local administration. Systemic formulations include those designed for injection (e.g., intramuscular, intravenous, or subcutaneous injection) or may be prepared for transdermal, transmucosal, or oral administration. A formulation will generally include a diluent as well as, in some cases, adjuvants, buffers, preservatives and the like. Compounds, or a pharmaceutically acceptable salt thereof, can be administered also in liposomal compositions or as microemulsions.

For injection, formulations can be prepared in conventional forms as liquid solutions or suspensions or as solid forms suitable for solution or suspension in liquid prior to injection or as emulsions. Suitable excipients include, for example, water, saline, dextrose, glycerol and the like. Such compositions may also contain amounts of nontoxic auxiliary substances such as wetting or emulsifying agents, pH buffering agents and the like, such as, for example, sodium acetate, sorbitan monolaurate, and so forth.

Various sustained release systems for drugs have also been devised. See, for example, U.S. Patent No. 5,624,677.

Systemic administration may also include relatively noninvasive methods such as the use of suppositories, transdermal patches, transmucosal delivery and intranasal administration. Oral administration is also suitable for compounds of the invention, or a pharmaceutically acceptable salt thereof. Suitable forms include syrups, capsules, and tablets, as is understood in the art.

Each compound, or a pharmaceutically acceptable salt thereof, as described herein, may be formulated in a variety of ways that are known in the art. For example, the first and second agents of the combination therapy may be formulated together or separately. Other modalities of combination therapy are described herein.

The individually or separately formulated agents can be packaged together as a kit. Non-limiting examples include, but are not limited to, kits that contain, e.g., two pills, a pill and a powder, a suppository and a liquid in a vial, two topical creams, etc. The kit can include optional components that aid in the administration of the unit dose to subjects, such as vials for reconstituting powder forms, syringes for injection, customized IV delivery systems, inhalers, etc. Additionally, the unit dose kit can contain instructions for preparation and administration of the compositions. The kit may be manufactured as a single use unit dose for one subject, multiple uses for a particular subject (at a constant dose or in which the individual compounds, or a pharmaceutically acceptable salt thereof, may vary in potency as therapy progresses); or the kit may contain multiple doses suitable for administration to multiple subjects ("bulk packaging"). The kit components may be assembled in cartons, blister packs, bottles, tubes, and the like.

Formulations for oral use include tablets containing the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, optionally substituted hydroxylpropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

Two or more compounds may be mixed together in a tablet, capsule, or other vehicle, or may be partitioned. In one example, the first compound is contained on the inside of the tablet, and the second compound is on the outside, such that a substantial portion of the second compound is released prior to the release of the first compound.

Formulations for oral use may also be provided as chewable tablets, or as hard gelatin capsules wherein the active ingredient is mixed with an inert solid diluent (e.g., potato starch, lactose, microcrystalline cellulose, calcium carbonate, calcium phosphate or kaolin), or as soft gelatin capsules wherein the active ingredient is mixed with water or an oil medium, for example, peanut oil, liquid paraffin, or olive oil. Powders, granulates, and pellets may be prepared using the ingredients mentioned above under tablets and capsules in a conventional manner using, e.g., a mixer, a fluid bed apparatus or a spray drying equipment.

Dissolution or diffusion-controlled release can be achieved by appropriate coating of a tablet, capsule, pellet, or granulate formulation of compounds, or by incorporating the compound, or a pharmaceutically acceptable salt thereof, into an appropriate matrix. A controlled release coating may include one or more of the coating substances mentioned above or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-optionally substituted hydroxylmethacrylate, methacrylate hydrogels, 1,3 butylene glycol, ethylene glycol methacrylate, or polyethylene glycols. In a controlled release matrix formulation, the matrix material may also include, e.g., hydrated methylcellulose, carnauba wax and stearyl alcohol, carbopol 934, silicone, glyceryl tristearate, methyl acrylate-methyl methacrylate, polyvinyl chloride, polyethylene, or halogenated fluorocarbon.

The liquid forms in which the compounds, or a pharmaceutically acceptable salt thereof, and compositions of the present invention can be incorporated for administration orally include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Generally, when administered to a human, the oral dosage of any of the compounds of the invention, or a pharmaceutically acceptable salt thereof, will depend on the nature of the compound, and can readily be determined by one skilled in the art. A dosage may be, for example, about 0.001 mg to about 2000 mg per day, about 1 mg to about 1000 mg per day, about 5 mg to about 500 mg per day, about 100 mg to about 1500 mg per day, about 500 mg to about 1500 mg per day, about 500 mg to about 2000 mg per day, or any range derivable therein.

In some embodiments, the pharmaceutical composition may further comprise an additional compound having antiproliferative activity. Depending on the mode of administration, compounds, or a pharmaceutically acceptable salt thereof, will be formulated into suitable compositions to permit facile delivery. Each compound, or a pharmaceutically acceptable salt thereof, of a combination therapy may be formulated in a variety of ways that are known in the art. For example, the first and second agents of the combination therapy may be formulated together or separately. Desirably, the first and second agents are formulated together for the simultaneous or near simultaneous administration of the agents.

It will be appreciated that the compounds and pharmaceutical compositions of the present invention can be formulated and employed in combination therapies, that is, the compounds and pharmaceutical compositions can be formulated with or administered concurrently with, prior to, or subsequent to, one or more other desired therapeutics or medical procedures. The particular combination of therapies (therapeutics or procedures) to employ in a combination regimen will take into account compatibility of the desired therapeutics or procedures and the desired therapeutic effect to be achieved. It will also be appreciated that the therapies employed may achieve a desired effect for the same disorder, or they may achieve different effects (e.g., control of any adverse effects).

Administration of each drug in a combination therapy, as described herein, can, independently, be one to four times daily for one day to one year, and may even be for the life of the subject. Chronic, long-term administration may be indicated.

### Methods of Use

In some embodiments, the invention discloses a method of treating a disease or disorder that is characterized by aberrant Ras activity due to a Ras mutant. In some embodiments, the disease or disorder is a cancer.

Accordingly, also provided is a method of treating cancer in a subject in need thereof, the method comprising administering to the subject a therapeutically effective amount of a compound of the present invention, or a pharmaceutically acceptable salt thereof, or a pharmaceutical composition comprising such a compound or salt. In some embodiments, the cancer is colorectal cancer, non-small cell lung cancer, small-cell lung cancer, pancreatic cancer, pancreatic ductal adenocarcinoma, appendiceal cancer, melanoma, acute myeloid leukemia, small bowel cancer, ampullary cancer, germ cell cancer, cervical cancer, cancer of unknown primary origin, endometrial cancer, esophagogastric cancer, GI neuroendocrine cancer, ovarian cancer, sex cord stromal tumor cancer, hepatobiliary cancer, or bladder cancer. In some embodiments, the cancer is appendiceal, endometrial or melanoma. Also provided is a method of treating a Ras protein-related disorder in a subject in need thereof, the method comprising administering to the subject a therapeutically effective amount of a compound of the present invention, or a pharmaceutically acceptable salt thereof, or a pharmaceutical composition comprising such a compound or salt.

In some embodiments, the compounds of the present invention or pharmaceutically acceptable salts thereof, pharmaceutical compositions comprising such compounds or salts, and methods provided herein may be used for the treatment of a wide variety of cancers including tumors such as lung, prostate, breast, brain, skin, cervical carcinomas, testicular carcinomas, etc. More particularly, cancers that may be treated by the compounds or salts thereof, pharmaceutical compositions comprising such compounds or salts, and methods of the invention include, but are not limited to tumor types such as astrocytic, breast, cervical, colorectal, endometrial, esophageal, gastric, head and neck, hepatocellular, laryngeal, lung, oral, ovarian, prostate, and thyroid carcinomas and sarcomas. Other cancers include, for example:
Cardiac, for example: sarcoma (angiosarcoma, fibrosarcoma, rhabdomyosarcoma, liposarcoma), myxoma, rhabdomyoma, fibroma, lipoma, and teratoma;
Lung, for example: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma;
Gastrointestinal, for example: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma);
Genitourinary tract, for example: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenomas, adenomatoid tumors, lipoma);
Liver, for example: hepatoma (hepatocellular carcinoma), cholangiocarcinoma, hepatoblastoma, angiosarcoma, hepatocellular adenoma, hemangioma;
Biliary tract, for example: gall bladder carcinoma, ampullary carcinoma, cholangiocarcinoma;
Bone, for example: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibrorna, osteoid osteoma, and giant cell tumors;
Nervous system, for example: skull (osteoma, hemangioma, granuloma, xanthoma, osteitis deformans), meninges (meningioma, meaningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, neurofibromatosis type 1, meningioma, glioma, sarcoma);
Gynecological, for example: uterus (endometrial carcinoma, uterine carcinoma, uterine corpus endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma);
Hematologic, for example: blood (myeloid leukemia (acute and chronic), acute lymphoblastic leukemia, chronic lymphocytic leukemia, myeloproliferative diseases (e.g., myelofibrosis and myeloproliferative neoplasms), multiple myeloma, myelodysplastic syndrome), Hodgkin's disease, non-Hodgkin's lymphoma (malignant lymphoma);
Skin, for example: malignant melanoma, basal cell carcinoma, squamous cell carcinoma, Kaposi's sarcoma, moles dysplastic nevi, lipoma, angioma, dermatofibroma, keloids, psoriasis; and
Adrenal glands, for example: neuroblastoma.

In some embodiments, the Ras protein is wild type (Ras^{WT}). Accordingly, in some embodiments, a compound of the present invention is employed in a method of treating a patient having a cancer comprising a Ras^{WT} (e.g., K-Ras^{WT}, H-Ras^{WT} or N-Ras^{WT}). In some embodiments, the Ras protein is Ras amplification (e.g., K-Ras^{amp}). Accordingly, in some embodiments, a compound of the present invention is employed in a method of treating a patient having a cancer comprising a Ras^{amp} (K-Ras^{amp}, H-Ras^{amp} or N-Ras^{amp}). In some embodiments, the cancer comprises a Ras mutation, such as a Ras mutation described herein. In some embodiments, a mutation is selected from:
(a) the following K-Ras mutants: G12D, G12V, G12C, G13D, G12R, G12A, Q61H, G12S, A146T, G13C, Q61L, Q61R, K117N, A146V, G12F, Q61K, L19F, Q22K, V14I, A59T, A146P, G13R, G12L, or G13V, and combinations thereof;
(b) the following H-Ras mutants: Q61R, G13R, Q61K, G12S, Q61L, G12D, G13V, G13D, G12C, K117N, A59T, G12V, G13C, Q61H, G13S, A18V, D119N, G13N, A146T, A66T, G12A, A146V, G12N, or G12R, and combinations thereof; and
(c) the following N-Ras mutants: Q61R, Q61K, G12D, Q61L, Q61H, G13R, G13D, G12S, G12C, G12V, G12A, G13V, G12R, P185S, G13C, A146T, G60E, Q61P, A59D, E132K, E49K, T50I, A146V, or A59T, and combinations thereof;
or a combination of any of the foregoing. In some embodiments, the cancer comprises a K-Ras mutation selected from the group consisting of G12C, G12D, G13C, G12V, G13D, G12R, G12S, Q61H, Q61K, Q61R and Q61L. In some embodiments, a compound of the present invention inhibits more than one Ras mutant. In some embodiments, a compound of the present invention inhibits Ras^{WT} in addition to one or more additional Ras mutations (e.g., K-, H- or N-Ras^{WT} and K-Ras G12D, G12V, G12C, G13D, G12R, G12A, Q61H, G12S, A146T, G13C, Q61L, Q61R, K117N, A146V, G12F, Q61K, L19F, Q22K, V14I, A59T, A146P, G13R, G12L, or G13V; K, H or N-Ras^{WT} and H-Ras Q61R, G13R, Q61K, G12S, Q61L, G12D, G13V, G13D, G12C, K117N, A59T, G12V, G13C, Q61H, G13S, A18V, D119N, G13N, A146T, A66T, G12A, A146V, G12N, or G12R; or K, H or N-Ras^{WT} and N-Ras Q61R, Q61K, G12D, Q61L, Q61H, G13R, G13D, G12S, G12C, G12V, G12A, G13V, G12R, P185S, G13C, A146T, G60E, Q61P, A59D, E132K, E49K, T50I, A146V, or A59T). In some embodiments, a compound of the present invention inhibits Ras^{amp} in addition to one or more additional Ras mutations (e.g., K-, H- or N-Ras^{amp} and K-Ras G12D, G12V, G12C, G13D, G12R, G12A, Q61H, G12S, A146T, G13C, Q61L, Q61R, K117N, A146V, G12F, Q61K, L19F, Q22K, V14I, A59T, A146P, G13R, G12L, or G13V; K, H or N-Ras^{amp} and H-Ras Q61R, G13R, Q61K, G12S, Q61L, G12D, G13V, G13D, G12C, K117N, A59T, G12V, G13C, Q61H, G13S, A18V, D119N, G13N, A146T, A66T, G12A, A146V, G12N, or G12R; or K, H or N-Ras^{amp} and N-Ras Q61R, Q61K, G12D, Q61L, Q61H, G13R, G13D, G12S, G12C, G12V, G12A, G13V, G12R, P185S, G13C, A146T, G60E, Q61P, A59D, E132K, E49K, T50I, A146V, or A59T).

Methods of detecting Ras mutations are known in the art. Such means include, but are not limited to direct sequencing, and utilization of a high-sensitivity diagnostic assay (with CE-IVD mark), e.g., as described in Domagala, et al., Pol J Pathol 3: 145-164 (2012), incorporated herein by reference in its entirety, including TheraScreen PCR; AmoyDx; PNAClamp; RealQuality; EntroGen; LightMix; StripAssay; Hybcell plexA; Devyser; Surveyor; Cobas; and TheraScreen Pyro. See, also, e.g., WO 2020/106640.

Also provided is a method of inhibiting a Ras protein in a cell, the method comprising contacting the cell with an effective amount of a compound of the present invention, or a pharmaceutically acceptable salt thereof. A method of inhibiting RAF-Ras binding, the method comprising contacting the cell with an effective amount of a compound of the present invention, or a pharmaceutically acceptable salt thereof, is also provided. The cell may be a cancer cell. The cancer cell may be of any type of cancer described herein. The cell may be in vivo or in vitro.

### Combination Therapy

The methods of the invention may include a compound of the invention used alone or in combination with one or more additional therapies (e.g., non-drug treatments or therapeutic agents). The dosages of one or more of the additional therapies (e.g., non-drug treatments or therapeutic agents) may be reduced from standard dosages when administered alone. For example, doses may be determined empirically from drug combinations and permutations or may be deduced by isobolographic analysis (e.g., Black et al., Neurology 65:S3-S6 (2005)).

A compound of the present invention may be administered before, after, or concurrently with one or more of such additional therapies. When combined, dosages of a compound of the invention and dosages of the one or more additional therapies (e.g., non-drug treatment or therapeutic agent) provide a therapeutic effect (e.g., synergistic or additive therapeutic effect). A compound of the present invention and an additional therapy, such as an anti-cancer agent, may be administered together, such as in a unitary pharmaceutical composition, or separately and, when administered separately, this may occur simultaneously or sequentially. Such sequential administration may be close or remote in time.

In some embodiments, the additional therapy is the administration of side-effect limiting agents (e.g., agents intended to lessen the occurrence or severity of side effects of treatment. For example, in some embodiments, the compounds of the present invention can also be used in combination with a therapeutic agent that treats nausea. Examples of agents that can be used to treat nausea include: dronabinol, granisetron, metoclopramide, ondansetron, and prochlorperazine, or pharmaceutically acceptable salts thereof.

In some embodiments, the one or more additional therapies includes a non-drug treatment (e.g., surgery or radiation therapy). In some embodiments, the one or more additional therapies includes a therapeutic agent (e.g., a compound or biologic that is an anti-angiogenic agent, signal transduction inhibitor, anti proliferative agent, glycolysis inhibitor, or autophagy inhibitor). In some embodiments, the one or more additional therapies includes a non-drug treatment (e.g., surgery or radiation therapy) and a therapeutic agent (e.g., a compound or biologic that is an anti-angiogenic agent, signal transduction inhibitor, anti proliferative agent, glycolysis inhibitor, or autophagy inhibitor). In other embodiments, the one or more additional therapies includes two therapeutic agents. In still other embodiments, the one or more additional therapies includes three therapeutic agents. In some embodiments, the one or more additional therapies includes four or more therapeutic agents.

In this Combination Therapy section, all references are incorporated by reference for the agents described, whether explicitly stated as such or not.

### Non-drug therapies

Examples of non-drug treatments include, but are not limited to, radiation therapy, cryotherapy, hyperthermia, surgery (e.g., surgical excision of tumor tissue), and T cell adoptive transfer (ACT) therapy.

In some embodiments, the compounds of the invention may be used as an adjuvant therapy after surgery. In some embodiments, the compounds of the invention may be used as a neo-adjuvant therapy prior to surgery.

Radiation therapy may be used for inhibiting abnormal cell growth or treating a hyperproliferative disorder, such as cancer, in a subject (e.g., mammal (e.g., human)). Techniques for administering radiation therapy are known in the art. Radiation therapy can be administered through one of several methods, or a combination of methods, including, without limitation, external-beam therapy, internal radiation therapy, implant radiation, stereotactic radiosurgery, systemic radiation therapy, radiotherapy, and permanent or temporary interstitial brachy therapy. The term "brachy therapy," as used herein, refers to radiation therapy delivered by a spatially confined radioactive material inserted into the body at or near a tumor or other proliferative tissue disease site. The term is intended, without limitation, to include exposure to radioactive isotopes (e.g., At-211, 1-131, I-125, Y-90, Re-186, Re-188, Sm-153, Bi-212, P-32, and radioactive isotopes of Lu). Suitable radiation sources for use as a cell conditioner of the present invention include both solids and liquids. By way of non-limiting example, the radiation source can be a radionuclide, such as I-125, 1-131, Yb-169, Ir-192 as a solid source, 1-125 as a solid source, or other radionuclides that emit photons, beta particles, gamma radiation, or other therapeutic rays. The radioactive material can also be a fluid made from any solution of radionuclide(s), e.g., a solution of I-125 or I-131, or a radioactive fluid can be produced using a slurry of a suitable fluid containing small particles of solid radionuclides, such as Au-198, or Y-90. Moreover, the radionuclide(s) can be embodied in a gel or radioactive micro spheres.

In some embodiments, the compounds of the present invention can render abnormal cells more sensitive to treatment with radiation for purposes of killing or inhibiting the growth of such cells. Accordingly, this invention further relates to a method for sensitizing abnormal cells in a mammal to treatment with radiation which comprises administering to the mammal an amount of a compound of the present invention, which amount is effective to sensitize abnormal cells to treatment with radiation. The amount of the compound in this method can be determined according to the means for ascertaining effective amounts of such compounds described herein. In some embodiments, the compounds of the present invention may be used as an adjuvant therapy after radiation therapy or as a neo-adjuvant therapy prior to radiation therapy.

In some embodiments, the non-drug treatment is a T cell adoptive transfer (ACT) therapy. In some embodiments, the T cell is an activated T cell. The T cell may be modified to express a chimeric antigen receptor (CAR). CAR modified T (CAR-T) cells can be generated by any method known in the art. For example, the CAR-T cells can be generated by introducing a suitable expression vector encoding the CAR to a T cell. Prior to expansion and genetic modification of the T cells, a source of T cells is obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available in the art may be used. In some embodiments, the T cell is an autologous T cell. Whether prior to or after genetic modification of the T cells to express a desirable protein (e.g., a CAR), the T cells can be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 7,572,631; 5,883,223; 6,905,874; 6,797,514; and 6,867,041.

### Therapeutic agents

A therapeutic agent may be a compound used in the treatment of cancer or symptoms associated therewith. A compound of the present invention may be combined with a second, third, or fourth therapeutic agent, or more. A compound of the present invention may be combined with one or more therapeutic agents along with one or more non-drug therapies.

For example, a therapeutic agent may be a steroid. Steroids are known in the art. Accordingly, in some embodiments, the one or more additional therapies includes a steroid. Suitable steroids may include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, fiucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts or derivatives thereof.

Further examples of therapeutic agents that may be used in combination therapy with a compound of the present invention include compounds described in the following patents: U.S. Patent Nos. 6,258,812, 6,630,500, 6,515,004, 6,713,485, 5,521,184, 5,770,599, 5,747,498, 5,990,141, 6,235,764, and 8,623,885, and International Patent Applications WO01/37820, WO01/32651, WO02/68406, WO02/66470, WO02/55501, WO04/05279, WO04/07481, WO04/07458, WO04/09784, WO02/59110, WO99/45009, WO00/59509, WO99/61422, WO00/12089, and WO00/02871.

A therapeutic agent may be a biologic (e.g., cytokine (e.g., interferon or an interleukin such as IL-2)) used in treatment of cancer or symptoms associated therewith. Biologics are known in the art. In some embodiments, the biologic is an immunoglobulin-based biologic, e.g., a monoclonal antibody (e.g., a humanized antibody, a fully human antibody, an Fc fusion protein, or a functional fragment thereof) that agonizes a target to stimulate an anti-cancer response or antagonizes an antigen important for cancer. Also included are antibody-drug conjugates.

A therapeutic agent may be a T-cell checkpoint inhibitor. Such checkpoint inhibitors are known in the art. In one embodiment, the checkpoint inhibitor is an inhibitory antibody (e.g., a monospecific antibody such as a monoclonal antibody). The antibody may be, e.g., humanized or fully human. In some embodiments, the checkpoint inhibitor is a fusion protein, e.g., an Fc-receptor fusion protein. In some embodiments, the checkpoint inhibitor is an agent, such as an antibody, that interacts with a checkpoint protein. In some embodiments, the checkpoint inhibitor is an agent, such as an antibody, that interacts with the ligand of a checkpoint protein. In some embodiments, the checkpoint inhibitor is an inhibitor (e.g., an inhibitory antibody or small molecule inhibitor) of CTLA-4 (e.g., an anti-CTLA-4 antibody or fusion a protein). In some embodiments, the checkpoint inhibitor is an inhibitor or antagonist (e.g., an inhibitory antibody or small molecule inhibitor) of PD-1. In some embodiments, the checkpoint inhibitor is an inhibitor or antagonist (e.g., an inhibitory antibody or small molecule inhibitor) of PD-L1. In some embodiments, the checkpoint inhibitor is an inhibitor or antagonist (e.g., an inhibitory antibody or Fc fusion or small molecule inhibitor) of PD-L2 (e.g., a PD-L2/lg fusion protein). In some embodiments, the checkpoint inhibitor is an inhibitor or antagonist (e.g., an inhibitory antibody or small molecule inhibitor) of B7-H3, B7-H4, BTLA, HVEM, TIM3, GAL9, LAG3, VISTA, KIR, 2B4, CD160, CGEN-15049, CHK 1, CHK2, A2aR, B-7 family ligands, or a combination thereof. In some embodiments, the checkpoint inhibitor is pembrolizumab, nivolumab, PDR001 (NVS), REGN2810 (Sanofi/Regeneron), a PD-L1 antibody such as, e.g., avelumab, durvalumab, atezolizumab, pidilizumab, JNJ-63723283 (JNJ), BGB-A317 (BeiGene & Celgene) or a checkpoint inhibitor disclosed in Preusser, M. et al. (2015) Nat. Rev. Neurol., including, without limitation, ipilimumab, tremelimumab, nivolumab, pembrolizumab, AMP224, AMP514/ MEDI0680, BMS936559, MEDI4736, MPDL3280A, MSB0010718C, BMS986016, IMP321, lirilumab, IPH2101, 1-7F9, and KW-6002.

A therapeutic agent may be an anti-TIGIT antibody, such as MBSA43, BMS-986207, MK-7684, COM902, AB154, MTIG7192A or OMP-313M32 (etigilimab). Other anti-TIGIT antibodies are known in the art.

A therapeutic agent may be an agent that treats cancer or symptoms associated therewith (e.g., a cytotoxic agent, non-peptide small molecules, or other compound useful in the treatment of cancer or symptoms associated therewith, collectively, an "anti-cancer agent"). Anti-cancer agents can be, e.g., chemotherapeutics or targeted therapy agents. Such agents are known in the art.

Anti-cancer agents include mitotic inhibitors, intercalating antibiotics, growth factor inhibitors, cell cycle inhibitors, enzymes, topoisomerase inhibitors, biological response modifiers, alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Further anti-cancer agents include leucovorin (LV), irenotecan, oxaliplatin, capecitabine, paclitaxel, and doxetaxel. In some embodiments, the one or more additional therapies includes two or more anti-cancer agents. The two or more anti-cancer agents can be used in a cocktail to be administered in combination or administered separately. Suitable dosing regimens of combination anti-cancer agents are known in the art and described in, for example, Saltz et al., Proc. Am. Soc. Clin. Oncol. 18:233a (1999), and Douillard et al., Lancet 355(9209):1041-1047 (2000).

Other non-limiting examples of anti-cancer agents include Gleevec^{®} (Imatinib Mesylate); Kyprolis^{®} (carfilzomib); Velcade^{®} (bortezomib); Casodex (bicalutamide); Iressa^{®} (gefitinib); alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; sarcodictyin A; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimustine; antibiotics such as the enediyne antibiotics (e.g., calicheamicin, such as calicheamicin gammall and calicheamicin omegall (see, e.g., Agnew, Chem. Intl. Ed Engl. 33:183-186 (1994)); dynemicin such as dynemicin A; bisphosphonates such as clodronate; an esperamicin; neocarzinostatin chromophore and related chromoprotein enediyne antiobiotic chromophores, aclacinomysins, actinomycin, authramycin, azaserine, bleomycins, cactinomycin, calicheamicin, carabicin, caminomycin, carminomycin, carzinophilin, chromomycins, dactinomycin, daunorubicin, detorubicin, 6-diazo- 5-oxo-L-norleucine, adriamycin (doxorubicin), morpholino-doxorubicin, cyanomorpholino-doxorubicin, 2-pyrrolino-doxorubicin, deoxydoxorubicin, epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenishers such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone such as epothilone B; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes such as T-2 toxin, verracurin A, roridin A and anguidine; urethane; vindesine; dacarbazine; mannomustine; mitobronitol; mitolactol; pipobroman; gacytosine; arabinoside ("Ara-C"); cyclophosphamide; thiotepa; taxoids, e.g., Taxol^{®} (paclitaxel), Abraxane^{®} (cremophor-free, albumin-engineered nanoparticle formulation of paclitaxel), and Taxotere^{®} (doxetaxel); chloranbucil; tamoxifen (Nolvadex^{™}); raloxifene; aromatase inhibiting 4(5)-imidazoles; 4-hydroxytamoxifen; trioxifene; keoxifene; LY 117018; onapristone; toremifene (Fareston^{®}); flutamide, nilutamide, bicalutamide, leuprolide, goserelin; chlorambucil; Gemzar^{®} gemcitabine; 6-thioguanine; mercaptopurine; platinum coordination complexes such as cisplatin, oxaliplatin and carboplatin; vinblastine; platinum; etoposide (VP-16); ifosfamide; mitoxantrone; vincristine; Navelbine^{®} (vinorelbine); novantrone; teniposide; edatrexate; daunomycin; aminopterin; ibandronate; irinotecan (e.g., CPT-11); topoisomerase inhibitor RFS 2000; difluoromethylornithine (DMFO); retinoids such as retinoic acid; esperamicins; capecitabine (e.g., Xeloda^{®}); and pharmaceutically acceptable salts of any of the above.

Additional non-limiting examples of anti-cancer agents include trastuzumab (Herceptin^{®}), bevacizumab (Avastin^{®}), cetuximab (Erbitux^{®}), rituximab (Rituxan^{®}), Taxol^{®}, Arimidex^{®}, ABVD, avicine, abagovomab, acridine carboxamide, adecatumumab, 17-N-allylamino-17-demethoxygeldanamycin, alpharadin, alvocidib, 3-aminopyridine-2-carboxaldehyde thiosemicarbazone, amonafide, anthracenedione, anti-CD22 immunotoxins, antineoplastics (e.g., cell-cycle nonspecific antineoplastic agents, and other antineoplastics described herein), antitumorigenic herbs, apaziquone, atiprimod, azathioprine, belotecan, bendamustine, BIBW 2992, biricodar, brostallicin, bryostatin, buthionine sulfoximine, CBV (chemotherapy), calyculin, dichloroacetic acid, discodermolide, elsamitrucin, enocitabine, eribulin, exatecan, exisulind, ferruginol, forodesine, fosfestrol, ICE chemotherapy regimen, IT-101, imexon, imiquimod, indolocarbazole, irofulven, laniquidar, larotaxel, lenalidomide, lucanthone, lurtotecan, mafosfamide, mitozolomide, nafoxidine, nedaplatin, olaparib, ortataxel, PAC-1, pawpaw, pixantrone, proteasome inhibitors, rebeccamycin, resiquimod, rubitecan, SN-38, salinosporamide A, sapacitabine, Stanford V, swainsonine, talaporfin, tariquidar, tegafur-uracil, temodar, tesetaxel, triplatin tetranitrate, tris(2-chloroethyl)amine, troxacitabine, uramustine, vadimezan, vinflunine, ZD6126, and zosuquidar.

Further non-limiting examples of anti-cancer agents include natural products such as vinca alkaloids (e.g., vinblastine, vincristine, and vinorelbine), epidipodophyllotoxins (e.g., etoposide and teniposide), antibiotics (e.g., dactinomycin (actinomycin D), daunorubicin, and idarubicin), anthracyclines, mitoxantrone, bleomycins, plicamycin (mithramycin), mitomycin, enzymes (e.g., L-asparaginase which systemically metabolizes L-asparagine and deprives cells which do not have the capacity to synthesize their own asparagine), antiplatelet agents, antiproliferative/antimitotic alkylating agents such as nitrogen mustards (e.g., mechlorethamine, cyclophosphamide and analogs, melphalan, and chlorambucil), ethylenimines and methylmelamines (e.g., hexamethylmelamine and thiotepa), CDK inhibitors (e.g., a CDK4/6 inhibitor such as abemaciclib, ribociclib, palbociclib; seliciclib, UCN-01, P1446A-05, PD-0332991, dinaciclib, P27-00, AT-7519, RGB286638, and SCH727965), alkyl sulfonates (e.g., busulfan), nitrosoureas (e.g., carmustine (BCNU) and analogs, and streptozocin), trazenes-dacarbazinine (DTIC), antiproliferative/antimitotic antimetabolites such as folic acid analogs, pyrimidine analogs (e.g., fluorouracil, floxuridine, and cytarabine), purine analogs and related inhibitors (e.g., mercaptopurine, thioguanine, pentostatin, and 2-chlorodeoxyadenosine), aromatase inhibitors (e.g., anastrozole, exemestane, and letrozole), and platinum coordination complexes (e.g., cisplatin and carboplatin), procarbazine, hydroxyurea, mitotane, aminoglutethimide, histone deacetylase (HDAC) inhibitors (e.g., trichostatin, sodium butyrate, apicidan, suberoyl anilide hydroamic acid, vorinostat, belinostat, LBH 589, romidepsin, ACY-1215, and panobinostat), mTOR inhibitors (e.g., vistusertib, temsirolimus, everolimus, ridaforolimus, and sirolimus), KSP(Eg5) inhibitors (e.g., Array 520), DNA binding agents (e.g., Zalypsis^{®}), PI3K inhibitors such as PI3K delta inhibitor (e.g., GS-1101 and TGR-1202), PI3K delta and gamma inhibitor (e.g., CAL-130), copanlisib, alpelisib and idelalisib; multi-kinase inhibitor (e.g., TG02 and sorafenib), hormones (e.g., estrogen) and hormone agonists such as luteinizing hormone releasing hormone (LHRH) agonists (e.g., goserelin, leuprolide and triptorelin), BAFF-neutralizing antibody (e.g., LY2127399), IKK inhibitors, p38MAPK inhibitors, anti-IL-6 (e.g., CNT0328), telomerase inhibitors (e.g., GRN 163L), aurora kinase inhibitors (e.g., MLN8237), cell surface monoclonal antibodies (e.g., anti-CD38 (HUMAX-CD38), anti-CSI (e.g., elotuzumab), HSP90 inhibitors (e.g., 17 AAG and KOS 953), P13K / Akt inhibitors (e.g., perifosine), Akt inhibitors (e.g., GSK-2141795), PKC inhibitors (e.g., enzastaurin), FTIs (e.g., Zarnestra^{™}), anti-CD138 (e.g., BT062), Torcl/2 specific kinase inhibitors (e.g., INK128), ER/UPR targeting agents (e.g., MKC-3946), cFMS inhibitors (e.g., ARRY-382), JAK1/2 inhibitors (e.g., CYT387), PARP inhibitors (e.g., olaparib and veliparib (ABT-888)), and BCL-2 antagonists.

In some embodiments, an anti-cancer agent is selected from mechlorethamine, camptothecin, ifosfamide, tamoxifen, raloxifene, gemcitabine, Navelbine^{®}, sorafenib, or any analog or derivative variant of the foregoing.

In some embodiments, the anti-cancer agent is a HER2 inhibitor. HER2 inhibitors are known in the art. Non-limiting examples of HER2 inhibitors include monoclonal antibodies such as trastuzumab (Herceptin^{®}) and pertuzumab (Perjeta^{®}); small molecule tyrosine kinase inhibitors such as gefitinib (Iressa^{®}), erlotinib (Tarceva^{®}), pilitinib, CP-654577, CP-724714, canertinib (CI 1033), HKI-272, lapatinib (GW-572016; Tykerb^{®}), PKI-166, AEE788, BMS-599626, HKI-357, BIBW 2992, ARRY-334543, and JNJ-26483327.

In some embodiments, an anti-cancer agent is an ALK inhibitor. ALK inhibitors are known in the art. Non-limiting examples of ALK inhibitors include ceritinib, TAE-684 (NVP-TAE694), PF02341066 (crizotinib or 1066), alectinib; brigatinib; entrectinib; ensartinib (X-396); lorlatinib; ASP3026; CEP-37440; 4SC-203; TL-398; PLB1003; TSR-011; CT-707; TPX-0005, and AP26113. Additional examples of ALK kinase inhibitors are described in examples 3-39 of WO05016894.

In some embodiments, an anti-cancer agent is an inhibitor of a member downstream of a Receptor Tyrosine Kinase (RTK)/Growth Factor Receptor (e.g., a SHP2 inhibitor (e.g., SHP099, TNO155, RMC-4550, RMC-4630, JAB-3068, JAB-3312, RLY-1971, ERAS-601, SH3809, PF-07284892, or BBP-398), or a pharmaceutically acceptable salt, solvate, isomer (e.g., stereoisomer), prodrug, or tautomer thereof), an SOS1 inhibitor (e.g., BI-1701963, BI-3406, SDR5, BAY-293, MRTX-0902, or RMC-5845, or a pharmaceutically acceptable salt, solvate, isomer (e.g., stereoisomer), prodrug, or tautomer thereof), a Raf inhibitor, a MEK inhibitor, an ERK inhibitor, a PI3K inhibitor, a PTEN inhibitor, an AKT inhibitor, or an mTOR inhibitor (e.g., mTORC1 inhibitor or mTORC2 inhibitor). In some embodiments, the anti-cancer agent is JAB-3312.

In some embodiments, an anti-cancer agent is a SOS1 inhibitor. SOS1 inhibitors are known in the art. In some embodiments, the SOS1 inhibitor is selected from those disclosed in WO 2022219035, WO 2022214594, WO 2022199670, WO 2022146698, WO 2022081912, WO 2022058344, WO 2022026465, WO 2022017519, WO 2021173524, WO 2021130731, WO 2021127429, WO 2021092115, WO 2021105960, WO 2021074227, WO 2020180768, WO 2020180770, WO 2020173935, WO 2020146470, WO 2019201848, WO 2019122129, WO 2018172250, and WO 2018115380, or a pharmaceutically acceptable salt, solvate, isomer (e.g., stereoisomer), prodrug, or tautomer thereof.

In some embodiments, an anti-cancer agent is an additional Ras inhibitor or a Ras vaccine, or another therapeutic modality designed to directly or indirectly decrease the oncogenic activity of Ras. Such agents are known in the art. In some embodiments, an anti-cancer agent is an additional Ras inhibitor. In some embodiments, the Ras inhibitor targets Ras in its active, or GTP-bound state (RAS(ON)). In some embodiments, the Ras inhibitor targets Ras in its inactive, or GDP-bound state(RAS(OFF)). For example, a KRAS(OFF) inhibitor may be selective for more than one mutant, or selective for one or more mutants and for wild-type (in either situation, a "pan-KRAS(OFF)" inhibitor). Numerous mutant-selective RAS (namely KRAS) and pan-KRAS inhibitors have been disclosed. In some embodiments, the Ras inhibitor is, such as an inhibitor of K-Ras G12C, such as AMG 510, MRTX1257, MRTX849, JNJ-74699157, LY3499446, ARS-1620, ARS-853, BPI-421286, LY3537982 (olomorasib), JDQ443 (opnurasib),, JAB-3312, JAB-21822 (glecirasib), JAB-21000, IBI351 (GFH925), ERAS-3490, RMC-6291, BI 1823911, D-1553, D3S-001, HBI-2438, HS-10370, MK-1084, YL-15293, BBO-11818, BBO-8520 (ON/OFF inhibitor), FMC-376 (ON/OFF inhibitor), GEC255, or GDC-6036. In some embodiments, the Ras inhibitor is an inhibitor of K-Ras G12D, such as MRTX1133, JAB-22000, MRTX282, ERAS-4, ERAS-5024, HRS-4642, BI-2852, ASP3082, TH-Z827, TH-7835, QTX-3046, GFH375 (VS-7375), INCB161734 and KD-8. In some embodiments, the Ras inhibitor is a K-Ras G12V inhibitor, such as JAB-23000. In some embodiments, the Ras inhibitor is JAB-23400. In some embodiments, the Ras inhibitor is selected from a Ras(ON) inhibitor (that is, Ras in its GTP-bound state) disclosed in the following, incorporated herein by reference in their entireties, or a pharmaceutically acceptable salt, solvate, isomer (e.g., stereoisomer), prodrug, or tautomer thereof: WO 2024/102421, WO 2023/240263, WO 2023/133543, WO 2023/060253, WO 2023/025832, WO 2023/015559, WO 2023/133543, WO 2022/251292, WO 2022/235870, WO 2022/235864, WO 2022/060836, WO 2021091982, WO 2021091967, WO 2021091956, and WO 2020132597, or PCT application serial number PCT/US2023/037057, PCT/US2024/023272 or PCT/US2024/023208, or WO 2024/067857, WO 2024/060966, WO 2024/017859, WO 2024/008834, WO 2024/008610, WO 2023/232776, WO 2023/208005, WO 2023/086341, WO 2023/025832, WO 2023/015559, CN 117720556, CN 117720555, CN 117720554, CN 1177534687, CN 11753685, or CN 11753684, each of which is incorporated by reference in its entirety, or a combination of any such RAS(ON) inhibitors. Methods of determining RAS(ON) inhibition are known in the art. See, e.g., WO 2021/091956 and WO 2022/060836. In some embodiments, the Ras(ON) inhibitor is RMC-6236, RMC-9805, RMC-6291, RMC-8839, RMC-0708 or RMC-5127. Other examples of Ras inhibitors are known in the art, such as in the following, incorporated herein by reference in their entireties: WO 2024/097559, WO 2024/088273, WO 2024/091409, WO 2024085661, WO 2024083258, WO 2024083256, WO 2024083246, WO 2024083168, WO 2024078555, WO 2024076674, WO 2024076672, WO 2024076670, WO 2024067714, WO 2024067575, WO 2024064335, WO 2024063578, WO 2024063576, WO 2024061370, WO 2024061333, WO 2024061267, WO 2024056063, WO 2024055112, WO 2024054926, WO 2024054647, WO 2024054625, WO 2024051763, WO 2024051721, WO 2024050742, WO 2024050640, WO 2024046406, WO 2024046370, WO 2024045066, WO 2024044667, WO 2024044649, WO 2024044334, WO 2024041621, WO 2024041606, WO 2024041589, WO 2024041573, WO 2024040131, WO 2024040109, WO 2024040080, WO 2024036270, WO 2024034657, WO 2024034593, WO 2024034591, WO 2024034123, WO 2024032747, WO 2024032704, WO 2024032703, WO 2024032702, WO 2024031088, WO 2024030647, WO 2024030633, WO 2024029613, WO 2024022507, WO 2024022444, WO 2024020159, WO 2024019103, WO 2024017859, WO 2024017392, WO 2024015731, WO 2024015262, WO 2024012456, WO 2024009191, WO 2024008179, WO 2024008178, WO 2024008068, WO 2024006445, WO 2024006424, WO 2024002373, WO 2023287896, WO 2023287730, WO 2023284881, WO 2023284730, WO 2023284537, WO 2023283933, WO 2023283213, WO 2023280960, WO 2023280280, WO2023278600, WO 2023280136, WO 2023280026, WO 2023278600, WO 2023274383, WO 2023274324, WO 2023034290, WO 2023020523, WO 2023020521, WO 2023020519, WO 2023020518, WO 2023018812, WO 2023018810, WO 2023018809, WO 2023018699, WO 2023015559, WO 2023014979, WO 2023014006, WO 2023010121, WO 2023009716, WO 2023009572, WO 2023004102, WO 2023003417, WO 2023001141, WO 2023001123, WO 2022271923, WO 2022271823, WO 2022271810, WO 2022271658, WO 2022269508, WO 2022266167, WO 2022266069, WO 2022266015, WO 2022265974, WO 2022261154, WO 2022261154, WO 2022251576, WO 2022251296, WO 2022237815, WO 2022232332, WO 2022232331, WO 2022232320, WO 2022232318, WO 2022223037, WO 2022221739, WO 2022221528, WO 2022221386, WO 2022216762, WO 2022192794, WO 2022192790, WO 2022188729, WO 2022187411, WO 2022184178, WO 2022173870, WO 2022173678, WO 2022135346, WO 2022133731, WO 2022133038, WO 2022133345, WO 2022132200, WO 2022119748, WO 2022109485, WO 2022109487, WO 2022066805, WO 2022002102, WO 2022002018, WO 2021259331, WO 2021257828, WO 2021252339, WO 2021248095, WO 2021248090, WO 2021248083, WO 2021248082, WO 2021248079, WO 2021248055, WO 2021245051, WO 2021244603, WO 2021239058, WO 2021231526, WO 2021228161, WO 2021219090, WO 2021219090, WO 2021219072, WO 2021218939, WO 2021217019, WO 2021216770, WO 2021215545, WO 2021215544, WO 2021211864, WO 2021190467, WO 2021185233, WO 2021180181, WO 2021175199, 2021173923, WO 2021169990, WO 2021169963, WO 2021168193, WO 2021158071, WO 2021155716, WO 2021152149, WO 2021150613, WO 2021147967, WO 2021147965, WO 2021143693, WO 2021142252, WO 2021141628, WO 2021139748, WO 2021139678, WO 2021129824, WO 2021129820, WO 2021127404, WO 2021126816, WO 2021126799, WO 2021124222, WO 2021121371, WO 2021121367, WO 2021121330, WO 2020050890, WO 2020047192, WO 2020035031, WO 2020028706, WO 2019241157, WO 2019232419, WO 2019217691, WO 2019217307, WO 2019215203, WO 2019213526, WO 2019213516, WO 2019155399, WO 2019150305, WO 2019110751, WO 2019099524, WO 2019051291, WO 2018218070, WO 2018217651, WO 2018218071, WO 2018218069, WO 2018206539, WO 2018143315, WO 2018140600, WO 2018140599, WO 2018140598, WO 2018140514, WO 2018140513, WO 2018140512, WO 2018119183, WO 2018112420, WO 2018068017, WO 2018064510, WO 2017201161, WO 2017172979, WO 2017100546, WO 2017087528, WO 2017058807, WO 2017058805, WO 2017058728, WO 2017058902, WO 2017058792, WO 2017058768, WO 2017058915, WO 2017015562, WO 2016168540, WO 2016164675, WO 2016049568, WO 2016049524, WO 2015054572, WO 2014152588, WO 2014143659 and WO 2013155223, CN 118005656, CN 117986263, CN 117982509, CN 117946135, CN 117924327, CN 117800976, CN 117683051, CN 117645627, CN 117624194, CN 117624190, CN 117586280, CN 117486901, CN 117466917, CN 117462688, CN 117362315, CN 117327102, CN 117327094, CN 117327074, CN 117285590, CN 117263959, CN 117247382, CN 117186095, CN 117164605, CN 116969977, CN 116925075, CN 116891489, CN 116731045, CN 116731044, CN 116554208, CN 116514846, CN 116478184, CN 116478141, CN 116410145, CN 116375742, CN 116354988, CN 116332948, CN 116332938, CN 116327956, CN 116262759, CN 116217592, CN 116199703, CN 116162099, CN 116143806, CN 116143805, CN 116120315, CN 116102559, CN 115960105, CN 115894520, CN 115872979, CN 115850267, CN 115785199, CN 115785124, CN 115724842, CN 115721720, CN 115716840, CN 115703775, CN 115611923, CN 115611898, CN 115583937, CN 115572278, CN 115557949, CN 115521312, CN 115504976, CN 115490709, CN 115466272, CN 115433183, CN 115433179, CN 115403575, CN 115385938, CN 115385937, CN 115385912, CN 115381786, CN 115368383, CN 115368382, CN 115368381, CN 115353506, CN 115322158, CN 115304623, CN 115304602, CN 115197245, CN 115181106, CN 114989195, CN 114989166, CN 114989147, CN 114920741, CN 114920739, CN 114907387, CN 114874234, CN 114874201, CN 114716436, CN 114716435, CN 114685532, CN 114685460, CN 114591319, CN 114539293, CN 114539286, CN 114539246, CN 114437107, CN 114437084, CN 114409653, CN 114380827, CN 114195804, CN 114195788, CN 114057776, CN 114057744, CN 114057743, CN 113999226, CN 113980032, CN 113980014, CN 113929676, CN 113754653, CN 113683616, CN 113563323, CN 113527299, CN 113527294, CN 113527293, CN 113493440, CN 113429405, CN 113248521, CN 113087700, CN 113024544, CN 113004269, CN 112920183, CN 112778284, CN 112390818, CN 112390788, CN 112300196, CN 112300194, CN 112300173, CN 112225734, CN 112142735, CN 112110918, CN 112094269, CN 112047937, and CN 109574871, including the RAS compound structures disclosed therein which are specifically incorporated herein by reference.

In any embodiment employing a RAS(OFF) inhibitor herein, a RAS(OFF) degrader targeting the OFF state of RAS may alternatively be employed. These degraders are known in the art. RAS degraders may be found, for example, in one or more of the following applications: WO 2024/083258, WO 2024083256, WO 2024055112, WO 2024054625, WO 2024050742, WO 2024044334, WO 2024040080, WO 2024034657, WO 2024034593, WO 2024034591, WO 2024034123, WO 2024029613, WO 2024020159, WO 2024019103, WO 2024017392, WO 2023185864, WO 2023171781, WO 2023141570, WO 2023138524, WO 2023130012, WO 2023116934, WO 2023099620, WO 2023081476, WO 2023077441, and CN 115785199, each of which is incorporated herein by reference in its entirety.

In some embodiments, the RAS(OFF) inhibitor is a peptide-based inhibitor. Peptide-based RAS(OFF) inhibitors have been developed that target specific regions of the RAS protein, such as the Switch II region or the RAS-effector interface. Non-limiting examples include the K-Ras-binding peptide (Krpep-2d), the Ras inhibitory peptide (RasIn) and LUNA18 (NCT05012618). Peptide-based RAS(OFF) inhibitors are a class of compounds that target the RAS protein by disrupting its interaction with its downstream effectors or other signaling proteins. These inhibitors are typically designed to mimic the binding motifs of RAS-interacting proteins or other RAS effectors, such as RAF or PI3K. By binding to RAS at the same site as these effectors, peptide-based inhibitors can effectively compete with these proteins and prevent the activation of downstream signaling pathways.

Peptide-based RAS(OFF) inhibitors can be further classified into two main categories: those that target the RAS-effector interface, and those that target other regions of the RAS protein. Peptide-based inhibitors that target the RAS-effector interface are designed to bind to the switch regions of RAS that are critical for its interaction with downstream effectors, such as RAF or PI3K. These inhibitors typically contain amino acid residues that are similar to those found in the binding motifs of RAS-interacting proteins or effectors and are often designed to form hydrogen bonds or other interactions with key residues on the surface of RAS.

Peptide-based RAS(OFF) inhibitors that target other regions of the RAS protein are typically designed to disrupt other interactions that are critical for the activation or signaling of RAS. For example, some peptide-based inhibitors are designed to bind to the hypervariable region of RAS, which is thought to play a role in membrane localization and anchoring of the protein. By binding to this region, peptide-based inhibitors can prevent the proper localization of RAS to the plasma membrane, which is necessary for its activation and signaling.

Several common motifs have been identified as important for the binding of RAS-interacting proteins and effectors and are often used in the design of peptide-based inhibitors. One example is the RAF-binding domain (RBD), which is found in many RAS-interacting proteins and is important for the interaction of RAS with downstream effectors such as RAF. The RBD contains a conserved amino acid sequence (Arg-Xaa-Arg) that is critical for binding to RAS, and this motif has been incorporated into several peptide-based inhibitors designed to disrupt the RAS-RAF interaction. Another example is the RAS-binding domain (RBD) of PI3K, which is important for the interaction of RAS with this downstream effector. The RBD of PI3K contains several conserved amino acid residues (such as Arg-Arg-Trp) that are critical for binding to RAS, and these motifs have been used in the design of peptide-based inhibitors that target the RAS-PI3K interaction. Other common motifs used in peptide-based RAS(OFF) inhibitors include the Ras-binding domain (RBD) of other RAS-interacting proteins such as RalGDS and SOS, as well as sequences that mimic the structure of the switch regions of RAS itself. These motifs are typically used to optimize the binding affinity and selectivity of the inhibitor for the desired target protein or interaction.

In some embodiments, the RAS(OFF) inhibitor is an antibody or antigenic binding peptide specific for RAS(OFF). Antibodies have been developed that bind to specific regions of the RAS protein, such as the Switch II region or the RAS-effector interface. For example, some antibodies have been developed that target the switch regions of RAS proteins, which are critical for the activation of these proteins and their interaction with downstream effectors. Binding of these antibodies to the switch regions can prevent the conformational changes required for RAS activation and downstream signaling. Another approach involves the use of antibodies that target RAS-interacting proteins or downstream effectors, such as RAF or PI3K. Binding of these antibodies to their target proteins can disrupt the RAS-dependent signaling pathways and inhibit the growth and survival of cancer cells. Additionally, some antibodies have been developed that can induce the internalization and degradation of RAS proteins, leading to their depletion and inhibition of downstream signaling. For example, some antibodies have been developed that recognize the unique structure of mutant RAS proteins and target them for degradation via the ubiquitin-proteasome pathway. Non-limiting examples of KRAS(OFF)-specific inhibitory antibodies include anti-p21ser, and K27 (DARPin) (see, e.g., Khan et al, Biochim Biophys Acta Mol Cell Res. 2020 Feb;1867(2):118570).

In some embodiments, a therapeutic agent that may be combined with a compound of the present invention is an inhibitor of the MAP kinase (MAPK) pathway (or "MAPK inhibitor"). Such agents are known in the art. MAPK inhibitors include, but are not limited to, one or more MAPK inhibitor described in Cancers (Basel) 2015 Sep; 7(3): 1758-1784. For example, the MAPK inhibitor may be selected from one or more of trametinib, binimetinib, selumetinib, cobimetinib, LErafAON (NeoPharm), ISIS 5132; vemurafenib, pimasertib, TAK733, RO4987655 (CH4987655); CI-1040; PD-0325901; CH5126766; MAP855; AZD6244; refametinib (RDEA 119/BAY 86-9766); GDC-0973/XL581; AZD8330 (ARRY-424704/ARRY-704); RO5126766 (Roche, described in PloS One. 2014 Nov 25;9(11)); and GSK1120212 (or JTP-74057, described in Clin Cancer Res. 2011 Mar 1;17(5):989-1000). The MAPK inhibitor may be PLX8394, LXH254, GDC-5573, or LY3009120. A MAPK pathway inhibitor may be a PI3Kα:RAS breaker, such as BBO-10203.

In some embodiments, an anti-cancer agent is a disrupter or inhibitor of the RAS-RAF-ERK or PI3K-AKT-TOR or PI3K-AKT signaling pathways. Such agents are known in the art. The PI3K/AKT inhibitor may include, but is not limited to, one or more PI3K/AKT inhibitor described in Cancers (Basel) 2015 Sep; 7(3): 1758-1784. For example, the PI3K/AKT inhibitor may be selected from one or more of NVP-BEZ235; BGT226; XL765/SAR245409; SF1126; GDC-0980; PI-103; PF-04691502; PKI-587; GSK2126458.

In some embodiments, an anti-cancer agent is a PD-1 or PD-L1 antagonist. Such agents are known in the art.

In some embodiments, additional therapeutic agents include ALK inhibitors, HER2 inhibitors, EGFR inhibitors, IGF-1R inhibitors, MEK inhibitors, PI3K inhibitors, AKT inhibitors, TOR inhibitors, MCL-1 inhibitors, BCL-2 inhibitors, SHP2 inhibitors, proteasome inhibitors, and immune therapies. In some embodiments, additional therapeutic agents include FGFR inhibitors, PARP inhibitors, BET inhibitors, PRMT5i inhibitors, MAT2A inhibitors, VEGF inhibitors, and HDAC inhibitors. In some embodiments, a therapeutic agent may be a pan-RTK inhibitor, such as afatinib.

IGF-1R inhibitors are known in the art and include linsitinib, or a pharmaceutically acceptable salt thereof.

EGFR inhibitors are known in the art and include, but are not limited to, small molecule antagonists, antibody inhibitors, or specific antisense nucleotide or siRNA. Useful antibody inhibitors of EGFR include cetuximab (Erbitux^{®}), panitumumab (Vectibix^{®}), zalutumumab, nimotuzumab, and matuzumab. Further antibody-based EGFR inhibitors include any anti-EGFR antibody or antibody fragment that can partially or completely block EGFR activation by its natural ligand. Non-limiting examples of antibody-based EGFR inhibitors include those described in Modjtahedi et al., Br. J. Cancer 1993, 67:247-253; Teramoto et al., Cancer 1996, 77:639-645; Goldstein et al., Clin. Cancer Res. 1995, 1:1311-1318; Huang et al., 1999, Cancer Res. 15:59(8):1935-40; and Yang et al., Cancer Res.1999, 59:1236-1243. The EGFR inhibitor can be monoclonal antibody Mab E7.6.3 (Yang, 1999 supra), or Mab C225 (ATCC Accession No. HB-8508), or an antibody or antibody fragment having the binding specificity thereof.

Small molecule antagonists of EGFR include gefitinib (Iressa^{®}), erlotinib (Tarceva^{®}), and lapatinib (TykerB^{®}). See, e.g., Yan et al., Pharmacogenetics and Pharmacogenomics in Oncology Therapeutic Antibody Development, BioTechniques 2005, 39(4):565-8; and Paez et al., EGFR Mutations in Lung Cancer Correlation with Clinical Response to Gefitinib Therapy, Science 2004, 304(5676):1497-500. In some embodiments, the EGFR inhibitor is osimertinib (Tagrisso^{®}). Further non-limiting examples of small molecule EGFR inhibitors include any of the EGFR inhibitors described in the following patent publications, and all pharmaceutically acceptable salts of such EGFR inhibitors: EP 0520722; EP 0566226; WO96/33980; U.S. Pat. No. 5,747,498; WO96/30347; EP 0787772; WO97/30034; WO97/30044; WO97/38994; WO97/49688; EP 837063; WO98/02434; WO97/38983; WO95/19774; WO95/19970; WO97/13771; WO98/02437; WO98/02438; WO97/32881; DE 19629652; WO98/33798; WO97/32880; WO97/32880; EP 682027; WO97/02266; WO97/27199; WO98/07726; WO97/34895; WO96/31510; WO98/14449; WO98/14450; WO98/14451; WO95/09847; WO97/19065; WO98/17662; U.S. Pat. No. 5,789,427; U.S. Pat. No. 5,650,415; U.S. Pat. No. 5,656,643; WO99/35146; WO99/35132; WO99/07701; and WO92/20642. Additional non-limiting examples of small molecule EGFR inhibitors include any of the EGFR inhibitors described in Traxler et al., Exp. Opin. Ther. Patents 1998, 8(12):1599-1625. In some embodiments, an EGFR inhibitor is an ERBB inhibitor. In humans, the ERBB family contains HER1 (EGFR, ERBB1), HER2 (NEU, ERBB2), HER3 (ERBB3), and HER (ERBB4). In some embodiments, the EGFR inhibitor may be bosutinib, crizotinib, dasatinib, erlotinib, gefitinib, lapatinib, pazopanib, ruxolitinib, sunitinib, vemurafenib, abrocitinib, asciminib, futibatinib, ibrutinib, imatinib, pacritinib, or sorafenib.

MEK inhibitors are known in the art and include, but are not limited to, pimasertib, selumetinib, cobimetinib (Cotellic^{®}), trametinib (Mekinist^{®}), and binimetinib (Mektovi^{®}). In some embodiments, a MEK inhibitor targets a MEK mutation that is a Class I MEK1 mutation selected from D67N; P124L; P124S; and L177V. In some embodiments, the MEK mutation is a Class II MEK1 mutation selected from ΔE51-Q58; ΔF53-Q58; E203K; L177M; C121S; F53L; K57E; Q56P; and K57N.

PI3K inhibitors are known in the art and include, but are not limited to, wortmannin; 17-hydroxywortmannin analogs described in WO06/044453; 4-[2-(1H-Indazol-4-yl)-6-[[4-(methylsulfonyl)piperazin-1-yl]methyl]thieno[3,2-d]pyrimidin-4-yl]morpholine (also known as pictilisib or GDC-0941 and described in WO09/036082 and WO09/055730); 2-methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in WO06/122806); (S)-I-(4-((2-(2-aminopyrimidin-5-yl)-7-methyl-4-morpholinothieno[3,2-d]pyrimidin-6-yl)methyl)piperazin-1-yl)-2-hydroxypropan-1-one (described in WO08/070740); LY294002 (2-(4-morpholinyl)-8-phenyl-4H-1-benzopyran-4-one (available from Axon Medchem); PI 103 hydrochloride (3-[4-(4-morpholinylpyrido-[3',2':4,5]furo[3,2-d]pyrimidin-2-yl] phenol hydrochloride (available from Axon Medchem); PIK 75 (2-methyl-5-nitro-2-[(6-bromoimidazo[1,2-a]pyridin-3-yl)methylene]-1-methylhydrazide-benzenesulfonic acid, monohydrochloride) (available from Axon Medchem); PIK 90 (N-(7,8-dimethoxy-2,3-dihydro-imidazo[I,2-c]quinazolin-5-yl)-nicotinamide (available from Axon Medchem); AS-252424 (5-[I-[5-(4-fluoro-2-hydroxy-phenyl)-furan-2-yl]-meth-(Z)-ylidene]-thiazolidine-2,4-dione (available from Axon Medchem); TGX-221 (7-methyl-2-(4-morpholinyl)-9-[1-(phenylamino)ethyl]-4H-pyrido-[1,2-a]pyrirnidin-4-one (available from Axon Medchem); XL-765; and XL-147. Other PI3K inhibitors include demethoxyviridin, perifosine, CAL101, PX-866, BEZ235, SF1126, INK1117, IPI-145, BKM120, XL147, XL765, Palomid 529, GSK1059615, ZSTK474, PWT33597, IC87114, TGI 00-115, CAL263, PI-103, GNE-477, CUDC-907, and AEZS-136.

AKT inhibitors are known in the art and include, but are not limited to, Akt-1-1 (inhibits Aktl) (Barnett et al., Biochem. J. 2005, 385(Pt. 2): 399-408); Akt-1-1,2 (inhibits Akl and 2) (Barnett et al., Biochem. J. 2005, 385(Pt. 2): 399-408); API-59CJ-Ome (e.g., Jin et al., Br. J. Cancer 2004, 91:1808-12); 1-H-imidazo[4,5-c]pyridinyl compounds (e.g., WO 05/011700); indole-3-carbinol and derivatives thereof (e.g., U.S. Pat. No. 6,656,963; Sarkar and Li J Nutr. 2004, 134(12 Suppl):3493S-3498S); perifosine (e.g., interferes with Akt membrane localization; Dasmahapatra et al. Clin. Cancer Res. 2004, 10(15):5242-52); phosphatidylinositol ether lipid analogues (e.g., Gills and Dennis Expert. Opin. Investig. Drugs 2004, 13:787-97); and triciribine (TCN or API-2 or NCI identifier: NSC 154020; Yang et al., Cancer Res. 2004, 64:4394-9).

mTOR inhibitors are known in the art and include, but are not limited to, ATP-competitive mTORC1/mTORC2 inhibitors, e.g., PI-103, PP242, PP30; Torin 1; FKBP12 enhancers; 4H-1-benzopyran-4-one derivatives; and rapamycin (also known as sirolimus) and derivatives thereof, including: temsirolimus (Torisel^{®}); everolimus (Afinitor^{®}; WO94/09010); ridaforolimus (also known as deforolimus or AP23573); rapalogs, e.g., as disclosed in WO98/02441 and WO01/14387, e.g. AP23464 and AP23841; 40-(2-hydroxyethyl)rapamycin; 40-[3-hydroxy(hydroxymethyl)methylpropanoate]-rapamycin (also known as CC1779); 40-epi-(tetrazolyt)-rapamycin (also called ABT578); 32-deoxorapamycin; 16-pentynyloxy-32(S)-dihydrorapanycin; derivatives disclosed in WO05/005434; derivatives disclosed in U.S. Patent Nos. 5,258,389, 5,118,677, 5,118,678, 5,100,883, 5,151,413, 5,120,842, and 5,256,790, and in WO94/090101, WO92/05179, WO93/111130, WO94/02136, WO94/02485, WO95/14023, WO94/02136, WO95/16691, WO96/41807, WO96/41807, and WO2018204416; and phosphorus-containing rapamycin derivatives (e.g., WO05/016252). In some embodiments, the mTOR inhibitor is a bisteric inhibitor (see, e.g., WO2018204416, WO2019212990 and WO2019212991), such as RMC-5552, having the structure

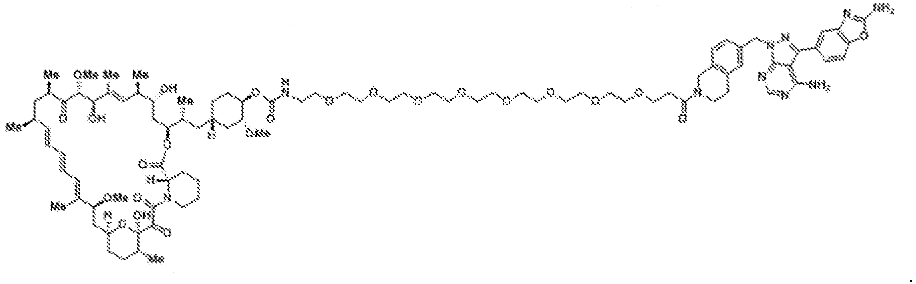

BRAF inhibitors that may be used in combination with compounds of the invention are known in the art and include, for example, vemurafenib, dabrafenib, and encorafenib. A BRAF may comprise a Class 3 BRAF mutation. In some embodiments, the Class 3 BRAF mutation is selected from one or more of the following amino acid substitutions in human BRAF: D287H; P367R; V459L; G466V;

G466E; G466A; S467L; G469E; N581S; N581I; D594N; D594G; D594A; D594H; F595L; G596D; G596R and A762E.

MCL-1 inhibitors are known in the art and include, but are not limited to, AMG-176, MIK665, and S63845. The myeloid cell leukemia-1 (MCL-1) protein is one of the key anti-apoptotic members of the B-cell lymphoma-2 (BCL-2) protein family. Over-expression of MCL-1 has been closely related to tumor progression as well as to resistance, not only to traditional chemotherapies but also to targeted therapeutics including BCL-2 inhibitors such as ABT-263.

In some embodiments, the additional therapeutic agent is a SHP2 inhibitor. SHP2 inhibitors are known in the art. SHP2 is a non-receptor protein tyrosine phosphatase encoded by the PTPN11 gene that contributes to multiple cellular functions including proliferation, differentiation, cell cycle maintenance and migration. SHP2 has two N-terminal Src homology 2 domains (N-SH2 and C-SH2), a catalytic domain (PTP), and a C-terminal tail. The two SH2 domains control the subcellular localization and functional regulation of SHP2. The molecule exists in an inactive, self-inhibited conformation stabilized by a binding network involving residues from both the N-SH2 and PTP domains. Stimulation by, for example, cytokines or growth factors acting through receptor tyrosine kinases (RTKs) leads to exposure of the catalytic site resulting in enzymatic activation of SHP2.

SHP2 is involved in signaling through the RAS-mitogen-activated protein kinase (MAPK), the JAK-STAT or the phosphoinositol 3-kinase-AKT pathways. Mutations in the PTPN11 gene and subsequently in SHP2 have been identified in several human developmental diseases, such as Noonan Syndrome and Leopard Syndrome, as well as human cancers, such as juvenile myelomonocytic leukemia, neuroblastoma, melanoma, acute myeloid leukemia and cancers of the breast, lung, and colon. Some of these mutations destabilize the auto-inhibited conformation of SHP2 and promote autoactivation or enhanced growth factor driven activation of SHP2. SHP2, therefore, represents a highly attractive target for the development of novel therapies for the treatment of various diseases including cancer. A SHP2 inhibitor (e.g., RMC-4550 or SHP099) in combination with a RAS pathway inhibitor (e.g., a MEK inhibitor) have been shown to inhibit the proliferation of multiple cancer cell lines in vitro (e.g., pancreas, lung, ovarian and breast cancer). Thus, combination therapy involving a SHP2 inhibitor with a RAS pathway inhibitor could be a general strategy for preventing tumor resistance in a wide range of malignancies.

Non-limiting examples of such SHP2 inhibitors that are known in the art, include: Chen et al. Mol Pharmacol. 2006, 70, 562; Sarver et al., J. Med. Chem. 2017, 62, 1793; Xie et al., J. Med. Chem. 2017, 60, 113734; and Igbe et al., Oncotarget, 2017, 8, 113734; and PCT applications: WO 2023282702, WO 2023280283, WO 2023280237, WO 2023018155, WO 2023011513, WO 2022271966, WO 2022271964, WO 2022271911, WO 2022259157, WO 2022242767, WO 2022241975, WO 2022237676, WO 2022237367, WO 2022237178, WO 2022235822, WO 20222084008, WO 2022135568, WO 2021176072, WO 2021171261, WO 2021149817, WO 2021148010, WO 2021147879, WO 2021143823, WO 2021143701, WO 2021143680, WO 2021121397, WO 2021119525, WO 2021115286, WO 2021110796, WO 2021088945, WO 2021073439, WO 2021061706, WO 2021061515, WO 2021043077, WO 2021033153, WO 2021028362, WO 2021033153, WO 2021028362, WO 2021018287, WO 2020259679, WO 2020249079, WO 2020210384, WO 2020201991, WO 2020181283, WO 2020177653, WO 2020165734, WO 2020165733, WO 2020165732, WO 2020156243, WO 2020156242, WO 2020108590, WO 2020104635, WO 2020094104, WO 2020094018, WO 2020081848, WO 2020073949, WO 2020073945, WO 2020072656, WO 2020065453, WO 2020065452, WO 2020063760, WO 2020061103, WO 2020061101, WO 2020033828, WO 2020033286, WO 2020022323, WO 2019233810, WO 2019213318, WO 2019183367, WO 2019183364, WO 2019182960, WO 2019167000, WO 2019165073, WO 2019158019, WO 2019152454, WO 2019051469, WO 2019051084, WO 2018218133, WO 2018172984, WO 2018160731, WO 2018136265, WO 2018136264, WO 2018130928, WO 2018129402, WO 2018081091, WO 2018057884, WO 2018013597, WO 2017216706, WO 2017211303, WO 2017210134, WO 2017156397, WO 2017100279, WO 2017079723, WO 2017078499, WO 2016203406, WO 2016203405, WO 2016203404, WO 2016196591, WO 2016191328, WO 2015107495, WO 2015107494, WO 2015107493, WO 2014176488, WO 2014113584, CN 115677661, CN 115677660, CN 115611869, CN 115521305, CN 115490697, CN 115466273, CN 115394612, CN 115304613, CN 115304612, CN 115300513, CN 115197225, CN 114957162, CN 114920759, CN 114716448, CN 114671879, CN 114539223, CN 114524772, CN 114213417, CN 114195799, CN 114163457, CN 113896710, CN 113248521, CN 113248449, CN 113135924, CN 113024508, CN 112920131, CN 112823796, CN 112409334, CN 112402385, CN 112174935, 111848599, CN 111704611, CN 111393459, CN 111265529, CN 110143949, CN 108113848, US 11179397, US 11044675, US 11034705, US 11033547, US 11001561, US 10988466, US 10954243, US 10934302, or US 10858359, or a pharmaceutically acceptable salt, solvate, isomer (e.g., stereoisomer), prodrug, or tautomer thereof, each of which is incorporated herein by reference.

In some embodiments, a SHP2 inhibitor binds in the active site. In some embodiments, a SHP2 inhibitor is a mixed-type irreversible inhibitor. In some embodiments, a SHP2 inhibitor binds an allosteric site e.g., a non-covalent allosteric inhibitor. In some embodiments, a SHP2 inhibitor is a covalent SHP2 inhibitor, such as an inhibitor that targets the cysteine residue (C333) that lies outside the phosphatase's active site. In some embodiments a SHP2 inhibitor is a reversible inhibitor. In some embodiments, a SHP2 inhibitor is an irreversible inhibitor.

In some embodiments, the additional therapeutic agent is selected from the group consisting of a MEK inhibitor, a HER2 inhibitor, a SHP2 inhibitor, a CDK4/6 inhibitor, an mTOR inhibitor, a SOS1 inhibitor, and a PD-L1 inhibitor. In some embodiments, the additional therapeutic agent is selected from the group consisting of a MEK inhibitor, a SHP2 inhibitor, and a PD-L1 inhibitor. See, e.g., Hallin et al., Cancer Discovery, DOI: 10.1158/2159-8290 (October 28, 2019) and Canon et al., Nature, 575:217 (2019). In some embodiments, a Ras inhibitor of the present invention is used in combination with a MEK inhibitor and a SOS1 inhibitor. In some embodiments, a Ras inhibitor of the present invention is used in combination with a PD-L1 inhibitor and a SOS1 inhibitor. In some embodiments, a Ras inhibitor of the present invention is used in combination with a PD-L1 inhibitor and a SHP2 inhibitor. In some embodiments, a Ras inhibitor of the present invention is used in combination with a MEK inhibitor and a SHP2 inhibitor. In some embodiments, a Ras inhibitor of the present invention is used in combination with a SHP2 inhibitor and a Ras inhibitor that inhibits multiple Ras isoforms and/or mutants (e.g., RMC-6236). In some embodiments, the cancer is lung cancer, and the treatment comprises administration of a Ras inhibitor of the present invention in combination with a second or third therapeutic agent, such as a SHP2 inhibitor and a Ras inhibitor that inhibits multiple Ras isoforms and/or mutants. In some embodiments, the cancer is colorectal cancer, and the treatment comprises administration of a Ras inhibitor of the present invention in combination with a second or third therapeutic agent, such as a SHP2 inhibitor and a Ras inhibitor that inhibits multiple Ras isoforms and/or mutants. In some embodiments, a Ras inhibitor of the present invention is used in combination with an immunotherapy, optionally in combination with a chemotherapeutic agent.

Proteasome inhibitors are known in the art and include, but are not limited to, carfilzomib (Kyprolis^{®}), bortezomib (Velcade^{®}), and oprozomib.

Immune therapies include, but are not limited to, monoclonal antibodies, immunomodulatory imides (ImiDs), GITR agonists, genetically engineered T-cells (e.g., CAR-T cells), bispecific antibodies (e.g., BiTEs), and anti-PD-1, anti-PD-L1, anti-CTLA4, anti-LAGI, and anti-OX40 agents). Other immune therapies are known in the art.

Immunomodulatory agents (ImiDs) are a class of immunomodulatory drugs (drugs that adjust immune responses) containing an imide group. The ImiD class includes thalidomide and its analogues (lenalidomide, pomalidomide, and apremilast).

Exemplary anti-PD-1 antibodies and methods for their use are described by Goldberg et al., Blood 2007, 110(1):186-192; Thompson et al., Clin. Cancer Res. 2007, 13(6):1757-1761; and WO06/121168 A1), as well as described elsewhere herein.

FGFR inhibitors are known in the art, such as pemigatinib and erdafitinib, including FGFR2 inhibitors and FGFR4 inhibitors. See, e.g., Cancers (Basel), 2021 Jun; 13(12) 2968.

BET inhibitors are known in the art, such as romidepsin, panobinostat and belinostat. See, e.g., British J. Cancer 124:1478 (2021).

PRMT5i inhibitors are known in the art, such as PF-0693999, PJ-68 and MRTX1719. See, e.g., Biomed. Pharmacotherapy 144:112252 (2021).

MAT2A inhibitors are known in the art, such as AG-270 and IDE397. See, e.g., Exp Opin Ther Patents (2022) DOl: 10.1080/13543776.2022.2119127.

GITR agonists include, but are not limited to, GITR fusion proteins and anti-GITR antibodies (e.g., bivalent anti-GITR antibodies), such as, a GITR fusion protein described in U.S. Pat. No. 6,111,090, , U.S. Pat. No. 8,586,023, WO2010/003118 and WO2011/090754; or an anti-GITR antibody described, e.g., in U.S. Pat. No. 7,025,962, EP 1947183, U.S. Pat. No. 7,812,135, U.S. Pat. No. 8,388,967, U.S. Pat. No. 8,591,886, U.S. Pat. No. 7,618,632, EP 1866339, and WO2011/028683, WO2013/039954, WO05/007190, WO07/133822, WO05/055808, WO99/40196, WO01/03720, WO99/20758, WO06/083289, WO05/115451, and WO2011/051726.

Another example of a therapeutic agent that may be used in combination with the compounds of the invention is an anti-angiogenic agent. Anti-angiogenic agents are known in the art and are inclusive of, but not limited to, in vitro synthetically prepared chemical compositions, antibodies, antigen binding regions, radionuclides, and combinations and conjugates thereof. An anti-angiogenic agent can be an agonist, antagonist, allosteric modulator, toxin or, more generally, may act to inhibit or stimulate its target (e.g., receptor or enzyme activation or inhibition), and thereby promote cell death or arrest cell growth. In some embodiments, the one or more additional therapies include an anti-angiogenic agent.

Anti-angiogenic agents can be MMP-2 (matrix-metalloproteinase 2) inhibitors, MMP-9 (matrix-metalloproteinase 9) inhibitors, and COX-II (cyclooxygenase 11) inhibitors. Non-limiting examples of anti-angiogenic agents include rapamycin, temsirolimus (CCI-779), everolimus (RAD001), sorafenib, sunitinib, and bevacizumab. Examples of useful COX-II inhibitors include alecoxib, valdecoxib, and rofecoxib. Examples of useful matrix metalloproteinase inhibitors are described in WO96/33172, WO96/27583, WO98/07697, WO98/03516, WO98/34918, WO98/34915, WO98/33768, WO98/30566, WO90/05719, WO99/52910, WO99/52889, WO99/29667, WO99007675, EP0606046, EP0780386, EP1786785, EP1181017, EP0818442, EP1004578, and US20090012085, and U.S. Patent Nos. 5,863,949 and 5,861,510. Preferred MMP-2 and MMP-9 inhibitors are those that have little or no activity inhibiting MMP-1. More preferred, are those that selectively inhibit MMP-2 or AMP-9 relative to the other matrix- metalloproteinases (i.e., MAP-1, MMP-3, MMP-4, MMP-5, MMP-6, MMP- 7, MMP- 8, MMP-10, MMP-11, MMP-12, and MMP-13). Some specific examples of MMP inhibitors are AG-3340, RO 32-3555, and RS 13-0830.

Further exemplary anti-angiogenic agents include KDR (kinase domain receptor) inhibitory agents (e.g., antibodies and antigen binding regions that specifically bind to the kinase domain receptor), anti-VEGF agents (e.g., antibodies or antigen binding regions that specifically bind VEGF (e.g., bevacizumab), or soluble VEGF receptors or a ligand binding region thereof) such as VEGF-TRAP^{™}, and anti-VEGF receptor agents (e.g., antibodies or antigen binding regions that specifically bind thereto), VEGF inhibitors, EGFR inhibitory agents (e.g., antibodies or antigen binding regions that specifically bind thereto) such as Vectibix^{®} (panitumumab), erlotinib (Tarceva^{®}), anti-Angl and anti-Ang2 agents (e.g., antibodies or antigen binding regions specifically binding thereto or to their receptors, e.g., Tie2/Tek), and anti-Tie2 kinase inhibitory agents (e.g., antibodies or antigen binding regions that specifically bind thereto). Other anti-angiogenic agents include Campath, IL-8, B-FGF, Tek antagonists (US2003/0162712; US6,413,932), anti-TWEAK agents (e.g., specifically binding antibodies or antigen binding regions, or soluble TWEAK receptor antagonists; see US6,727,225), ADAM distintegrin domain to antagonize the binding of integrin to its ligands (US 2002/0042368), specifically binding anti-eph receptor or anti-ephrin antibodies or antigen binding regions (U.S. Patent Nos. 5,981,245; 5,728,813; 5,969,110; 6,596,852; 6,232,447; 6,057,124 and patent family members thereof), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto). Additional anti-angiogenic agents include: SD-7784 (Pfizer, USA); cilengitide (Merck KgaA, Germany, EPO 0770622); pegaptanib octasodium, (Gilead Sciences, USA); Alphastatin, (BioActa, UK); M-PGA, (Celgene, USA, US 5712291); ilomastat, (Arriva, USA, US5892112); emaxanib, (Pfizer, USA, US 5792783); vatalanib, (Novartis, Switzerland); 2-methoxyestradiol (EntreMed, USA); TLC ELL-12 (Elan, Ireland); anecortave acetate (Alcon, USA); alpha-D148 Mab (Amgen, USA); CEP-7055 (Cephalon, USA); anti-Vn Mab (Crucell, Netherlands), DACantiangiogenic (ConjuChem, Canada); Angiocidin (InKine Pharmaceutical, USA); KM-2550 (Kyowa Hakko, Japan); SU-0879 (Pfizer, USA); CGP-79787 (Novartis, Switzerland, EP 0970070); ARGENT technology (Ariad, USA); YIGSR-Stealth (Johnson & Johnson, USA); fibrinogen-E fragment (BioActa, UK); angiogenic inhibitor (Trigen, UK); TBC-1635 (Encysive Pharmaceuticals, USA); SC-236 (Pfizer, USA); ABT-567 (Abbott, USA); Metastatin (EntreMed, USA); maspin (Sosei, Japan); 2-methoxyestradiol (Oncology Sciences Corporation, USA); ER-68203-00 (IV AX, USA); BeneFin (Lane Labs, USA); Tz-93 (Tsumura, Japan); TAN-1120 (Takeda, Japan); FR-111142 (Fujisawa, Japan, JP 02233610); platelet factor 4 (RepliGen, USA, EP 407122); vascular endothelial growth factor antagonist (Borean, Denmark); bevacizumab (pINN) (Genentech, USA); angiogenic inhibitors (SUGEN, USA); XL 784 (Exelixis, USA); XL 647 (Exelixis, USA); Mab, alpha5beta3 integrin, second generation (Applied Molecular Evolution, USA and MedImmune, USA); enzastaurin hydrochloride (Lilly, USA); CEP 7055 (Cephalon, USA and Sanofi-Synthelabo, France); BC 1 (Genoa Institute of Cancer Research, Italy); rBPI 21 and BPI-derived antiangiogenic (XOMA, USA); PI 88 (Progen, Australia); cilengitide (Merck KgaA, German; Munich Technical University, Germany, Scripps Clinic and Research Foundation, USA); AVE 8062 (Ajinomoto, Japan); AS 1404 (Cancer Research Laboratory, New Zealand); SG 292, (Telios, USA); Endostatin (Boston Childrens Hospital, USA); ATN 161 (Attenuon, USA); 2-methoxyestradiol (Boston Childrens Hospital, USA); ZD 6474, (AstraZeneca, UK); ZD 6126, (Angiogene Pharmaceuticals, UK); PPI 2458, (Praecis, USA); AZD 9935, (AstraZeneca, UK); AZD 2171, (AstraZeneca, UK); vatalanib (pINN), (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); vaccine, gene-based, VEGF-2, (Scripps Clinic and Research Foundation, USA); SPV5.2, (Supratek, Canada); SDX 103, (University of California at San Diego, USA); PX 478, (ProIX, USA); METASTATIN, (EntreMed, USA); troponin I, (Harvard University, USA); SU 6668, (SUGEN, USA); OXI 4503, (OXiGENE, USA); o-guanidines, (Dimensional Pharmaceuticals, USA); motuporamine C, (British Columbia University, Canada); CDP 791, (Celltech Group, UK); atiprimod (pINN), (GlaxoSmithKline, UK); E 7820, (Eisai, Japan); CYC 381, (Harvard University, USA); AE 941, (Aeterna, Canada); vaccine, angiogenic, (EntreMed, USA); urokinase plasminogen activator inhibitor, (Dendreon, USA); oglufanide (pINN), (Melmotte, USA); HIF-lalfa inhibitors, (Xenova, UK); CEP 5214, (Cephalon, USA); BAY RES 2622, (Bayer, Germany); Angiocidin, (InKine, USA); A6, (Angstrom, USA); KR 31372, (Korea Research Institute of Chemical Technology, South Korea); GW 2286, (GlaxoSmithKline, UK); EHT 0101, (ExonHit, France); CP 868596, (Pfizer, USA); CP 564959, (OSI, USA); CP 547632, (Pfizer, USA); 786034, (GlaxoSmithKline, UK); KRN 633, (Kirin Brewery, Japan); drug delivery system, intraocular, 2-methoxyestradiol; anginex (Maastricht University, Netherlands, and Minnesota University, USA); ABT 510 (Abbott, USA); AAL 993 (Novartis, Switzerland); VEGI (ProteomTech, USA); tumor necrosis factor-alpha inhibitors; SU 11248 (Pfizer, USA and SUGEN USA); ABT 518, (Abbott, USA); YH16 (Yantai Rongchang, China); S-3APG (Boston Childrens Hospital, USA and EntreMed, USA); Mab, KDR (ImClone Systems, USA); Mab, alpha5 beta (Protein Design, USA); KDR kinase inhibitor (Celltech Group, UK, and Johnson & Johnson, USA); GFB 116 (South Florida University, USA and Yale University, USA); CS 706 (Sankyo, Japan); combretastatin A4 prodrug (Arizona State University, USA); chondroitinase AC (IBEX, Canada); BAY RES 2690 (Bayer, Germany); AGM 1470 (Harvard University, USA, Takeda, Japan, and TAP, USA); AG 13925 (Agouron, USA); Tetrathiomolybdate (University of Michigan, USA); GCS 100 (Wayne State University, USA) CV 247 (Ivy Medical, UK); CKD 732 (Chong Kun Dang, South Korea); irsogladine, (Nippon Shinyaku, Japan); RG 13577 (Aventis, France); WX 360 (Wilex, Germany); squalamine, (Genaera, USA); RPI 4610 (Sirna, USA); heparanase inhibitors (InSight, Israel); KL 3106 (Kolon, South Korea); Honokiol (Emory University, USA); ZK CDK (Schering AG, Germany); ZK Angio (Schering AG, Germany); ZK 229561 (Novartis, Switzerland, and Schering AG, Germany); XMP 300 (XOMA, USA); VGA 1102 (Taisho, Japan); VE-cadherin-2 antagonists(ImClone Systems, USA); Vasostatin (National Institutes of Health, USA); Flk-1 (ImClone Systems, USA); TZ 93 (Tsumura, Japan); TumStatin (Beth Israel Hospital, USA); truncated soluble FLT 1 (vascular endothelial growth factor receptor 1) (Merck & Co, USA); Tie-2 ligands (Regeneron, USA); and thrombospondin 1 inhibitor (Allegheny Health, Education and Research Foundation, USA).

Further examples of therapeutic agents that may be used in combination with compounds of the invention include agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor, c-Met. Such agents are known in the art.

Another example of a therapeutic agent that may be used in combination with compounds of the invention is an autophagy inhibitor. Autophagy inhibitors are known in the art and include, but are not limited to chloroquine, 3- methyladenine, hydroxychloroquine (Plaquenil^{™}), bafilomycin A1, 5-amino-4-imidazole carboxamide riboside (AICAR), okadaic acid, autophagy-suppressive algal toxins which inhibit protein phosphatases of type 2A or type 1, analogues of cAMP, and drugs which elevate cAMP levels such as adenosine, LY204002, N6-mercaptopurine riboside, and vinblastine. In addition, antisense or siRNA that inhibits expression of proteins including but not limited to ATG5 (which are implicated in autophagy), may also be used. In some embodiments, the one or more additional therapies include an autophagy inhibitor.

Another example of a therapeutic agent that may be used in combination with compounds of the invention is an anti-neoplastic agent, which are known in the art. In some embodiments, the one or more additional therapies include an anti-neoplastic agent. Non-limiting examples of anti-neoplastic agents include acemannan, aclarubicin, aldesleukin, alemtuzumab, alitretinoin, altretamine, amifostine, aminolevulinic acid, amrubicin, amsacrine, anagrelide, anastrozole, ancer, ancestim, arglabin, arsenic trioxide, BAM-002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-NI, interferon alfa-n3, interferon alfacon-1, interferon alpha, natural, interferon beta, interferon beta-la, interferon beta-lb, interferon gamma, natural interferon gamma- la, interferon gamma-Ib, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburiembodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, virulizin, zinostatin stimalamer, or zoledronic acid; abarelix; AE 941 (Aeterna), ambamustine, antisense oligonucleotide, bcl-2 (Genta), APC 8015 (Dendreon), decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 Mab (Biomira), cancer Mab (Japan Pharmaceutical Development), HER-2 and Fc Mab (Medarex), idiotypic 105AD7 Mab (CRC Technology), idiotypic CEA Mab (Trilex), LYM-1-iodine 131 Mab (Techni clone), polymorphic epithelial mucin-yttrium 90 Mab (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

Additional examples of therapeutic agents that may be used in combination with compounds of the invention include ipilimumab (Yervoy^{®}); tremelimumab; galiximab; nivolumab, also known as BMS-936558 (Opdivo^{®}); pembrolizumab (Keytruda^{®}); avelumab (Bavencio^{®}); AMP224; BMS-936559; MPDL3280A, also known as RG7446; MEDI-570; AMG557; MGA271; IMP321; BMS-663513; PF-05082566; CDX-1127; anti-OX40 (Providence Health Services); huMAbOX40L; atacicept; CP-870893; lucatumumab; dacetuzumab; muromonab-CD3; ipilumumab; MEDI4736 (Imfinzi^{®}); MSB0010718C; AMP 224; adalimumab (Humira^{®}); ado-trastuzumab emtansine (Kadcyla^{®}); aflibercept (Eylea^{®}); alemtuzumab (Campath^{®}); basiliximab (Simulect^{®}); belimumab (Benlysta^{®}); basiliximab (Simulect^{®}); belimumab (Benlysta^{®}); brentuximab vedotin (Adcetris^{®}); canakinumab (Ilaris^{®}); certolizumab pegol (Cimzia^{®}); daclizumab (Zenapax^{®}); daratumumab (Darzalex^{®}); denosumab (Prolia^{®}); eculizumab (Soliris^{®}); efalizumab (Raptiva^{®}); gemtuzumab ozogamicin (Mylotarg^{®}); golimumab (Simponi^{®}); ibritumomab tiuxetan (Zevalin^{®}); infliximab (Remicade^{®}); motavizumab (Numax^{®}); natalizumab (Tysabri^{®}); obinutuzumab (Gazyva^{®}); ofatumumab (Arzerra^{®}); omalizumab (Xolair^{®}); palivizumab (Synagis^{®}); pertuzumab (Perjeta^{®}); pertuzumab (Perjeta^{®}); ranibizumab (Lucentis^{®}); raxibacumab (Abthrax^{®}); tocilizumab (Actemra^{®}); tositumomab; tositumomab-i-131; tositumomab and tositumomab-i-131 (Bexxar^{®}); ustekinumab (Stelara^{®}); AMG 102; AMG 386; AMG 479; AMG 655; AMG 706; AMG 745; and AMG 951.

The compounds described herein can be used in combination with the agents disclosed herein or other suitable agents, depending on the condition being treated. Hence, in some embodiments the one or more compounds of the disclosure will be co-administered with other therapies as described herein. When used in combination therapy, the compounds described herein may be administered with the second agent simultaneously or separately. This administration in combination can include simultaneous administration of the two agents in the same dosage form, simultaneous administration in separate dosage forms, and separate administration. That is, a compound described herein and any of the agents described herein can be formulated together in the same dosage form and administered simultaneously. Alternatively, a compound of the invention and any of the therapies described herein can be simultaneously administered, wherein both the agents are present in separate formulations. In another alternative, a compound of the present disclosure can be administered and followed by any of the therapies described herein, or vice versa. In some embodiments of the separate administration protocol, a compound of the invention and any of the therapies described herein are administered a few minutes apart, or a few hours apart, or a few days apart.

In some embodiments of any of the methods described herein, the first therapy (e.g., a compound of the invention) and one or more additional therapies are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours, up to 24 hours, or up to 1-7, 1-14, 1-21 or 1-30 days before or after the one or more additional therapies.

The invention also features kits including (a) a pharmaceutical composition including an agent (e.g., a compound of the invention) described herein, and (b) a package insert with instructions to perform any of the methods described herein. In some embodiments, the kit includes (a) a pharmaceutical composition including an agent (e.g., a compound of the invention) described herein, (b) one or more additional therapies (e.g., non-drug treatment or therapeutic agent), and (c) a package insert with instructions to perform any of the methods described herein.

As one aspect of the present invention contemplates the treatment of the disease or symptoms associated therewith with a combination of pharmaceutically active compounds that may be administered separately, the invention further relates to combining separate pharmaceutical compositions in kit form. The kit may comprise two separate pharmaceutical compositions: a compound of the present invention, and one or more additional therapies. The kit may comprise a container for containing the separate compositions such as a divided bottle or a divided foil packet. Additional examples of containers include syringes, boxes, and bags. In some embodiments, the kit may comprise directions for the use of the separate components. The kit form is particularly advantageous when the separate components are preferably administered in different dosage forms (e.g., oral and parenteral), are administered at different dosage intervals, or when titration of the individual components of the combination is desired by the prescribing health care professional.

### Enumerated embodiments.

EB1. The disclosure provides a compound, or a pharmaceutically acceptable salt thereof, having the structure of Formula I: 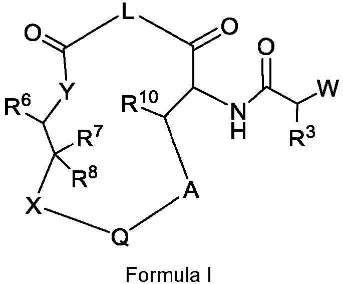 wherein:
   Q is an optionally substituted 7- to 12-membered bicyclic arylene, an optionally substituted 7-to 12-membered bicyclic heteroarylene, or an optionally substituted 7- to 12-membered bicyclic heterocyclylene, wherein a first ring in Q is bonded to X, and a second ring in Q is bonded to A;
   X is a bond; a straight chain C₁-C₃ alkylene optionally substituted with 1 to 3 substituents independently selected from fluoro, -CN, -C₁-C₃ alkyl, and -O-C₁-C₃ alkyl; -O-; -S(O)₀₋₂-; *-CH₂-O-; *-CH₂-S(O)₀₋₂-; *-O-CH₂-; or *-CH₂-S(O)₀₋₂-, wherein "*" represents a portion of X bound to -C(R⁷)(R⁸)-;
   Y is -O-, -NH- or -N(C₁-C₃ alkyl)-;
   R³ is optionally substituted C₁-C₆ alkyl, optionally substituted C₃-C₆ cycloalkyl, optionally substituted C₆-C₁₀ aryl, or optionally substituted 3- to 11-membered heterocyclyl;
      W is: 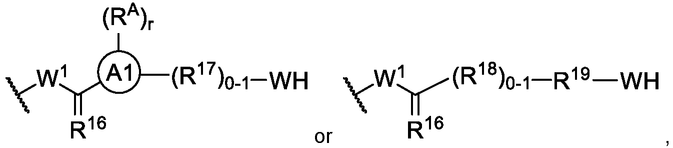
   wherein:
      ring A1 is a 4- to 8-membered cycloalkyl or a 4- to 8-membered heterocyclyl;
      W¹ is -N(R²⁰)-, -O-, or -C(R^{20a})(R^{20b})-;
         each R^{A} is each independently halo, cyano, hydroxyl, optionally substituted amino, optionally substituted C₁-C₆ heteroalkyl, or optionally substituted C₁-C₃ alkyl;
      r is 0, 1, 2, 3, or 4;
         R¹⁷, if present, is optionally substituted C₁-C₆ heteroalkylene or optionally substituted C₁-C₆ alkylene;
      R¹⁸, if present, is optionally substituted C₁-C₄ alkylene;
      R¹⁹ is optionally substituted C₁-C₆ heteroalkylene, optionally substituted C₁-C₆ alkylene, -NH-, or
         -N(optionally substituted C₁-C₆ alkyl) or a saturated, nitrogen-containing 3- to 8-membered heterocyclyl;
      R²⁰ is hydrogen or -C₁-C₃ alkyl;
      R²⁰ is taken together with one R^{A}, the atoms to which they are respectively attached and any intervening atoms, to form an optionally substituted, 5- to 8-membered heterocyclyl that is fused or spiro-fused to ring A, or
      R²⁰ is taken together with any methylene unit in R¹⁸, or any methylene unit in R¹⁹, the atoms to which they are respectively attached and any intervening atoms, to form an optionally substituted, 5- to 8-membered heterocyclyl;
      each of R^{20a} and R^{20b} is, independently, hydrogen, or -C₁-C₃ alkyl, or R^{20a} and R^{20b} are taken together with the carbon atom to which they are bound to form a 3- to 6-membered cycloalkyl ring;
      R¹⁶ is O, S, N-CN, or N-O-C₁-C₃ alkyl; 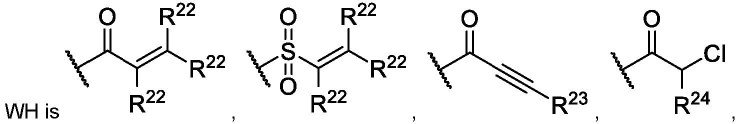 or 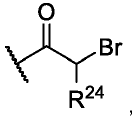
      each R²² is, independently, hydrogen, cyano, optionally substituted C₁-C₃ alkyl, or an optionally substituted 4- to 7-membered saturated heterocyclyl; or R²² is taken together with either of R¹⁷ or R¹⁹, the atoms to which they are attached and any intervening atoms to form an optionally substituted 5- to 8-membered ring system;
      R²³ is hydrogen, optionally substituted C₁-C₃ alkyl, or an optionally substituted 4- to 7-membered saturated heterocyclyl;
      R²⁴ is hydrogen, optionally substituted C₁-C₃ alkyl, or an optionally substituted 4- to 7-membered saturated heterocyclyl; or R²⁴ is taken together with either of R¹⁷ or R¹⁹, the atoms to which they are attached and any intervening atoms, to form an optionally substituted 5- to 8-membered ring system;
      A is optionally substituted C₂-C₄ alkylene, optionally substituted C₁-C₄ heteroalkylene, or optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
      L has the structure of Formula VIIa or Vllb: 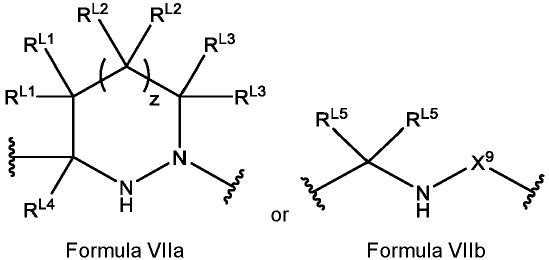
      z is 0, 1, or 2;
      X⁹ is -NR^{L6}-, -C(O)-, or -S(O)₂-;
      each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
      wherein L does not have the structure of 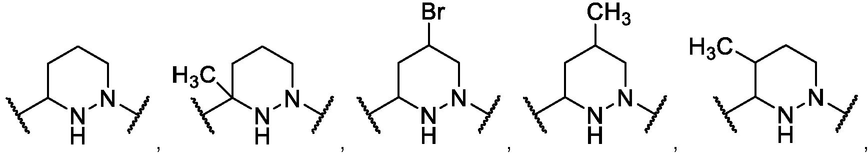 or 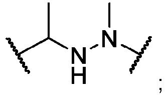
      R³ is optionally substituted C₁-C₆ alkyl, optionally substituted C₃-C₆ cycloalkyl, optionally substituted C₆ aryl, or optionally substituted 3- to 7-membered heterocyclyl;
      R¹⁰ is hydrogen, halogen, optionally substituted C₁-C₃ alkyl, or C₁-C₃ optionally substituted heteroalkyl;
      R⁷ is hydrogen, halogen, or optionally substituted C₁-C₃ alkyl;
      R⁸ is hydrogen, halogen, -OH, -CN, optionally substituted C₁-C₃ heteroalkyl, optionally substituted C₁-C₃ alkyl, optionally substituted C₂-C₆ alkenyl, optionally substituted C₂-C₆ alkynyl, optionally substituted C₆-C₁₀ aryl, optionally substituted 4- to 8-membered heteroaryl, optionally substituted C₃-C₆ cycloalkyl, or optionally substituted 3- to 7-membered heterocyclyl; or
      R⁷ and R⁸ are taken together to form =CH₂, an optionally substituted C₃-C₆ cycloalkyl, or a 3- to 7-membered saturated heterocyclyl; or
      R⁸ is taken together with a ring atom in Q, the carbon atom to which R⁷ is bound and X to form a 4- to 9-membered saturated or unsaturated heterocyclyl that is fused to Q;
      R⁶ is hydrogen or -CH₃;
      each R⁵ is, independently, halogen, optionally substituted C₁-C₃ alkyl, or optionally substituted C₁-C₃ haloalkyl; and
      p is 0, 1, 2, or 3, wherein:
         (i) the compound is not: 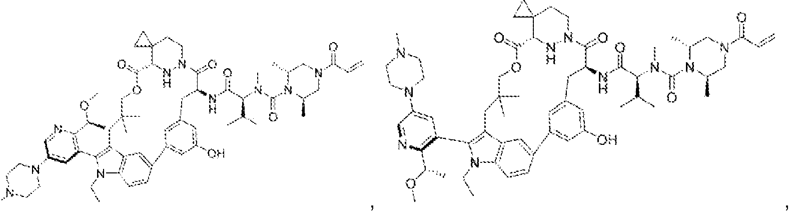 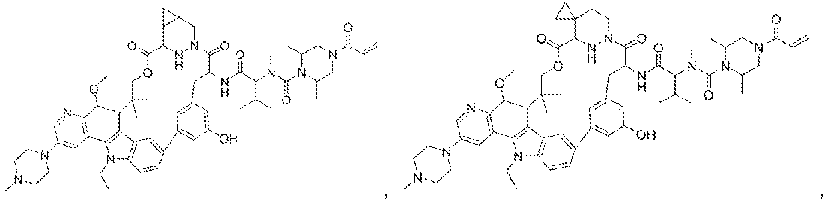 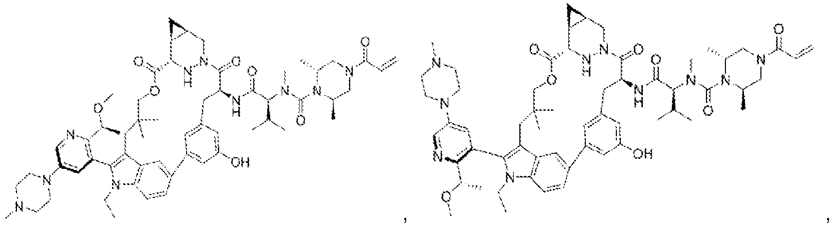 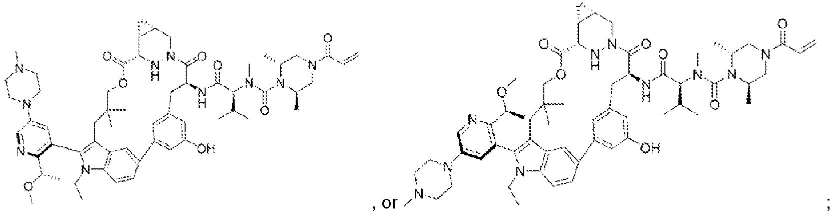 or
         (ii) when A is 1-hydroxy-benzene-3,5-diyl or optionally substituted phenylene, two R^{L1} substituents taken together form spiro cyclopropyl, and W is 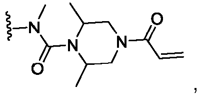 then, Q is not 1-ethyl-indole-2,5-diyl or indole-2,5-diyl substituted with C₁₋₄ alkyl; or
         (iii) when A is 1-hydroxy-benzene-3,5-diyl or optionally substituted phenylene, two R^{L1} substituents taken together form spiro cyclopropyl, and W is 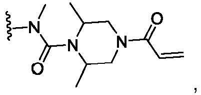 then, Q is not 1-ethyl-2,5-indol-diyl optionally substituted with 1, 2 or 3 substituents independently selected from halogen, C₁₋₄ alkyl and C₁₋₄ alkoxy, wherein the C₁₋₄ alkyl and C₁₋₄ alkoxy are each optionally substituted with 1, 2 or 3 substituents independently selected from halogen, OH, C₁₋₃ alkyl and C₁₋₃ alkoxy, wherein C₁₋₃ alkyl and C₁₋₃ alkoxy are each substituted with 1, 2 or 3 substituents independently selected from deuterium, halogen, OH, methyl, methoxy and OCF₃; or
         (iv) when A is 1-hydroxy-benzene-3,5-diyl or optionally substituted phenylene, R^{L1} and R^{L2} taken together form fused cyclopropyl, and W is 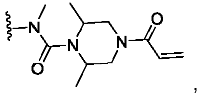 then Q is not 1-ethyl-2,5-indol-diyl or 2,5-indol-diyl substituted with C₁₋₄ alkyl; or
         (v) when A is 1-hydroxy-benzene-3,5-diyl or optionally substituted phenylene, R^{L1} and R^{L2} taken together form fused cyclopropyl, and W is 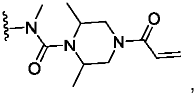 then Q is 1-ethyl-2,5-indol-diyl or 2,5-indol-diyl substituted with C₁₋₄ alkyl; or
         (vi) when A is 1-hydroxy-benzene-3,5-diyl or optionally substituted phenylene, R^{L1} and R^{L2} taken together form fused cyclopropyl, and W is 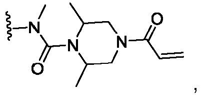 then Q is not 1-ethyl-2,5-indol-diyl optionally substituted with 1, 2 or 3 substituents independently selected from halogen, C₁₋₄ alkyl and C₁₋₄ alkoxy, wherein the C₁₋₄ alkyl and C₁₋₄ alkoxy are each optionally substituted with 1, 2 or 3 substituents independently selected from halogen, OH, C₁₋₃ alkyl and C₁₋₃ alkoxy, wherein C₁₋₃ alkyl and C₁₋₃ alkoxy are each substituted with 1, 2 or 3 substituents independently selected from deuterium, halogen, OH, methyl, methoxy and OCF₃; or
         (vii) when A is -hydroxy-benzene-3,5-diyl or optionally substituted phenylene and two R^{L1} substituents taken together form spiro cyclopropyl, then W is not 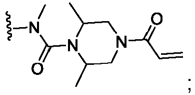 or
         (viii) when A is 1-hydroxy-benzene-3,5-diyl or optionally substituted phenylene, R^{L1} and R^{L2} taken together form fused cyclopropyl, then W is not 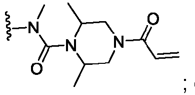 or
         (ix) when A is 1-hydroxy-benzene-3,5-diyl or optionally substituted phenylene, WH is 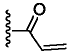 and two R^{L1} substituents taken together form spiro cyclopropyl or spiro cycloalkyl, then A1 is not 1,4-piperazin-diyl optionally substituted with methyl or C₁₋₄ alkyl; or
         (x) when A is 1-hydroxy-benzene-3,5-diyl or optionally substituted phenylene, WH is 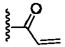 and R^{L1} and R^{L2} taken together form fused cyclopropyl or fused cycloalkyl, then A1 is not is not 1,4-piperazin-diyl optionally substituted with methyl or C₁₋₄ alkyl.
EB2. The disclosure provides a compound, or a pharmaceutically acceptable salt thereof, having the structure of Formula IIa: 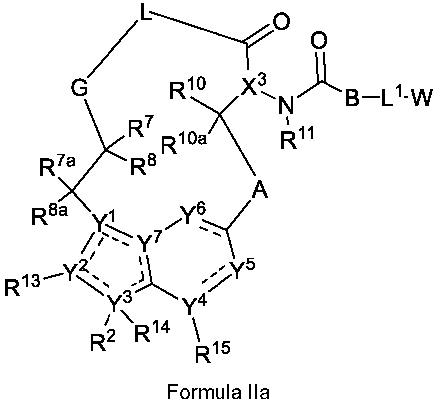
   wherein the dotted lines represent zero, one, two, three, or four non-adjacent double bonds;
   A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
   G is optionally substituted C₁-C₄ alkylene, optionally substituted C₁-C₄ alkenylene, optionally substituted C₁-C₄ heteroalkylene, -C(O)O-**C**H(R⁶)- where C is bound to -C(R⁷R⁸)-, -C(O)NH-**C**H(R⁶)-where C is bound to -C(R⁷R⁸)-, optionally substituted C₁-C₄ heteroalkylene, or 3- to 8-membered heteroarylene;
   L¹ is a linker;
   W is hydrogen, cyano, S(O)₂R', optionally substituted amino, optionally substituted amido, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3- to 11-membered heterocycloalkyl, optionally substituted 3- to 8-membered cycloalkyl, or optionally substituted 3- to 8-membered heteroaryl;
   L has the structure of Formula VIIa or Vllb: 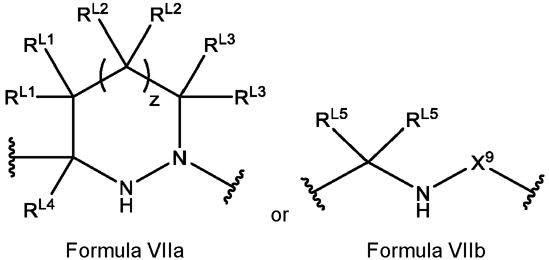
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 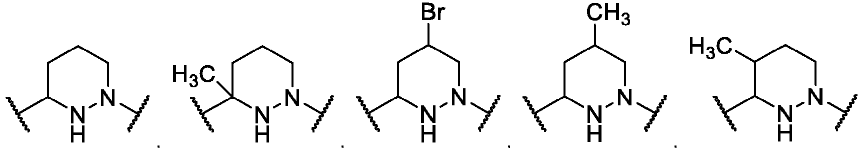 or 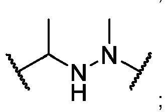
   X¹ is optionally substituted C₁-C₂ alkylene, NR, O, or S(O)_{q};
   X² is O or NH;
   X³ is N or CH;
   q is 0, 1, or 2;
   R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
   each R' is, independently, hydrogen or optionally substituted C₁-C₄ alkyl;
   Y¹ is C, CH, or N;
   Y², Y³, Y⁴, and Y⁷ are, independently, C or N;
   Y⁵ is CH, CH₂, or N;
   Y⁶ is C(O), CH, CH₂, or N;
   R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
   R¹³ and R² combine with the atoms to which they are attached to form an optionally substituted 3- to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl;
   R¹⁴ is absent or R² and R¹⁴ combine with the atom to which they are attached to form an optionally substituted 3- to 8-membered cycloalkyl or optionally substituted 3- to 14-membered heterocycloalkyl;
   R¹⁵ is absent, hydrogen, halogen, cyano, or methyl optionally substituted with 1 to 3 halogens;
   R⁵ is hydrogen, C₁-C₄ alkyl optionally substituted with halogen, cyano, hydroxy, or C₁-C₄ heteroalkyl, cyclopropyl, or cyclobutyl;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3- to 6-membered cycloalkyl or optionally substituted 3- to 7-membered heterocycloalkyl;
   R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
   R^{7a} and R^{8a} are, independently, hydrogen, halogen, optionally substituted C₁-C₃ alkyl, or combine with the carbon to which they are attached to form a carbonyl;
   R^{7'} is hydrogen, halogen, or optionally substituted C₁-C₃ alkyl;
   R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R^{7'} and R^{8'} combine with the carbon atom to which they are attached to form optionally substituted 3- to 6-membered cycloalkyl or optionally substituted 3- to 7-membered heterocycloalkyl;
   R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
   R⁹ and L combine with the atoms to which they are attached to form an optionally substituted 3- to 14-membered heterocycloalkyl;
   R^{9'} is hydrogen or optionally substituted C₁-C₆ alkyl; or
   R⁹ and R^{9'}, combined with the atoms to which they are attached, form a 3- to 6-membered cycloalkyl or a 3- to 6-membered heterocycloalkyl;
   R¹⁰ is hydrogen, halogen, hydroxy, optionally substituted C₁-C₃ heteroalkyl, or optionally substituted C₁-C₃ alkyl;
   R^{10a} is hydrogen or halogen;
   R¹¹ is hydrogen or optionally substituted C₁-C₃ alkyl; and
   R²¹ is hydrogen or optionally substituted C₁-C₃ alkyl wherein:
      (i) the compound is not 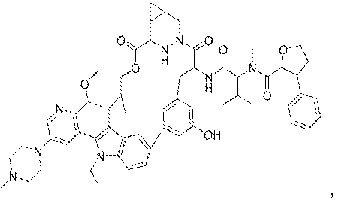 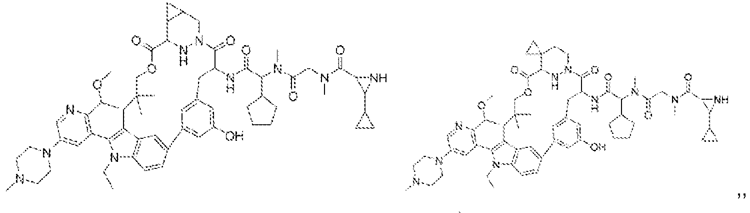 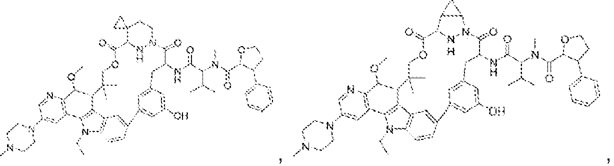 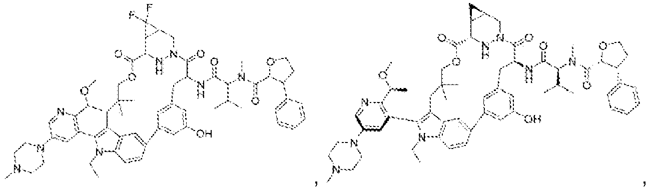 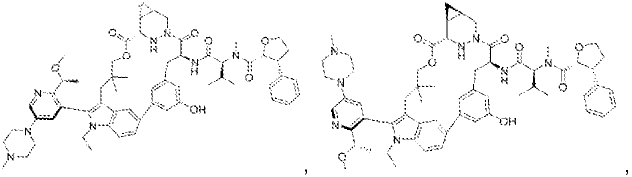 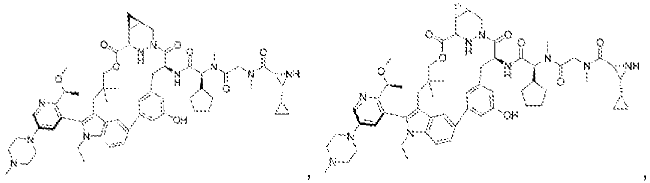 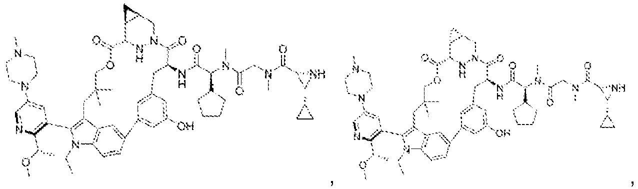 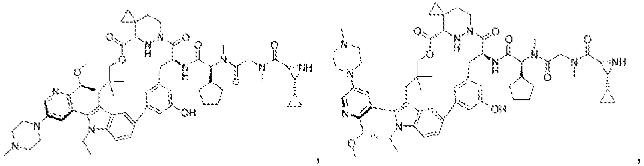 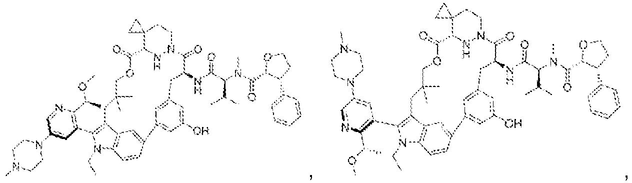 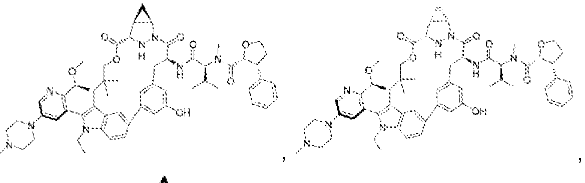 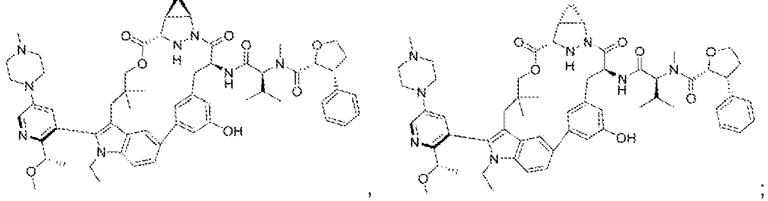 or
      (ii) when A is 1-hydroxy-benzene-3,5-diyl or optionally substituted phenylene, two R^{L1} substituents taken together form spiro cyclopropyl, L¹ is -N(CH₃)C(O)- or -N(CH₃)C(O)CH₂N(CH₃)- and B is - CH(R⁹)-, wherein R⁹ is isopropyl or cyclopentyl, then W is not 3-cyclopropylaziridin-2-yl, 3- 3-phenyltetrahydrofuran-2-yl, optionally substituted aziridin-2-yl or optionally substituted tetrahydrofuran-2-yl; or
      (iii) when A is 1-hydroxy-benzene-3,5-diyl or optionally substituted phenylene, two R^{L1} substituents taken together form spiro cyclopropyl, L¹ is -N(CH₃)C(O)- or -N(CH₃)C(O)CH₂N(CH₃)- and B is - CH(R⁹)-, wherein R⁹ is C₁₋₆ alkyl or 3- to 6-membered cycloalkyl, then W is not 3-cyclopropylaziridin-2-yl, 3-phenyltetrahydrofuran-2-yl, optionally substituted aziridin-2-yl or optionally substituted tetrahydrofuran-2-yl; or
      (iv) when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)- and B is -CH(R⁹)-, wherein R⁹ is isopropyl or cyclopentyl, then W is not 3-cyclopropylaziridin-2-yl, 3-phenyltetrahydrofuran-2-yl, optionally substituted aziridin-2-yl or optionally substituted tetrahydrofuran-2-yl; or
      (v) when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)- and B is -CH(R⁹)-, wherein R⁹ is C₁₋₆ alkyl or 3- to 6-membered cycloalkyl, then W is not 3-cyclopropylaziridin-2-yl, 3-phenyltetrahydrofuran-2-yl, optionally substituted aziridin-2-yl or optionally substituted tetrahydrofuran-2-yl; or
      (vi) when A is 1-hydroxy-benzene-3,5-diyl or optionally substituted phenylene, two R^{L1} substituents taken together form optionally substituted spiro cyclopropyl, L¹ is -N(CH₃)C(O)- or - N(CH₃)C(O)CH₂N(CH₃)-, R¹⁴ is absent and B is -CH(R⁹)-, wherein R⁹ is isopropyl or cyclopentyl, then R² is not ethyl or optionally substituted C₁₋₆ alkyl; or
      (vii) when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)-, R¹⁴ is absent and B is -CH(R⁹)-, wherein R⁹ is isopropyl or cyclopentyl, then R² is not ethyl or optionally substituted C₁₋₆ alkyl; or
      (viii) when A is 1-hydroxy-benzene-3,5-diyl or optionally substituted phenylene and two R^{L1} substituents taken together form optionally substituted spiro cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl; or
      (ix) when A is 1-hydroxy-benzene-3,5-diyl or optionally substituted phenylene, R^{L1} and R^{L2} taken together form optionally substituted fused cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl; or
      (x) when A is 1-hydroxy-benzene-3,5-diyl or optionally substituted phenylene and two R^{L1} substituents taken together form optionally substituted spiro cyclopropyl, then R² and R¹⁴ combine with the atom to which they are attached to form an optionally substituted 3- to 8-membered cycloalkyl or optionally substituted 3- to 14-membered heterocycloalkyl; or
      (xi) when A is 1-hydroxy-benzene-3,5-diyl or optionally substituted phenylene, R^{L1} and R^{L2} taken together form optionally substituted fused cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl.
EB3. The disclosure provides a compound, or a pharmaceutically acceptable salt thereof, having the structure of Formula Ilb: 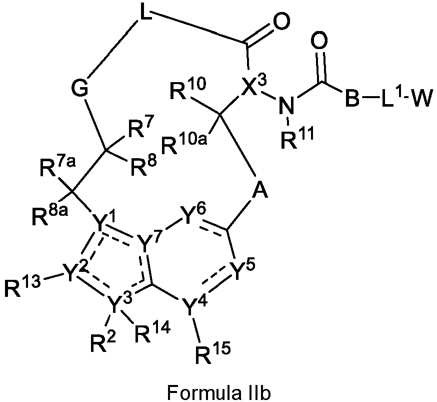
   wherein the dotted lines represent zero, one, two, three, or four non-adjacent double bonds;
   A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
   G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
   L¹ is a linker;
   W is a cross-linking group comprising a carbodiimide, an oxazoline, a thiazoline, a chloroethyl urea, a chloroethyl thiourea, a chloroethyl carbamate, a chloroethyl thiocarbamate, an aziridine, a trifluoromethyl ketone, a boronic acid, a boronic ester, an *N*-ethoxycarbonyl-2-ethoxy-1,2-dihydroquinoline (EEDQ), an iso-EEDQ or other EEDQ derivative, an epoxide, an oxazolium, or a glycal;
   L has the structure of Formula VIIa or Vllb: 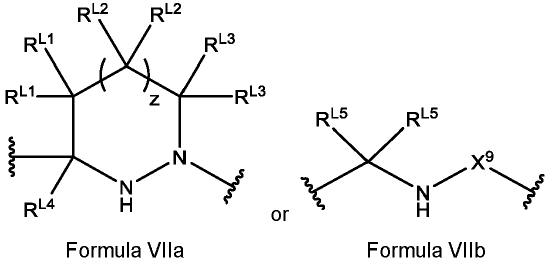
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 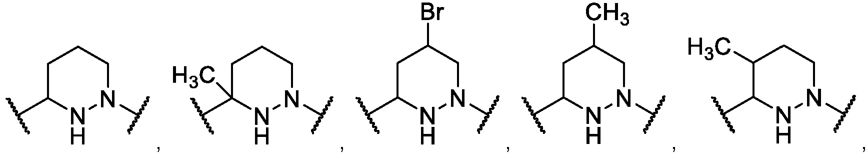 or 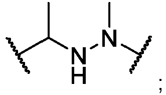
   X¹ is optionally substituted C₁-C₂ alkylene, NR, O, or S(O)_{q};
   X² is O or NH;
   X³ is N or CH;
   q is 0, 1, or 2;
   R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
   each R' is, independently, hydrogen or optionally substituted C₁-C₄ alkyl;
   Y¹ is C, CH, or N;
   Y², Y³, Y⁴, and Y⁷ are, independently, C or N;
   Y⁵ is CH, CH₂, or N;
   Y⁶ is C(O), CH, CH₂, or N;
   R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
   R¹³ and R² combine with the atoms to which they are attached to form an optionally substituted 3- to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
   R¹⁵ is absent, hydrogen, halogen, cyano, or methyl optionally substituted with 1 to 3 halogens;
   R⁵ is hydrogen, C₁-C₄ alkyl optionally substituted with halogen, cyano, hydroxy, or C₁-C₄ heteroalkyl, cyclopropyl, or cyclobutyl;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3- to 6-membered cycloalkyl or optionally substituted 3- to 7-membered heterocycloalkyl;
   R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
   R^{7a} and R^{8a} are, independently, hydrogen, halogen, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R^{7'} and R^{8'} combine with the carbon atom to which they are attached to form optionally substituted 3- to 6-membered cycloalkyl or optionally substituted 3- to 7-membered heterocycloalkyl;
   R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
   R⁹ and L combine with the atoms to which they are attached to form an optionally substituted 3- to 14-membered heterocycloalkyl;
   R^{9'} is hydrogen or optionally substituted C₁-C₆ alkyl; or
   R⁹ and R^{9'}, combined with the atoms to which they are attached, form a 3- to 6-membered cycloalkyl or a 3- to 6-membered heterocycloalkyl;
   R¹⁰ is **hydrogen,** halogen, hydroxy, optionally substituted C₁-C₃ heteroalkyl, or optionally substituted C₁-C₃ alkyl;
   R^{10a} is hydrogen or halogen;
   R¹¹ is hydrogen or optionally substituted C₁-C₃ alkyl; and
   R²¹ is hydrogen or optionally substituted C₁-C₃ alkyl wherein:
      (i) the compound is not 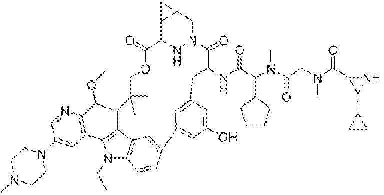 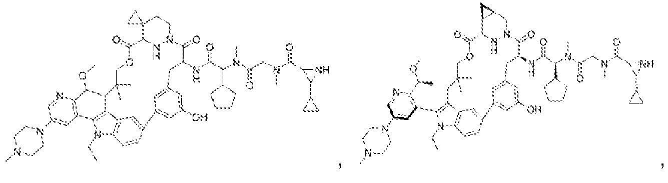 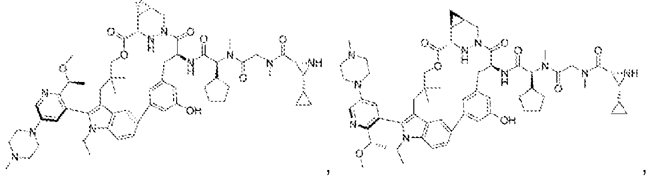 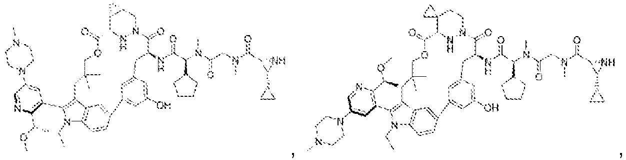 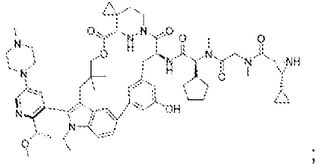 or
      (ii) when A is 1-hydroxy-benzene-3,5-diyl or optionally substituted phenylene, W is 3-cyclopropylaziridin-2-yl or optionally substituted aziridine and two R^{L1} substituents taken together form spiro cyclopropyl, then R² is not ethyl or C₁₋₆ alkyl; or
      (iii) when A is 1-hydroxy-benzene-3,5-diyl or optionally substituted phenylene, W is 3-cyclopropylaziridin-2-yl or optionally substituted aziridine and R^{L1} and R^{L2} taken together form fused cyclopropyl, then R² is not ethyl or C₁₋₆ alkyl; or
      (iv) when A is 1-hydroxy-benzene-3,5-diyl or optionally substituted phenylene and two R^{L1} substituents taken together form spiro cyclopropyl, then W is not 3-cyclopropylaziridin-2-yl or optionally substituted aziridine; or
      (v) when A is 1-hydroxy-benzene-3,5-diyl or optionally substituted phenylene and R^{L1} and R^{L2} taken together form fused cyclopropyl, then W is not 3-cyclopropylaziridin-2-yl or optionally substituted aziridine; or
      (vi) when A is 1-hydroxy-benzene-3,5-diyl or optionally substituted phenylene, W is 3-cyclopropylaziridin-2-yl or optionally substituted aziridine and two R^{L1} substituents taken together form spiro cyclopropyl, then L¹ is not -N(CH₃)C(O)CH₂N(CH₃)C(O)-; or
      (vii) when A is 1-hydroxy-benzene-3,5-diyl or optionally substituted phenylene, W is 3-cyclopropylaziridin-2-yl or optionally substituted aziridine and R^{L1} and R^{L2} taken together form fused cyclopropyl, then L¹ is not -N(CH₃)C(O)CH₂N(CH₃)C(O)-.
EB4. The disclosure provides a compound, or a pharmaceutically acceptable salt thereof, having the structure of Formula IIc: 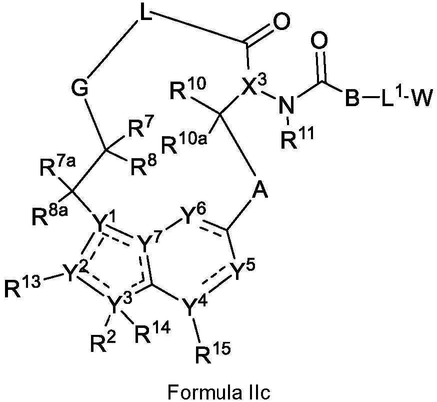
   wherein the dotted lines represent zero, one, two, three, or four non-adjacent double bonds;
   A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
   G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
   L¹ is a linker;
   W is a cross-linking group comprising a vinyl ketone, a vinyl sulfone, an ynone, a haloacetyl, or an alkynyl sulfone;
   L has the structure of Formula VIIa or Vllb: 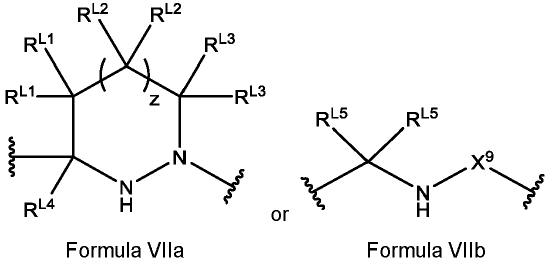
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 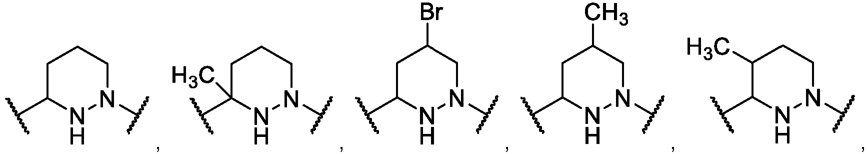 or 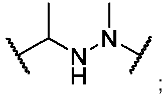
   X¹ is optionally substituted C₁-C₂ alkylene, NR, O, or S(O)_{q};
   X² is O or NH;
   X³ is N or CH;
   q is 0, 1, or 2;
   R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
   each R' is, independently, hydrogen or optionally substituted C₁-C₄ alkyl;
   Y¹ is C, CH, or N;
   Y², Y³, Y⁴, and Y⁷ are, independently, C or N;
   Y⁵ is CH, CH₂, or N;
   Y⁶ is C(O), CH, CH₂, or N;
   R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
   R¹³ and R² combine with the atoms to which they are attached to form an optionally substituted 3- to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
   R¹⁵ is absent, hydrogen, halogen, cyano, or methyl optionally substituted with 1 to 3 halogens;
   R⁵ is hydrogen, C₁-C₄ alkyl optionally substituted with halogen, cyano, hydroxy, or C₁-C₄ heteroalkyl, cyclopropyl, or cyclobutyl;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3- to 6-membered cycloalkyl or optionally substituted 3- to 7-membered heterocycloalkyl;
   R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
   R^{7a} and R^{8a} are, independently, hydrogen, halogen, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R^{7'} and R^{8'} combine with the carbon atom to which they are attached to form optionally substituted 3- to 6-membered cycloalkyl or optionally substituted 3- to 7-membered heterocycloalkyl;
   R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
   R⁹ and L combine with the atoms to which they are attached to form an optionally substituted 3- to 14-membered heterocycloalkyl;
   R^{9'} is hydrogen or optionally substituted C₁-C₆ alkyl; or
   R⁹ and R^{9'}, combined with the atoms to which they are attached, form a 3- to 6-membered cycloalkyl or a 3- to 6-membered heterocycloalkyl;
   R¹⁰ is hydrogen, halogen, hydroxy, optionally substituted C₁-C₃ heteroalkyl, or optionally substituted C₁-C₃ alkyl;
   R^{10a} is hydrogen or halogen;
   R¹¹ is hydrogen or optionally substituted C₁-C₃ alkyl; and
   R²¹ is hydrogen or optionally substituted C₁-C₃ alkyl; wherein:
      (i) the compound is not: 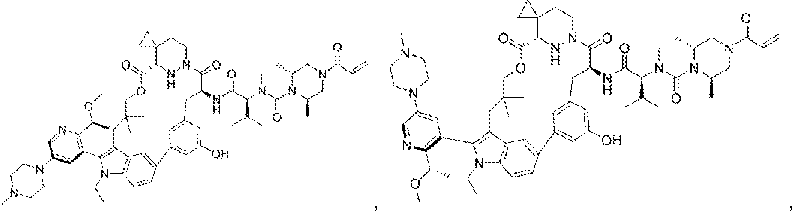 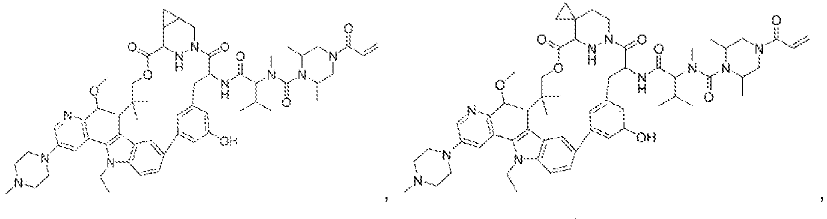 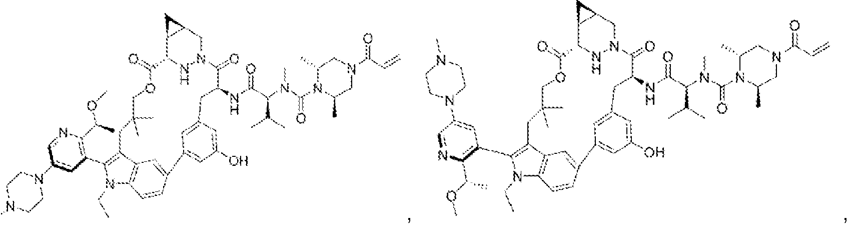 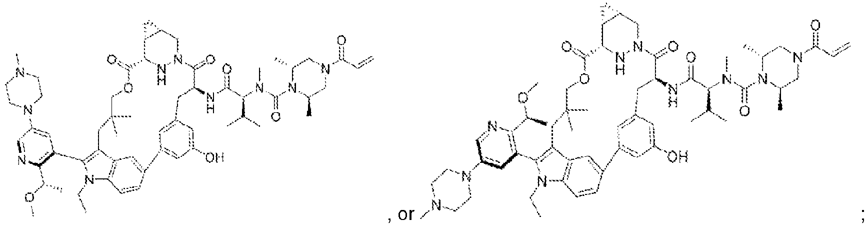 or
      (ii) when A is 1-hydroxy-benzene-3,5-diyl or optionally substituted phenylene, two R^{L1} substituents taken together form spiro cyclopropyl, and W is vinyl ketone, then R² is not ethyl or C₁₋₆ alkyl; or
      (iii) when A is 1-hydroxy-benzene-3,5-diyl or optionally substituted phenylene, R^{L1} and R^{L2} taken together form fused cyclopropyl, and W is vinyl ketone, then R² is not ethyl or C₁₋₆ alkyl; or
      (iv) when A is 1-hydroxy-benzene-3,5-diyl or optionally substituted phenylene and two R^{L1} substituents taken together form spiro cyclopropyl, then W is not vinyl ketone; or
      (v) when A is 1-hydroxy-benzene-3,5-diyl or optionally substituted phenylene, R^{L1} and R^{L2} taken together form fused cyclopropyl, then W is not vinyl ketone; or
      (vi) when A is 1-hydroxy-benzene-3,5-diyl or optionally substituted phenylene, W is vinyl ketone and two R^{L1} substituents taken together form spiro cyclopropyl or spiro cycloalkyl, then L¹ is other than 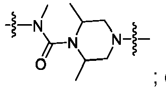 or
      (vii) when A is 1-hydroxy-benzene-3,5-diyl or optionally substituted phenylene, W is vinyl ketone and R^{L1} and R^{L2} taken together form fused cyclopropyl or fused cycloalkyl, then L¹ is other than 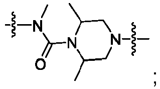 or
      (viii) when A is 1-hydroxy-benzene-3,5-diyl or optionally substituted phenylene, W is vinyl ketone and two R^{L1} substituents taken together form spiro cycloalkyl, then L¹ is not 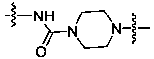 optionally substituted with 1, 2 or 3 independently selected C₁₋₆ alkyl; or
      (ix) when A is 1-hydroxy-benzene-3,5-diyl or optionally substituted phenylene, W is vinyl **ketone and** R^{L1} and R^{L2} taken together form fused cycloalkyl, then L¹ is not 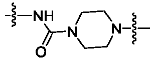 optionally substituted with 1, 2 or 3 independently selected C₁₋₆ alkyl.
EB5. The disclosure provides a compound, or a pharmaceutically acceptable salt thereof, having the structure of Formula III: 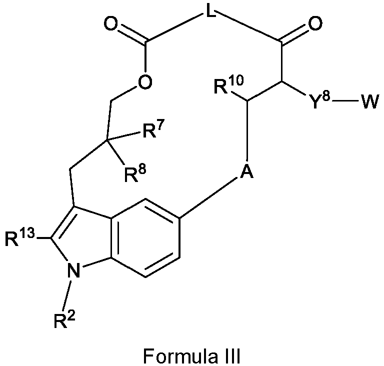
   wherein A is optionally substituted 3- to 6-membered cycloalkylene, optionally substituted 3-to 6-membered heterocycloalkylene, optionally substituted 6-membered arylene, optionally substituted 5- to 6-membered heteroarylene, optionally substituted C₂-C₄ alkylene, optionally substituted C₁-C₄ heteroalkylene or optionally substituted C₂-C₄ alkenylene;
   Y⁸ is 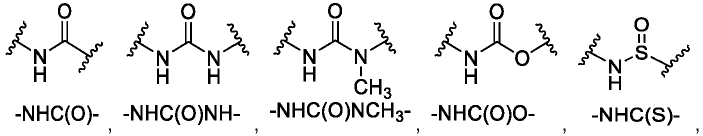 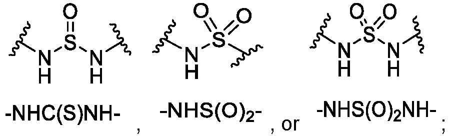
   W is hydrogen, C₁-C₄ alkyl, optionally substituted C₁-C₃ heteroalkyl, optionally substituted 3-to 10-membered heterocycloalkyl, optionally substituted 3- to 10-membered cycloalkyl, optionally substituted 6- to 10-membered aryl, or optionally substituted 5- to 10-membered heteroaryl;
   L has the structure of Formula VIIa or Vllb: 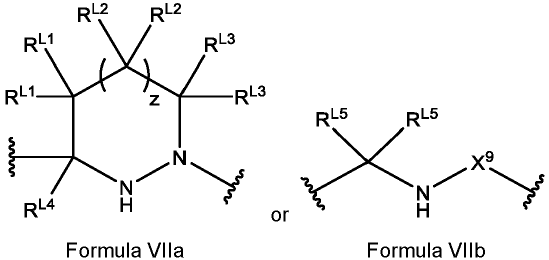
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl; or R^{L1} and R^{L2} taken together form a bond;
   wherein L does not have the structure of 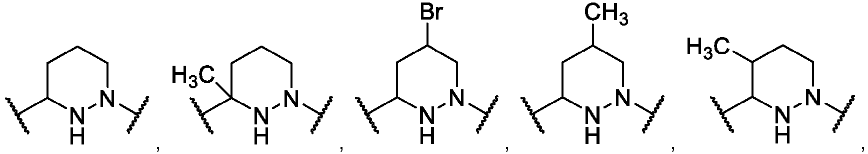 or 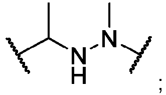
   X⁴ and X⁵ are each, independently, CH₂, CH(CH₃) or NH;
   R¹³ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 15-membered heterocycloalkyl, optionally substituted 6- to 10-membered aryl, or optionally substituted 5- to 10-membered heteroaryl;
   R² is hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl;
   R¹⁰ is hydrogen, hydroxy, optionally substituted C₁-C₆ alkoxy, optionally substituted C₁-C₃ alkyl, optionally substituted C₁-C₆ heteroalkyl, or optionally substituted 3- to 10-membered heterocycloalkyl; and
   R⁷ and R⁸ are each, independently, selected from fluoro or CH₃, or R⁷ and R⁸ combine with the atoms to which they are attached to make a 3-membered cycloalkyl; wherein:
      (i) the compound is not E10 to E52 in Table 2 and those set forth in Table 5; or
      (ii) when A is thiazole-2,4-diyl and two R^{L1} substituents taken together form spiro cyclopropyl, then R² is not optionally substituted C₁₋₆ alkyl; or
      (iii) when A is thiazole-2,4-diyl and two R^{L2} substituents taken together form spiro cyclopropyl, then R² is not optionally substituted C₁₋₆ alkyl; or
      (iv) when A is thiazole-2,4-diyl and R^{L1} and R^{L2} taken together form optionally substituted fused cyclopropyl, then R² is not optionally substituted C₁₋₆ alkyl; or
      (v) when A is thiazole-2,4-diyl and R^{L1} and R^{L2} taken together form a bond, then R² is not optionally substituted C₁₋₆ alkyl; or
      (vi) when A is thiazole-2,4-diyl and R^{L1} and R^{L2} taken together form fused cyclobutyl, then R² is not optionally substituted C₁₋₆ alkyl; or
      (vii) when A is thiazole-2,4-diyl and R^{L1} and R^{L3} together with the atoms to which they are attached form bridged cyclobutyl, then R² is not optionally substituted C₁₋₆ alkyl; or
      (viii) when A is thiazole-2,4-diyl and R^{L1} and R^{L3} together with the atoms to which they are attached form bridged cyclobutyl, then W is not optionally substituted cyclopropyl.
EB6. The disclosure provides a compound, or pharmaceutically acceptable salt thereof, having the structure of Formula IV: 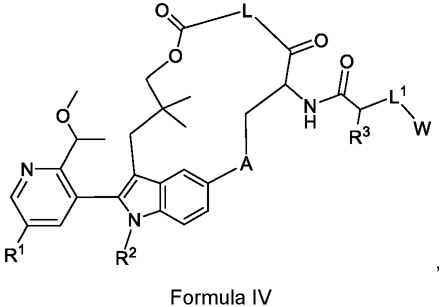
   wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
   L¹ is a linker;
   L has the structure of Formula VIIa or Vllb: 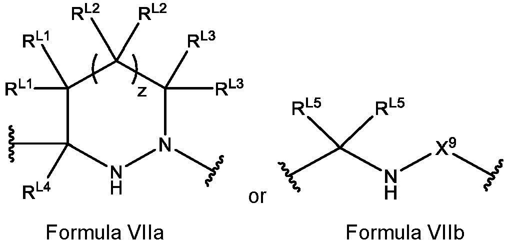
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 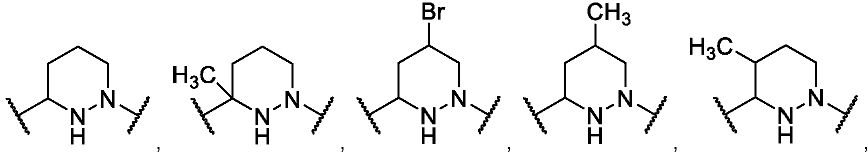 or 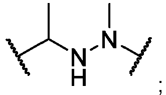
   W is a cross-linking group comprising a vinyl ketone, vinyl sulfone, ynone, or an alkynyl sulfone;
   R¹ is hydrogen, optionally substituted 3- to 10-membered heterocycloalkyl, or optionally substituted C₁-C₆ heteroalkyl;
   R² is optionally substituted C₁-C₆ alkyl; and
      R³ is optionally substituted C₁-C₆ alky optionally substituted C₁-C₃ heteroalkyl; wherein:
      (i) the compound is not: 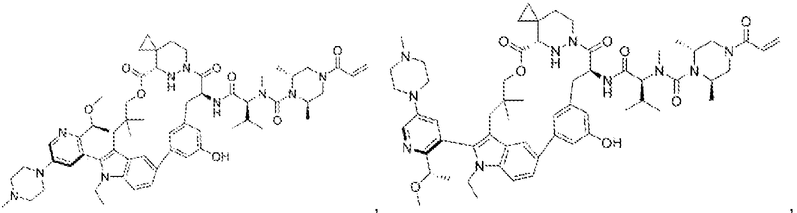 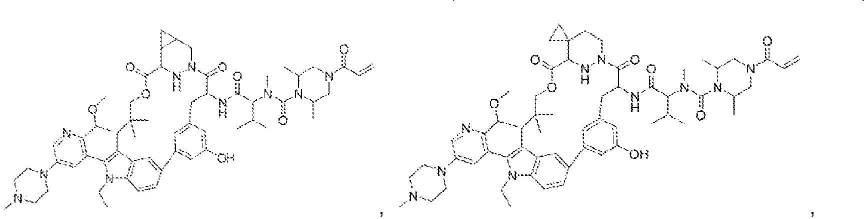 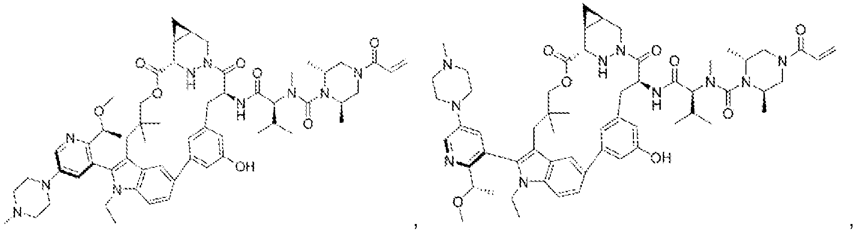 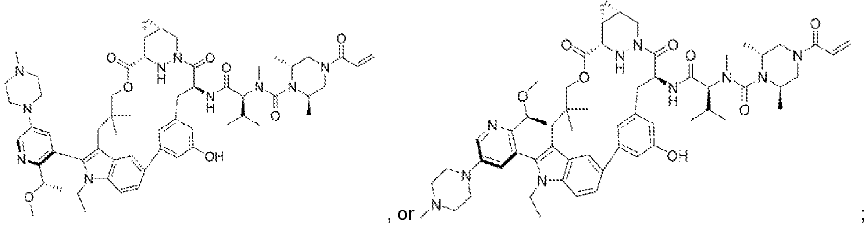 or
      (ii) when A is 1-hydroxy-benzene-3,5-diyl or optionally substituted phenylene, two R^{L1} substituents taken together form spiro cyclopropyl, and W is vinyl ketone, then R² is not ethyl; or
      (iii) when A is 1-hydroxy-benzene-3,5-diyl or optionally substituted phenylene, R^{L1} and R^{L2} taken together form fused cyclopropyl, and W is vinyl ketone, then R² is not ethyl; or
      (iv) when A is 1-hydroxy-benzene-3,5-diyl or optionally substituted phenylene and two R^{L1} substituents taken together form spiro cyclopropyl, then W is not vinyl ketone; or
      (v) when A is 1-hydroxy-benzene-3,5-diyl or optionally substituted phenylene, R^{L1} and R^{L2} taken together form fused cyclopropyl, then W is not vinyl ketone; or
      (vi) when A is 1-hydroxy-benzene-3,5-diyl or optionally substituted phenylene, W is vinyl ketone and two R^{L1} substituents taken together form spiro cyclopropyl or spiro cycloalkyl, then L¹ is other than 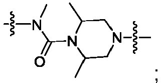 or
      (vii) when A is 1-hydroxy-benzene-3,5-diyl or optionally substituted phenylene, W is vinyl ketone and R^{L1} and R^{L2} taken together form fused cyclopropyl or fused cycloalkyl, then L¹ is not 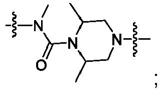 or
      (viii) when A is 1-hydroxy-benzene-3,5-diyl or optionally substituted phenylene, W is vinyl ketone and two R^{L1} substituents taken together form spiro cyclopropyl or spiro cycloalkyl, then L¹ is not 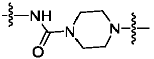 optionally substituted with 1, 2 or 3 independently selected C₁₋₆ alkyl; or
      (ix) when A is 1-hydroxy-benzene-3,5-diyl or optionally substituted phenylene, W is vinyl ketone and two R^{L1} and R^{L2} taken together form fused cyclopropyl or fused cycloalkyl, then L¹ is not 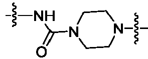 optionally substituted with 1, 2 or 3 independently selected C₁₋₆ alkyl.
EB7. The disclosure provides a compound, or a pharmaceutically acceptable salt thereof, having the structure of Formula V: 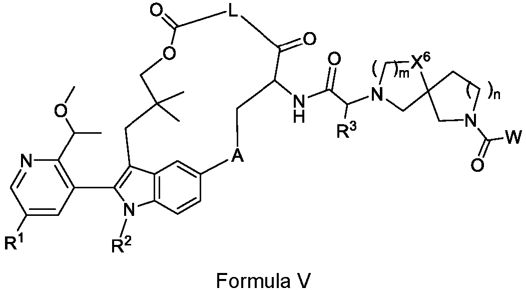
   wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
   L has the structure of Formula VIIa or Vllb: 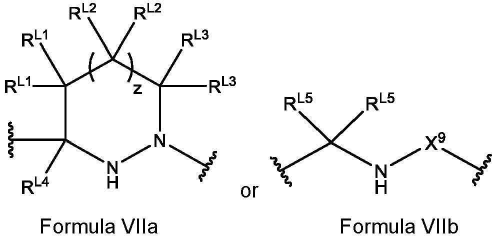
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 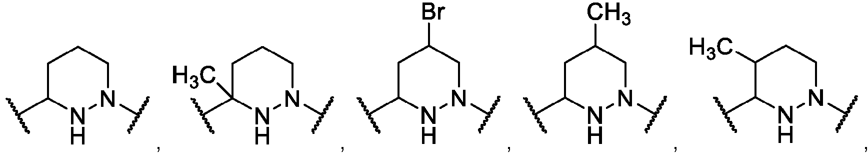 or 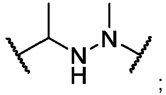
   W is a cross-linking group comprising an aziridine, an epoxide, a carbodiimide, an oxazoline, a thiazoline, a chloroethyl urea, a chloroethyl thiourea, a chloroethyl carbamate, a chloroethyl thiocarbamate, a trifluoromethyl ketone, a boronic acid, a boronic ester, an N-ethoxycarbonyl-2-ethoxy-1,2-dihydroquinoline (EEDQ), an iso-EEDQ or other EEDQ derivative, an oxazolium, or a glycal;
   X⁶ is CH₂ or O;
   m is 1 or 2;
   n is 0 or 1;
   R' is hydrogen or optionally substituted 3- to 10-membered heterocycloalkyl; and
   R² is optionally substituted C₁-C₆ alkyl.
EB8. The disclosure provides a compound, or a pharmaceutically acceptable salt thereof, having the structure of Formula VI: 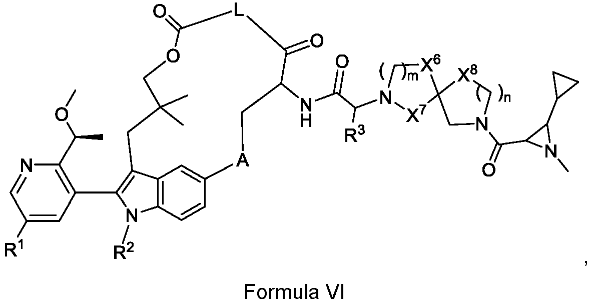
   wherein A is optionally substituted 3- to 6-membered heterocycloalkylene, optionally substituted 3- to 6-membered cycloalkylene, optionally substituted 6-membered arylene, or optionally substituted 5- to 10-membered heteroarylene;
   L has the structure of Formula VIIa or Vllb: 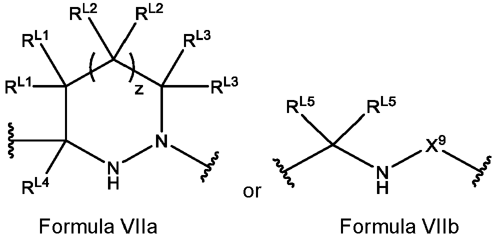
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   X⁶, X⁷, and X⁸ are each independently selected from CH₂, **CHF,** CF₂, C=O, or O;
   m is 1 or 2;
   n is 0 or 1;
   R' is hydrogen, optionally substituted C₁-C₆ heteroalkyl, or optionally substituted 3- to 10-membered heterocycloalkyl;
   R² is optionally substituted C₁-C₆ alkyl; and
   R³ is optionally substituted C₁-C₆ alkyl, optionally substituted C₁-C₆ heteroalkyl, optionally substituted 3- to 6-membered cycloalkyl, or optionally substituted heterocycloalkyl,
   and wherein each hydrogen is independently, optionally, isotopically enriched for deuterium.
EB9. The disclosure provides a compound having Formula VIIIa: 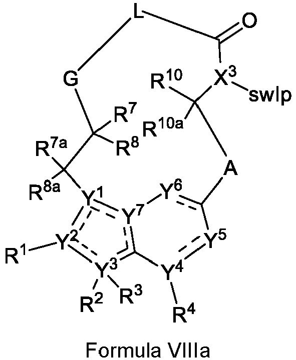 or a pharmaceutically acceptable salt thereof, wherein:
   the dotted lines represent zero, one, two, three, or four non-adjacent double bonds;
   L has the structure of Formula VIIa or Vllb: 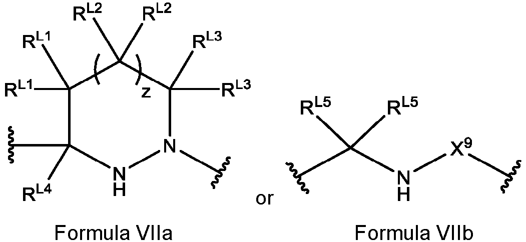
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 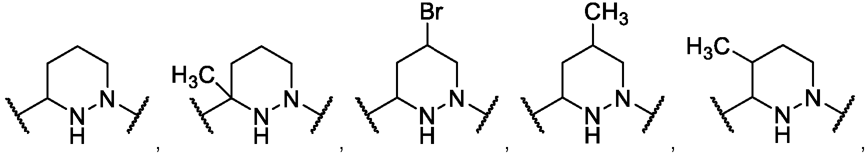 or 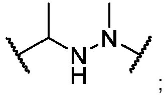
   A is optionally substituted C₂-C₄ alkylene, optionally substituted C₁-C₄ heteroalkylene, or optionally substituted C₂-C₄ alkenylene;
   G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3 to 8-membered heteroarylene;
   swlp (Switch I/P-loop) is an organic moiety that non-covalently binds to both the Switch I binding pocket and residues 12 or 13 of the P-loop of a Ras protein;
   X³ is N or CH;
   Y¹ is C, CH, or N;
   Y², Y³, Y⁴, and Y⁷ are, independently, C or N;
   Y⁵ is CH, CH₂, or N;
   Y⁶ is C(O), CH, CH₂, or N;
   R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl, or
   R¹ and R² combine with the atoms to which they are attached to form an optionally substituted 3 to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3 to 6-membered cycloalkyl, optionally substituted 3 to 7-membered heterocycloalkyl, optionally substituted 6-membered aryl, optionally substituted 5 or 6-membered heteroaryl;
   R³ is absent, or
   R² and R³ combine with the atom to which they are attached to form an optionally substituted 3 to 8-membered cycloalkyl or optionally substituted 3 to 14-membered heterocycloalkyl;
   R⁴ is absent, hydrogen, halogen, cyano, or methyl optionally substituted with 1 to 3 halogens;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3 to 6-membered cycloalkyl or optionally substituted 3 to 7-membered heterocycloalkyl;
   R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3 to 6-membered cycloalkyl, or optionally substituted 3 to 7-membered heterocycloalkyl;
   R^{7a} and R^{8a} are, independently, hydrogen, halo, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
   R^{7'} and R^{8'} combine with the carbon atom to which they are attached to form optionally substituted 3 to 6-membered cycloalkyl or optionally substituted 3 to 7-membered heterocycloalkyl;
   R¹⁰ is hydrogen, halo, hydroxy, C₁-C₃ alkoxy, or C₁-C₃ alkyl; and
   R^{10a} is hydrogen or halo.
EB10. In embodiment EB10, the disclosure provides a compound of any of embodiments EB1 to EB9 or a pharmaceutically acceptable salt thereof,
   wherein L has the structure of Formula Vlla: 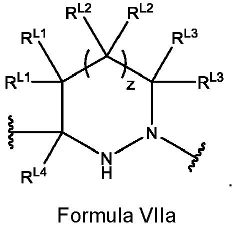
EB11. In embodiment EB11, the disclosure provides a compound of any of embodiments EB1 to
EB10 or a pharmaceutically acceptable salt thereof, wherein z is 0.
EB12. In embodiment EB12, the disclosure provides a compound of embodiment 11, or a pharmaceutically acceptable salt thereof, wherein L has the structure of Formula Vlla-1: 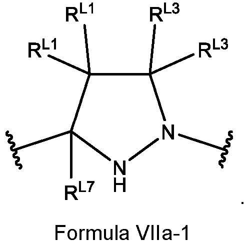
EB13. In embodiment EB13, the disclosure provides a compound of embodiment EB12, or a pharmaceutically acceptable salt thereof, wherein L has the structure of 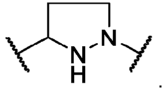
EB14. In embodiment EB13, the disclosure provides a compound of any of embodiments EB1 to EB10, or a pharmaceutically acceptable salt thereof, wherein z is 1.
EB15. In embodiment EB15, the disclosure provides a compound of embodiment EB14, or a pharmaceutically acceptable salt thereof, wherein L has the structure of Formula Vlla-2: 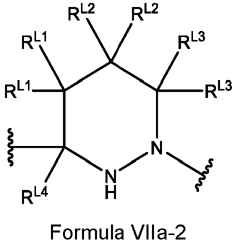
EB16. The disclosure provides a compound of embodiment EB15, or a pharmaceutically acceptable salt thereof, wherein L has the structure of Formula Vlla-3: 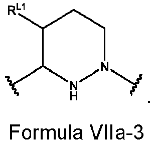
EB17. The disclosure provides a compound of embodiment EB15, or a pharmaceutically acceptable salt thereof, wherein L has the structure of Formula Vlla-4: 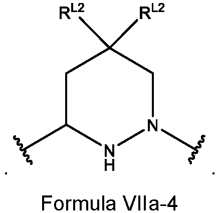
EB18. The disclosure provides a compound of embodiment EB15, or a pharmaceutically acceptable salt thereof, wherein L has the structure of Formula Vlla-5 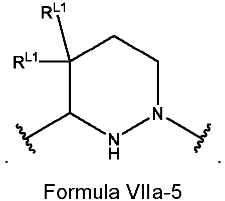
EB19. The disclosure provides a compound of any of embodiments EB1 to EB 10, or a pharmaceutically acceptable salt thereof, wherein z is 2.
EB20. The disclosure provides a compound of embodiment EB19, or a pharmaceutically acceptable salt thereof, wherein L has the structure of Formula Vlla-6: 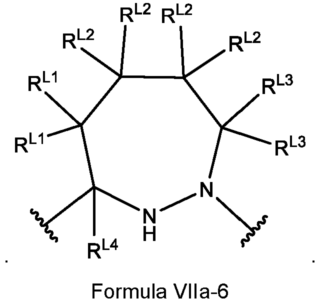
EB21. The disclosure provides a compound of any of embodiments EB1 to EB 20, or a pharmaceutically acceptable salt thereof, wherein R^{L1} is hydrogen.
EB22. The disclosure provides a compound of any of embodiments EB1 to EB 21, or a pharmaceutically acceptable salt thereof, wherein R^{L1} is optionally substituted C₁-C₆ alkyl.
EB23. The disclosure provides a compound of embodiment EB22, or a pharmaceutically acceptable salt thereof, wherein R^{L1} is methyl, ethyl, or trifluoromethyl.
EB24. The disclosure provides a compound of any of embodiments EB1 to EB 23, or a pharmaceutically acceptable salt thereof, wherein R^{L1} is optionally substituted C₁-C₆ heteroalkyl.
EB25. The disclosure provides a compound of any of embodiments EB1 to EB 24, or a pharmaceutically acceptable salt thereof, wherein R^{L1} is methoxy or ethoxy.
EB26. The disclosure provides a compound of any of embodiments EB1 to EB 25, or a pharmaceutically acceptable salt thereof, wherein R^{L1} is optionally substituted C₂-C₆ alkynyl.
EB27. The disclosure provides a compound of embodiment EB6, or a pharmaceutically acceptable salt thereof, wherein R^{L1} is ethynyl.
EB28. The disclosure provides a compound of any of embodiments EB1 to EB 27, or a pharmaceutically acceptable salt thereof, wherein R^{L2} is hydrogen.
EB29. The disclosure provides a compound of any of embodiments EB1 to EB 28, or a pharmaceutically acceptable salt thereof, wherein R^{L2} is halogen.
EB30. The disclosure provides a compound of embodiment 29, or a pharmaceutically acceptable salt thereof, wherein R^{L2} is fluoro.
EB31. The disclosure provides a compound of any of embodiments EB1 to EB 30, or a pharmaceutically acceptable salt thereof, wherein R^{L3} is hydrogen.
EB32. The disclosure provides a compound of any of embodiments EB1 to EB 30, or a pharmaceutically acceptable salt thereof, wherein R^{L3} is optionally substituted C₁-C₆ alkyl.
EB33. The disclosure provides a compound of embodiment 29, or a pharmaceutically acceptable salt thereof, wherein R^{L3} is methyl.
EB 34. The disclosure provides a compound of any of embodiments EB1 to EB 33, or a pharmaceutically acceptable salt thereof, wherein R^{L4} is hydrogen.
EB35. The disclosure provides a compound of any of embodiments EB1 to EB 34, or a pharmaceutically acceptable salt thereof, wherein R^{L1} and R^{L4} combine to form an optionally substituted C₄ cycloalkyl.
EB36. The disclosure provides a compound of any of embodiments EB1 to EB 35, or a pharmaceutically acceptable salt thereof, wherein R^{L1} and R^{L3} combine to form an optionally substituted C₄ cycloalkyl.
EB37. The disclosure provides a compound of any of embodiments EB1 to EB 36, or a pharmaceutically acceptable salt thereof, wherein R^{L1} and R^{L3} combine to form an optionally substituted C₅ cycloalkyl.
EB38. The disclosure provides a compound of any of embodiments EB1 to EB 37, or a pharmaceutically acceptable salt thereof, wherein two R^{L1} combine to form an optionally substituted C₃-C₆ cycloalkyl.
EB39. The disclosure provides a compound of any of embodiments EB1 to EB 36, or a pharmaceutically acceptable salt thereof, wherein R^{L1} and R^{L2} combine to form an optionally substituted C₃-C₆ cycloalkyl.
EB40. The disclosure provides a compound of any of embodiments EB1 to EB 39, or a pharmaceutically acceptable salt thereof, wherein L is: 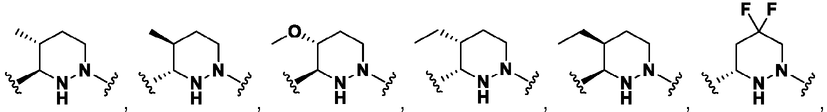 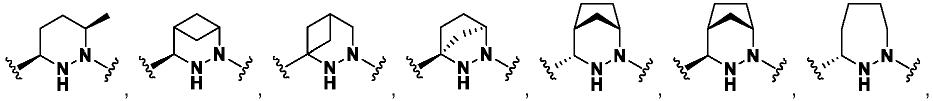 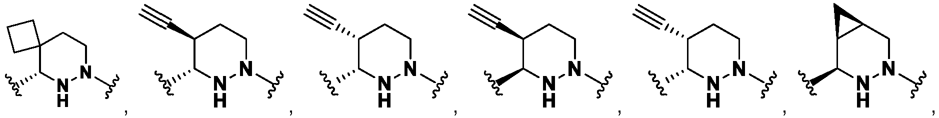 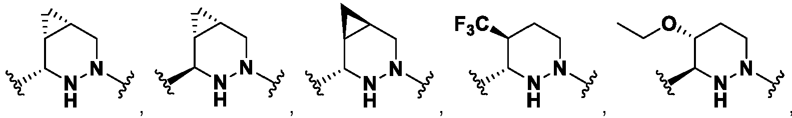 or 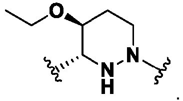
EB41. The disclosure provides a compound of embodiment EB40, or a pharmaceutically acceptable salt thereof, wherein L is: 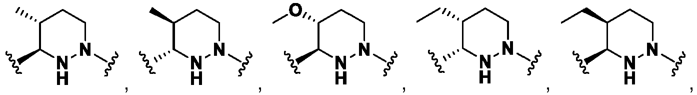 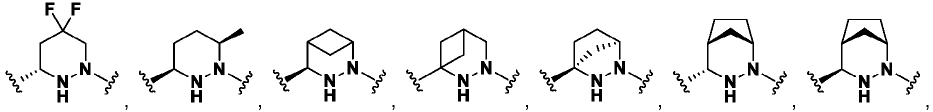 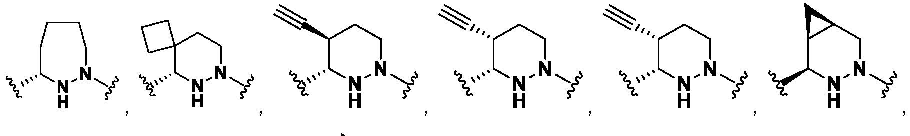 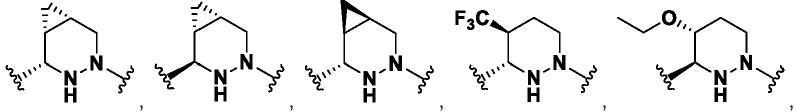 or 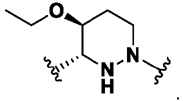
EB42. The disclosure provides a compound of any of embodiments EB1 to EB9, or a pharmaceutically acceptable salt thereof, wherein L has the structure of Formula Vllb: 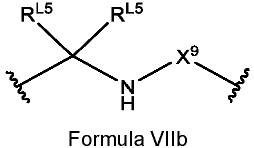
EB43. The disclosure provides a compound of embodiment EB42, or a pharmaceutically acceptable salt thereof, wherein X⁹ is -NR^{L6}-.
EB44. The disclosure provides a compound of embodiment EB42 or EB43, or a pharmaceutically acceptable salt thereof, wherein L has the structure of Formula Vllb-1: 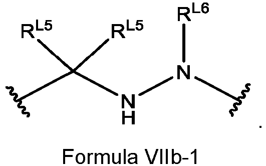
EB45. The disclosure provides a compound of embodiment EB42 or EB43, or a pharmaceutically acceptable salt thereof, wherein L has the structure of Formula Vllb-2: 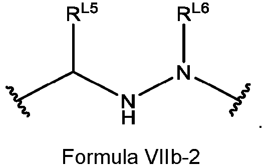
EB46. The disclosure provides a compound of any of embodiments EB42 to EB45, or a pharmaceutically acceptable salt thereof, wherein R^{L6} is optionally substituted C₁-C₆ alkyl.
EB47. The disclosure provides a compound of any of embodiments EB42 to EB45, or a pharmaceutically acceptable salt thereof, wherein R^{L6} is methyl.
EB48. The disclosure provides a compound of embodiment EB42, or a pharmaceutically acceptable salt thereof, wherein X⁹ is -C(O)-.
EB49. The disclosure provides a compound of embodiment EB42, or a pharmaceutically acceptable salt thereof, wherein X⁹ is -S(O)₂-.
EB50. The disclosure provides a compound of any of embodiments EB42 to EB49, or a pharmaceutically acceptable salt thereof, wherein R^{L5} is hydrogen.
EB51. The disclosure provides a compound of any of embodiments EB42 to EB50, or a pharmaceutically acceptable salt thereof, wherein R^{L5} is optionally substituted C₁-C₆ alkyl.
EB52. The disclosure provides a compound of any of embodiments EB42 to EB51, or a pharmaceutically acceptable salt thereof, wherein R^{L5} is optionally substituted C₃-C₈ cycloalkyl.
EB53. The disclosure provides a compound of any of embodiments EB42 to EB49, or a pharmaceutically acceptable salt thereof, wherein two R^{L5} combine to form an optionally substituted C₃-C₈ cycloalkyl.
EB54. The disclosure provides a compound of any of embodiments EB1 to EB9, or a pharmaceutically acceptable salt thereof, wherein L is: 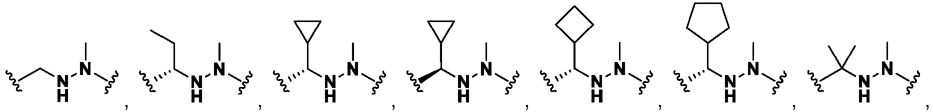 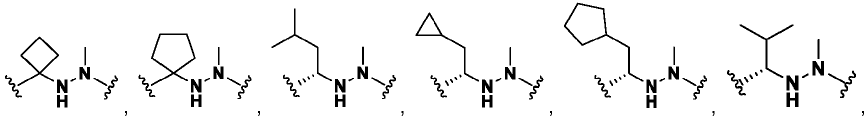 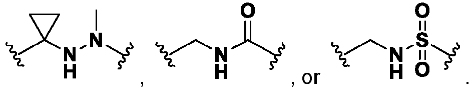
EB55. The disclosure provides a compound of embodiment EB54, or a pharmaceutically acceptable salt thereof, wherein L is: 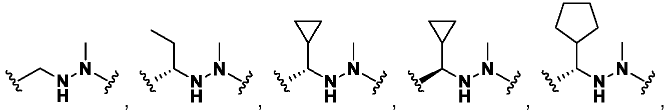 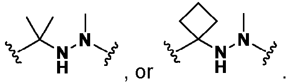
EB56. The disclosure provides a compound of any of embodiments EB1 to EB55, or a pharmaceutically acceptable salt thereof, wherein L does not have the structure of: 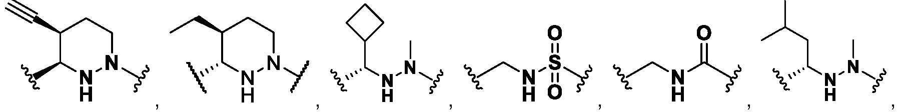 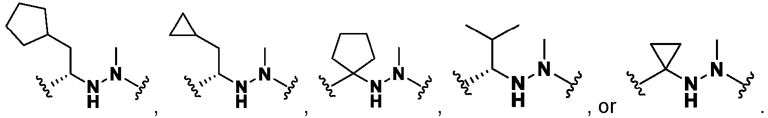
EB57. The disclosure provides a compound of any of embodiments EB1 to EB56, or a pharmaceutically acceptable salt thereof, wherein A is optionally substituted 3- to 6-membered heterocycloalkylene, optionally substituted 6-membered arylene, or optionally substituted 5- to 10-membered heteroarylene.
EB58. The disclosure provides a compound of embodiment EB57, or a pharmaceutically acceptable salt thereof, wherein A is optionally substituted 6-membered arylene.
EB59. The disclosure provides a compound of embodiment EB58, or a pharmaceutically acceptable salt thereof, wherein A is 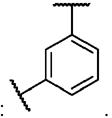
EB60. The disclosure provides a compound of embodiment EB57, or a pharmaceutically acceptable salt thereof, wherein A is optionally substituted 3- to 6-membered heterocycloalkylene.
EB61. The disclosure provides a compound of embodiment EB60, or a pharmaceutically acceptable salt thereof, wherein A is 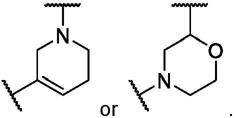
Eb62. The disclosure provides a compound of embodiment EB57, or a pharmaceutically acceptable salt thereof, wherein A is optionally substituted 5- to 10-membered heteroarylene.
EB63. The disclosure provides a compound of embodiment EB62, or a pharmaceutically acceptable salt thereof, wherein A is 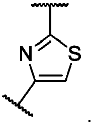
EB64. The disclosure provides a compound of any of embodiments EB2 to EB63, or a pharmaceutically acceptable salt thereof, wherein R² is ethyl or haloethyl.
EB65. The disclosure provides a compound of any of embodiments EB1 and EB7 to EB64, or a pharmaceutically acceptable salt thereof, wherein R³ is optionally substituted C₁-C₆ alkyl.
EB66. The disclosure provides a compound of or a pharmaceutically acceptable salt thereof, of Table 1.
EB67. The disclosure provides a pharmaceutical composition comprising a compound, or a pharmaceutically acceptable salt thereof, of any of embodiments EB1 to EB66 and a pharmaceutically acceptable excipient.
EB68. The disclosure provides a method of treating cancer in a subject in need thereof, the method comprising administering to the subject a therapeutically effective amount of a compound, or a pharmaceutically acceptable salt thereof, of any of embodiments EB1 to EB66 or a pharmaceutical composition of embodiment EB67.
EB69. The disclosure provides a method of embodiment EB68, wherein the cancer is pancreatic cancer, colorectal cancer, non-small cell lung cancer, or endometrial cancer.
EB70. The disclosure provides a method of embodiment EB68 or EB69, wherein the cancer comprises a Ras mutation.
EB71. The disclosure provides a method of treating a Ras protein-related disorder in a subject in need thereof, the method comprising administering to the subject a therapeutically effective amount of a compound of any embodiments EB1 to EB66, or a pharmaceutically acceptable salt thereof, or a pharmaceutical composition of embodiments EB67.
EB72. The disclosure provides a method of inhibiting a Ras protein in a cell, the method comprising contacting the cell with an effective amount of a compound of any of embodiments EB1 to EB 66, or a pharmaceutically acceptable salt thereof, or a pharmaceutical composition of embodiment EB 67. EB73. The disclosure provides a method of embodiment EB71 or EB72, wherein the Ras protein K-Ras.
EB74. The disclosure provides a method of embodiment EB72 or EB73, wherein the cell is a cancer cell.
EB75. The disclosure provides a method of embodiment EB74, wherein the cancer cell is a pancreatic cancer cell, a colorectal cancer cell, a non-small cell lung cancer cell, or an endometrial cancer cell. EB76. The disclosure provides a method of any of embodiments EB68 to EB75, wherein the method further comprises administering an additional anti-cancer therapy.
EB77. The disclosure provides a method of embodiment EB76, wherein the additional anti-cancer therapy is an EGFR inhibitor, a second Ras inhibitor, a SHP2 inhibitor, a SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, a HER2 inhibitor, or a combination thereof.

### Examples

The disclosure is further illustrated by the following examples and synthesis examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure or scope of the appended claims.

### Chemical Syntheses

Definitions used in the following examples and elsewhere herein are:
- B₂pin₂: Bis(pinacolato)diboron
- BINAP: 2,2'-Bis(diphenylphosphino)-1,1'-binaphthyl
- CH₂Cl₂, DCM: Methylene chloride, Dichloromethane
- CH₃CN, MeCN: Acetonitrile
- Cul: Copper (I) iodide
- DIPEA, DIEA: Diisopropylethyl amine
- DMF: N,N-Dimethylformamide
- EA: Ethyl acetate
- EDCI: N-Ethyl-N'-carbodiimide hydrochloride
- EtOAc: Ethyl acetate
- h: hour
- H₂O: Water
- HCl: Hydrochloric acid
- HOBt: Hydroxybenzotriazole
- K₃PO₄: Potassium phosphate (tribasic)
- MeOH: Methanol
- Na₂SO₄: Sodium sulfate
- NMM: N-methylmorpholine
- NMP: N-methyl pyrrolidone
- Pd(dppf)Cl₂: [1,1'-Bis(diphenylphosphino)ferrocene]dichloropalladium(II)
- PE: Petroleum ether
- rt: Room temperature
- TFA: Trifluoroacetic acid

### Instrumentation

Mass spectrometry data collection took place with a Shimadzu LCMS-2020, an Agilent 1260LC-6120/6125MSD, a Shimadzu LCMS-2010EV, or a Waters Acquity UPLC, with either a Qda detector or SQ Detector 2. Samples were injected in their liquid phase onto a C-18 reverse phase. The compounds were eluted from the column using an acetonitrile gradient and fed into the mass analyzer. Initial data analysis took place with either Agilent ChemStation, Shimadzu LabSolutions, or Waters MassLynx. NMR data was collected with either a Bruker AVANCE III HD 400MHz, a Bruker Ascend 500MHz instrument, or a Varian 400MHz, and the raw data was analyzed with either TopSpin or Mestrelab mnova.

Preparation of Intermediate 3 Analog Examples 1-27 shown below refer to analogs of compound 3 of Scheme 1 described above (see "Methods of Synthesis" section). Other references to Scheme 1 below refer to this same Scheme.

### Synthesis of Intermediate 3 (Scheme 1) Analogs

### Analog Example 1. Ethyl (methylamino)glycinate

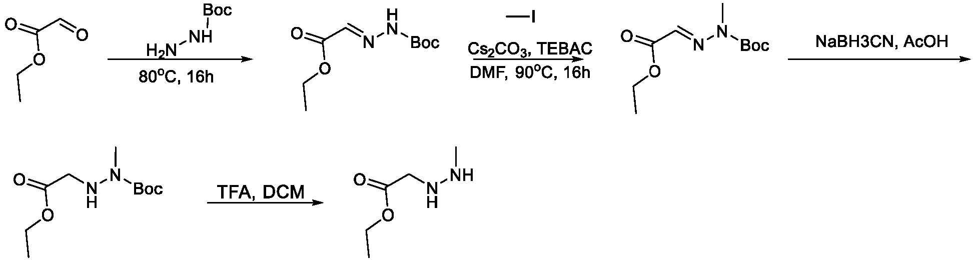

### Step 1

A 40 mL vial was charged with ethyl 2-oxoacetate (1 g, 9.80 mmol, 1 equiv) and *tert-butyl* hydrazinecarboxylate (1 g, 7.57 mmol, 0.77 equiv) at room temperature. The resulting mixture was stirred overnight at 80 °C. This resulted in tert-butyl (E)-2-(2-ethoxy-2-oxoethylidene) hydrazine-1-carboxylate as a light yellow oil. The crude product was used in the next step without further purification. LCMS (ESI): m/z [M-H] calc'd for C₉H₁₆N₂O₄ 216.1; found 215.2.

### Step 2

To a 100 mL round-bottom flask was added *tert-butyl* (E)-2-(2-ethoxy-2-oxoethylidene) hydrazine-1-carboxylate (2 g, 9.25 mmol, 1 equiv), DMF (20 mL), methyl iodide (3.94 g, 27.75 mmol, 3 equiv), Cs₂CO₃ (6.03 g, 18.50 mmol, 2 equiv) and benzyltriethylammonium chloride (2.11 g, 9.249 mmol, 1 equiv) at room temperature. The resulting mixture was stirred overnight at 90 °C under an argon atmosphere. The mixture was quenched with H₂O (50 mL) and extracted with EtOAc (3 x 50 mL), dried over anhydrous Na₂SO₄. After filtration, the filtrate was concentrated under reduced pressure. The crude product was purified by reverse phase flash chromatography to afford tert-butyl (E)-2-(2-ethoxy-2-oxoethylidene)-1-methylhydrazine-1-carboxylate (1.2 g, 56.34% yield) as light yellow oil. LCMS (ESI): m/z [M+H] calc'd for C₁₀H₁₈N₂O₄ 230.1; found 231.2

### Step 3

To a stirring solution of *tert-butyl* (E)-2-(2-ethoxy-2-oxoethylidene)-1-methylhydrazine-1-carboxylate (1.267 g, 5.50 mmol, 1 equiv) in AcOH (12 mL) was added NaBH₃CN (0.86 g, 13.76 mmol, 2.5 equiv) at 0 °C. The resulting mixture was stirred for 1 h at room temperature. The mixture was acidified to pH 9 with saturated NaHCO₃ (aq.). The resulting mixture was extracted with EtOAc (3 x 20 mL). The organic layers were dried over anhydrous Na₂SO₄. After filtration, the filtrate was concentrated under reduced pressure. The crude product was purified by reverse phase flash chromatography to afford *tert*-butyl 2-(2-ethoxy-2-oxoethyl)-1-methylhydrazine-1-carboxylate (1 g, 78.24% yield) as a light yellow oil. LCMS (ESI): m/z [M+H] calc'd for C₁₀H₂₀N₂O₄ 232.1; found 233.3

### Step 4

To a solution of *tert*-butyl 2-(2-ethoxy-2-oxoethyl)-1-methylhydrazine-1-carboxylate (1.5 g, 6.46 mmol, 1 equiv) in DCM (20 mL) is added TFA (10 mL, 134.63 mmol, 20.85 equiv) at 0 °C. The resulting mixture was stirred for 1 h at room temperature under an argon atmosphere. The mixture was concentrated under reduced pressure, affording ethyl (methylamino) glycinate as a light yellow oil. LCMS (ESI): m/z [M+H] calc'd for C₁₀H₂₀N₂O₄ 232.1; found 233.3

### Analog Example 2. methyl 2,3-diazabicyclo[3.1.1]heptane-4-carboxylate

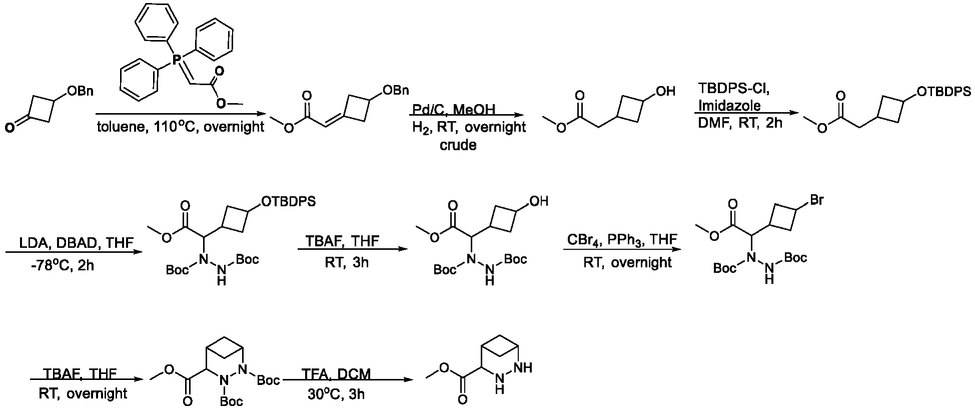

### Step 1

To a 2000 mL 3-necked round-bottom flask was added 3-(benzyloxy) cyclobutan-1-one (125 g, 709.36 mmol, 1 equiv), toluene (1000 mL) and methyl 2-(triphenyl-lambda-5-phosphanylidene) acetate (237.18 g, 709.36 mmol, 1 equiv) at room temperature. The resulting mixture was stirred overnight at 110 °C then cooled and concentrated under reduced pressure. The residue was purified by column chromatography to afford methyl 2-[3-(benzyloxy) cyclobutylidene] acetate (160 g, 97% yield) as a colorless oil. LCMS (ESI): m/z [M+H] calc'd for C₁₄H₁₆O₃ 232.1; found 233.1

### Step 2

To a 2000 mL 3-necked round-bottom flask was added methyl 2-[3-(benzyloxy) cyclobutylidene] acetate (160 g, 688.83 mmol, 1 equiv), MeOH (1200 mL) and Pd/C (40 g) at room temperature. The resulting mixture was stirred overnight at room temperature under a hydrogen atmosphere. The reaction was filtered, and the filter cake was washed with MeOH (3 x 100 mL). The filtrate was concentrated under reduced pressure to afford methyl 2-(3-hydroxycyclobutyl) acetate (90 g, crude) as a colorless oil. LCMS (ESI): m/z [M+H] calc'd for C₇H₁₂O₃ 144.1; found 145.1

### Step 3

To a 250 mL round-bottom flask were added methyl 2-(3-hydroxycyclobutyl) acetate (10 g, 69.36 mmol, 1.00 equiv), DMF (100 mL), Imidazole (11.81 g, 173.41 mmol, 2.5 equiv), and *tert-*Butylchlorodiphenylsilane (22.88 g, 83.24 mmol, 1.2 equiv) at 0 °C. The resulting mixture was stirred for 2 h at room temperature. Upon completion the reaction was quenched with H₂O (50 mL) and extracted with EtOAc (3 x 50 mL). The combined organic layers were washed with water (3 x 50 mL), and dried over anhydrous Na₂SO₄. After filtration, the filtrate was concentrated under reduced pressure. The residue was purified by reverse flash chromatography to afford methyl *2-(3-((tert-*butyldiphenylsilyl)oxy)cyclobutyl)acetate (20 g, 75% yield) as colorless oil. LCMS (ESI): m/z [M+H] calc'd for C₂₃H₃₀O₃Si 382.2; found 383.3

### Step 4

To a solution of methyl 2-{3-[(*tert*-butyldiphenylsilyl)oxy]cyclobutyl}acetate (18 g, 47.05 mmol, 1.00 equiv) in THF (200 mL) at -78 °C is added LDA (35.29 mL, 70.56 mmol, 1.5 equiv). The resulting mixture was stirred for 1h at -78 °C under an argon atmosphere. To the mixture was added DBAD (16.25 g, 70.58 mmol, 1.5 equiv) dropwise over 10 min. The resulting mixture was stirred for additional 1 h at -78 °C. The reaction was quenched with H₂O and extracted with EtOAc (3 x 100 mL). The combined organic layers were washed with water (3 x 50 mL) and dried over anhydrous Na₂SO₄. After filtration, the filtrate was concentrated under reduced pressure. The residue was purified by column chromatography to afford methyl 2-[(tert-butoxycarbonyl)[(tert-butoxycarbonyl)amino]amino]-2-{3-[(tert-butyldiphenylsilyl)oxy]cyclobutyl}acetate (20 g, 69% yield) as a light yellow solid. LCMS (ESI): m/z [M+H] calc'd for C₃₃H₄₈N₂O₇Si 612.3; found 635.3 [M+Na]

### Step 5

To a stirring solution of methyl 2-[(*tert*-butoxycarbonyl)[(tert-butoxycarbonyl)amino]amino]-2-{3-[(*tert*-butyldiphenylsilyl)oxy]cyclobutyl}acetate (20 g, 32.64 mmol, 1 equiv) in THF (150 mL) was added TBAF (65.27 mL, 65.27 mmol, 2 equiv) at 0 °C. The resulting mixture was stirred for 3 h at room temperature. The reaction was then was concentrated under reduced pressure. The residue was purified by silica gel column chromatography to afford methyl 2-[(*tert*-butoxycarbonyl)[(tert-butoxycarbonyl)amino]amino]-2-(3-hydroxycyclobutyl)acetate (10 g, 81% yield) as a colorless solid. LCMS (ESI): m/z [M+H] calc'd for C₁₇H₃₀N₂Or 374.2; found 375.2

### Step 6

To a solution of methyl 2-[(*tert*-butoxycarbonyl)[(tert-butoxycarbonyl)amino]amino]-2-(3-hydroxycyclobutyl)acetate (10 g, 26.71 mmol, 1 equiv) in THF (100 mL) was added PPh₃ (9.11 g, 34.72 mmol, 1.3 equiv) and CBr₄ (10.63 g, 32.05 mmol, 1.2 equiv) at 0 °C. The resulting mixture was stirred overnight at room temperature then quenched with H₂O (200 mL) and extracted with EtOAc (3 x 200 mL). The combined organic layers were dried over anhydrous Na₂SO₄. After filtration, the filtrate was concentrated under reduced pressure. The residue was purified by column chromatography to afford methyl 2-(3-bromocyclobutyl)-2-[(tert-butoxycarbonyl) [(tert-butoxycarbonyl) amino] amino] acetate (7 g, 59% yield) as a yellow oil. LCMS (ESI): m/z [M+H] calc'd for C₁₇H₂₉N₂O₆Br 436.1; found 437.2

### Step 7

To a solution of methyl 2-(3-bromocyclobutyl)-2-[(*tert*-butoxycarbonyl) [(*tert*-butoxycarbonyl) amino] amino] acetate (7 g, 16.01 mmol, 1 equiv) in THF (70 mL) was added TBAF (80.03 mL, 80.03 mmol, 5 equiv) at 0 °C. The resulting mixture was stirred for overnight at room temperature under then concentrated under reduced pressure. The resulting residue was diluted with H₂O (100 mL) and extracted with EtOAc (3 x 100 mL). The combined organic layers were washed with water (1 x 100 mL), and dried over anhydrous Na₂SO₄. After filtration, the filtrate was concentrated under reduced pressure. The residue was purified by column chromatography to afford 2,3-di-tert-butyl 4-methyl 2,3-diazabicyclo [3.1.1] heptane-2,3,4-tricarboxylate (3 g, 52% yield) as a yellow solid. LCMS (ESI): m/z [M+H] calc'd for C₁₇H₂₈N₂O₆ 356.2; found 357.2

### Step 8

To a solution of 2,3-di-tert-butyl 4-methy-2,3-diazabicyclo[3.1.1]heptane-2,3,4-tricarboxylate (3 g, 8.417 mmol, 1 equiv) in DCM (15 mL) was added TFA (6 mL) at 0 °C. The resulting mixture was stirred for 3 h at 30 °C. The reaction was concentrated under reduced pressure. This resulted in methyl 2,3-diazabicyclo[3.1.1]heptane-4-carboxylate (2.5 g, crude) as yellow oil. The crude product was used in the next step directly without further purification. LCMS (ESI): m/z [M+H] calc'd for C₇H₁₂N₂O₂ 156.1; found 157.2

### Analog Example 3. methyl 4-vinylhexahydropyridazine-3-carboxylate

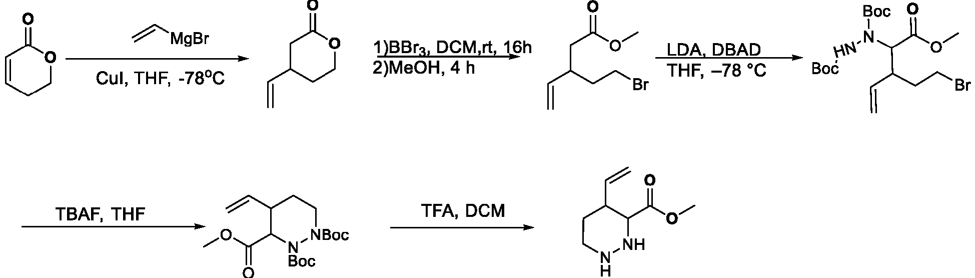

### Step 1

To a stirred solution of Cul (14.56 g, 76.45 mmol, 1.5 equiv) in THF (50 mL) at -78 °C was added bromo(ethenyl)magnesium (76.45 mL, 76.45 mmol, 1.5 equiv) in portions under nitrogen atmosphere. The resulting mixture was stirred for 1 h at -78 °C under nitrogen atmosphere. To the above mixture was added 5,6-dihydropyran-2-one (5 g, 50.97 mmol, 1 equiv) dropwise over 1 h at -78 °C. The resulting mixture was stirred for an additional 1 h at -60 °C. The reaction was quenched with aq. sat. NH₄Cl (50 mL) at 0 °C. The resulting mixture was extracted with EtOAc (3 x 100 mL). The combined organic layers were washed with brine (3 x 100 mL), dried over anhydrous Na₂SO₄. After filtration, the filtrate was concentrated under reduced pressure. The residue was purified by silica gel column chromatography to afford 4-ethenyloxan-2-one (4 g, 62.21% yield) as a light yellow oil. ¹H NMR (300 MHz, Chloroform-d) δ = 5.88 - 5.70 (m, 1H), 5.16 - 4.98 (m, 2H), 4.44 (dt, J = 11.3, 4.7 Hz, 1H), 4.30 (ddd, J = 11.3, 9.8, 3.9 Hz, 1H), 2.73 (ddd, J = 11.3, 5.3, 1.6 Hz, 1H), 2.73 - 2.60 (m, 1H), 2.45 - 2.29 (m, 1H), 2.09 - 1.94 (m, 1H), 1.72 (dtd, J = 14.3, 9.6, 4.8 Hz, 1H).

### Step 2

To a stirred solution of 4-ethenyloxan-2-one (4 g, 31.71 mmol, 1 equiv) in DCM (40 mL) at 0 °C was added BBr₃ (5.99 mL, 63.414 mmol, 2 equiv) under nitrogen atmosphere. The resulting mixture was stirred overnight at room temperature. To the above mixture was added MeOH (8 mL) at 0 °C. The resulting mixture was stirred for an additional 4 h at room temperature. The reaction was quenched with aq. sat. NaHCO₃ (50 mL) at 0 °C. The resulting mixture was extracted with CH₂Cl₂ (3 x 50 mL). The combined organic layers were washed with brine (3 x 50 mL) and dried over anhydrous Na₂SO₄. After filtration, the filtrate was concentrated under reduced pressure. The residue was purified by silica gel column chromatography to afford methyl 3-(2-bromoethyl)pent-4-enoate (4.5 g, 64.19% yield) as a light yellow oil. ¹H NMR (300 MHz, Chloroform-d) δ 5.60 (ddd, J = 17.1, 10.2, 8.6 Hz, 1H), 5.22 - 5.07 (m, 2H), 3.68 (s, 3H), 3.45 (ddd, J = 10.0, 7.5, 5.1 Hz, 1H), 3.34 (ddd, J = 10.0, 8.2, 7.1 Hz, 1H), 2.78 (dtd, J = 16.1, 8.2, 4.6 Hz, 1H), 2.49 - 2.28 (m, 2H), 2.11 - 1.78 (m, 2H).

### Step 3

To a stirred solution of methyl 3-(2-bromoethyl)pent-4-enoate (4.5 g, 20.35 mmol, 1 equiv) in THF (45 mL) at -78 °C was added LDA (20.35 mL, 40.70 mmol, 2 equiv) dropwise under nitrogen atmosphere. The resulting mixture was stirred for 1 h at -78 °C. To the above mixture was added DBAD (5.62 g, 24.42 mmol, 1.2 equiv) at -78 °C. The resulting solution was stirred for additional 2 h at -78 °C. The reaction was quenched with aq. sat. NH₄Cl (50 mL) at 0 °C then extracted with EtOAc (3 x 100 mL). The combined organic layers were washed with brine (3 x 100 mL), dried over anhydrous Na₂SO₄. After filtration, the filtrate was concentrated under reduced pressure. The residue was purified by silica gel column chromatography to afford methyl 3-(2-bromoethyl)-2-[(tert-butoxycarbonyl)[(*tert*-butoxycarbonyl)amino]amino]pent-4-enoate (2.2 g, 23.95% yield) as a light yellow oil. ¹H NMR (300 MHz, Chloroform-d) δ 5.68 (dt, J = 18.5, 9.7 Hz, 1H), 5.17 (m, J = 17.9 Hz, 2H), 3.77 (s, 3H), 3.55 (s, 3H), 3.47 - 3.26 (m, 1H), 2.18 (s, 2H), 1.48 (s, 18H).

### Step 4

To a stirred solution of methyl 3-(2-bromoethyl)-2-[(tert-butoxycarbonyl)[(tert-butoxycarbonyl)amino]amino]pent-4-enoate (2.2 g, 4.87 mmol, 1 equiv) in THF (20 mL) was added TBAF (9.75 mL, 9.75 mmol, 2 equiv) at 0 °C under nitrogen atmosphere. The resulting mixture was stirred for 2 h at room temperature then diluted with brine (20 mL) and extracted with EtOAc (3 x 50 mL). The combined organic layers were washed with brine (3 x 50 mL), dried over anhydrous Na₂SO₄. After filtration, the filtrate was concentrated under reduced pressure and purified by silica gel column chromatography to afford 1,2-di-tert-butyl 3-methyl 4-ethenyl-1,2-diazinane-1,2,3-tricarboxylate (1.7 g, 94.15% yield) as a light yellow oil. LCMS (ESI): m/z [M+H] calc'd for C₁₈H₃₀N₂O₆ 370.2; found 371.2

### Step 5

To a stirred solution of 1,2-di-tert-butyl 3-methyl 4-ethenyl-1,2-diazinane-1,2,3-tricarboxylate (2 g, 5.40 mmol, 1 equiv) in DCM was added TFA (2 mL) dropwise at 0 °C under argon atmosphere. The resulting mixture was stirred for 1 h at room temperature then concentrated under reduced pressure. The crude product was used in the next step directly without further purification. This resulted in methyl 4-ethenyl-1,2-diazinane-3-carboxylate (900 mg, 93.04% yield) as a yellow oil. LCMS (ESI): m/z [M+H] calc'd for C₈H₁₄N₂O₂ 170.1; found 171.2.

### Analog Example 4. Synthesis of methyl 4-((triisopropylsilyl)ethynyl)hexahydropyridazine-3-carboxylate

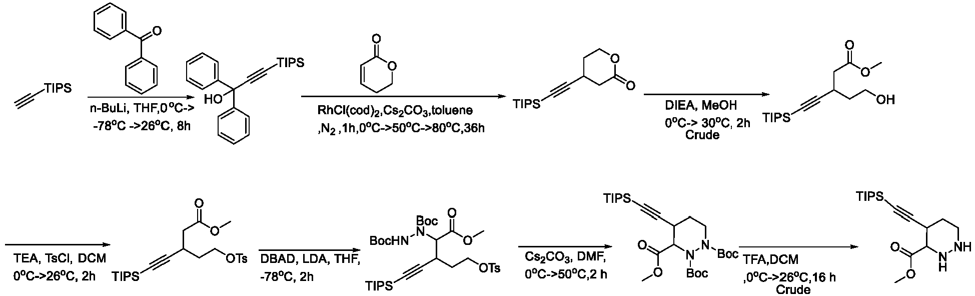

### Step 1

To a stirred solution of ethynyltriisopropylsilane (31.28 g, 171.49 mmol, 1.25 equiv) in THF (200 mL) at 0 °C was added butyllithium (68.60 mL, 171.49 mmol, 1.25 equiv) dropwise under an argon atmosphere. The resulting mixture was stirred for 30 min at 26 °C. The reaction was then cooled to -78 °C and benzophenone (25 g, 137.20 mmol, 1.00 equiv) was added dropwise over 30 min. The resulting mixture was stirred for additional 2 h at -78 °C then warmed to room temperature and stirred for 5 h. The reaction was quenched by the addition of sat. NH₄Cl (aq.) (500 mL) at 0 °C. The aqueous layer was extracted with EtOAc (3 x 500 mL). The resulting mixture was concentrated under vacuum. The resulting residue was purified by silica gel column chromatography, eluted with PE / EA (1:1) to afford 1,1-diphenyl-3-(triisopropylsilyl)prop-2-yn-1-ol (24.1 g, 40% yield) as an off-white oil. ¹H NMR (300 MHz, DMSO-d6) δ 7.62 - 7.52 (m, 4H), 7.36 - 7.25 (m, 4H), 7.30 - 7.19 (m, 1H), 7.24 - 7.15 (m, 1H), 6.77 (s, 1H), 4.03 (q, J = 7.1 Hz, 1H), 1.99 (s, 1H), 1.17 (t, J = 7.1 Hz, 1H), 1.08 (s, 18H), 1.06 (d, J = 11.2 Hz, 4H).

### Step 2

To a 500 mL 3-necked round-bottom flask was added RhCl(cod)₂ (5.49 g, 7.65 mmol, 0.025 equiv), Cs₂CO₃ (9.96 g, 30.58 mmol, 0.1 equiv), and toluene (200 mL) and the solution cooled to 0 °C. The reaction was then placed under a nitrogen atmosphere and the resulting mixture was stirred for 1 h at 50 °C. 1,1-diphenyl-3-(triisopropylsilyl)prop-2-yn-1-ol (30 g, 82.281 mmol, 1.00 equiv) was added followed by 5,6-dihydropyran-2-one (8.07 g, 82.281 mmol, 1.00 equiv). The resulting mixture was stirred for 36 h at 80 °C. The reaction was cooled to 0 °C and quenched by the addition of Water (500 mL). The aqueous layer was extracted with EtOAc (3 x 500 mL). The resulting mixture was concentrated under reduced pressure. The residue was purified by silica gel column chromatography, eluted with PE / EA (2:1) to afford 4-((triisopropylsilyl)ethynyl)tetrahydro-2H-pyran-2-one (20 g,86% yield) as an off-white oil. LCMS (ESI): m/z [M+H] calc'd for C₁₆H₂₈O₂Si 280.2; found 281.1

### Step 3

A solution of 4-((triisopropylsilyl)ethynyl)tetrahydro-2H-pyran-2-one (20 g, 71.30 mmol, 1 equiv) and DIEA (27.65 g, 213.92 mmol, 3 equiv) in MeOH (200 mL) was stirred for 2 h at 30 °C under an argon atmosphere. The reaction was quenched by the addition of sat. NH₄Cl (aq.) (300 mL) at 0 °C. The aqueous layer was extracted with EtOAc (3 x 300 mL). The resulting mixture was concentrated under vacuum. The crude product (17.5 g, crude) was used in the next step directly without further purification. LCMS (ESI): m/z [M+H] calc'd for C₁₇H₃₂O₃Si 312.2; found 313.1.

### Step 4

To a 500 mL 3-necked round-bottom flask was added methyl 3-(2-hydroxyethyl)-5-(triisopropylsilyl)pent-4-ynoate (17.5 g, 55.99 mmol, 1 equiv) and DCM (200 mL) at 0 °C. Next, TEA (17.00 g, 167.988 mmol, 3 equiv) and TsCl (16.01 g, 83.994 mmol, 1.5 equiv) were added sequentially. The resulting mixture was stirred for 2 h at 25 °C under argon atmosphere. The reaction was quenched by the addition of Water (150 mL) at 0 °C and the aqueous layer was extracted with EtOAc (3 x 150 mL). The resulting mixture was concentrated under reduced pressure. The residue was purified by silica gel column chromatography, eluted with PE / EA (3:1) to afford methyl 3-(2-(tosyloxy)ethyl)-5-(triisopropylsilyl)pent-4-ynoate (14.2 g, 54% yield) as an off-white oil. LCMS (ESI): m/z [M+Na] calc'd for C₂₄H₃₈O₅NaSSi 488.2; found 489.4.

### Step 5

To a stirred solution of methyl 3-(2-(tosyloxy)ethyl)-5-(triisopropylsilyl)pent-4-ynoate (14.2 g, 30.42 mmol, 1 equiv) in THF (160 mL) at -78 °C was added LDA (18.26 mL, 45.64 mmol, 1.5 equiv) dropwise under argon atmosphere. The resulting mixture was stirred for 1 h then DBAD (10.51 g, 45.639 mmol, 1.5 equiv) was added dropwise over 5 min. The resulting mixture was stirred for an additional 1 h then quenched by the addition of sat. NH₄Cl (aq.) (200 mL). The aqueous layer was extracted with EtOAc (3 x 200 mL). The resulting mixture was concentrated under reduced pressure. The residue was purified by Prep-TLC (PE / EA 1:1) to afford di-*tert*-butyl1-(1-methoxy-1-oxo-3-(2-(tosyloxy)ethyl)-5-(triisopropylsilyl)pent-4-yn- 2-yl)hydrazine-1,2-dicarboxylate (13.1 g, 61 % yield) as a light yellow oil. LCMS (ESI): m/z [M+H] calc'd for C₃₄H₅₆N₂O₉SSi 696.3; found 697.3.

### Step 6

A solution of di-tert-butyl1-(1-methoxy-1-oxo-3-(2-(tosyloxy)ethyl)-5-(triisopropylsilyl)pent-4-yn- 2-yl)hydrazine-1,2-dicarboxylate (13.1 g, 18.94 mmol, 1 equiv) and Cs₂CO₃ (30.85 g, 94.70 mmol, 5 equiv) in DMF (2000 mL) was stirred for 2 h at 50 °C under an argon atmosphere. The reaction was quenched by the addition of sat. NH₄Cl (aq.) (500 mL) at 0 °C and the aqueous layer extracted with EtOAc (3 x 500 mL). The combined organic layers were washed with brine (3 x 900 mL) and dried over anhydrous Na₂SO₄. After filtration, the filtrate was concentrated under reduced pressure. The residue was purified by silica gel column chromatography, eluted with PE / EA (1:1) to afford 1,2-di-*tert-*butyl 3-methyl 4-((triisopropylsilyl)ethynyl)tetrahydropyridazine-1,2,3-tricarboxylate (6.6 g, 56% yield) as a light yellow oil. LCMS (ESI): m/z [M+Na] calc'd for C₂₇H₄₈N₂O₆NaSi 546.3; found 547.3.

### Step 7

A solution of 1,2-di-*tert*-butyl 3-methyl 4-((triisopropylsilyl)ethynyl)tetrahydropyridazine-1,2,3-tricarboxylate (5.6 g, 10.671 mmol, 1 equiv) and TFA (30 mL) in DCM (60 mL) was stirred for 16 h at room temperature. The resulting mixture was washed with Brine (3 x 100 mL) then concentrated under reduced pressure. The crude product (3.6 g, crude) was used in the next step directly without further purification. LCMS (ESI): m/z [M+H] calc'd for C₁₇H₃₂N₂O₂Si 324.2; found 325.2.

### Analog Example 5. ethyl 4-ethoxyhexahydropyridazine-3-carboxylate

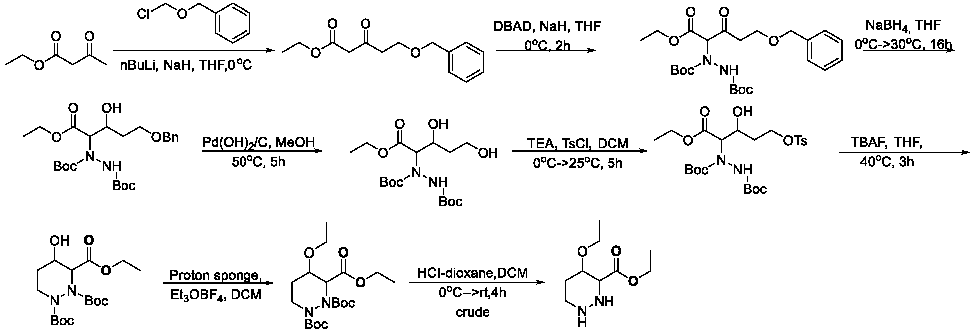

### Step 1

To a 1 L flask was added methyl acetoacetate (20 g, 172.24 mmol, 1 equiv), THF (200 mL) and NaH (7.58 g, 189.51 mmol, 1.10 equiv, 60%) at 0°C. The resulting mixture was stirred for 10min at 0°C under argon atmosphere. To the above mixture was added n-BuLi (75.79 mL, 189.46 mmol, 1.1 equiv) dropwise at 0°C. The resulting mixture was stirred for an additional 10 min at 0°C. To the above mixture was added [chloromethoxy)methyl]benzene (21.58 g, 137.79 mmol, 0.80 equiv) dropwise at 0 °C. The resulting mixture was stirred for an additional 10 min then quenched with sat. NH₄Cl (aq.). The resulting mixture was extracted with EtOAc (3 x 200 mL) and the combined organic layers dried over anhydrous Na₂SO₄. After filtration, the filtrate was concentrated under reduced pressure. The residue was purified by silica gel column chromatography, eluted with PE / EA (5:1) to afford ethyl 5-(benzyloxy)-3-oxopentanoate (20 g, 46.39% yield) as a yellow oil. LCMS (ESI): m/z [M+H] calc'd for C₁₄H₁₈O₄ 250.1; found 251.2.

### Step 2

To a 100 mL round-bottom flask was added ethyl 5-(benzyloxy)-3-oxopentanoate (9 g, 35.95 mmol, 1 equiv), THF (90 mL) and NaH (2.16 g, 53.93 mmol, 1.5 equiv, 60%) at 0 °C. The resulting mixture was stirred for 1h at 0°C under argon atmosphere. To the above mixture was added DBAD (16.56 g, 71.91 mmol, 2.0 equiv) dropwise and the resulting mixture was stirred for additional 1h at room temperature. The reaction was quenched with sat. NH₄Cl (aq.) then extracted with EtOAc (3 x 500mL). The combined organic layers were dried over anhydrous Na₂SO₄. After filtration, the filtrate was concentrated under reduced pressure and the resulting residue was purified by silica gel column chromatography, eluted with PE / EA (7:1) to afford di-*tert*-butyl 1-(5-(benzyloxy)-1-ethoxy-1,3-dioxopentan-2-yl)hydrazine-1,2-dicarboxylate (10 g, 57.87% yield) as a yellow oil. LCMS (ESI): m/z [M+Na] calc'd for C₂₄H₃₆N₂O₈Na 502.2; found 503.2.

### Step 3

To a 250mL 3-necked round-bottom flask was added ethyl di-*tert*-butyl 1-(5-(benzyloxy)-1-ethoxy-1,3-dioxopentan -2-yl)hydrazine-1,2-dicarboxylate (10 g, 20.81 mmol, 1 equiv), THF (100 mL) and NaBH₄ (2.36 g, 62.43 mmol, 3.0 equiv) at 0 °C. The resulting mixture was stirred overnight at 30 °C. The reaction was then quenched with sat. NH₄Cl (aq.). The aqueous layer was extracted with EtOAc (3 x 300 mL), and the combined organics dried over anhydrous Na₂SO₄. After filtration, the filtrate was concentrated under reduced pressure. The residue was purified by silica gel column chromatography, eluted with PE / EA (4:1) to afford di-tert-butyl 1-(5-(benzyloxy)-1-ethoxy-3-hydroxy-1-oxopentan-2-yl)hydrazine-1,2-dicarboxylate (7 g, 69.71% yield) as a yellow oil. LCMS (ESI): m/z [M+Na] calc'd for C₂₄H₃₆N₂O₈Na 504.2; found 505.2.

### Step 4

To a 250 mL round-bottom flask was added di-*tert*-butyl 1-(5-(benzyloxy)-1-ethoxy-3-hydroxy-1-oxopentan-2-yl) hydrazine-1,2-dicarboxylate (7 g, 14.5 mmol, 1 equiv) and Pd(OH)₂/C (2.55 g, 18.13 mmol, 1.25 equiv) at room temperature. The resulting mixture was stirred for 5h at 50 °C under H₂ atmosphere. The resulting mixture was filtered and the filter cake was washed with EtOAc (3 x 100 mL). The filtrate was concentrated under reduced pressure. This resulted in di-tert-butyl 1-(1-ethoxy-3,5-dihydroxy-1-oxopentan-2-yl)hydrazine-1,2-dicarboxylate (5 g, 87.83% yield) as a yellow oil. LCMS (ESI): m/z [M+Na] calc'd for C₁₇H₃₂N₂O₈Na 415.2; found 415.2.

### Step 5

To a 250mL round-bottom flask was added di-*tert*-butyl 1-(1-ethoxy-3,5-dihydroxy-1-oxopentan-2-yl)hydrazine- 1,2-dicarboxylate (5 g, 12.74 mmol, 1 equiv), DCM (50 mL), TEA (3.87 g, 38.22 mmol, 3 equiv) and TsCl (3.64 g, 19.11 mmol, 1.5 equiv) at 0°C. The resulting mixture was stirred for 5h at 25 °C under an argon atmosphere then concentrated under reduced pressure. The resulting mixture was diluted with brine (100 mL) and extracted with EtOAc (4 x 100mL). The combined organic layers were dried over anhydrous Na2SO4. After filtration, the filtrate was concentrated under reduced pressure. The residue was purified by silica gel column chromatography, eluted with PE / EA (2:1) to afford di-*tert*-butyl 1-(1-ethoxy-3-hydroxy-1-oxo-5-(tosyloxy) pentan-2-yl)hydrazine-1,2-dicarboxylate (6 g, 86.15% yield) as a yellow oil. LCMS (ESI): m/z [M+Na] calc'd for C₂₄H₃₈N₂O₁₀NaS 569.2; found 569.3.

### Step 6

Into a 1L round-bottom flask were added di-*tert*-butyl 1-(1-ethoxy-3-hydroxy-1-oxo-5-(tosyloxy) pentan-2-yl)hydrazine-1,2-dicarboxylate (6 g, 10.976 mmol, 1 equiv), THF (600 mL) and TBAF (54.88 mL, 54.880 mmol, 1mol/L, 5 equiv) at 0 °C. The resulting mixture was stirred for 3h at 40 °C under nitrogen atmosphere. Desired product could be detected by LCMS. The resulting mixture was concentrated under reduced pressure. The residue was purified by silica gel column chromatography, eluted with PE / EA (2:1) to afford 1,2-di-*tert*-butyl 3-ethyl 4-hydroxy-1,2- diazinane-1,2,3-tricarboxylate (1.9 g, 60.49% yield) as a yellow oil. LCMS (ESI): m/z [M+H] calc'd for C₁₇H₃₀N₂O₇ 374.2; found 375.3.

### Step 7

To a stirred mixture of 1,2-di-*tert*-butyl 3-ethyl 4-hydroxy-1,2-diazinane-1,2,3-tricarboxylate (2.0 g, 5.34 mmol, 1 equiv) in DCM (20 mL) was added N1,N1,N8,N8-tetramethylnaphthalene-1,8-diamine (4.58 g, 21.36 mmol, 4 equiv) and Et₃OBF₄ (2.37 g, 16.02 mmol, 3 equiv) in portions at 0 °C under an argon atmosphere. The resulting mixture was stirred for 16 h at 20 °C then diluted with water (50 mL). The aqueous layer was extracted with EtOAc (3 x 50 mL). The combined organics were concentrated under reduced pressure and the resulting residue was purified by silica gel column chromatography, eluted with PE / EA (1:1) to afford 1,2-di-*tert*-butyl 3-ethyl 4-ethoxy-1,2-diazinane-1,2,3-tricarboxylate (1.05 g, 43% yield) as a yellow oil. LCMS (ESI): m/z [M+H] calc'd for C₁₉H₃₄N₂O₇ 402.2; found 403.1.

### Step 8

To a stirred solution of 1,2-di-*tert*-butyl 3-ethyl 4-ethoxy-1,2-diazinane-1,2,3-tricarboxylate (1.05 g, 2.61 mmol, 1 equiv) in DCM (10 mL) was added 4M HCl in 1,4-dioxane (5 mL) in portions at 0 °C under an argon atmosphere. The resulting mixture was stirred for 1 h at 20 °C then concentrated under reduced pressure. The crude product (620 mg) was used in the next step directly without further purification. LCMS (ESI): m/z [M+H] calc'd for C₉H₁₈N₂O₃ 202.1; found 203.1.

### Analog Example 6. ethyl (3S,4S)-4-methoxyhexahydropyridazine-3-carboxylate

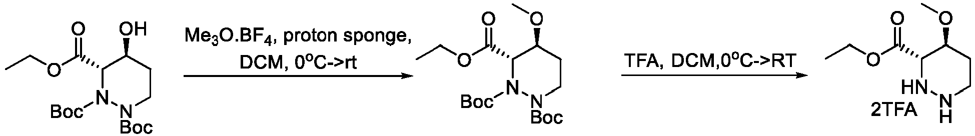

### Step 1

To a stirred solution of 1,2-di-*tert*-butyl 3-ethyl (3S,4S)-4-hydroxytetrahydropyridazine-1,2,3-tricarboxylate (12.5 g, 33.38 mmol, 1 equiv) in DCM (130 mL) was added 1-N,1-N,8-N,8-N-tetramethylnaphthalene-1,8-diamine (28.62 g, 133.53 mmol, 4 equiv) at 0 °C. To the above mixture was added Me₃O.BF₄ (14.81 g, 100.15 mmol, 3 equiv) at 0 °C. The resulting mixture was stirred for an additional 2 h at 25 °C then diluted with water (150 mL). The resulting mixture was extracted with CH₂Cl₂ (2 x 150 mL). The combined organic layers were washed with brine (3 x 150 mL) and dried over anhydrous Na₂SO₄. After filtration, the filtrate was concentrated under reduced pressure. The residue was purified by silica gel column chromatography, eluted with PE / EA (3:1) to afford 1,2-di-tert-butyl 3-ethyl (3S,4S)-4-methoxytetrahydropyridazine-1,2,3-tricarboxylate (6.1 g, 39.9% yield) as a yellow oil. LCMS (ESI): m/z [M+H] calc'd for C₁₈H₃₂N₂O₇ 388.1; found 389.1.

### Step 2

To a stirred solution of 1,2-di-*tert*-butyl 3-ethyl (3S,4S)-4-methoxytetrahydropyridazine-1,2,3-tricarboxylate (1 g, 2.57 mmol, 1 equiv) in DCM (10 mL) was added TFA (3.5 mL, 47.12 mmol, 12.83 equiv) dropwise at 0 °C. The resulting mixture was stirred for 4 h at 20 °C then diluted with toluene (10 mL). The resulting mixture was concentrated under reduced pressure. This resulted in ethyl (3S,4S)-4-methoxyhexahydropyridazine-3-carboxylate TFA salt (1.50 g, crude) as a light yellow oil. LCMS (ESI): m/z [M+H] calc'd for C₈H₁₆N₂O₃ 1881; found 189.1.

### Analog Example 7. Methyl-(4-methylhexahydropyridazine-3-carboxylate

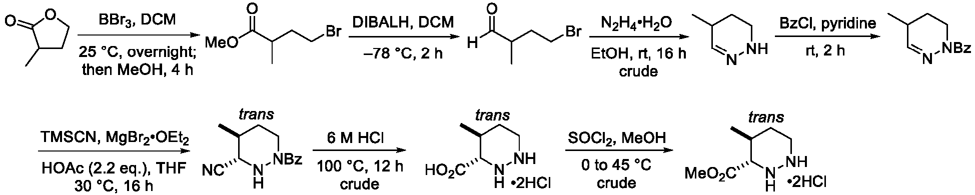

### Step 1

To a stirred solution of 3-methyldihydrofuran-2(3*H*)-one (10 g, 99.88 mmol, 1 equiv) in DCM (50 mL) was added BBr₃ (26.27 g, 104.87 mmol, 1.05 equiv) dropwise at 0 °C under an argon atmosphere. The reaction mixture was allowed to warm up to room temperature and stirred overnight. Then the reaction mixture was cooled to 0 °C and MeOH (20 mL) was added. The reaction mixture was allowed to warm up to room temperature and stirred for 1 h. The resulting mixture was concentrated under reduced pressure. The residue was purified by silica gel column chromatography, eluted with PE / EA (10:1) to afford methyl 4-bromo-2-methylbutanoate (19.4 g, 99% yield) as a colorless oil. ¹H NMR (300 MHz, CDCl3) *δ* 3.70 (s, 3H), 3.43 (t, J = 6.8 Hz, 2H), 2.80 - 2.64 (m, 1H), 2.34 - 2.18 (m, 1H), 2.01- 1.85 (m, 1H), 1.20 (d, J = 7.2 Hz, 3H).

### Step 2

To a stirred solution of methyl 4-bromo-2-methylbutanoate (16.4 g, 84.07 mmol, 1 equiv) in DCM (250 mL) was added DIBAL-H (92.49 mL, 92.48 mmol, 1.1 equiv) dropwise at -78 °C under an argon atmosphere over 10 min. After stirring for 2 h the reaction was quenched by the addition of sat. NH₄Cl (aq.) (200 mL). The resulting mixture was extracted with CH₂Cl₂ (3 x 100 mL). The combined organic layers were washed with brine and dried over anhydrous Na₂SO₄. After filtration, the filtrate was concentrated under reduced pressure to give 4-bromo-2-methylbutanal (15.6 g, 97% yield) as a colorless oil. The crude product was used in the next step directly without further purification. LCMS (ESI): m/z [M+H] calc'd for C₅H₉BrO 376.2; found 377.3.

### Step 3

To a stirred solution of 4-bromo-2-methylbutanal (15 g, 90.89 mmol, 1 equiv) in EtOH (250 mL) was added hydrazine (36.41 g, 908.93 mmol, 10 equiv, 80%) dropwise at 0 °C under argon atmosphere. The reaction mixture was allowed to warm up to room temperature and stirred overnight. The reaction was quenched by the addition of Water (600 mL) at room temperature. The resulting mixture was extracted with EtOAc (3 x 200 mL). The combined organic layers were washed with brine and dried over anhydrous Na₂SO₄. After filtration, the filtrate was concentrated under reduced pressure to give 4-methyl-1,4,5,6-tetrahydropyridazine (9 g, crude) as a colorless oil. The crude product was used in the next step directly without further purification. LCMS (ESI): m/z [M+H] calc'd for C₅H₁₀N₂ 98.1; found 99.1.

### Step 4

To a stirred solution of 4-methyl-1,4,5,6-tetrahydropyridazine (10.7 g, 109.01 mmol, 1 equiv) in pyridine (200 mL) was added benzoyl chloride (18.39 g, 130.82 mmol, 1.2 equiv) dropwise at 0 °C under argon atmosphere. The reaction mixture was allowed to warm up to room temperature and stirred for 2 h. The resulting mixture was concentrated under reduced pressure and the residue was purified by silica gel column chromatography, eluted with PE / EA (1:1) to afford (4-methyl-5,6-dihydropyridazin-1(4*H*)-yl)(phenyl)methanone (10.1 g, 45% yield) as an off-white solid. LCMS (ESI): m/z [M+H] calc'd for C₁₂H₁₄N₂O 202.1; found 203.1.

### Step 5

To a stirred solution of 1-benzoyl-4-methyl-5,6-dihydro-4H-pyridazine (8.4 g, 41.53 mmol, 1 equiv) and MgBr₂·OEt₂ (10.72 g, 41.53 mmol, 1 equiv) in THF (100 mL) was added TMSCN (20.60 g, 207.65 mmol, 5 equiv) and HOAc (5.24 mL, 91.37 mmol, 2.2 equiv) dropwise at room temperature under argon atmosphere. The reaction mixture was stirred overnight then quenched by the addition of sat. NH₄Cl (aq.) (100 mL). The resulting mixture was extracted with EtOAc (3 x 50 mL). The combined organic layers were washed with brine and dried over anhydrous Na₂SO₄. After filtration, the filtrate was concentrated under reduced pressure. The residue was purified by silica gel column chromatography, eluted with PE / EA (1:1) to afford (*trans*)-1-benzoyl-4-methylhexahydropyridazine-3-carbonitrile (3.86 g, 55% yield) as an off-white solid and (*cis*)-1-benzoyl-4-methylhexahydropyridazine-3-carbonitrile (1.29 g, 18% yield) as an off-white solid. LCMS (ESI): m/z [M+H] calc'd for C₁₃H₁₅N₃O 229.1; found 230.2.

### Step 6

To a round bottom flask charged with (*trans*)-1-benzoyl-4-methylhexahydropyridazine-3-carbonitrile (5.1 g, 22.24 mmol, 1 equiv) was added 6 M HCl (150 mL) at room temperature under argon atmosphere. The reaction mixture was allowed to warm up to 100 °C and stirred for 12 h. The resulting mixture was washed with EA (3 x 50 mL) and the aqueous phase was concentrated under reduced pressure to give (*trans*)-4-methylhexahydropyridazine-3-carboxylic acid (7.7 g, crude) as a brown solid. The crude product was used in the next step directly without further purification. LCMS (ESI): m/z [M+H] calc'd for C₆H₁₂N₂O₂ 144.1; found 145.1.

### Step 7

To a stirred solution of (*trans*)-4-methylhexahydropyridazine-3-carboxylic acid (7.7 g, 53.41 mmol, 1 equiv) in MeOH (200 mL) was added SOCl₂ (30 mL, 413.55 mmol, 7.74 equiv) dropwise at 0 °C under argon atmosphere. The reaction mixture was heated to 45 °C and stirred for 12 h. The resulting mixture was concentrated under reduced pressure. The crude product was used in the next step directly without further purification. LCMS (ESI): m/z [M+H] calc'd for C₇H₁₄N₂O₂ 158.1; found 159.1.

### Analog Example 8. methyl 6-methylhexahydropyridazine-3-carboxylate

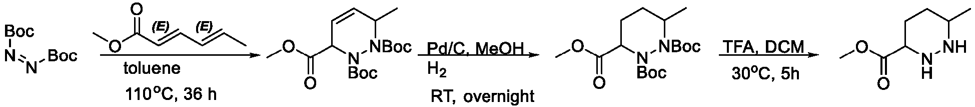

### Step 1

To a 250 mL round-bottom flask was added (*Z*)-N-[(*tert*-butoxycarbonyl) imino] (*tert*-butoxy) formamide (10 g, 43.42 mmol, 1 equiv), toluene (100 mL) and methyl (2*E*,4*E*)-hexa-2,4-dienoate (5.48 g, 43.43 mmol, 1.00 equiv) at room temperature. The resulting mixture was stirred for 36 h at 100°C. The resulting mixture was concentrated under reduced pressure and the residue was purified by reverse flash chromatography with the following conditions: column, C₁₈ silica gel; mobile phase, ACN in Water (0.1% FA), 60% to 100% gradient in 30 min; detector, UV 210 nm.to afford 1,2-di-*tert*-butyl 3-methyl 6-methyl-3,6-dihydropyridazine-1,2,3-tricarboxylate (12 g, 72% yield) as a colorless oil. LCMS (ESI): m/z [M+H] calc'd for C₁₇H₂₈N₂O₆ 356.2; found 379.2 [M+Na]⁺.

### Step 2

To a 250 mL round-bottom flask was added 1,2-di-*tert*-butyl 3-methyl 6-methyl-3,6-dihydropyridazine-1,2,3-tricarboxylate (12 g, 33.66 mmol, 1 equiv), MeOH (100 mL) and Pd/C (3.58 g, 33.66 mmol, 1 equiv) at room temperature. The resulting reaction was stirred overnight at 30 °C under hydrogen atmosphere. The mixture was filtered, and the filter cake was washed with MeOH (3 x 20 mL). The filtrate was concentrated under reduced pressure. to afford 1,2-di-*tert*-butyl 3-methyl 6-methyl-1,2-diazinane-1,2,3-tricarboxylate (12 g, crude) as a colorless oil. LCMS (ESI): m/z [M+H] calc'd for C₁₇H₃₀N₂O₆ 358.2; found 381.2 [M+Na]⁺.

### Step 3

To a 250 mL round-bottom flask was added 1,2-di-*tert*-butyl 3-methyl 6-methyl-1,2-diazinane-1,2,3-tricarboxylate (12 g, 33.47 mmol, 1 equiv), DCM (50 mL) and TFA (15 mL) at 0°C. The resulting mixture was stirred for 5 h at 30 °C then concentrated under reduced pressure to afford methyl 6-methyl-1,2-diazinane-3-carboxylate (10 g, crude) as a yellow oil. The crude product was used in the next step directly without further purification. LCMS (ESI): m/z [M+H] calc'd for C₇H₁₄N₂O₂ 158.1; found 159.2.

### Analog Example 9. ethyl 2-cyclopropyl-2-(2-methylhydrazineyl)acetate

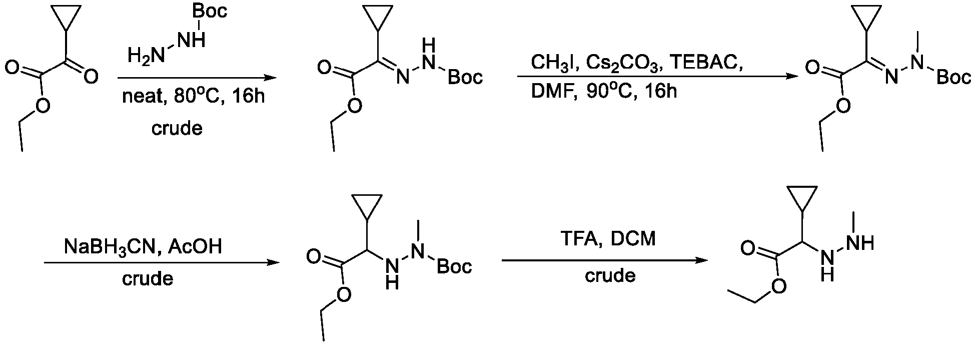

### Step 1

A mixture of ethyl 2-cyclopropyl-2-oxoacetate (2.84 g, 20 mmol, 1 equiv) and *tert-*butoxycarbohydrazide (2.64 g, 20 mmol, 1 equiv) was stirred for 16h at 80 °C. This resulted in *tert-*butyl (*E*)-2-(1-cyclopropyl-2-ethoxy-2-oxoehylidene)hydrazine-1-carboxylate (5.4 g, crude) as a yellow oil. The crude product was used in the next step directly without further purification. LCMS (ESI): m/z [M+H] calc'd for C₁₂H₂₀N₂O₄ 256.1; found 257.1.

### Step 2

To a stirred solution of *tert*-butyl (E)-2-(1-cyclopropyl-2-ethoxy-2-oxoethylidene)hydrazine-1-carboxylate (2.56 g, 10 mmol, 1 equiv), Cs₂CO₃ (6.5 g, 20 mmol, 2 equiv) and benzyltriethylazanium chloride (2.28 g, 10 mmol, 1 equiv) in DMF (20 mL) was added methyl iodide (4.26 g, 30 mmol, 3 equiv) dropwise at 0 °C. The reaction was stirred overnight at 50 °C then diluted with water (100 mL). The resulting mixture was extracted with EtOAc (3 x 100 mL). The combined organic layers were washed with brine (3 x 100 mL), dried over anhydrous Na₂SO₄. After filtration, the filtrate was concentrated under reduced pressure. The crude product was purified by reverse phase flash with the following conditions (Column: C18 spherical 20-35um 100A 120 g; Mobile Phase A: Water (0.1%NH₄HCO₃), Mobile Phase B: ACN; Flow rate: 75 mL/min; Gradient: 30% B to 70% B in 20 min.) to afford *tert-*butyl (*E*)-2-(1-cyclopropyl-2-ethoxy-2-oxoethylidene)-1-methylhydrazine-1-carboxylate (1.63 g, 60% yield) as a yellow solid. LCMS (ESI): m/z [M+H] calc'd for C₁₃H₂₂N₂O₄ 270.1; found 271.2.

### Step 3

To a stirred solution of *tert*-butyl (*E*)-2-(1-cyclopropyl-2-ethoxy-2-oxoethylidene)-1-methylhydrazine-1-carboxylate (1.35 g, 5.0 mmol, 1 equiv) in AcOH (13 mL) was added NaBH₃CN (630mg, 10 mmol, 2 equiv) in portions at 0 °C under an argon atmosphere. The resulting mixture was stirred for 2 h at room temperature then basified to pH 8 with saturated NaHCO₃ (aq.). The resulting mixture was extracted with EtOAc (3 x 100 mL). The combined organic layers were dried over anhydrous Na₂SO₄. After filtration, the filtrate was concentrated under reduced pressure. The crude product was used in the next step directly without further purification. This resulted in tert-butyl 2-(1-cyclopropyl-2-ethoxy-2-oxoethyl)-1-methylhydrazine-1-carboxylate (1.3 g, crude) as yellow oil. LCMS (ESI): m/z [M+H] calc'd for C₁₃H₂₄N₂O₄ 272.1; found 273.2.

### Step 4

To a stirred solution of *tert*-butyl 2-(1-cyclopropyl-2-ethoxy-2-oxoethyl)-1-methylhydrazine-1-carboxylate (1.3 g, crude) in DCM (39.00 mL) was added TFA (13.00 mL) dropwise at 0 °C under an argon atmosphere. The resulting mixture was stirred for 1 h at room temperature then concentrated under reduced pressure. This resulted in ethyl 2-cyclopropyl-2-(2-methylhydrazineyl)acetate 2,2,2-trifluoroacetate (1100 mg, crude) as a yellow oil. The crude product was used in the next step directly without further purification. LCMS (ESI): m/z [M+H] calc'd for C₈H₁₆N₂O₄ 172.1; found 173.2.

### Analog Example 10. ethyl 2-cyclobutyl-2-(2-methylhydrazineyl)acetate

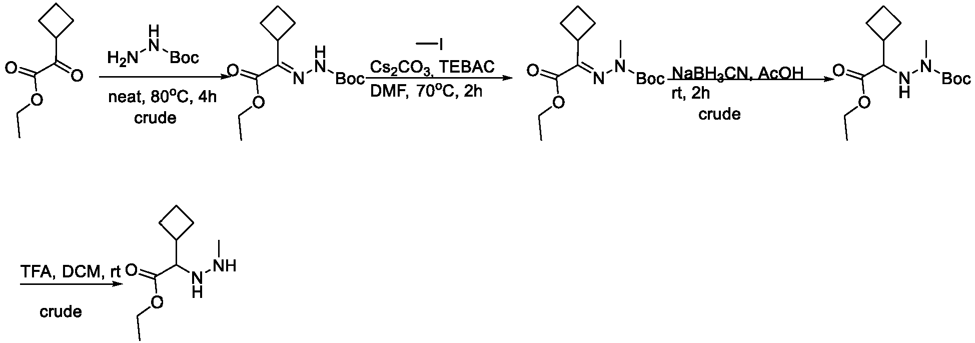

### Step 1

A mixture of ethyl 2-cyclobutyl-2-oxoacetate (2 g, 12.80 mmol, 1 equiv) and *tert-*butoxycarbohydrazide (1.69 g, 12.80 mmol, 1 equiv) was stirred overnight at 80 °C. This resulted in ethyl (2*E*)-2-{[(*tert*-butoxycarbonyl)amino]imino}-2-cyclobutylacetate (3.6 g, crude) as a yellow solid. The crude product was used in the next step directly without further purification. LCMS (ESI): m/z [M+H] calc'd for C₁₃H₂₂N₂O₄ 270.1; found 271.2.

### Step 2

To a stirred solution of ethyl (2*E*)-2-{[(*tert*-butoxycarbonyl)amino]imino}-2-cyclobutylacetate (2 g, 7.40 mmol, 1 equiv), Cs₂CO₃ (4.82 g, 14.80 mmol, 2 equiv) and benzyltriethylazanium chloride (1.69 g, 7.40mmol, 1 equiv) in DMF (20 mL) was added methyl iodide (3.15 g, 22.19 mmol, 3 equiv) dropwise at 0 °C. The reaction was stirred overnight at 50 °C then diluted with water (30 mL). The resulting mixture was extracted with EtOAc (3 x 50 mL). The combined organic layers were washed with brine (3 x 100 mL), dried over anhydrous Na₂SO₄. After filtration, the filtrate was concentrated under reduced pressure. The crude product was purified by reverse phase flash with the following conditions (Column: C18 spherical 20-35um 100A 120 g; Mobile Phase A: Water(0.1%NH₄HCO₃), Mobile Phase B: ACN; Flow rate: 75 mL/min; Gradient: 30% B to 70% B in 20 min.) to afford ethyl (2*E*)-2-{[(*tert*-butoxycarbonyl)(methyl)amino]imino}-2-cyclobutylacetate (1.3 g, 55.61% yield) as a yellow solid. LCMS (ESI): m/z [M+H] calc'd for C₁₄H₂₄N₂O₄ 284.2; found 285.2.

### Step 3

To a stirred solution of ethyl (2*E*)-2-{[(*tert*-butoxycarbonyl)(methyl)amino]imino}-2-cyclobutylacetate (1.3 g, 4.57 mmol, 1 equiv) in AcOH (13 mL) was added NaBH₃CN (574.58 mg, 9.14 mmol, 2 equiv) in portions at 0 °C under argon atmosphere. The reaction was stirred for 2 h at room temperature then basified to pH 8 with saturated NaHCO₃ (aq.). The resulting mixture was extracted with EtOAc (3 x 100 mL). The combined organic layers were dried over anhydrous Na₂SO₄. After filtration, the filtrate was concentrated under reduced pressure. This resulted in ethyl 2-{[(*tert-*butoxycarbonyl)(methyl)amino]amino}-2-cyclobutylacetate (1.2 g, crude) as a yellow oil. The crude product was used in the next step directly without further purification. LCMS (ESI): m/z [M+H] calc'd for C₁₄H₂₆N₂O₄ 286.2; found 287.2.

### Step 4

To a stirred solution of ethyl 2-{[(*tert*-butoxycarbonyl)(methyl)amino]amino}-2-cyclobutylacetate (1.3 g, 4.54 mmol, 1 equiv) in DCM (39.00 mL) was added TFA (13.00 mL, 175.02 mmol, 38.55 equiv) dropwise at 0 °C under argon atmosphere. The resulting mixture was stirred for 1 h at room temperature then concentrated under reduced pressure. This resulted in ethyl 2-cyclobutyl-2-(2-methylhydrazin-1-yl)acetate (900 mg, crude) as a yellow oil. The crude product was used in the next step directly without further purification. LCMS (ESI): m/z [M+H] calc'd for C₉H₁₈N₂O₂ 186.2; found 187.2.

### Analog Example 11. 2,3-di-tert-butyl 1-methyl 2,3-diazabicyclo[2.2.1]heptane-1,2,3-tricarboxylate

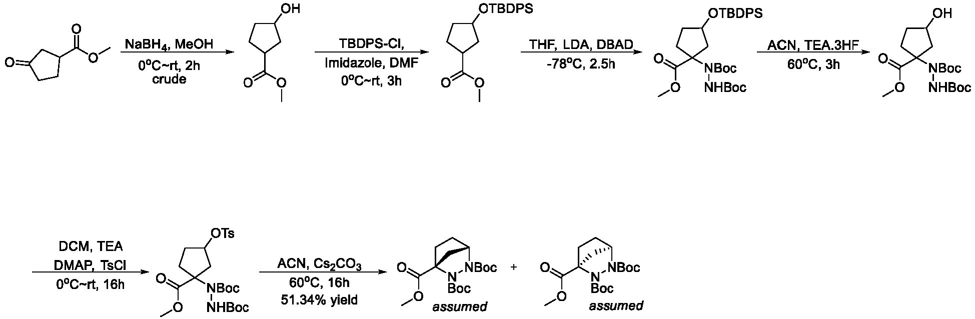

### Step 1

To a 500 mL round-bottom flask was added methyl 3-oxocyclopentane-1-carboxylate (25 g, 175.87 mmol, 1 equiv), MeOH (250 mL) and NaBH₄ (6.65 g, 175.87 mmol, 1 equiv) at 0 °C. The resulting mixture was stirred for 2h at room temperature then quenched by the addition of sat. NH₄Cl (aq.) (100mL) at 0 °C. The aqueous layer was extracted with EtOAc (2 x 200 mL). The combined organics were concentrated to afford methyl 3-hydroxycyclopentane-1-carboxylate (22 g, crude) as a light yellow oil. LCMS (ESI): m/z [M+H] calc'd for C₇H₁₂O₃ 144.1; found 145.2.

### Step 2

To a 500 mL round-bottom flask was added methyl 3-hydroxycyclopentane-1-carboxylate (23 g, 159.53 mmol, 1 equiv), DMF (230 mL) and Imidazole (54.30 g, 797.67 mmol, 5 equiv), TBDPS-Cl (65.77 g, 239.30 mmol, 1.5 equiv) at 0 °C. The resulting mixture was stirred for 3 h at room temperature then quenched by the addition of sat. NH₄Cl (aq.) at 0 °C. The resulting mixture was washed with EtOAc (2 x 2 L). The combined organic layers were washed with brine (3 x 500 mL), dried over anhydrous Na₂SO₄. After filtration, the filtrate was concentrated under reduced pressure. The residue was purified by silica gel column chromatography, eluted with PE / EA (3:1) to afford methyl 3-((*tert*-butyldiphenylsilyl)oxy)cyclopentane-1-carboxylate (46 g, 75.37% yield) as a light yellow oil. LCMS (ESI): m/z [M+H] calc'd for C₂₃H₃₀O₃Si 382.2; found 383.1.

### Step 3

To a mixture of methyl 3-((*tert*-butyldiphenylsilyl)oxy)cyclopentane-1-carboxylate (45 g, 117.62 mmol, 1.00 equiv) in THF (1 L) was added LDA (82.34 mL, 164.67 mmol, 1.4 equiv, 2M in THF) dropwise at -78 °C. The resulting mixture was stirred for 1h at -78 °C. To the above mixture was added DBAD (37.92 g, 164.67 mmol, 1.4 equiv) dropwise over 10 min at -78 °C. The resulting mixture was stirred for additional 1 h then quenched by the addition of sat. NH₄Cl (aq.) at 0 °C. The resulting mixture was extracted with EtOAc (3 x 500 mL). The combined organic layers were concentrated under reduced pressure. The residue was purified by silica gel column chromatography, eluted with PE / EA (1:1) to afford methyl 1- di-tert-butyl 1-(3-((*tert*-butyldiphenylsilyl)oxy)-1-(methoxycarbonyl)cyclopentyl)hydrazine-1,2-dicarboxylate (60 g, 83.24% yield) as a light yellow oil. LCMS (ESI): m/z [M-H] calc'd for C₃₃H₄₈N₂O₇Si 612.3; found 611.2.

### Step 4

To a stirred solution of methyl 1- di-*tert*-butyl 1-(3-((*tert*-butyldiphenylsilyl)oxy)-1-(methoxycarbonyl)cyclopentyl)hydrazine-1,2-dicarboxylate (60 g, 97.91 mmol, 1 equiv) in THF (600 mL) was added TEA•3HF (500 mL, 3684.59 mmol, 37.63 equiv) dropwise at room temperature. The resulting mixture was stirred for 3 h at 60°C then concentrated under reduced pressure. The residue was purified by silica gel column chromatography, eluted with PE / EA (1:1) to afford di-*tert*-butyl 1-(3-hydroxy-1-(methoxycarbonyl)cyclopentyl)hydrazine-1,2-dicarboxylate (12 g, 32.73% yield) as a light yellow oil. LCMS (ESI): m/z [M+H] calc'd for C₁₇H₃₀N₂O₇ 374.2; found 319.1 [M+2Na]⁺.

### Step 5

To a stirred solution of di-*tert*-butyl 1-(3-hydroxy-1-(methoxycarbonyl)cyclopentyl)hydrazine-1,2-dicarboxylate (12 g, 32.05 mmol, 1 equiv) in DCM (120 mL) was added TEA (9 mL, 64.75 mmol, 2.02 equiv), DMAP (0.8 g, 6.55 mmol, 0.20 equiv) and TsCl (9.18 g, 48.154 mmol, 1.50 equiv) in portions at 0 °C. The resulting mixture was stirred for 16 h at room temperature then concentrated under reduced pressure. The residue was purified by silica gel column chromatography, eluted with PE / EA (1:1) to afford di-*tert*-butyl 1-(1-(methoxycarbonyl)-3-(tosyloxy)cyclopentyl)hydrazine-1,2-dicarboxylate (13 g, 76.74% yield) as a light yellow oil. LCMS (ESI): m/z [M+H] calc'd for C₂₄H₃₆N₂O₉S 528.2; found 529.3.

### Step 6

To a 2 L round-bottom flask was added di-*tert*-butyl 1-(1-(methoxycarbonyl)-3-(tosyloxy)cyclopentyl)hydrazine-1,2-dicarboxylate (13 g, 24.59 mmol, 1 equiv), ACN (1000 mL) and Cs₂CO₃ (40 g, 122.76 mmol, 4.99 equiv) at room temperature. The resulting mixture was stirred for 16 h at 60 °C. The resulting mixture was filtered; the filter cake was washed with DCM (3 x 300 mL). The filtrate was concentrated under reduced pressure. The residue was purified by silica gel column chromatography, eluted with PE / EA (1:1) to afford methyl 2,3-diazabicyclo[2.2.1]heptane-1-carboxylate (4.5 g, 51.34% yield) as a light yellow oil. The diastereomers were purified by reversed-phase flash chromatography with the following conditions: Column: N--CHIRALPAK IA-3 (Lot No.IA3SCK-TJ008), 3*100mm, 3.0um; Mobile Phase B: EtOH (20mMNH₃); Flow rate: 2 mL/min; Gradient: isocratic 5% B; Wave Length: 220 nm to afford 2,3-di-*tert*-butyl 1-methyl (1R,4S)-2,3-diazabicyclo[2.2.1]heptane-1,2,3-tricarboxylate (1 g, 11.41%, RT1: 0.881 min in SFC) and 2,3-di-*tert-*butyl 1-methyl (1S,4R)-2,3-diazabicyclo[2.2.1]heptane-1,2,3-tricarboxylate (720 mg, RT2: 1.436 min in SFC) as a light yellow oil. LCMS (ESI): m/z [M+H] calc'd for C₁₇H₂₈N₂O₆ 356.2; found 357.2.

### Analog Example 12. methyl 3,4-diazabicyclo[4.1.0]heptane-2-carboxylate

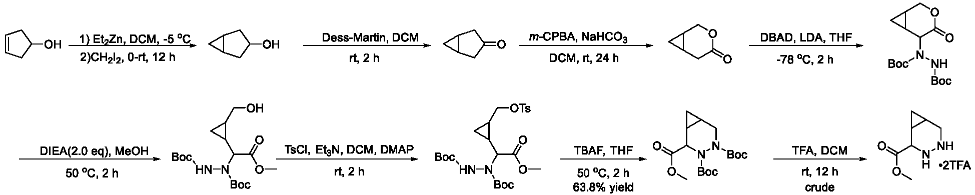

### Step 1

To a solution of cyclopent-3-en-1-ol (30 g, 356.64 mmol, 1 equiv) and diethylzinc (92.52 mL, 748.94 mmol, 2.1 equiv) in DCM (200 mL) under N₂ atmosphere at -5 °C was added CH₂I₂ (200.60 g, 748.95 mmol, 2.1 equiv) over 60 min using a syringe pump. The reaction was slowly warmed to rt and stirred overnight, at which time the mixture was opened to air and slowly quenched by the addition of dilute HCl (aq, 150 mL). The mixture was diluted with DCM (200 mL) and filtered. The organic layer was separated and washed with H₂O (100 mL) and brine (100 mL). The combined organics were dried over Na₂SO₄, filtered and concentrated to an oil which was purified by column chromatography (0-20% EtOAc/hexanes, silica) to give endo-bicyclo [3.1.0] hexan-3-ol (21 g, 60.0 % yield). ¹H NMR (400 MHz, CDCl₃) δ 4.37 (t, *J* = 6.5 Hz, 1H), 2.14 - 2.07 (m, 2H), 1.73 (d, *J* = 14.2 Hz, 1H), 1.39 (br s, 1H), 1.31 - 1.25 (m, 2H), 0.56 - 0.45 (m, 2H).

### Step 2

To a solution of bicyclo [3.1.0] hexan-3-ol (21 g, 213.97 mmol, 1 equiv) in DCM (800 mL) was added DMP (181.51 g, 427.94 mmol, 2 equiv) portion wise at room temperature, and the resulting mixture was stirred for 2 h. After completion of the reaction as monitored by TLC, the reaction was quenched with (1:1) NaHCO₃:Na₂S₂O₃ (400 mL), followed by the addition of 100 mL of water. The resulting mixture was stirred for approximately 1 h to obtain two clear layers, which were separated. The aqueous layer was extracted with DCM (2 x 500 mL). The combined organic phases were dried over anhydrous Na₂SO₄ and filtered. The crude product bicyclo [3.1.0] hexan-3-one (18 g, 87.5% yield) was used in the next step directly without further purification. ¹H-NMR (400 MHz, CDCl₃) δ 2.64-2.47 (m, 2H), 2.15 (d, *J* = 20.2 Hz, 2H), 1.60-1.44 (m, 2H), 0.99-0.80 (m, 1 H), -0.14-0.02 (m, 1 H).

### Step 3

A solution of bicyclo [3.1.0] hexan-3-one (15 g, 156.04 mmol, 1 equiv), *m*-CPBA (40.39 g, 234.06 mmol, 1.5 equiv) and NaHCO₃ (19.66 g, 234.06 mmol, 1.5 equiv) in DCM (500 mL) was stirred at room temperature for 24 h. After completion of the reaction as monitored by TLC, the resulting solution was quenched with saturated Na₂SO₃ solution, then extracted with DCM. The combined organics were dried over Na₂SO₄ and concentrated. The residue was purified by silica gel column chromatography, eluted with PE / EA (5:1) to afford 3-oxabicyclo [4.1.0] heptan-4-one (12 g, 68.5% yield) as a colorless oil. ¹H NMR (400 MHz, CDCl₃) δ 4.58 (dd, *J* = 11.5, 2.4 Hz, 1H), 4.36 (ddd, *J* = 11.5, 2.6, 1.0 Hz, 1H), 2.88 - 2.66 (m, 2H), 1.42 - 1.24 (m, 2H), 0.83 - 0.77 (m, 1H), 0.61- 0.54 (m, 1H).

### Step 4

A solution of 3-oxabicyclo [4.1.0] heptan-4-one (12 g, 107.02 mmol, 1 equiv) in THF (100 mL) was treated with LDA (133.78 mL, 267.55 mmol, 2.5 equiv) for 30 min at -78 °C under nitrogen atmosphere followed by the addition of DBAD (36.96 g, 160.53 mmol, 1.5 equiv) in THF (200 mL) dropwise at -78 °C. The resulting mixture was stirred for an additional 2 h at -78 °C. After completion, the reaction was quenched with sat. NH₄Cl (aq.) and then extracted with EtOAc (3 x 100 mL). The combined organic layers were washed with brine (3 x 100 mL), dried over anhydrous Na₂SO₄. After filtration, the filtrate was concentrated under reduced pressure. The residue was purified by silica gel column chromatography, eluted with PE / EA (1:1) to afford di-*tert*-butyl 1-(4-oxo-3-oxabicyclo [4.1.0] heptan-5-yl) hydrazine-1,2-dicarboxylate (21 g, 57.3% yield) as a white solid. LCMS (ESI): m/z [M+H] calc'd for C₁₆H₂₆N₂O₆ 342.2; found 365.2 [M+Na]⁺.

### Step 5

To a 2 L 3-necked round-bottom flask was added di-tert-butyl 1-(4-oxo-3-oxabicyclo [4.1.0] heptan-5-yl) hydrazine-1,2-dicarboxylate (15 g, 43.81 mmol, 1 equiv) and MeOH (800 mL) at room temperature. To the above solution was added DIEA (17.21 g, 133.179 mmol, 2.0 equiv) dropwise at room temperature. The resulting mixture was stirred for 2 h at 50 °C then concentrated under reduced pressure. The resulting residue was purified by silica gel column chromatography, eluted with PE / EA (5:1) to afford di-*tert*-butyl 1-(1-((1*R*,2*S*)-2-(hydroxymethyl) cyclopropyl)-2-methoxy-2-oxoethyl) hydrazine-1,2-dicarboxylate (8 g, 48.7% yield, PE / EA = 3:1, R_{f} = 0.5) and di-*tert*-butyl 1-(1-((1*S*,2*R*)-2-(hydroxymethyl) cyclopropyl)-2-methoxy-2-oxoethyl) hydrazine-1,2-dicarboxylate (4.5 g, 27.4% yield, PE / EA = 3:1, R_{f} = 0.2) as a light yellow oil. LCMS (ESI): m/z [M+H] calc'd for C₁₇H₃₀N₂O₇ 374.2; found 375.2.

### Step 6

To a solution of di-*tert*-butyl 1-(1-((1R,2S)-2-(hydroxymethyl) cyclopropyl)-2-methoxy-2-oxoethyl) hydrazine-1,2-dicarboxylate (7 g, 18.69 mmol, 1 equiv) in DCM (100 mL) was added Et₃N (3.78 g, 37.390 mmol, 2 equiv) and DMAP (0.33 g, 3.739 mmol, 0.2 equiv) at 0 °C under nitrogen atmosphere followed by TsCl (4.28 g, 22.434 mmol, 1.2 equiv) in portions at 0°C. The resulting mixture was stirred for 2 h at room temperature. After completion of the reaction as monitored by TLC, the mixture was diluted with water (100 mL) and extracted with DCM (3 x 100 mL). The combined organic layers were dried over anhydrous Na₂SO₄. After filtration, the filtrate was concentrated under reduced pressure. The residue was purified by silica gel column chromatography, eluted with PE / EA (1:2) to afford di-*tert*-butyl 1-(2-methoxy-2-oxo-1-((1*R*,2*S*)-2-((tosyloxy) methyl) cyclopropyl) ethyl) hydrazine-1,2-dicarboxylate (6.8 g, 68.8% yield) as a white solid. LCMS (ESI): m/z [M+H] calc'd for C₂₄H₃₆N₂O₉S 528.2; found 551.2 [M+Na]⁺.

### Step 7

A solution of di-*tert*-butyl 1-(2-methoxy-2-oxo-1-((1*R*,2*S*)-2-((tosyloxy) methyl) cyclopropyl) ethyl) hydrazine-1,2-dicarboxylate (6.5 g, 12.296 mmol, 1 equiv) in THF (50 mL) was treated with TBAF (1M in THF) (30.74 mL, 30.740 mmol, 2.5 equiv) at room temperature. The resulting mixture was stirred for 2 h at 50 °C under nitrogen atmosphere then concentrated under reduced pressure. The residue was purified by silica gel column chromatography, eluted with PE / EA (1:1) to afford 3,4-di-*tert*-butyl 2-methyl (1*R*,6*S*)-3,4-diazabicyclo [4.1.0] heptane-2,3,4-tricarboxylate (2.8 g, 63.8% yield) as off-white oil. LCMS (ESI): m/z [M+H] calc'd for C₁₇H₂₈N₂O₆ 356.2; found 357.2.

### Step 8

A solution of 3,4-di-*tert*-butyl 2-methyl (1*R*,6*S*)-3,4-diazabicyclo [4.1.0] heptane-2,3,4-tricarboxylate (1 g, 2.80 mmol, 1 equiv) in TFA (2 mL) and DCM (6 mL) was stirred for 12 h at room temperature. After completion of the reaction as monitored by LCMS, the resulting mixture was concentrated under reduced pressure. The crude product methyl (1*R*,6*S*)-3,4-diazabicyclo [4.1.0] heptane-2-carboxylate trifluoroacetic acid salt (1.2 g, crude) was used in the next step directly without further purification. LCMS (ESI): m/z [M+H] calc'd for C₇H₁₂N₂O₂ 156.1; found 157.0.

### Analog Example 13. methyl 2,3-diazabicyclo[3.2.1]octane-4-carboxylate

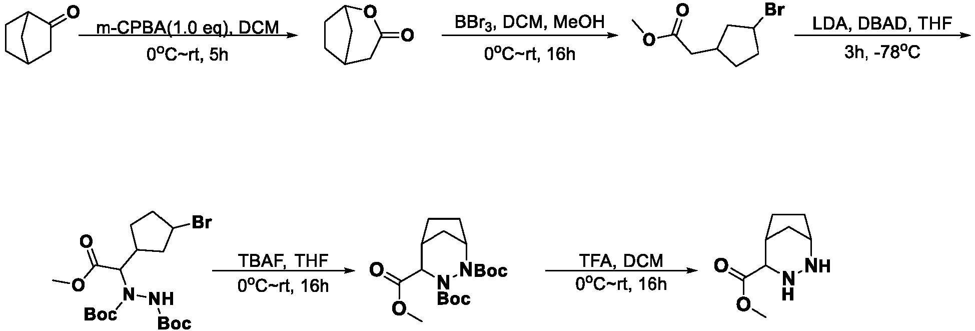

### Step 1

To a stirred solution of bicyclo[2.2.1]heptan-2-one (17 g, 154.33 mmol, 1 equiv) in DCM (800 mL) was added m-CPBA (26.63 g, 154.32 mmol, 1 equiv) in portions at 0 °C. The reaction was stirred for 5 h at room temperature then quenched with water at 0 °C. The resulting mixture was extracted with CH₂Cl₂ (3 x400 mL). The combined organics were concentrated under reduced pressure and the residue was purified by silica gel column chromatography, eluted with PE / EA (8:1) to afford 2-oxabicyclo[3.2.1]octan-3-one (12 g, 61.64% yield) as a light yellow oil. LCMS (ESI): m/z [M+H] calc'd for C₇H₁₀O₂ 126.1; found 253.3 [2M+H]⁺.

### Step 2

To a stirred solution of 2-oxabicyclo[3.2.1]octan-3-one (12 g, 95.12 mmol, 1 equiv) in DCM (120 mL) was added BBr₃ (9.6 mL, 101.55 mmol, 1.07 equiv) dropwise at 0 °C. The resulting mixture was stirred for 12 h at room temperature then MeOH (24 mL, 592.772 mmol, 6.23 equiv) was added dropwise at 0 °C. The resulting mixture was stirred for additional 4h at room temperature then quenched with Water at 0 °C. The resulting mixture was washed with DCM (2 x 200 mL) and the combined organic layers were washed with brine (3 x 500 mL), dried over anhydrous Na₂SO₄. After filtration, the filtrate was concentrated under reduced pressure. The residue was purified by silica gel column chromatography, eluted with PE / EA (3:1) to afford methyl 2-(3-bromocyclopentyl)acetate (17.41 g, 82.78% yield) as a light yellow oil. LCMS (ESI): m/z [M+H] calc'd for C₈H₁₃BrO₂ 220.0; found 262.3 [M+H+ACN]⁺.

### Step 3

To a stirred solution of methyl 2-(3-bromocyclopentyl)acetate (16.9 g, 76.43 mmol, 1 equiv) in THF (169 mL) at -78 °C was added LDA (53.7 mL, 395.99 mmol, 5.18 equiv, 2M in THF) dropwise under argon atmosphere. The resulting mixture was stirred for 1h at -78 °C. Then, to the above mixture was added DBAD (24.751 g, 107.49 mmol, 1.41 equiv) in portions over 40 min at -78 °C. The resulting mixture was stirred for an additional 1 h at -78°C then quenched with sat. NH₄Cl (aq.) at 0 °C. The aqueous layer was extracted with EtOAc (3 x 500 mL). The combined organic layers were concentrated under reduced pressure. The residue was purified by silica gel column chromatography, eluted with PE / EA (10:1) to afford di-*tert*-butyl 1-(1-(3-bromocyclopentyl)-2-methoxy-2-oxoethyl)hydrazine-1,2-dicarboxylate (13 g, 37.68% yield) as a light yellow oil. LCMS (ESI): m/z [M+H] calc'd for C₁₈H₃₁BrN₂O₆ 450.1; found 451.2.

### Step 4

To a stirred solution of di-*tert*-butyl 1-(1-(3-bromocyclopentyl)-2-methoxy-2-oxoethyl)hydrazine-1,2-dicarboxylate (17.4 g, 38.55 mmol, 1 equiv) in THF (530 mL) at 0 °C was added TBAF (42.4 mL, 1M in THF) dropwise. The resulting mixture was stirred overnight at room temperature. The reaction was quenched by the addition of sat. NH₄Cl (aq.) at 0 °C. The resulting mixture was washed with EtOAc (2 x 500 mL). The combined organic layers were washed with brine (3 x 500 mL), dried over anhydrous Na₂SO₄. After filtration, the filtrate was concentrated under reduced pressure. The residue was purified by reverse flash chromatography with the following conditions: column, C18 silica gel; mobile phase, MeCN in Water (0.1% FA), 10% to 50% gradient in 10 min; detector, UV 254 nm.to afford 2,3-di-*tert*-butyl 4-methyl 2,3-diazabicyclo[3.2.1]octane-2,3,4-tricarboxylate (6 g, 42.01% yield) as a light yellow oil. LCMS (ESI): m/z [M+H] calc'd for C₁₈H₃₀N₂O₆ 370.2; found 371.1.

### Step 5

To a stirred solution of 2,3-di-*tert*-butyl 4-methyl 2,3-diazabicyclo[3.2.1]octane-2,3,4-tricarboxylate (5.8 g, 15.65 mmol, 1 equiv) in DCM (60 mL) at 0 °C was added TFA (20 mL) dropwise. The resulting mixture was stirred for 16 h at room temperature then concentrated under reduced pressure. This resulted in methyl 2,3-diazabicyclo[3.2.1]octane-4-carboxylate (5.6 g, crude) as a light yellow oil. LCMS (ESI): m/z [M+H] calc'd for C₈H₁₄N₂O₂ 170.2; found 171.1.

### Analog Example 14. methyl 2,3-diazabicyclo[3.1.1]heptane-1-carboxylate

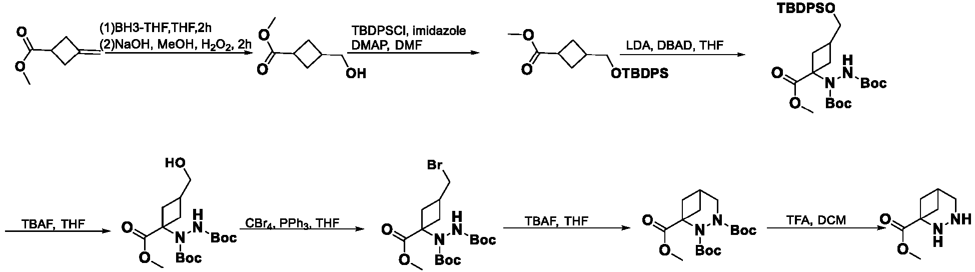

### Step 1

To a stirred solution of methyl 3-methylidenecyclobutane-1-carboxylate (5 g, 39.63 mmol, 1 equiv) in THF (50 mL) was added BH₃-THF (17.03 g, 198.17 mmol, 5 equiv) dropwise at -10 °C under argon atmosphere. The mixture was stirred for 2 h at room temperature then MeOH (25 mL) was added dropwise over 3 min at -20°C. The mixture was stirred for additional 30 min at -20 °C. To the above mixture were added 3M NaOH (aq.) (6.61 mL, 19.81 mmol, 0.50 equiv) and H₂O₂(30%) (5.39 g, 158.53 mmol, 4 equiv) in portions over 5 min at -20 °C. The resulting mixture was stirred for an additional 2 h at room temperature then quenched by the addition of sat. NH₄Cl (aq.) (50 mL) at 0 °C. The resulting mixture was extracted with EtOAc (3 x 100 mL). The combined organic layers were dried over anhydrous Na₂SO₄. After filtration, the filtrate was concentrated under reduced pressure. The crude product was used in the next step directly without further purification. This resulted in methyl 3-(hydroxymethyl)cyclobutane-1-carboxylate (5 g, crude) as a colorless oil. LCMS (ESI): m/z [M+H] calc'd for C₇H₁₂O₃ 144.1; found 145.2

### Step 2

To a stirred solution of methyl 3-(hydroxymethyl)cyclobutane-1-carboxylate (6.5 g, 45.09 mmol, 1 equiv), imidazole (9.21 g, 135.25 mmol, 3 equiv) and DMAP (0.55 g, 4.51 mmol, 0.1 equiv) in DMF (70 mL) at 0 °C was added TBDPSCI (18.59 g, 67.63 mmol, 1.5 equiv) in portions under argon atmosphere. The reaction mixture was stirred for 1 h at room temperature then diluted with water (200 mL). The resulting mixture was extracted with EtOAc (3 x 200 mL). The combined organic layers were washed with brine (3 x 500 mL), dried over anhydrous Na₂SO₄. After filtration, the filtrate was concentrated under reduced pressure. After filtration, the filtrate was concentrated under reduced pressure. The residue was purified by silica gel column chromatography, eluted with PE / EA (5:1) to afford methyl 3-{[(tert-butyldiphenylsilyl)oxy]methyl}cyclobutane-1-carboxylate (12.5 g, 68.85% yield) as a colorless oil. LCMS (ESI): m/z [M+H] calc'd for C₂₃H₃₀O₃Si 382.2; found 383.2.

### Step 3

To a stirred solution of methyl 3-{[(*tert*-butyldiphenylsilyl)oxy]methyl}cyclobutane-1-carboxylate (13.5 g, 35.28 mmol, 1 equiv) in THF (130 mL, 1604.56 mmol, 45.47 equiv) at -78 °C was added LDA (26.47 mL, 52.93 mmol, 1.50 equiv) dropwise under argon atmosphere. The mixture was stirred for 1 h at -78 °C then DBAD (12.19 g, 52.930 mmol, 1.5 equiv) was added in portions over 20 min. The resulting mixture was stirred for an additional 2 h at -60 °C then quenched by the addition of sat. NH₄Cl (aq.) (5 mL) at -60 °C. The resulting mixture was diluted with water (200 mL) and extracted with EtOAc (3 x 200 mL). The combined organic layers were dried over anhydrous Na₂SO₄. After filtration, the filtrate was concentrated under reduced pressure. The residue was purified by silica gel column chromatography, eluted with PE / EA (5:1) to afford methyl 1-[(*tert*-butoxycarbonyl)[(*tert-*butoxycarbonyl)amino]amino]-3-{[(*tert*-butyldiphenylsilyl)oxy]methyl}cyclobutane-1-carboxylate (13.5 g, 56.18% yield) as a colorless oil. LCMS (ESI): m/z [M+H] calc'd for C₃₃H₄₈N₂O₇Si 612.3; found 635.2 [M+Na]⁺.

### Step 4

To a stirred solution of methyl 1-[(*tert*-butoxycarbonyl)[(*tert*-butoxycarbonyl)amino]amino]-3-{[(*tert*-butyldiphenylsilyl)oxy]methyl}cyclobutane-1-carboxylate (7 g, 11.42 mmol, 1 equiv) in THF (70 mL) at 0 °C was added TBAF (5.97 g, 22.84 mmol, 2 equiv) in portions. The resulting mixture was stirred for 2 h at room temperature then concentrated under reduced pressure. The residue was purified by silica gel column chromatography, eluted with PE / EA (5:1) to afford methyl 1-[(*tert-*butoxycarbonyl)[(*tert*-butoxycarbonyl)amino]amino]-3-(hydroxymethyl)cyclobutane-1-carboxylate (2.5 g, 55.53% yield) as a colorless oil. LCMS (ESI): m/z [M+H] calc'd for C₁₇H₃₀N₂O₇ 374.2; found 375.3.

### Step 5

To a stirred solution of methyl 1-[(*tert*-butoxycarbonyl)[(*tert*-butoxycarbonyl)amino]amino]-3-(hydroxymethyl)cyclobutane-1-carboxylate (2.5 g, 6.67 mmol, 1 equiv) and PPh₃ (3.50 g, 13.35 mmol, 2 equiv) in THF (25 mL) at 0 °C was added CBr₄ (4.43 g, 13.35 mmol, 2 equiv) in portions. The resulting reaction was stirred for 2 h at room temperature then diluted with water (20 mL). The resulting mixture was extracted with EtOAc (3 x 20 mL). The combined organic layers were dried over anhydrous Na₂SO₄. After filtration, the filtrate was concentrated under reduced pressure. The residue was purified by silica gel column chromatography, eluted with PE / EA (3:1) to afford methyl 3-(bromomethyl)-1-[(tert-butoxycarbonyl)[(tert-butoxycarbonyl)amino]amino]cyclobutane-1-carboxylate (2.5 g, 77.06% yield) as a colorless oil. LCMS (ESI): m/z [M+H] calc'd for C₁₇H₂₉BrN₂O₆ 436.1; found 457.1 [M+Na]⁺.

### Step 6

To a stirred solution of methyl 3-(bromomethyl)-1-[(*tert*-butoxycarbonyl)[(*tert-*butoxycarbonyl)amino]amino]cyclobutane-1-carboxylate (2 g, 4.57 mmol, 1 equiv) in THF (20 mL, 246.85 mmol, 53.98 equiv) at 0 °C was added TBAF (5.98 g, 22.87 mmol, 5.00 equiv) in portions under argon atmosphere. The reaction mixture was stirred for 2 h at room temperature then diluted with water (100 mL). The resulting mixture was extracted with EtOAc (3 x 100 mL). The combined organic layers were dried over anhydrous Na₂SO₄. After filtration, the filtrate was concentrated under reduced pressure. The residue was purified by silica gel column chromatography, eluted with PE / EA (3:1) to afford 2,3-di-*tert*-butyl 1-methyl 2,3-diazabicyclo[3.1.1]heptane-1,2,3-tricarboxylate (700 mg, 38.65% yield) as a white solid. LCMS (ESI): m/z [M+H] calc'd for C₁₇H₂₈N₂O₆ 356.2; found 379.2 [M+Na]⁺.

### Step 7

To a stirred solution of 2,3-di-*tert*-butyl 1-methyl 2,3-diazabicyclo[3.1.1]heptane-1,2,3-tricarboxylate (500 mg, 1.40 mmol, 1 equiv) in DCM (15 mL) was added TFA (5.00 mL, 67.31 mmol, 47.98 equiv) dropwise at 0 °C under argon atmosphere. The resulting mixture was stirred for 1 h at room temperature then concentrated under reduced pressure. The crude product was used in the next step directly without further purification. This resulted in methyl 2,3-diazabicyclo[3.1.1]heptane-1-carboxylate (220 mg, crude) as a yellow oil. LCMS (ESI): m/z [M+H] calc'd for C₇H₁₂N₂O₂ 156.2; found 157.2.

### Analog Example 15. methyl 5,5-difluorohexahydropyridazine-3-carboxylate

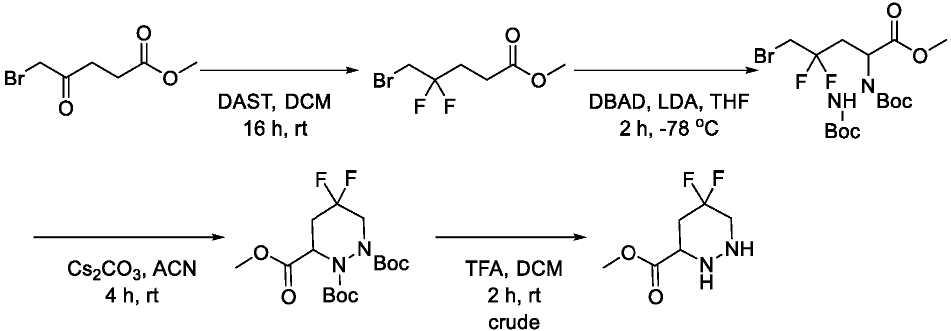

### Step 1

To a solution of methyl 5-bromo-4-oxopentanoate (10 g, 47.83 mmol, 1 equiv) in DCM (200 mL) was added DAST (30.84 g, 191.35 mmol, 4 equiv) dropwise at 0 °C. The mixture was stirred overnight at room temperature. After completion of the reaction was monitored by TLC, the reaction was quenched by the addition of sat. NaHCO₃ (aq.) (10 mL) at 0 °C and extracted with CH₂Cl₂ (3 x 50 mL). The combined organics were dried over anhydrous Na₂SO₄. After filtration, the filtrate was concentrated under reduced pressure. The residue was purified by silica gel column chromatography, eluted with PE/EA (15:1) to afford methyl 5-bromo-4,4-difluoropentanoate (5.3 g, 47% yield) as a yellow oil. ¹H NMR (400 MHz, CDCl₃) δ 3.71 (s, 3H), 3.54 (t, *J* = 12.9 Hz, 2H), 2.60 - 2.53 (m, 2H), 2.48 - 2.35 (m, 2H). ¹⁹F NMR (282 MHz, CDCl₃) δ -100.37.

### Step 2

To a solution of methyl 5-bromo-4,4-difluoropentanoate (5.3 g, 22.94 mmol, 1 equiv) in THF (100 mL) was added to at LDA (28.7 mL, 57.35 mmol, 2.5 equiv) in portions at -78 °C under an argon atmosphere. After 20 min, a solution of DBAD (9.72 g, 42.21 mmol, 1.84 equiv) in THF (10 mL) was added and the mixture was stirred for additional 1 h at -78 °C. After completion of the reaction was monitored by LCMS, the reaction was quenched by sat. NH₄Cl (aq.) (20 mL) at -78 °C and the mixture was extracted with EtOAc (3 x 20 mL). The combined organic layers were washed with brine (3 x 50 mL), dried over anhydrous Na₂SO₄. After filtration, the filtrate was concentrated under reduced pressure. The residue was purified by silica gel column chromatography, eluted with PE/EA (9:1) to afford methyl 5-bromo-2-[(*tert*-butoxycarbonyl)[(*tert*-butoxycarbonyl) amino]amino]-4,4-difluoropentanoate (3.8 g, 35% yield) as yellow oil. LCMS (ESI): m/z [M+H] calc'd for C₁₆H₂₇BrF₂N₂O₆ 460.1; found 483.0 [M+Na]⁺.

### Step 3

To a 250 mL round-bottom flask was added methyl 5-bromo-2-[(*tert*-butoxycarbonyl) [(*tert* butoxycarbonyl) amino] amino]-4,4-difluoropentanoate (3.8 g, 8.23 mmol, 1 equiv), Cs₂CO₃ (12.00 g, 36.824 mmol, 4.47 equiv) and ACN (40.0 mL). The mixture was stirred for 12 h at 30 °C. After completion of the reaction, as monitored by LCMS, the resulting mixture was concentrated under reduced pressure. The residue was diluted with brine (100 mL) and extracted with EtOAc (5 x 30 mL). The combined organic layers were washed with brine (3 x 50 mL), dried over anhydrous Na₂SO₄. After filtration, the filtrate was concentrated under reduced pressure. The residue was purified by silica gel column chromatography, eluted with PE/EA (4 :1) to afford 1,2-di-tert-butyl 3-methyl 5,5-difluoro-1,2-diazinane-1,2,3-tricarboxylate (2.6 g, 82% yield) as a yellow oil. LCMS (ESI): m/z [M+H] calc'd for C₁₆H₂₆F₂N₂O₆ 380.2.1; found 444.0 [M+Na+ACN]⁺.

### Step 4

To a stirred solution of 1,2-di-tert-butyl 3-methyl 5,5-difluoro-1,2-diazinane-1,2,3-tricarboxylate (2.6 g, 6.835 mmol, 1 equiv) in DCM (12 mL) was added TFA (4.0 mL, 45.8 mmol) dropwise at 0 °C. Then the resulting mixture was stirred for 2 h at 25 °C then concentrated under reduced pressure. This resulted in methyl 5,5-difluoro-1,2-diazinane-3-carboxylate trifluoroacetic acid salt (2.1 g, crude) as a yellow oil. The crude resulting mixture was used in the next step directly without further purification. LCMS (ESI): m/z [M+H] calc'd for C₆H₁₀F₂N₂O₂ 180.2.1; found 181.1.

### Analog Example 16. ethyl (methylamino)alaninate

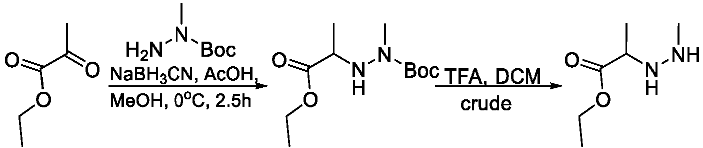

### Step 1

To a 100 mL round-bottom flask was added ethyl 2-oxopropanoate (2 g, 17.224 mmol, 1 equiv), tert-butyl 1-methylhydrazine-1-carboxylate (2.52 g, 17.22 mmol, 1 equiv), MeOH (30 mL) and AcOH (0.10 g, 1.72 mmol, 0.1 equiv) at 0 °C. The resulting mixture was stirred for 30 min at 0 °C. To the above mixture was added NaBH₃CN (2.16 g, 34.44 mmol, 2 equiv) in portions at 0 °C. The resulting mixture was stirred for 2 h at room temperature. The reaction was quenched by the addition of Water/Ice (50 mL) at 0 °C and extracted with CH₂Cl₂ (3 x 50 mL). The organic layers were washed with water (3 x50 mL), dried over anhydrous Na₂SO₄. After filtration, the filtrate was concentrated under reduced pressure. The residue was purified by silica gel column chromatography, eluted with PE / EA (1:1) to afford tert-butyl 2-(1-ethoxy-1-oxopropan-2-yl)-1-methylhydrazine-1-carboxylate (2.8 g, 66.00% yield) as a colorless oil. LCMS (ESI): m/z [M+H] calc'd for C₁₁H₂₂N₂O₄ 246.2; found 269.1 [M+Na]⁺.

### Step 2

To a 100 mL round-bottom flask was added *tert*-butyl 2-(1-ethoxy-1-oxopropan-2-yl)-1-methylhydrazine-1-carboxylate (1.6 g, 6.496 mmol, 1 equiv), DCM (20 mL) and TFA (5 mL) dropwise at 0 °C. The resulting mixture was stirred for 1 h at 0 °C then concentrated under reduced pressure. This resulted in ethyl (methylamino)alaninate (1.2 g, 88.45% yield) as a white solid. LCMS (ESI): m/z [M+H] calc'd for C₆H₁₄N₂O₂ 146.2; found 147.1.

### Analog Example 17. ethyl 2-(2-methylhydrazineyl)butanoate

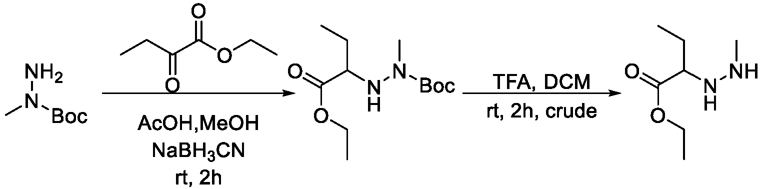

### Step 1

To a stirred mixture of *N*-methyl *tert*-butoxycarbohydrazide (2g, 13.68 mmol, 1equiv) in MeOH (20 mL) was added CH₃COOH (0.16 g, 2.73 mmol, 0.2 equiv) and ethyl 2-oxobutanoate (1.78 g, 13.68 mmol, 1 equiv) at 0°C under argon atmosphere. The resulting mixture was stirred for 1 h at room temperature then cooled to 0 °C followed by the addition of NaBH₃CN (1.72 g, 27.36 mmol, 2 equiv). The resulting mixture was stirred for 1 h at room temperature then quenched with Water (10 mL) at room temperature. The resulting mixture was extracted with CH₂Cl₂ (3 x 50 mL). The combined organic layers were washed with brine (3 x 5 mL), dried over anhydrous Na₂SO₄. After filtration, the filtrate was concentrated under reduced pressure. The residue was purified by silica gel column chromatography, eluted with PE / EA (1:1) to afford *tert*-butyl 2-(1-ethoxy-1-oxobutan-2-yl)-1-methylhydrazine-1-carboxylate (2.3 g, 64.5% yield) as a brown yellow oil. LCMS (ESI): m/z [M+H] calc'd for C₁₂H₂₄N₂O₄ 260.2; found 205.2 [M-56+H]⁺.

### Step 2

To a stirred mixture of *tert*-butyl 2-(1-ethoxy-1-oxobutan-2-yl)-1-methylhydrazine-1-carboxylate (3.35 g, 12.86 mmol, 1 equiv) in DCM (30 mL) was added TFA (15 mL) dropwise at 0°C under argon atmosphere. The resulting mixture was stirred for 2h at room temperature then concentrated under reduced pressure. This resulted in ethyl 2-(2-methylhydrazineyl)butanoate (2.9 g, crude) as a brown yellow solid. LCMS (ESI): m/z [M+H] calc'd for C₇H₁₆N₂O 160.2; found 161.3.

### Analog Example 18. ethyl 3-cyclopropyl-2-(2-methylhydrazineyl)propanoate

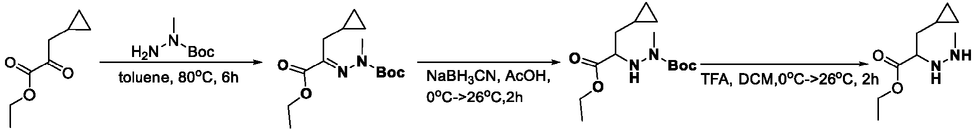

### Step 1

A mixture of ethyl 3-cyclopropyl-2-oxopropanoate (650 mg, 4.16 mmol, 1 equiv) and *tert-*butyl-1-methylhydrazine-1-carboxylate (3042.10 mg, 20.81 mmol, 5 equiv) in Toluene (7 mL) was stirred for 6 h at 80 °C under argon atmosphere. The resulting mixture was diluted with EtOAc (20 mL) and concentrated under reduced pressure. The crude product (1.2 g, crude) was used in the next step directly without further purification. LCMS (ESI): m/z [M+H] calc'd for C₁₄H₂₄N₂O₄ 284.2; found 285.2.

### Step 2

A mixture of *tert*-butyl(*E*)-2-(3-cyclopropyl-1-ethoxy-1-oxopropan-2-ylidene)-1-methylhydrazine-1-carboxylate (1.2 g, 4.22 mmol, 1 equiv) and NaBH₃CN (662.97 mg, 10.55 mmol, 2.5 equiv) in AcOH (15 mL) was stirred for 2 h at 26 °C under argon atmosphere. The reaction was quenched by the addition of NaHCO₃ (aq.) (100 mL) at 0 °C. The aqueous layer was extracted with EtOAc (3 x 100 mL). The combined organics were concentrated under reduced pressure. The residue was purified by reverse flash chromatography with the following conditions: column, C18 silica gel; mobile phase, ACN in Water (0.5% NH₄HCO₃), 30% to 55% gradient in 25 min; detector, UV 220 nm. This resulted in tert-butyl2-(3-cyclopropyl-1-ethoxy-1-oxopropa n-2-yl)-1-methylhydrazine-1-carboxylate (0.8 g, 66 % yield). LCMS (ESI): m/z [M+H] calc'd for C₁₄H₂₆N₂O₄ 286.2; found 287.2.

### Step 3

A mixture of ethyl 2-{[(*tert*-butoxycarbonyl)(methyl)amino]amino}-3-cyclopropylpropanoate (800 mg, 2.79 mmol, 1 equiv) and TFA (4 mL) in DCM (8 mL) was stirred for 2 h at 26 °C under argon atmosphere. The resulting mixture was diluted with EtOAc (10 mL) then concentrated under reduced pressure. The crude product was used in the next step directly without further purification. LCMS (ESI): m/z [M+H] calc'd for C₉H₁₈N₂O₂ 186.2; found 187.2.

### Analog Example 19. ethyl 2-methyl-2-(2-methylhydrazineyl)propanoate

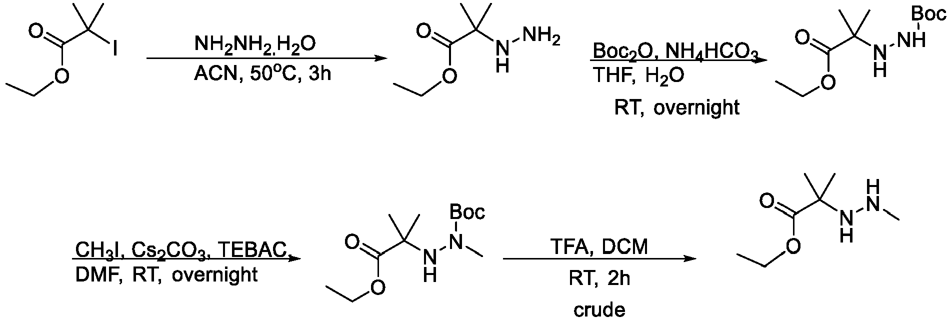

### Step 1

To a 40 mL vial was added ethyl 2-iodo-2-methylpropanoate (1 g, 4.1 mmol, 1 equiv), ACN (10 mL), and NH₂NH₂.H₂O (0.78 g, 12.39 mmol, 3 equiv, 80%) at 0 °C. The resulting mixture was stirred for 3 h at 50°C then concentrated under reduced pressure to afford ethyl 2-hydrazinyl-2-methylpropanoate (500 mg, 82% yield) as a colorless oil. LCMS (ESI): m/z [M+H] calc'd for C₆H₁₄N₂O₂ 146.1; found 147.2.

### Step 2

To a 40 mL vial was added ethyl 2-hydrazinyl-2-methylpropanoate (450 mg, 3.07 mmol, 1 equiv), H₂O (2.5 mL), THF (2.5 mL), NaHCO₃ (608.36 mg, 7.69 mmol, 2.5 equiv), and Boc₂O (1007.72 mg, 4.61 mmol, 1.5 equiv) at 0°C. The resulting mixture was stirred overnight at room temperature then extracted with EtOAc (3 x 10 mL). The combined organic layers were washed with water (3 x 10 mL), dried over anhydrous Na₂SO₄. After filtration, the filtrate was concentrated under reduced pressure. The residue was purified by reversed-phase flash chromatography with the following conditions: column, C₁₈ silica gel; mobile phase A: Water (10mmol/L NH₄HCO₃), mobile phase B: ACN , 30% to 50% gradient in 20 min; detector, UV 200 nm to afford ethyl 2-{[(*tert-*butoxycarbonyl)amino]amino}-2-methylpropanoate (440 mg, 58% yield) as a colorless oil. LCMS (ESI): m/z [M+H] calc'd for C₁₁H₂₂N₂O₄ 246.2; found 493.2 [2M+H]⁺.

### Step 3

To a 40 mL vial was added ethyl 2-{[(*tert*-butoxycarbonyl)amino]amino}-2-methylpropanoate (400 mg, 1.62 mmol, 1 equiv), DMF (5 mL), Cs₂CO₃ (1058.25 mg, 3.24 mmol, 2 equiv), benzyltriethylazanium chloride (369.90 mg, 1.62 mmol, 1 equiv), and CH₃I (276.61 mg, 1.94 mmol, 1.2 equiv) at 0 °C. The resulting mixture was stirred overnight at room temperature then quenched by the addition of water (5 mL) at 0°C.The resulting mixture was extracted with EtOAc (3 x 10 mL). The combined organic layers were washed with water (3 x 10 mL), dried over anhydrous Na₂SO₄. After filtration, the filtrate was concentrated under reduced pressure. The residue was purified by reversed-phase flash chromatography with the following conditions: column, C₁₈ silica gel; mobile phase A: Water (10mmol/L NH₄HCO₃), mobile phase B: ACN, 35% to 60% gradient in 18 min; detector, UV 200 nm. to afford ethyl 2-{[(tert-butoxycarbonyl)(methyl)amino]amino}-2-methylpropanoate (100 mg, 23% yield) as colorless oil. LCMS (ESI): m/z [M+H] calc'd for C₁₂H₂₄N₂O₄ 260.2; found 261.2.

### Step 4

To an 8 mL vial were added ethyl 2-{[(*tert*-butoxycarbonyl)(methyl)amino]amino}-2-methylpropanoate (100 mg, 0.384 mmol, 1 equiv), DCM (1.5 mL) and TFA (0.3 mL) at 0 °C. The resulting mixture was stirred for 2h at room temperature then concentrated under reduced pressure to afford ethyl 2-methyl-2-(2-methylhydrazin-1-yl)propanoate (70 mg, crude) as a yellow oil. The crude product was used in the next step directly without further purification. LCMS (ESI): m/z [M+H] calc'd for C₇H₁₆N₂O 160.2; found 161.2.

### Analog Example 20. ethyl 6,7-diazaspiro[3.5]nonane-5-carboxylate

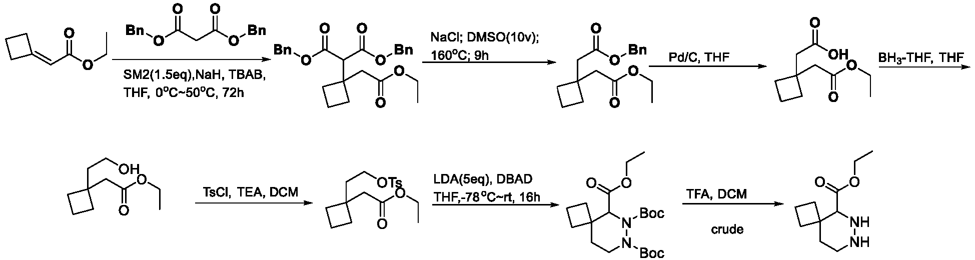

### Step 1

A solution of NaH (2.14 g, 89.17 mmol, 2.5 equiv) and ethyl 2-cyclobutylideneacetate (5 g, 35.66 mmol, 1 equiv) in THF (100 mL) was stirred for 30 min at room temperature under argon atmosphere. To the above mixture was added TBAB (3.45 g, 10.70 mmol, 0.3 equiv) and 1,3-dibenzyl propanedioate (32.45 g, 114.13 mmol, 3.2 equiv) in Et2O (25 mL) dropwise over 10 min at -10 °C. The resulting mixture was stirred for 3 days at 50 °C then diluted with water (100 mL). The resulting mixture was extracted with EtOAc (3 x 100 mL). The combined organic layers were dried over anhydrous Na₂SO₄. After filtration, the filtrate was concentrated under reduced pressure. The residue was purified by reverse flash chromatography with the following conditions: column, C18 silica gel; mobile phase, ACN in Water (0.1% FA), 10% to 50% gradient in 10 min; detector, UV 254 nm. This resulted in 1,3-dibenzyl 2-[1-(2-ethoxy-2-oxoethyl)cyclobutyl]propanedioate (9 g, 53.50% yield) as a yellow oil. LCMS (ESI): m/z [M+H] calc'd for C₂₅H₂₈O₆ 424.2; found 425.2.

### Step 2

A solution of 1,3-dibenzyl 2-[1-(2-ethoxy-2-oxoethyl)cyclobutyl]propanedioate (10 g, 23.55 mmol, 1 equiv) and NaCl (4.13 g, 70.67 mmol, 3 equiv) in DMSO (120 mL) was stirred overnight at 160 °C. The resulting mixture was extracted with EtOAc (3 x 100 mL). The combined organic layers were washed with brine (3 x 300 mL), dried over anhydrous Na₂SO₄. After filtration, the filtrate was concentrated under reduced pressure. The residue was purified by reverse flash chromatography with the following conditions: column, C18 silica gel; mobile phase, MeCN in Water (0.1% FA), 10% to 50% gradient in 10 min; detector, UV 254 nm. This resulted in ethyl 2-{1-[2-(benzyloxy)-2-oxoethyl]cyclobutyl}acetate (5 g, 69.44% yield) as a yellow oil. LCMS (ESI): m/z [M+H] calc'd for C₁₇H₂₂O₄ 290.2; found 291.0.

### Step 3

A solution of ethyl 2-{1-[2-(benzyloxy)-2-oxoethyl]cyclobutyl}acetate (5 g, 17.22 mmol, 1 equiv) and Pd/C (2.49 g, 23.41 mmol, 1.36 equiv) in THF (50 mL) was stirred overnight at room temperature under hydrogen atmosphere. The precipitated solids were collected by filtration and washed with EtOAc (3 x 50 mL). The filtrate was concentrated under reduced pressure. This resulted in [1-(2-ethoxy-2-oxoethyl)cyclobutyl]acetic acid (3 g, crude) as a yellow oil. The crude product was used in the next step directly without further purification. LCMS (ESI): m/z [M+H] calc'd for C₁₀H₁₆O₄ 200.1; found 201.2.

### Step 4

To a stirred solution of [1-(2-ethoxy-2-oxoethyl)cyclobutyl]acetic acid (1.7 g, 8.49 mmol, 1 equiv) in THF (20 mL) was added BH₃•THF (8.49 mL, 16.98 mmol, 2.00 equiv) dropwise at 0 °C under argon atmosphere. The resulting mixture was stirred for 1 h at room temperature then cooled to 0°C and quenched by the addition of MeOH (10 mL). The resulting mixture was concentrated under reduced pressure. This resulted in ethyl 2-[1-(2-hydroxyethyl)cyclobutyl]acetate (1.5 g, crude) as a colorless oil. The crude product was used in the next step directly without further purification. LCMS (ESI): m/z [M+H] calc'd for C₁₀H₁₈O₃ 186.1; found 187.2.

### Step 5

To a stirred solution of ethyl 2-[1-(2-hydroxyethyl)cyclobutyl]acetate (800 mg, 4.29 mmol, 1 equiv) and TEA (1303.96 mg, 12.88 mmol, 3 equiv) in DCM (8 mL) at 0 °C was added TsCl (1.64 g, 8.59 mmol, 2 equiv) in portions. The reaction mixture was stirred for 2 h at room temperature then diluted with water (20 mL). The resulting mixture was extracted with EtOAc (3 x 20 mL). The combined organic layers were dried over anhydrous Na₂SO₄. After filtration, the filtrate was concentrated under reduced pressure. The residue was purified by Prep-TLC (PE / EA 3:1) to afford ethyl 2-(1-{2-[(4-methylbenzenesulfonyl)oxy]ethyl}cyclobutyl)acetate (900 mg, 58.47% yield) as a yellow oil. LCMS (ESI): m/z [M+H] calc'd for C₁₇H₂₄O₅S 340.1; found 358.1 [M+NH₄]⁺.

### Step 6

A solution of ethyl 2-(1-{2-[(4-methylbenzenesulfonyl)oxy]ethyl}cyclobutyl)acetate (700 mg, 2.05 mmol, 1 equiv) in THF (10 mL) at -78 °C was treated with LDA (5.14 mL, 10.28 mmol, 5 equiv) for 1 h under argon atmosphere, followed by the addition of DBAD (710.21 mg, 3.08 mmol, 1.5 equiv) dropwise. The resulting mixture was stirred for 2 h at -60 °C then quenched by the addition of sat. NH₄Cl (aq.) (20 mL) at 0 °C. The resulting mixture was diluted with water (30 mL) and extracted with EtOAc (3 x 50 mL). The combined organic layers were dried over anhydrous Na₂SO₄. After filtration, the filtrate was concentrated under reduced pressure. The residue was purified by Prep-TLC (PE / EA 3:1) to afford 6,7-di-tert-butyl 5-ethyl 6,7-diazaspiro[3.5]nonane-5,6,7-tricarboxylate (370 mg, 31.61% yield) as a colorless oil. LCMS (ESI): m/z [M+H] calc'd for C₂₀H₃₄N₂O₆ 398.2; found 399.2.

### Step 7

To a stirred solution of 6,7-di-tert-butyl 5-ethyl 6,7-diazaspiro[3.5]nonane-5,6,7-tricarboxylate (350 mg, 0.87 mmol, 1 equiv) in DCM (9 mL) was added TFA (3 mL, 40.38 mmol, 45.99 equiv) dropwise at 0 °C. The resulting mixture was stirred for 1 h at room temperature then concentrated under reduced pressure. This resulted in ethyl 6,7-diazaspiro[3.5]nonane-5-carboxylate (180 mg, crude) as a colorless oil. The crude product was used in the next step directly without further purification. LCMS (ESI): m/z [M+H] calc'd for C₁₀H₁₈N₂O₂ 198.2; found 199.2.

### Analog Example 21. tert-butyl 2-(1-(methoxycarbonyl)cyclopropyl)-1-methylhydrazine-1-carboxylate

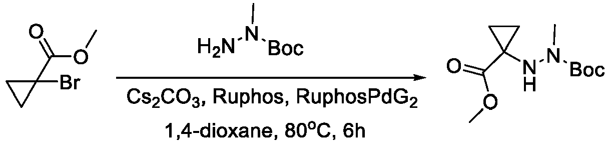

### Step 1

To a stirred solution of methyl 1-bromocyclopropane-1-carboxylate (1.5 g, 8.37 mmol, 1 equiv) and tert-butyl 1-methylhydrazine-1-carboxylate (1.10 g, 7.54 mmol, 0.9 equiv) in dioxane (20 mL) was added Cs₂CO₃ (6.83 g, 20.94 mmol, 2.5 equiv), Ruphos (1.17 g, 2.51 mmol, 0.3 equiv) and RuPhos-PdCl-2nd G (977.54 mg, 1.25 mmol, 0.15 equiv). After stirring for 6 h at 80 °C under a nitrogen atmosphere, the resulting mixture was concentrated under reduced pressure. The resulting mixture was diluted with brine (20 mL) and extracted with EtOAc (3 x 20 mL). The combined organic layers were washed with brine (3 x 30 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (10 mmol/L NH₄HCO₃), 5% to 100% gradient in 20 min; detector, UV 220 nm. This resulted in *tert-*butyl 2-(1-(methoxycarbonyl) cyclopropyl)-1-methylhydrazine-1-carboxylate (580 mg, 28.3% yield) as a white solid. LCMS (ESI): m/z [M+H] calc'd for C₁₁H₂₀N₂O₄ 244.1; found 245.2.

### Analog Example 22. di-tert-butyl 1-(1-(ethoxycarbonyl)cyclobutyl)-2-methylhydrazine-1,2-dicarboxylate

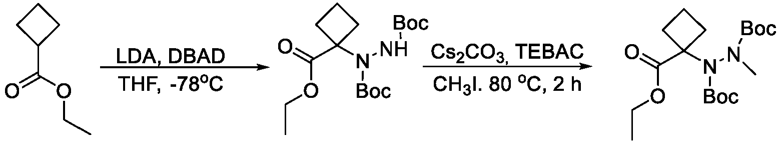

### Step 1

To a stirred solution of ethyl cyclobutanecarboxylate (1.5 g, 11.70 mmol, 1 equiv) in THF (15 ml) was added LDA (8.8 mL, 17.55 mmol, 1.5 equiv) dropwise at -78 °C under argon atmosphere. The mixture was stirred for 1 h at -78 °C then a solution of DBAD (3.23 g, 14.044 mmol, 1.2 equiv) in THF (10 mL) was added and the mixture was stirred for additional 2 h. The reaction was then quenched with sat. NH₄Cl (aq.) at 0 °C and the mixture extracted with EtOAc (3 x 30 mL). The combined organic layers were washed with water (3 x 30 mL), dried over anhydrous Na₂SO₄. After filtration, the filtrate was concentrated under reduced pressure. The residue was purified by silica gel column chromatography, eluted with PE / EA (2:1) to afford di-*tert*-butyl 1-(1-(ethoxycarbonyl)cyclobutyl)hydrazine-1,2-dicarboxylate (1.1 g, 26.2% yield) as a white solid. LCMS (ESI): m/z [M+H] calc'd for C₁₇H₃₀N₂O₆ 258.2; found 259.2.

### Step 2

To a stirred solution of di-*tert*-butyl 1-(1-(ethoxycarbonyl)cyclobutyl)hydrazine-1,2-dicarboxylate (700 mg, 1.95 mmol, 1 equiv) and Cs₂CO₃ (1.2 g, 3.90 mmol, 2 equiv) in DCM (20 mL) was added benzyltriethylazanium chloride (889.65 mg, 3.90 mmol, 2 equiv) and CH₃I (554.40 mg, 3.90 mmol, 2 equiv) dropwise at 25 °C under air atmosphere. Then the mixture was heated to 80 °C and stirred for 2 h. The resulting mixture was diluted with water (20 mL) and extracted with CH₂Cl₂ (3 x 20 mL). The combined organic layers were washed with water (3 x 20 mL), dried over anhydrous Na₂SO₄. After filtration, the filtrate was concentrated under reduced pressure. The residue was purified by silica gel column chromatography, eluted with PE / EA (3:1) to afford ethyl 1-[(*tert-*butoxycarbonyl)[(*tert*-butoxycarbonyl)(methyl)amino]amino]cyclobutane-1-carboxylate (600 mg, 82.4% yield) as a white solid. LCMS (ESI): m/z [M+H] calc'd for C₁₈H₃₂N₂O₆ 272.2; found 273.2.

### Analog Example 23. methyl 1-(2-methylhydrazineyl)cyclopentane-1-carboxylate

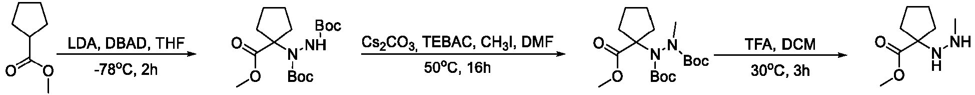

### Step 1

To a 50 mL 3-necked round-bottom flask was added methyl cyclopentanecarboxylate (1 g, 7.80 mmol, 1 equiv), THF (10 mL) and LDA (5.85 mL, 11.70 mmol, 1.5 equiv) at -78 °C. The resulting mixture was stirred for 1 h then DBAD (2.34 g, 10.143 mmol, 1.3 equiv) was added dropwise over 10 min at -78 °C. The resulting mixture was stirred for an additional 1 h then quenched with water (100 mL) and extracted with EtOAc (3 x 150 mL). The combined organic layers were washed with water (2 x 150 mL), dried over anhydrous Na₂SO₄. After filtration, the filtrate was concentrated under reduced pressure. The residue was purified by silica gel column chromatography, eluted with PE / EA (6:1) to afford methyl 1-[(*tert*-butoxycarbonyl)[(*tert*-butoxycarbonyl)amino]amino]cyclopentane-1-carboxylate (1.5 g, 53% yield) as a yellow solid. LCMS (ESI): m/z [M+H] calc'd for C₁₇H₃₀N₂O₆ 358.2; found 359.2.

### Step 2

To a 40 mL vial was added methyl 1-[(*tert*-butoxycarbonyl)[(*tert-*butoxycarbonyl)amino]amino]cyclopentane-1-carboxylate (300 mg, 0.83 mmol, 1 equiv), DMF (5 mL), Cs₂CO₃ (545.40 mg, 1.67 mmol, 2 equiv), TEBAC (190.64 mg, 0.837 mmol, 1 equiv), and CH₃I (142.56 mg, 1.00 mmol, 1.2 equiv) at 0 °C. The resulting mixture was heated to 50 °C and stirred for 16 h. The reaction was then filtered, and the filter cake was washed with EtOAc (3 x 3 mL). The filtrate was concentrated under reduced pressure and redissolved in EtOAc (5 mL). Water (50 mL) was added, and the mixture extracted with EtOAc (3 x 50 mL). The combined organic layers were dried over anhydrous Na₂SO₄. After filtration, the filtrate was concentrated under reduced pressure. The residue was purified by Prep-TLC (PE / EA 7:1) to afford methyl 1-[(*tert*-butoxycarbonyl)[(*tert-*butoxycarbonyl) (methyl)amino]amino]cyclopentane-1-carboxylate (270 mg, 86% yield) as a yellow solid. LCMS (ESI): m/z [M+H] calc'd for C₁₈H₃₂N₂O₆ 372.2; found 373.2.

### Step 3

To a 40 mL vial was added methyl 1-[(*tert*-butoxycarbonyl)[(*tert-*butoxycarbonyl)(methyl)amino]amino] cyclopentane-1-carboxylate (275 mg, 0.73 mmol, 1 equiv), DCM (3 mL) and TFA (0.4 mL) at 0 °C. The resulting mixture was stirred for 3 h at 30 °C then concentrated under reduced pressure. This resulted in methyl 1-(2-methylhydrazin-1-yl)cyclopentane-1-carboxylate (180 mg, crude) as yellow oil. The crude product was used in the next step directly without further purification. LCMS (ESI): m/z [M+H] calc'd for C₈H₁₆N₂O₂ 172.2; found 173.2.

### Analog Example 24. methyl (methylamino)-L-leucinate

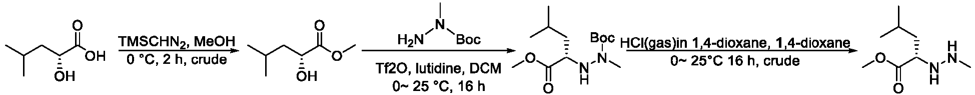

### Step 1

To a 250 mL 3-necked round-bottom flask was added (R)-2-hydroxy-4-methylpentanoic acid (5 g, 37.83 mmol, 1 equiv) and MeOH (50 mL) at 0 °C. To the above mixture was added TMSCHN₂ (94.58 mL, 189.16 mmol, 5 equiv, 2M in hexanes) dropwise at 0 °C. The resulting mixture was stirred for 2 h then concentrated under reduced pressure to afford methyl (*R*)-2-hydroxy-4-methylpentanoate (6.75 g, crude) as a yellow oil. The crude product was used in the next step directly without further purification. LCMS (ESI): m/z [M+H] calc'd for C₇H₁₄O₃ 146.1; found 147.1.

### Step 2

A solution of methyl (*R*)-2-hydroxy-4-methylpentanoate (5.3 g, 36.25 mmol, 1 equiv) in DCM (53 mL) at 0 °C was treated with lutidine (8.3 mL, 71.26 mmol, 1.97 equiv) and Tf₂O (8 mL, 47.35 mmol, 1.31 equiv) for 1 h followed by the addition of *tert*-butyl 1-methylhydrazine-1-carboxylate (9.5 mL, 64.00 mmol, 1.77 equiv) in portions. The reaction mixture was stirred for an additional 16 h at 25 °C then quenched with sat. NH₄Cl (aq.). The resulting mixture was extracted with CH₂Cl₂ (3 x 50 mL). The combined organic layers were washed with brine (2 x 50 mL), dried over anhydrous Na₂SO₄. After filtration, the filtrate was concentrated under reduced pressure. The residue was purified by silica gel column chromatography, eluted with PE / EA (5:1) to afford *tert-*butyl (S)-2-(1-methoxy-4-methyl-1-oxopentan-2-yl)-1-methylhydrazine-1-carboxylate (5.3 g, 53 % yield) as a light yellow oil. LCMS (ESI): m/z [M+H] calc'd for C₁₃H₂₆N₂O₄ 274.2; found 219.1 [M-56+H]⁺.

### Step 3

To a stirred solution of *tert*-butyl (S)-2-(1-methoxy-4-methyl-1-oxopentan-2-yl)-1-methylhydrazine-1-carboxylate (5.3 g, 19.31 mmol, 1 equiv) in 1,4-dioxane (53 mL) was added HCl(gas) in 1,4-dioxane (50 mL, 200.00 mmol, 10.35 equiv) dropwise at 25 °C. The resulting mixture was stirred for 16 h at 25 °C then concentrated under reduced pressure to afford methyl (methylamino)-*L*-leucinate (3.6 g, crude) as a yellow solid. The crude product was used in the next step directly without further purification. LCMS (ESI): m/z [M+H] calc'd for C₈H₁₈N₂O₂ 174.1; Found 175.2.

### Analog Example 25. methyl 3-cyclopentyl-2-(2-methylhydrazineyl)propanoate

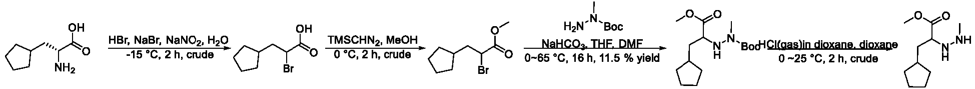

### Step 1

A solution of NaBr (9.61 g, 93.39 mmol, 3.67 equiv) in HBr (40 mL, 0.75mol/L, 7.500 mmol) was treated with NaNO₂ (2.28 g, 33.07 mmol, 1.30 equiv) for 5 min at -15°C followed by the addition of (*R*)-2-amino-3-cyclopentylpropanoic acid (4 g, 25.44 mmol, 1 equiv) in portions at -15°C. The resulting mixture was stirred for an additional 2 h then acidified to pH 3 with HCl (1 M). The aqueous layer was extracted with EtOAc (3 x 25 mL). The combined organic layers were dried with Na₂SO₄ and concentrated under reduced pressure. This resulted in 2-bromo-3-cyclopentylpropanoic acid (4.7 g, crude) as a yellow oil. The crude product was used in the next step directly without further purification.

### Step 2

To a solution of 2-bromo-3-cyclopentylpropanoic acid (4.7 g, 21.25 mmol, 1 equiv) in MeOH (50 mL) was added TMSCHN₂ (53.14 mL, 106.29 mmol, 5 equiv) at 0 °C. The resulting mixture was stirred for 2 h then concentrated under reduced pressure to afford methyl 2-bromo-3-cyclopentylpropanoate (4.2 g, crude) as a yellow oil. The crude product was used in the next step directly without further purification.

### Step 3

To a 250 mL vial was added tert-butyl 1-methylhydrazine-1-carboxylate (2.4 mL, 16.17 mmol, 1.00 equiv), NaHCO₃ (3.50 g, 41.66 mmol, 2.58 equiv), methyl 2-bromo-3-cyclopentylpropanoate (4.49 g, 19.08 mmol, 1.18 equiv), THF (22 mL), and DMF (22 mL) at 0 °C. The resulting mixture was stirred for an additional 16 h at 65 °C then quenched with sat. NH₄Cl (aq.). The aqueous layer was extracted with EtOAc (3 x 50 mL). The combined organics were washed with water (3 x 25 mL) then concentrated under reduced pressure. The residue was purified by silica gel column chromatography, eluted with PE / EA (9:1) to afford *tert*-butyl 2-(3-cyclopentyl-1-methoxy-1-oxopropan-2-yl)-1-methylhydrazine-1-carboxylate (560 mg, 11.5% yield) as a colorless oil. LCMS (ESI): m/z [M+H] calc'd for C₁₅H₂₈N₂O₄ 300.2; found 245.1 [M-56+H]⁺.

### Step 4

A solution of *tert*-butyl 2-(3-cyclopentyl-1-methoxy-1-oxopropan-2-yl)-1-methylhydrazine-1-carboxylate (300 mg, 0.999 mmol, 1 equiv) in dioxane (3 mL) was treated with HCl(gas) in dioxane (3 mL, 12.000 mmol, 12.02 equiv) at 0 °C. The resulting mixture was stirred for 2 h then concentrated under reduced pressure to afford methyl 3-cyclopentyl-2-(2-methylhydrazineyl)propanoate (270 mg, crude) as a light yellow oil. The crude product was used in the next step directly without further purification. LCMS (ESI): m/z [M+H] calc'd for C₁₀H₂₀N₂O₂ 200.2; Found 201.2.

### Analog Example 26. ethyl (methylamino)valinate

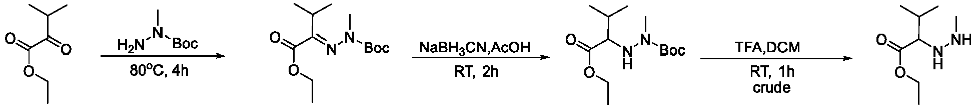

### Step 1

To a 40 mL vial was added ethyl 3-methyl-2-oxobutanoate (5 g, 34.68 mmol, 1 equiv) and tert-butyl 1-methylhydrazine-1-carboxylate (5.069 g, 34.67 mmol, 1.00 equiv) at 25 °C. The resulting mixture was stirred for 4 h at 80 °C. This resulted in tert-butyl (E)-2-(1-ethoxy-3-methyl-1-oxobutan-2-ylidene)-1-methylhydrazine-1-carboxylate (9.36 g, 84% yield) as a yellow oil. LCMS (ESI): m/z [M+H] calc'd for C₁₃H₂₄N₂O₄ 272.2; found 273.3.

### Step 2

To a 250 mL round-bottom flask was added *tert-*butyl (*E*)-2-(1-ethoxy-3-methyl-1-oxobutan-2-ylidene)-1-methylhydrazine-1-carboxylate (4.34 g, 15.93 mmol, 1 equiv), AcOH (45 mL) and NaBH₃CN (2.47 g, 39.30 mmol, 2.47 equiv) at 0 °C. The resulting mixture was stirred for 2 h at 25 °C then quenched with water (100 mL) and extracted with EtOAc (3 x 100 mL). The combined organic layers were washed with water (2 x 100 mL), dried over anhydrous Na₂SO₄. After filtration, the filtrate was concentrated under reduced pressure. The crude product was purified by reverse phase flash with the following conditions (column, C₁₈ silica gel; mobile phase, ACN in Water (10 mmol/L NH₄HCO₃), 5% to 40% gradient in 5 min, 40% to 70% gradient in 30 min; detector, UV 220 nm) to afford tert-butyl 2-(1-ethoxy-3-methyl-1-oxobutan-2-yl)-1-methylhydrazine-1-carboxylate (3.267 g, 74% yield) as a yellow oil. LCMS (ESI): m/z [M+H] calc'd for C₁₃H₂₆N₂O₄ 274.1; found 275.1.

### Step 3

To a 100 mL round-bottom flask was added *tert*-butyl 2-(1-ethoxy-3-methyl-1-oxobutan-2-yl)-1-methylhydrazine-1-carboxylate (1 g, 3.64 mmol, 1 equiv), DCM (7.5 mL) and TFA (2.5 mL) at 0 °C. The resulting mixture was stirred for 1 h at 25 °C then concentrated under vacuum to afford ethyl (methylamino)valinate (1.5 g, crude) as a yellow oil. LCMS (ESI): m/z [M+H] calc'd for C₈H₁₈N₂O₂ 174.1; found 175.3.

### Analog Example 27. ethyl 4-(trifluoromethyl)hexahydropyridazine-3-carboxylate

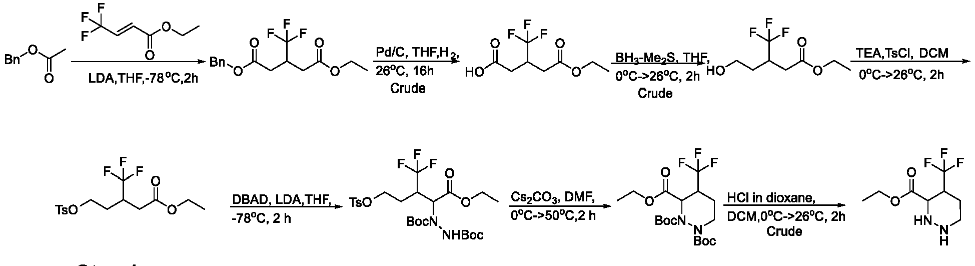

### Step 1

To a stirred mixture of benzyl acetate (35.73 g, 237.93 mmol, 2 equiv) in THF (200 mL) was added LDA (57.10 mL, 142.75 mmol, 1.2 equiv) dropwise at -78 °C under argon atmosphere. The resulting mixture was stirred for 1 h at 25 °C. Then the solution was cooled to -78 °C and ethyl (2*Z*)-4,4,4-trifluorobut-2-enoate (20 g, 118.96 mmol, 1 equiv) was added. The resulting mixture was stirred for 1 h at 26 °C then quenched by the addition of sat. NH₄Cl (aq.) (500 mL). The aqueous layer was extracted with EtOAc (3 x 500 mL). The combined organics were concentrated under vacuum. The residue was purified by silica gel column chromatography, eluted with PE / EA (1:1) to afford 1-benzyl 5-ethyl 3-(trifluoromethyl)pentanedioate (20.3 g, 50% yield) as an off-white oil. LCMS (ESI): m/z [M+H] calc'd for C₁₅H₁₇F₃O₄ 318.1; found 319.1.

### Step 2

To a solution of 1-benzyl 5-ethyl 3-(trifluoromethyl)pentanedioate (10.6 g, 33.30 mmol, 1 equiv) in THF (120 mL) was added Pd/C (5 g, 46.984 mmol, 1.41 equiv) in a pressure tank. The mixture was hydrogenated at 26 °C under 30 psi of hydrogen pressure for 16 h then filtered through a Celite pad and concentrated under reduced pressure. The crude product (7.3 g, crude) was used in the next step directly without further purification. LCMS (ESI): m/z [M+H] calc'd for C₈H₁₁F₃O₄ 228.1; found 229.1.

### Step 3

A mixture of 5-ethoxy-5-oxo-3-(trifluoromethyl)pentanoic acid (7.3 g, 31.99 mmol, 1 equiv) and BH₃-Me₂S (12.15 g, 159.97 mmol, 5 equiv) in THF (100 mL) was stirred for 2 h at 26 °C under an argon atmosphere. The reaction was then cooled to 0 °C and quenched with MeOH (30 mL). The resulting mixture was concentrated under reduced pressure to obtain ethyl 5-hydroxy-3-(trifluoromethyl)pentanoate (5.1g, crude). The crude product was used in the next step directly without further purification. LCMS (ESI): m/z [M+H] calc'd for C₈H₁₃F₃O₃ 214.1; found 215.1.

### Step 4

To a solution of ethyl 5-hydroxy-3-(trifluoromethyl)pentanoate (5.1 g, 23.81 mmol, 1 equiv) and TEA (7.23 g, 71.43 mmol, 3 equiv) in DCM (50 mL) was added TsCl (6.81 g, 35.716 mmol, 1.5 equiv). The reaction was stirred for 2 h at 26 °C under an argon atmosphere. The reaction was cooled to 0 °C and quenched by the addition of sat. NH₄Cl (aq.) (200mL). The aqueous layer was extracted with EtOAc (3 x 300 mL). The resulting mixture was concentrated under reduced pressure. The residue was purified by silica gel column chromatography, eluted with PE / EA (1:1) to afford ethyl 5-[(4-methylbenzenesulfonyl)oxy]-3-(trifluoromethyl)pentanoate (4.4 g, 53% yield) as an off-white oil. LCMS (ESI): m/z [M+H] calc'd for C₁₅H₁₉F₃O₅S 368.1; found 369.1.

### Step 5

To a solution of ethyl-5-(tosyloxy)-3-(trifluoromethyl)pentanoate (5.5 g, 14.93 mmol, 1 equiv) in THF (70 mL) at -78 °C was added LDA (8.96 mL, 22.39 mmol, 1.5 equiv) dropwise under argon atmosphere. The resulting mixture was stirred for 1 h then DBAD (5.16 g, 22.396 mmol, 1.5 equiv) was added dropwise over 10 min. The reaction mixture was stirred for an additional 1 h then quenched by the addition of sat. NH₄Cl (aq.) (300 mL). The aqueous layer was extracted with EtOAc (3 x 300 mL). The combined organics were concentrated under reduced pressure. The residue was purified by silica gel column chromatography, eluted with PE / EA (1:1) to afford di-*tert*-butyl1-(1-ethoxy-1-oxo-5-(tosyloxy)-3-(trifluoromethyl)pentan-2-yl)hydrazine-1,2-dicarboxylate (4.4 g, 49.23%yield) as a light yellow oil. LCMS (ESI): m/z [M+H] calc'd for C₂₅H₃₇F₃N₂O₉S 598.2; found 621.1 [M+Na]⁺.

### Step 6

A solution of di-*tert*-butyl1-(1-ethoxy-1-oxo-5-(tosyloxy)-3-(trifluoromethyl)pentan-2-yl)hydrazine-1,2-dicarboxylate (4.4 g, 7.350 mmol, 1 equiv) and Cs₂CO₃ (11.97 g, 36.750 mmol, 5 equiv) in DMF (440 mL) was stirred for 2 h at 50 °C under argon atmosphere. The reaction was then cooled to 0 °C and quenched by the addition of sat. NH₄Cl (aq.) (200 mL). The aqueous layer was extracted with EtOAc (3 x 300 mL). The combined organics were concentrated under reduced pressure. The residue was purified by silica gel column chromatography, eluted with PE / EA (1:1) to afford 1,2-di-tert-butyl 3-ethyl-4-(trifluoromethyl)tetrahydropyridazine-1,2,3-tricarboxylate (2.1 g, 67% yield) as a light yellow oil. LCMS (ESI): m/z [M+H] calc'd for C₁₈H₂₉F₃N₂O₆ 426.2; found 449.2 [M+Na]⁺.

### Step 7

To a solution 1,2-di-tert-butyl 3-ethyl 4-(trifluoromethyl)-1,2-diazinane-1,2,3-tricarboxylate (2.1 g, 4.92 mmol, 1 equiv) in DCM (20 mL) was added 4M HCl (gas) in 1,4-dioxane (10 mL) and the reaction stirred for 2 h at 26 °C under argon atmosphere. The resulting mixture was then concentrated under reduced pressure. The crude product (1.5 g, crude) was used in the next step directly without further purification. LCMS (ESI): m/z [M+H] calc'd for C₈H₁₃F₃N₂O₂ 226.1; found 227.2.

### Synthesis of (S)-3-bromo-5-iodo-2- (1-methoxyethyl) pyridine - used in preparation of Exemplary Intermediate 1 (Scheme 1)

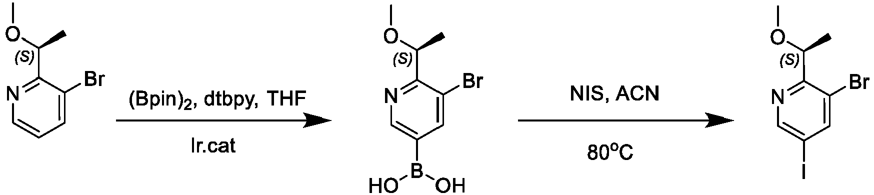

### Step 1

To a stirred solution of 3-bromo-2-[(1*S*)-1-methoxyethyl]pyridine (80.00 g, 370.24 mmol, 1.00 equiv) and bis(pinacolato)diboron (141.03 g, 555.3 mmol, 1.50 equiv) in THF (320 mL) was added dtbpy (14.91 g, 55.5 mmol) and Chloro(1,5-cyclooctadiene)iridium(I) dimer (7.46 g, 11.1 mmol) under argon atmosphere. The resulting mixture was stirred for 16 h at 75 °C under argon atmosphere. The mixture was concentrated under reduced pressure. The resulting mixture was dissolved in EtOAc (200 mL) and the mixture was adjusted to pH 10 with Na₂CO₃ (40 g) and NaOH (10 g) (mass 4:1) in water (600 mL). The aqueous layer was extracted with EtOAc (800mL). The aqueous phase was acidified to pH = 6 with HCl (6 *N*) to precipitate the desired solid to afford 5-bromo-6-[(1*S*)-1-methoxyethyl]pyridin-3-ylboronic acid (50g, 52.0%yield) as a light-yellow solid. ICMS (ESI): m/z [M+H] calc'd for C₈H₁₁BBrNO₃ 259.0; found 260.0.

### Step 2

To a stirred solution of 5-bromo-6-[(1*S*)-1-methoxyethyl]pyridin-3-ylboronic acid (23.00 g, 88.5 mmol) in ACN (230 mL) were added NIS (49.78 g, 221.2 mmol) at room temperature under argon atmosphere. The resulting mixture was stirred for overnight at 80 °C under argon atmosphere. The resulting mixture was concentrated under reduced pressure. The resulting mixture was dissolved in DCM (2.1 L) and washed with Na₂S₂O₃ (3 x 500 mL). The organic layer was dried over anhydrous Na₂SO₄. After filtration, the filtrate was concentrated under reduced pressure. The residue was purified by silica gel column chromatography to afford (S)-3-bromo-5-iodo-2-(1-methoxyethyl)pyridine (20 g, 66.0%yield). LCMS (ESI): m/z [M+H] calc'd for C₈H₉BrINO 340.9; found 341.7.

### Synthesis of 5-bromo-3-(3-((tert-butyldiphenylsilyl)oxy)-2,2-dimethylpropyl)-2-iodo-1H-indole - used in preparation of Exemplary Intermediate 1 (Scheme 1)

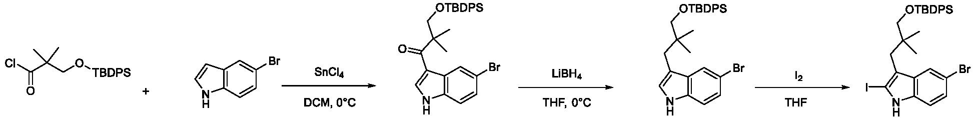

### Step 1

To a mixture of 3-((*tert*-butyldiphenylsilyl)oxy)-2,2-dimethylpropanoyl chloride (65 g, 137 mmol, crude) in DCM (120 mL) at 0 °C under an atmosphere of nitrogen was added 1M SnCl₄ in DCM (137 mL, 137 mmol) slowly. The mixture was stirred at 0 °C for 30 min, then a solution of 5-bromo-1*H-*indole (26.8 g, 137 mmol) in DCM (40 mL) was added dropwise. The mixture was stirred at 0 °C for 45 min, then diluted with EtOAc (300 mL), washed with brine (100 mL x 4), dried over Na₂SO₄ and filtered. The filtrate was concentrated under reduced pressure and the residue was purified by silica gel column chromatography to give 1-(5-bromo-1*H*-indol-3-yl)-3-((*tert*-butyldiphenylsilyl)oxy)-2,2-dimethylpropan-1-one (55 g, 75% yield). LCMS (ESI): m/z [M+Na] calc'd for C₂₉H₃₂BrNO₂SiNa 556.1; found 556.3.

### Step 2

To a mixture of 1-(5-bromo-1*H*-indol-3-yl)-3-((*tert*-butyldiphenylsilyl)oxy)-2,2-dimethylpropan-1-one (50 g, 93.6 mmol) in THF (100 mL) at 0 °C under an atmosphere of nitrogen was added LiBH₄ (6.1 g, 281 mmol). The mixture was heated to 60 °C and stirred for 20 h, then MeOH (10 mL) and EtOAc (100 mL) was added and the mixture washed with brine (50 mL), dried over Na₂SO₄, filtered and the filtrate concentrated under reduced pressure. The residue was diluted with DCM (50 mL), cooled to 10 °C and diludine (9.5 g, 37.4 mmol) and TsOH.H₂O (890 mg, 4.7 mmol) added. The mixture was stirred at 10 °C for 2 h, filtered, then the filtrate concentrated under reduced pressure and the residue was purified by silica gel column chromatography to give 1-(5-bromo-1*H*-indol-3-yl)-3-((*tert*-butyldiphenylsilyl)oxy)-2,2-dimethylpropan-1-one (41 g, 84% yield). LCMS (ESI): m/z [M+H] calc'd for C₂₉H₃₄BrNOSi 519.2; found 520.1; ¹H NMR (400 MHz, CDCl₃) δ 7.96 (s, 1H), 7.75 - 7.68 (m, 5H), 7.46 - 7.35 (m, 6H), 7.23 - 7.19 (m, 2H), 6.87 (d, *J* = 2.1 Hz, 1H), 3.40 (s, 2H), 2.72 (s, 2H), 1.14 (s, 9H), 0.89 (s, 6H).

### Step 3

To a mixture of 1-(5-bromo-1*H*-indol-3-yl)-3-((*tert*-butyldiphenylsilyl)oxy)-2,2-dimethylpropan-1-one (1.5 g, 2.9 mmol) and I₂ (731 mg, 2.9 mmol) in THF (15 mL) at room temperature was added AgOTf (888 mg, 3.5 mmol). The mixture was stirred at rt for 2 h, then diluted with EtOAc (200 mL) and washed with saturated Na₂S₂O₃ (100 mL), then dried over anhydrous Na₂SO₄ and filtered. The filtrate was concentrated under reduced pressure and the residue was purified by silica gel column chromatography to give 5-bromo-3-(3-((tert-butyldiphenylsilyl)oxy)-2,2-dimethylpropyl)-2-iodo-1H-indole (900 mg, 72% yield) as a solid. ¹H NMR (400 MHz, DMSO-*d*₆) δ 11.70 (s, 1H), 7.68 (d, *J* = 1.3 Hz, 1H), 7.64 - 7.62 (m, 4H), 7.46 - 7.43 (m, 6H), 7.24 - 7.22 (d, 1H), 7.14 - 7.12 (dd, *J* = 8.6, 1.6 Hz, 1H), 3.48 (s, 2H), 2.63 (s, 2H), 1.08 (s, 9H), 0.88 (s, 6H).

### Exemplary Intermediate 1 (Scheme 1). Synthesis of tert-butyl ((6³S,4S,Z)-11-ethyl-1²-(2-((S)-1-methoxyethyl)-5-(4-methylpiperazin-1-yl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)carbamate

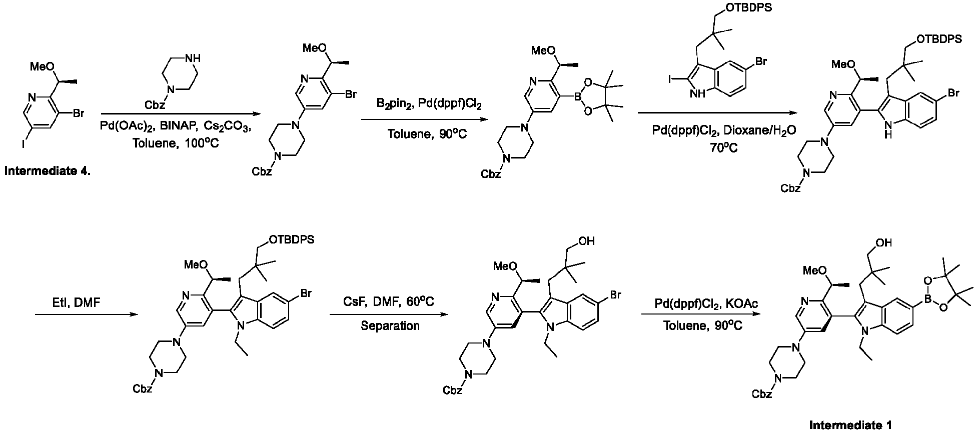

### Step 1

To a 3L 3-necked round-bottom flask purged and maintained with an inert atmosphere of argon, was added 3-bromo-5-iodo-2-[(1*S*)-1-methoxyethyl]pyridine (147 g, 429.8 mmol) (see above) benzyl piperazine-1-carboxylate (94.69 g, 429.8 mmol), Pd(OAc)₂ (4.83 g, 21.4 mmol), BINAP (5.35 g, 8.6 mmol), Cs₂CO₃ (350.14 g, 1074.6 mmol), and toluene (1 L). The resulting solution was stirred overnight at 100 °C. The reaction mixture was cooled to 25 °C then concentrated under reduced pressure. The residue was purified by silica gel column to afford benzyl (S)-4-(5-bromo-6-(1-methoxyethyl)pyridin-3-yl)piperazine-1-carboxylate (135 g, 65.1% yield) as a dark yellow solid. LCMS (ESI): m/z [M+H] calc'd for C₂₀H₂₄BrN₃O₃ 433.1; found 434.1.

### Step 2

To a 3-L 3-necked round-bottom flask purged and maintained with an inert atmosphere of argon, was added benzyl 4-[5-bromo-6-[(1*S*)-1-methoxyethyl]pyridin-3-yl]piperazine-1-carboxylate (135 g, 310.8 mmol), bis(pinacolato)diboron (86.82 g, 341.9 mmol), Pd(dppf)Cl₂ (22.74 g, 31.0 mmol), KOAc (76.26 g, 777.5 mmol), and Toluene (1 L). The resulting solution was stirred for 2 days at 90 °C. The reaction mixture was cooled to 25 °C then concentrated under reduced pressure. The residue was purified by neutral alumina column to afford benzyl (*S*)-4-(6-(1-methoxyethyl)-5-(4,4,5,5-tetramethyl-1,3,2-dioxaborolan-2-yl)pyridin-3-yl)piperazine-1-carboxylate (167 g, crude) as a dark yellow solid. LCMS (ESI): m/z [M+H] calc'd for C₂₆H₃₆BN₃O₅ 481.3; found 482.1.

### Step 3

To a 3-L 3-necked round-bottom flask purged and maintained with an inert atmosphere of argon, was added (*S*)-4-(6-(1-methoxyethyl)-5-(4,4,5,5-tetramethyl-1,3,2-dioxaborolan-2-yl)pyridin-3-yl)piperazine-1-carboxylate (167 g, 346.9 mmol), 5-bromo-3-[3-[(*tert*-butyldiphenylsilyl)oxy]-2,2-dimethylpropyl]-2-iodo-1H-indole (224.27 g, 346.9 mmol) (see above), Pd(dppf)Cl₂ (25.38 g, 34.6 mmol), dioxane (600 mL), H₂O (200 mL), K₃PO₄ (184.09 g, 867.2 mmol), and Toluene (200 mL). The resulting solution was stirred overnight at 70 °C in an oil bath. The reaction mixture was cooled to 25 °C then concentrated under reduced pressure. The residue was purified by silica gel column to afford benzyl (*S*)-4-(5-(5-bromo-3-(3-((*tert*-butyldiphenylsilyl)oxy)-2,2-dimethylpropyl)-1*H*-indol-2-yl)-6-(1-methoxyethyl)pyridin-3-yl)piperazine-1-carboxylate (146 g, 48.1% yield) as a yellow solid. LCMS (ESI): m/z [M+H] calc'd for C₄₉H₅₇BrN₄O₄Si 872.3; found 873.3.

### Step 4

To a stirred solution of benzyl (*S*)-4-(5-(5-bromo-3-(3-((*tert*-butyldiphenylsilyl)oxy)-2,2-dimethylpropyl)-1*H*-indol-2-yl)-6-(1-methoxyethyl)pyridin-3-yl)piperazine-1-carboxylate (146 g, 167.0 mmol) and Cs₂CO₃ (163.28 g, 501.1 mmol) in DMF (1200 mL) was added C₂H₅I (52.11 g, 334.0 mmol) in portions at 0 °C under nitrogen atmosphere then stirred at 25 °C for 12 h. The resulting mixture was diluted with EA (1 L) and washed with brine (3 x 1.5L). The organic layers were dried over anhydrous Na₂SO₄. After filtration, the filtrate was concentrated under reduced pressure to give benzyl (*S*)-4-(5-(5-bromo-3-(3-((*tert*-butyldiphenylsilyl)oxy)-2,2-dimethylpropyl)-1-ethyl-1*H*-indol-2-yl)-6-(1-methoxyethyl)pyridin-3-yl)piperazine-1-carboxylate (143 g, crude) as a yellow solid that was used directly for next step without further purification. LCMS (ESI): m/z [M+H] calc'd for C₅₁H₆₁BrN₄O₄Si 900.4; found 901.4.

### Step 5

To a stirred solution of benzyl (*S*)-4-(5-(5-bromo-3-(3-((*tert*-butyldiphenylsilyl)oxy)-2,2-dimethylpropyl)-1-ethyl-1*H*-indol-2-yl)-6-(1-methoxyethyl)pyridin-3-yl)piperazine-1-carboxylate (143 g, 158.5 mmol) in DMF (1250 mL) was added CsF (72.24 g, 475.5 mmol). The reaction mixture was stirred at 60 °C for 2 days under nitrogen atmosphere. The resulting mixture was diluted with EA (1 L) and washed with brine (3 x 1L). Then the organic phase was concentrated under reduced pressure. The residue was purified by silica gel column chromatography to afford two atropisomers of benzyl (S)-4-(5-(5-bromo-1-ethyl-3-(3-hydroxy-2,2-dimethylpropyl)-1*H*-indol-2-yl)-6-(1-methoxyethyl)pyridin-3-yl)piperazine-1-carboxylate A (38 g, 36% yield, RT = 1.677 min in 3 min LCMS(0.1% FA)) and **B** (34 g, 34% yield, RT = 1.578 min in 3 min LCMS(0.1% FA)) both as yellow solid. LCMS (ESI): m/z [M+H] calc'd for C₃₅H₄₃BrN₄O₄ 663.2; found 662.2.

### Step 6

To a 500-mL 3-necked round-bottom flask purged and maintained with an inert atmosphere of nitrogen, was placed benzyl (*S*)-4-(5-(5-bromo-1-ethyl-3-(3-hydroxy-2,2-dimethylpropyl)-1*H*-indol-2-yl)-6-(1-methoxyethyl)pyridin-3-yl)piperazine-1-carboxylate (14 g, 21.1 mmol), bis(pinacolato)diboron (5.89 g, 23.21 mmol), Pd(dppf)Cl₂ (1.54 g, 2.1 mmol), KOAc (5.18 g, 52.7 mmol), and Toluene (150 mL). The resulting solution was stirred for 5 h at 90 °C. The reaction mixture was cooled to 25 °C and concentrated under reduced pressure. The residue was purified by silica gel column chromatography to afford benzyl (*S*)-4-(5-(1-ethyl-3-(3-hydroxy-2,2-dimethylpropyl)-5-(4,4,5,5-tetramethyl-1,3,2-dioxaborolan-2-yl)-1*H*-indol-2-yl)-6-(1-methoxyethyl)pyridin-3-yl)piperazine-1-carboxylate (12 g, 76.0% yield) as a yellow solid. LCMS (ESI): m/z [M+H] calc'd for C₄₁H₅₅BN₄O₆ 710.4; found 711.3.

### Exemplary Intermediate 2 (Scheme 1). Ethyl (S)-2-((tert-butoxycarbonyl)amino)-3-((S)-morpholin-2-yl)propanoate

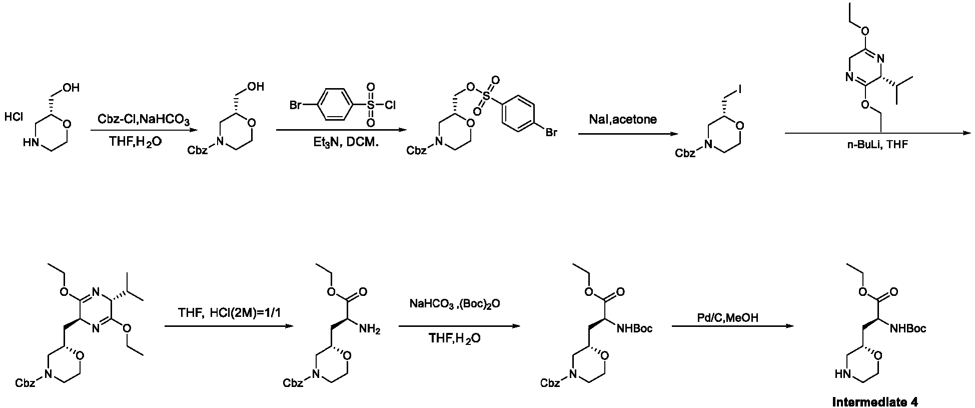

### Step 1

To a round-bottom flask was added (2*R*)-morpholin-2-ylmethanol hydrochloride (350 g, 2278.49 mmol, 1.00 equiv), THF (1.75 L), H₂O (1.75 L) and NaHCO₃ (478.8 g, 5700.00 mmol, 2.50 equiv). The resulting solution was stirred for 30 min at 25 °C. The mixture was then cooled to 5 °C and CbzCl (387.6 g, 2272.07 mmol, 1.00 equiv) was added dropwise. The resulting solution was stirred for 2 h at 25 °C. then extracted with EA (2 x 5 L). The combined organics were washed with brine (2 x 7 L). The mixture was dried over anhydrous Na₂SO₄ and concentrated to afford benzyl (2*R*)-2-(hydroxymethyl)morpholine-4-carboxylate (520 g, 90.8%) of as a light yellow liquid. LCMS (ESI): m/z [M+H] calc'd for C₁₃H₁₇NO₄ 251.1; found 252.1.

### Step 2

To a round-bottom flask was added benzyl (2*R*)-2-(hydroxymethyl)morpholine-4-carboxylate (520 g, 2069.38 mmol, 1.00 equiv), DCM (5.2 L) and Et₃N (418.5 g, 4135.78 mmol, 2.00 equiv) at 25 °C and the mixture cooled to 0 °C. To the above mixture was added 4-bromobenzenesulfonyl chloride (528.3 g, 2067.62 mmol, 1.00 equiv) in portions. The resulting solution was stirred overnight at 25 °C, then washed with H₂O (3 x 5 L), brine (2 x 5 L) then dried over anhydrous NaₛSO₄. Concentration under reduced pressure afforded ethyl 4-chloro-5-methyl-1*H*-pyrazole-3-carboxylate (850 g, 87.6%) of as a light yellow oil. LCMS (ESI): m/z [M+H] calc'd for C₁₉H₂₀BrNO₆ 469.0; found 470.0.

### Step 3

To a round-bottom flask was added benzyl (2*R*)-2-{[(4-bromobenzenesulfonyl)oxy]methyl}morpholine-4-carboxylate (850 g, 1807.24 mmol, 1.00 equiv), acetone (8.5 L) and NaI (541.79 g, 3614.48 mmol, 2.00 equiv) at 25 °C. The resulting solution was stirred for 16 h at 60 °C then concentrated under reduced pressure. This was diluted with EA (8.0 L). The mixture was washed with H₂O (3 x 8 L) and brine (3 x 8 L) then dried over anhydrous Na₂SO₄ and concentrated under reduced pressure. This resulted in benzyl (2*R*)-2-(iodomethyl) morpholine-4-carboxylate (560 g, 85.8%) as a yellow liquid. LCMS (ESI): m/z [M+H] calc'd for C₁₃H₁₆INO₃ 361.0; found 362.0.

### Step 4

To a round-bottom flask was added benzyl (*S*)-2-(((2*S*,5*R*)-3,6-diethoxy-5-isopropyl-2,5-dihydropyrazin-2-yl)methyl)morpholine-4-carboxylate (300 g, 1413.04 mmol, 1.00 equiv) and THF (2.6 L) at room temperature, then the solution cooled down to -78 °C. To the above mixture was added n-BuLi (0.678 L, 1695.00 mmol, 1.20 equiv) dropwise. The resulting mixture was stirred for additional 1.5 h at -78 °C. To the above solution was added benzyl (2*R*)-2-(iodomethyl)morpholine-4-carboxylate (561 g, 1554.47 mmol, 1.10 equiv) dropwise. The resulting mixture was stirred for additional 2 h at room temperature. The reaction was quenched with sat. NH₄Cl (2 L) at 0 °C. The aqueous layer was extracted with EtOAc (3 x 1 L). The combined organics were washed with H₂O (3 x 3 L) and brine (3 x 3 L). The crude mixture was dried over anhydrous Na₂SO₄ and concentrated under reduced pressure. This resulted benzyl (2*S*)-2-[[(2*S*,5*R*)-3,6-diethoxy-5-isopropyl-2,5-dihydropyrazin-2-yl]methyl]morpholine-4-carboxylate in (380 g, 60.35%) of as a yellow solid. LCMS (ESI): m/z [M+H] calc'd for C₂₄H₃₅N₃O₅ 445.3; found 446.3.

### Step 5

To a round-bottom flask was added benzyl (2*S*)-2-[[(2*S*,5*R*)-3,6-diethoxy-5-isopropyl-2,5-dihydropyrazin-2-yl]methyl]morpholine-4-carboxylate (380 g, 852.86 mmol, 1.00 equiv) , THF (1.9 L) and HCl (2M,1.9 L) at room temperature. The resulting solution was stirred for 2 h. The mixture was then washed with EtOAc (1 x 2 L). The organic layer was extracted with H₂O (1 x 5 L) and the water layers combined. The water layer was neutralized to pH 7 with saturated NaHCO₃ (aq.). The aqueous layer was extracted with EtOAc (3 x 3 L). The resulting mixture was washed with brine (2 x 5 L), dried over anhydrous Na₂SO₄ and concentrated under reduced pressure. This resulted in benzyl (2S)-2-[(2S)-2-amino-3-ethoxy-3-oxopropyl]morpholine-4-carboxylate (200 g, 69.71%) as a yellow oil. LCMS (ESI): m/z [M+H] calc'd for C₁₇H₂₄N₂O₅ 336.2; found 337.2.

### Step 6

To a round-bottom flask was added benzyl (2*S*)-2-[(2*S*)-2-amino-3-ethoxy-3-oxopropyl]morpholine-4-carboxylate (200 g, 594.55 mmol, 1.00 equiv) THF (200 mL), H₂O (1.9 L), NaHCO₃ (99.89 g, 1189.10 mmol, 2 equiv) and Boc₂O (389.28 g, 1783.67 mmol, 3 equiv) at room temperature. The resulting solution was stirred overnight at room temperature. After quenching with H₂O, the aqueous layer was extracted with EA (2 x 2 L). The combined organic layers were washed with H₂O (2 x 5 L) of water and brine (2 x 5 L) of brine. The crude mixture was dried over anhydrous sodium sulfate and concentrated. The residue was purified by silica gel column chromatography to afford benzyl (2*S*)-2-[(2*S*)-2-[(*tert*-butoxycarbonyl)amino]-3-ethoxy-3-oxopropyl]morpholine-4-carboxylate (200 g, 77.06%) as a white solid. LCMS (ESI): m/z [M+H] calc'd for C₂₂H₃₂N₂O₇ 436.2; found 437.2.

### Step 7

To a round-bottom flask was added benzyl (2*S*)-2-[(2*S*)-2-[(*tert*-butoxycarbonyl)amino]-3-ethoxy-3-oxopropyl]morpholine-4-carboxylate (200 g, 458.19 mmol, 1.00 equiv), MeOH (1 L) and Pd/C (48.76 g, 45.82 mmol, 0.10 equiv, 10%) at room temperature. The resulting mixture was stirred overnight at room temperature under a hydrogen atmosphere then filtered, the filter cake was washed with MeOH (3 x 1 L). The filtrate was concentrated under reduced pressure. This resulted in ethyl (2*S*)-2-[(*tert*-butoxycarbonyl)amino]-3-[(2S)-morpholin-2-yl]propanoate (140 g, crude) as a yellow oil. LCMS (ESI): m/z [M+H] calc'd for C₁₄H₂₆N₂O₅ 302.2; found 303.2.

### N-(1-(4-(dimethylamino)-4-methylpent-2-ynoyl)-4-fluoropiperidine-4-carbonyl)-N-methyl-L-valine - used in various Examples below

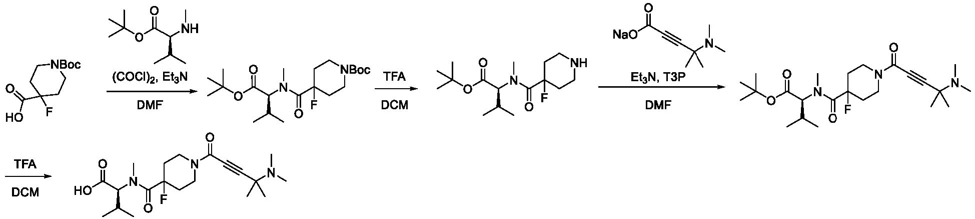

### Step 1

To a mixture of 1-[(*tert*-butoxy)carbonyl]-4-fluoropiperidine-4-carboxylic acid (2.0 g, 8.1 mmol) in DCM (20 mL) was added oxalic dichloride (1.34 g, 10.5 mmol) and DMF (30 mg, 0.4 mmol). The resulting solution was stirred at room temperature for 1 h. Et₃N (3.2 g, 3.2 mmol) and (2S)-3-methyl-2-(methylamino)butanoic acid (1.25 g, 9.5 mmol) were added and the mixture was stirred at room temperature for 1 h. H₂O (100 mL) was added and the mixture was extracted with EtOAc (50 mL x 3). The combined organic layers were concentrated under reduced pressure and the residue was purified by silica gel column chromatography to give *tert*-butyl (*S*)-4-((1-(*tert*-butoxy)-3-methyl-1-oxobutan-2-yl)(methyl)carbamoyl)-4-fluoropiperidine-1-carboxylate (1.34 g, 45% yield) as a solid. LCMS (ESI): m/z [M+Na] calc'd for C₂₁H₃₇FN₂O₅Na 439.3; found 439.3

### Step 2

A mixture of *tert*-butyl (*S*)-4-((1-(*tert*-butoxy)-3-methyl-1-oxobutan-2-yl)(methyl)carbamoyl)-4-fluoropiperidine-1-carboxylate (290 mg, 0.70 mmol) in DCM (4 mL) and TFA (2 mL) was stirred at room temperature for 2 h, then concentrated under reduced pressure to give *N*-(4-fluoropiperidine-4-carbonyl)-*N*-methyl-*L*-valine, which was used directly in the next step without further purification. LCMS (ESI): m/z [M+H] calc'd for C₁₂H₂₁FN₂O₃ 260.2; found 261.2.

### Step 3

To a solution of the *tert-*butyl *N*-(4-fluoropiperidine-4-carbonyl)-*N*-methyl-L-valinate (1.7 g, 5.3 mmol), sodium 4-(dimethylamino)-4-methylpent-2-ynoate (1.67 g, 9.4 mmol) and Et₃N (2.73 g, 36.9 mmol) in DMF (20 mL) stirred at 5 °C was added T3P (4.11 g, 10.7 mmol, 50wt% in EtOAc). The reaction mixture was stirred at 5 °C for 1 h. The resulting mixture was quenched with H₂O (100 mL) and extracted with EtOAc (50 mL x 3). The combined organic layers were concentrated and purified by silica gel column chromatography to give *tert*-butyl *N*-(1-(4-(dimethylamino)-4-methylpent-2-ynoyl)-4-fluoropiperidine-4-carbonyl)-*N*-methyl-L-valinate (1.6 g, 74.0% yield) as a solid. LCMS (ESI): m/z [M+H] calc'd for C₂₄H₄₀FN₃O₄ 453.3; found 454.2.

### Step 4

To a solution of *tert*-butyl *N*-(1-(4-(dimethylamino)-4-methylpent-2-ynoyl)-4-fluoropiperidine-4-carbonyl)-*N*-methyl-L-valinate (50 mg, 0.11 mmol) in DCM (2 mL) was added TFA (1 mL). The reaction mixture was stirred at 20 °C for 2 h, then concentrated under reduced pressure to afford crude *N*-(1-(4-(dimethylamino)-4-methylpent-2-ynoyl)-4-fluoropiperidine-4-carbonyl)-*N*-methyl-L-valine. It was used for the next step directly without further purification. LCMS (ESI): m/z [M+H] calc'd for C₂₀H₃₂FN₃PO₄ 397.2; found 398.3.

### Synthesis of Exemplary Compounds

### Example A40. 1-(4-(dimethylamino)-4-methylpent-2-ynoyl)-N-((2S)-1-(((12S,12S)-21-ethyl-22-(2-((S)-1-methoxyethyl)pyridin-3-yl)-4,4,10-trimethyl-7,11-dioxo-21H-6-oxa-9,10-diaza-1(4,2)-morpholina-2(5,3)-indolacyclotridecaphane-12-yl)amino)-3-methyl-1-oxobutan-2-yl)-4-fluoro-N-methylpiperidine-4-carboxamide

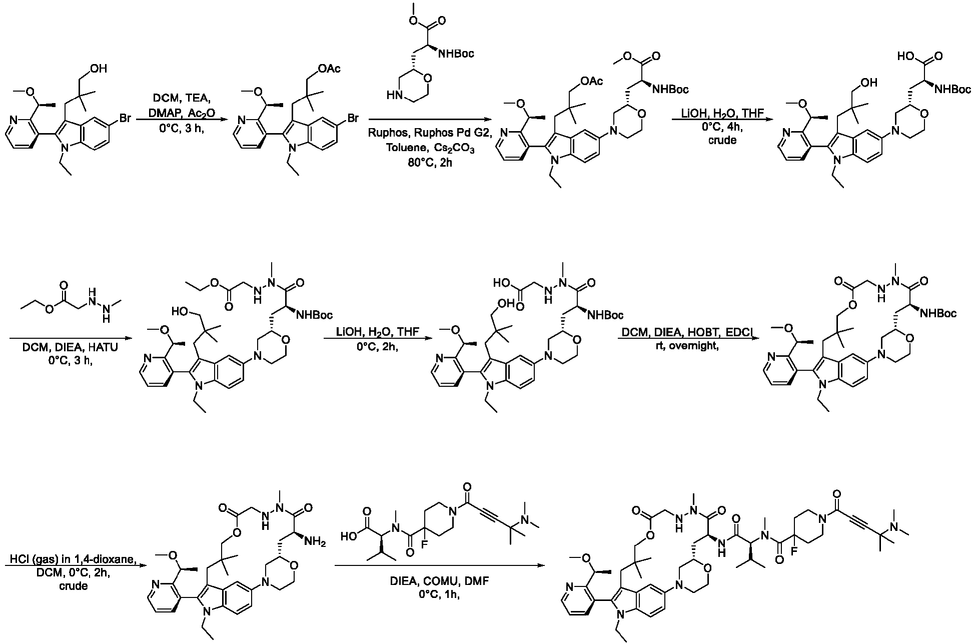

### Step 1

To a stirred solution of (*S*)-3-(5-bromo-1-ethyl-2-(2-(1-methoxyethyl)pyridin-3-yl)- 1*H*-indol-3-yl)-2,2-dimethylpropan-1-ol (5 g, 11.23 mmol, 1 equiv) and TEA (3.90 mL, 28.07 mmol, 2.5 equiv) in DCM (50 mL) was added Ac₂O (1.17 mL, 12.35 mmol, 1.1 equiv) and DMAP (274.29 mg, 2.245 mmol, 0.2 equiv) in portions at 0 °C. The resulting mixture was stirred for 3 h at 0 °C then washed with water (2 x 50 mL), and dried over anhydrous Na₂SO₄. After filtration, the filtrate was concentrated under reduced pressure. The residue was purified by silica gel column chromatography, eluted with PE / EA (1:1) to afford (*S*)-3-(5-bromo-1-ethyl-2-(2-(1-methoxyethyl)pyridin-3-yl)-1*H*-indol-3-yl)-2,2-dimethylpropyl acetate (4.2 g, 76.76% yield) as a light yellow solid. LCMS (ESI): m/z [M+H] calc'd for C₂₅H₃₁BrN₂O₃ ESI-MS 486.2; found: 487.2.

### Step 2

To a solution of (*S*)-3-(5-bromo-1-ethyl-2-(2-(1-methoxyethyl)pyridin-3-yl)-1*H*-indol-3-yl)-2,2-dimethylpropyl acetate (2 g, 4.10 mmol, 1 equiv) and methyl (2*S*)-2-[(tert-butoxycarbonyl)amino]-3-[(2*S*)-morpholin-2-yl]propanoate (1.77 g, 6.15 mmol, 1.5 equiv) in toluene (20 mL) was added Cs₂CO₃ (3.34 g, 10.26 mmol, 2.5 equiv), Ruphos (574.41 mg, 1.23 mmol, 0.3 equiv) and Ruphos Pd G2 (478.04 mg, 0.62 mmol, 0.15 equiv) at room temperature under nitrogen atmosphere. The resulting mixture was stirred for 2 h at 80 °C under nitrogen atmosphere. The reaction was cooled to 0 °C and quenched with sat. NH₄Cl (aq.). The resulting mixture was extracted with EtOAc (3 x 100 mL). The combined organic layers were washed with water (3 x 50 mL), and dried over anhydrous Na₂SO₄. After filtration, the filtrate was concentrated under reduced pressure. The residue was purified by silica gel column chromatography, eluted with PE / EA (1:9) to afford methyl (*S*)-3-((*S*)-4-(3-(3-acetoxy-2,2-dimethylpropyl)-1-ethyl-2-(2-((*S*)-1-methoxyethyl)pyridin-3-yl)-1H-indol-5-yl)morpholin-2-yl)-2-((*tert-*butoxycarbonyl)amino)propanoate (1.7 g, 59.63% yield) as a light yellow solid. LCMS (ESI): m/z [M+H] calc'd for C₃₈H₅₄N₄O₈ ESI-MS 694.4; found: 695.4.

### Step 3

To a stirred solution of methyl (*S*)-3-((*S*)-4-(3-(3-acetoxy-2,2-dimethylpropyl)-1-ethyl-2-(2-((*S*)-1-methoxyethyl)pyridin-3-yl)-1*H*-indol-5-yl)morpholin-2-yl)-2-((*tert*-butoxycarbonyl)amino)propanoate (1 g, 1.44 mmol, 1 equiv) in THF (10 mL) was added LiOH (363.63 mg, 15.18 mmol, 10.55 equiv) in H₂O (5 mL) dropwise at 0 °C. The resulting mixture was stirred for 4 h at 0 °C then neutralized to pH 7 with HCl (1M). The resulting mixture was extracted with EtOAc (3 x 20 mL). The combined organic layers were washed with water (2 x 10 mL), and dried over anhydrous Na₂SO₄. After filtration, the filtrate was concentrated under reduced pressure. This resulted in (2*S*)-2-[(*tert-*butoxycarbonyl)amino]-3-[(2*S*)-4-[(2M)-1-ethyl-3-(3-hydroxy-2,2-dimethylpropyl)-2-{2-[(1*S*)-1-methoxyethyl]pyridin-3-yl}indol-5-yl]morpholin-2-yl]propanoic acid (1.1 g, crude) as a light yellow solid. LCMS (ESI): m/z [M+H] calc'd for C₃₅H₅₀N₄O₇ ESI-MS 638.4; found: 639.3.

### Step 4

To a stirred solution of methyl 2-(2-methylhydrazin-1-yl)acetate (740.01 mg, 6.26 mmol, 10 equiv) and DIEA (1.09 mL, 6.26 mmol, 10 equiv) in DCM (8 mL) was added (2*S*)-2-[(*tert-*butoxycarbonyl)amino]-3-[(2*S*)-4-[(2M)-1- ethyl-3-(3-hydroxy-2,2-dimethylpropyl)-2-{2-[(1*S*)-1-methoxyethyl]pyridin-3-yl}indol-5-yl]morpholin-2-yl]propanoic acid (400.15 mg, 0.63 mmol, 1.00 equiv) and HATU (476.36 mg, 1.25 mmol, 2 equiv) in portions at 0 °C. The resulting mixture was stirred for 3 h at 0 °C then quenched with sat. NH₄Cl (aq.). The resulting mixture was extracted with DCM (3 x 20 mL). The combined organic layers were washed with water (3 x 10 mL), and dried over anhydrous Na₂SO₄. After filtration, the filtrate was concentrated under reduced pressure. The residue was purified by silica gel column chromatography, eluted with CH₂Cl₂/MeOH (10:1) to afford methyl 2-[(2S)-2-[(*tert*-butoxycarbonyl)amino]-3-[(2*S*)-4-[(2M)-1-ethyl-3-(3-hydroxy-2,2-dimethylpropyl)-2-{2-[(1*S*)-1-methoxyethyl]pyridin-3-yl}indol-5-yl]morpholin-2-yl]-N-methylpropanehydrazido]acetate (400 mg, 86.42% yield) as a white solid. LCMS (ESI): m/z [M+H] calc'd for C₄₀H₆₀N₆O₈ ESI-MS 752.5; found: 753.5.

### Step 5

To a solution of methyl 2-[(2*S*)-2-[(tert-butoxycarbonyl)amino]-3-[(2*S*)-4-[(2M)-1-ethyl-3-(3-hydroxy-2,2-dimethylpropyl)-2-{2-[(1*S*)-1-methoxyethyl]pyridin-3-yl}indol-5-yl]morpholin-2-yl]-N-methylpropanehydrazido]acetate (310 mg, 0.42 mmol, 1 equiv) in THF (10 mL) was added LiOH (36.0 mg, 1.5 mL, 3.5 equiv) in H₂O (5 mL) dropwise at 0 °C. The mixture was stirred for 2 h at 0 °C then acidified to pH 6 with HCl (1M). The resulting mixture was extracted with EtOAc (3 x 20 mL). The combined organic layers were washed with water (3 x 10 mL), and dried over anhydrous Na₂SO₄. After filtration, the filtrate was concentrated under reduced pressure. This afforded [(2*S*)-2-[(*tert-*butoxycarbonyl)amino]-3-[(2*S*)-4-[(2M)-1-ethyl-3-(3-hydroxy-2,2-dimethylpropyl)-2-{2-[(1*S*)-1-methoxyethyl]pyridin-3-yl}indol-5-yl]morpholin-2-yl]-N-methylpropanehydrazido]acetic acid (300 mg, 98.65% yield) as a white solid. LCMS (ESI): m/z [M+H] calc'd for C₃₈H₅₆N₆O₈ ESI-MS 724.4; found: 725.2.

### Step 6

To a stirred solution of [(2*S*)-2-[(tert-butoxycarbonyl)amino]-3-[(2*S*)-4-[(2M)-1-ethyl-3-(3-hydroxy-2,2- dimethylpropyl)-2-{2-[(1*S*)-1-methoxyethyl] pyridin-3-yl}indol-5-yl] morpholin-2-yl]-N-methylpropanehydrazido]acetic acid (280 mg, 0.39 mmol, 1 equiv) and DIEA (3.36 mL, 19.3 mmol, 50 equiv) in DCM (60 mL) was added HOBT (417.55 mg, 3.09 mmol, 8 equiv) and EDCI (2.22 g, 11.58 mmol, 30 equiv) in portions at 0°C. The resulting mixture was stirred overnight at room temperature. The mixture was washed with sat. NH₄Cl (aq.) (3 x 10 mL), sat. NaHCO₃ (aq.) (3 x 10 mL), and the combined organics were dried over anhydrous Na₂SO₄. After filtration, the filtrate was concentrated under reduced pressure. The residue was purified by Prep-TLC (PE / EA 1:1) to afford *tert-*butyl ((1²*S*,12*S*)-2¹-ethyl-2²-(2-((*S*)-1-methoxyethyl)pyridin-3-yl)-4,4,10-trimethyl-7,11-dioxo-2¹*H*-6-oxa-9,10-diaza-1(4,2)-morpholina-2(5,3)-indolacyclotridecaphane-12-yl)carbamate (120 mg, 43.95% yield) as a white solid. LCMS (ESI): m/z [M+H] calc'd for C₃₈H₅₄N₆O₇ ESI-MS 706.4; found: 707.4.

### Step 7

To a solution of *tert*-butyl ((1²*S*,12*S*)-2¹-ethyl-2²-(2-((*S*)-1-methoxyethyl)pyridin-3-yl)-4,4,10-trimethyl-7,11-dioxo-2¹*H*-6-oxa-9,10-diaza-1(4,2)-morpholina-2(5,3)-indolacyclotridecaphane-12-yl)carbamate (110 mg, 0.16 mmol, 1 equiv) in DCM (3 mL) was added HCl (gas) in 1,4-dioxane (1.5 mL) at 0 °C. The mixture was stirred for 2 h then concentrated under reduced pressure. This resulted in (1²*S*,12*S*)-12-amino-2¹-ethyl-2²-(2-((*S*)-1-methoxyethyl)pyridin-3-yl)-4,4,10-trimethyl-2¹*H*-6-oxa-9,10-diaza-1(4,2)-morpholina-2(5,3)-indolacyclotridecaphane-7,11-dione (140 mg, crude) as a green solid. LCMS (ESI): m/z [M+H] calc'd for C₃₃H₄₆N₆O₅ ESI-MS 606.4; found: 607.4.

### Step 8

To a solution of (1²*S*,12*S*)-12-amino-2¹-ethyl-2²-(2-((*S*)-1-methoxyethyl)pyridin-3-yl)-4,4,10-trimethyl-2¹*H*-6-oxa-9,10-diaza-1(4,2)-morpholina-2(5,3)-indolacyclotridecaphane-7,11-dione (110 mg, 0.18 mmol, 1 equiv) and DIEA (315.78 uL, 1.81 mmol, 10 equiv) in DMF(1 mL) was added (2S)-2-(1-{1-[4-(dimethylamino)-4-methylpent-2-ynoyl]-4-fluoropiperidin-4-yl}-N-methylformamido)-3-methylbutanoic acid (108.09 mg, 0.27 mmol, 1.5 equiv) and COMU (85.40 mg, 0.20 mmol, 1.1 equiv) in portions at 0 °C. The resulting mixture was stirred for 1 h at 0 °C. The mixture was directly purified by Prep-HPLC with the following conditions (Column: YMC-Actus Triart C18 ExRS, 30 * 150 mm, 5µm; Mobile Phase A: Water (10 mmol/L NH₄HCO₃+0.1% NH₃.H₂O), Mobile Phase B: ACN; Flow rate: 60 mL/min; Gradient: 41% B to 51% B in 8 min, 51% B; Wave Length: 254/220 nm; RT1(min): 9.03; Number Of Runs: 0) to afford 1-(4-(dimethylamino)-4-methylpent-2-ynoyl)-*N*-((2*S*)-1-(((1²*S*,12*S*)-2¹-ethyl-2²-(2-((*S*)-1-methoxyethyl)pyridin-3-yl)-4,4,10-trimethyl-7,11-dioxo-2¹*H*-6-oxa-9,10-diaza-1(4,2)-morpholina-2(5,3)-indolacyclotridecaphane-12-yl)amino)-3-methyl-1-oxobutan-2-yl)-4-fluoro-*N-*methylpiperidine-4-carboxamide (10 mg, 5.59% yield, 95.7% purity at 220 nm, 95.9% purity at 254 nm) as a white solid LCMS (ESI): m/z [M+H] calc'd for C₅₃H₇₆FN₉O₈ ESI-MS 985.6; found 986.5. ¹H NMR (400 MHz, DMSO-*d*₆) δ 8.74 (dd, *J* = 4.8, 1.7 Hz, 1H), 8.11 - 7.69 (m, 2H), 7.50 (q, *J* = 7.7, 4.7 Hz, 1H), 7.40 (d, *J* = 8.8 Hz, 1H), 6.95 (d, *J* = 12.1 Hz, 2H), 5.40 (s, 1H), 5.21 (s, 1H), 4.62 - 4.31 (m, 1H), 4.14 (d, *J* = 6.1 Hz, 3H), 4.08 - 3.90 (m, 2H), 3.89 - 3.73 (m, 4H), 3.72 - 3.56 (m, 3H), 3.50 - 3.38 (m, 3H), 3.09 - 3.97 (m, 6H), 2.92 - 2.74 (m, 5H), 2.68 - 2.56 (m, 2H), 2.31 - 1.76 (m, 14H), 1.46 - 1.29 (m, 9H), 1.22 (d, *J* = 15.4 Hz, 1H), 1.07 (t, *J* = 7.1 Hz, 3H), 0.97 - 0.86 (m, 3H), 0.85 - 0.72 (m, 3H), 0.65 (s, 3H), 0.52 (s, 3H).

### Example A48. 1-(4-(dimethylamino)-4-methylpent-2-ynoyl)-N-((2S)-1-(((2²S,6⁴S,4S)-1¹-ethyl-1²-(2-((S)-1-methoxyethyl)pyridin-3-yl)-10,10-dimethyl-5,7-dioxo-1¹H-8-oxa-6²,6³-diaza-2(4,2)-morpholina-1(5,3)-indola-6(2,4)-bicyclo[3.1.1]heptanacycloundecaphane-4-yl)amino)-3-methyl-1-oxobutan-2-yl)-4-fluoro-N-methylpiperidine-4-carboxamide

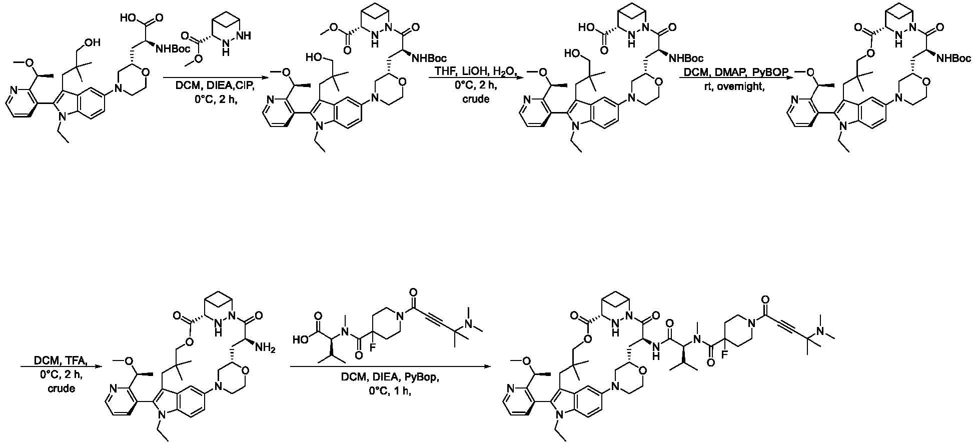

### Step 1

To a stirred solution of (*S*)-2-((*tert*-butoxycarbonyl)amino)-3-((*S*)-4-(1-ethyl-3-(3-hydroxy-2,2-dimethylpropyl)-2-(2-((*S*)-1-methoxyethyl)pyridin-3-yl)-1*H*-indol-5-yl)morpholin-2-yl)propanoic acid (300 mg, 0.470 mmol, 1 equiv) and DIEA (0.82 mL, 4.70 mmol, 10 equiv) in DCM (5 mL) was added methyl (4S)-2,3-diazabicyclo[3.1.1]heptane-4-carboxylate (58.68 mg, 0.38 mmol, 0.8 equiv) and CIP (130.82 mg, 0.47 mmol, 1.0 equiv) in portions at 0 °C. The resulting mixture was stirred for 2 h at 0 °C then quenched with saturated NH₄Cl (aq.). The resulting mixture was extracted with CH₂Cl₂ (2 x 20 mL). The combined organic layers were washed with water (2 x 10 mL), and dried over anhydrous Na₂SO₄. After filtration, the filtrate was concentrated under reduced pressure. The residue was purified by Prep-TLC (CH₂Cl₂ / MeOH 12:1) to afford methyl (*S*)-2-((S)-2-((*tert*-butoxycarbonyl)amino)-3-((*S*)-4-(1-ethyl-3-(3-hydroxy-2,2-dimethylpropyl)-2-(2-((*S*)-1-methoxyethyl)pyridin-3-yl)-1*H*-indol-5-yl)morpholin-2-yl)propanoyl)-2,3-diazabicyclo[3.1.1]heptane-4-carboxylate (177 mg, 48% yield) as a light yellow solid. LCMS (ESI): m/z [M+H] calc'd for C₄₂H₆₀N₆O₈ ESI-MS 776.5; found: 777.4.

### Step 2

To a stirred solution of methyl (*S*)-2-((*S*)-2-((*tert*-butoxycarbonyl)amino)-3-((*S*)-4-(1-ethyl-3-(3-hydroxy-2,2-dimethylpropyl)-2-(2-((*S*)-1-methoxyethyl)pyridin-3-yl)-1*H*-indol-5-yl)morpholin-2-yl)propanoyl)-2,3-diazabicyclo[3.1.1]heptane-4-carboxylate (280 mg, 0.36 mmol, 1 equiv) in THF (3 mL) was added LiOH (73.36 mg, 3.06 mmol, 8.5 equiv) in H₂O (3 mL) dropwise at 0 °C. The resulting mixture was stirred for 2 h at 0°C then acidified to pH 5 with 1M aq. HCl. The resulting mixture was extracted with EtOAc (2 x 10 mL), and dried over anhydrous Na₂SO₄. After filtration, the filtrate was concentrated under reduced pressure to afford (*S*)-2-((S)-2-((tert-butoxycarbonyl)amino)-3-((*S*)-4-(1-ethyl-3-(3-hydroxy-2,2- dimethylpropyl)-2-(2-((*S*)-1-methoxyethyl)pyridin-3-yl)-1*H*-indol-5-yl)morpholin-2-yl)propanoyl)-2,3-diazabicyclo[3.1.1]heptane-4-carboxylic acid (270 mg, crude) as a light yellow crude solid. LCMS (ESI): m/z [M+H] calc'd for C₄₁H₅₈N₆O₈ ESI-MS 762.4; found: 763.5.

### Step 3

To a stirred solution of (*S*)-2-((*S*)-2-((*tert*-butoxycarbonyl)amino)-3-((*S*)-4-(1-ethyl-3-(3-hydroxy-2,2- dimethylpropyl)-2-(2-((*S*)-1-methoxyethyl)pyridin-3-yl)-1*H*-indol-5-yl)morpholin-2-yl)propanoyl)-2,3-diazabicyclo[3.1.1]heptane-4-carboxylic acid (250 mg, 0.32 mmol, 1 equiv) in DCM (125 mL) was added DMAP (313.66 mg, 2.57 mmol, 8 equiv) and PyBop (668.04 mg, 1.28 mmol, 4 equiv) in portions at 0 °C. The resulting mixture was stirred overnight at room temperature. The mixture was washed with saturated NH₄Cl (aq.) (2 x 50 mL), and dried over anhydrous Na₂SO₄. After filtration, the filtrate was concentrated under reduced pressure. The residue was purified by Prep-TLC (CH₂Cl₂ / MeOH 10:1) to afford *tert-*butyl ((2²*S*,6⁴*S*,4*S*)-1¹-ethyl-1²-(2-((*S*)-1-methoxyethyl)pyridin-3-yl)-10,10-dimethyl-5,7-dioxo-1¹*H*-8-oxa-6²,6³-diaza-2(4,2)-morpholina-1(5,3)-indola-6(2,4)-bicyclo[3.1.1]heptanacycloundecaphane-4-yl)carbamate (230 mg, 96.21% yield) as a light yellow solid. LCMS (ESI): m/z [M+H] calc'd for C₄₁H₅₆N₆O₇ ESI-MS 744.4; found: 745.3.

### Step 4

To a solution of *tert-*butyl ((2²*S*,6⁴*S*,4*S*)-1¹-ethyl-1²-(2-((*S*)-1-methoxyethyl)pyridin-3-yl)-10,10-dimethyl-5,7-dioxo-1¹*H*-8-oxa-6²,6³-diaza-2(4,2)-morpholina-1(5,3)-indola-6(2,4)-bicyclo[3.1.1]heptanacycloundecaphane-4-yl)carbamate (130 mg, 0.18 mmol, 1 equiv) in DCM (1.3 mL) was added HCl (gas) in 1,4-dioxane (1.3 mL, 42.79 mmol, 245.18 equiv). The mixture was stirred for 2 h at 0 °C then concentrated under reduced pressure. This resulted in (2²*S*,6⁴*S*,4*S*)-4-amino-1¹-ethyl-1²-(2-((*S*)-1-methoxyethyl)pyridin-3-yl)-10,10-dimethyl-1¹*H*-8-oxa-6²,6³-diaza-2(4,2)-morpholina-1(5,3)-indola-6(2,4)-bicyclo[3.1.1]heptanacycloundecaphane-5,7-dione (130 mg, crude) as a light yellow solid. LCMS (ESI): m/z [M+H] calc'd for C₃₆H₄₈N₆O₅ ESI-MS 644.4; found: 645.4.

### Step 5

To a stirred solution of (2²*S*,6⁴*S*,4*S*)-4-amino-1¹-ethyl-1²-(2-((*S*)-1-methoxyethyl)pyridin-3-yl)-10,10-dimethyl-1¹*H*-8-oxa-6²,6³-diaza-2(4,2)-morpholina-1(5,3)-indola-6(2,4)-bicyclo[3.1.1]heptanacycloundecaphane-5,7-dione (120 mg, 0.19 mmol, 1 equiv) and DIEA (324.16 uL, 1.86 mmol, 10.00 equiv) in DCM (1.5 mL) was added (2*S*)-2-(1-{1-[4-(dimethylamino)-4-methylpent-2-ynoyl]-4-fluoropiperidin-4-yl}-N-methylformamido)-3-methylbutanoic acid (147.95 mg, 0.37 mmol, 2.00 equiv) and PyBop (106.53 mg, 0.21 mmol, 1.10 equiv) in portions at 0 °C. The reaction was stirred for 1 h at 0 °C then quenched with saturated NH₄Cl (aq.). The resulting mixture was extracted with CH₂Cl₂ (2 x 10 mL). The combined organic layers were washed with water (2 x 5 mL), and dried over anhydrous Na₂SO₄. After filtration, the filtrate was concentrated under reduced pressure. The crude product was purified by Prep-HPLC with the following conditions (Column: YMC-Actus Triart C18 ExRS, 30*150 mm, 5µm; Mobile Phase A: Water(10 mmol / L NH₄HCO₃), Mobile Phase B: ACN; Flow rate: 60 mL / min; Gradient: 48% B to 63% B in 8 min, 63% B; Wave Length: 254 / 220 nm; RT1(min): 7.8; Number Of Runs: 0) to afford 1-(4-(dimethylamino)-4-methylpent-2-ynoyl)-N-((2*S*)-1-(((2²*S*,6⁴*S*,4*S*)-1¹-ethyl-1²-(2-((*S*)-1-methoxyethyl)pyridin-3-yl)-10,10-dimethyl-5,7-dioxo-1¹*H*-8-oxa-6²,6³-diaza-2(4,2)-morpholina-1(5,3)-indola-6(2,4)-bicyclo[3.1.1]heptanacycloundecaphane-4-yl)amino)-3-methyl-1-oxobutan-2-yl)-4-fluoro-N-methylpiperidine-4-carboxamide (6.3 mg, 3.3% yield, 94.1% purity at 220 nm, 94.7% purity at 254 nm) as a white solid. LCMS (ESI): m/z [M+H] calc'd for C₅₆H₇₈FN₉O₈ ESI-MS 1023.6; found: 1024.4. ¹H NMR (400 MHz, DMSO-*d₆*) δ 8.73 (dd, *J* = 4.7, 1.7 Hz, 1H), 7.87 - 7.78 (m, 1H), 7.64 - 7.56(m, 1H), 7.53 - 7.46 (m, 1H), 7.42 (d, *J* = 8.9 Hz, 1H), 7.18 - 7.09 (m, 1H), 7.02 (d, *J* = 8.6 Hz, 1H), 6.33 (s, 1H), 5.48 - 5.28 (m, 1H), 4.60 - 4.18 (m, 2H), 4.44 - 4.28 (m, 1H), 4.27 - 4.01 (m, 4H), 3.76-3.97 (m, 2H), 3.73 - 3.44 (m, 6H), 3.16 - 2.92 (m, 6H), 2.91 - 2.72 (m, 3H), 2.71 - 2.57 (m, 2H), 2.48 - 2.36 (m, 1H), 2.33 - 2.21 (m, 1H), 2.21 (d, *J* = 2.5 Hz, 6H), 2.18 - 1.72 (m, 8H), 1.53 - 1.38 (m, 4H), 1.36 (d, *J* = 2.2 Hz, 6H), 1.29 - 1.66 (m, 1H), 1.11 - 0.91 (m, 4H), 0.87 (d, *J* = 6.4 Hz, 2H), 0.87 - 0.69 (m, 4H), 0.69 - 0.44 (m, 6H).

### Example A4. (1S,2S)-N-((6⁴S,4S,Z)-11-ethyl-12-(2-((S)-1-methoxyethyl)-5-(4-methylpiperazin-1-yl)pyridin-3-yl)-10,10-dimethyl-5,7-dioxo-11H-8-oxa-62,63-diaza-2(4,2)-thiazola-1(5,3)-indola-6(2,4)-bicyclo[3.1.1]heptanacycloundecaphane-4-yl)-2-methylcyclopropane-1-carboxamide

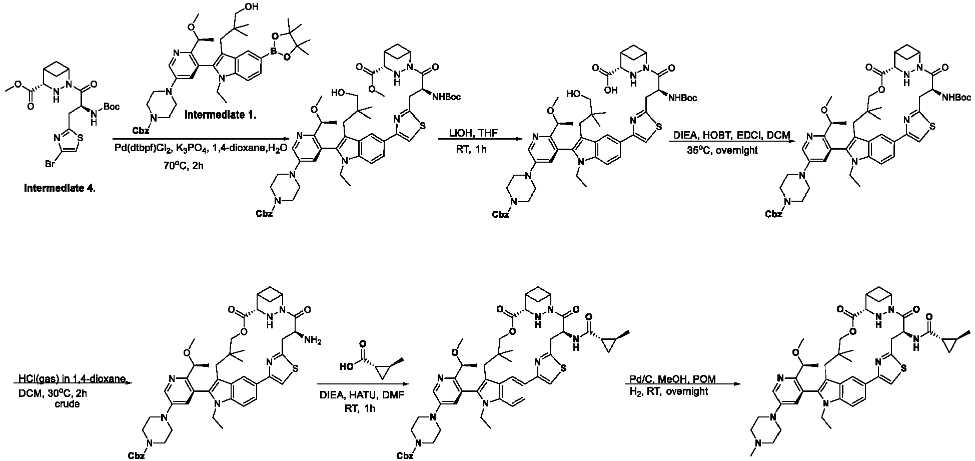

### Step 1

To a 25 mL sealed tube was added methyl (*R*)-2-((*S*)-3-(4-bromothiazol-2-yl)-2-((*tert-*butoxycarbonyl)amino) propanoyl)-2,3-diazabicyclo[3.1.1]heptane-4-carboxylate (350 mg, 0.72 mmol, 1 equiv) and benzyl 4-[(5M)-5-[1-ethyl-3-(3-hydroxy-2,2-dimethylpropyl)-5-(4,4,5,5-tetramethyl-1,3,2-dioxaborolan-2-yl)indol-2-yl]-6-[(1S)-1-methoxyethyl]pyridin-3-yl]piperazine-1-carboxylate (508.29 mg, 0.72 mmol, 1 equiv), K₃PO₄ (379.52 mg, 1.79 mmol, 2.5 equiv), Pd(dtbpf)Cl₂ (93.22 mg, 0.14 mmol, 0.2 equiv), 1,4-dioxane (5 mL), and H₂O (1 mL) at room temperature. The resulting mixture was stirred for 2 h at 70 °C under an argon atmosphere then filtered, the filter cake was washed with EtOAc (3 x 20 mL). The combined organic layers were washed with H₂O (3 x 10 mL) and dried over anhydrous Na₂SO₄. After filtration, the filtrate was concentrated under reduced pressure. The residue was purified by Prep-TLC to afford methyl (*R*)-2-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1H-indol-5-yl)thiazol-2-yl)-2-((*tert*-butoxycarbonyl)amino)propanoyl)-2,3-diazabicyclo[3.1.1]heptane-4-carboxylate (600 mg, 84% yield) as a yellow solid. LCMS (ESI): m/z [M+H] calc'd for C₅₃H₆₈N₈O₉S ESI-MS 992.5; found: 993.5.

### Step 2

To a 40 mL vial was added methyl (*R*)-2-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-2-((*tert*-butoxycarbonyl)amino)propanoyl)-2,3-diazabicyclo[3.1.1]heptane-4-carboxylate (600 mg, 0.6 mmol, 1 equiv) ,THF (6 mL) and LiOH (2.01 mL, 6.04 mmol, 10 equiv) at 0 °C. The resulting mixture was stirred for 1 h at room temperature then acidified to pH 5 with HCl (aq.). This was extracted with EtOAc (3 x 20 mL), the combined organic layers were washed with water (3 x 10 mL) and dried over anhydrous Na₂SO₄. After filtration, the filtrate was concentrated under reduced pressure to afford (*R*)-2-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-2-((*tert-*butoxycarbonyl)amino)propanoyl)-2,3-diazabicyclo[3.1.1]heptane-4-carboxylic acid (560 mg, 94% yield) as a yellow solid which was used without further purification. LCMS (ESI): m/z [M+H] calc'd for C₅₂H₆₆N₈O₉S ESI-MS 978.5; found: 979.5.

### Step 3

To a round-bottom flask was added (*R*)-2-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-2-((*tert*-butoxycarbonyl)amino)propanoyl)-2,3-diazabicyclo[3.1.1]heptane-4-carboxylic acid (560 mg, 0.57 mmol, 1 equiv), DCM (110 mL), DIEA (2217.45 mg, 17.16 mmol, 30 equiv), HOBT (772.77 mg, 5.72 mmol, 10 equiv), and EDCI (3288.94 mg, 17.16 mmol, 30 equiv) at 0 °C. The resulting mixture was stirred for overnight at 35 °C under air atmosphere then quenched with H₂O (50 mL) and extracted with EtOAc (3 x 50 mL). The combined organic layers were washed with water (3 x 50 mL) and dried over anhydrous Na₂SO₄. After filtration, the filtrate was concentrated under reduced pressure. The residue was purified by Prep-TLC to afford benzyl 4-(5-((6⁴*R*,4*S*, *Z*)-4-((*tert-*butoxycarbonyl) amino)-1¹-ethyl-10,10-dimethyl-5,7-dioxo-1¹*H*-8-oxa-6²,6³-diaza-2(4,2)-thiazola-1(5,3)-indola-6(2,4)-bicyclo[3.1.1] heptanacycloundecaphane-1²-yl)-6-((*S*)-1-methoxyethyl)pyridin-3-yl)piperazine-1-carboxylatebenzyl (270 mg, 49% yield) as a yellow solid. LCMS (ESI): m/z [M+H] calc'd for C₅₂H₆₄N₈O₈S ESI-MS 960.5; found: 961.3.

### Step 4

To a 40 mL vial was added benzyl 4-(5-((6⁴*R*,4*S*, *Z*)-4-((*tert*-butoxycarbonyl) amino)-1¹-ethyl-10,10-dimethyl-5,7-dioxo-1¹*H*-8-oxa-6²,6³-diaza-2(4,2)-thiazola-1(5,3)-indola-6(2,4)-bicyclo[3.1.1] heptanacycloundecaphane-1²-yl)-6-((*S*)-1-methoxyethyl)pyridin-3-yl)piperazine-1-carboxylatebenzyl (230 mg, 0.24 mmol, 1 equiv), DCM (5 mL) and 4M HCl in 1,4-dioxane (1.5 mL) at 0 °C. The resulting mixture was stirred for 2 h at 30 °C then concentrated under reduced pressure to afford benzyl 4-(5-((6⁴*R*,4*S*, *Z*)-4-amino-1¹-ethyl-10,10-dimethyl-5,7-dioxo-1¹*H*-8-oxa-6²,6³-diaza-2(4,2)-thiazola-1(5,3)-indola-6(2,4)-bicyclo[3.1.1] heptanacycloundecaphane-1²-yl)-6-((*S*)-1-methoxyethyl)pyridin-3-yl)piperazine-1-carboxylate (300 mg, crude) as a yellow solid. The crude product was used in the next step directly without further purification. LCMS (ESI): m/z [M+H] calc'd for C₄₇H₅₆N₈O₆S ESI-MS 860.4; found: 861.3.

### Step 5

To a 40 mL vial was added benzyl 4-(5-((6⁴R,4S, *Z*)-4-amino-1¹-ethyl-10,10-dimethyl-5,7-dioxo-1¹*H*-8-oxa-6²,6³-diaza-2(4,2)-thiazola-1(5,3)-indola-6(2,4)-bicyclo[3.1.1]heptanacycloundecaphane-1²-yl)-6-((*S*)-1-methoxyethyl) pyridin-3-yl)piperazine-1-carboxylate (240 mg, 0.28 mmol, 1 equiv), DMF (4 mL), DIEA (360.24 mg, 2.79 mmol, 10 equiv), (1S,2S)-2-methylcyclopropane-1-carboxylic acid (55.81 mg, 0.56 mmol, 2 equiv), and HATU (158.97 mg, 0.42 mmol, 1.5 equiv) at 0 °C. The resulting mixture was stirred for 1 h at room temperature then quenched with H₂O (20 mL) and extracted with EtOAc (3 x 20 mL). The combined organic layers were washed with H₂O (2 x 10 mL) and dried over anhydrous Na₂SO₄. After filtration, the filtrate was concentrated under reduced pressure. The residue was purified by Prep-TLC to afford benzyl 4-(5-((6⁴*R*,4*S*, *Z*)-1¹-ethyl-10,10-dimethyl-4-((1*S*,2*S*)-2-methylcyclopropane-1-carboxamido)-5,7-dioxo-1¹*H-*8-oxa-6²,6³-diaza-2(4,2)-thiazola-1(5,3)-indola-6(2,4)-bicyclo[3.1.1]heptanacycloundecaphane-1²-yl)-6-((*S*)-1-methoxyethyl) pyridin-3-yl)piperazine-1-carboxylate (250 mg, 95% yield) as a yellow solid. LCMS (ESI): m/z [M+H] calc'd for C₅₂H₆₂N₈O₇S ESI-MS 942.4; found: 943.2.

### Step 6

To a 40 mL vial was added benzyl 4-(5-((6⁴*R*,4*S*, Z)-1¹-ethyl-10,10-dimethyl-4-((1*S*,2*S*)-2-methylcyclopropane-1-carboxamido)-5,7-dioxo-1¹*H*-8-oxa-6²,6³-diaza-2(4,2)-thiazola-1(5,3)-indola-6(2,4)-bicyclo[3.1.1] heptanacycloundecaphane-1²-yl)-6-((*S*)-1-methoxyethyl)pyridin-3-yl)piperazine-1-carboxylate (180 mg, 0.19 mmol, 1 equiv), MeOH (4 mL), Paraformaldehyde (171.91 mg, 1.91 mmol, 10 equiv), and 10%wt Pd/C (60.93 mg) at room temperature. The resulting mixture was stirred overnight at room temperature under hydrogen atmosphere. The resulting mixture was filtered, the filter cake was washed with EtOAc (3 x 10 mL) and the filtrate was concentrated under reduced pressure. The crude product (110 mg) was purified by Prep-HPLC to afford (1*S,*2*S*)-*N*-((6⁴*R*,4*S*, *Z*)-1¹-ethyl-1²-(2-((*S*)-1-methoxyethyl)-5-(4-methylpiperazin-1-yl)pyridin-3-yl)-10,10-dimethyl-5,7-dioxo-1¹*H-*8-oxa-6²,6³-diaza-2(4,2)-thiazola-1(5,3)-indola-6(2,4)-bicyclo[3.1.1]heptanacycloundecaphane-4-yl)-2-methylcyclopropane-1-carboxamide (50 mg, 31% yield, 95.8% purity@254nm, 96.0% purity@220 nm) as a white solid. LCMS (ESI): m/z [M+H] calc'd for C₄₅H₅₈N₈O₅S ESI-MS 822.4; found: 823.4. ¹H NMR (400 MHz, DMSO-d₆) δ 8.54 (d, J = 8.9 Hz, 1H), 8.42 (dd, J = 15.0, 2.2 Hz, 2H), 7.80 (s, 1H), 7.72 (dd, J = 8.7, 1.6 Hz, 1H), 7.55 (d, J = 8.7 Hz, 1H), 7.22 (d, J = 2.9 Hz, 1H), 5.95 (d, J = 11.1 Hz, 1H), 5.38 (t, J = 8.2 Hz, 1H), 4.67 (d, J = 11.0 Hz, 1H), 4.49 (q, J = 4.8 Hz, 1H), 4.28 (dd, J = 14.6, 7.2 Hz, 1H), 4.14 (q, J = 6.7 Hz, 2H), 3.55 (q, J = 10.8 Hz, 2H), 3.26 (d, J = 10.2 Hz, 5H), 3.19 (s, 4H), 2.91 (d, J = 14.4 Hz, 1H), 2.63 (q, J = 5.9 Hz, 1H), 2.45 (t, J = 5.1 Hz, 5H), 2.37 - 2.28 (m, 1H), 2.21 (s, 3H), 2.16 (t, J = 9.8 Hz, 1H), 1.58 (t, J = 9.3 Hz, 1H), 1.50 (t, J = 4.7 Hz, 1H), 1.33 (d, J = 6.1 Hz, 3H), 1.23 (s, 0H), 1.07 (d, J = 1.9 Hz, 4H), 0.94 - 0.86 (m, 7H), 0.56 (dd, J = 8.1, 4.9 Hz, 1H), 0.33 (s, 3H).

### Example A46. (2S)-N-((6⁴S,4S,Z)-1¹-ethyl-1²-(2-((S)-1-methoxyethyl)-5-(4-methylpiperazin-1-yl)pyridin-3-yl)-10,10-dimethyl-5,7-dioxo-11H-8-oxa-6²,6³-diaza-2(4,2)-thiazola-1(5,3)-indola-6(2,4)-bicyclo[3.1.1]heptanacycloundecaphane-4-yl)-3-methyl-2-(1,3,3-trimethylureido)butanamide

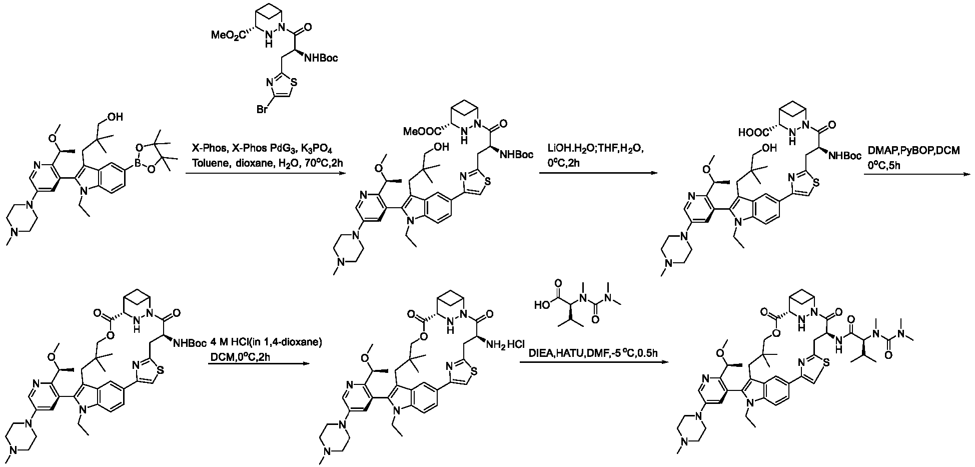

### Step 1

A solution of (*S*)-3-(1-ethyl-2-(2-(1-methoxyethyl)-5-(4-methylpiperazin-1-yl)pyridin-3-yl)-5-(4,4,5,5-tetramethyl-1,3,2-dioxaborolan-2-yl)-1*H*-indol-3-yl)-2,2-dimethylpropan-1-ol (312 mg, 0.53 mmol, 1 equiv), methyl (*S*)-2-((*S*)-3-(4-bromothiazol-2-yl)-2-((tert-butoxycarbonyl)amino)propanoyl)-2,3-diazabicyclo[3.1.1]heptane-4-carboxylate (310.23 mg, 0.63 mmol, 1.2 equiv), X-Phos (100.73 mg, 0.211 mmol, 0.4 equiv), K₃PO₄ (336.39 mg, 1.58 mmol, 3 equiv) and X-Phos-Pd-G3 (89.43 mg, 0.11 mmol, 0.2 equiv) in Toluene (3 mL), H₂O (1 mL) and dioxane (1 mL) was stirred for 2h at 70 °C under nitrogen atmosphere. The resulting mixture was diluted with water (10 mL) and extracted with EtOAc (3 x 10mL). The combined organic layers were washed with brine (3 x 10 mL) and dried over anhydrous Na₂SO₄. After filtration, the filtrate was concentrated under reduced pressure. The residue was purified by silica gel column chromatography to afford methyl (*S*)-2-((*S*)-2-((*tert-*butoxycarbonyl)amino)-3-(4-(1-ethyl-3-(3-hydoxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)propanoyl)-2,3-diazabicyclo[3.1.1]heptane-4-carboxylate (300 mg, 65.04% yield) as an off-white solid. LCMS (ESI): m/z [M+H] calc'd for C₄₆H₆₄N₈O₇S ESI-MS 872.5; found: 873.4.

### Step 2

To a stirred solution of methyl (*S*)-2-((*S*)-2-((*tert*-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H-*indol-5-yl)thiazol-2-yl)propanoyl)-2,3-diazabicyclo[3.1.1]heptane-4-carboxylate (300 mg, 0.34 mmol, 1 equiv) in THF (3 mL) and H₂O(1 mL) was added LiOH.H2O (72.09 mg, 1.72 mmol, 5 equiv) at 0 °C under nitrogen atmosphere. The reaction was stirred for 2 h at 0 °C then acidified to pH 6 with 1 M HCl. The resulting mixture was extracted with EtOAc (3 x 10 mL). The combined organic layers were washed with brine (3 x 10 mL) and dried over anhydrous Na₂SO₄. After filtration, the filtrate was concentrated under reduced pressure to afford (*S*)-2-((*S*)-2-((*tert*-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H-*indol-5-yl)thiazol-2-yl)propanoyl)-2,3-diazabicyclo[3.1.1]heptane-4-carboxylic acid (290 mg, 98.25% yield) as an off-white solid. LCMS (ESI): m/z [M+H] calc'd for C₄₅H₆₂N₈O₇S ESI-MS 858.5; found: 859.3.

### Step 3

To a stirred solution of (*S*)-2-((*S*)-2-((*tert*-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)propanoyl)-2,3-diazabicyclo[3.1.1]heptane-4-carboxylic acid (210 mg, 0.24 mmol, 1 equiv) in DCM (42 mL) was added DMAP (89.59 mg, 0.732 mmol, 3 equiv) and PyBOP (763.25 mg, 1.464 mmol, 6 equiv) in portions at 0 °C. The reaction was stirred for 5h at room temperature then diluted with water (100 mL). The resulting mixture was extracted with CH₂Cl₂ (3 x 50 mL). The combined organic layers were washed with brine (3 x 100 mL) and dried over anhydrous Na₂SO₄. After filtration, the filtrate was concentrated under reduced pressure and the residue was purified by silica gel column chromatography to afford tert-butyl ((6⁴*S*,4*S*,*Z*)-1¹-ethyl-1²-(2-((*S*)-1-methoxyethyl)-5-(4-methylpiperazin-1-yl)pyridin-3-yl)-10,10-dimethyl-5,7-dioxo-11*H*-8-oxa-6²,6³-diaza-2(4,2)-thiazola-1(5,3)-indola-6(2,4)-bicyclo[3.1.1]heptanacycloundecaphane-4-yl)carbamate(100 mg, 48.76% yield) as an off-white solid. LCMS (ESI): m/z [M+H] calc'd for C₄₅H₆₀N₈O₆S ESI-MS 840.4; found: 841.6.

### Step 4

To a stirred solution of *tert*-butyl ((6⁴*S*,4*S*,*Z*)-1¹-ethyl-1²-(2-((*S*)-1-methoxyethyl)-5-(4-methylpiperazin-1-yl)pyridin-3-yl)-10,10-dimethyl-5,7-dioxo-1¹H-8-oxa-6²,6³-diaza-2(4,2)-thiazola-1(5,3)-indola-6(2,4)-bicyclo[3.1.1]heptanacycloundecaphane-4-yl)carbamate (100 mg, 0.12 mmol, 1 equiv) in DCM (0.5 mL) was added HCl(gas) in 1,4-dioxane (1.5 mL) at 0 °C under nitrogen atmosphere . The reaction was stirred for 2 h at 0 °C then concentrated under reduced pressure. This resulted in (6⁴*S*,4*S*,*Z*)-4-amino-1¹-ethyl-1²-(2-((*S*)-1-methoxyethyl)-5-(4-methylpiperazin-1-yl)pyridin-3-yl)-10,10-dimethyl-1¹*H*-8-oxa-6²,6³-diaza-2(4,2)-thiazola-1(5,3)-indola-6(2,4)-bicyclo[3.1.1]heptanacycloundecaphane-5,7-dione hydrogen chloride salt (100 mg, crude) as an off-white solid. LCMS (ESI): m/z [M+H] calc'd for C₄₀H₅₂N₈O₄S ESI-MS 740.4; found: 741.3.

### Step 5

To a stirred solution of (6⁴*S*,4*S*,*Z*)-4-amino-1¹-ethyl-1²-(2-((*S*)-1-methoxyethyl)-5-(4-methylpiperazin-1-yl)pyridin-3-yl)-10,10-dimethyl-1¹*H*-8-oxa-6²,6³-diaza-2(4,2)-thiazola-1(5,3)-indola-6(2,4)-bicyclo[3.1.1]heptanacycloundecaphane-5,7-dione hydrogen chloride (98 mg, 0.13 mmol, 1 equiv) and N-(dimethylcarbamoyl)-N-methyl-L-valine(30.54 mg, 0.15 mmol, 1.2 equiv) in DMF (2 mL) was added DIEA (486.66 mg, 3.78 mmol, 30 equiv) and HATU (71.59 mg, 0.19 mmol, 1.5 equiv) in portions at 0 °C. The reaction was stirred for 30 min at 0 °C then diluted with water (10mL). The resulting mixture was extracted with EtOAc (3 x 10mL). The combined organic layers were washed with water (3 x 10 mL) and dried over anhydrous Na₂SO₄. After filtration, the filtrate was concentrated under reduced pressure. The crude product was purified by Prep-HPLC with the following conditions (Column: XBridge Prep Phenyl OBD Column, 19*150 mm, 5µm; Mobile Phase A: Water(10 mmol/L NH4HCO3+0.1%NH3.H2O), Mobile Phase B: MeOH-----Preparative; Flow rate: 60 mL/min; Gradient: 69% B to 83% B in 10 min, 83% B; Wave Length: 254/220 nm; RT1(min): 10.17; Number Of Runs: 0) to afford (2*S*)-N-((6⁴*S*,4*S*,*Z*)-1¹-ethyl-1²-(2-((*S*)-1-methoxyethyl)-5-(4-methylpiperazin-1-yl)pyridin-3-yl)-10,10-dimethyl-5,7-dioxo-1¹*H*-8-oxa-6²,6³-diaza-2(4,2)-thiazola-1(5,3)-indola-6(2,4)-bicyclo[3.1.1]heptanacycloundecaphane-4-yl)-3-methyl-2-(1,3,3-trimethylureido)butanamide (4.6 mg, 3.94% yield, 96.0%purity@220nm,96.1%purity@254nm) as an off white solid. LCMS (ESI): m/z [M+H] calc'd for C_{40\9}H₆₈N₁₀O₆S ESI-MS 924.5; found: 925.7. ¹H NMR (400 MHz, DMSO-*d₆*) δ 8.45 - 8.30 (m, 3H), 7.75 (s, 1H), 7.66 (d, *J* = 8.6 Hz, 1H), 7.49 (d, *J* = 8.6 Hz, 1H), 7.17 (d, *J* = 2.9 Hz, 1H), 5.93 (d, *J* = 11.0 Hz, 1H), 5.28 (t, *J* = 8.0 Hz, 1H), 4.63 (d, *J* = 11.0 Hz,1H), 4.44 (q, *J* = 4.9 Hz, 1H), 4.28 - 4.14 (m, 1H), 4.08 (d, *J* = 6.3 Hz, 2H), 3.78 (d, *J* = 10.9 Hz, 1H), 3.53 - 3.46 (m, 1H), 3.23 (t, *J* = 5.8 Hz, 4H), 3.14 (s, 3H), 3.02 (dd, *J* = 15.0, 7.3 Hz, 1H), 2.86 (d, *J* = 14.2 Hz, 1H), 2.58 (q, *J* = 5.9 Hz,1H), 2.50 (s, 4H), 2.43 (d, *J* = 9.6 Hz, 5H), 2.23 (s, 4H), 2.09 (dt, *J* = 10.6, 6.5 Hz, 2H), 1.53 (t, *J* = 9.4 Hz, 1H), 1.27 (d, *J* = 6.1 Hz, 3H), 0.81 (q, *J* = 7.5 Hz, 12H), 0.26 (s, 3H).

### Example A47. (S)-N-((S,Z)-21-ethyl-22-(2-((S)-1-methoxyethyl)-5-(4-methylpiperazin-1-yl)pyridin-3-yl)-4,4,10-trimethyl-7,11-dioxo-21H-6-oxa-9,10-diaza-1(4,2)-thiazola-2(5,3)-indolacyclotridecaphane-12-yl)-3-methyl-2-(1,3,3-trimethylureido)butanamide

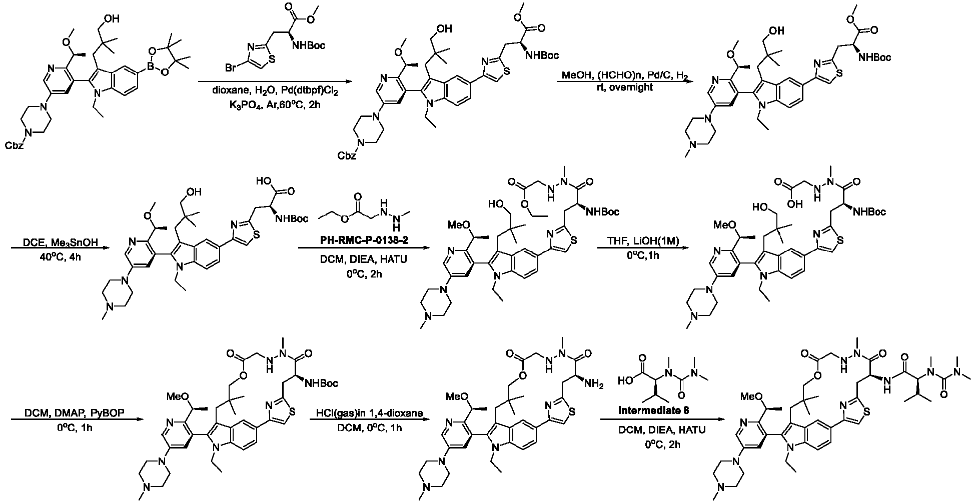

### Step 1

To a stirred solution of benzyl (*S*)-4-(5-(1-ethyl-3-(3-hydroxy-2,2-dimethylpropyl)-5-(4,4,5,5-tetramethyl-1,3,2-dioxaborolan-2-yl)-1*H*-indol-2-yl)-6-(1-methoxyethyl)pyridin-3-yl)piperazine-1-carboxylate (5.3 g, 7.46 mmol, 1 equiv), methyl (*S*)-3-(4-bromothiazol-2-yl)-2-((tert-butoxycarbonyl)amino)propanoate (3.27 g, 8.95 mmol, 1.2 equiv) and Pd(dtbpf)Cl₂ (0.97 g, 1.49 mmol, 0.2 equiv) in dioxane (50 mL) was added K₃PO₄ (3.96 g, 18.64 mmol, 2.5 equiv) in H₂O (10 mL) dropwise at 0°C under an argon atmosphere. The reaction was stirred for 2h at 60°C then quenched by sat. NH₄Cl (aq.) (100 mL) at 0°C. The resulting mixture was extracted with DCM / MeOH (10:1) (3 x100 mL). The combined organic layers were washed with water (3x100 mL), dried over anhydrous Na₂SO₄. After filtration, the filtrate was concentrated under reduced pressure to afford benzyl 4-(5-(5-(2-((*S*)-2-((tert-butoxycarbonyl)amino)-3-methoxy-3-oxopropyl)thiazol-4-yl)-1-ethyl-3-(3-hydroxy-2,2-dimethylpropyl)-1*H*-indol-2-yl)-6-((*S*)-1-methoxyethyl)pyridin-3-yl)piperazine-1-carboxylate (7.234 g, crude) as a brown solid. LCMS (ESI): m/z [M+H] calc'd for C₄₇H₆₀N₆O₈S ESI-MS 868.4; found: 869.5.

### Step 2

To a stirred solution of benzyl 4-(5-(5-(2-((*S*)-2-((tert-butoxycarbonyl)amino)-3-methoxy-3-oxopropyl)thiazol-4-yl)-1-ethyl-3-(3-hydroxy-2,2-dimethylpropyl)-1*H*-indol-2-yl)-6-((*S*)-1-methoxyethyl)pyridin-3-yl)piperazine-1-carboxylate (2 g, 2.30 mmol, 1 equiv) in MeOH (50 mL) was added Pd/C (2 g, 18.79 mmol, 8.17 equiv) and Paraformaldehyde (1.04 g, 11.51 mmol, 5 equiv) in portions at room temperature. The resulting mixture was stirred overnight at room temperature under hydrogen atmosphere then filtered; the filter cake was washed with DCM (4 x 100 mL). The filtrate was concentrated under reduced pressure. The residue was purified by silica gel column chromatography to afford methyl (*S*)-2-((tert-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)propanoate (920 mg, 53.38% yield) as a brown solid. LCMS (ESI): m/z [M+H] calc'd for C₄₀H₅₆N₆O₆S ESI-MS 748.4; found: 749.4.

### Step 3

To a stirred solution of methyl (*S*)-2-((tert-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)propanoate (920 mg, 1.23 mmol, 1 equiv) in DCE (27.6 mL) was added Me₃SnOH (2.2 g, 12.28 mmol, 10 equiv) at 0°C. The resulting mixture was stirred for 4h at 40°C then filtered; the filter cake was washed with DCM (3 x 100 mL). The filtrate was concentrated under reduced pressure. This resulted in (*S*)-2-((tert-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)propanoic acid (960 mg, crude) as a yellow solid. LCMS (ESI): m/z [M+H] calc'd for C₃₉H₅₄N₆O₆S ESI-MS 734.4; found: 735.3.

### Step 4

To a stirred solution of (*S*)-2-((*tert*-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)propanoic acid (960 mg, 1.31 mmol, 1 equiv) in DCM (10 mL) was added DIEA (3.19 mL, 18.28 mmol, 14 equiv), ethyl (methylamino)glycinate (1.035 g, 7.84 mmol, 6 equiv) and HATU (496.66 mg, 1.31 mmol, 1 equiv) in portions at 0°C. The resulting mixture was stirred for 2h at 0°C then quenched by the addition of sat. NH₄Cl (aq.) (100mL) at 0°C. The resulting mixture was extracted with DCM/ MeOH(10:1) (3 x 100 mL). The combined organic layers were washed with water (2 x 100 mL), dried over anhydrous Na₂SO₄. After filtration, the filtrate was concentrated under reduced pressure. The residue was purified by reversed-phase flash chromatography to afford ethyl ((*S*)-2-((*tert-*butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)-N-methylpropanamido)glycinate (240 mg, 21%yield) as a yellow solid. LCMS (ESI): m/z [M+H] calc'd for C₄₄H₆₄N₈O₇S ESI-MS 848.5; found: 849.6.

### Step 5

To a stirred solution of ethyl ((*S*)-2-((*tert*-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)-N-methylpropanamido)glycinate (240 mg, 0.28 mmol, 1 equiv) in THF (3 mL) was added LiOH (1M in H2O) (1.5 mL) dropwise at 0°C. The reaction was stirred for 1h at 0°C then acidified to pH 6 with HCl (1M) (aq.). The resulting mixture was purified by reversed-phase flash chromatography to afford ((S)-2-((*tert*-butoxycarbonyl)amino)-3-(4-(1-ethyl-3-(3-hydroxy-2,2-dimethylpropyl)-2-(2-((S)-1-methoxyethyl)-5-(4-methylpiperazin-1-yl)pyridin-3-yl)-1*H*-indol-5-yl)thiazol-2-yl)-N-methylpropanamido)glycine (241 mg, 91% yield) as a yellow oil. LCMS (ESI): m/z [M+H] calc'd for C₄₂H₆₀N₈O₇S ESI-MS 820.4; found: 821.6.

### Step 6

To a stirred solution of ((*S*)-2-((*tert*-butoxycarbonyl)amino)-3-(4-(1-ethyl-3-(3-hydroxy-2,2-dimethylpropyl)-2-(2-((*S*)-1-methoxyethyl)-5-(4-methylpiperazin-1-yl)pyridin-3-yl)-1*H*-indol-5-yl)thiazol-2-yl)-N-methylpropanamido)glycine (272 mg, 0.33 mmol, 1 equiv) in DCM (410 mL) was added DMAP (161.89 mg, 1.32 mmol, 4 equiv) followed by PyBOP (1.379 g, 2.65 mmol, 8 equiv) in portions at 0°C. The reaction was stirred for 1h at 0°C then quenched by sat. NH₄Cl (aq.) (100mL) at 0°C. The resulting mixture was extracted with DCM/ MeOH (10:1) (3 x 100 mL). The combined organic layers were washed with water (2 x 100 mL) and dried over anhydrous Na₂SO₄. After filtration, the filtrate was concentrated under reduced pressure and the residue was purified by silica gel column chromatography to afford tert-butyl ((*S*, *Z*)-21-ethyl-22-(2-((*S*)-1-methoxyethyl)-5-(4-methylpiperazin-1-yl)pyridin-3-yl)-4,4,10-trimethyl-7,11-dioxo-21*H*-6-oxa-9,10-diaza-1(4,2)-thiazola-2(5,3)-indolacyclotridecaphane-12-yl)carbamate (112 mg, 42%yield) as a yellow solid. LCMS (ESI): m/z [M+H] calc'd for C₄₂H₅₈N₈O₆S ESI-MS 802.4; found: 803.5.

### Step 7

To a stirred solution of *tert*-butyl ((*S*, *Z*)-21-ethyl-22-(2-((*S*)-1-methoxyethyl)-5-(4-methylpiperazin-1-yl)pyridin-3-yl)-4,4,10-trimethyl-7,11-dioxo-21*H*-6-oxa-9,10-diaza-1(4,2)-thiazola-2(5,3)-indolacyclotridecaphane-12-yl)carbamate (110 mg, 0.14 mmol, 1 equiv) in DCM (2 mL) was added HCl(gas) in 1,4-dioxane (2 mL) dropwise at 0°C. The reaction was stirred for 1h at 0°C then concentrated under reduced pressure to afford (*S, Z*)-12-amino-21-ethyl-22-(2-((*S*)-1-methoxyethyl)-5-(4-methylpiperazin-1-yl)pyridin-3-yl)-4,4,10-trimethyl-21*H*-6-oxa-9,10-diaza-1(4,2)-thiazola-2(5,3)-indolacyclotridecaphane-7,11-dione(125 mg, crude) as a yellow solid. LCMS (ESI): m/z [M+H] calc'd for C₃₇H₅₀N₈O₄S ESI-MS 702.4; found: 703.6.

### Step 8

To a stirred solution of (*S*, *Z*)-12-amino-21-ethyl-22-(2-((*S*)-1-methoxyethyl)-5-(4-methylpiperazin-1-yl)pyridin-3-yl)-4,4,10-trimethyl-21*H*-6-oxa-9,10-diaza-1(4,2)-thiazola-2(5,3)-indolacyclotridecaphane-7,11-dione (125 mg, 0.18 mmol, 1 equiv) in DCM (2 mL) was added DIEA (309.76 uL, 1.78 mmol, 10 equiv), N-(dimethylcarbamoyl)-N-methyl-L-valine (43.16 mg, 0.214 mmol, 1.2 equiv) and HATU (74.38 mg, 0.196 mmol, 1.1 equiv) in portions at 0°C. The reaction was stirred for 2h at 0°C then quenched by sat. NH₄Cl (aq.) (50mL) at 0°C. The resulting mixture was extracted with DCM/ MeOH(10:1) (3 x 50 mL). The combined organic layers were washed with water (2 x 50 mL) and dried over anhydrous Na₂SO₄. After filtration, the filtrate was concentrated under reduced pressure. The residue was purified by reversed-phase flash chromatography to afford (*S*)-N-((*S, Z*)-21-ethyl-22-(2-((*S*)-1-methoxyethyl)-5-(4-methylpiperazin-1-yl)pyridin-3-yl)-4,4,10-trimethyl-7,11-dioxo-21*H*-6-oxa-9,10-diaza-1(4,2)-thiazola-2(5,3)-indolacyclotridecaphane-12-yl)-3-methyl-2-(1,3,3-trimethylureido)butanamide (28.4 mg, 17%yield, 98.3% purity @ 254nm, 98.1% purity @ 220nm) as a white solid. LCMS (ESI): m/z [M+H] calc'd for C₄₆H₆₆N₁₀O₆S ESI-MS 886.5; found: 887.3. ¹H NMR (400 MHz, DMSO-d6) δ 8.45 (d, J = 2.6 Hz, 2H), 8.35 (d, J = 8.0 Hz, 1H), 7.80 (s, 1H), 7.71 (dd, J = 8.7, 1.6 Hz, 1H), 7.53 (d, J = 8.6 Hz, 1H), 7.30 (d, J = 2.9 Hz, 1H), 5.48 (d, J = 8.1 Hz, 2H), 4.35 (s, 1H), 4.32 - 4.18 (m, 1H), 4.16 - 4.04 (m, 2H), 4.00 - 3.90 (m, 1H), 3.83 (t, J = 11.3 Hz, 2H), 3.68 (d, J = 10.6 Hz, 1H), 3.35 (s, 1H), 3.29 - 3.23 (m, 4H), 2.94 (s, 6H), 2.84 (d, J = 12.3 Hz, 1H), 2.75 (s, 6H), 2.67 (s, 3H), 2.45 (t, J = 4.9 Hz, 4H), 2.22 (s, 3H), 2.16 - 2.06 (m, 1H), 1.36 (d, J = 6.1 Hz, 3H), 1.28 - 1.19 (m, 1H), 1.10 (t, J = 7.0 Hz, 3H), 0.90 - 0.79 (m, 6H), 0.61 (d, J = 6.2 Hz, 6H).

### Example A45. (1R,5S,6r)-N-((S,Z)-22-(5-(4-cyclopropylpiperazin-1-yl)-2-((S)-1-methoxyethyl)pyridin-3-yl)-21-ethyl-4,4,10-trimethyl-7,11-dioxo-21H-6-oxa-9,10-diaza-1(4,2)-thiazola-2(5,3)-indolacyclotridecaphane-12-yl)-3-oxabicyclo[3.1.0]hexane-6-carboxamide

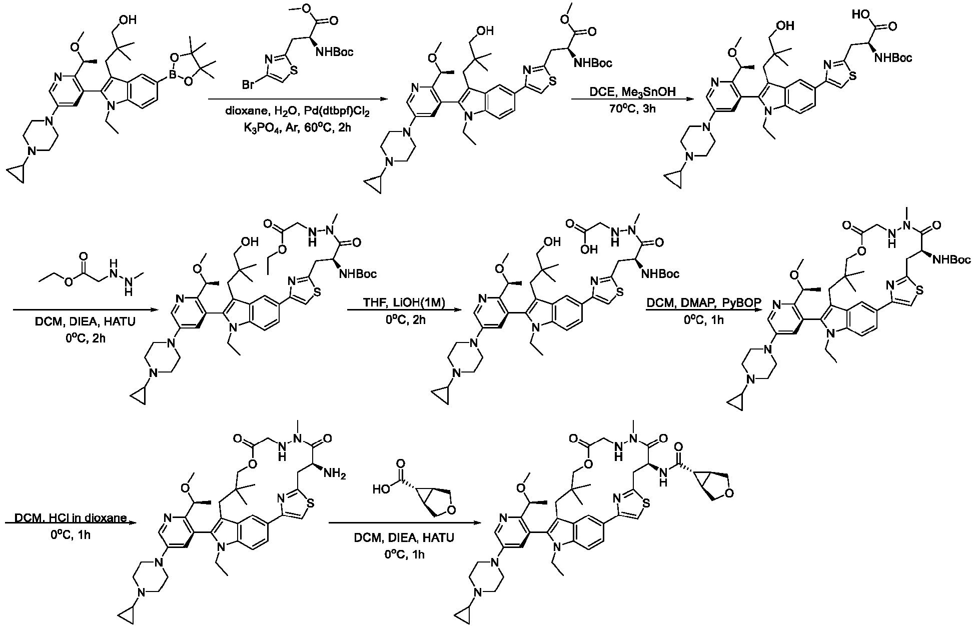

### Step 1

To a stirred solution of (*S*)-3-(2-(5-(4-cyclopropylpiperazin-1-yl)-2-(1-methoxyethyl)pyridin-3-yl)-1-ethyl-5-(4,4,5,5-tetramethyl-1,3,2-dioxaborolan-2-yl)-1*H*-indol-3-yl)-2,2-dimethylpropan-1-ol (1.75 g, 2.84 mmol, 1 equiv), methyl (*S*)-3-(4-bromothiazol-2-yl)-2-((*tert*-butoxycarbonyl)amino)propanoate (1.24 g, 3.41 mmol, 1.2 equiv) and Pd(dtbpf)Cl₂ (369.92 mg, 0.57 mmol, 0.2 equiv) in dioxane (20 mL) was added K₃PO₄ (1.5 g, 7.10 mmol, 2.5 equiv) in H₂O (4 mL) dropwise at 0°C under an argon atmosphere. The resulting mixture was stirred for 2h at 60°C. The reaction was quenched by the addition of 100 mL of NH₄Cl(aq). The mixture was extracted with DCM / MeOH (10:1) (3 x 100 mL). The combined organic layers were washed with water (3 x 100 mL) and dried over anhydrous Na₂SO₄. After filtration, the filtrate was concentrated under reduced pressure. The residue was purified by silica gel column chromatography to afford methyl (*S*)-2-((*tert*-butoxycarbonyl)amino)-3-(4-(2-(5-(4-cyclopropylpiperazin-1-yl)-2-((S)-1-methoxyethyl)pyridin-3-yl)-1-ethyl-3-(3-hydroxy-2,2-dimethylpropyl)-1*H*-indol-5-yl)thiazol-2-yl)propanoate (2.0 g, 91% yield) as a brown solid. LCMS (ESI): m/z [M+H] calc'd for C₄₂H₅₈N₆O₆S ESI-MS 774.4; found: 775.4.

### Step 2

To a stirred solution of methyl (*S*)-2-((tert-butoxycarbonyl)amino)-3-(4-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)propanoate (2.2 g, 2.84 mmol, 1 equiv) in DCE (66 mL) was added Me₃SnOH (5.13 g, 28.39 mmol, 10 equiv) at room temperature. The resulting mixture was stirred for 3h at 70°C then filtered; the filter cake was washed with DCM / MeOH (10:1) (3 x 50 mL). The filtrate was concentrated under reduced pressure to afford (*S*)-2-((tert-butoxycarbonyl)amino)-3-(4-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)propanoic acid (1.524 g, 70% yield) as a brown solid. LCMS (ESI): m/z [M+H] calc'd for C₄₁H₅₆N₆O₆S ESI-MS 760.4; found: 761.4.

### Step 3

To a stirred solution of (*S*)-2-((tert-butoxycarbonyl)amino)-3-(4-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)propanoic acid (1.165 g, 1.53 mmol, 1 equiv) in DCM (10 mL) was added DIEA (1.98 g, 15.31 mmol, 10 equiv), ethyl (methylamino)glycinate (710 mg, 5.36 mmol, 3.5 equiv) and HATU (580 mg, 1.531 mmol, 1 equiv) in portions at 0°C. The resulting mixture was stirred for 2h at 0°C. The reaction was quenched with NH₄Cl(aq) (100mL) at 0 °C then extracted with DCM/MeOH (10:1) (3 x100 mL). The combined organic layers were washed with water (3x80 mL), dried over anhydrous Na₂SO₄. After filtration, the filtrate was concentrated under reduced pressure. The residue was purified by silica gel column chromatography to afford ethyl ((*S*)-2-((tert-butoxycarbonyl)amino)-3-(4-(2-(5-(4-cyclopropylpiperazin-1-y)-2-((*S*)-1-methoxyethyl)pyridin-3-yl)-1-ethyl-3-(3-hydroxy-2,2-dimethylpropyl)-1*H*-indol-5-yl)thiazol-2-yl)-N-methylpropanamido)glycinate (705 mg, 52% yield) as a light yellow solid. LCMS (ESI): m/z [M+H] calc'd for C₄₆H₆₆N₈O₇S ESI-MS 874.5; found: 875.4.

### Step 4

To a stirred solution of ethyl ((*S*)-2-((tert-butoxycarbonyl)amino)-3-(4-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-N-methylpropanamido)glycinate (700 mg, 0.80 mmol, 1 equiv) in THF (6 mL) was added LiOH (1M) (3 mL) dropwise at 0°C. The resulting mixture was stirred for 1h at 0°C. The mixture was acidified to pH 5 with HCl (1M) (aq.). The resulting mixture was purified by reversed-phase flash chromatography to afford ((*S*)-2-((tert-butoxycarbonyl)amino)-3-(4-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-N-methylpropanamido)glycine (372 mg, 54% yield) as a light yellow solid. LCMS (ESI): m/z [M+H] calc'd for C₄₄H₆₂N₈O₇S ESI-MS 846.5; found: 846.4.

### Step 5

To a stirred solution of ((*S*)-2-((tert-butoxycarbonyl)amino)-3-(4-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-N-methylpropanamido)glycine (370 mg, 0.44 mmol, 1 equiv) in DCM (74 mL) was added DMAP (213.45 mg, 1.75 mmol, 4 equiv) and PyBOP (1818.45 mg, 3.50 mmol, 8 equiv) in portions at 0°C. The resulting mixture was stirred for 1h at 0°C. The reaction was washed with water (3 x 100 mL), dried over anhydrous Na₂SO₄. After filtration, the filtrate was concentrated under reduced pressure. The residue was purified by silica gel column chromatography to afford tert-butyl ((*S*, *Z*)-22-(5-(4-cyclopropylpiperazin-1-yl)-2-((*S*)-1-methoxyethyl)pyridin-3-yl)-21-ethyl-4,4,10-trimethyl-7,11-dioxo-21*H*-6-oxa-9,10-diaza-1(4,2)-thiazola-2(5,3)-indolacyclotridecaphane-12-yl)carbamate (101 mg, 27%yield) as a light yellow solid. LCMS (ESI): m/z [M+H] calc'd for C₄₄H₆₀N₈O₆S ESI-MS 828.4; found: 829.3.

### Step 6

To a stirred solution of tert-butyl ((*S*, *Z*)-22-(5-(4-cyclopropylpiperazin-1-yl)-2-((*S*)-1-methoxyethyl)pyridin-3-yl)-21-ethyl-4,4,10-trimethyl-7,11-dioxo-21*H*-6-oxa-9,10-diaza-1(4,2)-thiazola-2(5,3)-indolacyclotridecaphane-12-yl)carbamate (99 mg, 0.12 mmol, 1 equiv) in DCM (2 mL) was added HCl (gas) in 1,4-dioxane (2 mL) dropwise at 0°C. The resulting mixture was stirred for 1h at 0°C then concentrated under reduced pressure. This resulted in (*S, Z*)-12-amino-22-(5-(4-cyclopropylpiperazin-1-yl)-2-((S)-1-methoxyethyl)pyridin-3-yl)-21-ethyl-4,4,10-trimethyl-21*H*-6-oxa-9,10-diaza-1(4,2)-thiazola-2(5,3)-indolacyclotridecaphane-7,11-dione(125 mg, crude) as a yellow solid. LCMS (ESI): m/z [M+H] calc'd for C₃₉H₅₂N₈O₄S ESI-MS 728.4; found: 729.6.

### Step 7

To a stirred solution of (*S, Z*)-12-amino-22-(5-(4-cyclopropylpiperazin-1-yl)-2-((*S*)-1-methoxyethyl)pyridin-3-yl)-21-ethyl-4,4,10-trimethyl-21*H*-6-oxa-9,10-diaza-1(4,2)-thiazola-2(5,3)-indolacyclotridecaphane-7,11-dione (125 mg, 0.17 mmol, 1 equiv) in DCM (2 mL) was added DIEA (298.69 uL, 1.71 mmol, 10 equiv), (1*R*,5*S*,6r)-3-oxabicyclo[3.1.0]hexane-6-carboxylic acid (26.37 mg, 0.21 mmol, 1.2 equiv) and HATU (71.72 mg, 0.19 mmol, 1.1 equiv) in portions at 0°C. The resulting mixture was stirred for 1h at 0°C. The reaction was quenched with NH₄Cl(aq) at 0 °C then extracted with DCM/MeOH (10:1) (3 x 50 mL). The combined organic layers were concentrated under reduced pressure and purified by reversed-phase flash chromatography to afford (1*R*,5*S*,6r)-N-((*S, Z*)-22-(5-(4-cyclopropylpiperazin-1-yl)-2-((*S*)-1-methoxyethyl)pyridin-3-yl)-21-ethyl-4,4,10-trimethyl-7,11-dioxo-21*H*-6-oxa-9,10-diaza-1(4,2)-thiazola-2(5,3)-indolacyclotridecaphane-12-yl)-3-oxabicyclo[3.1.0]hexane-6-carboxamide (28.5 mg, 19% yield, 98.2% purity @ 254nm, 97.6% purity @ 220nm) as a white solid. LCMS (ESI): m/z [M+H] calc'd for C₄₅H₅₈N₈O₆S ESI-MS 838.4; found: 839.3. ¹H NMR (400 MHz, DMSO-d₆) δ 8.46 (d, *J* = 2.9 Hz, 2H), 8.34 (s, 1H), 7.81 (s, 1H), 7.72 (dd, *J* = 8.6, 1.6 Hz, 1H), 7.54 (d, *J* = 8.6 Hz, 1H), 7.28 (d, *J* = 2.9 Hz, 1H), 5.57 (t, *J* = 8.7 Hz, 1H), 5.30 (s, 1H), 4.31 - 4.19 (m, 1H), 4.17 - 3.90 (m, 4H), 3.82 - 3.69 (m, 3H), 3.68 - 3.55 (m, 3H), 3.41 (d, *J* = 14.9 Hz, 1H), 3.20 (d, *J* = 19.0 Hz, 5H), 3.01 (s, 3H), 2.92 (s, 3H), 2.82 (d, *J* = 13.9 Hz, 1H), 2.68 (t, *J* = 4.7 Hz, 4H), 2.39 - 2.31 (m, 1H), 1.95 - 1.85 (m, 2H), 1.70 - 1.62 (m, 1H), 1.54 (s, 1H), 1.35 (d, *J* = 6.2 Hz, 3H), 1.10 (t, *J* = 6.8 Hz, 3H), 0.65 (s, 6H), 0.44 (d, *J* = 6.2Hz, 2H), 0.38 - 0.30 (m, 2H).

### Example A10. (1S,2S)-N-((6³S,4S,Z)-1¹-ethyl-1²-(5-((S)-hexahydropyrazino[2,1-c][1,4]oxazin-8(1H)-yl)-2-((S)-1-methoxyethyl)pyridin-3-yl)-10,10-dimethyl-5,7-dioxo-6¹,6²,6³,6⁴,6⁵,6⁶-hexahydro-1¹H-8-oxa-2(4,2)-thiazola-1(5,3)-indola-6(1,3)-pyridazinacycloundecaphane-4-yl)-2-methylcyclopropane-1-carboxamide

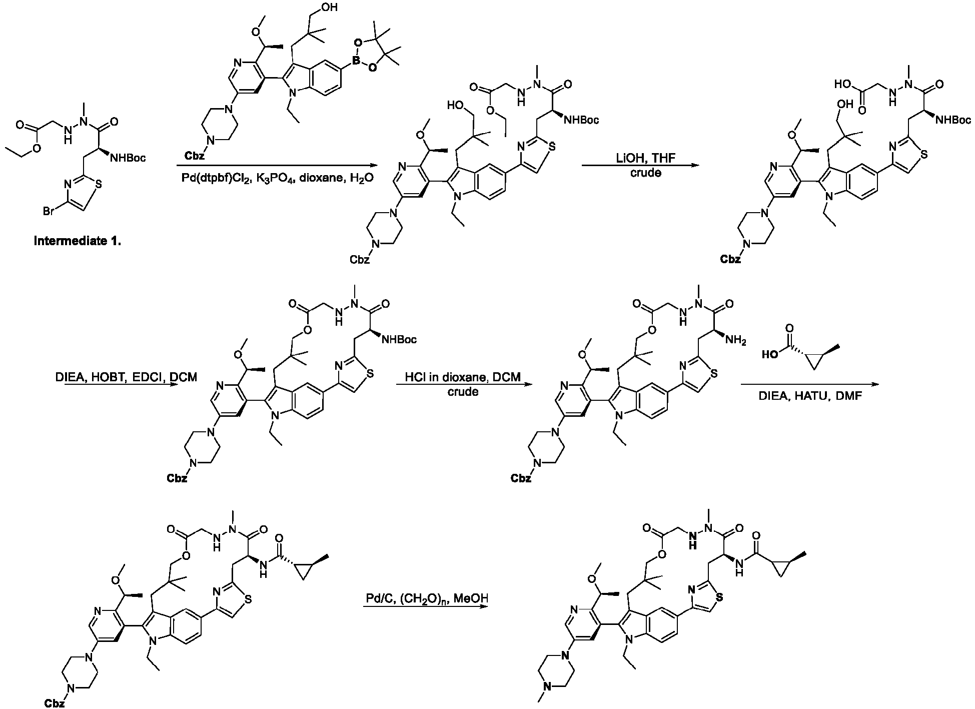

### Step 1

To a 50 mL sealed tube was added ethyl (*S*)-(3-(4-bromothiazol-2-yl)-2-((*tert-*butoxycarbonyl)amino)-N-methylpropanamido)glycinate (600 mg, 1.30 mmol, 1 equiv), dioxane (5 mL), H₂O (1 mL), benzyl (S)-4-(5-(1-ethyl-3-(3-hydroxy-2,2-dimethylpropyl)-5-(4,4,5,5-tetramethyl-1,3,2-dioxaborolan-2-yl)-1*H*-indol-2-yl)-6-(1-methoxyethyl)pyridin-3-yl)piperazine-1-carboxylate (962.17 mg, 1.35 mmol, 1.05 equiv), Pd(dtbpf)Cl₂ (168.06 mg, 0.26 mmol, 0.2 equiv) and K₃PO₄ (957.87 mg, 4.51 mmol, 3.5 equiv) at room temperature. The resulting mixture was then stirred for 2 h at 70 °C under argon atmosphere. This was quenched with H₂O (20 mL), extracted with EtOAc (3 x 20 mL) and dried over anhydrous Na₂SO₄. After filtration, the filtrate was concentrated under reduced pressure and purified by reverse phase flash chromatography to afford benzyl 4-(5-(5-(2-((*S*)-2-((*tert-*butoxycarbonyl) amino)-3-(2-(2-ethoxy-2-oxoethyl)-1-methylhydrazineyl)-3-oxopropyl) thiazol-4-yl)-1-ethyl-3-(3-hydroxy-2,2-dimethylpropyl)-1*H*-indol-2-yl)-6-((S)-1-methoxyethyl) pyridin-3-yl) piperazine-1-carboxylate (690 mg, 55.22% yield) as a light yellow solid. LCMS (ESI): m/z [M+H] calc'd for C₅₁H₆₈N₈O₉S ESI-MS 968.4; found: 969.2.

### Step 2

To a 40 mL vial was added benzyl 4-(5-(5-(2-((*S*)-2-((*tert*-butoxycarbonyl) amino)-3-(2-(2-ethoxy-2-oxoethyl)-1-methylhydrazineyl)-3-oxopropyl) thiazol-4-yl)-1-ethyl-3-(3-hydroxy-2,2-dimethylpropyl)-1*H*-indol-2-yl)-6-((S)-1-methoxyethyl) pyridin-3-yl) piperazine-1-carboxylate (695 mg, 0.72 mmol, 1 equiv), THF (3 mL), H₂O (3 mL) and LiOH (34.35 mg, 1.43 mmol, 2 equiv) at 0 °C. The resulting mixture was stirred for 1 h at room temperature. The mixture was acidified to pH 4 with 3M aq. HCl and extracted with EA (3 x 20 mL). The combined organic layers were dried over anhydrous Na₂SO₄. After filtration, the filtrate was concentrated under reduced pressure to afford ((*S*)-3-(4-(2-(5-(4-((benzyloxy)carbonyl) piperazin-1-yl)-2-((*S*)-1-methoxyethyl) pyridin-3-yl)-1-ethyl-3-(3-hydroxy-2,2-dimethylpropyl)-1*H*-indol-5-yl) thiazol-2-yl)-2-((*tert*-butoxycarbonyl) amino)-N-methylpropanamido) glycine as a light yellow solid. LCMS (ESI): m/z [M+H] calc'd for C₄₉H₆₄N₈O₉S ESI-MS 940.4; found: 941.5.

### Step 3

To a round-bottom flask was added ((*S*)-3-(4-(2-(5-(4-((benzyloxy)carbonyl) piperazin-1-yl)-2-((*S*)-1-methoxyethyl) pyridin-3-yl)-1-ethyl-3-(3-hydroxy-2,2-dimethylpropyl)-1*H*-indol-5-yl) thiazol-2-yl)-2-((*tert*-butoxycarbonyl) amino)-N-methylpropanamido) glycine (800 mg, 0.85 mmol, 1 equiv), DCM (200 mL), DIEA (3295.86 mg, 25.50 mmol, 30 equiv), HOBT (1148.59 mg, 8.50 mmol, 10 equiv) and EDCI (4888.44 mg, 25.50 mmol, 30 equiv) at 0 °C. The resulting mixture was stirred overnight at room temperature under argon atmosphere then concentrated under reduced pressure. This was diluted with H₂O (100 mL), extracted with EA (3 x 200 mL) and dried over anhydrous Na₂SO₄. After filtration, the filtrate was concentrated under reduced pressure and purified by reverse phase flash chromatography to afford benzyl 4-(5-((*S*, *Z*)-1²-((*tert*-butoxycarbonyl) amino)-2¹-ethyl-4,4,10-trimethyl-7,1¹-dioxo-2¹*H*-6-oxa-9,10-diaza-1(4,2)-thiazola-2(5,3)-indolacyclotridecaphane-2²-yl)-6-((*S*)-1-methoxyethyl) pyridin-3-yl) piperazine-1-carboxylate (300 mg, 38.23% yield) as a light yellow solid. LCMS (ESI): m/z [M+H] calc'd for C₄₉H₆₂N₈O₈S ESI-MS 922.4; found: 923.5.

### Step 4

To a 40 mL vial was added benzyl 4-(5-((S, *Z*)-1²-((*tert*-butoxycarbonyl) amino)-2¹-ethyl-4,4,10-trimethyl-7,1¹-dioxo-2¹*H*-6-oxa-9,10-diaza-1(4,2)-thiazola-2(5,3)-indolacyclotridecaphane-2²-yl)-6-((*S*)-1-methoxyethyl) pyridin-3-yl) piperazine-1-carboxylate (150 mg, 0.16 mmol, 1 equiv), DCM (2 mL) and 4M HCl in dioxane (1 mL) at 0 °C. The resulting mixture was stirred for 1 h at room temperature concentrated under reduced pressure. This resulted in benzyl 4-(5-((*S*, *Z*)-1²-amino-2¹-ethyl-4,4,10-trimethyl-7,1¹-dioxo-2¹*H*-6-oxa-9,10-diaza-1(4,2)-thiazola-2(5,3)-indolacyclotridecaphane-2²-yl)-6-((*S*)-1-methoxyethyl) pyridin-3-yl) piperazine-1-carboxylate as a light yellow solid which was used without further purification. LCMS (ESI): m/z [M+H] calc'd for C₄₄H₅₄N₈O₆S ESI-MS 822.3; found: 823.5.

### Step 5

To a 40 mL vial was added 4-(5-((*S*, *Z*)-1²-amino-2¹-ethyl-4,4,10-trimethyl-7,1¹-dioxo-2¹*H*-6-oxa-9,10-diaza-1(4,2)-thiazola-2(5,3)-indolacyclotridecaphane-2²-yl)-6-((*S*)-1-methoxyethyl) pyridin-3-yl) piperazine-1-carboxylate (150 mg, 0.18 mmol, 1 equiv), DMF (2 mL), DIEA (235.56 mg, 1.82 mmol, 10 equiv), (1*S*,2*S*)-2-methylcyclopropane-1-carboxylic acid (91.23 mg, 0.91 mmol, 5 equiv) and HATU (69.30 mg, 0.18 mmol, 1 equiv) at 0 °C. The resulting mixture was stirred for 10 min at room temperature then diluted with 20 mL of brine. This was extracted with EA (3 x 20 mL) and dried over anhydrous Na₂SO₄. After filtration, the filtrate was concentrated under reduced pressure then purified by Prep-TLC to afford benzyl 4-(5-((*S*, *Z*)-21-ethyl-4,4,10-trimethyl-1²-((1*S*,2*S*)-2-methylcyclopropane-1-carboxamido)-7,1¹-dioxo-2¹*H*-6-oxa-9,10-diaza-1(4,2)-thiazola-2(5,3)-indolacyclotridecaphane-2²-yl)-6-((S)-1-methoxyethyl) pyridin-3-yl) piperazine-1-carboxylate (130 mg, 78.81% yield) as a light yellow solid. LCMS (ESI): m/z [M+H] calc'd for C₄₉H₆₀N₈O₇S ESI-MS 904.4; found: 905.4.

### Step 6

To a 40 mL vial was added benzyl 4-(5-((*S, Z*)-21-ethyl-4,4,10-trimethyl-1²-((1*S*,2*S*)-2-methylcyclopropane-1-carboxamido)-7,1¹-dioxo-2¹*H*-6-oxa-9,10-diaza-1(4,2)-thiazola-2(5,3)-indolacyclotridecaphane-2²-yl)-6-((*S*)-1-methoxyethyl) pyridin-3-yl) piperazine-1-carboxylate (130 mg, 0.14 mmol, 1 equiv), MeOH (2 mL), Pd/C (30 mg, 0.28 mmol, 1.96 equiv) and methoxymethanol amine (63.27 mg, 1.44 mmol, 10 equiv) at room temperature. The resulting mixture was stirred overnight at room temperature under hydrogen atmosphere then filtered, and the filter cake washed with EA (3x 20 mL). The filtrate was concentrated under reduced pressure and the resting residue was purified by reverse phase flash chromatography to afford (2*S*)-*N*-((*S,Z*)-2¹-ethyl-2²-(2-((*S*)-1-methoxyethyl)-5-(4-methylpiperazin-1-yl)pyridin-3-yl)-4,4,10-trimethyl-7,1¹-dioxo-2¹*H*-6-oxa-9,10-diaza-1(4,2)-thiazola-2(5,3)-indolacyclotridecaphane-1²-yl)-2-methylcyclopropane-1-carboxamide (16 mg, 13.86% yield) as a white solid. LCMS (ESI): m/z [M+H] calc'd for C₄₂H₅₆N₈O₅S ESI-MS 784.4; found: 785.4.¹H NMR (400 MHz, DMSO-d6) δ = 8.45 (s, 1H), 8.37 (dd, J = 19.1, 6.4 Hz, 2H), 7.78 (d, J = 4.2 Hz, 1H), 7.71 (d, J = 8.6 Hz, 1H), 7.53 (d, J = 8.6 Hz, 1H), 7.27 (s, 1H), 5.65 - 5.47 (m, 1H), 5.30 (s, 1H), 4.22 (d, J = 15.9 Hz, 1H), 4.08 (dt, J = 20.6, 7.4 Hz, 3H), 4.01 - 3.87 (m, 1H), 3.72 (s, 2H), 3.33 - 3.20 (m, 6H), 3.00 (s, 3H), 2.96 (s, 3H), 2.79 (d, J = 14.0 Hz, 1H), 2.55 (s, 4H), 2.46 (d, J = 5.3 Hz, 1H), 2.34 (s, 3H), 1.49 - 1.28 (m, 4H), 1.08 (d, J = 7.5 Hz, 3H), 1.04 (s, 4H), 0.83 (d, J = 6.4 Hz, 1H), 0.64 (d, J = 13.1 Hz, 6H), 0.46 (dd, J = 8.2, 4.8 Hz, 1H).

### Example A12. (1S,2S)-N-((63S,64R,4S,Z)-11,64-diethyl-12-(2-((S)-1-methoxyethyl)-5-(4-methylpiperazin-1-yl)pyridin-3-yl)-10,10-dimethyl-5,7-dioxo-61,62,63,64,65,66-hexahydro-11H-8-oxa-2(4,2)-thiazola-1(5,3)-indola-6(1,3)-pyridazinacycloundecaphane-4-yl)-2-methylcyclopropane-1-carboxamide

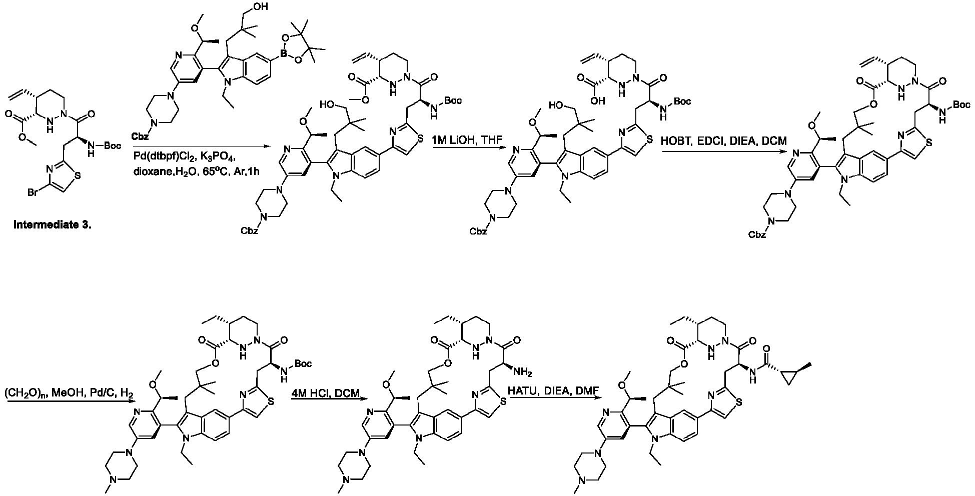

### Step 1

A solution of methyl (3*S*,4*S*)-1-[(2*S*)-3-(4-bromo-1,3-thiazol-2-yl)-2-[(*tert-*butoxycarbonyl)amino]propanoyl]-4-ethenyl-1,2-diazinane-3-carboxylate (200 mg, 0.397 mmol, 1 equiv), benzyl 4-[(5M)-5-[1-ethyl-3-(3-hydroxy-2,2-dimethylpropyl)-5-(4,4,5,5-tetramethyl-1,3,2-dioxaborolan-2-yl)indol-2-yl]-6-[(1*S*)-1-methoxyethyl]pyridin-3-yl]piperazine-1-carboxylate (310.60 mg, 0.43 mmol, 1.1 equiv), Pd(dtbpf)Cl₂ (51.79 mg, 0.08 mmol, 0.2 equiv) and K₃PO₄ (210.83 mg, 0.99 mmol, 2.5 equiv) in dioxane (2 mL) and H₂O (0.4 mL) was stirred for 1 h at 65 °C under argon atmosphere. The reaction mixture was diluted with H₂O (20 mL). The resulting mixture was extracted with EtOAc (3 x 20 mL) and the combined organic layers were dried over anhydrous Na₂SO₄. After filtration, the filtrate was concentrated under reduced pressure and the resulting residue was purified by Prep-TLC to afford methyl (3*S*,4*S*)-1-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1H-indol-5-yl)thiazol-2-yl)-2-((*tert*-butoxycarbonyl)amino)propanoyl)-4-vinylhexahydropyridazine-3-carboxylate (300 mg, 71.22% yield) as a yellow solid. LCMS (ESI): m/z [M+H] calc'd for C₅₄H₇₀N₈O₉S ESI-MS 1006.5; found: 1007.3.

### Step 2

To a stirred solution of methyl (3*S*,4*S*)-1-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-2-((*tert*-butoxycarbonyl)amino)propanoyl)-4-vinylhexahydropyridazine-3-carboxylate (330 mg, 0.03 mmol, 1 equiv) in THF (6 mL) and H₂O (2 mL) was added LiOH (15.69 mg, 0.66 mmol, 2 equiv) in portions at 0 °C. The resulting mixture was stirred for 2 h at room temperature. The mixture was acidified to pH 3 with 1M HCl. The resulting mixture was extracted with EtOAc (3 x 20 mL). The combined organic layers were dried over anhydrous Na₂SO₄. After filtration, the filtrate was concentrated under reduced pressure. This afforded (3*S*,4*S*)-1-((S)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-2-((*tert*-butoxycarbonyl)amino)propanoyl)-4-vinylhexahydropyridazine-3-carboxylic acid (300 mg, 82.97% yield) as a yellow solid. The crude product was used in the next step directly without further purification. LCMS (ESI): m/z [M+H] calc'd for C₅₃H₆₈N₈O₉S ESI-MS 992.5; found: 993.7.

### Step 3

To a stirred solution of (3*S*,4*S*)-1-((*S*)-3-(4-(2-(5-(4-((benzyloxy)carbonyl)piperazin-1-yl)-2-((*S*)-1-methoxyethyl)pyridin-3-yl)-1-ethyl-3-(3-hydroxy-2,2-dimethylpropyl)-1*H*-indol-5-yl)thiazol-2-yl)-2-((*tert*-butoxycarbonyl)amino)propanoyl)-4-vinylhexahydropyridazine-3-carboxylic acid (300 mg, 0.30 mmol, 1 equiv), DIEA (1.17 g, 9.06 mmol, 30 equiv) and HOBT (0.41 g, 3.02 mmol, 10 equiv) in DCM (30 mL) was added EDCI (1.74 g, 9.06 mmol, 30 equiv) in portions at 0 °C under argon atmosphere. The reaction was stirred overnight at room temperature then quenched by the addition of Water/Ice (10 mL) at 0 °C. The resulting mixture was extracted with EtOAc (3 x 30 mL) and the combined organic layers were dried over anhydrous Na₂SO₄. After filtration, the filtrate was concentrated under reduced pressure and purified by Prep-TLC to afford benzyl 4-(5-((6³*S*,6⁴*S*,4*S,Z*)-4-((*tert-*butoxycarbonyl)amino)-1¹-ethyl-10,10-dimethyl-5,7-dioxo-64-vinyl-6¹,6²,6³,6⁴,6⁵,6⁶-hexahydro-1¹*H*-8-oxa-2(4,2)-thiazola-1(5,3)-indola-6(1,3)-pyridazinacycloundecaphane-1²-yl)-6-((*S*)-1-methoxyethyl)pyridin-3-yl)piperazine-1-carboxylate (120 mg, 36.66% yield) as a yellow solid. LCMS (ESI): m/z [M+H] calc'd for C₅₃H₆₆N₈O₈S ESI-MS 974.5; found: 975.6.

### Step 4

A solution of benzyl 4-(5-((6³*S*,6⁴*S*,4*S,Z*)-4-((*tert*-butoxycarbonyl)amino)-1¹-ethyl-10,10-dimethyl-5,7-dioxo-64-vinyl-6¹,6²,6³,6⁴,6⁵,6⁶-hexahydro-1¹*H*-8-oxa-2(4,2)-thiazola-1(5,3)-indola-6(1,3)-pyridazinacycloundecaphane-1²-yl)-6-((S)-1-methoxyethyl)pyridin-3-yl)piperazine-1-carboxylate (110 mg, 0.11 mmol, 1 equiv), Pd/C (109.95 mg) and paraformaldehyde (109.99 mg, 2.45 mmol, 21.64 equiv) in MeOH (2 mL) was stirred overnight at room temperature under hydrogen atmosphere. The resulting mixture was filtered, the filter cake was washed with EtOAc (3 x 10 mL). The filtrate was concentrated under reduced pressure affording *tert*-butyl ((6³*S*,6⁴*R*,4*S,Z*)-1¹,6⁴-diethyl-1²-(2-((*S*)-1-methoxyethyl)-5-(4-methylpiperazin-1-yl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)carbamate (100 mg, 93.09% yield) as a yellow solid which was used without further purification. LCMS (ESI): m/z [M+H] calc'd for C₄₆H₆₄N₈O₆S ESI-MS 856.5; found: 857.9.

### Step 5

To a stirred solution of *tert*-butyl ((6³*S*,6⁴*R*,4*S,Z*)-1¹,6⁴-diethyl-1²-(2-((*S*)-1-methoxyethyl)-5-(4-methylpiperazin-1-yl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)carbamate (120 mg, 0.14 mmol, 1 equiv) in DCM (6 mL) was added 4M HCl in 1,4-dioxane (1 mL, 4.00 mmol, 28.57 equiv) dropwise at 0 °C. The resulting mixture was stirred for 1 h at room temperature then concentrated under reduced pressure and the crude product was used in the next step directly without further purification. This resulted in (6³*S*,6⁴*R*,4*S,Z*)-4-amino-1¹,64-diethyl-1²-(2-((*S*)-1-methoxyethyl)-5-(4-methylpiperazin-1-yl)pyridin-3-yl)-10,10-dimethyl-6¹,6²,6³,6⁴,6⁵,6⁶-hexahydro-1¹*H*-8-oxa-2(4,2)-thiazola-1(5,3)-indola-6(1,3)-pyridazinacycloundecaphane-5,7-dione (110 mg, 93.41% yield) as a yellow solid. LCMS (ESI): m/z [M+H] calc'd for C₄₁H₅₆N₈O₄S ESI-MS 756.4; found: 757.2.

### Step 6

To a stirred solution of (6³*S*,6⁴*R*,4*S,*Z)-4-amino-1¹,64-diethyl-1²-(2-((*S*)-1-methoxyethyl)-5-(4-methylpiperazin-1-yl)pyridin-3-yl)-10,10-dimethyl-6¹,6²,6³,6⁴,6⁵,6⁶-hexahydro-1¹H-8-oxa-2(4,2)-thiazola-1(5,3)-indola-6(1,3)-pyridazinacycloundecaphane-5,7-dione (120 mg, 0.159 mmol, 1 equiv), (1*S*,2*S*)-2-methylcyclopropane-1-carboxylic acid (23.81 mg, 0.24 mmol, 1.5 equiv) and DIEA (409.76 mg, 3.18 mmol, 20 equiv) in DMF (1.5 mL) was added HATU (90.41 mg, 0.24 mmol, 1.5 equiv) in portions at 0 °C. The resulting mixture was stirred for 1 h at room temperature then quenched with Water/Ice at 0 °C. This was extracted with EtOAc (3 x20 mL). The combined organic layers were washed with brine (3x50 mL) and dried over anhydrous Na₂SO₄. After filtration, the filtrate was concentrated under reduced pressure and the crude product was purified by reverse phase flash chromatography to afford (1*S*,2*S*)-N-((6³*S*,6⁴*R*,4*S,Z*)-1¹,6⁴-diethyl-1²-(2-((*S*)-1-methoxyethyl)-5-(4-methylpiperazin-1-yl)pyridin-3-yl)-10,10-dimethyl-5,7-dioxo-6¹,6²,6³,6⁴,6⁵,6⁶-hexahydro-1¹*H*-8-oxa-2(4,2)-thiazola-1(5,3)-indola-6(1,3)-pyridazinacycloundecaphane-4-yl)-2-methylcyclopropane-1-carboxamide (14.4 mg, 9.83% yield, 90.8% purity @254nm, 88.3% purity @220nm) as a white solid. LCMS (ESI): m/z [M+H] calc'd for C₄₆H₆₂N₈O₅S ESI-MS 838.5; found: 839.9. ¹H NMR (400 MHz, DMSO-d6) δ 8.50 (d, J = 9.0 Hz, 1H), 8.44 (d, J = 2.6 Hz, 2H), 7.79 (s, 1H), 7.72 (dd, J = 8.6, 1.6 Hz, 1H), 7.55 (d, J = 8.6 Hz, 1H), 7.20 (d, J = 2.9 Hz, 1H), 5.61 (t, J = 9.1 Hz, 1H), 5.26 (d, J = 12.6 Hz, 1H), 4.44 (dd, J = 12.8, 3.6 Hz, 1H), 4.34 - 4.24 (m, 1H), 4.15 (p, J = 6.9, 6.2 Hz, 2H), 4.05 (d, J = 12.4 Hz, 1H), 3.63 (d, J = 11.0 Hz, 1H), 3.43 (d, J = 10.9 Hz, 1H), 3.30 (m, 4H), 3.19 (m, 3H), 3.14 (dd, J = 14.8, 9.3 Hz, 1H), 2.98 - 2.87 (m, 1H), 2.81 (s, 1H), 2.49 - 2.38 (m, 5H), 2.21 (s, 3H), 1.97 (s, 1H), 1.80 (d, J = 12.7 Hz, 2H), 1.68 (s, 1H), 1.49 (dd, J = 8.2, 4.5 Hz, 1H), 1.32 (d, J = 6.1 Hz, 3H), 1.09 - 1.03 (m, 5H), 0.93 - 0.81 (m, 10H), 0.55 (dd, J = 7.9, 5.1 Hz, 1H), 0.34 (s, 3H).

### Example A17. (1S,2S)-N-((64S,4S)-11-ethyl-12-(2-((S)-1-methoxyethyl)-5-(4-methylpiperazin-1-yl)pyridin-3-yl)-10,10-dimethyl-5,7-dioxo-11H-8-oxa-62,63-diaza-1(5,3)-indola-6(2,4)-bicyclo[3.1.1]heptana-2(1,3)-benzenacycloundecaphane-4-yl)-2-methylcyclopropane-1-carboxamide

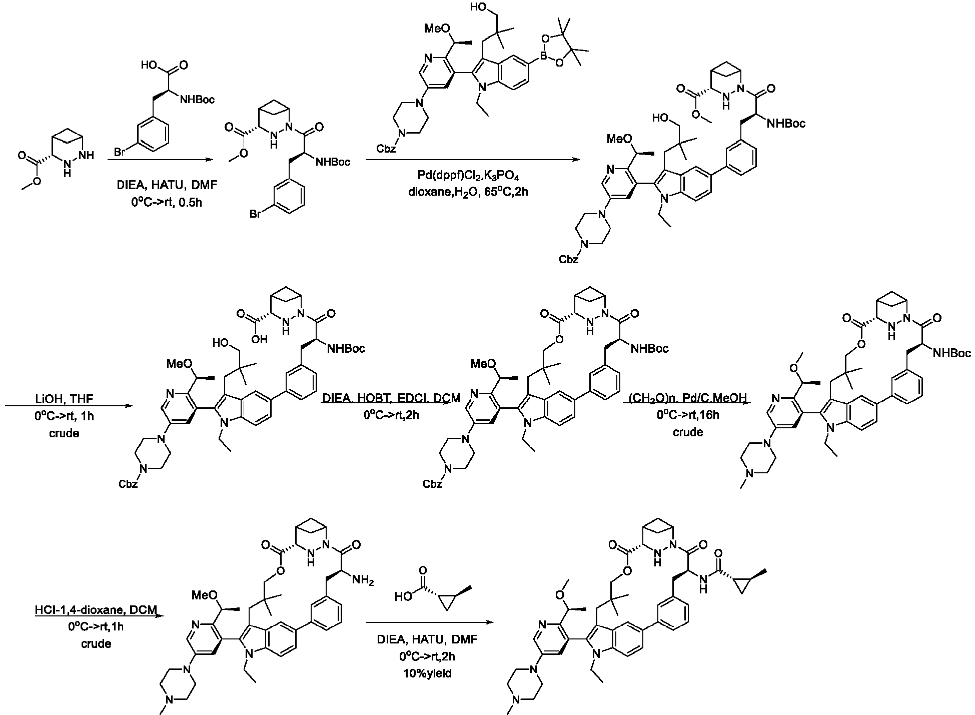

### Step 1

To a stirred solution of methyl (4*S*)-2,3-diazabicyclo[3.1.1]heptane-4-carboxylate (200mg, 1.02mol, 1 equiv) and DIEA (2482.58 mg, 19.215 mmol, 15 equiv) in DMF were added (2S)-3-(3-bromophenyl)-2-[(*tert*-butoxycarbonyl)amino] propanoic acid (440.77 mg, 1.281 mmol, 1.00 equiv) and HATU (730.36 mg, 1.921 mmol, 1.5 equiv) at 0 °C under an argon atmosphere. The resulting mixture was stirred for additional 0.5 h at room temperature. The resulting mixture was diluted with water (100 mL) then extracted with EtOAc (3 x 100mL) and dried over anhydrous Na₂SO₄. After filtration, the filtrate was concentrated under reduced pressure and the residue was purified by reverse flash chromatography to give methyl (4*S*)-2-[(2*S*)-3- (3-bromophenyl)-2-[(*tert-*butoxycarbonyl)amino]propanoyl]-2,3-diazabicyclo[3.1.1]heptane-4-carboxylate (250 mg, 40% yield) as a yellow oil. LCMS (ESI): m/z [M+H] calc'd for C₂₁H₂₈BrN₃O₅ ESI-MS 481.1; found: 482.2.

### Step 2

To a solution of methyl (4*S*)-2-[(2*S*)-3-(3-bromophenyl)-2-[(*tert-*butoxycarbonyl)amino]propanoyl]-2,3-diazabicyclo [3.1.1]heptane-4-carboxylate (220 mg, 0.456 mmol, 1 equiv) and benzyl4-[(5M)-5-[1-ethyl-3-(3-hydroxy-2,2-dimethylpropyl)-5-(4,4,5,5-tetramethyl-1,3,2-dioxaborolan-2-yl)indol-2-yl]-6-[(1*S*)-1-methoxyethyl]pyridin-3-yl]piperazine-1-carboxylate (291.73 mg, 0.41 mmol, 0.9 equiv) in dioxane (3 mL) and H₂O (0.6 mL) was added K₃PO₄ (242.02 mg, 1.14 mmol, 2.5 equiv) and Pd(dppf)Cl₂ (66.74 mg, 0.09 mmol, 0.2 equiv). After stirring for 2 h at 65 °C under an argon atmosphere. The resulting mixture was diluted with H₂O (15 mL), extracted with EtOAc (3 x 15mL) and dried over anhydrous Na₂SO₄. This was concentrated under reduced pressure and the residue was purified by Prep-TLC) to afford methyl(4*S*)-2-[(2*S*)-3-{3-[(2M)-2-(5-{4-[(benzyloxy)carbonyl] piperazin-1-yl}-2-[(1*S*)-1-methoxyethyl]pyridin-3-yl)-1-ethyl-3-(3-hydroxy-2,2-dimethylpropyl)indol-5-yl]phenyl}-2-[(*tert*-butoxycarbonyl)amino]propanoyl]-2,3-diazabicyclo[3.1.1]heptane-4-carboxylate (216 mg, 45% yield) as a white solid. LCMS (ESI): m/z [M+H] calc'd for C₅₆H₇₁N₇O₉ ESI-MS 985.5; found: 985.6.

### Step 3

To a stirred mixture of methyl (4*S*)-2-[(2*S*)-3-{3-[(2M)-2-(5-{4-[(benzyloxy)carbonyl]piperazin-1-yl}-2-[(1*S*)-1- methoxyethyl]pyridin-3-yl)-1-ethyl-3-(3-hydroxy-2,2-dimethylpropyl)indol-5-yl]phenyl}-2-[(*tert*-butoxycarbonyl)amino]propanoyl]-2,3-diazabicyclo[3.1.1]heptane-4-carboxylate (166 mg, 0.153 mmol, 1 equiv) in THF (3 mL) was added H₂O (1.5 mL) and LiOH•H₂O (31.91 mg, 0.760 mmol, 5.0 equiv) in portions at 0 °C under nitrogen atmosphere. The resulting mixture was stirred for additional 1 h at room temperature then acidified to pH 6 with 1M HCl. The resulting mixture was extracted with EtOAc (3 x 30mL and dried over anhydrous Na₂SO₄. After filtration, the filtrate was concentrated under reduced pressure and the crude product (150 mg) was used in the next step directly without further purification. LCMS (ESI): m/z [M+H] calc'd for C₅₅H₆₉N₇O₉ ESI-MS 971.5; found: 972.4.

### Step 4

To a stirred mixture of (4*S*)-2-[(2*S*)-3-{3-[(2M)-2-(5-{4-[(benzyloxy)carbonyl]piperazin-1-yl}-2-[(1*S*)-1-methoxyethyl] pyridin-3-yl)-1-ethyl-3-(3-hydroxy-2,2-dimethylpropyl)indol-5-yl]phenyl}-2-[(*tert-*butoxycarbonyl)amino]propanoyl]-2,3-diazabicyclo[3.1.1]heptane-4-carboxylic acid (150 mg, 0.15 mmol, 1 equiv) and DIEA (598.24 mg, 4.62 mmol, 30 equiv) in DCM (25 mL) was added HOBT (208.49 mg, 1.54 mmol, 10 equiv) and EDCI (887.32 mg, 4.62 mmol, 30 equiv) at 0 °C under nitrogen atmosphere. The resulting mixture was stirred for an additional 16 h at room temperature then concentrated under reduced pressure. The resulting residue was diluted with H₂O (50 mL)and extracted with EtOAc (3 x 50mL) and dried over anhydrous Na₂SO₄. The resulting mixture was concentrated under reduced pressure and the residue purified by Prep-TLC to afford benzyl-4-(5-((64*S*,4*S*)-4-((*tert*-butoxycarbonyl) amino)-11-ethyl-10,10- dimethyl-5,7-dioxo-11H-8-oxa-62,63-diaza-1(5,3)-indola-6(2,4)-bicyclo[3.1.1]heptana-2(1,3)-benzenacycloundecaphane-12-yl)-6-((*S*)-1-methoxyethyl)pyridin-3-yl)piperazine-1-carboxylate (40 mg, 27% yield) as a white solid. LCMS (ESI): m/z [M+H] calc'd for C₅₅H₆₇N₇O₈ ESI-MS 953.5; found: 954.6.

### Step 5

To a stirred mixture of benzyl 4-(5-((64*S*,4*S*)-4-((*tert*-butoxycarbonyl)amino)-11-ethyl-10,10-dimethyl-5,7-dioxo-11*H*-8-oxa-62,63-diaza-1(5,3)-indola-6(2,4)-bicyclo[3.1.1]heptana-2(1,3)-benzenacycloundecaphane-12-yl)-6-((*S*)-1-methoxyethyl)pyridin-3-yl)piperazine-1-carboxylate (40 mg, 0.04 mmol, 1 equiv) and Paraformaldehyde (40 mg, 0.44 mmol, 10.59 equiv) in MeOH (2 mL) was added Pd/C (40 mg, 0.37 mmol, 8.97 equiv) at 0 °C under hydrogen atmosphere. The resulting mixture was stirred for additional 16 h at room temperature, then filtered, and the filter cake was washed with MeOH (3 x 10 mL). The filtrate was concentrated under reduced pressure and was used in the next step directly without further purification. LCMS (ESI): m/z [M+H] calc'd for C₄₈H₆₃N₇O₆ ESI-MS 833.5; found: 834.6

### Step 6

To a stirred solution of *tert*-butyl ((64*S*,4*S*)-11-ethyl-12-(2-((S)-1-methoxyethyl)-5-(4-methylpiperazin-1-yl)pyridin-3-yl)- 10,10-dimethyl-5,7-dioxo-11*H*-8-oxa-62,63-diaza-1(5,3)-indola-6(2,4)-bicyclo[3.1.1]heptana-2(1,3)-benzenacycloundecaphane-4-yl)carbamate (28 mg, 0.03 mmol, 1 equiv) in DCM (3.0 mL) was added 4M HCl (gas)in 1,4-dioxane (1.5 mL) dropwise at 0 °C. The resulting mixture was stirred for additional 1 h at room temperature and concentrated under reduced pressure. The crude product (30 mg) was used in the next step directly without further purification. LCMS (ESI): m/z [M+H] calc'd for C₄₃H₅₅N₇O₄ ESI-MS 733.4; found: 734.4.

### Step 7

To a stirred mixture of (64*S*,4*S*)-4-amino-11-ethyl-12-(2-((*S*)-1-methoxyethyl)-5-(4-methylpiperazin-1-yl)pyridin-3-yl)-10,10-dimethyl-11*H*-8-oxa-62,63-diaza-1(5,3)-indola-6(2,4)-bicyclo[3.1.1]heptana-2(1,3)-benzenacycloundecaphane-5,7-dione (30 mg, 0.04 mmol, 1.00 equiv), DMF (1.0 mL) and DIEA (79.2 mg, 0.06 mmol, 15.0 equiv) was added (1S,2S)-2-methyl cyclopropane-1-carboxylic acid (18.41 mg, 0.18 mmol, 4.5 equiv) and HATU (233.13 mg, 0.615 mmol, 15 equiv) at 0 °C. The resulting mixture was stirred for 2 h at room temperature then diluted with water (10 mL). The resulting mixture was extracted with EtOAc (3 x 10mL), dried over anhydrous Na₂SO₄, filtered and the filtrate was concentrated under reduced pressure. The crude product (20 mg) was purified by Prep-HPLC to afford (1*S*,2*S*)-N-((64*S*,4*S*)-11-ethyl-12-(2-((*S*)-1-methoxyethyl)-5-(4-methylpiperazin-1-yl)pyridin-3-yl)-10,10-dimethyl-5,7-dioxo-11H-8-oxa-62,63-diaza-1(5,3)-indola-6(2,4)-bicyclo[3.1.1]heptana-2(1,3)-benzenacycloundecaphane-4-yl)-2-methylcyclopropane-1-carboxamide (2.9 mg, 10 %yield, 95.6% purity at 254 nm) as a white solid. LCMS (ESI): m/z [M+H] calc'd for C₄₈H₆₁N₇O₅ ESI-MS 815.5; found: 816.5. ¹H NMR (400 MHz, DMSO-d6) δ 8.38 (d, J = 2.8 Hz, 1H), 8.31 (d, J = 8.4 Hz, 1H), 7.77 (d, J = 7.3 Hz, 2H), 7.60 - 7.34 (m, 3H), 7.34 - 7.10 (m, 2H), 7.02 (d, J = 7.5 Hz, 1H), 6.01 (d, J = 10.8 Hz, 1H), 5.37 (q, J = 7.1 Hz, 1H), 4.42 (q, J = 4.9 Hz, 1H), 4.24 - 4.08 (m, 1H), 4.08 - 3.90 (m, 2H), 3.71 (d, J = 11.2 Hz, 1H), 3.44 (d, J = 4.6 Hz, 2H), 3.20 (t, J = 5.1 Hz, 4H), 2.98 - 2.79 (ddd, J = 34.7, 13.3, 6.3 Hz, 3H), 2.66 - 2.50 (m, 1H), 2.38 (s, 4H), 2.33 - 2.22 (m, 1H), 2.15 (s, 4H), 2.02 (t, J = 9.8 Hz, 1H), 1.39 (dt, J = 7.8, 3.3 Hz, 1H), 1.30 (d, J = 6.1 Hz, 3H), 1.17 (s, 1H), 1.08 (t, J = 9.2 Hz, 1H), 1.02 - 0.90 (m, 7H), 0.86 - 0.70 (m, 2H), 0.68 (s, 3H), 0.58 - 0.29 (m, 4H).

### Example A25. (1S,2S)-N-((22S,64S,4S)-11-ethyl-12-(2-((S)-1-methoxyethyl)-5-(4-methylpiperazin-1-yl)pyridin-3-yl)-1 0,1 0-dimethyl-5,7 -dioxo-11H-8-oxa-62,63-diaza-2(4,2)-morpholina-1(5,3)-indola-6(2,4)-bicyclo[3.1.1]heptanacycloundecaphane-4-yl)-2-methylcyclopropane-1-carboxamide

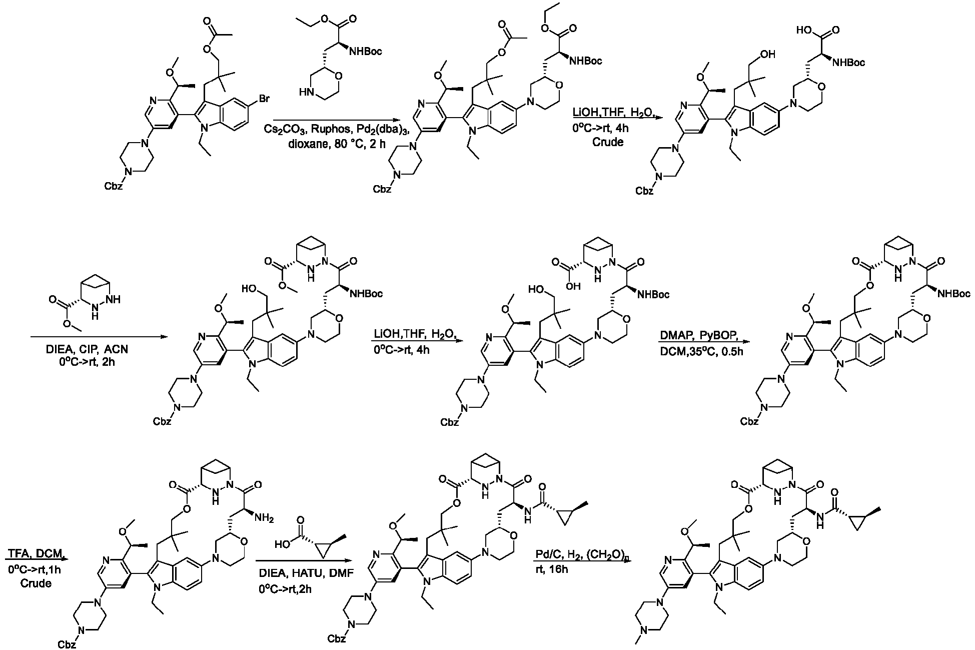

### Step 1

A solution of benzyl-(*S*)-4-(5-(3-(3-acetoxy-2,2-dimethylpropyl)-5-bromo-1-ethyl-1*H*-indol-2-yl)-6-(1-methoxyethyl) pyridin-3-yl)piperazine-1-carboxylate (1.5 g, 2.126 mmol, 1 equiv) and ethyl-(*S*)-2-((*tert*-butoxycarbonyl)amino)-3- ((*S*)-morpholin-2-yl)propanoate (0.77 g, 2.55 mmol, 1.2 equiv) , Cs₂CO₃ (1.73 g, 5.32 mmol, 2.5 equiv), Ruphos (0.20 g, 0.43 mmol, 0.2 equiv) , Pd₂(dba)₃ (0.19 g, 0.21 mmol, 0.1 equiv) in Dioxane (150 mL) was stirred for 2 h at 80 °C under an argon atmosphere. The resulting mixture was diluted with water (100 mL) then extracted with EtOAc (3 x 100 mL). The combined organic layers were washed with brine (3 x 200 mL), dried over anhydrous Na₂SO₄ and the resulting solution was concentrated under reduced pressure then purified by silica gel column chromatography to afford benzyl-4-(5-(3-(3-acetoxy-2,2-dimethylpropyl)-5-((*S*)-2-((*S*)-2-(*(tert-*butoxycarbonyl)amino)-3-ethoxy-3- oxopropyl)morpholino)-1-ethyl-1*H*-indol-2-yl)-6-((*S*)-1-methoxyethyl)pyridin-3-yl)piperazine-1-carboxylate (1.6 g, 81% yield) as a brown solid. LCMS (ESI): m/z [M+H] calc'd for C₅₁H₇₀N₆O₁₀ ESI-MS 926.5; found: 927.4.

### Step 2

To a round-bottom flask was added benzyl-4-(5-(3-(3-acetoxy-2,2-dimethylpropyl)-5-((*S*)-2-((*S*)-2-(*(tert*-butoxycarbonyl)amino)-3-ethoxy-3-oxopropyl)morpholino)-1-ethyl-1*H*-indol-2-y1)-6-((*S*)-1-methoxyethyl)pyridin-3-yl)piperazine-1-carboxylate (1.6 g, 1.73 mmol, 1 equiv),THF (20 mL) , and H₂O (10 mL) at 0° C. LiOH (0.21 g, 8.63 mmol, 5 equiv) is then added and the resulting mixture was stirred for 4 h at room temperature under argon atmosphere. The mixture was then acidified to pH 5 with 1M HCl. The aqueous layer was extracted with EtOAc (3 x 90 mL), dried over anhydrous Na₂SO₄ and the resulting mixture was concentrated under reduced pressure. The crude product (1.64 g, crude) was used in the next step directly without further purification. LCMS (ESI): m/z [M+H] calc'd for C₄₇H₆₉N₆O₉ ESI-MS 856.5; found: 857.6.

### Step 3

To a 40 mL vial were added methyl (S)-2,3-diazabicyclo[3.1.1]heptane-4-carboxylate (200 mg, 1.28 mmol, 1 equiv) and ACN (6 mL) at 0 °C. Next, DIEA (2478.6 mg, 19.22 mmol, 15 equiv), (*S*)-3-((*S*)-4-(2-(5-(4-((benzyloxy)carbonyl)piperazin-1-yl)-2-((*S*)-1-methoxyethyl)pyridine-3-yl)-1-ethyl-3-(3-hydroxy-2,2-dimethylpropyl)-1*H*-indol-5-yl)morpholin-2-yl)-2-((*tert*-butoxycarbonyl)amino)propanoic acid (1646.24 mg, 1.92 mmol, 1.5 equiv) , and CIP (535.06 mg, 1.92 mmol, 1.5 equiv) are added at 0 °C, The resulting mixture was stirred for 2 h at room temperature under argon atmosphere then diluted with H₂O (100 mL). The resulting mixture was extracted with EtOAc (3 x 100 mL) and dried over anhydrous Na₂SO₄. After filtration, the filtrate was concentrated under reduced pressure and purified by Prep-TLC to afford methyl(*S*)-2-((*S*)-3-((*S*)-4-(2-(5-(4-((benzyloxy)carbonyl)piperazin-1-yl)-2-((*S*)-1-methoxyethyl)pyridine-3-yl)-1-ethyl-3-(3-hydroxy-2,2-dimethylpropyl)-1*H*-indol-5-yl)morpholin-2-yl)-2-((*tert*-butoxycarbonyl)amino)propanoyl)-2,3-diazabicyclo[3.1.1]heptane-4-carboxylate (520 mg, 40% yield) as a brown solid. LCMS (ESI): m/z [M+H] calc'd for C₅₄H₇₄N₈O₁₀ ESI-MS 994.6; found: 995.7.

### Step 4

To a 40 mL vial was added methyl (*S*)-2-((*S*)-3-((*S*)-4-(2-(5-(4-((benzyloxy)carbonyl)piperazin-1-yl)-2-((*S*)-1-met hoxyethyl)pyridin-3-yl)-1-ethyl-3-(3-hydroxy-2,2-dimethylpropyl)-1*H*-indol-5-yl)morpholin-2-yl)-2-((*tert*-butoxycarbonyl)amino)propanoyl)-2,3-diazabicyclo[3.1.1]heptane-4-carboxylate (520 mg, 0.52 mmol, 1 equiv), THF (5 mL), and H₂O (5 mL) at 0° C. To the mixture was added LiOH (37.54 mg, 1.57 mmol, 3 equiv) at 0 °C. The resulting mixture was stirred for 4 h at room temperature under an argon atmosphere, then acidified to pH 5 with 1M HCl. The aqueous layer was extracted with EtOAc (3 x 80 mL) and the resulting mixture was concentrated under reduced pressure. The crude product (480 mg, crude) was used in the next step directly without further purification. LCMS (ESI): m/z [M+H] calc'd for C₅₃H₇₂N₈O₁₀ ESI-MS 980.5; found: 981.5.

### Step 5

To a round-bottom flask was added (*S*)-2-((*S*)-3-((*S*)-4-(2-(5-(4-((benzyloxy)carbonyl)piperazin- 1-yl)-2-((*S*)-1-methoxyethyl)pyridin-3-yl)-1-ethyl-3-(3-hydroxy-2,2-dimethylpropyl)-1*H*-indol-5-yl)morpholin-2-yl)-2-((*tert*-butoxycarbonyl)amino)propanoyl)-2,3-diazabicyclo[3.1.1]heptane-4-carboxylic acid (480 mg, 0.49 mmol, 1 equiv) and DCM (50 mL) at 0 °C. To the solution was added DMAP (298.83 mg, 2.45 mmol, 5 equiv) and PyBOP (2545.77 mg, 4.89 mmol, 10 equiv) at 0 °C. The resulting mixture was stirred for 0.5 h at 35 ° C under argon atmosphere. The resulting mixture was concentrated under reduced pressure then diluted with H₂O(50 mL). The mixture was extracted with EtOAc (3 x 60 mL), dried over anhydrous Na₂SO₄ and the resulting mixture was concentrated under reduced pressure. The crude product was purified by reverse phase flash with the following conditions (0.5% NH₄HCO₃) to afford benzyl4-(5-((22*S,*64*S,*4*S*)-4-((*tert-*butoxycarbonyl)amino)-11-ethyl-10,10-dimethyl-5,7-dioxo-11H-8-oxa-62,63-diaza-2(4,2)-morpholina-1(5,3)-indola-6(2,4)-bicyclo[3.1.1]heptanacycloundecaphane-12-yl)-6-((*S*)-1-methoxyethyl)pyridin-3-yl)piperazine-1-carboxylate (80 mg, 16 % yield) as an off-white solid. LCMS (ESI): m/z [M+H] calc'd for C₅₃H₇₀N₈O₉ ESI-MS 962.5; found: 963.4.

### Step 6

To a 40 mL vial was added benzyl-4-(5-((22*S*,64*S*,4*S*)-4-((*tert*-butoxycarbonyl)amino)-11-ethyl-10,10-dimethyl -5,7-dioxo-11*H*-8-oxa-62,63-diaza-2(4,2)-morpholina-1(5,3)-indola-6(2,4)-bicyclo[3.1.1]heptanacycloundecaphane-12-yl)-6-((*S*)-1-methoxyethyl)pyridin-3-yl)piperazine-1-carboxylate (75 mg, 0.08 mmol, 1 equiv) and DCM (1 mL) at 0 °C. Next, TFA (0.5 mL) was added at 0 °C. The resulting mixture was stirred for 1 h at room temperature under argon atmosphere then concentrated under reduced pressure. The resulting mixture was diluted with water (20 mL) and the mixture adjusted pH to 8 with saturated NaHCO₃ (aq.). The aqueous layer was extracted with EtOAc (3 x 50 mL), dried over anhydrous Na₂SO₄ and the resulting mixture was concentrated under reduced pressure. The crude product (80 mg, crude) was used in the next step directly without further purification. LCMS (ESI): m/z [M+H] calc'd for C₄₈H₆₂N₈O₇ ESI-MS 862.5; found: 863.4.

### Step 7

To a 40 mL vial was added benzyl-4-(5-((22*S*,64*S*,4*S*)-4-amino-11-ethyl-10,10-dimethyl-5,7-dioxo-11*H*-8-oxa-62,63-diaza-2(4,2)-morpholina-1(5,3)-indola-6(2,4)-bicyclo[3.1.1]heptanacycloundecaphane-12-yl)-6-((*S*)-1-methoxyethyl)pyridin-3-yl)piperazine-1-carboxylate (80 mg, 0.09 mmol, 1 equiv) and DMF (2 mL) at 0 °C. Next was added DIEA (179.70 mg, 1.40 mmol, 15 equiv), (1*S*,2*S*)-2-methylcyclopropane-1-carboxylic acid (13.92 mg, 0.14 mmol, 1.5 equiv), and HATU (52.87 mg, 0.14 mmol, 1.5 equiv) at 0 °C. The resulting mixture was stirred for 2 h at room temperature under argon atmosphere then diluted with H₂O (70 mL). This was extracted with EtOAc (3 x 50 mL) and dried over anhydrous Na₂SO₄. After filtration, the filtrate was concentrated under reduced pressure and purified by Prep-TLC to afford the penultimate compound in the scheme above (40 mg, 45% yield) as an off-white solid. LCMS (ESI): m/z [M+H] calc'd for C₅₃H₆₈N₈O₈ ESI-MS 944.5; found: 945.6.

### Step 8

To a solution of the product of Step 7 (37 mg, 0.04 mmol, 1 equiv) and paraformaldehyde (11.7 mg, 0.40 mmol, 10 equiv) in MeOH (1 mL) was added Pd/C (10 mg) in a 40 mL vial. The mixture was hydrogenated at room temperature under hydrogen pressure for 16 h. The reaction mixture was filtered through a Celite pad and concentrated under reduced pressure. The crude product was purified by Prep-HPLC to afford (1*S*,2*S*)-*N*-((22*S*,64*S*,4*S*)-11-ethyl-12-(2-((*S*)-1-methoxyethyl)-5-(4-methylpiperazin-1-yl)pyridin-3-yl) -10,10-dimethyl-5,7-dioxo-11*H*-8-oxa-62,63-diaza-2(4,2)-morpholina-1(5,3)-indola-6(2,4)-bicyclo[3.1.1]heptanacycloundecaphane-4-yl)-2-methylcyclopropane-1-carboxamide (1.7 mg, 4 % yield, 92.2% purity @254nm, 91.4% purity @220nm)) as a white solid. LCMS (ESI): m/z [M+H] calc'd for C₄₆H₆₄N₈O₆ ESI-MS 824.5; found: 825.4. ¹H NMR (400 MHz, DMSO-d6) δ 8.42 - 8.28 (m, 1H), 8.09 (d, J = 7.9 Hz, 1H), 7.41 - 7.22 (m, 1H), 7.19 - 7.09 (m, 2H), 6.96 (d, J = 8.8 Hz, 1H), 6.12 (s, 1H), 5.29 (s, 1H), 4.53 (d, J = 5.1 Hz, 1H), 4.26 (d, J = 11.1 Hz, 1H), 4.06 - 3.95 (m, 2H), 3.96 - 3.77 (m, 2H), 3.62 - 3.47 (m, 2H), 3.42 (d, J = 9.8 Hz, 2H), 3.18 (t, J = 5.4 Hz, 5H), 2.93 (s, 3H), 2.72 - 2.50 (m, 3H), 2.38 (t, J = 5.0 Hz, 5H), 2.26 (q, J = 5.5 Hz, 1H), 2.14 (s, 3H), 2.12 - 1.99 (m, 1H), 1.88 (t, J = 11.3 Hz, 1H), 1.72 - 1.60 (m, 1H), 1.45 - 1.26 (m, 5H), 1.17 (d, J = 1.7 Hz, 1H), 0.93 (s, 7H), 0.81 - 0.72 (m, 2H), 0.63 (s, 3H), 0.48 (s, 3H), 0.37 (dt, J = 8.0, 4.1 Hz, 1H).

### Example A59. Synthesis of N-((1S)-1-cyclopentyl-2-(((6³S,4S)-1²-(5-(4-cyclopropylpiperazin-1-yl)-2-((S)-1-methoxyethyl)pyridin-3-yl)-1¹-ethyl-10,10-dimethyl-5,7-dioxo-1¹H-8-oxa-2(2,5)-oxadiazola-1(5,3)-indola-6((2,3)-diazabicyclo[3.1.1]heptyl)cycloundecaphane-4-yl)amino)-2-oxoethyl)-N-methyl-5-(N-methylacetamido)thiazole-2-carboxamide

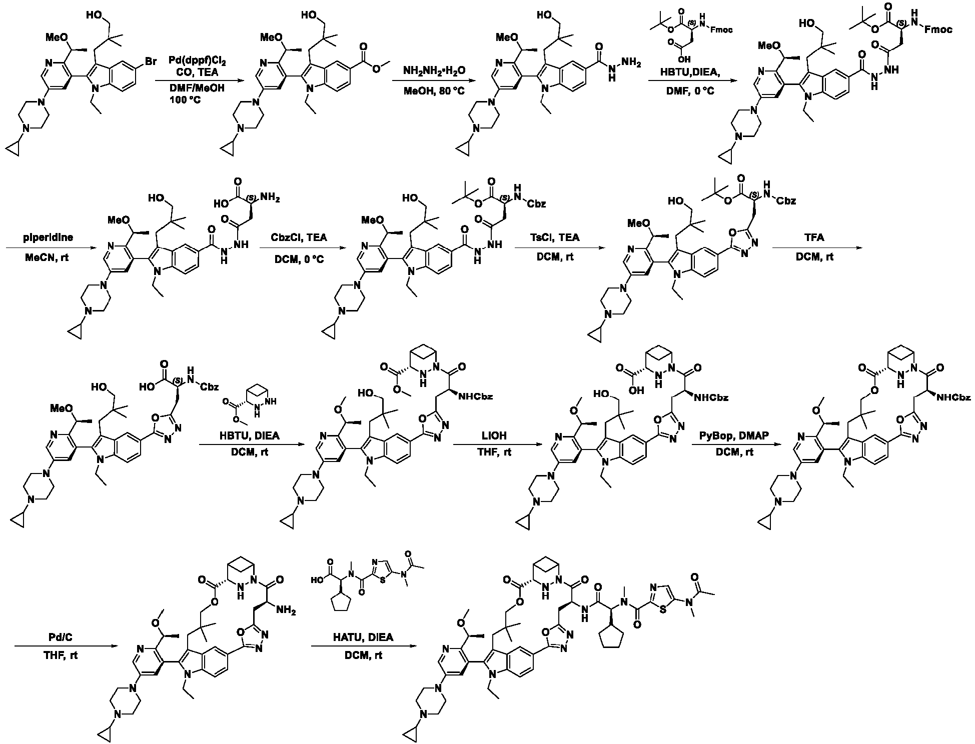

**Step 1.** A solution of 3-[(2*M*)-5-bromo-2-[5-(4-cyclopropylpiperazin-1-yl)-2-[(1*S*)-1-methoxyethyl]pyridin-3-yl]-1-ethylindol-3-yl]-2,2-dimethylpropan-1-ol (15.0 g, 26.3 mmol), TEA (5.33 g, 52.7 mmol) and Pd(dppf)Cl₂ (1.93 g, 2.63 mmol) in MeOH (40 mL) and DMF (200 mL) was stirred for 24 h at 100 °C under an atmosphere of carbon monoxide. The reaction mixture was quenched by the addition of H₂O (1000 mL) and the resulting mixture was extracted with EtOAc (3 x 500 mL), treated with brine (3 x 500 mL), dried over anhydrous Na₂SO₄, filtered, and concentrated under reduced pressure. The residue was purified by silica gel column chromatography to afford methyl (2M)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carboxylate (12.5 g, 78% yield) as a yellow solid. LCMS (ESI) *m*/*z:* [M + H] calcd for C₃₂H₄₄N₄O₄: 549.3; found 549.3.

**Step 2.** A solution of methyl (2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carboxylate (13.1 g, 23.9 mmol) and hydrazine hydrate (75 mL) in MeOH (130 mL) was stirred for 16 h at 80°C under an atmosphere of argon gas. The reaction mixture was quenched by the addition of H₂O (300 mL), extracted with EtOAc (3 x 300 mL), treated with brine (3 x 300 mL), dried over anhydrous Na₂SO₄, filtered, and concentrated under reduced pressure to give (2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carbohydrazide (12.4 g, crude) as a yellow solid. This material was taken to the next reaction without further purification. LCMS (ESI) *m*/*z:* [M + H] calcd for C₃₁H₄₄N₆O₃: 549.3; found 549.4.

**Step 3.** To a stirred solution of (2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carbohydrazide (15.0 g, crude), DIPEA (35.3 g, 273 mmol) and (3*S*)-4-(tert-butoxy)-3-{[(9*H*-fluoren-9-ylmethoxy)carbonyl]amino}-4-oxobutanoic acid (16.9 g, 41.0 mmol) in DMF (150 mL) was added HBTU (12.4 g, 32.8 mmol) in portions at 0 °C under an atmosphere of argon gas. The resulting mixture was stirred for 1 h at 0 °C under an atmosphere of argon gas. The reaction mixture was quenched by the addition of H₂O (500 mL), extracted with EtOAc (3 x 500 mL), treated with brine (3 x 500 mL), dried over anhydrous Na₂SO₄, filtered, and concentrated under reduced pressure. The residue was purified by silica gel column chromatography to give *tert*-butyl (2*S*)-3-{*N*'-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carbonyl]hydrazinecarbonyl}-2-{[(9*H*-fluoren-9-ylmethoxy)carbonyl]amino}propanoate (17.0 g, 18.04 mmol, 62% yield over 2 steps) as a yellow solid. LCMS (ESI) *m*/*z:* [M + H] calcd for C₅₄H₆₇N₇O₈: 942.5; found 942.5.

**Step 4.** A solution of *tert*-butyl (2*S*)-3-{*N'*-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carbonyl]hydrazinecarbonyl}-2-{[(9*H*-fluoren-9-ylmethoxy)carbonyl]amino}propanoate (11.0 g, 11.7 mmol) and piperidine (22 mL) in MeCN (100 mL) was stirred for 1 h at room temperature under an atmosphere of argon. The reaction mixture was quenched by the addition of H₂O (200 mL), extracted with EtOAc (3 x 200 mL), treated with brine (3 x 50 mL), dried over anhydrous Na₂SO₄, filtered, and concentrated under reduced pressure to afford *tert*-butyl (2*S*)-2-amino-4-{[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indol-5-yl]formohydrazido}-4-oxobutanoate (10.3 g, crude) as a yellow solid. The crude material was taken forward to the next reaction without further purification.

**Step 5.** To a stirred solution of *tert*-butyl (2*S*)-2-amino-4-{[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indol-5-yl]formohydrazido}-4-oxobutanoate (10.8 g, crude) and TEA (3.80 g, 37.5 mmol) in DCM (120 mL) was added CbzCl (3.84 g, 22.5 mmol) dropwise at 0 °C under an atmosphere of argon. The reaction mixture was stirred for 2 h at 0 °C and was then quenched by the addition of H₂O (200 mL), extracted with DCM (3 x 200 mL), treated with brine (3 x 100 mL), dried over anhydrous Na₂SO₄, filtered, and concentrated under reduced pressure. The residue was purified by reversed-phase chromatography to give *tert*-butyl (2*S*)-2-{[(benzyloxy)carbonyl]amino}-3-{*N*'-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carbonyl]hydrazinecarbonyl}propanoate (11.0 g, 70% yield over 2 steps) as a yellow solid. LCMS (ESI) *m*/*z:* [M + H] calcd for C₄₇H₆₃N₇O₈: 854.5; found 854.5.

**Step 6.** To a stirred solution of *tert*-butyl (2*S*)-2-{[(benzyloxy)carbonyl]amino}-3-{*N*'-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indole-5-carbonyl]hydrazinecarbonyl}propanoate (11.0 g, 12.9 mmol) and TEA (3.91 g, 38.6 mmol) in DCM (110 mL) was added TsCl (3.68 g, 19.3 mmol) dropwise at 0 °C under an atmosphere of argon gas. The reaction mixture was stirred for 3 h at 0 °C and was then quenched by the addition of H₂O (200 mL), extracted with CH₂Cl₂ (3 x 200 mL), treated with brine (4 x 100mL), dried over anhydrous Na₂SO₄, filtered, and concentrated under reduced pressure. The residue was purified by silica gel column chromatography to afford *tert*-butyl (2*S*)-2-{[(benzyloxy)carbonyl]amino}-3-{5-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indol-5-yl]-1,3,4-oxadiazol-2-yl}propanoate (7.50 g, 63% yield) as a yellow solid. LCMS (ESI) *m*/*z:* [M + H] calcd for C₄₇H₆₁N₇O₇: 836.5; found 836.8.

**Step 7.** To a stirred solution of *tert*-butyl (2*S*)-2-{[(benzyloxy)carbonyl]amino}-3-{5-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indol-5-yl]-1,3,4-oxadiazol-2-yl}propanoate (7.00 g, 8.37 mmol) in DCM (70 mL) was added TFA (35 mL) dropwise at 0 °C under an atmosphere of argon. The reaction mixture was stirred for 1 h at room temperature and was then concentrated under reduced pressure to give (*S*)-2-(((benzyloxy)carbonyl)amino)-3-(5-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)-1,3,4-oxadiazol-2-yl)propanoic acid (7.20 g, crude) as a yellow solid. This material was taken to the next reaction without further purification. LCMS (ESI) *m*/*z:* [M + H] calcd for C₄₃H₅₃N₇O₇: 780.4; found 780.4.

**Step 8.** To a stirred solution of (*S*)-2-(((benzyloxy)carbonyl)amino)-3-(5-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)-1 ,3,4-oxadiazol-2-yl)propanoic acid (8.20 g, crude), methyl (*S*)-2,3-diazabicyclo[3.1.1]heptane-4-carboxylate TFA salt (2.46 g, 15.8 mmol), and DIEA (13.6 g, 105 mmol) in DCM (80 mL) was added HBTU (4.78 g, 12.6 mmol) in portions at 0 °C under an atmosphere of argon. The reaction mixture was stirred for 1 h at room temperature and was then quenched by the addition of H₂O (100 mL), extracted with DCM (3 x 100 mL), treated with brine (3 x 100 mL), dried over anhydrous Na₂SO₄, filtered, and concentrated under reduced pressure. The residue was purified by silica gel column chromatography to afford methyl (4*S*)-2-[(2*S*)-2-{[(benzyloxy)carbonyl]amino}-3-{5-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indol-5-yl]-1,3,4-oxadiazol-2-yl}propanoyl]-2,3-diazabicyclo[3.1.1]heptane-4-carboxylate (4.10 g, 47% yield over 2 steps) as a yellow solid. LCMS (ESI) *m*/*z:* [M + H] calcd for C₅₀H₆₃N₉O₈: 918.5; found 918.5.

**Step 9.** A solution of methyl (4*S*)-2-[(2*S*)-2-{[(benzyloxy)carbonyl]amino}-3-{5-[(2*M*)-2-[5-(4-cyclopropylpiperazin-1-yl)-2-[(1*S*)-1-methoxyethyl]pyridin-3-yl]-1-ethyl-3-(3-hydroxy-2,2-dimethylpropyl)indol-5-yl]-1,3,4-oxadiazol-2-yl}propanoyl]-2,3-diazabicyclo[3.1.1]heptane-4-carboxylate (5.40 g, 5.88 mmol) in THF (50 mL) was added LiOH (704 mg, 29.4 mmol) dropwise at 0 °C under an atmosphere or argon. The reaction mixture was stirred for 1 h at room temperature and was Then acidified to pH 6 by the addition of conc. aq. HCl solution. The resulting mixture was extracted with EtOAc (3 x 100 mL), treated with brine (4 x 50 mL), dried over anhydrous Na₂SO₄, filtered, and concentrated under reduced pressure to give (*S*)-2-((*S*)-2-(((benzyloxy)carbonyl)amino)-3-(5-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)-1,3,4-oxadiazol-2-yl)propanoyl)-2,3-diazabicyclo[3.1.1]heptane-4-carboxylic acid (4.50 g, crude) as a yellow solid. This material was taken to the next reaction without further purification.

**Step 10.** To a stirred solution of (*S*)-2-((*S*)-2-(((benzyloxy)carbonyl)amino)-3-(5-(2-(5-(4-cyclopropylpiperazin-1-yl)-2-((*S*)-1-methoxyethyl)pyridin-3-yl)-1-ethyl-3-(3-hydroxy-2,2-dimethylpropyl)-1*H*-indol-5-yl)-1,3,4-oxadiazol-2-yl)propanoyl)-2,3-diazabicyclo[3.1.1]heptane-4-carboxylic acid (3.6 g, crude) and DMAP (1.95 g, 15.9 mmol) in DCM (600 mL) was added PyBOP (20.7 g, 39.8 mmol) in portions at 0 °C under an atmosphere of argon gas. The reaction mixture was stirred for 16 h at room temperature and was then quenched by the addition of H₂O (100 mL) and concentrated under reduced pressure. The resulting mixture was extracted with EtOAc (3 x 200 mL), treated with brine (4 x 100 mL), dried over anhydrous Na₂SO₄, filtered, and concentrated under reduced pressure. The residue was purified by reversed-phase chromatography to afford benzyl ((6³*S*,4*S*)-1²-(5-(4-cyclopropylpiperazin-1-yl)-2-((*S*)-1-methoxyethyl)pyridin-3-yl)-1¹-ethyl-10,10-dimethyl-5,7-dioxo-1¹*H*-8-oxa-2(2,5)-oxadiazola-1(5,3)-indola-6((2,3)-diazabicyclo[3.1.1]heptyl)cycloundecaphane-4-yl)carbamate (1.20 g, 29% yield over 2 steps) as a yellow solid. LCMS (ESI) *m*/*z:* [M + H] calcd for C₄₉H₅₉N₉O₇: 886.5; found 886.6.

**Step 11.** A solution of benzyl ((6³*S*,4*S*)-1²-(5-(4-cyclopropylpiperazin-1-yl)-2-((*S*)-1-methoxyethyl)pyridin-3-yl)-1¹-ethyl-10,10-dimethyl-5,7-dioxo-1¹*H*-8-oxa-2(2,5)-oxadiazola-1(5,3)-indola-6((2,3)-diazabicyclo[3.1.1]heptyl)cycloundecaphane-4-yl)carbamate (210 mg, 0.237 mmol) and Pd/C (105 mg, 10%) in THF (3 mL) was stirred for 16 h at 40°C under an atmosphere of argon gas. The resulting mixture was filtered, the filter cake was washed with CH₂Cl₂ (4 x 15 mL), and the filtrate was concentrated under reduced pressure to give (6⁴*S*,4*S*)-4-amino-1²-(5-(4-cyclopropylpiperazin-1-yl)-2-((*S*)-1-methoxyethyl)pyridin-3-yl)-11-ethyl-10,10-dimethyl-1¹*H*-8-oxa-6²,6³-diaza-2(2,5)-oxadiazola-1(5,3)-indola-6(2,4)-bicyclo[3.1.1]heptanacycloundecaphane-5,7-dione (190 mg, crude), which was taken directly to the next reaction without further purification.

**Step 12.** To a stirred solution of (6⁴*S*,4*S*)-4-amino-1²-(5-(4-cyclopropylpiperazin-1-yl)-2-((*S*)-1-methoxyethyl)pyridin-3-yl)-11-ethyl-10,10-dimethyl-1¹*H*-8-oxa-6²,6³-diaza-2(2,5)-oxadiazola-1(5,3)-indola-6(2,4)-bicyclo[3.1.1]heptanacycloundecaphane-5,7-dione (150 mg, crude), DIPEA (258 mg, 1.99 mmol) and (*S*)-cyclopentyl({*N*-methyl-1-[5-(*N*-methylacetamido)-1,3-thiazol-2-yl]formamido})acetic acid (102 mg, 0.298 mmol) in DCM (2 mL) was added HATU (91.0 mg, 0.239 mmol) in portions at 0 °C under an atmosphere of argon gas. The resulting mixture was stirred for 1 h at room temperature and was then quenched by the addition of H₂O (5 mL), extracted with EtOAc (3 x 5 mL), treated with brine (4 x 5 mL), dried over anhydrous Na₂SO₄, filtered, and concentrated under reduced pressure. The residue (200mg) was purified by reversed-phase prep-HPLC to afford *N*-((1*S*)-1-cyclopentyl-2-(((6³*S*,4*S*)-1²-(5-(4-cyclopropylpiperazin-1-yl)-2-((*S*)-1-methoxyethyl)pyridin-3-yl)-1¹-ethyl-10,10-dimethyl-5,7-dioxo-1¹*H*-8-oxa-2(2,5)-oxadiazola-1(5,3)-indola-6((2,3)-diazabicyclo[3.1.1]heptyl)cycloundecaphane-4-yl)amino)-2-oxoethyl)-*N*-methyl-5-(*N-*methylacetamido)thiazole-2-carboxamide (64.4 mg, 32% yield over 2 steps) as a white solid. LCMS (ESI) *m*/*z:* [M + H] calcd for C₅₆H₇₂N₁₂O₈S: 1073.5; found 1073.7; ¹*H* NMR (400 MHz, DMSO-*d₆*) δ 8.78 - 8.28 (m, 2H), 8.16 (d, *J* = 14.0 Hz, 1H), 7.88 (t, *J* = 7.2 Hz, 1H), 7.85 - 7.68 (m, 2H), 7.30 (s, 1H), 6.65 - 6.39 (m, 1H), 5.81 - 4.80 (m, 2H), 4.70 - 4.45 (m, 2H), 4.27 (q, *J* = 7.1 Hz, 1H), 4.08 (s, 2H), 3.75 - 3.63 (m, 1H), 3.55 (d, *J* = 9.1 Hz, 4H), 3.43 (s, 1H), 3.23 (s, 6H), 3.08 - 3.03 (m, 3H), 2.90 (s, 2H), 2.67 (d, *J* = 5.4 Hz, 6H), 2.41 - 2.31 (m, 4H), 2.17 (t, *J* = 9.7 Hz, 1H), 1.85 - 1.52 (m, 3H), 1.43 (m, 5H), 1.35 (d, *J* = 6.1 Hz, 3H), 1.06 - 0.97 (m, 4H), 0.54 (s, 3H), 0.44 (d, *J* = 6.2 Hz, 2H), 0.34 (s, 2H), .

### Example A60. Synthesis of N-((1S)-prop-2-yl-2-(((6³S,4S)-1²-(5-(4-cyclopropylpiperazin-1-yl)-2-((S)-1-methoxyethyl)pyridin-3-yl)-1¹-ethyl-10,10-dimethyl-5,7-dioxo-1¹H-8-oxa-2(2,5)-oxadiazola-1(5,3)-indola-6((2,3)-diazabicyclo[3.1.1]heptyl)cycloundecaphane-4-yl)amino)-2-oxoethyl)-N-methyl-5-(N-methylacetamido)thiazole-2-carboxamide

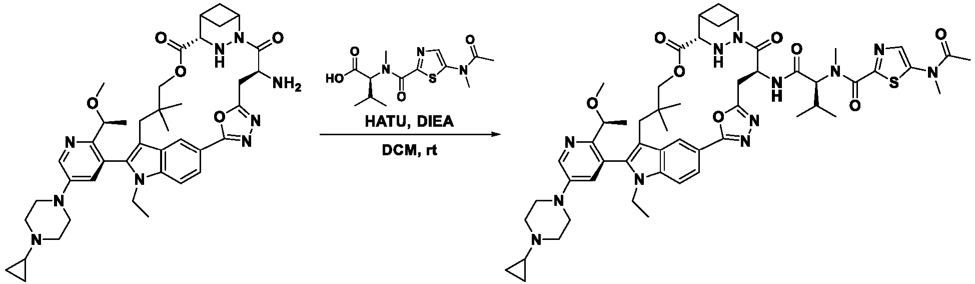

**Step 1.** To a stirred solution of (6⁴*S*,4*S*)-4-amino-1²-(5-(4-cyclopropylpiperazin-1-yl)-2-((*S*)-1-methoxyethyl)pyridin-3-yl)-11-ethyl-10,10-dimethyl-1¹*H*-8-oxa-6²,6³-diaza-2(2,5)-oxadiazola-1(5,3)-indola-6(2,4)-bicyclo[3.1.1]heptanacycloundecaphane-5,7-dione (200 mg, 0.266 mmol), (2S)-3-methyl-2-{*N*-methyl-1-[5-(*N*-methylacetamido)-1,3-thiazol-2-yl]formamido}butanoic acid (100 mg, 0.319 mmol) and DIPEA (344 mg, 2.66 mmol) in DCM (2 mL) were added HATU (152 mg, 0.399 mmol) in portions at 0 °C under an atmosphere of argon gas. The reaction mixture was stirred for 1 h at room temperature and was then quenched by the addition of H₂O (2 mL), extracted with CH₂Cl₂ (3 x 2 mL), treated with brine, dried over anhydrous Na₂SO₄, filtered, and concentrated under reduced pressure. The residue was purified by reversed-phase prep-HPLC to afford *N*-((1*S*)-prop-2-yl-2-(((6³*S*,4*S*)-1²-(5-(4-cyclopropylpiperazin-1-yl)-2-((*S*)-1-methoxyethyl)pyridin-3-yl)-1¹-ethyl-10,10-dimethyl-5,7-dioxo-1¹*H*-8-oxa-2(2,5)-oxadiazola-1(5,3)-indola-6((2,3)-diazabicyclo[3.1.1]heptyl)cycloundecaphane-4-yl)amino)-2-oxoethyl)-*N*-methyl-5-(*N-*methylacetamido)thiazole-2-carboxamide (59.1 mg, 20% yield) as a white solid. LCMS (ESI) *m*/*z:* [M + H] calcd for C₅₄H₇₀N₁₂O₈S: 1047.5; found 1047.7; ¹*H* NMR (400 MHz, DMSO-*d₆*) *δ* 8.88 - 8.37 (m, 2H), 8.16 (d, *J* = 15.5 Hz, 1H), 7.92 - 7.72 (m, 3H), 7.31 (d, *J =* 3.3 Hz, 1H), 6.74 - 6.27 (m, 1H), 5.63 (s, 1H), 5.43 (d, *J* = 10.2 Hz, 1H), 4.62 - 4.47 (m, 2H), 4.27 (d, *J* = 8.2 Hz, 1H), 4.07 (d, *J* = 7.2 Hz, 2H), 3.80 - 3.50 (m, 5H), 3.32 - 3.16 (m, 6H), 3.08 - 3.03 (m, 3H), 2.89 (s, 2H), 2.67 (d, *J* = 5.6 Hz, 6H), 2.38 (d, *J* = 5.0 Hz, 4H), 2.30 - 2.09 (m, 2H), 1.67 (q, *J* = 8.2, 6.1 Hz, 2H), 1.38 - 1.32 (m, 3H), 1.06 - 0.95 (m, 4H), 0.86 - 0.70 (m, 8H), 0.62 - 0.50 (m, 3H), 0.47 - 0.41 (m, 2H), 0.34 (q, *J* = 3.3 Hz, 2H).

**Table 3: Exemplary Compounds Prepared by Methods of the Present Invention**

| **Ex#** | **MS Found** |
|---|---|
| A1 | 825.4 |
| A2 | 825.3 |
| A3 | 825.6 |
| A4 | 823.4 |
| A5 | 825.4 |
| A6 | 825.6 |
| A7 | 825.35 |
| A8 | 797.6 |
| A9 | 841.6 |
| A10 | 785.4 |
| A11 | 839.6 |
| A12 | 839.8 |
| A13 | 839.9 |
| A14 | 837.5 |
| A15 | 799.5 |
| A16 | 813.6 |
| A17 | 816.55 |
| A18 | 806.25 |
| A19 | 770.5 |
| A20 | 787.4 |
| A21 | 837.45 |
| A22 | 813.3 |
| A23 | 841.35 |
| A24 | 867.4 |
| A25 | 825.35 |
| A26 | 847.3 |
| A27 | 839.4 |
| A28 | 825.55 |
| A29 | 839.401 |
| A30 | 823.6 |
| A31 | 823.55 |
| A32 | 851.35 |
| A33 | 855.45 |
| A34 | 855.45 |
| A35 | 823.4 |
| A36 | 823.4 |
| A37 | 823.4 |
| A38 | 827.35 |
| A39 | 879.3 |
| A40 | 986.45 |
| A41 | 823.3 |
| A42 | 811.3 |
| A43 | 835.3 |
| A44 | 835.3 |
| A45 | 839.25 |
| A46 | 925.65 |
| A47 | 887.35 |
| A48 | 1024.4 |
| A49 | 995.55 |
| A50 | 877.25 |
| A51 | 957.45 |
| A52 | 1040.8 |
| A53 | 1011.6 |
| A54 | 1044.75 |
| A55 | 1006.5 |
| A56 | 941.55 |
| A57 | 1060.5 |
| A58 | 893.5 |
| A59 | 1073.65 |
| A60 | 1047.65 |

| | |
|---|---|
| Note: values may differ slightly from values found elsewhere in this application due to different measurements and rounding. | |

### Biological Assays

### Disruption of B-Raf Ras-binding Domain (BRAF^{RBD}) Interaction with K-Ras by Compounds of the Invention (also called a FRET assay or an MOA assay)

The purpose of this biochemical assay is to measure the ability of test compounds to facilitate ternary complex formation between a nucleotide-loaded K-Ras isoform and cyclophilin A; the resulting ternary complex disrupts binding to a BRAF^{RBD} construct, inhibiting K-Ras signaling through a RAF effector. Data is reported as IC50 values. Other Ras variants may be used.

In assay buffer containing 25 mM HEPES pH 7.3, 0.002% Tween20, 0.1% BSA, 100 mM NaCl and 5 mM MgCl₂, tag less cyclophilin A, His6-K-Ras-GMPPNP, and GST-BRAF^{RBD} are combined in a 384-well assay plate at final concentrations of 25 µM, 12.5 nM and 50 nM, respectively. Compound is present in plate wells as a 10-point 3-fold dilution series starting at a final concentration of 30 µM. After incubation at 25°C for 3 hours, a mixture of Anti-His Eu-W1024 and anti-GST allophycocyanin is then added to assay sample wells at final concentrations of 10 nM and 50 nM, respectively, and the reaction incubated for an additional 1.5 hours. TR-FRET signal is read on a microplate reader (Ex 320 nm, Em 665/615 nm). Compounds that facilitate disruption of a K-Ras:RAF complex are identified as those eliciting a decrease in the TR-FRET ratio relative to DMSO control wells.

The compounds in Table 1 except A11, A18, A19, A23, A24, A27, A29, A38, A42, A43, A56 and A58, exhibited an IC50 of less than 3 µM in at least one of the following: K-Ras Q61H, G12C, G12D, G12R, G12S, G12V, G12A, G13C, G13D and wild-type; N-Ras Q61K, Q61R, G12C and wild-type; and H-Ras G13R, Q61L and wild-type.

While the invention has been described in connection with specific embodiments thereof, it will be understood that it is capable of further modifications and this application is intended to cover any variations, uses, or adaptations of the invention following, in general, the principles of the invention and including such departures from the present disclosure come within known or customary practice within the art to which the invention pertains and may be applied to the essential features set forth herein.

All publications, patents and patent applications are herein incorporated by reference in their entirety to the same extent as if each individual publication, patent or patent application was specifically and individually indicated to be incorporated by reference in its entirety.

**The following clauses, describing aspects of the invention, are part of the description:**
1. A compound, or a pharmaceutically acceptable salt thereof, having the structure of Formula I: 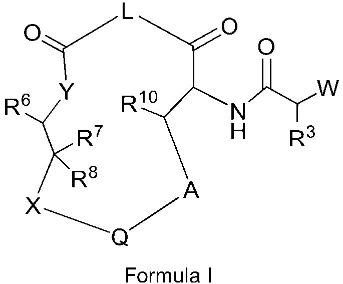 wherein:
   Q is an optionally substituted 7- to 12-membered bicyclic arylene, an optionally substituted 7- to 12-membered bicyclic heteroarylene, or an optionally substituted 7- to 12-membered bicyclic heterocyclylene, wherein a first ring in Q is bonded to X, and a second ring in Q is bonded to A;
   X is a bond; a straight chain C₁-C₃ alkylene optionally substituted with 1 to 3 substituents independently selected from fluoro, -CN, -C₁-C₃ alkyl, and -O-C₁-C₃ alkyl; -O-; -S(O)₀₋₂-; *-CH₂-O-; *-CH₂-S(O)₀₋₂-; *-O-CH₂-; or *-CH₂-S(O)₀₋₂-, wherein "*" represents a portion of X bound to -C(R⁷)(R⁸)-;
   Y is -O-, -NH- or -N(C₁-C₃ alkyl)-;
   R³ is optionally substituted C₁-C₆ alkyl, optionally substituted C₃-C₆ cycloalkyl, optionally substituted C₆-C₁₀ aryl, or optionally substituted 3- to 11-membered heterocyclyl;
      W is: 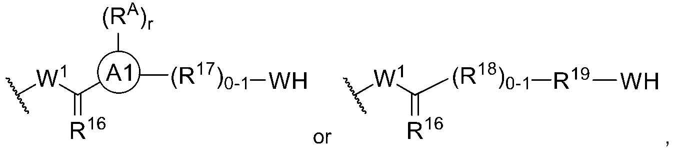
   wherein:
      ring A1 is a 4- to 8-membered cycloalkyl or a 4- to 8-membered heterocyclyl;
      W¹ is -N(R²⁰)-, -O-, or -C(R^{20a})(R^{20b})-; each R^{A} is each independently halo, cyano, hydroxyl, optionally substituted amino, optionally substituted C₁-C₆ heteroalkyl, or optionally substituted C₁-C₃ alkyl;
      r is 0, 1, 2, 3, or 4;
         R¹⁷, if present, is optionally substituted C₁-C₆ heteroalkylene or optionally substituted C₁-C₆ alkylene;
      R¹⁸, if present, is optionally substituted C₁-C₄ alkylene;
      R¹⁹ is optionally substituted C₁-C₆ heteroalkylene, optionally substituted C₁-C₆ alkylene, -NH-, or -N(optionally substituted C₁-C₆ alkyl) or a saturated, nitrogen-containing 3- to 8-membered heterocyclyl;
      R²⁰ is hydrogen or -C₁-C₃alkyl;
      R²⁰ is taken together with one R^{A}, the atoms to which they are respectively attached and any intervening atoms, to form an optionally substituted, 5- to 8-membered heterocyclyl that is fused or spiro-fused to ring A, or
      R²⁰ is taken together with any methylene unit in R¹⁸, or any methylene unit in R¹⁹, the atoms to which they are respectively attached and any intervening atoms, to form an optionally substituted, 5- to 8-membered heterocyclyl;
      each of R^{20a} and R^{20b} is, independently, hydrogen, or -C₁-C₃ alkyl, or R^{20a} and R^{20b} are taken together with the carbon atom to which they are bound to form a 3- to 6-membered cycloalkyl ring;
      R¹⁶ is O, S, N-CN, or N-O-C₁-C₃alkyl;
      WH is 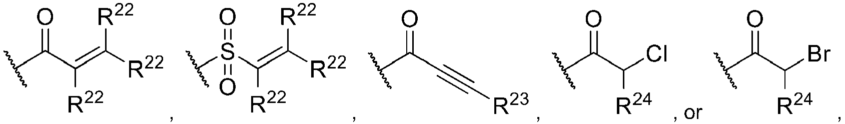
      each R²² is, independently, hydrogen, cyano, optionally substituted C₁-C₃ alkyl, or an optionally substituted 4- to 7-membered saturated heterocyclyl; or R²² is taken together with either of R¹⁷ or R¹⁹, the atoms to which they are attached and any intervening atoms to form an optionally substituted 5- to 8-membered ring system;
      R²³ is hydrogen, optionally substituted C₁-C₃ alkyl, or an optionally substituted 4- to 7-membered saturated heterocyclyl;
      R²⁴ is hydrogen, optionally substituted C₁-C₃ alkyl, or an optionally substituted 4- to 7-membered saturated heterocyclyl; or R²⁴ is taken together with either of R¹⁷ or R¹⁹, the atoms to which they are attached and any intervening atoms, to form an optionally substituted 5- to 8-membered ring system;
      A is optionally substituted C₂-C₄ alkylene, optionally substituted C₁-C₄ heteroalkylene, or optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
      L has the structure of Formula VIIa or VIIb: 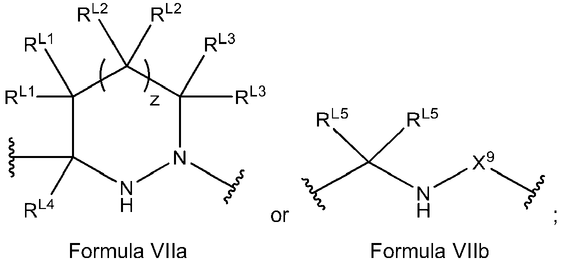
      z is 0, 1, or 2;
      X⁹ is -NR^{L6}-, -C(O)-, or -S(O)₂-;
      each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
      wherein L does not have the structure of 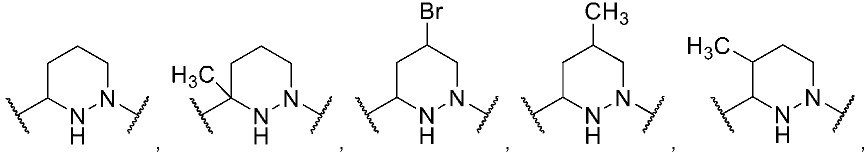 or 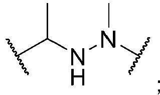 or
      optionally, wherein L is not 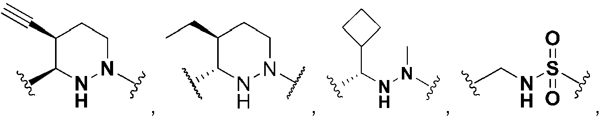 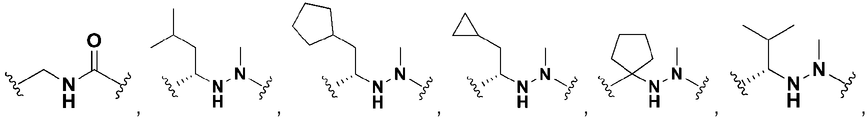 or 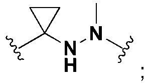
      R³ is optionally substituted C₁-C₆ alkyl, optionally substituted C₃-C₆ cycloalkyl, optionally substituted C₆ aryl, or optionally substituted 3- to 7-membered heterocyclyl;
      R¹⁰ is hydrogen, halogen, optionally substituted C₁-C₃ alkyl, or C₁-C₃ optionally substituted heteroalkyl;
      R⁷ is hydrogen, halogen, or optionally substituted C₁-C₃ alkyl;
      R⁸ is hydrogen, halogen, -OH, -CN, optionally substituted C₁-C₃ heteroalkyl, optionally substituted C₁-C₃ alkyl, optionally substituted C₂-C₆ alkenyl, optionally substituted C₂-C₆ alkynyl, optionally substituted C₆-C₁₀aryl, optionally substituted 4- to 8-membered heteroaryl, optionally substituted C₃-C₆ cycloalkyl, or optionally substituted 3- to 7-membered heterocyclyl; or
      R⁷ and R⁸ are taken together to form =CH₂, an optionally substituted C₃-C₆ cycloalkyl, or a 3- to 7-membered saturated heterocyclyl; or
      R⁸ is taken together with a ring atom in Q, the carbon atom to which R⁷ is bound and X to form a 4- to 9-membered saturated or unsaturated heterocyclyl that is fused to Q;
      R⁶ is hydrogen or -CH₃;
      each R⁵ is, independently, halogen, optionally substituted C₁-C₃ alkyl, or optionally substituted C₁-C₃ haloalkyl; and
      p is 0, 1, 2, or 3, wherein:
         (i) the compound is not: 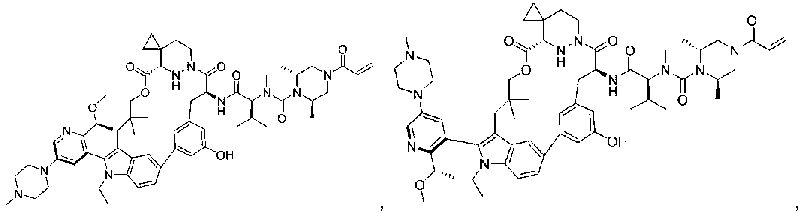 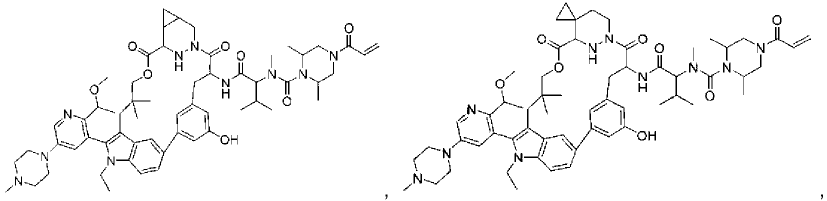 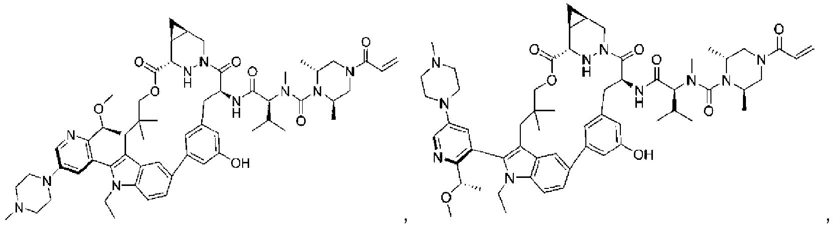 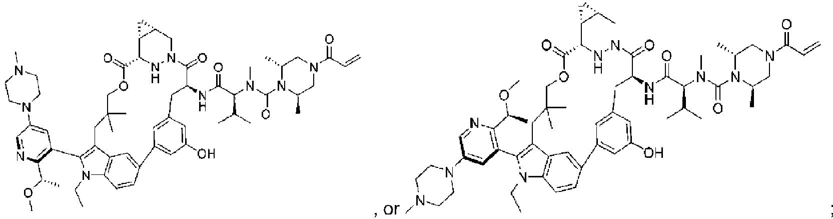 or
         (ii) when A is 1-hydroxy-benzene-3,5-diyl or optionally substituted phenylene, two R^{L1} substituents taken together form spiro cyclopropyl, and W is 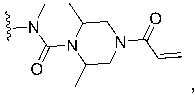 then, Q is not 1-ethyl-indole-2,5-diyl or indole-2,5-diyl substituted with C₁₋₄ alkyl; or
         (iii) when A is 1-hydroxy-benzene-3,5-diyl or optionally substituted phenylene, two R^{L1} substituents taken together form spiro cyclopropyl, and W is 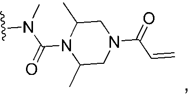 then, Q is not 1-ethyl-2,5-indol-diyl optionally substituted with 1, 2 or 3 substituents independently selected from halogen, C₁₋₄ alkyl and C₁₋₄ alkoxy, wherein the C₁₋₄ alkyl and C₁₋₄ alkoxy are each optionally substituted with 1, 2 or 3 substituents independently selected from halogen, OH, C₁₋₃ alkyl and C₁₋₃ alkoxy, wherein C₁₋₃ alkyl and C₁₋₃ alkoxy are each substituted with 1, 2 or 3 substituents independently selected from deuterium, halogen, OH, methyl, methoxy and OCF₃; or
         (iv) when A is 1-hydroxy-benzene-3,5-diyl or optionally substituted phenylene, R^{L1} and R^{L2} taken together form fused cyclopropyl, and W is 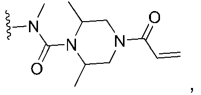 then Q is not 1-ethyl-2,5-indol-diyl or 2,5-indol-diyl substituted with C₁₋₄ alkyl; or
         (v) when A is 1-hydroxy-benzene-3,5-diyl or optionally substituted phenylene, R^{L1} and R^{L2} taken together form fused cyclopropyl, and W is 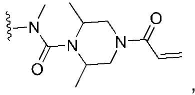 then Q is 1-ethyl-2,5-indol-diyl or 2,5-indol-diyl substituted with C₁₋₄ alkyl; or
         (vi) when A is 1-hydroxy-benzene-3,5-diyl or optionally substituted phenylene, R^{L1} and R^{L2} taken together form fused cyclopropyl, and W is 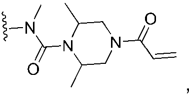 then Q is not 1-ethyl-2,5-indol-diyl optionally substituted with 1, 2 or 3 substituents independently selected from halogen, C₁₋₄ alkyl and C₁₋₄ alkoxy, wherein the C₁₋₄ alkyl and C₁₋₄ alkoxy are each optionally substituted with 1, 2 or 3 substituents independently selected from halogen, OH, C₁₋₃ alkyl and C₁₋₃ alkoxy, wherein C₁₋₃ alkyl and C₁₋₃ alkoxy are each substituted with 1, 2 or 3 substituents independently selected from deuterium, halogen, OH, methyl, methoxy and OCF₃; or
         (vii) when A is -hydroxy-benzene-3,5-diyl or optionally substituted phenylene and two R^{L1} substituents taken together form spiro cyclopropyl, then W is not 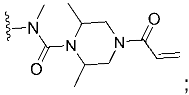 or
         (viii) when A is 1-hydroxy-benzene-3,5-diyl or optionally substituted phenylene, R^{L1} and R^{L2} taken together form fused cyclopropyl, then W is not 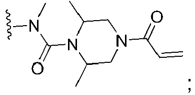
         (ix) when A is 1-hydroxy-benzene-3,5-diyl or optionally substituted phenylene, WH is 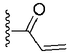 and two R^{L1} substituents taken together form spiro cyclopropyl or spiro cycloalkyl, then A1 is not 1,4-piperazin-diyl optionally substituted with methyl or C₁₋₄ alkyl; or
         (x) when A is 1-hydroxy-benzene-3,5-diyl or optionally substituted phenylene, WH is 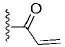 and R^{L1} and R^{L2} taken together form fused cyclopropyl or fused cycloalkyl, then A1 is not is not 1,4-piperazin-diyl optionally substituted with methyl or C₁₋₄ alkyl.
2. A compound, or a pharmaceutically acceptable salt thereof, having the structure of Formula IIa: 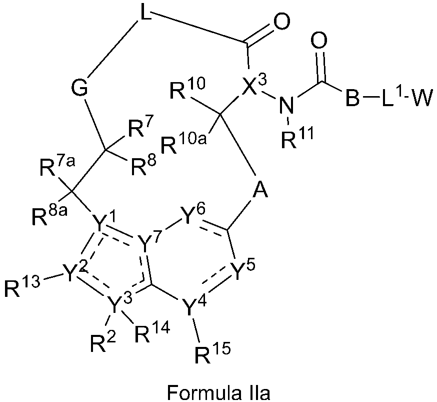
   wherein the dotted lines represent zero, one, two, three, or four non-adjacent double bonds;
   A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
   G is optionally substituted C₁-C₄ alkylene, optionally substituted C₁-C₄ alkenylene, optionally substituted C₁-C₄ heteroalkylene, -C(O)O-**C**H(R⁶)- where **C** is bound to -C(R⁷R⁸)-, -C(O)NH-**C**H(R⁶)-where **C** is bound to -C(R⁷R⁸)-, optionally substituted C₁-C₄ heteroalkylene, or 3- to 8-membered heteroarylene;
   L¹ is a linker;
   W is hydrogen, cyano, S(O)₂R', optionally substituted amino, optionally substituted amido, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3- to 11-membered heterocycloalkyl, optionally substituted 3- to 8-membered cycloalkyl, or optionally substituted 3- to 8-membered heteroaryl;
   L has the structure of Formula VIIa or Vllb: 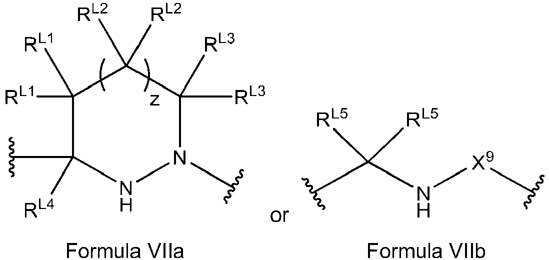
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 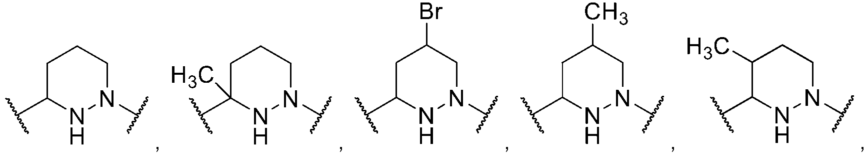 or 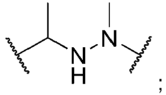 or
   optionally, wherein L is not 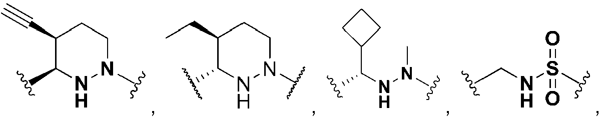 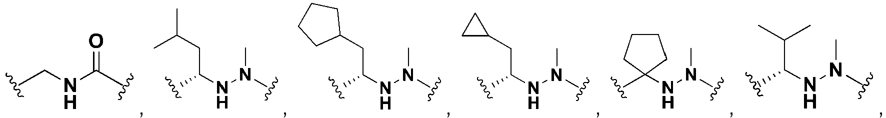 or 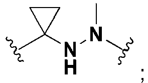
   X¹ is optionally substituted C₁-C₂ alkylene, NR, O, or S(O)_{q};
   X² is O or NH;
   X³ is N or CH;
   q is 0, 1, or 2;
   R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
   each R' is, independently, hydrogen or optionally substituted C₁-C₄ alkyl;
   Y¹ is C, CH, or N;
   Y², Y³, Y⁴, and Y⁷ are, independently, C or N;
   Y⁵ is CH, CH₂, or N;
   Y⁶ is C(O), CH, CH₂, or N;
   R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
   R¹³ and R² combine with the atoms to which they are attached to form an optionally substituted 3-to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl;
   R¹⁴ is absent or R² and R¹⁴ combine with the atom to which they are attached to form an optionally substituted 3- to 8-membered cycloalkyl or optionally substituted 3- to 14-membered heterocycloalkyl;
   R¹⁵ is absent, hydrogen, halogen, cyano, or methyl optionally substituted with 1 to 3 halogens;
   R⁵ is hydrogen, C₁-C₄ alkyl optionally substituted with halogen, cyano, hydroxy, or C₁-C₄ heteroalkyl, cyclopropyl, or cyclobutyl;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3- to 6-membered cycloalkyl or optionally substituted 3- to 7-membered heterocycloalkyl;
   R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
   R^{7a} and R^{8a} are, independently, hydrogen, halogen, optionally substituted C₁-C₃ alkyl, or combine with the carbon to which they are attached to form a carbonyl;
   R^{7'} is hydrogen, halogen, or optionally substituted C₁-C₃ alkyl;
   R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R^{7'} and R^{8'} combine with the carbon atom to which they are attached to form optionally substituted 3- to 6-membered cycloalkyl or optionally substituted 3- to 7-membered heterocycloalkyl;
   R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
   R⁹ and L combine with the atoms to which they are attached to form an optionally substituted 3-to 14-membered heterocycloalkyl;
   R^{9'} is hydrogen or optionally substituted C₁-C₆ alkyl; or
   R⁹ and R^{9'}, combined with the atoms to which they are attached, form a 3- to 6-membered cycloalkyl or a 3- to 6-membered heterocycloalkyl;
   R¹⁰ is hydrogen, halogen, hydroxy, optionally substituted C₁-C₃ heteroalkyl, or optionally substituted C₁-C₃ alkyl;
   R^{10a} is hydrogen or halogen;
   R¹¹ is hydrogen or optionally substituted C₁-C₃ alkyl; and
   R²¹ is hydrogen or optionally substituted C₁-C₃ alkyl wherein:
      (i) the compound is not 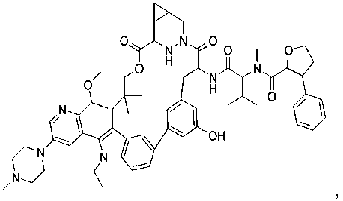 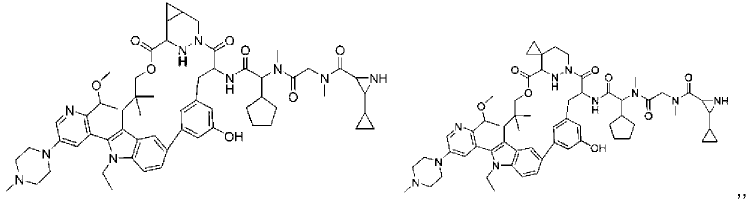 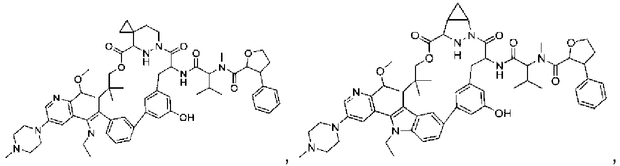 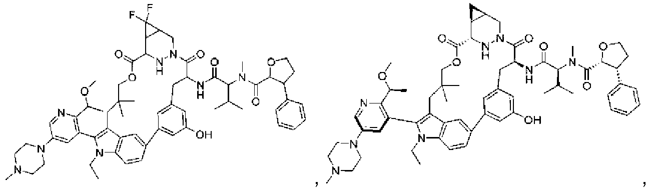 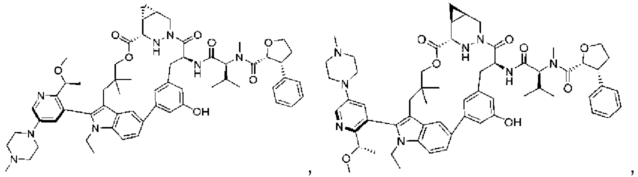 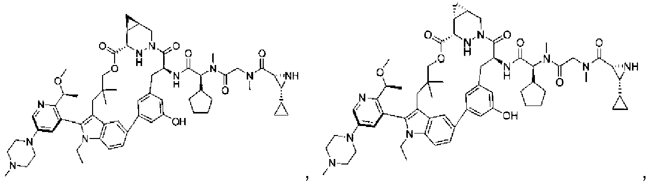 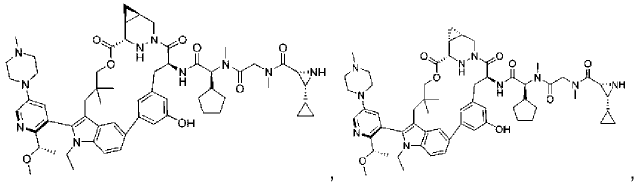 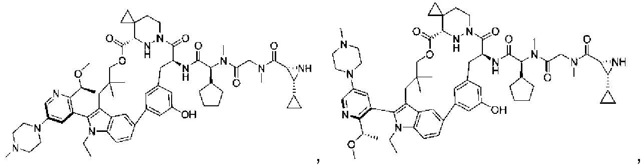 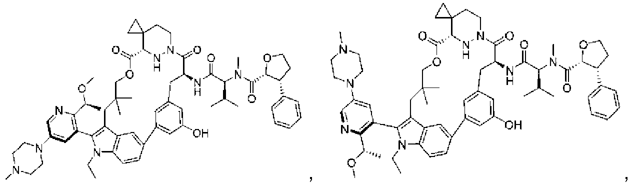 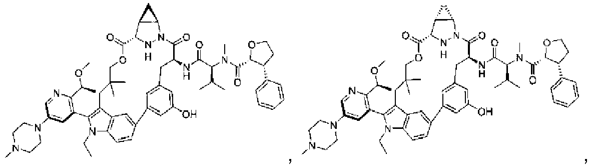 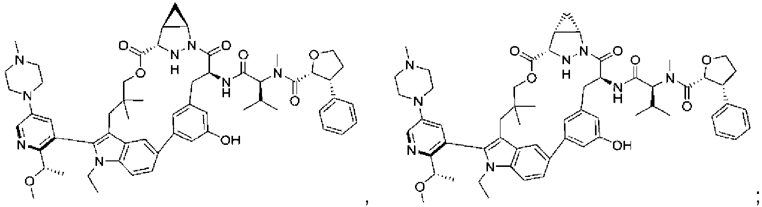 or
      (ii) when A is 1-hydroxy-benzene-3,5-diyl or optionally substituted phenylene, two R^{L1} substituents taken together form spiro cyclopropyl, L¹ is -N(CH₃)C(O)- or -N(CH₃)C(O)CH₂N(CH₃)- and B is -CH(R⁹)-, wherein R⁹ is isopropyl or cyclopentyl, then W is not 3-cyclopropylaziridin-2-yl, 3-phenyltetrahydrofuran-2-yl, optionally substituted aziridin-2-yl or optionally substituted tetrahydrofuran-2-yl; or
      (iii) when A is 1-hydroxy-benzene-3,5-diyl or optionally substituted phenylene, two R^{L1} substituents taken together form spiro cyclopropyl, L¹ is -N(CH₃)C(O)- or -N(CH₃)C(O)CH₂N(CH₃)- and B is -CH(R⁹)-, wherein R⁹ is C₁₋₆ alkyl or 3- to 6-membered cycloalkyl, then W is not 3-cyclopropylaziridin-2-yl, 3-phenyltetrahydrofuran-2-yl, optionally substituted aziridin-2-yl or optionally substituted tetrahydrofuran-2-yl; or
      (iv) when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)- and B is - CH(R⁹)-, wherein R⁹ is isopropyl or cyclopentyl, then W is not 3-cyclopropylaziridin-2-yl, 3-phenyltetrahydrofuran-2-yl, optionally substituted aziridin-2-yl or optionally substituted tetrahydrofuran-2-yl; or
      (v) when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)- and B is - CH(R⁹)-, wherein R⁹ is C₁₋₆ alkyl or 3- to 6-membered cycloalkyl, then W is not 3-cyclopropylaziridin-2-yl, 3-phenyltetrahydrofuran-2-yl, optionally substituted aziridin-2-yl or optionally substituted tetrahydrofuran-2-yl; or
      (vi) when A is 1-hydroxy-benzene-3,5-diyl or optionally substituted phenylene, two R^{L1} substituents taken together form optionally substituted spiro cyclopropyl, L¹ is -N(CH₃)C(O)- or -N(CH₃)C(O)CH₂N(CH₃)-, R¹⁴ is absent and B is -CH(R⁹)-, wherein R⁹ is isopropyl or cyclopentyl, then R² is not ethyl or optionally substituted C₁₋₆ alkyl; or
      (vii) when A is 1-hydroxy-benzene-3,5-diyl or optionally substituted phenylene, R^{L1} and R^{L2} taken together form optionally substituted fused cyclopropyl, L¹ is -N(CH₃)C(O)- or -N(CH₃)C(O)CH₂N(CH₃)-, R¹⁴ is absent and B is -CH(R⁹)-, wherein R⁹ is isopropyl or cyclopentyl, then R² is not ethyl or optionally substituted C₁₋₆ alkyl; or
      (viii) when A is 1-hydroxy-benzene-3,5-diyl or optionally substituted phenylene and two R^{L1} substituents taken together form optionally substituted spiro cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl; or
      (ix) when A is 1-hydroxy-benzene-3,5-diyl or optionally substituted phenylene, R^{L1} and R^{L2} taken together form optionally substituted fused cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl; or
      (x) when A is 1-hydroxy-benzene-3,5-diyl or optionally substituted phenylene and two R^{L1} substituents taken together form optionally substituted spiro cyclopropyl, then R² and R¹⁴ combine with the atom to which they are attached to form an optionally substituted 3- to 8-membered cycloalkyl or optionally substituted 3- to 14-membered heterocycloalkyl; or
      (xi) when A is 1-hydroxy-benzene-3,5-diyl or optionally substituted phenylene, R^{L1} and R^{L2} taken together form optionally substituted fused cyclopropyl, then R² is optionally substituted C₂-C₆ alkenyl, optionally substituted C₂-C₆ alkynyl, optionally substituted 3- to 6-membered cycloalkyl, optionally substituted 3- to 14-membered heterocycloalkyl, optionally substituted 6-membered aryl, optionally substituted 5- to 10-membered heteroaryl.
3. A compound, or a pharmaceutically acceptable salt thereof, having the structure of Formula IIb: 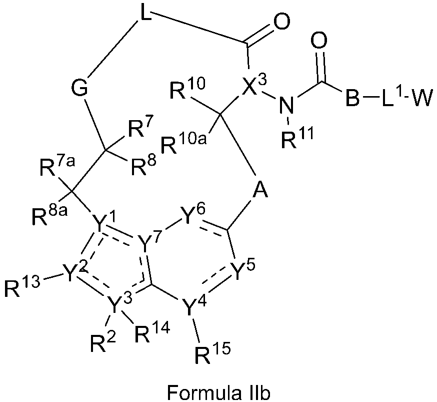
   wherein the dotted lines represent zero, one, two, three, or four non-adjacent double bonds;
   A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
   G is optionally substituted C₁-C₄ alkylene, optionally substituted C₁-C₄ alkenylene, optionally substituted C₁-C₄ heteroalkylene, -C(O)O-**C**H(R⁶)- where **C** is bound to -C(R⁷R⁸)-, -C(O)NH-**C**H(R⁶)-where **C** is bound to -C(R⁷R⁸)-, optionally substituted C₁-C₄ heteroalkylene, or 3- to 8-membered heteroarylene;
   L¹ is a linker;
   W is a cross-linking group comprising a carbodiimide, an oxazoline, a thiazoline, a chloroethyl urea, a chloroethyl thiourea, a chloroethyl carbamate, a chloroethyl thiocarbamate, an aziridine, a trifluoromethyl ketone, a boronic acid, a boronic ester, an N-ethoxycarbonyl-2-ethoxy-1,2-dihydroquinoline (EEDQ), an iso-EEDQ or other EEDQ derivative, an epoxide, an oxazolium, or a glycal;
   L has the structure of Formula VIIa or Vllb: 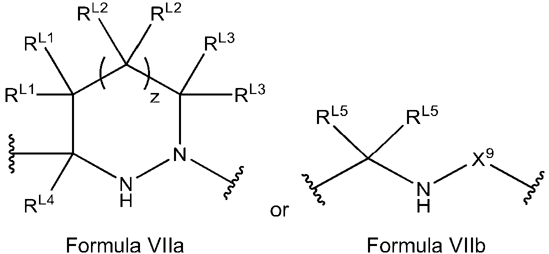
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 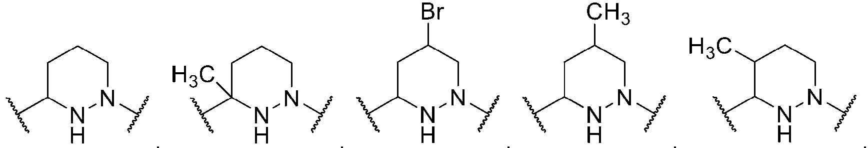 or 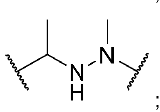 or
   optionally, wherein L is not 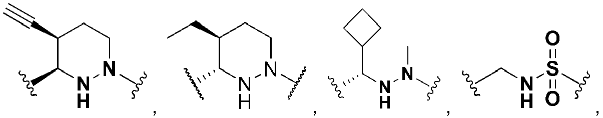 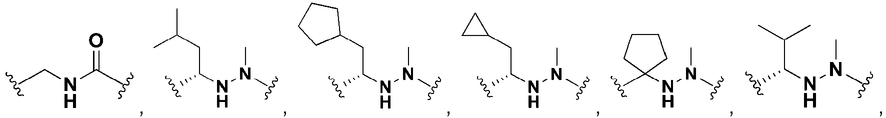 or 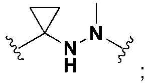
   X¹ is optionally substituted C₁-C₂ alkylene, NR, O, or S(O)_{q};
   X² is O or NH;
   X³ is N or CH;
   q is 0, 1, or 2;
   R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
   each R' is, independently, hydrogen or optionally substituted C₁-C₄ alkyl;
   Y¹ is C, CH, or N;
   Y², Y³, Y⁴, and Y⁷ are, independently, C or N;
   Y⁵ is CH, CH₂, or N;
   Y⁶ is C(O), CH, CH₂, or N;
   R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
   R¹³ and R² combine with the atoms to which they are attached to form an optionally substituted 3-to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
   R¹⁵ is absent, hydrogen, halogen, cyano, or methyl optionally substituted with 1 to 3 halogens;
   R⁵ is hydrogen, C₁-C₄ alkyl optionally substituted with halogen, cyano, hydroxy, or C₁-C₄ heteroalkyl, cyclopropyl, or cyclobutyl;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3- to 6-membered cycloalkyl or optionally substituted 3- to 7-membered heterocycloalkyl;
   R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
   R^{7a} and R^{8a} are, independently, hydrogen, halogen, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R^{7'} and R^{8'} combine with the carbon atom to which they are attached to form optionally substituted 3- to 6-membered cycloalkyl or optionally substituted 3- to 7-membered heterocycloalkyl;
   R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
   R⁹ and L combine with the atoms to which they are attached to form an optionally substituted 3-to 14-membered heterocycloalkyl;
   R^{9'} is hydrogen or optionally substituted C₁-C₆ alkyl; or
   R⁹ and R^{9'}, combined with the atoms to which they are attached, form a 3- to 6-membered cycloalkyl or a 3- to 6-membered heterocycloalkyl;
   R¹⁰ is hydrogen, halogen, hydroxy, optionally substituted C₁-C₃ heteroalkyl, or optionally substituted C₁-C₃ alkyl;
   R^{10a} is hydrogen or halogen;
   R¹¹ is hydrogen or optionally substituted C₁-C₃ alkyl; and
   R²¹ is hydrogen or optionally substituted C₁-C₃ alkyl wherein:
      (i) the compound is not 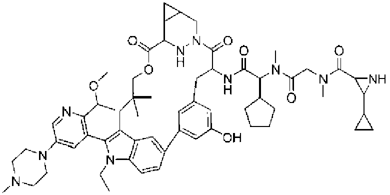 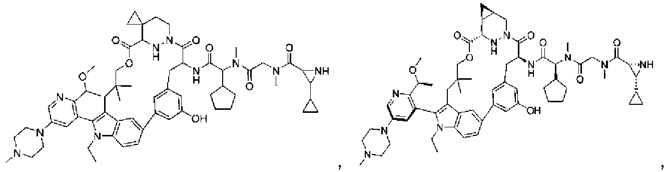 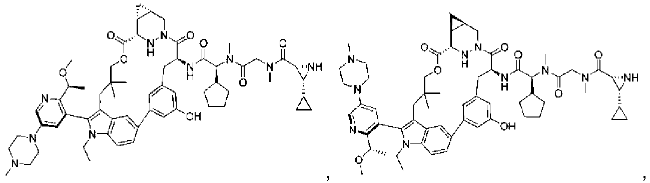 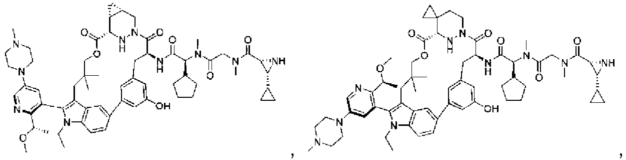 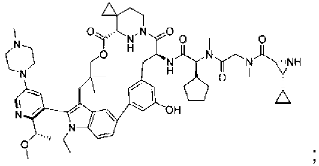 or
      (ii) when A is 1-hydroxy-benzene-3,5-diyl or optionally substituted phenylene, W is 3-cyclopropylaziridin-2-yl or optionally substituted aziridine and two R^{L1} substituents taken together form spiro cyclopropyl, then R² is not ethyl or C₁₋₆ alkyl; or
      (iii) when A is 1-hydroxy-benzene-3,5-diyl or optionally substituted phenylene, W is 3-cyclopropylaziridin-2-yl or optionally substituted aziridine and R^{L1} and R^{L2} taken together form fused cyclopropyl, then R² is not ethyl or C₁₋₆ alkyl; or
      (iv) when A is 1-hydroxy-benzene-3,5-diyl or optionally substituted phenylene and two R^{L1} substituents taken together form spiro cyclopropyl, then W is not 3-cyclopropylaziridin-2-yl or optionally substituted aziridine; or
      (v) when A is 1-hydroxy-benzene-3,5-diyl or optionally substituted phenylene and R^{L1} and R^{L2} taken together form fused cyclopropyl, then W is not 3-cyclopropylaziridin-2-yl or optionally substituted aziridine; or
      (vi) when A is 1-hydroxy-benzene-3,5-diyl or optionally substituted phenylene, W is 3-cyclopropylaziridin-2-yl or optionally substituted aziridine and two R^{L1} substituents taken together form spiro cyclopropyl, then L¹ is not -N(CH₃)C(O)CH₂N(CH₃)C(O)-; or
      (vii) when A is 1-hydroxy-benzene-3,5-diyl or optionally substituted phenylene, W is 3-cyclopropylaziridin-2-yl or optionally substituted aziridine and R^{L1} and R^{L2} taken together form fused cyclopropyl, then L¹ is not -N(CH₃)C(O)CH₂N(CH₃)C(O)-.
4. A compound, or a pharmaceutically acceptable salt thereof, having the structure of Formula IIc: 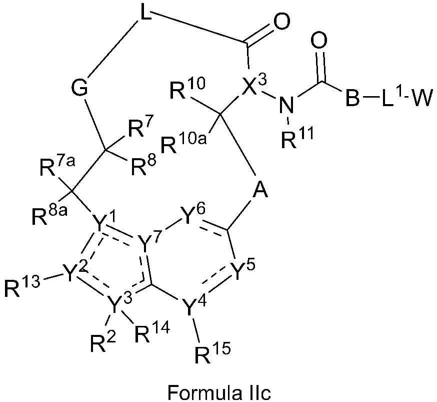
   wherein the dotted lines represent zero, one, two, three, or four non-adjacent double bonds;
   A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
   B is absent, -CH(R⁹)-, >C=CR⁹R^{9'}, or >CR⁹R^{9'} where the carbon is bound to the carbonyl carbon of -N(R¹¹)C(O)-, optionally substituted 3- to 6-membered cycloalkylene, optionally substituted 3- to 6-membered heterocycloalkylene, optionally substituted 6-membered arylene, or optionally substituted 5- to 6-membered heteroarylene;
   G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3- to 8-membered heteroarylene;
   L¹ is a linker;
   W is a cross-linking group comprising a vinyl ketone, a vinyl sulfone, an ynone, a haloacetyl, or an alkynyl sulfone;
   L has the structure of Formula VIIa or VIIb: 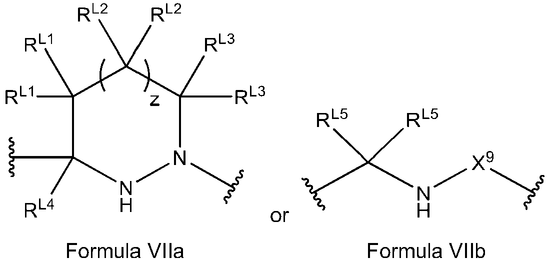
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 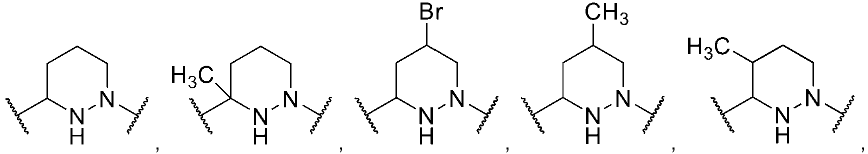 or 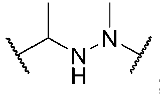 ; or
   optionally, wherein L is not 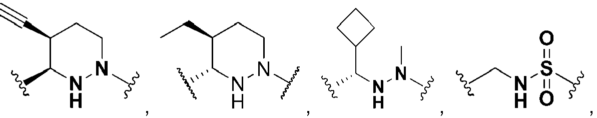 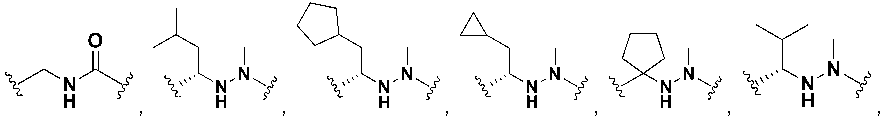 or 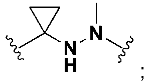
   X¹ is optionally substituted C₁-C₂ alkylene, NR, O, or S(O)_{q};
   X² is O or NH;
   X³ is N or CH;
   q is 0, 1, or 2;
   R is hydrogen, cyano, optionally substituted C₁-C₄ alkyl, optionally substituted C₂-C₄ alkenyl, optionally substituted C₂-C₄ alkynyl, C(O)R', C(O)OR', C(O)N(R')₂, S(O)R', S(O)₂R', or S(O)₂N(R')₂;
   each R' is, independently, hydrogen or optionally substituted C₁-C₄ alkyl;
   Y¹ is C, CH, or N;
   Y², Y³, Y⁴, and Y⁷ are, independently, C or N;
   Y⁶ is CH, CH₂, or N;
   Y⁶ is C(O), CH, CH₂, or N;
   R¹³ is cyano, optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 6-membered heterocycloalkyl, optionally substituted 6- to 10-membered aryl, or optionally substituted 5- to 10-membered heteroaryl, or
   R¹³ and R² combine with the atoms to which they are attached to form an optionally substituted 3-to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl; R¹⁴ is absent or R² and R¹⁴ combine with the atom to which they are attached to form an optionally substituted 3- to 8-membered cycloalkyl or optionally substituted 3- to 14-membered heterocycloalkyl;
   R¹⁵ is absent, hydrogen, halogen, cyano, or methyl optionally substituted with 1 to 3 halogens;
   R⁵ is hydrogen, C₁-C₄ alkyl optionally substituted with halogen, cyano, hydroxy, or C₁-C₄ heteroalkyl, cyclopropyl, or cyclobutyl;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3- to 6-membered cycloalkyl or optionally substituted 3- to 7-membered heterocycloalkyl;
   R⁸ is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3- to 6-membered cycloalkyl, or optionally substituted 3- to 7-membered heterocycloalkyl;
   R^{7a} and R^{8a} are, independently, hydrogen, halogen, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ heteroalkyl, optionally substituted C₁-C₃ alkyl, optionally substituted C₂-C₆ alkenyl, optionally substituted C₂-C₆ alkynyl, optionally substituted 3- to 8-membered cycloalkyl, optionally substituted 3- to 14-membered heterocycloalkyl, optionally substituted 5- to 10-membered heteroaryl, or optionally substituted 6- to 10-membered aryl, or
   R^{7'} and R^{8'} combine with the carbon atom to which they are attached to form optionally substituted 3- to 6-membered cycloalkyl or optionally substituted 3- to 7-membered heterocycloalkyl;
   R⁹ is hydrogen, fluoro, optionally substituted C₁-C₆ alkyl, optionally substituted C₁-C₆ heteroalkyl, optionally substituted 3- to 6-membered cycloalkyl, or optionally substituted 3- to 7-membered heterocycloalkyl; or
   R⁹ and L combine with the atoms to which they are attached to form an optionally substituted 3-to 14-membered heterocycloalkyl;
   R^{9'} is hydrogen or optionally substituted C₁-C₆ alkyl; or
   R⁹ and R^{9'}, combined with the atoms to which they are attached, form a 3- to 6-membered cycloalkyl or a 3- to 6-membered heterocycloalkyl;
   R¹⁰ is hydrogen, halogen, hydroxy, optionally substituted C₁-C₃ heteroalkyl, or optionally substituted C₁-C₃ alkyl;
   R^{10a} is hydrogen or halogen;
   R¹¹ is hydrogen or optionally substituted C₁-C₃ alkyl; and
   R²¹ is hydrogen or optionally substituted C₁-C₃ alkyl; wherein:
      (i) the compound is not: 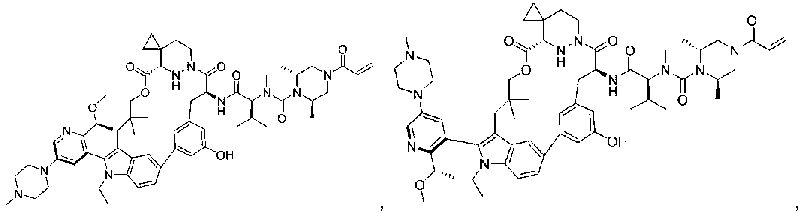 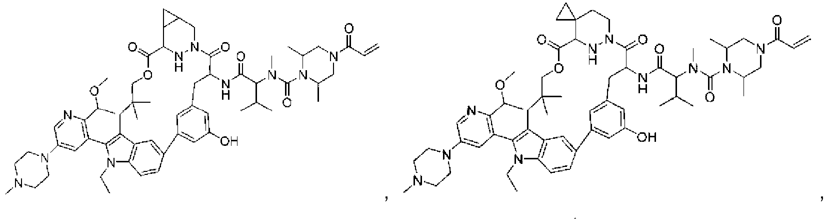 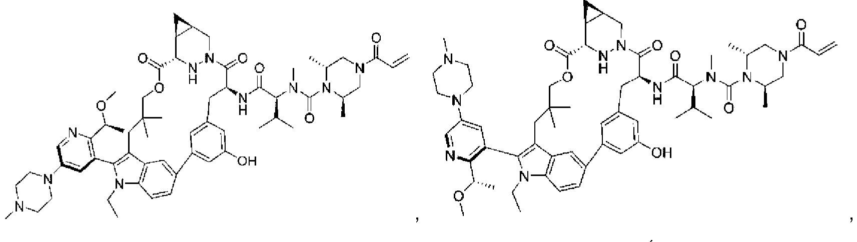 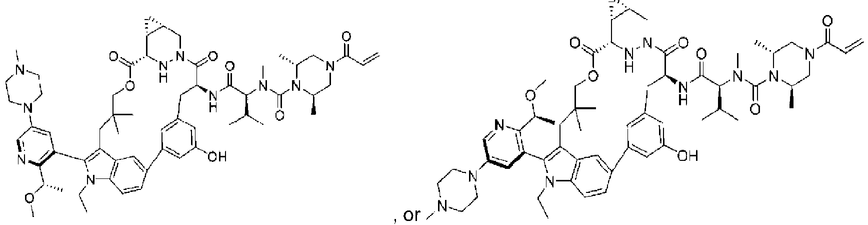 ; or
      (ii) when A is 1-hydroxy-benzene-3,5-diyl or optionally substituted phenylene, two R^{L1} substituents taken together form spiro cyclopropyl, and W is vinyl ketone, then R² is not ethyl or C₁₋₆ alkyl; or
      (iii) when A is 1-hydroxy-benzene-3,5-diyl or optionally substituted phenylene, R^{L1} and R^{L2} taken together form fused cyclopropyl, and W is vinyl ketone, then R² is not ethyl or C₁₋₆ alkyl; or
      (iv) when A is 1-hydroxy-benzene-3,5-diyl or optionally substituted phenylene and two R^{L1} substituents taken together form spiro cyclopropyl, then W is not vinyl ketone; or
      (v) when A is 1-hydroxy-benzene-3,5-diyl or optionally substituted phenylene, R^{L1} and R^{L2} taken together form fused cyclopropyl, then W is not vinyl ketone; or
      (vi) when A is 1-hydroxy-benzene-3,5-diyl or optionally substituted phenylene, W is vinyl ketone and two R^{L1} substituents taken together form spiro cyclopropyl or spiro cycloalkyl, then L¹ is other than 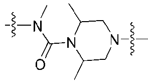 ; or
      (vii) when A is 1-hydroxy-benzene-3,5-diyl or optionally substituted phenylene, W is vinyl ketone and R^{L1} and R^{L2} taken together form fused cyclopropyl or fused cycloalkyl, then L¹ is other than 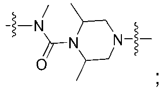 or
      (viii) when A is 1-hydroxy-benzene-3,5-diyl or optionally substituted phenylene, W is vinyl ketone and two R^{L1} substituents taken together form spiro cycloalkyl, then L¹ is not 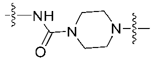 optionally substituted with 1, 2 or 3 independently selected C₁₋₆ alkyl; or
      (ix) when A is 1-hydroxy-benzene-3,5-diyl or optionally substituted phenylene, W is vinyl ketone and R^{L1} and R^{L2} taken together form fused cycloalkyl, then L¹ is not 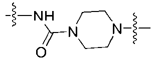 optionally substituted with 1, 2 or 3 independently selected C₁₋₆ alkyl.
5. A compound, or a pharmaceutically acceptable salt thereof, having the structure of Formula III: 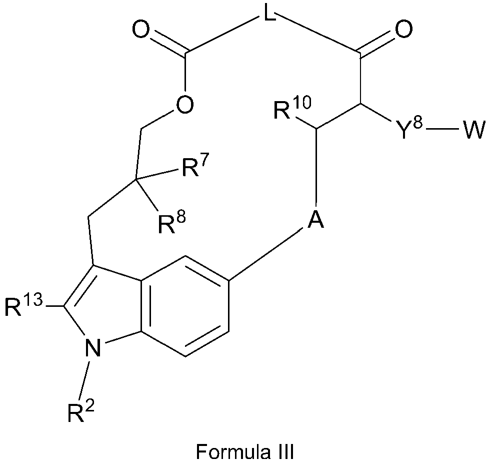
   wherein A is optionally substituted 3- to 6-membered cycloalkylene, optionally substituted 3- to 6-membered heterocycloalkylene, optionally substituted 6-membered arylene, optionally substituted 5- to 6-membered heteroarylene, optionally substituted C₂-C₄ alkylene, optionally substituted C₁-C₄ heteroalkylene or optionally substituted C₂-C₄ alkenylene;
   Y⁸ is 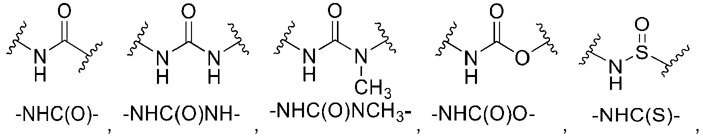 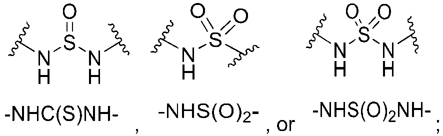
   W is hydrogen, C₁-C₄ alkyl, optionally substituted C₁-C₃ heteroalkyl, optionally substituted 3- to 10-membered heterocycloalkyl, optionally substituted 3- to 10-membered cycloalkyl, optionally substituted 6- to 10-membered aryl, or optionally substituted 5- to 10-membered heteroaryl;
   L has the structure of Formula VIIa or Vllb: 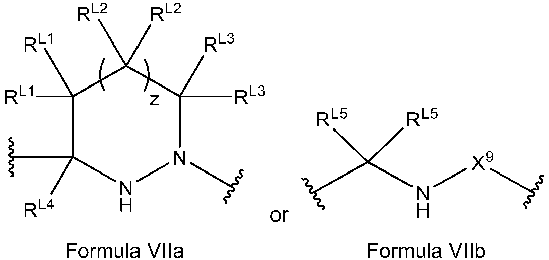
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl; or R^{L1} and R^{L2} taken together form a bond;
   wherein L does not have the structure of 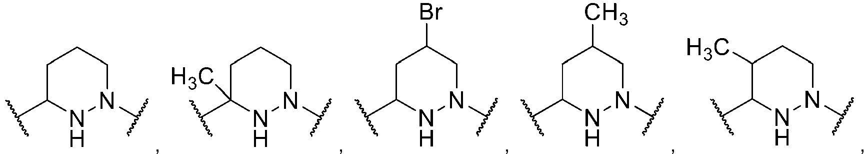 or 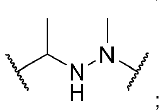 or
   optionally, wherein L is not 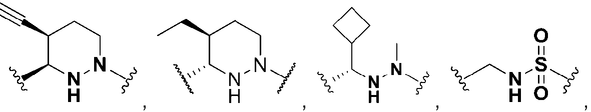 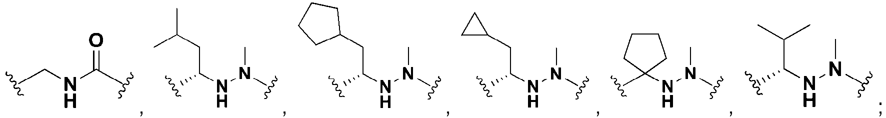 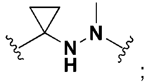
   X⁴ and X⁵ are each, independently, CH₂, CH(CH₃) or NH;
   R¹³ is optionally substituted C₁-C₆ alkyl, optionally substituted C₁-C₆ heteroalkyl, optionally substituted 3- to 6-membered cycloalkyl, optionally substituted 3- to 6-membered cycloalkenyl, optionally substituted 3- to 15-membered heterocycloalkyl, optionally substituted 6- to 10-membered aryl, or optionally substituted 5- to 10-membered heteroaryl;
   R² is hydrogen, optionally substituted C₁-C₆ alkyl, optionally substituted C₂-C₆ alkenyl, optionally substituted C₂-C₆ alkynyl, optionally substituted 3- to 6-membered cycloalkyl, optionally substituted 3- to 7-membered heterocycloalkyl, optionally substituted 6-membered aryl, optionally substituted 5- or 6-membered heteroaryl;
   R¹⁰ is hydrogen, hydroxy, optionally substituted C₁-C₆ alkoxy, optionally substituted C₁-C₃ alkyl, optionally substituted C₁-C₆ heteroalkyl, or optionally substituted 3- to 10-membered heterocycloalkyl; and
   R⁷ and R⁸ are each, independently, selected from fluoro or CH₃, or R⁷ and R⁸ combine with the atoms to which they are attached to make a 3-membered cycloalkyl; wherein:
      (i) the compound is not E10 to E52 in Table 2 and those set forth in Table 5; or
      (ii) when A is thiazole-2,4-diyl and two R^{L1} substituents taken together form spiro cyclopropyl, then R² is not optionally substituted C₁₋₆ alkyl; or
      (iii) when A is thiazole-2,4-diyl and two R^{L2} substituents taken together form spiro cyclopropyl, then R² is not optionally substituted C₁₋₆ alkyl; or
      (iv) when A is thiazole-2,4-diyl and R^{L1} and R^{L2} taken together form optionally substituted fused cyclopropyl, then R² is not optionally substituted C₁₋₆ alkyl; or
      (v) when A is thiazole-2,4-diyl and R^{L1} and R^{L2} taken together form a bond, then R² is not optionally substituted C₁₋₆ alkyl; or
      (vi) when A is thiazole-2,4-diyl and R^{L1} and R^{L2} taken together form fused cyclobutyl, then R² is not optionally substituted C₁₋₆ alkyl; or
      (vii) when A is thiazole-2,4-diyl and R^{L1} and R^{L3} together with the atoms to which they are attached form bridged cyclobutyl, then R² is not optionally substituted C₁₋₆ alkyl; or
      (viii) when A is thiazole-2,4-diyl and R^{L1} and R^{L3} together with the atoms to which they are attached form bridged cyclobutyl, then W is not optionally substituted cyclopropyl.
6. A compound, or pharmaceutically acceptable salt thereof, having the structure of Formula IV: 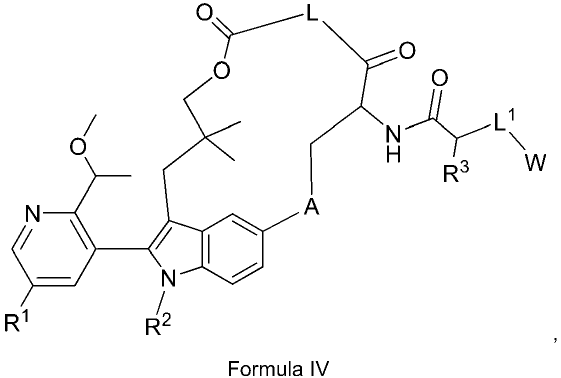
   wherein A is optionally substituted 3- to 6-membered heterocycloalkylene, optionally substituted 3- to 6-membered cycloalkylene, optionally substituted 6-membered arylene, or optionally substituted 5-to 10-membered heteroarylene;
   L¹ is a linker;
   L has the structure of Formula VIIa or Vllb: 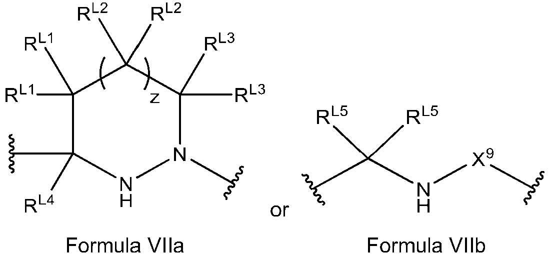
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 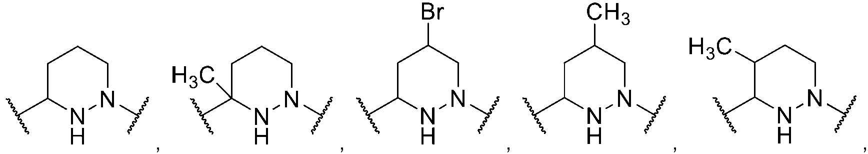 or 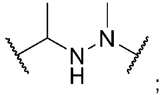 or
   optionally, wherein L is not 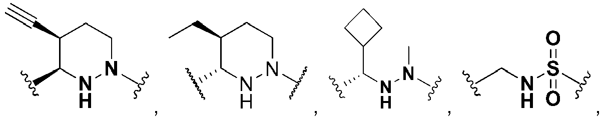 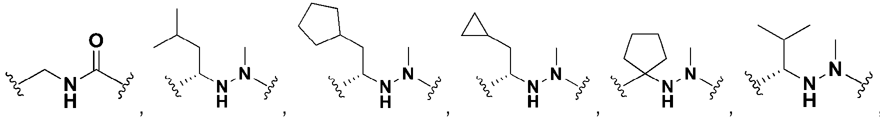 or 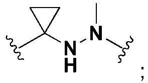
   W is a cross-linking group comprising a vinyl ketone, vinyl sulfone, ynone, or an alkynyl sulfone;
   R¹ is hydrogen, optionally substituted 3- to 10-membered heterocycloalkyl, or optionally substituted C₁-C₆ heteroalkyl;
   R² is optionally substituted C₁-C₆ alkyl; and
   R³ is optionally substituted C₁-C₆ alkyl or optionally substituted C₁-C₃ heteroalkyl;
      (i) the compound is not: 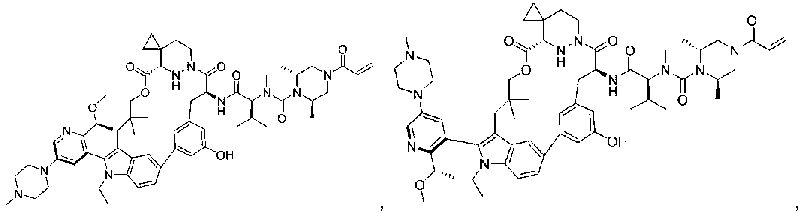 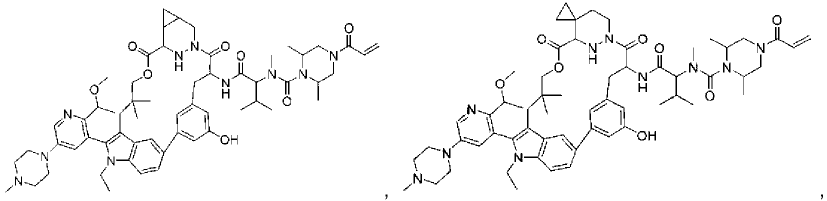 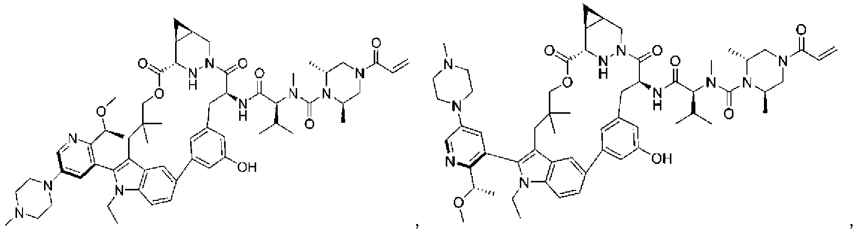 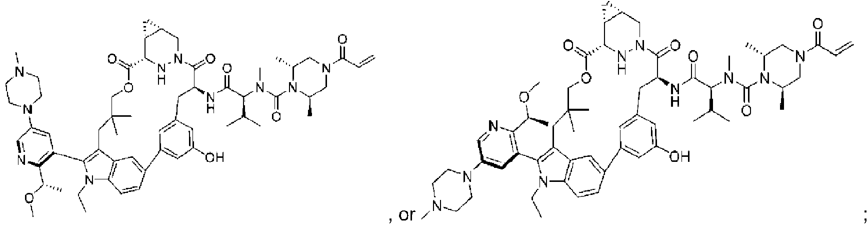 or
      (ii) when A is 1-hydroxy-benzene-3,5-diyl or optionally substituted phenylene, two R^{L1} substituents taken together form spiro cyclopropyl, and W is vinyl ketone, then R² is not ethyl; or
      (iii) when A is 1-hydroxy-benzene-3,5-diyl or optionally substituted phenylene, R^{L1} and R^{L2} taken together form fused cyclopropyl, and W is vinyl ketone, then R² is not ethyl; or
      (iv) when A is 1-hydroxy-benzene-3,5-diyl or optionally substituted phenylene and two R^{L1} substituents taken together form spiro cyclopropyl, then W is not vinyl ketone; or
      (v) when A is 1-hydroxy-benzene-3,5-diyl or optionally substituted phenylene, R^{L1} and R^{L2} taken together form fused cyclopropyl, then W is not vinyl ketone; or
      (vi) when A is 1-hydroxy-benzene-3,5-diyl or optionally substituted phenylene, W is vinyl ketone and two R^{L1} substituents taken together form spiro cyclopropyl or spiro cycloalkyl, then L¹ is other than 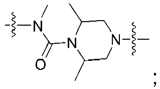 or
      (vii) when A is 1-hydroxy-benzene-3,5-diyl or optionally substituted phenylene, W is vinyl ketone and R^{L1} and R^{L2} taken together form fused cyclopropyl or fused cycloalkyl, then L¹ is not 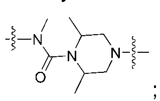 or
      (viii) when A is 1-hydroxy-benzene-3,5-diyl or optionally substituted phenylene, W is vinyl ketone and two R^{L1} substituents taken together form spiro cyclopropyl or spiro cycloalkyl, then L¹ is not 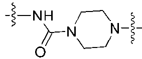 optionally substituted with 1, 2 or 3 independently selected C₁₋₆ alkyl; or
      (ix) when A is 1-hydroxy-benzene-3,5-diyl or optionally substituted phenylene, W is vinyl ketone and two R^{L1} and R^{L2} taken together form fused cyclopropyl or fused cycloalkyl, then L¹ is not 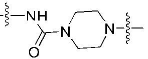 optionally substituted with 1, 2 or 3 independently selected C₁₋₆ alkyl.
7. A compound, or a pharmaceutically acceptable salt thereof, having the structure of Formula V: 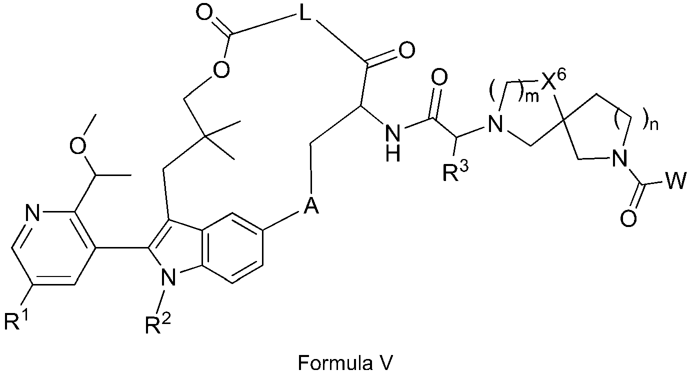
   wherein A is optionally substituted 3- to 6-membered heterocycloalkylene, optionally substituted 3- to 6-membered cycloalkylene, optionally substituted 6-membered arylene, or optionally substituted 5-to 10-membered heteroarylene;
   L has the structure of Formula VIIa or Vllb: 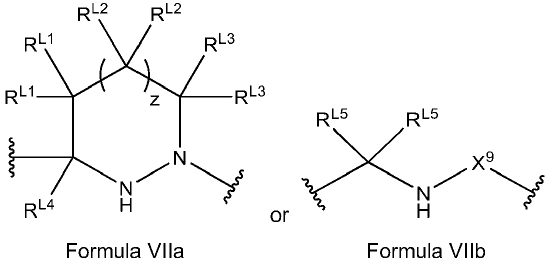
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 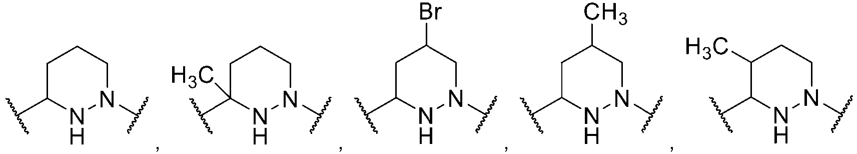 , or 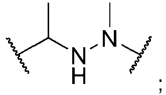 or
   optionally, wherein L is not 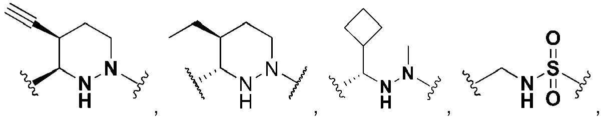 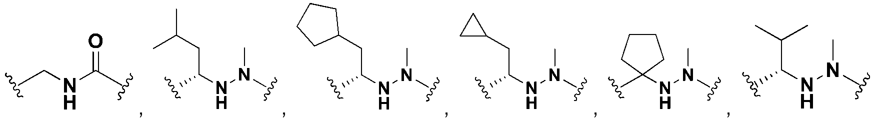 , or 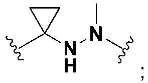
   W is a cross-linking group comprising an aziridine, an epoxide, a carbodiimide, an oxazoline, a thiazoline, a chloroethyl urea, a chloroethyl thiourea, a chloroethyl carbamate, a chloroethyl thiocarbamate, a trifluoromethyl ketone, a boronic acid, a boronic ester, an N-ethoxycarbonyl-2-ethoxy-1,2-dihydroquinoline (EEDQ), an iso-EEDQ or other EEDQ derivative, an oxazolium, or a glycal;
   X⁶ is CH₂ or O;
   m is 1 or 2;
   n is 0 or 1;
   R¹ is hydrogen or optionally substituted 3- to 10-membered heterocycloalkyl; and
   R² is optionally substituted C₁-C₆ alkyl.
8. A compound, or a pharmaceutically acceptable salt thereof, having the structure of Formula VI: 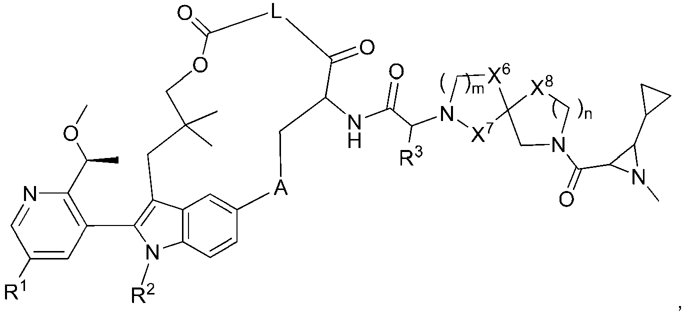 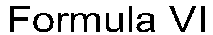
   wherein A is optionally substituted 3- to 6-membered heterocycloalkylene, optionally substituted 3- to 6-membered cycloalkylene, optionally substituted 6-membered arylene, or optionally substituted 5-to 10-membered heteroarylene;
   L has the structure of Formula VIIa or Vllb: 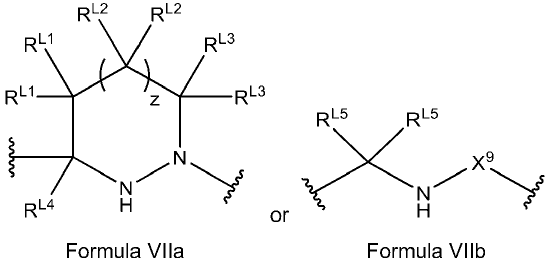
   optionally, wherein L is not 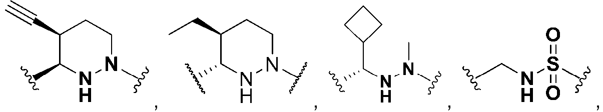 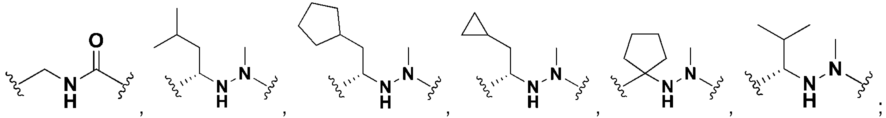 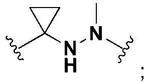
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   X⁶, X⁷, and X⁸ are each independently selected from CH₂, CHF, CF₂, C=O, or O;
   m is 1 or 2;
   n is 0 or 1;
   R¹ is hydrogen, optionally substituted C₁-C₆ heteroalkyl, or optionally substituted 3- to 10-membered heterocycloalkyl;
   R² is optionally substituted C₁-C₆ alkyl; and
   R³ is optionally substituted C₁-C₆ alkyl, optionally substituted C₁-C₆ heteroalkyl, optionally substituted 3- to 6-membered cycloalkyl, or optionally substituted heterocycloalkyl,
   and wherein each hydrogen is independently, optionally, isotopically enriched for deuterium.
9. A compound having Formula VIIIa: 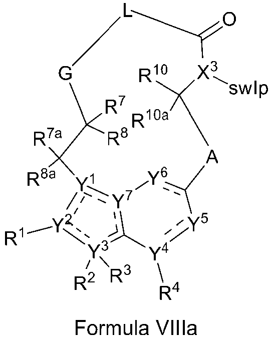 or a pharmaceutically acceptable salt thereof, wherein:
   the dotted lines represent zero, one, two, three, or four non-adjacent double bonds;
   L has the structure of Formula VIIa or Vllb:

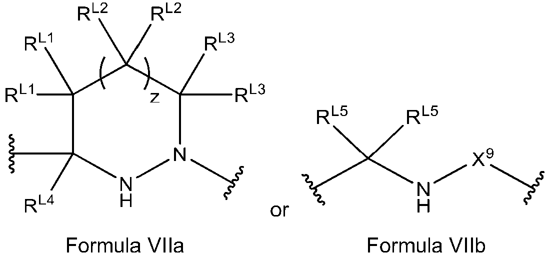

   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 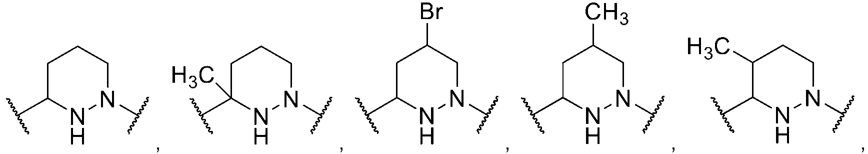 or 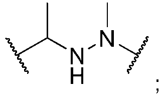 or
   optionally, wherein L is not 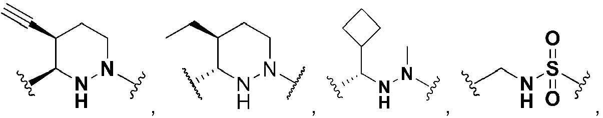 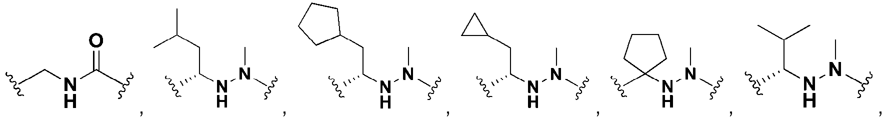 or 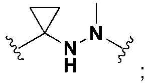
   A is optionally substituted C₂-C₄ alkylene, optionally substituted C₁-C₄ heteroalkylene, or optionally substituted C₂-C₄ alkenylene;
   G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3 to 8-membered heteroarylene;
   swlp (Switch I/P-loop) is an organic moiety that non-covalently binds to both the Switch I binding pocket and residues 12 or 13 of the P-loop of a Ras protein;
   X³ is N or CH;
   Y¹ is C, CH, or N;
   Y², Y³, Y⁴, and Y⁷ are, independently, C or N;
   Y⁶ is CH, CH₂, or N;
   Y⁶ is C(O), CH, CH₂, or N;
   R¹ is cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl, or
   R¹ and R² combine with the atoms to which they are attached to form an optionally substituted 3 to 14-membered heterocycloalkyl;
   R² is absent, hydrogen, optionally substituted C₁-C₆ alkyl, optionally substituted C₂-C₆ alkenyl, optionally substituted C₂-C₆ alkynyl, optionally substituted 3 to 6-membered cycloalkyl, optionally substituted 3 to 7-membered heterocycloalkyl, optionally substituted 6-membered aryl, optionally substituted 5 or 6-membered heteroaryl;
   R³ is absent, or
   R² and R³ combine with the atom to which they are attached to form an optionally substituted 3 to 8-membered cycloalkyl or optionally substituted 3 to 14-membered heterocycloalkyl;
   R⁴ is absent, hydrogen, halogen, cyano, or methyl optionally substituted with 1 to 3 halogens;
   R⁶ is hydrogen or methyl; R⁷ is hydrogen, halogen, or optionally substituted C₁-C₃ alkyl, or
   R⁶ and R⁷ combine with the carbon atoms to which they are attached to form an optionally substituted 3 to 6-membered cycloalkyl or optionally substituted 3 to 7-membered heterocycloalkyl;
   R⁸ is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3 to 6-membered cycloalkyl, or optionally substituted 3 to 7-membered heterocycloalkyl;
   R^{7a} and R^{8a} are, independently, hydrogen, halo, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
   R ⁷' and R^{8'} combine with the carbon atom to which they are attached to form optionally substituted 3 to 6-membered cycloalkyl or optionally substituted 3 to 7-membered heterocycloalkyl;
   R¹⁰ is hydrogen, halo, hydroxy, C₁-C₃ alkoxy, or C₁-C₃ alkyl; and
   R^{10a} is hydrogen or halo.
10. The compound of clause 9, or a pharmaceutically acceptable salt thereof, wherein the compound has formula VIIIb, VIIIc, VIIId, VIIIe, VIIIf or VIIIg: 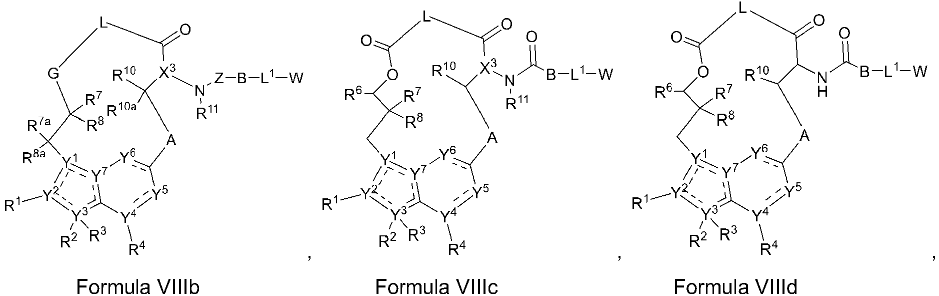 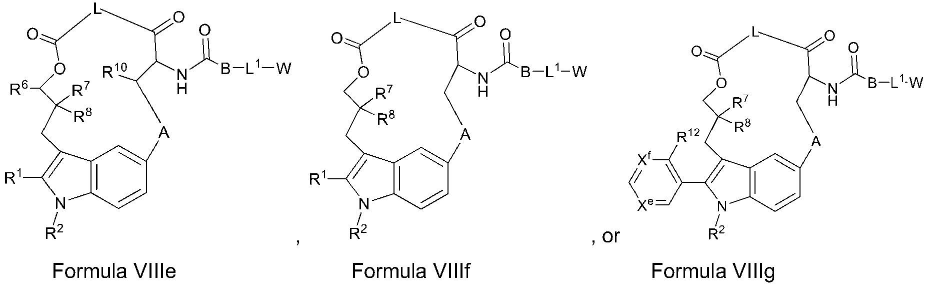
   wherein at each occurrence,
   each L is independently having Formula VIIa or Vllb: 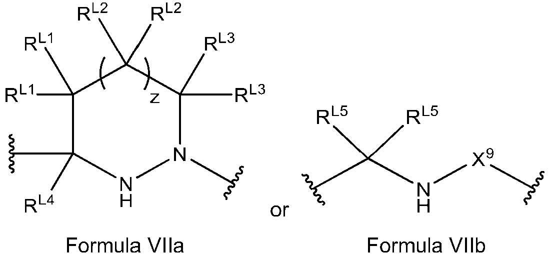
   z is 0, 1, or 2;
   X⁹ is -NR^{L6}-, -C(O)-, or -S(O)₂-;
   each of R^{L1}, R^{L2}, R^{L3}, R^{L4}, R^{L4}, R^{L5}, and R^{L6} is, independently, hydrogen, halogen, hydroxyl, optionally substituted C₁-C₆ alkyl, optionally substituted C₂-C₆ alkenyl, optionally substituted C₂-C₆ alkynyl, optionally substituted C₁-C₆ heteroalkyl, or optionally substituted C₃-C₈ cycloalkyl; or any two of R^{L1}, R^{L2}, R^{L3}, R^{L4}, R^{L4}, R^{L5}, and R^{L6} together with the atoms to which they are attached and any intervening atoms to form an optionally substituted C₃-C₈ cycloalkyl or a 3- to 8-membered heterocyclyl;
   wherein L does not have the structure of 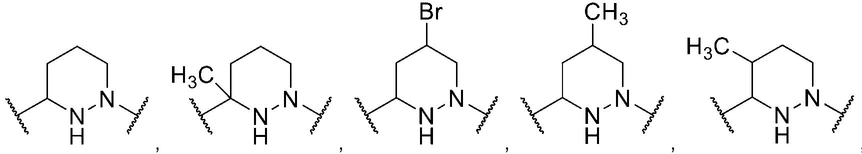 or 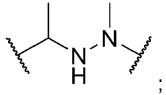
   G is optionally substituted C₁-C₄ alkylene, optionally substituted C₁-C₄ alkenylene, optionally substituted C₁-C₄ heteroalkylene, -C(O)O-CH(R⁶)- where C is bound to -C(R⁷R⁸)-, -C(O)NH-CH(R⁶)-where C is bound to -C(R⁷R⁸)-, optionally substituted C₁-C₄ heteroalkylene, or 3 to 8-membered heteroarylene;
   Z is -C(O)- or -S(O)₂-;
   each B is independently absent, -NH-, -N(CH₃)-, -O-, -CH(R⁹)- or >C=CR⁹R^{9'} where the carbon is bound to the carbonyl carbon of -N(R¹¹)C(O)-, optionally substituted 3 to 6-membered cycloalkylene, optionally substituted 3 to 6-membered heterocycloalkylene, optionally substituted 6-membered arylene, or 5 to 6-membered heteroarylene;
   each L¹ is independently absent or a linker;
   each W is independently hydrogen, cyano, optionally substituted amino, optionally substituted amido, optionally substituted C₁-C₄ alkoxy, optionally substituted C₁-C₄ hydroxyalkyl, optionally substituted C₁-C₄ aminoalkyl, optionally substituted C₁-C₄ haloalkyl, optionally substituted C₁-C₄ alkyl, optionally substituted C₁-C₄ guanidinoalkyl, C₀-C₄ alkyl optionally substituted 3 to 11-membered heterocycloalkyl, optionally substituted 3 to 10-membered cycloalkyl, optionally substituted 6 to 10-membered aryl, or optionally substituted 3 to 10-membered heteroaryl;
   each X³ is independently N or CH;
   X^{e} is N, CH, or CR¹⁷;
   X^{f} is N or CH;
   R¹² is optionally substituted C₁-C₆ alkyl or optionally substituted C₁-C₆ heteroalkyl;
   R¹⁷ is optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl.
   each Y¹ is independently C, CH, or N;
   Y², Y³, Y⁴, and Y⁷ are, independently, C or N;
   each Y⁵ is independently CH, CH₂, or N;
   each Y⁶ is independently C(O), CH, CH₂, or N;
   each R¹ is independently cyano, optionally substituted C₁-C₆ alkyl, optionally substituted C₁-C₆ heteroalkyl, optionally substituted 3 to 6-membered cycloalkyl, optionally substituted 3 to 6-membered cycloalkenyl, optionally substituted 3 to 6-membered heterocycloalkyl, optionally substituted 6 to 10-membered aryl, or optionally substituted 5 to 10-membered heteroaryl, or
   R¹ and R² combine with the atoms to which they are attached to form an optionally substituted 3 to 14-membered heterocycloalkyl;
   each R² is independently absent, hydrogen, optionally substituted C₁-C₆ alkyl, optionally substituted C₂-C₆ alkenyl, optionally substituted C₂-C₆ alkynyl, optionally substituted 3 to 6-membered cycloalkyl, optionally substituted 3 to 7-membered heterocycloalkyl, optionally substituted 6-membered aryl, optionally substituted 5 or 6-membered heteroaryl;
   each R³ is independently absent, or
   R² and R³ combine with the atom to which they are attached to form an optionally substituted 3 to 8-membered cycloalkyl or optionally substituted 3 to 14-membered heterocycloalkyl;
   each R⁴ is independently absent, hydrogen, halogen, cyano, or methyl optionally substituted with 1 to 3 halogens;
   each R⁶ is independently hydrogen or methyl; each R⁷ is independently hydrogen, halogen, or optionally substituted C₁-C₃ alkyl, or
   R⁶ and R⁷ combine with the carbon atoms to which they are attached to form an optionally substituted 3 to 6-membered cycloalkyl or optionally substituted 3 to 7-membered heterocycloalkyl;
   each R⁸ is independently hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
   R⁷ and R⁸ combine with the carbon atom to which they are attached to form C=CR^{7'}R^{8'}; C=N(OH), C=N(O-C₁-C₃ alkyl), C=O, C=S, C=NH, optionally substituted 3 to 6-membered cycloalkyl, or optionally substituted 3 to 7-membered heterocycloalkyl;
   R^{7a} and R^{8a} are, independently, hydrogen, halo, optionally substituted C₁-C₃ alkyl, or combine with the carbon to which they are attached to form a carbonyl;
   R^{7'} is hydrogen, halogen, or optionally substituted C₁-C₃ alkyl; R^{8'} is hydrogen, halogen, hydroxy, cyano, optionally substituted C₁-C₃ alkoxy, optionally substituted C₁-C₃ alkyl, optionally substituted C₂-C₆ alkenyl, optionally substituted C₂-C₆ alkynyl, optionally substituted 3 to 8-membered cycloalkyl, optionally substituted 3 to 14-membered heterocycloalkyl, optionally substituted 5 to 10-membered heteroaryl, or optionally substituted 6 to 10-membered aryl, or
   R^{7'} and R^{8'} combine with the carbon atom to which they are attached to form optionally substituted 3 to 6-membered cycloalkyl or optionally substituted 3 to 7-membered heterocycloalkyl;
   each R⁹ is independently hydrogen, F, optionally substituted C₁-C₆ alkyl, optionally substituted C₁-C₆ heteroalkyl, optionally substituted 3 to 6-membered cycloalkyl, or optionally substituted 3 to 7-membered heterocycloalkyl;
   R⁹ and L combine with the atoms to which they are attached to form an optionally substituted 3 to 14-membered heterocycloalkyl;
   ach R^{9'} is independently hydrogen or optionally substituted C₁-C₆ alkyl;
   each R¹⁰ is independently hydrogen, halo, hydroxy, C₁-C₃ alkoxy, or C₁-C₃ alkyl;
   R^{10a} is hydrogen or halo;
   R¹¹ is hydrogen or C₁-C₃ alkyl.
11. The compound of any one of clauses 1 to 10, or a pharmaceutically acceptable salt thereof, wherein L has the structure of Formula VIIa, Formula VIIa-1, Formula VIIa-2, Formula VIIa-3, Formula Vlla-4, Formula Vlla-5, Formula Vlla-6, Formula VIIb, Formula VIIb-1, Formula VIIb-2, or : 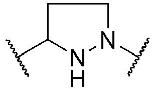 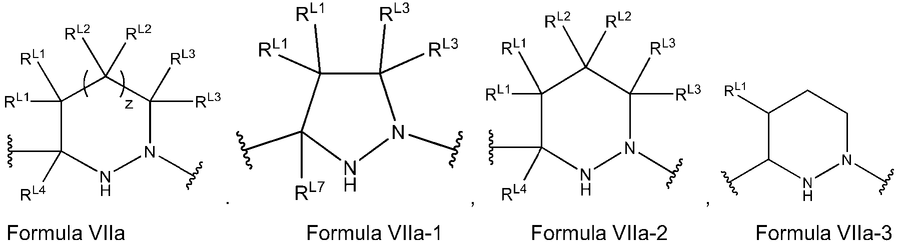 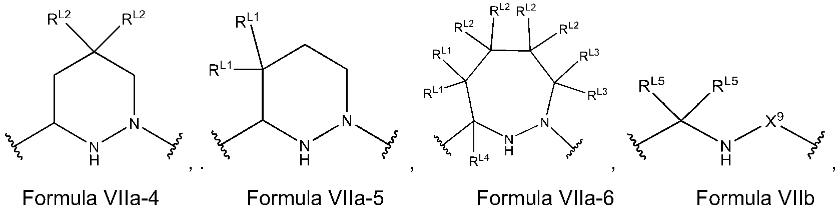 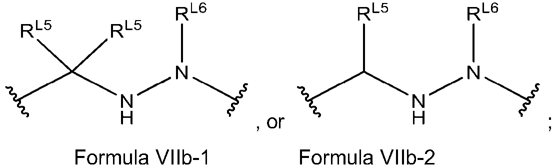 wherein X⁹ is -NR^{L6}-; or X⁹ is -C(O)-; or X⁹ is S(O)₂-.
12. The compound of any one of clauses 1 to 11, or a pharmaceutically acceptable salt thereof, wherein z is 0; or z is 1; or z is 2.
13. The compound of any one of clauses 1 to 12, or a pharmaceutically acceptable salt thereof, wherein R^{L1} is hydrogen; or R^{L1} is optionally substituted C₁-C₆ alkyl; or R^{L1} is methyl, ethyl, or trifluoromethyl; or R^{L1} is optionally substituted C₁-C₆ heteroalkyl; or R^{L1} is methoxy or ethoxy; R^{L1} is optionally substituted C₂-C₆ alkynyl; or R^{L1} is ethynyl.
14. The compound of any one of clauses 1 to 13, or a pharmaceutically acceptable salt thereof, wherein R^{L2} is hydrogen; or R^{L2} is halogen; or R^{L2} is fluoro.
15. The compound of any one of clauses 1 to 14, or a pharmaceutically acceptable salt thereof, wherein R^{L3} is hydrogen; or R^{L3} is optionally substituted C₁-C₆ alkyl; or R^{L3} is methyl.
16. The compound of any one of clauses 1 to 15, or a pharmaceutically acceptable salt thereof, wherein:
   R^{L4} is hydrogen; or
   R^{L1} and R^{L4} combine to form an optionally substituted C₄ cycloalkyl; or
   R^{L1} and R^{L3} combine to form an optionally substituted C₄ cycloalkyl; or
   R^{L1} and R^{L3} combine to form an optionally substituted C₅ cycloalkyl; or
   two R^{L1} combine to form an optionally substituted C₃-C₆ cycloalkyl; or
   R^{L1} and R^{L2} combine to form an optionally substituted C₃-C₆ cycloalkyl.
17. The compound of any one of clauses 1 to 16, or a pharmaceutically acceptable salt thereof, wherein L is: 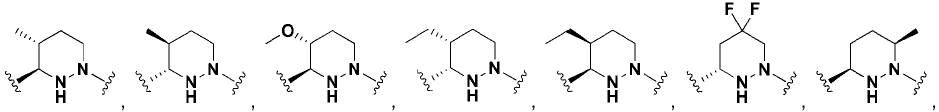 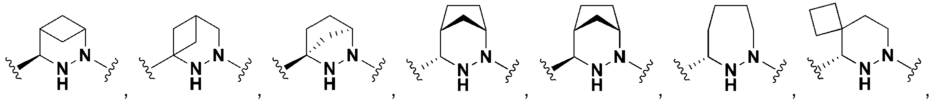 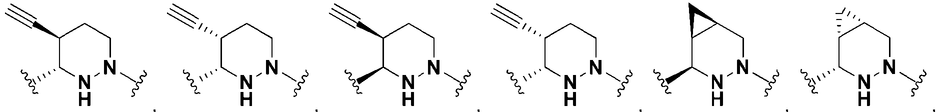 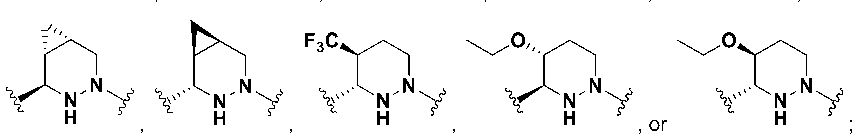 or
   L is: 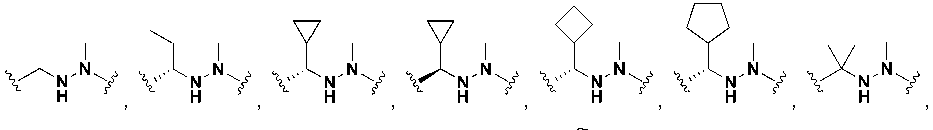 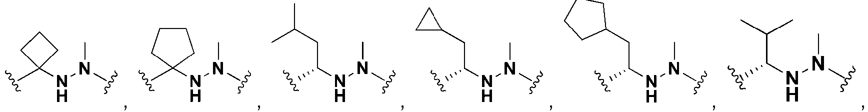 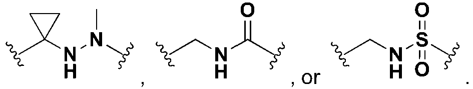
18. The compound of any one of clauses 1 to 17, or a pharmaceutically acceptable salt thereof, wherein R^{L6} is optionally substituted C₁-C₆ alkyl; or R^{L6} is methyl.
19. The compound of any one of clauses 1 to 18, or a pharmaceutically acceptable salt thereof, wherein R^{L5} is hydrogen; or R^{L5} is optionally substituted C₁-C₆ alkyl; or R^{L5} is optionally substituted C₃-C₈ cycloalkyl; or two R^{L5} combine to form an optionally substituted C₃-C₈ cycloalkyl.
20. The compound of any one of clauses 1 to 19, or a pharmaceutically acceptable salt thereof, wherein:
   A is optionally substituted 3- to 6-membered heterocycloalkylene, optionally substituted 6-membered arylene, or optionally substituted 5- to 10-membered heteroarylene; or
   A is optionally substituted 6-membered arylene; or
   A is 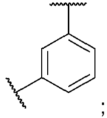 or
   A is optionally substituted 3- to 6-membered heterocycloalkylene;
   A is 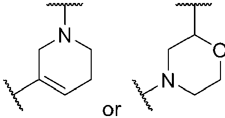 ; or A is optionally substituted 5- to 10-membered heteroarylene; or
   A is 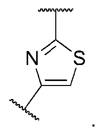
21. The compound of any one of clauses 2 to 20, or a pharmaceutically acceptable salt thereof, wherein R² is ethyl or haloethyl.
22. The compound of any one of clauses 1 and 6 to 21, or a pharmaceutically acceptable salt thereof, wherein R³ is optionally substituted C₁-C₆ alkyl.
23. A compound, or a pharmaceutically acceptable salt thereof, of Table 1.
24. A pharmaceutical composition comprising a compound, or a pharmaceutically acceptable salt thereof, of any one of clauses 1 to 23 and a pharmaceutically acceptable excipient.
25. A method of treating cancer in a subject in need thereof, the method comprising administering to the subject a therapeutically effective amount of a compound, or a pharmaceutically acceptable salt thereof, of any one of clauses 1 to 23 or a pharmaceutical composition of clause 24,
   optionally wherein the cancer is pancreatic cancer, colorectal cancer, non-small cell lung cancer, or endometrial cancer;
   or optionally wherein the cancer comprises a Ras mutation; or
   optionally wherein the method further comprises administering an additional anti-cancer therapy, optionally wherein the additional anti-cancer therapy is an EGFR inhibitor, a second Ras inhibitor, a SHP2 inhibitor, a SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, a HER2 inhibitor, or a combination thereof.
26. A method of treating a Ras protein-related disorder in a subject in need thereof, the method comprising administering to the subject a therapeutically effective amount of a compound of any one of clauses 1 to 23, or a pharmaceutically acceptable salt thereof, or a pharmaceutical composition of clause 24, optionally wherein the Ras protein is K-Ras; or
   optionally wherein the method further comprises administering an additional anti-cancer therapy, optionally wherein the additional anti-cancer therapy is an EGFR inhibitor, a second Ras inhibitor, a SHP2 inhibitor, a SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, a HER2 inhibitor, or a combination thereof.
27. A method of inhibiting a Ras protein in a cell, the method comprising contacting the cell with an effective amount of a compound of any one of clauses 1 to 23, or a pharmaceutically acceptable salt thereof, or a pharmaceutical composition of clause 24,
   optionally wherein the Ras protein is K-Ras;
   or optionally wherein the cell is a cancer cell, optionally wherein the cancer cell is a pancreatic cancer cell, a colorectal cancer cell, a non-small cell lung cancer cell, or an endometrial cancer cell; or
   optionally wherein the method further comprises administering an additional anti-cancer therapy, optionally wherein the additional anti-cancer therapy is an EGFR inhibitor, a second Ras inhibitor, a SHP2 inhibitor, a SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK4/6 inhibitor, a HER2 inhibitor, or a combination thereof.

## Claims

1. A compound having the structure of formula [A] or formula [B]:
or a salt thereof, wherein: 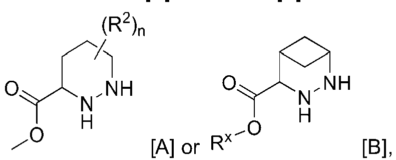 R^{x} is C₁-C₆ alkyl; and 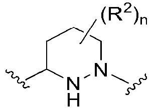 has the structure of Formula Vlla-2: 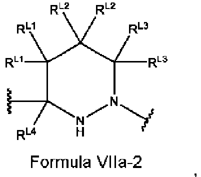 wherein each R^{L1}, R^{L2}, R^{L3}, and R^{L4} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, and R^{L4} together with the atoms to which they are attached and any intervening atoms form an optionally substituted C₄-C₈ cycloalkyl or a 3-to 8-membered heterocyclyl; and 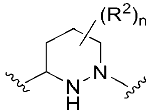 does not have the structure of:

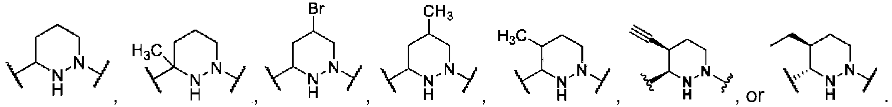

2. The compound of claim 1, or a salt thereof, wherein:
R^{L4} is hydrogen; or
R^{L1} and R^{L4} combine to form an optionally substituted C₄ cycloalkyl; or
R^{L1} and R^{L3} combine to form an optionally substituted C₄ cycloalkyl; or
R^{L1} and R^{L3} combine to form an optionally substituted C₅ cycloalkyl; or
two R^{L1} combine to form an optionally substituted C₄-C₆ cycloalkyl; or
R^{L1} and R^{L2} combine to form an optionally substituted C₄-C₆ cycloalkyl.

3. The compound of claims 1 or 2, wherein the compound is: 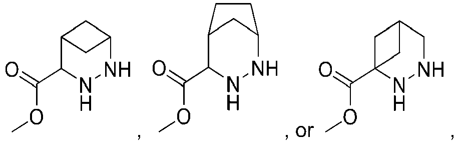 or is a salt thereof;
optionally wherein the compound is 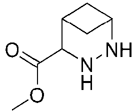 or a salt thereof;
optionally wherein the compound is

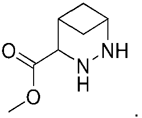

4. The compound of claim 1 or 2, wherein the compound has the structure of formula [A-1] or formula [B-1]:

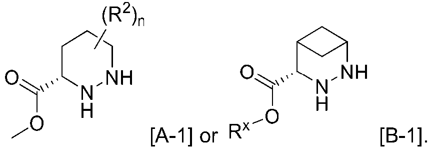

5. The compound of any one of claims 1 to 4, wherein the compound is 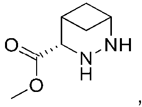 or a salt thereof; optionally wherein the compound is

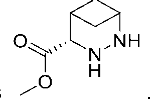

6. A compound having the structure of formula [A-2] or formula [B-2]: 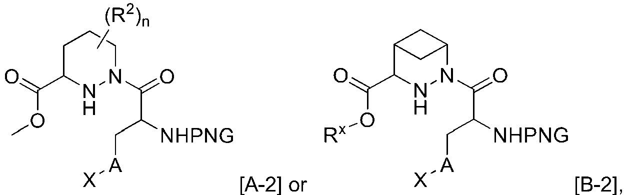 or a salt thereof, wherein:
X is halogen;
PNG is a nitrogen protecting group;
A is optionally substituted C₂-C₄ alkylene, optionally substituted C₁-C₄ heteroalkylene, optionally substituted C₂-C₄ alkenylene, optionally substituted 3- to 6-membered cycloalkylene, optionally substituted 3- to 6-membered heterocycloalkylene, optionally substituted 6-membered arylene, or optionally substituted 5- to 10-membered heteroarylene;
R^{x} is C₁-C₆ alkyl; and 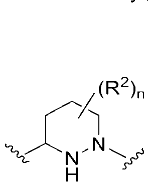 has the structure of Formula Vlla-2: 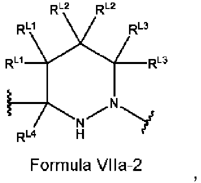
wherein each R^{L1}, R^{L2}, R^{L3}, and R^{L4} is, independently, hydrogen, halogen, hydroxyl, optionally substituted C₁-C₆ alkyl, optionally substituted C₂-C₆ alkenyl, optionally substituted C₂-C₆ alkynyl, or optionally substituted C₁-C₆ heteroalkyl; or any two of R^{L1}, R^{L2}, R^{L3}, and R^{L4} together with the atoms to which they are attached and any intervening atoms form an optionally substituted C₃-C₈ cycloalkyl or a 3-to 8-membered heterocyclyl; and 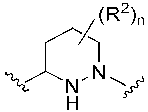 does not have the structure of:

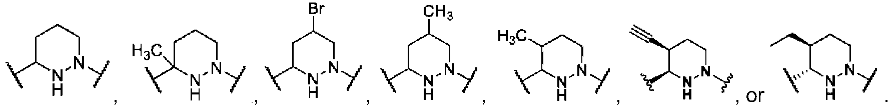

7. The compound of claim 6, or a salt thereof, wherein:
R^{L4} is hydrogen; or
R^{L1} and R^{L4} combine to form an optionally substituted C₄ cycloalkyl; or
R^{L1} and R^{L3} combine to form an optionally substituted C₄ cycloalkyl; or
R^{L1} and R^{L3} combine to form an optionally substituted C₅ cycloalkyl; or
two R^{L1} combine to form an optionally substituted C₄-C₆ cycloalkyl; or
R^{L1} and R^{L2} combine to form an optionally substituted C₄-C₆ cycloalkyl.

8. The compound of claim 6 or 7, or salt thereof, wherein A is: 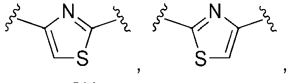 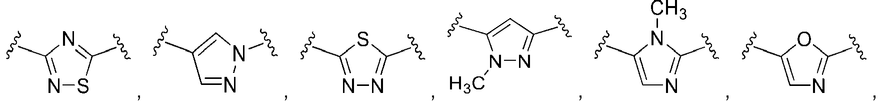 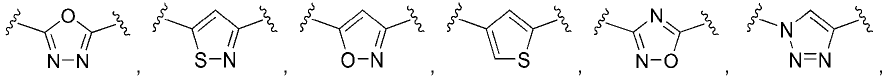 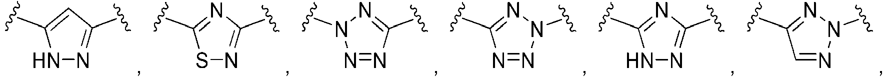 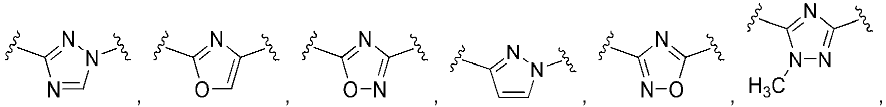 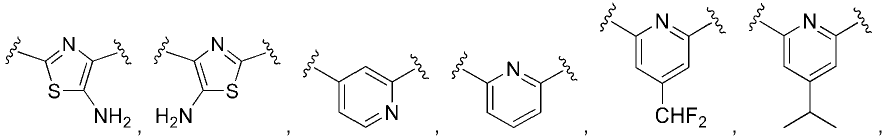 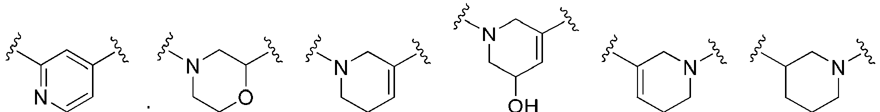 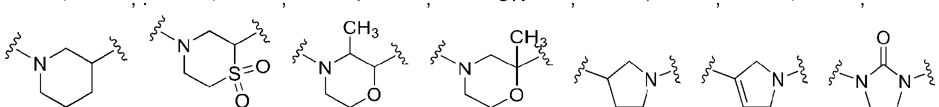 or

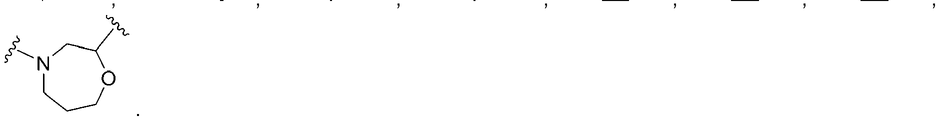

9. The compound of any one of claims 6 to 8, wherein the compound has the structure of: 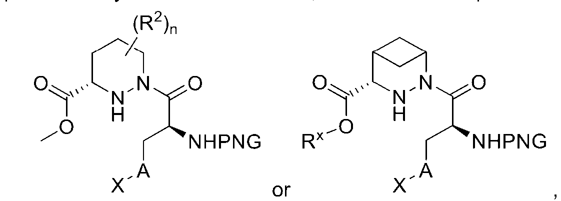 or is a salt thereof.

10. The compound of any one of claims 6 to 9, wherein the compound is: 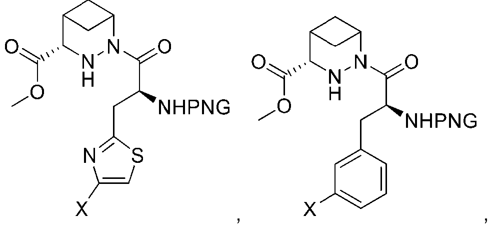 or a salt thereof; optionally wherein the compound is: 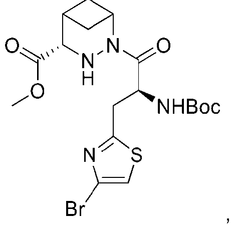 or a salt thereof.

11. A method of preparing a compound of formula [2], the method comprising deprotecting a compound of formula [4] to prepare a compound of formula [3]: 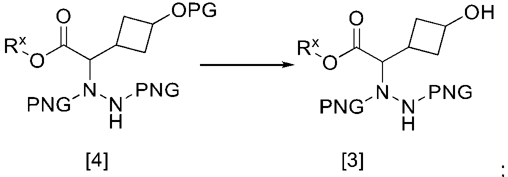
and halogenating the compound of formula [3] to produce the compound of formula [2]: 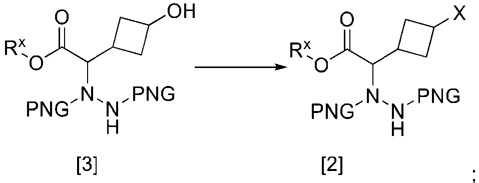
wherein each PNG is independently a nitrogen protecting group, PG is an oxygen protecting group, R^{x} is C₁-C₆ alkyl, and X is halogen.

12. A method of preparing a compound of formula [B] of any one of claims 1 to 5, or a salt thereof, the method comprising:
cyclizing a compound of formula [2] to form a compound of formula [1]: 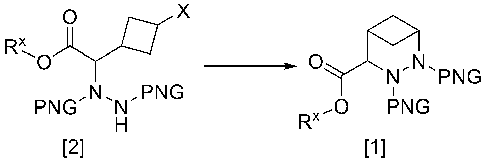
and deprotecting the compound of formula [1] to prepare the compound of formula [B], or salt thereof: 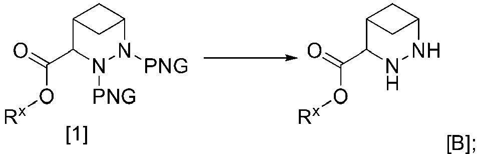
wherein each PNG is independently a nitrogen protecting group and X is halogen.

13. A method of preparing a compound of formula [B] of any one of claims 1 to 5, or a salt thereof, the method comprising the method of claim 11 followed by the method of claim 12.

14. The method of claim 12 or 13, wherein the compound of formula [B] is:

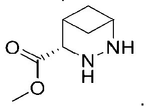

15. A method of preparing a compound formula [A-2] of any one of claims 6 to 10, or salt thereof, comprising coupling a compound of formula [5] with a compound of formula [6]:

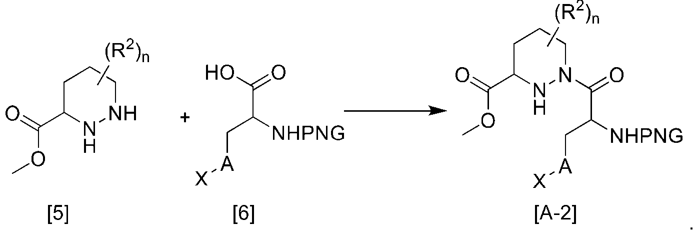

16. A method of preparing the compound of formula [B-2] of any one of claims 6 or 8 to 10, or salt thereof, comprising coupling a compound of formula [7] with a compound of formula [6]:

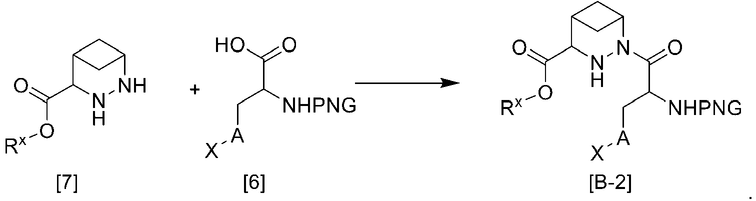

17. The method of claim 15 or 16, comprising coupling compound [8] with a compound of formula [9]:

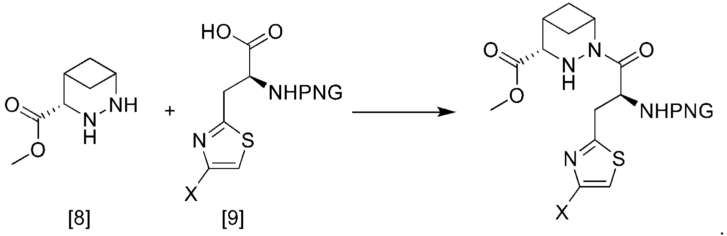

18. The compound of any one of claims 6 to 10, or the method of any one of claims 11 to 17, wherein X is Br and/or wherein each PNG is Boc.
